(19)

Europäisches
Patentamt
European
Patent Office
Office européen
des brevets

(11) **EP 3 423 828 B1**

(12) **EUROPEAN PATENT SPECIFICATION**

(45) Date of publication and mention
of the grant of the patent:
**27.08.2025 Bulletin 2025/35**

(21) Application number: **17760558.1**

(22) Date of filing: **27.02.2017**

(51) International Patent Classification (IPC):
*G16B 20/20* (2019.01)    *C12Q 1/6806* (2018.01)
*C12Q 1/6886* (2018.01)    *G01N 33/50* (2006.01)
*G01N 33/574* (2006.01)    *C40B 20/04* (2006.01)
*C40B 40/06* (2006.01)    *C40B 70/00* (2006.01)

(52) Cooperative Patent Classification (CPC):
(C-Sets available)
**C12Q 1/6886; C12Q 1/6806; G16B 20/20;**
C12Q 2600/156; Y02A 90/10          (Cont.)

(86) International application number:
**PCT/US2017/019763**

(87) International publication number:
**WO 2017/151524 (08.09.2017 Gazette 2017/36)**

(54) **METHODS AND SYSTEMS FOR EVALUATING TUMOR MUTATIONAL BURDEN**

VERFAHREN UND SYSTEME ZUR BEURTEILUNG EINER TUMORMUTATIONSLAST

PROCÉDÉS ET SYSTÈMES PERMETTANT D'ÉVALUER LA CHARGE MUTATIONNELLE D'UNE
TUMEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.02.2016 US 201662301534 P**

(43) Date of publication of application:
**09.01.2019 Bulletin 2019/02**

(73) Proprietors:
• **Foundation Medicine, Inc.
Cambridge, MA 02141 (US)**
• **Genentech, Inc.
South San Francisco, CA 94080 (US)**

(72) Inventors:
• **CHALMERS, Zachary R
Cambridge
MA 02141 (US)**
• **CONNELLY, Caitlin F.
Cambridge, MA 02141 (US)**
• **FABRIZIO, David
Cambridge
MA 02141 (US)**
• **FRAMPTON, Garrett, Michaell
Cambridge, MA 02141 (US)**
• **HEDGE, Priti
South San Francisco
CA 94080 (US)**
• **KOWANETZ, Marcin
South San Francisco, CA 94080 (US)**
• **STEPHENS, Philip J.
Cambridge, MA 02141 (US)**
• **SUN, James, Xin
Cambridge, MA 02141 (US)**
• **YELENSKY, Roman
Newton, MA 02462 (US)**

(74) Representative: **Mewburn Ellis LLP
Aurora Building
Counterslip
Bristol BS1 6BX (GB)**

(56) References cited:
WO-A1-2013/070634      WO-A1-2015/164862
WO-A2-2016/018481      US-A1- 2012 208 706
US-A1- 2014 296 081      US-A1- 2014 336 996

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

EP 3 423 828 B1

- FABBRI G ET AL: "Analysis of the chronic lymphocytic leukemia coding genome: role of NOTCH1 mutational activation", JOURNAL OF EXPERIMENTAL MEDICINE, vol. 208, no. 7, 13 June 2011 (2011-06-13), pages 1389 - 1401, XP055053050, ISSN: 0022-1007, DOI: 10.1084/jem.20110921
- JOHNSON D B ET AL: "Targeted Next Generation Sequencing Identifies Markers of Response to PD-1 Blockade", CANCER IMMUNOLOGY RESEARCH, vol. 4, no. 11, 26 September 2016 (2016-09-26), US, pages 959 - 967, XP055486375, ISSN: 2326-6066, DOI: 10.1158/2326-6066.CIR-16-0143
- ROSENBERG J E ET AL: "Atezolizumab in patients with locally advanced and metastatic urothelial carcinoma who have progressed following treatment with platinum-based chemotherapy: a single-arm, multicentre, phase 2 trial", LANCET, ELSEVIER, AMSTERDAM, NL, vol. 387, no. 10031, 4 March 2016 (2016-03-04), pages 1909 - 1920, XP029530539, ISSN: 0140-6736, DOI: 10.1016/S0140-6736(16)00561-4
- ROSENBERG J E ET AL: "Supplement to: Rosenberg JE, Hoffman-Censits J, Powles T, et al. Atezolizumabin patients with locally advanced and metastatic urothelial carcinoma who haveprogressed following treatment with platinum-based chemotherapy: a single-arm,multicentre, phase 2 trial", INTERNET CITATION, 4 March 2016 (2016-03-04), pages Frontpg - 24, XP002776669, Retrieved from the Internet <URL:https://ars.els-cdn.com/content/image/1-s2.0-S0140673616005614-mmc1.pdf> [retrieved on 1077]
- TAMBORERO D ET AL: "OncodriveCLUST: exploiting the positional clustering of somatic mutations to identify cancer genes", BIOINFORMATICS, vol. 29, no. 18, 24 July 2013 (2013-07-24), pages 2238 - 2244, XP055217187, ISSN: 1367-4803, DOI: 10.1093/bioinformatics/btt395
- CHALMERS Z R ET AL: "Analysis of 100,000 human cancer genomes reveals the landscape of tumor mutational burden", GENOME MEDICINE, vol. 9, no. 1, 19 April 2017 (2017-04-19), XP055510901, DOI: 10.1186/s13073-017-0424-2
- CAMPESATO ET AL.: "Comprehensive cancer-gene panels can be used to estimate mutational load and predict clinical benefit to PD-1 blockade in clinical practice", ONCOTARGET, vol. 6, no. 33, 27 October 2015 (2015-10-27), pages 34221 - 34227, XP055415163

(52) Cooperative Patent Classification (CPC): (Cont.)

C-Sets
**C12Q 1/6806, C12Q 2535/122, C12Q 2537/165, C12Q 2565/519**

**Description**

**CROSS REFERENCE TO RELATED APPLICATIONS**

This application claims the benefit of U.S. Provisional Application No. 62/301,534, filed February 29, 2016.

**FIELD OF INVENTION**

The invention relates to methods of evaluating gene alterations such as tumor mutational burden.

**BACKGROUND OF THE INVENTION**

Cancer cells accumulate mutations during cancer development and progression. These mutations may be the consequence of intrinsic malfunction of DNA repair, replication, or modification, or exposures to external mutagens. Certain mutations have conferred growth advantages on cancer cells and are positively selected in the microenvironment of the tissue in which the cancer arises. While the selection of advantageous mutations contributes to tumorigenesis, the likelihood of generating tumor neoantigens and subsequent immune recognition may also increase as mutations develop (Gubin and Schreiber. Science 350:158-9, 2015). Therefore, total mutation burden, as measured by whole exome sequencing (WES), can be used to guide patient treatment decisions, for example, to predict a durable response to a cancer immunotherapy. However, translating genomic studies to routine clinical practice remains problematic as whole exome sequencing is not widely available and is expensive, time intensive, and technically challenging.

Therefore, the need still exists for novel approaches, including genomic profiling targeting a subset of genome or exome, to accurately measure mutation load in tumor samples.

Campesato, L.F. et al. (2017), "Comprehensive cancer-gene panels can be used to estimate mutational load and predict clinical benefit to PD-1 blockade in clinical practice", Oncotarget, vol. 6, no. 33, pages 34221-34227, discloses methods for determining mutational load of a tumor, including filtering germline variants and enabling unambiguous identification of somatic mutations in therapeutically relevant cancer-genes. However, this disclosure does not teach means to purposively exclude functional alterations having an effect on cell division, growth or survival.

**SUMMARY OF THE INVENTION**

The invention is defined in the appended claims.

The invention is based, at least in part, on the discovery that profiling a small fraction of the genome or exome from a patient sample, *e.g.*, using a hybrid capture-based, next-generation sequencing (NGS) platform, serves as an effective surrogate for the analysis of total mutation load. Using methods that include a targeted NGS approach for detecting mutational burden has several advantages, including, but not limited to, faster, *e.g.,* more clinically manageable turnaround times (~2 weeks), standardized informatics pipelines, and more manageable costs, compared to, *e.g.,* whole genome or whole exome sequencing. The methods disclosed herein have other advantages over traditional markers, such as protein expression detected by histochemistry, since the present methods produce an objective measure (*e.g.,* mutation load) rather than a subjective measure (*e.g.,* pathology scoring). The methods disclosed herein also allow for simultaneous detection of actionable alterations for targeted therapies, as well as mutational burden for immune therapies. These methods can provide clinically actionable predictors of a response to therapies in patients with cancer.

Accordingly, the invention provides, computer-implemented methods of evaluating the mutation load in a sample, by providing a sequence of a set of subgenomic intervals from the sample; and determining a value for the mutational load, wherein the value is a function of the number of alterations in the set of subgenomic intervals. The set of subgenomic intervals are from a predetermined set of genes that does not include the entire genome or exome, wherein the predetermined set of genes comprises a plurality of genes, which in mutant form, are associated with an effect on cell division, growth or survival, or are associated with cancer

In certain embodiments, the set of subgenomic intervals is a set of coding subgenomic intervals. In other embodiments, the set of subgenomic intervals contains both a coding subgenomic interval and a non-coding subgenomic interval. In certain embodiments, the value for the mutation load is a function of the number of an alteration (*e.g.,* a somatic alteration) in the set of subgenomic intervals. The number of an alteration excludes a functional alteration and a germline alteration. In some embodiments, the sample is a tumor sample or a sample derived from a tumor. The methods described herein can also include, *e.g.,* one or more of: acquiring a library comprising a plurality of tumor members from the sample; contacting the library with a bait set to provide selected tumor members by hybridization, thereby providing a library catch; acquiring a read for a subgenomic interval comprising an alteration from the tumor member from the library catch; aligning the read by an alignment method; assigning a nucleotide value from the read for a preselected nucleotide position; and selecting a set of subgenomic intervals from a set of the assigned nucleotide positions, wherein the set of subgenomic intervals are from a predetermined set of genes.

In one aspect, the invention features a method of evaluating the mutation load in a sample, *e.g.,* a tumor sample (*e.g.,* a sample acquired from a tumor). The method includes:

a) providing a sequence, *e.g.,* a nucleotide sequence, of a set of subgenomic intervals (*e.g.,* coding subgenomic intervals) from the sample, wherein the set of subgenomic intervals are from a predetermined set of genes; and

b) determining a value for the mutation load, wherein the value is a function of the number of an alteration (*e.g.,* one or more alterations), *e.g.,* a somatic alteration (*e.g.,* one or more somatic alterations), in the set of subgenomic intervals.

In certain embodiments, the number of an alteration excludes a functional alteration in a subgenomic interval. In other embodiments, the number of an alteration excludes a germline alteration in a subgenomic interval. In certain embodiments, the number of an alteration excludes a functional alteration in a subgenomic interval and a germline alteration in a subgenomic interval.

In certain embodiments, the set of subgenomic intervals comprises coding subgenomic intervals. In other embodiments, the set of subgenomic intervals comprises non-coding subgenomic intervals. In certain embodiments, the set of subgenomic intervals comprises coding subgenomic intervals. In other embodiments, the set of subgenomic intervals comprises one or more coding subgenomic intervals and one or more non-coding subgenomic intervals. In certain embodiments, about 5% or more, about 10% or more, about 20% or more, about 30% or more, about 40% or more, about 50% or more, about 60% or more, about 70% or more, about 80% or more, about 90% or more, or about 95% or more, of the subgenomic intervals in the set of subgenomic intervals are coding subgenomic intervals. In other embodiments, about 90% or less, about 80% or less, about 70% or less, about 60% or less, about 50% or less, about 40% or less, about 30% or less, about 20% or less, about 10% or less, or about 5% or less, of the subgenomic intervals in the set of subgenomic intervals are non-coding subgenomic intervals.

In other embodiments, the set of subgenomic intervals does not comprise the entire genome or the entire exome. In other embodiments, the set of coding subgenomic intervals does not comprise the entire exome.

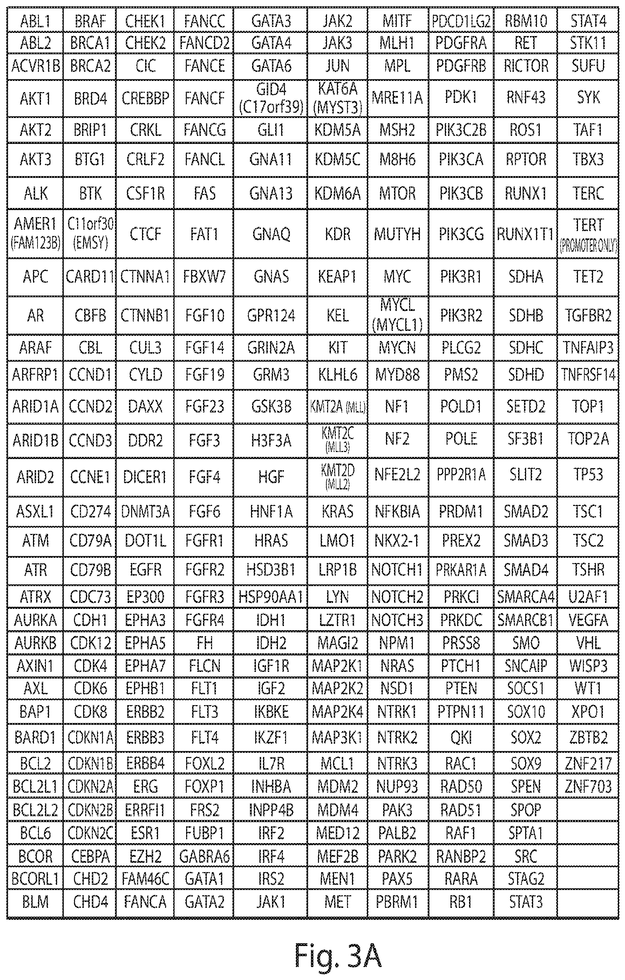

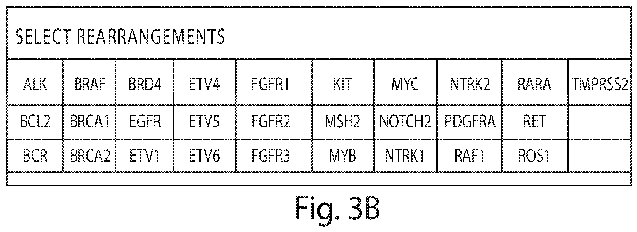

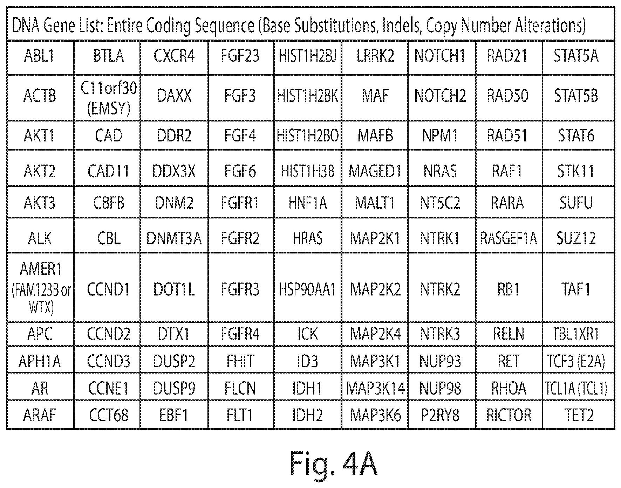

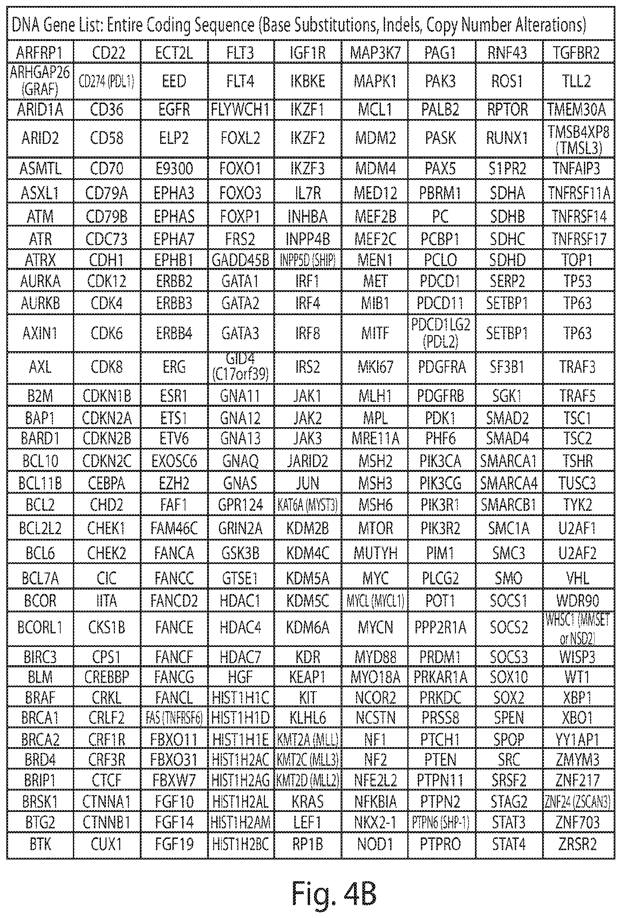

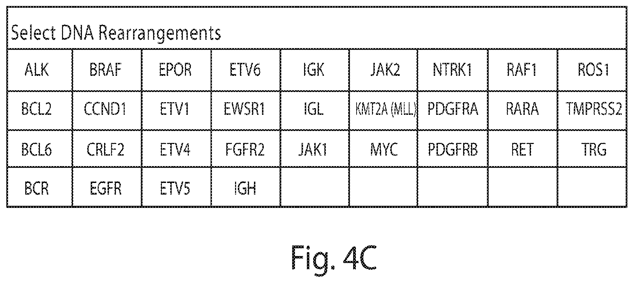

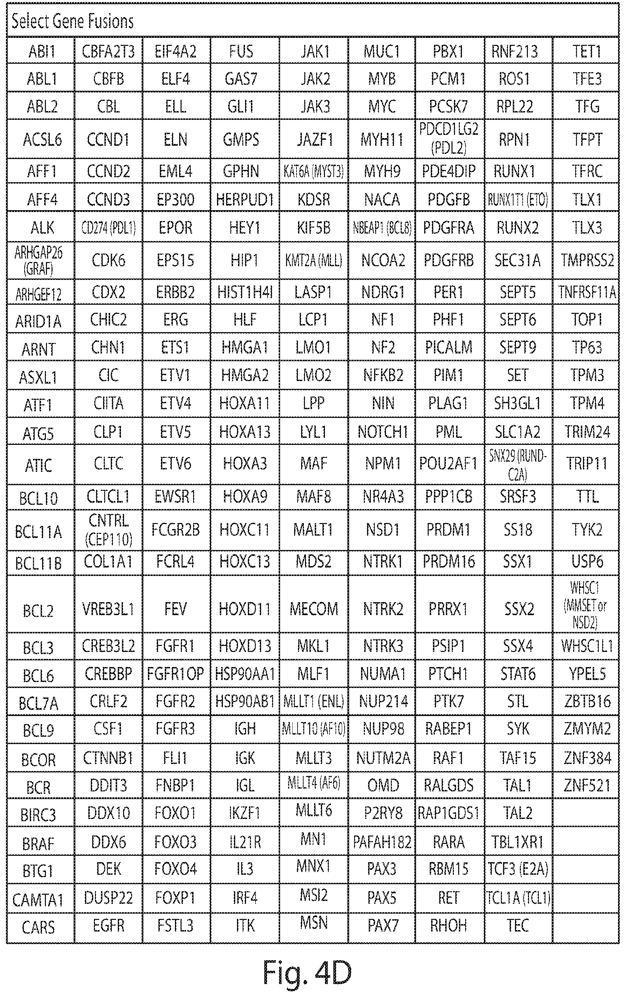

In certain embodiments, the predetermined set of genes does not comprise the entire genome or the entire exome. In other embodiments, the predetermined set of genes comprises or consists of one or more genes set forth in **Tables 1-4 or FIGs. 3A-4D.**

In certain embodiments, the value is expressed as a function of the predetermined set of genes. In certain embodiments, the value is expressed as a function of the coding regions of the predetermined set of genes. In other embodiments, the value is expressed as a function of the non-coding regions of the predetermined set of genes. In certain embodiments, the value is expressed as a function of the exons of the predetermined set of genes. In other embodiments, the value is expressed as a function of the introns of the predetermined set of genes.

In certain embodiments, the value is expressed as a function of the predetermined set of genes sequenced. In certain embodiments, the value is expressed as a function of the coding regions of the predetermined set of genes sequenced. In other embodiments, the value is expressed as a function of the non-coding regions of the predetermined set of genes sequenced. In certain embodiments, the value is expressed as a function of the exons of the predetermined set of genes sequenced. In other embodiments, the value is expressed as a function of the introns of the predetermined set of genes sequenced.

In certain embodiments, the value is expressed as a function of the number of an alteration (*e.g.,* a somatic alteration) in a preselected number of positions of the predetermined set of genes. In certain embodiments, the value is expressed as a function of the number of an alteration (*e.g.,* a somatic alteration) in a preselected number of positions of the coding regions of the predetermined set of genes. In other embodiments, the value is expressed as a function of the number of an alteration (*e.g.,* a somatic alteration) in a preselected number of positions of the non-coding regions of the predetermined set of genes. In certain embodiments, the value is expressed as a function of the number of an alteration (*e.g.,* a somatic alteration) in a preselected number of positions of the exons of the predetermined set of genes. In other embodiments, the value is expressed as a function of the number of an alteration (*e.g.,* a somatic alteration) in a preselected number of positions of the introns of the predetermined set of genes.

In certain embodiments, the value is expressed as a function of the number of an alteration (*e.g.,* a somatic alteration) in a preselected number of positions of the predetermined set of genes sequenced. In certain embodiments, the value is expressed as a function of the number of an alteration (*e.g.,* a somatic alteration) in a preselected number of positions of the coding regions of the predetermined set of genes sequenced. In other embodiments, the value is expressed as a function of the number of an alteration (*e.g.,* a somatic alteration) in a preselected number of positions of the non-coding regions of the predetermined set of genes sequenced. In certain embodiments, the value is expressed as a function of the number of an alteration (*e.g.,* a somatic alteration) in a preselected number of positions of the exons of the predetermined set of genes sequenced. In other embodiments, the value is expressed as a function of the number of an alteration (*e.g.,* a somatic alteration) in a preselected number of positions of the introns of the predetermined set of genes sequenced.

In certain embodiments, the value is expressed as a function of the number of an alteration (*e.g.,* a somatic alteration) per a preselected unit, *e.g.,* as a function of the number of a somatic alteration per megabase.

In certain embodiments, the value is expressed as a function of the number of an alteration (*e.g.,* a somatic alteration) per megabase in the predetermined set of genes. In certain embodiments, the value is expressed as a function of the number of an alteration (*e.g.,* a somatic alteration) per megabase in the coding regions of the predetermined set of genes. In other embodiments, the value is expressed as a function of the number of an alteration (*e.g.,* a somatic alteration) per megabase in the non-coding regions of the predetermined set of genes. In certain embodiments, the value is expressed as a function of the number of an alteration (*e.g.,* a somatic alteration) per megabase in the exons of the predetermined set of genes. In other embodiments, the value is expressed as a function of the number of an alteration (*e.g.*, somatic alteration) per megabase in the introns of the predetermined set of genes.

In certain embodiments, the value is expressed as a function of the number of an alteration (*e.g.,* a somatic alteration) per megabase in the predetermined set of genes sequenced. In certain embodiments, the value is expressed as a function of the number of an alteration (*e.g.,* a somatic alteration) per megabase in the coding regions of the predetermined set of genes sequenced. In other embodiments, the value is expressed as a function of the number of an alteration (*e.g.,* a somatic alteration) per megabase in the non-coding regions of the predetermined set of genes sequenced. In certain embodiments, the value is expressed as a function of the number of an alteration (*e.g.,* a somatic alteration) per megabase in the exons of the predetermined set of genes sequenced. In other embodiments, the value is expressed as a function of the number of an alteration (*e.g.,* a somatic alteration) per megabase in the introns of the predetermined set of genes sequenced.

In certain embodiments, the mutation load is extrapolated to a larger portion of the genome, *e.g.,* to the exome or the entire genome, *e.g.,* to obtain the total mutation load. In other embodiments, the mutation load is extrapolated to a larger portion of the exome, *e.g.,* to the entire exome.

In certain embodiments, the sample is from a subject. In certain embodiments, the subject has a disorder, *e.g.*, a cancer. In other embodiments, the subject is receiving, or has received, a therapy, *e.g.,* an immunotherapy.

In certain embodiments, the mutation load is expressed as a percentile, *e.g.*, among the mutation loads in samples from a reference population. In certain embodiments, the reference population includes patients having the same type of cancer as the subject. In other embodiments, the reference population includes patients who are receiving, or have received, the same type of therapy, as the subject.

In another aspect, the invention features a method of evaluating the mutation load in a sample, *e.g.,* a tumor sample or a sample derived from a tumor. The method includes:

(i) acquiring a library comprising a plurality of tumor members from the sample;
(ii) contacting the library with a bait set to provide selected tumor members, wherein said bait set hybridizes with the tumor member, thereby providing a library catch;
(iii) acquiring a read for a subgenomic interval comprising an alteration (*e.g.,* a somatic alteration) from a tumor member from said library catch, *e.g.,* by a next-generation sequencing method;
(iv) aligning said read by an alignment method;
(v) assigning a nucleotide value from said read for a preselected nucleotide position;
(vi) selecting a set of subgenomic intervals (*e.g.*, coding subgenomic intervals) from a set of the assigned nucleotide positions, wherein the set of subgenomic intervals are from a predetermined set of genes; and
(vii) determining a value for the mutational load, wherein the value is a function of the number of an alteration (*e.g.,* one or more alterations), *e.g.,* a somatic alteration (*e.g.,* one or more somatic alterations), in the set of subgenomic intervals.

In certain embodiments, the number of an alteration (*e.g.*, a somatic alteration) excludes a functional alteration in a subgenomic interval. In other embodiments, the number of an alteration excludes a germline alteration in a subgenomic interval. In certain embodiments, the number of an alteration (*e.g.*, a somatic alteration) excludes a functional alteration in a subgenomic interval and a germline alteration in a subgenomic interval.

Various types of alterations (*e.g.*, somatic alterations) can be evaluated and used for the analysis of mutation load, in a method or system as described herein.

*Somatic Alterations*

In certain embodiments, the alteration evaluated in accordance with a method described herein is an alteration (*e.g.,* a somatic alteration).

In certain embodiments, the alteration (*e.g.,* somatic alteration) is a coding short variant, *e.g.*, a base substitution or an indel (insertion or deletion). In certain embodiments, the alteration (*e.g.,* somatic alteration) is a point mutation. In other embodiments, the alteration (*e.g.*, somatic alteration) is other than a rearrangement, *e.g.*, other than a translocation.

In certain embodiments, the alteration (*e.g.*, somatic alteration) is a splice variant.

In certain embodiments, the alteration (*e.g.,* somatic alteration) is a silent mutation, *e.g.*, a synonymous alteration. In other embodiments, the alteration (*e.g.*, somatic alteration) is a non-synonymous single nucleotide variant (SNV). In other embodiments, the alteration (*e.g.*, somatic alteration) is a passenger mutation, *e.g.*, an alteration that has no detectable effect on the fitness of a clone of cells. In certain embodiments, the alteration (*e.g.*, somatic alteration) is a variant of unknown significance (VUS), *e.g.,* an alteration, the pathogenicity of which can neither be confirmed nor ruled out. In certain embodiments, the alteration (*e.g.*, somatic alteration) has not been identified as being associated with a cancer phenotype.

In certain embodiments, the alteration (*e.g.*, somatic alteration) is not associated with, or is not known to be associated with, an effect on cell division, growth or survival. In other embodiments, the alteration (*e.g.*, somatic alteration) is associated with an effect on cell division, growth or survival.

In certain embodiments, an increased level of a somatic alteration is an increased level of one or more classes or types of a somatic alteration (*e.g.*, a rearrangement, a point mutation, an indel, or any combination thereof). In certain embodiments, an increased level of a somatic alteration is an increased level of one class or type of a somatic alteration (*e.g.,* a rearrangement only, a point mutation only, or an indel only). In certain embodiments, an increased level of a somatic alteration is an increased level of a somatic alteration at a preselected position (*e.g.,* an alteration described herein). In certain embodiments, an increased level of a somatic alteration is an increased level of a preselected somatic alteration (*e.g.*, an alteration described herein).

*Functional Alterations*

The number of an alteration (*e.g.,* a somatic alteration) excludes a functional alteration in a subgenomic interval.

The functional alteration is an alteration that, compared with a reference sequence, *e.g.,* a wild-type or unmutated sequence, has an effect on cell division, growth or survival, *e.g.,* promotes cell division, growth or survival. The functional alteration is identified as such by inclusion in a database of functional alterations, *e.g.*, the COSMIC database (cancer.sanger.ac.uk/cosmic; Forbes et al. Nucl. Acids Res. 2015; 43 (D1): D805-D811). In other embodiments, the functional alteration is an alteration with known functional status, *e.g.*, occurring as a known somatic alteration in the COSMIC database. In certain embodiments, the functional alteration is an alteration with a likely functional status, *e.g.,* a truncation in a tumor suppressor gene. In certain embodiments, the functional alteration is a driver mutation, *e.g.*, an alteration that gives a selective advantage to a clone in its microenvironment, *e.g.*, by increasing cell survival or reproduction. In other embodiments, the functional alteration is an alteration capable of causing clonal expansions. In certain embodiments, the functional alteration is an alteration capable of causing one, two, three, four, five, or all of the following: (a) self-sufficiency in a growth signal; (b) decreased, *e.g.*, insensitivity, to an antigrowth signal; (c) decreased apoptosis; (d) increased replicative potential; (e) sustained angiogenesis; or (f) tissue invasion or metastasis.

In certain embodiments, the functional alteration is not a passenger mutation, *e.g.*, is not an alteration that has no detectable effect on the fitness of a clone of cells. In certain embodiments, the functional alteration is not a variant of unknown significance (VUS), *e.g.,* is not an alteration, the pathogenicity of which can neither be confirmed nor ruled out.

In certain embodiments, a plurality (*e.g.*, about 10%, 20%, 30%, 40%, 50%, 60%, 70%, 80%, 90%, or more) of functional alterations in a preselected tumor gene in the predetermined set of genes are excluded. In certain embodiments, all functional alterations in a preselected gene (*e.g.,* tumor gene) in the predetermined set of genes are excluded. In certain embodiments, a plurality of functional alterations in a plurality of preselected genes (*e.g.*, tumor genes) in the predetermined set of genes are excluded. In certain embodiments, all functional alterations in all genes (*e.g.,* tumor genes) in the predetermined set of genes are excluded.

*Germline Mutations*

The number of an alteration excludes a germline mutation in a subgenomic interval. In certain embodiments, the somatic alteration is not identical or similar to, *e.g.,* is distinguishable from, a germline mutation.

In certain embodiments, the germline alteration is a single nucleotide polymorphism (SNP), a base substitution, an indel (*e.g.*, an insertion or a deletion), or a silent mutation (*e.g.*, synonymous mutation).

In certain embodiments, the germline alteration is excluded by use of a method that does not use a comparison with a matched normal sequence. In other embodiments, the germline alteration is excluded by a method comprising the use of an SGZ algorithm. In certain embodiments, the germline alteration is identified as such by inclusion in a database of germline alterations, *e.g.*, the dbSNP database (www.ncbi.nlm.nih.gov/SNP/index.html; Sherry et al. Nucleic Acids Res. 2001; 29(1): 308-311). In other embodiments, the germline alteration is identified as such by inclusion in two or more counts of the ExAC database (exac.broadinstitute.org; Exome Aggregation Consortium et al. "Analysis of protein-coding genetic variation in 60,706 humans," bioRxiv preprint. October 30, 2015). In some embodiments, the germline alteration is identified as such by inclusion in the 1000 Genome Project database (www.1000genomes.org; McVean et al. Nature.

2012; 491, 56-65). In some embodiments, the germline alteration is identified as such by inclusion in the ESP database (Exome Variant Server, NHLBI GO Exome Sequencing Project (ESP), Seattle, WA (evs.gs.washington.edu/EVS/).

*MULTIGENE ANALYSIS*

The methods and systems described herein evaluate, *e.g.*, a set of subgenomic intervals, *e.g.*, from a predetermined set of genes.

In certain embodiments, the predetermined set of genes comprises a plurality of genes, which in mutant form, are associated with an effect on cell division, growth or survival, or are associated with a cancer, *e.g.,* a cancer described herein.

In certain embodiments, the predetermined set of genes comprises at least about 50 or more, about 100 or more, about 150 or more, about 200 or more, about 250 or more, about 300 or more, about 350 or more, about 400 or more, about 450 or more, about 500 or more, about 550 or more, about 600 or more, about 650 or more, about 700 or more, about 750 or more, or about 800 or more genes, *e.g.,* as described herein. In some embodiments, the predetermined set of genes comprises at least about 50 or more, about 100 or more, about 150 or more, about 200 or more, about 250 or more, about 300 or more, or all of the genes or gene products chosen from **Tables 1-4 or FIGs. 3A-4D.**

In certain embodiments, the method further comprises acquiring a library comprising a plurality of tumor members from the sample. In certain embodiments, the method further comprises contacting a library with a bait set to provide selected tumor members, wherein said bait set hybridizes with a tumor member from the library, thereby providing a library catch. In certain embodiments, the method further comprises acquiring a read for a subgenomic interval comprising the alteration (*e.g.*, somatic alteration) from a tumor member from a library or library catch, thereby acquiring a read for the subgenomic interval, *e.g.*, by a next-generation sequencing method. In certain embodiments, the method further comprises aligning a read for the subgenomic interval by an alignment method, *e.g.*, an alignment method described herein. In certain embodiments, the method further comprises assigning a nucleotide value for a preselected nucleotide position from a read for the subgenomic interval, *e.g.,* by a mutation calling method described herein.

In certain embodiments, the method further comprises one, two, three, four, or all of:

(a) acquiring a library comprising a plurality of tumor members from the sample;
(b) contacting the library with a bait set to provide selected tumor members, wherein said bait set hybridizes with the tumor member, thereby providing a library catch;
(c) acquiring a read for a subgenomic interval comprising the alteration (*e.g.*, somatic alteration) from a tumor member from said library catch, thereby acquiring a read for the subgenomic interval, *e.g.,* by a next-generation sequencing method;
(d) aligning said read by an alignment method, *e.g.*, an alignment method described herein; or
(e) assigning a nucleotide value from said read for a preselected nucleotide position, *e.g.,* by a mutation calling method described herein.

In certain embodiments, acquiring a read for the subgenomic interval comprises sequencing a subgenomic interval from at least about 50 or more, about 100 or more, about 150 or more, about 200 or more, about 250 or more, about 300 or more, about 350 or more, about 400 or more, about 450 or more, about 500 or more, about 550 or more, about 600 or more, about 650 or more, about 700 or more, about 750 or more, or about 800 or more genes. In certain embodiments, acquiring a read for the subgenomic interval comprises sequencing a subgenomic interval from at least about 50 or more, about 100 or more, about 150 or more, about 200 or more, about 250 or more, about 300 or more, or all of the genes or gene products chosen from **Tables 1-4 or FIGs. 3A-4D.**

In certain embodiments, acquiring a read for the subgenomic interval comprises sequencing with greater than about 250X average unique coverage. In other embodiments, acquiring a read for the subgenomic interval comprises sequencing with greater than about 500X average unique coverage. In other embodiments, acquiring a read for the subgenomic interval comprises sequencing with greater than about 1,000X average unique coverage.

In certain embodiments, acquiring a read for the subgenomic interval comprises sequencing with greater than about 250X average unique coverage, at greater than about 99% of genes (*e.g.,* exons) sequenced. In other embodiments, acquiring a read for the subgenomic interval comprises sequencing with greater than about 500X average unique coverage, at greater than about 95% of genes (*e.g.*, exons) sequenced. In certain embodiments, acquiring a read for the subgenomic interval comprises sequencing with greater than about 250X average, greater than about 500X average, or greater than about 1,000X average, unique coverage, at greater than about 99% of genes (*e.g.*, exons) sequenced.

In certain embodiments, the sequence, *e.g.*, a nucleotide sequence, of a set of subgenomic intervals (*e.g.*, coding subgenomic intervals), described herein, is provided by a method described herein. In certain embodiments, the sequence is provided without using a method that includes a matched normal control (*e.g.,* a wild-type control), a matched tumor control (*e.g.,* primary vs. metastatic), or both.

*SGZ ANALYSIS*

In certain embodiments, the germline alteration is excluded by a method or system comprising the use of an SGZ algorithm.

In certain embodiments, the method further comprises characterizing a variant, *e.g.,* an alteration, in the tumor sample by:

a) acquiring:

i) a sequence coverage input (SCI), which comprises, for each of a plurality of selected subgenomic intervals, a value for normalized sequence coverage at the selected subgenomic intervals, wherein SCI is a function of the number of reads for a subgenomic interval and the number of reads for a process-matched control;

ii) an SNP allele frequency input (SAFI), which comprises, for each of a plurality of selected germline SNPs, a value for the allele frequency in the tumor sample, wherein SAFI is based, at least in part, on a minor or alternative allele frequency in the tumor sample; and

iii) a variant allele frequency input (VAFI), which comprises the allele frequency for said variant in the tumor sample;

b) acquiring values, as a function of SCI and SAFI, for:

i) a genomic segment total copy number (C) for each of a plurality of genomic segments;

ii) a genomic segment minor allele copy number (M) for each of a plurality of genomic segments; and

iii) sample purity (p),

wherein the values of C, M, and p are obtained by fitting a genome-wide copy number model to SCI and SAFI; and

c) acquiring:

a value for mutation type, g, for which is indicative of the variant, being somatic, a subclonal somatic variant, germline, or not-distinguishable, and is a function of VAFI, p, C, and M.

In certain embodiments, the method further comprises sequencing each of a plurality of selected subgenomic intervals, each of a plurality of selected germline SNPs, and a variant (*e.g.*, an alteration), wherein the average sequence coverage prior to normalization is at least about 250x, *e.g.,* at least about 500x.

In certain embodiments, fitting the genome-wide copy number model to SCI comprises using the equation of:

$$\log Ratio_i = \log_2 \frac{pC_i + 2(1-p)}{p\psi + 2(1-p)},$$

where $\psi$ is tumor ploidy.

In certain embodiments, fitting the genome-wide copy number model to SAFI comprises using the equation of:

$$AF = \frac{pM + 1(1-p)}{pC + 2(1-p)},$$

where AF is allele frequency.

In certain embodiments, g is determined by determining the fit of values for VAFI, p, C, and M to a model for somatic/germline status. In certain embodiments, the value of g is acquired by:

$$AF = \frac{pM + g(1-p)}{pC + 2(1-p)},$$

where AF is allele frequency.

In certain embodiments, a value of g that is 0, or close to 0 indicates that the variant is a somatic variant; a value of g that is 1, or close to 1 indicates that the variant is a germline variant; a value of g that is less than 1 but more than 0 indicates an indistinguishable result; or a value of g that is significantly less than 0 indicates that the variant is a subclonal somatic variant.

The SGZ algorithm is described in International Application Publication No. WO2014/183078 and U.S. Application Publication No. 2014/0336996. The SGZ algorithm is also described in Sun et al. Cancer Research 2014; 74(19S):1893-1893.

*SAMPLES, E.G., TUMOR SAMPLES*

The methods and systems described herein can be used to evaluate mutation load in various types of samples from a number of different sources.

In some embodiments, the sample is a tumor sample or a sample derived from a tumor. In certain embodiments, the sample is acquired from a solid tumor, a hematological cancer, or a metastatic form thereof. In certain embodiments, the sample is obtained from a subject having a cancer, or a subject who is receiving a therapy or has received a therapy, as described herein.

In some embodiments, the sample (*e.g.*, tumor sample) comprises one or more of: premalignant or malignant cells; cells from a solid tumor, a soft tissue tumor or a metastatic lesion; tissue or cells from a surgical margin; a histologically normal tissue; one or more circulating tumor cells (CTC); a normal adjacent tissue (NAT); a blood sample from the same subject having or at risk of having the tumor; or an FFPE sample. In certain embodiments, the sample comprises a circulating tumor DNA (ctDNA).

In certain embodiments, the sample is a FFPE sample. In certain embodiments, the FFPE sample has one, two or all of the following properties: (a) has a surface area of about 10 mm$^2$ or greater, about 25 mm$^2$ or greater, or about 50 mm$^2$ or greater; (b) has a sample volume of about 1 mm$^3$ or greater, about 2 mm$^3$ or greater, about 3 mm$^3$ or greater, about 4 mm$^3$ or greater, or about 5 mm$^3$ or greater; or (c) has a nucleated cellularity of about 50% or more, about 60% or more, about 70% or more, about 80% or more, or about 90% or more, or about 10,000 cells or more, about 20,000 cells or more, about 30,000 cells or more, about 40,000 cells or more, or about 50,000 cells or more.

*Systems*

In another aspect, the invention features a system for evaluating the mutation load in a sample (*e.g.,* a tumor sample or a sample derived from a tumor). The system includes at least one processor operatively connected to a memory, the at least one processor when executing is configured to:

a) acquire a sequence, *e.g.,* a nucleotide sequence, of a set of subgenomic intervals (*e.g.,* coding subgenomic intervals) from the sample, wherein the set of coding subgenomic intervals are from a predetermined set of genes; and
b) determine a value for the mutational load, wherein the value is a function of the number of an alteration (*e.g.,* a somatic alteration) in the set of subgenomic intervals.

In certain embodiments, said number of an alteration excludes: (i) a functional alteration in a subgenomic interval (*e.g.,* a coding subgenomic interval), (ii) a germline alteration in a subgenomic interval (*e.g.*, a coding subgenomic interval), or (iii) both.

*APPLICATIONS*

In some embodiment, the method further comprises selecting a treatment responsive to the evaluation of mutation load, *e.g.,* an increased level of the mutation load. In some embodiment, the method further comprises administering a treatment responsive to the evaluation of mutation load, *e.g.,* an increased level of the mutation load. In some embodiment, the method further comprises classifying the sample or the subject from which the sample was derived responsive to an evaluation of mutational load. In some embodiment, the method further comprises generating and delivering a report, *e.g.,* an electronic, web-based, or paper report, to the patient or to another person or entity, a caregiver, a physician, an oncologist, a hospital, clinic, third-party payor, insurance company or government office. In some embodiment, the report comprises output from the method which includes the mutation load.

Additional aspects or embodiments of the invention include one or more of the following.

*ALIGNMENT*

Methods disclosed herein can integrate the use of multiple, individually tuned, alignment methods or algorithms to optimize performance in sequencing methods, particularly in methods that rely on massively parallel sequencing of a large number of diverse genetic events in a large number of diverse genes, *e.g.,* methods of analyzing tumor samples, *e.g..* from a cancer described herein. In embodiments, multiple alignment methods that are individually customized or tuned to each of a number of variants in different genes are used to analyze reads. In embodiments, tuning can be a function of (one or more of) the gene (or other subgenomic interval) being sequenced, the tumor type in the sample, the variant being sequenced, or a characteristic of the sample or the subject. The selection or use of alignment conditions that are individually tuned to a number of subject intervals (*e.g.*, subgenomic intervals, expressed subgenomic intervals, or both) to be sequenced allows optimization of speed, sensitivity and specificity. The method is particularly effective when the alignments of reads for a relatively large number of diverse subject intervals (*e.g.*, subgenomic intervals, expressed subgenomic intervals, or both) are optimized.

Accordingly, in one aspect, the invention features a method of analyzing a sample, *e.g.*, a tumor sample from a hematologic malignancy (or premaligancy), *e.g.*, a hematologic malignancy (or premaligancy) described herein. The method comprises:

(a) acquiring one or a plurality of libraries comprising a plurality members from a sample, *e.g.*, a plurality of tumor members from a tumor sample;

(b) optionally, enriching the one or a plurality of libraries for preselected sequences, *e.g.*, by contacting the one or a plurality of libraries with a bait set (or plurality of bait sets) to provide selected members (sometimes referred to herein as library catch);

(c) acquiring a read for a subject interval, *e.g.*, a subgenomic interval or an expressed subgenomic interval, from a member, *e.g.*, a tumor member from a library or library catch, *e.g.*, by a method comprising sequencing, *e.g.*, with a next-generation sequencing method;

(d) aligning said read by an alignment method, *e.g.*, an alignment method described herein; and

(e) assigning a nucleotide value (*e.g.*, calling a mutation, *e.g.*, with a Bayesian method) from said read for the preselected nucleotide position,

thereby analyzing said tumor sample,
optionally wherein:
a read from each of X unique subject intervals (*e.g.*, subgenomic intervals, expressed subgenomic intervals, or both) is aligned with a unique alignment method, wherein unique subject interval (*e.g.*, subgenomic interval or expressed subgenomic interval) means different from the other X-1 subject intervals (*e.g.*, subgenomic intervals, expressed subgenomic intervals, or both), and wherein unique alignment method means different from the other X-1 alignment methods, and X is at least 2.

In an embodiment, the method comprises acquiring a library from which a member corresponding to a subgenomic interval and a member corresponding to an expressed subgenomic interval, are each obtained.

In an embodiment, the method comprises acquiring a first library from which a member corresponding to a subgenomic interval is obtained and acquiring a second library from which a member corresponding to an expressed subgenomic interval is obtained.

In an embodiment, a bait set is used to provide members or a library catch comprising both a subgenomic interval and an expressed interval.

In an embodiment, a first bait set is used to provide members or a library catch comprising a subgenomic interval and a second bait set is used to provide members or a library catch comprising an expressed subgenomic interval.

In an embodiment, step (b) is present. In an embodiment step (b) is absent.

In an embodiment, X is at least 3, 4, 5, 10, 15, 20, 30, 50, 100, 200, 300, 400, 500, 600, 700, 800, 900, or 1,000.

In an embodiment, subject intervals (*e.g.*, subgenomic intervals, expressed subgenomic intervals, or both) from at least X genes, *e.g.* at least X genes from **Tables 1-4 or FIGs. 3A-4D,** are aligned with unique alignment methods, and X is equal to 2, 3, 4, 5, 10, 15, 20, 30, 40, 50, 60, 70, 80, 90, 100, 200, 300, 400, 500, or greater.

In an embodiment, a method (*e.g.*, element (d) of the method recited above) comprises selecting or using an alignment method for analyzing, *e.g.*, aligning, a read,
wherein said alignment method is a function of, is selected responsive to, or is optimized for, one or more or all of:

(i) tumor type. *e.g.*, the tumor type in said sample;

(ii) the gene, or type of gene, in which said subject interval (*e.g.*, subgenomic interval or expressed subgenomic interval) being sequenced is located, *e.g.*, a gene or type of gene characterized by a preselected or variant or type of variant, *e.g.*, a mutation, or by a mutation of a preselected frequency;

(iii) the site (*e.g.*, nucleotide position) being analyzed;

(iv) the type of variant, *e.g.*, a substitution, within the subject interval (*e.g.*, subgenomic interval or expressed subgenomic interval) being evaluated;

(v) the type of sample, *e.g.*, an FFPE sample, a blood sample, or a bone marrow aspirate sample; and

(vi) sequence in or near said subgenomic interval being evaluated, *e.g.*, the expected propensity for misalignment for said subject interval (*e.g.*, subgenomic interval or expressed subgenomic interval), *e.g.*, the presence of repeated sequences in or near said subject interval (*e.g.*, subgenomic interval or expressed subgenomic interval).

As referred to elsewhere herein, a method is particularly effective when the alignment of reads for a relatively large number of subject intervals (*e.g.*, subgenomic intervals, expressed subgenomic intervals, or both) is optimized. Thus, in an embodiment, at least X unique alignment methods are used to analyze reads for at least X unique subgenomic intervals, wherein unique means different from the other X-1, and X is equal to 2, 3, 4, 5, 10, 15, 20, 30, 50, 100, 200, 300, 400, 500, 600, 700, 800, 900, 1,000, or greater.

In an embodiment, subject intervals (*e.g.*, subgenomic intervals, expressed subgenomic intervals, or both) from at least X genes from **Tables 1-4 or FIGs. 3A-4D,** are analyzed, and X is equal to 2, 3, 4, 5, 10, 15, 20, 30, 40, 50, 60, 70, 80, 90, 100, 200, 300, 400, 500, or greater.

In an embodiment, a unique alignment method is applied to subject intervals (*e.g.*, subgenomic intervals, expressed subgenomic intervals, or both) in each of at least 3, 5, 10, 20, 40, 50, 60, 70, 80, 90, 100, 200, 300, 400, or 500 different genes.

In an embodiment, a nucleotide position in at least 20, 40, 60, 80, 100, 120, 140, 160 or 180, 200, 300, 400, or 500 genes, *e.g.,* genes from **Tables 1-4 or FIGs. 3A-4D,** is assigned a nucleotide value. In an embodiment a unique alignment method is applied to subject intervals (*e.g.,* subgenomic intervals or expressed subgenomic intervals) in each of at least 10, 20, 30, 40, or 50% of said genes analyzed.

Methods disclosed herein allow for the rapid and efficient alignment of troublesome reads, *e.g.,* a read having a rearrangement. Thus, in an embodiment where a read for a subject interval (*e.g.,* a subgenomic interval or an expressed subgenomic interval) comprises a nucleotide position with a rearrangement, *e.g.*, a translocation, the method can comprise using an alignment method that is appropriately tuned and that includes:

selecting a rearrangement reference sequence for alignment with a read, wherein said rearrangement reference sequence is preselected to align with a preselected rearrangement (in embodiments the reference sequence is not identical to the genomic rearrangement);

comparing, *e.g.*, aligning, a read with said preselected rearrangement reference sequence.

In embodiments, other methods are used to align troublesome reads. These methods are particularly effective when the alignment of reads for a relatively large number of diverse subgenomic intervals is optimized. By way of example, a method of analyzing a tumor sample can comprise:

performing a comparison, *e.g.*, an alignment comparison, of a read under a first set of parameters (*e.g.*, a first mapping algorithm or with a first reference sequence), and determining if said read meets a first predetermined alignment criterion (*e.g.*, the read can be aligned with said first reference sequence, *e.g.*, with less than a preselected number of mismatches);

if said read fails to meet the first predetermined alignment criterion, performing a second alignment comparison under a second set of parameters, (*e.g.*, a second mapping algorithm or with a second reference sequence); and,

optionally, determining if said read meets said second predetermined criterion (*e.g.,* the read can be aligned with said second reference sequence with less than a preselected number of mismatches),

wherein said second set of parameters comprises use of a set of parameters, *e.g.,* said second reference sequence, which, compared with said first set of parameters, is more likely to result in an alignment with a read for a preselected variant, *e.g.,* a rearrangement, *e.g.,* an insertion, deletion, or translocation.

These and other alignment methods are discussed in more detail elsewhere herein, *e.g.,* in the section entitled "Alignment" in the Detailed Description. Elements of that module can be included in methods of analyzing a tumor. In embodiments, alignment methods from the section entitled "Alignment" (in the Summary and/or Detailed Description) are combined with mutation calling methods from the section entitled "Mutation Calling" (in the Summary and/or Detailed Description) and/or a bait set from the section entitled "Bait" (in the Summary) and/or the sections entitled "Design and Construction of Baits" and "Bait Synthesis" in the Detailed Description). The method can be applied to a set of subject intervals (*e.g.*, subgenomic intervals, expressed subgenomic intervals, or both) from the section entitled "Gene Selection" (in the Summary and/or Detailed Description).

*MUTATION CALLING*

Methods disclosed herein can integrate the use of customized or tuned mutation calling parameters to optimize performance in sequencing methods, particularly in methods that rely on massively parallel sequencing of a large number of diverse genetic events in a large number of diverse genes, *e.g.,* from tumor samples, *e.g.,* from a cancer described herein. In embodiments of the method mutation calling for each of a number of preselected subject intervals (*e.g.*, subgenomic intervals, expressed subgenomic intervals, or both) is, individually, customized or fine-tuned. The customization or tuning can be based on one or more of the factors described herein, *e.g.,* the type of cancer in a sample, the gene in which subject interval (*e.g.*, subgenomic interval or expressed subgenomic interval) to be sequenced is located, or the variant to be sequenced. This selection or use of alignment conditions finely tuned to a number of subject intervals (*e.g.*, subgenomic intervals, expressed subgenomic intervals, or both) to be sequenced allows optimization of speed, sensitivity and specificity. The method is particularly effective when the alignment of reads for a relatively large number of diverse subject intervals (*e.g.*, subgenomic intervals, expressed subgenomic intervals, or both) is optimized.

Accordingly, in one aspect, the invention features a method of analyzing a sample, *e.g.*, a tumor sample from a hematologic malignancy (or premaligancy), *e.g.*, a hematologic malignancy (or premaligancy) described herein. The method comprises:

(a) acquiring one or a plurality of libraries comprising a plurality members from a sample, *e.g.*, a plurality of tumor members from the sample, *e.g.*, the tumor sample;
(b) optionally, enriching the one or a plurality of libraries for preselected sequences, *e.g.*, by contacting the library with a bait set (or plurality of bait sets) to provide selected members, *e.g.*, a library catch;
(c) acquiring a read for a subject interval (*e.g.*, a subgenomic interval or an expressed subgenomic interval) from a member, *e.g.*, a tumor member from said library or library catch, *e.g.*, by a method comprising sequencing, *e.g.*, with a next-generation sequencing method;
(d) aligning said read by an alignment method, *e.g.*, an alignment method described herein; and
(e) assigning a nucleotide value (*e.g.*, calling a mutation, *e.g.*, with a Bayesian method or a calling method described herein) from said read for the preselected nucleotide position, thereby analyzing said tumor sample.

optionally wherein a nucleotide value is assigned for a nucleotide position in each of X unique subject intervals (subgenomic intervals, expressed subgenomic intervals, or both) is assigned by a unique calling method, wherein unique subject interval (*e.g.*, subgenomic interval or expressed subgenomic interval) means different from the other X-1 subject intervals (*e.g.*, subgenomic intervals, expressed subgenomic intervals, or both), and wherein unique calling method means different from the other X-1 calling methods, and X is at least 2. The calling methods can differ, and thereby be unique, *e.g.*, by relying on different Bayesian prior values.

In an embodiment, the method comprises acquiring a library from which a member corresponding to a subgenomic interval and a member corresponding to an expressed subgenomic interval, are each obtained.

In an embodiment, the method comprises acquiring a first library from which a member corresponding to a subgenomic interval is obtained and acquiring a second library from which a member corresponding to an expressed subgenomic interval is obtained.

In an embodiment a bait set is used to provide members or a library catch comprising both a subgenomic interval and an expressed interval.

In an embodiment a first bait set is used to provide members or a library catch comprising a subgenomic interval and a second bait set is used to provide members or a library catch comprising an expressed subgenomic interval.

In an embodiment, step (b) is present. In an embodiment, step (b) is absent.

In an embodiment, assigning said nucleotide value is a function of a value which is or represents the prior (*e.g.*, literature) expectation of observing a read showing a preselected variant, *e.g.*, a mutation, at said preselected nucleotide position in a tumor of type.

In an embodiment, the method comprises assigning a nucleotide value (*e.g.*, calling a mutation) for at least 10, 20, 40, 50, 60, 70, 80, 90, 100, 200, 300, 400, 500, 600, 700, 800, 900, or 1,000 preselected nucleotide positions, wherein each assignment is a function of a unique (as opposed to the value for the other assignments) value which is or represents the prior (*e.g.*, literature) expectation of observing a read showing a preselected variant, *e.g.*, a mutation, at said preselected nucleotide position in a tumor of type.

In an embodiment, assigning said nucleotide value is a function of a set of values which represent the probabilities of observing a read showing said preselected variant at said preselected nucleotide position if the variant is present in the sample at a frequency (*e.g.*, 1%, 5%, 10%, etc.) and/or if the variant is absent (*e.g.*, observed in the reads due to base-calling error alone).

In an embodiment, a method (*e.g.*, step (e) of the method recited above) comprises a mutation calling method. The mutation calling methods described herein can include the following:

acquiring, for a preselected nucleotide position in each of said X subject intervals (*e.g.*, subgenomic intervals, expressed subgenomic intervals, or both):

(i) a first value which is or represents the prior (*e.g.*, literature) expectation of observing a read showing a preselected variant, *e.g.*, a mutation, at said preselected nucleotide position in a tumor of type X; and
(ii) a second set of values which represent the probabilities of observing a read showing said preselected variant at said preselected nucleotide position if the variant is present in the sample at a frequency (*e.g.*, 1%, 5%, 10%, etc.) and/or if the variant is absent (*e.g.,* observed in the reads due to base-calling error alone);

responsive to said values, assigning a nucleotide value (*e.g.,* calling a mutation) from said reads for each of said preselected nucleotide positions by weighing, *e.g.*, by a Bayesian method described herein, the comparison among the values in the second set using the first value (*e.g.,* computing the posterior probability of the presence of a mutation), thereby analyzing said sample.

In an embodiment, the method comprises one or more or all of:

(i) assigning a nucleotide value (*e.g.,* calling a mutation) for at least 10, 20, 40, 50, 60, 70, 80, 90, 100, 200, 300, 400, 500, 600, 700, 800, 900, or 1,000 preselected nucleotide positions, wherein each assignment is based on a unique (as opposed to the other assignments) first and/or second values;

(ii) the assignment of method of (i), wherein at least 10, 20, 30, 40, 50, 60, 70, 80, 90, 100, 200, 300, 400, or 500 of the assignments are made with first values which are a function of a probability of a preselected variant being present of less than 5, 10, or 20%, *e.g.*, of the cells in a preselected tumor type;

(iii) assigning a nucleotide value (*e.g.*, calling a mutation) for at least X preselected nucleotide positions, each of which of which being associated with a preselected variant having a unique (as opposed to the other X-1 assignments) probability of being present in a tumor of preselected type, *e.g.*, the tumor type of said sample, wherein, optionally, each of said of X assignments is based on a unique (as opposed to the other X-1 assignments) first and/or second value (wherein X= 2, 3, 5, 10, 20, 40, 50, 60, 70, 80, 90, 100, 200, 300, 400, or 500);

(iv) assigning a nucleotide value (*e.g.,* calling a mutation) at a first and a second nucleotide position, wherein the likelihood of a first preselected variant at said first nucleotide position being present in a tumor of preselected type (*e.g.*, the tumor type of said sample) is at least 2, 5, 10, 20, 30, or 40 times greater than the likelihood of a second preselected variant at said second nucleotide position being present, wherein, optionally, each assignment is based on a unique (as opposed to the other assignments) first and/or second value;

(v) assigning a nucleotide value to a plurality of preselected nucleotide positions (*e.g.*, calling mutations), wherein said plurality comprises an assignment for variants falling into one or more, *e.g.*, at least 3, 4, 5, 6, 7, or all, of the following probability percentage ranges:

less than or equal to 0.01;
greater than 0.01 and less than or equal to 0.02;
greater than 0.02 and less than or equal to 0.03;
greater than 0.03 and less than or equal to 0.04;
greater than 0.04 and less than or equal to 0.05;
greater than 0.05 and less than or equal to 0.1;
greater than 0.1 and less than or equal to 0.2;
greater than 0.2 and less than or equal to 0.5;
greater than 0.5 and less than or equal to 1.0;
greater than 1.0 and less than or equal to 2.0;
greater than 2.0 and less than or equal to 5.0;
greater than 5.0 and less than or equal to 10.0;
greater than 10.0 and less than or equal to 20.0;
greater than 20.0 and less than or equal to 50.0; and
greater than 50 and less than or equal to 100.0 %;

wherein, a probability range is the range of probabilities that a preselected variant at a preselected nucleotide position will be present in a tumor of preselected type (*e.g.,* the tumor type of said sample) or the probability that a preselected variant at a preselected nucleotide position will be present in the recited % of the cells in a tumor sample, a library from the tumor sample, or library catch from that library, for a preselected type (*e.g.,* the tumor type of said sample), and wherein, optionally, each assignment is based on a unique first and/or second value (*e.g.*, unique as opposed to the other assignments in a recited probability range or unique as opposed to the first and/or second values for one or more or all of the other listed probability ranges).

(vi) assigning a nucleotide value (*e.g.,* calling a mutation) for at least 1, 2, 3, 5, 10, 20, 40, 50, 60, 70, 80, 90, 100, 200, 300, 400, 500, 600, 700, 800, 900, or 1,000 preselected nucleotide positions each, independently, having a preselected variant present in less than 50, 40, 25, 20, 15, 10, 5, 4, 3, 2, 1, 0.5, 0.4, 0.3, 0.2, or 0.1 % of the DNA in said sample, wherein, optionally, each assignment is based on a unique (as opposed to the other assignments) first and/or second value;

(vii) assigning a nucleotide value (*e.g.*, calling a mutation) at a first and a second nucleotide position, wherein the likelihood of a preselected variant at the first position in the DNA of said sample is at least 2, 5, 10, 20, 30, or 40 times greater than the likelihood of a preselected variant at said second nucleotide position in the DNA of said sample, wherein, optionally, each assignment is based on a unique (as opposed to the other assignments) first and/or second value;

(viii) assigning a nucleotide value (*e.g.,* calling a mutation) in one or more or all of the following:

(1) at least 1, 2, 3, 4 or 5 preselected nucleotide positions having a preselected variant present in less than 1% of the cells in said sample, of the nucleic acids in a library from said sample, or the nucleic acid in a library catch from that library;

(2) at least 1, 2, 3, 4 or 5 preselected nucleotide positions having a preselected variant present in 1- 2% of the cells in said sample, of the nucleic acid in a library from said sample, or the nucleic acid in a library catch from that library ;

(3) at least 1, 2, 3, 4 or 5 preselected nucleotide positions having a preselected variant present in greater than 2% and less than or equal to 3% of the cells in said sample, of the nucleic acid in a library from said sample, or the nucleic acid in a library catch from that library

(4) at least 1, 2, 3, 4 or 5 preselected nucleotide positions having a preselected variant present in greater than 3% and less than or equal to 4% of the cells in said sample, of the nucleic acid in a library from said sample, or the nucleic acid in a library catch from that library;

(5) at least 1, 2, 3, 4 or 5 preselected nucleotide positions having a preselected variant present in greater than 4% and less than or equal to 5% of the cells in said sample, of the nucleic acid in a library from said sample, or the nucleic acid in a library catch from that library;

(6) at least 1, 2, 3, 4 or 5 preselected nucleotide positions having a preselected variant present in greater than 5% and less than or equal to 10% of the cells in said sample, of the nucleic acid in a library from said sample, or the nucleic acid in a library catch from that library;

(7) at least 1, 2, 3, 4 or 5 preselected nucleotide positions having a preselected variant present in greater than 10% and less than or equal to 20% of the cells in said sample, of the nucleic acid in a library from said sample, or the nucleic acid in a library catch from that library;

(8) at least 1, 2 3, 4 or 5 preselected nucleotide positions having a preselected variant present in greater than 20% and less than or equal to 40% of the cells in said sample, of the nucleic acid in a library from said sample, or the nucleic acid in a library catch from that library;

(9) at least 1, 2 3, 4 or 5 preselected nucleotide positions having a preselected variant present at greater than 40% and less than or equal to 50% of the cells in said sample, of the nucleic acid in a library from said sample, or the nucleic acid in a library catch from that library; or

(10) at least 1, 2 3, 4 or 5 preselected nucleotide positions having a preselected variant present in greater than 50% and less than or equal to 100% of the cells in said sample, of the nucleic acid in a library from said sample, or the nucleic acid in a library catch from that library;

wherein, optionally, each assignment is based on a unique first and/or second value (*e.g.*, unique as opposed to the other assignments in the recited range (*e.g.,* the range in (1) of less than 1%) or unique as opposed to a first and/or second values for a determination in one or more or all of the other listed ranges); or

(ix) assigning a nucleotide value (*e.g.*, calling a mutation) at each of X nucleotide positions, each nucleotide position, independently, having a likelihood (of a preselected variant being present in the DNA of said sample) that is unique as compared with the likelihood for a preselected variant at the other X-1 nucleotide positions, wherein X is equal to or greater than 1, 2, 3, 5, 10, 20, 40, 50, 60, 70, 80, 90, 100, 200, 300, 400, 500, 600, 700, 800, 900, or 1,000, and wherein each assignment is based on a unique (as opposed to the other assignments) first and/or second value.

In embodiments of the method, a "threshold value" is used to evaluate reads, and select from the reads a value for a nucleotide position, *e.g.*, calling a mutation at a specific position in a gene. In embodiments of the method, a threshold value for each of a number of preselected subject intervals (*e.g.*, subgenomic intervals, expressed subgenomic intervals, or both) is customized or fine-tuned. The customization or tuning can be based on one or more of the factors described herein, *e.g.*, the type of cancer in a sample, the gene in which the subject interval (subgenomic interval or expressed subgenomic interval) to be sequenced is located, or the variant to be sequenced. This provides for calling that is finely tuned to each of a number of subject intervals (*e.g.*, subgenomic intervals, expressed subgenomic intervals, or both) to be sequenced. The method is particularly effective when a relatively large number of diverse subgenomic intervals are analyzed.

Thus, in another embodiment the method of analyzing a tumor comprises the following mutation calling method:

acquiring, for each of said X subject intervals (*e.g.*, subgenomic intervals, expressed subgenomic intervals, or both), a threshold value, wherein each of said acquired X threshold values is unique as compared with the other X-1 threshold values, thereby providing X unique threshold values;

for each of said X subject intervals (*e.g.*, subgenomic intervals, expressed subgenomic intervals, or both), comparing an observed value which is a function of the number of reads having a preselected nucleotide value at a preselected nucleotide position with its unique threshold value, thereby applying to each of said X subject intervals (*e.g.*, subgenomic intervals, expressed subgenomic intervals, or both), its unique threshold value; and

optionally, responsive to the result of said comparison, assigning a nucleotide value to a preselected nucleotide position,

wherein X is equal to or greater than 2.

In an embodiment, the method includes assigning a nucleotide value to at least 2, 3, 5, 10, 20, 40, 50, 60, 70, 80, 90, 100, 200, 300, 400, 500, 600, 700, 800, 900, or 1,000 preselected nucleotide positions, each having, independently, a first value that is a function of a probability that is less than 0.5, 0.4, 0.25, 0.15, 0.10, 0.05, 0.04, 0.03, 0.02, or 0.01.

In an embodiment, the method includes assigning a nucleotide value to at each of at least X nucleotide positions, each independently having a first value that is unique as compared with the other X-1 first values, and wherein each of said X first values is a function of a probability that is less than 0.5, 0.4, 0.25, 0.15, 0.10, 0.05, 0.04, 0.03, 0.02, or 0.01, wherein X is equal to or greater than 1, 2, 3, 5, 10, 20, 40, 50, 60, 70, 80, 90, 100, 200, 300, 400, 500, 600, 700, 800, 900, or 1,000.

In an embodiment, a nucleotide position in at least 20, 40, 60, 80, 100, 120, 140, 160 or 180, 200, 300, 400, or 500 genes, *e.g.,* genes from **Tables 1-4 or FIGs. 3A-4D,** is assigned a nucleotide value. In an embodiment unique first and/or second values are applied to subject intervals (*e.g.*, subgenomic intervals, expressed subgenomic intervals, or both) in each of at least 10, 20, 30, 40, or 50% of said genes analyzed.

Embodiments of the method can be applied where threshold values for a relatively large number of subject intervals (*e.g.*, subgenomic intervals, expressed subgenomic intervals, or both), are optimized, as is seen, *e.g.*, from the following embodiments.

In an embodiment, a unique threshold value is applied to subject intervals, *e.g.*, subgenomic intervals or expressed subgenomic intervals, in each of at least 3, 5, 10, 20, 40, 50, 60, 70, 80, 90, 100, 200, 300, 400, 500, 600, 700, 800, 900, or 1,000 different genes.

In an embodiment, a nucleotide position in at least 20, 40, 60, 80, 100, 120, 140, 160 or 180, 200, 300, 400, or 500 genes, *e.g.,* genes from **Tables 1-4 or FIGs. 3A-4D,** is assigned a nucleotide value. In an embodiment a unique threshold value is applied to a subgenomic interval in each of at least 10, 20, 30, 40, or 50% of said genes analyzed.

In an embodiment, a nucleotide position in at least 5, 10, 20, 30, or 40 genes from **Tables 1-4 or FIGs. 3A-4D** is assigned a nucleotide value. In an embodiment a unique threshold value is applied to a subject interval (*e.g.*, a subgenomic interval or an expressed subgenomic interval) in each of at least 10, 20, 30, 40, or 50% of said genes analyzed.

These and other mutation calling methods are discussed in more detail elsewhere herein, *e.g.,* in the section entitled "Mutation." Elements of that module can be included in methods of analyzing a tumor. In embodiments, alignment methods from the section entitled "Mutation Calling" are combined with alignment methods from the section entitled "Alignment" and/or a bait set from the section entitled "Bait." The method can be applied to a set of subject intervals (*e.g.*, subgenomic intervals, expressed subgenomic intervals, or both) from the section entitled "Gene Selection."

*BAIT*

Methods described herein provide for optimized sequencing of a large number of genes and gene products from samples, *e.g.,* tumor samples, *e.g.,* from a cancer described herein, from one or more subjects by the appropriate selection of baits, *e.g.*, baits for use in solution hybridization, for the selection of target nucleic acids to be sequenced. The efficiency of selection for various subject intervals (*e.g.*, subgenomic intervals, expressed subgenomic intervals, or both), or classes thereof, are matched according to bait sets having a preselected efficiency of selection. As used in this section, "efficiency of selection" refers to the level or depth of sequence coverage as it is adjusted according to a target subject interval(s) (*e.g.*, subgenomic interval(s), expressed subgenomic interval(s), or both).

Thus a method (*e.g.*, step (b) of the method recited above) comprises contacting the library with a plurality of baits to provide selected members (*e.g.,* a library catch).

Accordingly, in one aspect, the invention features a method of analyzing a sample, *e.g.,* a tumor sample from a cancer, *e.g.*, a cancer described herein. The method comprises:

(a) acquiring one or a plurality of libraries comprising a plurality of members (*e.g.,* target members) from a sample, *e.g.,* a plurality of tumor members from a tumor sample;

(b) contacting the one or a plurality of libraries with a bait set (or a plurality of bait sets) to provide selected members (*e.g.*, a library catch);

(c) acquiring a read for a subject interval, *e.g.,* a subgenomic interval, an expressed subgenomic interval, or both, from a member, *e.g.*, a tumor member from said library or library catch, *e.g.*, by a method comprising sequencing, *e.g.,* with a next-generation sequencing method;

(d) aligning said read by an alignment method, *e.g.,* an alignment method described herein; and

(e) assigning a nucleotide value (*e.g.*, calling a mutation, *e.g.*, with a Bayesian method or a method described herein) from said read for the preselected nucleotide position,

thereby analyzing said tumor sample,

optionally wherein the method comprises contacting the library with a plurality, *e.g.*, at least two, three, four, or five, of baits or bait sets, wherein each bait or bait set of said plurality has a unique (as opposed to the other bait sets in the plurality), preselected efficiency for selection. E.g., each unique bait or bait set provides for a unique depth of sequencing. The term "bait set", as used herein, collectively refers to one bait or a plurality of bait molecules.

In an embodiment, the method comprises acquiring a library from which a member corresponding to a subgenomic interval and a member corresponding to an expressed genomic interval, are each obtained.

In an embodiment, the method comprises acquiring a first library from which a member corresponding to a subgenomic interval is obtained and acquiring a second library from which a member corresponding to an expressed subgenomic interval is obtained.

In an embodiment a bait set is used to provide members or a library catch comprising both a subgenomic interval and an expressed interval.

In an embodiment a first bait set is used to provide members or a library catch comprising a subgenomic interval and a second bait set is used to provide members or a library catch comprising an expressed subgenomic interval.

In an embodiment, the efficiency of selection of a first bait set in the plurality differs from the efficiency of a second bait set in the plurality by at least 2 fold. In an embodiment, the first and second bait sets provide for a depth of sequencing that differs by at least 2 fold.

In an embodiment, the method comprises contacting one, or a plurality of the following bait sets with the library:

a) a bait set that selects sufficient members comprising a subgenomic interval to provide for about 500X or higher sequencing depth, *e.g.,* to sequence a mutation present in no more than 5% of the cells from the sample;

b) a bait set that selects sufficient members comprising a subgenomic interval to provide for about 200X or higher, *e.g.,* about 200X to about 500X, sequencing depth, *e.g.,* to sequence a mutation present in no more than 10% of the cells from the sample;

c) a bait set that selects sufficient members comprising a subgenomic interval to provide for about 10-100X sequencing depth, *e.g.,* to sequence one or more subgenomic intervals (*e.g.,* exons) that are chosen from: i) a pharmacogenomic (PGx) single nucleotide polymorphism (SNP) that may explain the ability of patient to metabolize different drugs, or ii) genomic SNPs that may be used to uniquely identify (*e.g.*, fingerprint) a patient;

d) a bait set that selects sufficient members comprising a subject interval (*e.g.*, a subgenomic interval or an expressed subgenomic interval) to provide for about 5-50 X sequencing depth, *e.g.,* to detect a structural breakpoint, such as a genomic translocation or an indel. For example, detection of an intronic breakpoint requires 5-50X sequence-pair spanning depth to ensure high detection reliability. Such bait sets can be used to detect, for example, translocation/indel-prone cancer genes; or

e) a bait set that selects sufficient members comprising a subject interval (*e.g.*, a subgenomic interval or an expressed subgenomic interval) to provide for about 0.1-300X sequencing depth, *e.g.,* to detect copy number changes. In one embodiment, the sequencing depth ranges from about 0.1-10X sequencing depth to detect copy number changes. In other embodiments, the sequencing depth ranges from about 100-300X to detect genomic SNPs/loci that is used to assess copy number gains/losses of genomic DNA or loss-of-heterozygosity (LOH). Such bait sets can be used to detect, for example, amplification/deletion-prone cancer genes.

The level of sequencing depth as used herein (*e.g.,* X-fold level of sequencing depth) refers to the level of coverage of reads (*e.g.,* unique reads), after detection and removal of duplicate reads, *e.g.,* PCR duplicate reads.

In one embodiment, the bait set selects a subject interval (*e.g.*, a subgenomic interval or an expressed subgenomic interval) containing one or more rearrangements, *e.g.*, an intron containing a genomic rearrangement. In such embodiments, the bait set is designed such that repetitive sequences are masked to increase the selection efficiency. In those embodiments where the rearrangement has a known juncture sequence, complementary bait sets can be designed to the juncture sequence to increase the selection efficiency.

In embodiments, the method comprises the use of baits designed to capture two or more different target categories, each category having a different bait design strategies. In embodiments, the hybrid capture methods and compositions disclosed herein capture a defined subset of target sequences (*e.g.*, target members) and provide homogenous coverage of the target sequence, while minimizing coverage outside of that subset. In one embodiment, the target sequences include the entire exome out of genomic DNA, or a selected subset thereof. In another embodiment, the target sequences include a large chromosomal region, *e.g.,* a whole chromosome arm. The methods and compositions disclosed herein provide different bait sets for achieving different depths and patterns of coverage for complex target nucleic acid sequences (*e.g.*, nucleic acid libraries).

In an embodiment, the method comprises providing selected members of one or a plurality of nucleic acid libraries (*e.g.,* a library catch). The method includes:

providing one or a plurality of libraries (*e.g.,* one or a plurality of nucleic acid libraries) comprising a plurality of members, *e.g.*, target nucleic acid members (*e.g.*, including a plurality of tumor members, reference members, and/or PGx members);

contacting the one or a plurality of libraries, *e.g.*, in a solution-based reaction, with a plurality of baits (*e.g.*, oligonucleotide baits) to form a hybridization mixture comprising a plurality of bait/member hybrids;

separating the plurality of bait/member hybrids from said hybridization mixture, *e.g.*, by contacting said hybridization mixture with a binding entity that allows for separation of said plurality of bait/member hybrids,

thereby providing a library catch (*e.g.,* a selected or enriched subgroup of nucleic acid molecules from the one or a plurality of libraries),

optionally wherein the plurality of baits includes two or more of the following:

a) a first bait set that selects a high-level target (*e.g.,* one or more tumor members that include a subject interval (*e.g., a* subgenomic interval or an expressed subgenomic interval), such as a gene, an exon, or a base) for which the deepest coverage is required to enable a high level of sensitivity for an alteration (*e.g.*, one or more mutations) that appears at a low frequency, *e.g.,* about 5% or less (*i.e.*, 5% of the cells from the sample harbor the alteration in their genome). In one embodiment; the first bait set selects (*e.g.,* is complementary to) a tumor member that includes an alteration (*e.g., a* point mutation) that requires about 500X or higher sequencing depth;

b) a second bait set that selects a mid-level target (*e.g.,* one or more tumor members that include a subject interval (*e.g.*, a subgenomic interval or an expressed subgenomic interval) such as a gene, an exon, or a base) for which high coverage is required to enable high level of sensitivity for an alteration (*e.g.,* one or more mutations) that appears at a higher frequency than the high-level target in a), *e.g.,* a frequency of about 10% (*i.e.*, 10% of the cells from the sample harbor the alteration in their genome). In one embodiment; the second bait set selects (*e.g.,* is complementary to) a tumor member that includes an alteration (*e.g.*, a point mutation) that requires about 200X or higher sequencing depth;

c) a third bait set that selects a low-level target (*e.g.,* one or more PGx members that includes a subject interval (*e.g., a* subgenomic interval or an expressed subgenomic interval), such as a gene, an exon, or a base) for which low-medium coverage is required to enable high level of sensitivity, *e.g.,* to detect heterozygous alleles. For example, detection of heterozygous alleles requires 10-100X sequencing depth to ensure high detection reliability. In one embodiment, the third bait set selects one or more subject intervals (*e.g.*, subgenomic intervals, expressed subgenomic intervals, or both, *e.g.,* exons) that are chosen from: a) a pharmacogenomic (PGx) single nucleotide polymorphism (SNP) that may explain the ability of patient to metabolize different drugs, or b) genomic SNPs that may be used to uniquely identify (*e.g.*, fingerprint) a patient;

d) a fourth bait set that selects a first intron target (*e.g.,* a member that includes an intron sequence) for which low-medium coverage is required, *e.g.,* to detect a structural breakpoint, such as a genomic translocation or an indel. For example, detection of an intronic breakpoint requires 5-50X sequence-pair spanning depth to ensure high detection reliability. Said fourth bait sets can be used to detect, for example, translocation/indel-prone cancer genes; or

e) a fifth bait set that selects a second intron target (*e.g.,* an intron member) for which sparse coverage is required to improve the ability to detect copy number changes. For example, detection of a one-copy deletion of several terminal exons requires 0.1-300X coverage to ensure high detection reliability. In one embodiment, the coverage depth ranges from about 0.1-10X to detect copy number changes. In other embodiments, the coverage depth ranges from about 100-300X to detect genomic SNPs/loci to assess copy number gains/losses of genomic DNA or loss-of-heterozygosity (LOH). Said fifth bait sets can be used to detect, for example, amplification/deletion-prone cancer genes.

Any combination of two, three, four or more of the aforesaid bait sets can be used, for example, a combination of the first and the second bait sets; first and third bait sets; first and fourth bait sets; first and fifth bait sets; second and third bait sets; second and fourth bait sets; second and fifth bait sets; third and fourth bait sets; third and fifth bait sets; fourth and fifth bait sets; first, second and third bait sets; first, second and fourth bait sets; first, second and fifth bait sets; first, second, third, fourth bait sets; first, second, third, fourth and fifth bait sets, and so on.

In one embodiment, each of the first, second, third, fourth, or fifth bait set has a preselected efficiency for selection (*e.g.,* capture). In one embodiment, the value for efficiency of selection is the same for at least two, three, four of all five baits according to a)-e). In other embodiments, the value for efficiency of selection is different for at least two, three, four of all five baits according to a)-e).

In some embodiments, at least two, three, four, or all five bait sets have a preselected efficiency value that differ. For example, a value for efficiency of selection chosen from one of more of:

(i) the first preselected efficiency has a value for first efficiency of selection that is at least about 500X or higher sequencing depth (*e.g.*, has a value for efficiency of selection that is greater than the second, third, fourth or fifth preselected efficiency of selection (*e.g.*, about 2-3 fold greater than the value for the second efficiency of selection; about 5-6 fold greater than the value for the third efficiency of selection; about 10 fold greater than the value for the fourth efficiency of selection; about 50 to 5,000-fold greater than the value for the fifth efficiency of selection);

(ii) the second preselected efficiency has a value for second efficiency of selection that is at least about 200X or higher sequencing depth, *e.g.*, has a value for efficiency of selection that is greater than the third, fourth or fifth preselected efficiency of selection (*e.g.*, about 2 fold greater than the value for the third efficiency of selection; about 4 fold greater than the value for the fourth efficiency of selection; about 20 to 2,000-fold greater than the value for the fifth efficiency of selection);

(iii) the third preselected efficiency has a value for third efficiency of selection that is at least about 100X or higher sequencing depth, *e.g.*, has a value for efficiency of selection that is greater than the fourth or fifth preselected efficiency of selection (*e.g.*, about 2 fold greater than the value for the fourth efficiency of selection; about 10 to 1000-fold greater than the value for the fifth efficiency of selection);

(iv) the fourth preselected efficiency has a value for fourth efficiency of selection that is at least about 50X or higher sequencing depth, *e.g.*, has a value for efficiency of selection that is greater than the fifth preselected efficiency of selection (*e.g.*, about 50 to 500-fold greater than the value for the fifth efficiency of selection); or

(v) the fifth preselected efficiency has a value for fifth efficiency of selection that is at least about 10X to 0.1X sequencing depth.

In certain embodiments, the value for efficiency of selection is modified by one or more of: differential representation of different bait sets, differential overlap of bait subsets, differential bait parameters, mixing of different bait sets, and/or using different types of bait sets. For example, a variation in efficiency of selection (*e.g.*, relative sequence coverage of each bait set/target category) can be adjusted by altering one or more of:

(i) Differential representation of different bait sets - The bait set design to capture a given target (*e.g.,* a target member) can be included in more/fewer number of copies to enhance/reduce relative target coverage depths;

(ii) Differential overlap of bait subsets - The bait set design to capture a given target (*e.g.,* a target member) can include a longer or shorter overlap between neighboring baits to enhance/reduce relative target coverage depths;

(iii) Differential bait parameters - The bait set design to capture a given target (*e.g.,* a target member) can include sequence modifications/shorter length to reduce capture efficiency and lower the relative target coverage depths;

(iv) Mixing of different bait sets - Bait sets that are designed to capture different target sets can be mixed at different molar ratios to enhance/reduce relative target coverage depths;

(v) Using different types of oligonucleotide bait sets - In certain embodiments, the bait set can include:

(a) one or more chemically (*e.g.*, non-enzymatically) synthesized (*e.g.*, individually synthesized) baits,

(b) one or more baits synthesized in an array,

(c) one or more enzymatically prepared, *e.g.*, *in vitro* transcribed, baits;

(d) any combination of (a), (b) and/or (c),

(e) one or more DNA oligonucleotides (*e.g.*, a naturally or non-naturally occurring DNA oligonucleotide),

(f) one or more RNA oligonucleotides (*e.g.*, a naturally or non-naturally occurring RNA oligonucleotide),

(g) a combination of (e) and (f), or

(h) a combination of any of the above.

The different oligonucleotide combinations can be mixed at different ratios, *e.g.,* a ratio chosen from 1:1, 1:2, 1:3, 1:4, 1:5, 1:10, 1:20, 1:50; 1:100, 1:1000, or the like. In one embodiment, the ratio of chemically-synthesized bait to array-generated bait is chosen from 1:5, 1:10, or 1:20. The DNA or RNA oligonucleotides can be naturally- or non-naturally-occurring. In certain embodiments, the baits include one or more non-naturally-occurring nucleotides to, *e.g.,* increase melting temperature. Exemplary non-naturally occurring oligonucleotides include modified DNA or RNA nucleotides. Exemplary modified nucleotides (*e.g.*, modified RNA or DNA nucleotides) include, but are not limited to, a locked nucleic acid (LNA), wherein the ribose moiety of an LNA nucleotide is modified with an extra bridge connecting the 2' oxygen and 4' carbon; peptide nucleic acid (PNA), *e.g.,* a PNA composed of repeating N-(2-aminoethyl)-glycine units linked by peptide bonds; a DNA or RNA oligonucleotide modified to capture low GC regions; a bicyclic nucleic acid (BNA); a crosslinked oligonucleotide; a modified 5-methyl deoxycytidine; and 2,6-diaminopurine. Other modified DNA and RNA nucleotides are known in the art.

In certain embodiments, a substantially uniform or homogeneous coverage of a target sequence (*e.g.,* a target member) is obtained. For example, within each bait set/target category, uniformity of coverage can be optimized by modifying bait parameters, for example, by one or more of:

(i) Increasing/decreasing bait representation or overlap can be used to enhance/reduce coverage of targets (*e.g.,* target members), which are under/over-covered relative to other targets in the same category;

(ii) For low coverage, hard to capture target sequences (*e.g.*, high GC content sequences), expand the region being targeted with the bait sets to cover, *e.g.,* adjacent sequences (*e.g.*, less GC-rich adjacent sequences);

(iii) Modifying a bait sequence can be used to reduce secondary structure of the bait and enhance its efficiency of selection;

(iv) Modifying a bait length can be used to equalize melting hybridization kinetics of different baits within the same category. Bait length can be modified directly (by producing baits with varying lengths) or indirectly (by producing baits of consistent length, and replacing the bait ends with arbitrary sequence);

(v) Modifying baits of different orientation for the same target region (*i.e.* forward and reverse strand) may have different binding efficiencies. The bait set with either orientation providing optimal coverage for each target may be selected;

(vi) Modifying the amount of a binding entity, *e.g.,* a capture tag (*e.g.* biotin), present on each bait may affect its binding efficiency. Increasing/decreasing the tag level of baits targeting a specific target may be used to enhance/reduce the relative target coverage;

(vii) Modifying the type of nucleotide used for different baits can be used to affect binding affinity to the target, and enhance/reduce the relative target coverage; or

(viii) Using modified oligonucleotide baits, *e.g.*, having more stable base pairing, can be used to equalize melting hybridization kinetics between areas of low or normal GC content relative to high GC content.

For example, different types of oligonucleotide bait sets can be used.

In one embodiment, the value for efficiency of selection is modified by using different types of bait oligonucleotides to encompass pre-selected target regions. For example, a first bait set (*e.g.,* an array-based bait set comprising 10,000-50,000 RNA or DNA baits) can be used to cover a large target area (*e.g.,* 1-2MB total target area). The first bait set can be spiked with a second bait set (*e.g.,* individually synthesized RNA or DNA bait set comprising less than 5,000 baits) to cover a pre-selected target region (*e.g.,* selected subgenomic intervals of interest spanning, *e.g.,* 250kb or less, of a target area) and/or regions of higher secondary structure, *e.g.*, higher GC content. Selected subject intervals (*e.g.*, subgenomic intervals, expressed subgenomic intervals, or both) of interest may correspond to one or more of the genes or gene products described herein, or a fragment thereof. The second bait set may include about 1-5,000, 2-5,000, 3-5,000, 10-5,000, 100-5,000, 500-5,000, 100-5,000, 1,000-5,000, 2,000-5,000 baits depending on the bait overlap desired. In other embodiments, the second bait set can include selected oligo baits (*e.g.,* less than 400, 200, 100, 50, 40, 30, 20, 10, 5, 4, 3, 2 or 1 baits) spiked into the first bait set. The second bait set can be mixed at any ratio of individual oligo baits. For example, the second bait set can include individual baits present as a 1:1 equimolar ratio. Alternatively, the second bait set can include individual baits present at a different ratio (*e.g.,* 1:5, 1:10, 1:20), for example, to optimize capture of certain targets (*e.g.,* certain targets can have a 5-10X of the second bait set compared to other targets).

In other embodiments, the efficiency of selection is adjusted by leveling the efficiency of individual baits within a group (*e.g.,* a first, second or third plurality of baits) by adjusting the relative abundance of the baits, or the density of the binding entity (*e.g.,* the hapten or affinity tag density) in reference to differential sequence capture efficiency observed when using an equimolar mix of baits, and then introducing a differential excess of a first group of baits to the overall bait mix relative to a second group of baits.

In an embodiment, the method comprises the use of a plurality of bait sets that includes a bait set that selects a tumor member, *e.g.,* a nucleic acid molecule comprising a subject interval (*e.g.*, a subgenomic interval or an expressed subgenomic interval) from a tumor cell (also referred to herein as "a tumor bait set"). The tumor member can be any nucleotide sequence present in a tumor cell, *e.g.,* a mutated, a wild-type, a PGx, a reference or an intron nucleotide sequence, as described herein, that is present in a tumor or cancer cell. In one embodiment, the tumor member includes an alteration (*e.g.,* one or more mutations) that appears at a low frequency, *e.g.,* about 5% or less of the cells from the tumor sample harbor the alteration in their genome. In other embodiments, the tumor member includes an alteration (*e.g.,* one or more mutations) that appears at a frequency of about 10% of the cells from the tumor sample. In other embodiments, the tumor member includes a subgenomic interval from a PGx gene or gene product, an intron sequence, *e.g.,* an intron sequence as described herein, a reference sequence that is present in a tumor cell.

In another aspect, the invention features a bait set described herein, combinations of individual bait sets described herein, *e.g.,* combinations described herein. The bait set(s) can be part of a kit which can optionally comprise instructions, standards, buffers or enzymes or other reagents.

*Gene Selection*

Preselected subject intervals, *e.g.*, subgenomic intervals, expressed subgenomic intervals, or both, for analysis, *e.g.,* a group or set of subgenomic intervals for sets or groups of genes and other regions, are described herein.

Thus, in embodiments a method comprises sequencing, *e.g.*, by a next-generation sequencing method, a subject interval (*e.g.,* a subgenomic interval or an expressed subgenomic interval) from at least 5, 6, 7, 8, 9, 10, 15, 20, 25, 30, 40, 50, 60, 70, 80, 90, 100, 200, 300, 400, 500, or more genes or gene products from the acquired nucleic acid sample, wherein the genes or gene products are chosen from **Tables 1-4 or FIGs. 3A-4D,** thereby analyzing the tumor sample, *e.g.*, from a cancer described herein.

Accordingly, in one aspect, the invention features a method of analyzing a sample, *e.g.*, a tumor sample from a hematologic malignancy (or premaligancy), *e.g.*, a hematologic malignancy (or premaligancy) described herein. The method comprises:

(a) acquiring one or a plurality of libraries comprising a plurality members from a sample, *e.g.,* a plurality of tumor members from a tumor sample from a hematologic malignancy (or premaligancy), *e.g.*, a hematologic malignancy (or premaligancy) described herein;
(b) optionally, enriching the one or a plurality of libraries for preselected sequences, *e.g.*, by contacting the one or a plurality of libraries with a bait set (or plurality of bait sets) to provide selected members (*e.g.*, a library catch);
(c) acquiring a read for a subject interval (*e.g.*, a subgenomic interval or an expressed subgenomic interval) from a member, *e.g.*, a tumor member from said library or library catch, *e.g.,* by a method comprising sequencing, *e.g.*, with a next-generation sequencing method;
(d) aligning said read by an alignment method, *e.g.,* an alignment method described herein; and
(e) assigning a nucleotide value (*e.g.,* calling a mutation, *e.g.,* with a Bayesian method or a method described herein) from said read for the preselected nucleotide position,

thereby analyzing said tumor sample,
optionally wherein the method comprises sequencing, *e.g.*, by a next-generation sequencing method, a subject interval (*e.g.,* a subgenomic interval or an expressed subgenomic interval) from at least 5, 6, 7, 8, 9, 10, 15, 20, 25, 30, 40, 50, 60, 70, 80, 90, 100, 200, 300, 400, 500, or more genes or gene products from the sample, wherein the genes or gene products are chosen from **Tables 1-4 or FIGs. 3A-4D.**

In an embodiment, step (b) is present. In an embodiment, step (b) is absent.

In another embodiment, subject intervals (*e.g.*, subgenomic intervals, expressed subgenomic intervals, or both) of one of the following sets or groups are analyzed. *E.g.*, subject intervals (*e.g.*, subgenomic intervals, expressed subgenomic intervals, or both) associated with a tumor or cancer gene or gene product, a reference (*e.g.,* a wild-type) gene or gene product, and a PGx gene or gene product, can provide a group or set of subgenomic intervals from the tumor sample.

In an embodiment, the method acquires a read, *e.g.*, sequences, a set of subject intervals (*e.g.*, subgenomic intervals, expressed subgenomic intervals, or both) from the tumor sample, wherein the subject intervals (*e.g.*, subgenomic intervals, expressed subgenomic intervals, or both) are chosen from at least 1, 2, 3, 4, 5, 6, 7 or all of the following:

A) at least 5, 6, 7, 8, 9, 10, 15, 20, 25, 30, 40, 50, 60, 70, 80, 90, 100, 200, 300, 400, 500, or more subject intervals. *e.g.*, subgenomic intervals, or expressed subgenomic intervals, or both, from a mutated or wild-type gene or gene product according to **Tables 1-4 or FIGs. 3A-4D;**
B) at least 5, 6, 7, 8, 9, 10, 15, 20, 25, 30, 40, 50, 60, 70, 80, 90, 100, 200, 300, 400, 500, or more subject intervals (*e.g.*, subgenomic intervals, expressed subgenomic intervals, or both) from a gene or gene product that is associated with a tumor or cancer (*e.g.,* is a positive or negative treatment response predictor, is a positive or negative prognostic factor for, or enables differential diagnosis of a tumor or cancer, *e.g.,* a gene or gene product according to **Tables 1-4 or FIGs. 3A-4D**);
C) at least 5, 6, 7, 8, 9, 10, 15, 20, 25, 30, 40, 50, 60, 70, 80, 90, 100, 200, 300, 400, 500, or more of subject intervals (*e.g.*, subgenomic intervals, expressed subgenomic intervals, or both) from a mutated or wild-type gene or gene product (*e.g.*, single nucleotide polymorphism (SNP)) of a subgenomic interval that is present in a gene or gene product associated with one or more of drug metabolism, drug responsiveness, or toxicity (also referred to herein as "PGx" genes) chosen from **Tables 1-4 or FIGs. 3A-4D;**
D) at least 5, 6, 7, 8, 9, 10, 15, 20, 25, 30, 40, 50, 60, 70, 80, 90, 100, 200, 300, 400, 500, or more of subject intervals (*e.g.*, subgenomic intervals, expressed subgenomic intervals, or both) from a mutated or wild-type PGx gene or gene product (*e.g.*, single nucleotide polymorphism (SNP)) of a subject interval (*e.g.*, a subgenomic interval or an expressed subgenomic interval) that is present in a gene or gene product chosen from **Tables 1-4 or FIGs. 3A-4D** associated with one or more of: (i) better survival of a cancer patient treated with a drug (*e.g.,* better survival of a breast cancer patient treated with paclitaxel); (ii) paclitaxel metabolism; (iii) toxicity to a drug; or (iv) a side effect to a drug;

E) a plurality of translocation alterations involving at least 5, 6, 7, 8, 9, 10, 15, 20, 25, 30, 40, 50, 60, 70, 80, 90, 100, 200, 300, 400, 500, or more genes or gene products according to **Tables 1-4 or FIGs. 3A-4D;**

F) at least five genes or gene products selected from **Tables 1-4 or FIGs. 3A-4D,** wherein an allelic variation, *e.g.*, at the preselected position, is associated with a preselected type of tumor and wherein said allelic variation is present in less than 5% of the cells in said tumor type;

G) at least five genes or gene products selected from **Tables 1-4 or FIGs. 3A-4D,** which are embedded in a GC-rich region; or

H) at least five genes or gene products indicative of a genetic (*e.g.*, a germline risk) factor for developing cancer (*e.g.*, the gene or gene product is chosen from **Tables 1-4 or FIGs. 3A-4D**).

In yet another embodiment, the method acquires a read, *e.g.*, sequences, a set of subject intervals (*e.g.*, subgenomic intervals, expressed subgenomic intervals, or both) from the tumor sample, wherein the subject intervals (*e.g.*, subgenomic intervals, expressed subgenomic intervals, or both) are chosen from 5, 6, 7, 8, 9, 10, 15, 20, 25, 30, 40, 50, 60, 70, 80, 90, 100, 200, 300, 400, or all of the genes or gene products described in **Table 1.**

In yet another embodiment, the method acquires a read, *e.g.*, sequences, a set of subject intervals (*e.g.*, subgenomic intervals, expressed subgenomic intervals, or both) from the tumor sample, wherein the subject intervals (*e.g.*, subgenomic intervals, expressed subgenomic intervals, or both) are chosen from 5, 6, 7, 8, 9, 10, 15, 20, 25, 30, or all of the genes or gene products described in Table 2.

In yet another embodiment, the method acquires a read, *e.g.*, sequences, a set of subject intervals (*e.g.*, subgenomic intervals, expressed subgenomic intervals, or both) from the tumor sample, wherein the subject intervals (*e.g.*, subgenomic intervals, expressed subgenomic intervals, or both) are chosen from 5, 6, 7, 8, 9, 10, 15, 20, 25, 30, 40, 50, 60, 70, 80, 90, 100, 200, 300, or all of the genes or gene products described in **Table 3.**

In yet another embodiment, the method acquires a read, *e.g.*, sequences, a set of subject intervals (*e.g.*, subgenomic intervals, expressed subgenomic intervals, or both) from the tumor sample, wherein the subject intervals (*e.g.*, subgenomic intervals, expressed subgenomic intervals, or both) are chosen from 5, 6, 7, 8, 9, 10, 15, 20, 25, 30, 40, 50, 60, 70, 80, or all of the genes or gene products described in **Table 4.**

These and other sets and groups of subgenomic intervals are discussed in more detail elsewhere herein, *e.g.,* in the section entitled "Gene Selection."

Any of the methods described herein can be combined with one or more of the embodiments below.

In other embodiments, the sample is a tumor sample, *e.g.*, includes one or more premalignant or malignant cells. In certain embodiments, the sample, *e.g.*, the tumor sample, is acquired from a hematologic malignancy (or premaligancy), *e.g.*, a hematologic malignancy (or premaligancy) described herein. In certain embodiments, the sample, *e.g.,* the tumor sample, is acquired from a solid tumor, a soft tissue tumor or a metastatic lesion. In other embodiments, the sample, *e.g.,* the tumor sample, includes tissue or cells from a surgical margin. In certain embodiments, the sample, *e.g.,* the tumor sample, includes tumor-infiltrating lymphocytes. The sample can be histologically normal tissue. In another embodiment, the sample, *e.g.*, tumor sample, includes one or more circulating tumor cells (CTC) (*e.g.,* a CTC acquired from a blood sample). In an embodiment, the sample, *e.g..* the tumor sample, includes one or more non-malignant cells. In an embodiment, the sample, *e.g.*, the tumor sample, includes one or more tumor-infiltrating lymphocytes.

In one embodiment, the method further includes acquiring a sample, *e.g.,* a tumor sample as described herein. The sample can be acquired directly or indirectly. In an embodiment, the sample is acquired, *e.g.*, by isolation or purification, from a sample that contains both a malignant cell and a non-malignant cell (*e.g.*, tumor-infiltrating lymphocyte).

In other embodiments, the method includes evaluating a sample, *e.g.*, a histologically normal sample, *e.g.,* from a surgical margin, using the methods described herein. Applicants have discovered that samples obtained from histologically normal tissues (*e.g.*, otherwise histologically normal tissue margins) may still have an alteration as described herein. The methods may thus further include re-classifying a tissue sample based on the presence of the detected alteration.

In another embodiment, at least 10, 20, 30, 40, 50, 60, 70, 80, or 90% of the reads acquired or analyzed are for subject intervals (*e.g.,* subgenomic intervals, expressed subgenomic intervals, or both) from genes described herein, *e.g.,* genes from **Tables 1-4 or FIGs. 3A-4D.**

In an embodiment, at least 10, 20, 30, 40, 50, 60, 70, 80, or 90% of the mutation calls made in the method are for subject intervals (*e.g.*, subgenomic intervals, expressed subgenomic intervals, or both) from genes or gene products described herein, *e.g.*, genes or gene products from **Tables 1-4 or FIGs. 3A-4D.**

In an embodiment, at least 10, 20, 30, 40, 50, 60, 70, 80, or 90% of the unique threshold values used the method are for subject intervals (*e.g.*, subgenomic intervals, expressed subgenomic intervals, or both) from genes or gene products described herein, *e.g.,* genes or gene products from **Tables 1-4 or FIGs. 3A-4D.**

In an embodiment, at least 10, 20, 30, 40, 50, 60, 70, 80, or 90% of the mutation calls annotated, or reported to a third party, are for subject intervals (*e.g.*, subgenomic intervals, expressed subgenomic intervals, or both) from genes or gene products described herein, *e.g.*, genes or gene products from **Tables 1-4 or FIGs. 3A-4D.**

In an embodiment, the method comprises acquiring a nucleotide sequence read obtained from a tumor and/or control nucleic acid sample (*e.g.,* an FFPE-derived nucleic acid sample).

In an embodiment, the reads are provided by a NGS sequencing method.

In an embodiment, the method includes providing one or a plurality of libraries of nucleic acid members and sequencing preselected subgenomic intervals from a plurality of members of said one or a plurality of libraries. In embodiments the method can include a step of selecting a subset of said one or a plurality of libraries for sequencing, *e.g.*, a solution-based selection or a solid support-(*e.g.,* array-) based selection.

In an embodiment, the method includes the step of contacting one or a plurality of libraries with a plurality of baits to provide a selected subgroup of nucleic acids, *e.g.,* a library catch. In one embodiment, the contacting step is effected in solution hybridization. In another embodiment, the contacting step is effected in a solid support, *e.g.,* an array. In certain embodiments, the method includes repeating the hybridization step by one or more additional rounds of hybridization. In some embodiments, the methods further include subjecting the library catch to one or more additional rounds of hybridization with the same or different collection of baits.

In yet other embodiments, the methods further include analyzing the library catch. In one embodiment, the library catch is analyzed by a sequencing method, *e.g.,* a next-generation sequencing method as described herein. The methods include isolating a library catch by, *e.g.*, solution hybridization, and subjecting the library catch by nucleic acid sequencing. In certain embodiments, the library catch can be re-sequenced. Next-generation sequencing methods are known in the art, and are described, *e.g.,* in Metzker, M. (2010) Nature Biotechnology Reviews 11:31-46.

In an embodiment, the assigned value for a nucleotide position is transmitted to a third party, optionally, with explanatory annotation.

In an embodiment, the assigned value for a nucleotide position is not transmitted to a third party.

In an embodiment, the assigned value for a plurality of nucleotide position is transmitted to a third party, optionally, with explanatory annotations, and the assigned value for a second plurality of nucleotide position is not transmitted to a third party.

In an embodiment, at least 0.01, 0.02, 0.03, 0.04, 0.05, 0.1, 0.2, 0.3, 0.4, 0.5, 0.6, 0.7, 0.8, 0.9, 1.0, 2.0, 5.0, 10, 15, or 30 megabases, *e.g.*, genomic bases, are sequenced.

In an embodiment, the method comprises evaluating a plurality of reads that include at least one SNP.

In an embodiment, the method comprises determining an SNP allele ratio in the sample and/or control read.

In an embodiment, the method comprises assigning one or more reads to a subject, *e.g.,* by barcode deconvolution.

In an embodiment, the method comprises assigning one or more reads as a tumor read or a control read, *e.g.,* by barcode deconvolution.

In an embodiment, the method comprises mapping, *e.g.*, by alignment with a reference sequence, each of said one or more reads.

In an embodiment, the method comprises memorializing a called mutation.

In an embodiment, the method comprises annotating a called mutation, *e.g.*, annotating a called mutation with an indication of mutation structure, *e.g.*, a missense mutation, or function, *e.g.,* a disease phenotype.

In an embodiment, the method comprises acquiring nucleotide sequence reads for tumor and control nucleic acid.

In an embodiment, the method comprises calling a nucleotide value, *e.g.,* a variant, *e.g.*, a mutation, for each of the subject intervals (*e.g.*, subgenomic intervals, expressed subgenomic intervals, or both), *e.g.*, with a Bayesian calling method or a non-Bayesian calling method.

In an embodiment, multiple samples, *e.g.*, from different subjects, are processed simultaneously.

The methods disclosed herein can be used to detect alterations present in the genome or transcriptome of a subject, and can be applied to DNA and RNA sequencing, *e.g.*, targeted RNA and/or DNA sequencing. Thus, another aspect featured in the invention includes methods for targeted RNA sequencing, *e.g.*, sequencing of a cDNA derived from an RNA acquired from a sample, *e.g.,* an FFPE-sample, a blood sample, or a bone marrow aspirate sample, to detect an alteration described herein. The alteration can be rearrangement, *e.g.*, a rearrangement encoding a gene fusion. In other embodiments, the method includes detection of a change (*e.g.,* an increase or decrease) in the level of a gene or gene product, *e.g.,* a change in expression of a gene or gene product described herein. The methods can, optionally, include a step of enriching a sample for a target RNA. In other embodiments, the methods include the step of depleting the sample of certain high abundance RNAs, *e.g.*, ribosomal or globin RNAs. The RNA sequencing methods can be used, alone or in combination with the DNA sequencing methods described herein. In one embodiment, the method includes performing a DNA sequencing step and an RNA sequencing step. The methods can be performed in any order. For example, the method can include confirming by RNA sequencing the expression of an alteration described herein, *e.g.,* confirming expression of a mutation or a fusion detected by the DNA sequencing methods of the invention. In other embodiments, the method includes performing an RNA sequencing step, followed by a DNA sequencing step.

In another aspect, the invention features a method comprising building a database of sequencing/alignment artifacts for the targeted subgenomic regions. In an embodiment, the database can be used to filter out spurious mutation calls and improve specificity. In an embodiment the database is built by sequencing unrelated non-tumor (*e.g.*, FFPE, blood, or bone marrow aspirate) samples or cell-lines and recording non-reference allele events that appear more frequently than expected due to random sequencing error alone in 1 or more of these normal samples. This approach may classify germline variation as artifact, but that is acceptable in a method concerned with somatic mutations. This misclassification of germline variation as artifact may be ameliorated if desired by filtering this database for known germline variations (removing common variants) and for artifacts that appear in only 1 individual (removing rarer variations).

Methods disclosed herein allow integration of a number of optimized elements including optimized bait-based selection, optimized alignment, and optimized mutation calling, as applied, *e.g.*, to cancer related segments of the genome. Methods described herein provide for NGS-based analysis of tumors that can be optimized on a cancer-by-cancer, gene-by-gene and site-by-site basis. This can be applied *e.g.,* to the genes/sites and tumor types described herein. The methods optimize levels of sensitivity and specificity for mutation detection with a given sequencing technology. Cancer by cancer, gene by gene, and site by site optimization provides very high levels of sensitivity/specificity (*e.g.,* >99% for both) that are essential for a clinical product.

Methods described herein provide for clinical and regulatory grade comprehensive analysis and interpretation of genomic aberrations for a comprehensive set of plausibly actionable genes (which may typically range from 50 to 500 genes) using next-generation sequencing technologies from routine, real-world samples in order to inform optimal treatment and disease management decisions.

Methods described herein provide one-stop-shopping for oncologists/pathologists to send a tumor sample and receive a comprehensive analysis and description of the genomic and other molecular changes for that tumor, in order to inform optimal treatment and disease management decisions.

Methods described herein provide a robust, real-world clinical oncology diagnostic tool that takes standard available tumor samples and in one test provides a comprehensive genomic and other molecular aberration analysis to provide the oncologist with a comprehensive description of what aberrations may be driving the tumor and could be useful for informing the oncologists treatment decisions.

Methods described herein provide for a comprehensive analysis of a patient's cancer genome, with clinical grade quality. Methods include the most relevant genes and potential alterations and include one or more of the analysis of mutations (*e.g.*, indels or base substitutions), copy number, rearrangements, *e.g.*, translocations, expression, and epigenetic markers. The output of the genetic analysis can be contextualized with descriptive reporting of actionable results. Methods connect the use with an up to date set of relevant scientific and medical knowledge.

Methods described herein provide for increasing both the quality and efficiency of patient care. This includes applications where a tumor is of a rare or poorly studied type such that there is no standard of care or the patient is refractory to established lines of therapy and a rational basis for selection of further therapy or for clinical trial participation could be useful. E.g., the methods allow, at any point of therapy, selection where the oncologist would benefit by having the full "molecular image" and/or "molecular sub-diagnosis" available to inform decision making.

Methods described herein can comprise providing a report, *e.g.*, in electronic, web-based, or paper form, to the patient or to another person or entity, *e.g.,* a caregiver, *e.g.,* a physician, *e.g.,* an oncologist, a hospital, clinic, third-party payor, insurance company or government office. The report can comprise output from the method, *e.g.,* the identification of nucleotide values, the indication of the presence or absence of an alteration, mutation, or wild-type sequence, *e.g.*, for subject intervals (*e.g.*, subgenomic intervals, expressed subgenomic intervals, or both) associated with a tumor of the type of the sample. The report can also comprise information on the role of a sequence, *e.g.,* an alteration, mutation, or wild-type sequence, in disease. Such information can include information on prognosis, resistance, or potential or suggested therapeutic options. The report can comprise information on the likely effectiveness of a therapeutic option, the acceptability of a therapeutic option, or the advisability of applying the therapeutic option to a patient, *e.g.,* a patient having a sequence, alteration identified in the test, and in embodiments, identified in the report. *E.g.,* the report can include information, or a recommendation on, the administration of a drug, *e.g.,* the administration at a preselected dosage or in a preselected treatment regimen, *e.g.,* in combination with other drugs, to the patient. In an embodiment, not all mutations identified in the method are identified in the report. *E.g.,* the report can be limited to mutations in genes having a preselected level of correlation with the occurrence, prognosis, stage, or susceptibility of the cancer to treatment, *e.g.*, with a preselected therapeutic option. Methods featured herein allow for delivery of the report, *e.g.,* to an entity described herein, within 7, 14, or 21 days from receipt of the sample by the entity practicing the method.

Thus, methods featured in the invention allow a quick turnaround time, *e.g.*, within 7, 14 or 21 days of receipt of sample.

Methods described herein can also be used to evaluate a histologically normal sample, *e.g.,* samples from surgical margins. If one or more alterations as described herein is detected, the tissue can be re-classified, *e.g.*, as malignant or premalignant, and/or the course of treatment can be modified.

In certain aspects, the sequencing methods described herein are useful in non-cancer applications, *e.g.*, in forensic applications (*e.g.*, identification as alternative to, or in addition to, use of dental records), paternity testing, and disease diagnosis and prognosis, *e.g.*, for an infectious disease, an autoimmune disorder, cystic fibrosis, Huntington's Disease, Alzheimer's Disease, among others. For example, identification of genetic alterations by the methods described herein can indicate the presence or risk of an individual for developing a particular disorder.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In addition, the materials, methods, and examples are illustrative only and not intended to be limiting.

Other features and advantages of the invention will be apparent from the detailed description, drawings, and from the claims.

## BRIEF DESCRIPTION OF THE DRAWINGS

**FIGs. 1A-1F** show a flowchart depiction of an embodiment of a method for multigene analysis of a tumor sample.

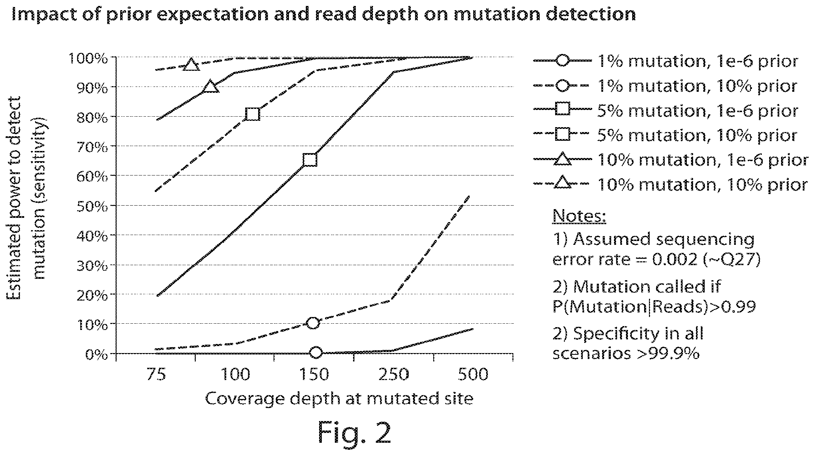

**FIG. 2** depicts the impact of prior expectation and read depth on mutation detection.

**FIGs. 3A-3B** depict additional exemplary genes that can be evaluated (*e.g.,* in solid tumors) in accordance with the methods described herein.

**FIGs. 4A-4D** depict additional exemplary genes that can be evaluated (*e.g.*, in hematologic malignancies or sarcomas) in accordance with the methods described herein.

**FIGs. 5-6** depict scatter plots showing the correlation between the whole exome mutational burden and the mutational burden measured from targeted genes.

**FIGs. 7A-7D** depict the tumor mutational burden distribution in lung cancer. TMB were determined by comprehensive genomic profiling in 10,676 cases of lung adenocarcinoma (**FIG. 7A**), 1,960 cases of lung squamous cell carcinoma (**FIG. 7B**), 220 cases of lung large cell carcinoma (**FIG. 7C**), and 784 cases of lung small cell carcinoma clinical specimens (**FIG. 7D**), respectively.

**FIGs. 8A-8E** depict the genetic alteration prevalence in lung cancer. Twenty-five genes frequently alternated in lung adenocarcinoma (**FIG. 8A**), lung squamous cell carcinoma (**FIG. 8B**), lung large cell carcinoma (**FIG. 8C**), and lung small cell carcinoma (**FIG. 8D**) were identified by comprehensive genomic profiling, respectively. Aggregated gene prevalence of all four subtypes of lung cancer (**FIG. 8E**) is shown. SV: short variants; CNA: copy number alterations; RE: rearrangements; Multiple: multiple types of alterations in the same gene.

**FIGs. 9A-9B** depict the tumor mutational burden distribution in colorectal adenocarcinoma. TMB were determined by comprehensive genomic profiling in 6,742 cases of colon adenocarcinoma (**FIG. 9A**) and 1,176 cases of rectum adenocarcinoma clinical specimens (**FIG. 9B**), respectively.

**FIGs. 10A-10C** depict the genetic alteration prevalence in colorectal adenocarcinoma. Twenty-five genes frequently alternated in colon adenocarcinoma (**FIG. 10A**) and rectum adenocarcinoma (**FIG. 10B**) were identified by comprehensive genomic profiling, respectively. Aggregated gene prevalence of colorectal adenocarcinoma (**FIG. 10C**) is shown. SV: short variants; CNA: copy number alterations; RE: rearrangements; Multiple: multiple types of alterations in the same gene.

**FIG. 11** depicts the tumor mutational burden distribution in twenty-four types of neoplasms. TMB were determined by comprehensive genomic profiling in a total of 15508 cases of clinical specimens, including, for example, tumors of bladder, brain, breast, cervix, head and neck, liver, ovary, pancreas, prostate, skin, stomach, and uterus.

## DETAILED DESCRIPTION

The invention is based, at least in part, on the discovery that profiling a small fraction of the genome or exome from a patient sample, *e.g.*, using a hybrid capture-based, next-generation sequencing (NGS) platform, serves as an effective surrogate for the analysis of total mutation load.

Without being bound by theory, the likelihood of generating immunogenic tumor neoantigens is believed to increase in a probabilistic fashion as mutations develop, increasing the likelihood of immune recognition (Gubin and Schreiber. Science 350:158-9, 2015). Assessing total mutational load, however, requires whole exome sequencing (WES). This approach necessitates specialized tissue processing, a matched normal specimen, and is largely performed as a research tool currently. Given the technical and informatics challenges of performing WES in clinical settings, surrogate methods of detecting mutational burden are needed. The methods including validated hybrid capture-based NGS platform described herein have several pragmatic advantages, including, for example, more clinically-feasible turnaround times (~2 weeks), standardized informatics pipelines, and more manageable costs. This approach has other advantages over traditional markers, such as protein expression detected by histochemistry, since it produces an objective (*e.g.*, mutation load) rather than a subjective measure (pathology scoring) (Hansen and Siu. *JAMA Oncol* 2(1):15-6, 2016). Further, this platform facilitates simultaneous detection of actionable alterations relevant for targeted therapies.

Accordingly, the invention provides, at least in part, methods of evaluating the mutation load in a sample, by providing a sequence of a set of subgenomic intervals from the sample; and determining a value for the mutational load, wherein the value is a function of the number of alterations in the set of subgenomic intervals. In certain embodiments, the set of subgenomic intervals are from a predetermined set of genes, for example, a predetermined set of genes that does not include the entire genome or exome. In certain embodiments, the set of subgenomic intervals is a set of coding subgenomic intervals. In other embodiments, the set of subgenomic intervals contains both a coding subgenomic interval and a non-coding subgenomic interval. In certain embodiments, the value for the mutation load is a function of the number of an alteration (*e.g.*, a somatic alteration) in the set of subgenomic intervals. In certain embodiments, the number of alterations excludes a functional alteration, a germline alteration, or both. In some embodiments, the sample is a tumor sample or a sample derived from a tumor. The methods described herein can also include, *e.g.*, one or more of: acquiring a library comprising a plurality of tumor members from the sample; contacting the library with a bait set to provide selected tumor members by hybridization, thereby providing a library catch; acquiring a read for a subgenomic interval comprising an alteration from the tumor member from the library catch; aligning the read by an alignment method; assigning a nucleotide value from the read for a preselected nucleotide position; and selecting a set of subgenomic intervals from a set of the assigned nucleotide positions, wherein the set of subgenomic intervals are from a predetermined set of genes. Systems for evaluating the mutation load in a sample are also disclosed.

Certain terms are first defined. Additional terms are defined throughout the specification.

As used herein, the articles "a" and "an" refer to one or to more than one (*e.g.,* to at least one) of the grammatical object of the article.

"About" and "approximately" shall generally mean an acceptable degree of error for the quantity measured given the nature or precision of the measurements. Exemplary degrees of error are within 20 percent (%), typically, within 10%, and more typically, within 5% of a given value or range of values.

"Acquire" or "acquiring" as the terms are used herein, refer to obtaining possession of a physical entity, or a value, *e.g.,* a numerical value, by "directly acquiring" or "indirectly acquiring" the physical entity or value. "Directly acquiring" means performing a process (*e.g.*, performing a synthetic or analytical method) to obtain the physical entity or value. "Indirectly acquiring" refers to receiving the physical entity or value from another party or source (*e.g.,* a third party laboratory that directly acquired the physical entity or value). Directly acquiring a physical entity includes performing a process that includes a physical change in a physical substance, *e.g.,* a starting material. Exemplary changes include making a physical entity from two or more starting materials, shearing or fragmenting a substance, separating or purifying a substance, .combining two or more separate entities into a mixture, performing a chemical reaction that includes breaking or forming a covalent or non covalent bond. Directly acquiring a value includes performing a process that includes a physical change in a sample or another substance, *e.g.*, performing an analytical process which includes a physical change in a substance, *e.g.,* a sample, analyte, or reagent (sometimes referred to herein as "physical analysis"), performing an analytical method, *e.g.,* a method which includes one or more of the following: separating or purifying a substance, *e.g.*, an analyte, or a fragment or other derivative thereof, from another substance; combining an analyte, or fragment or other derivative thereof, with another substance, *e.g.*, a buffer, solvent, or reactant; or changing the structure of an analyte, or a fragment or other derivative thereof, *e.g.,* by breaking or forming a covalent or non-covalent bond, between a first and a second atom of the analyte; or by changing the structure of a reagent, or a fragment or other derivative thereof, *e.g.,* by breaking or forming a covalent or non-covalent bond, between a first and a second atom of the reagent.

"Acquiring a sequence" or "acquiring a read" as the term is used herein, refers to obtaining possession of a nucleotide sequence or amino acid sequence, by "directly acquiring" or "indirectly acquiring" the sequence or read. "Directly acquiring" a sequence or read means performing a process (*e.g.*, performing a synthetic or analytical method) to obtain the sequence, such as performing a sequencing method (*e.g.,* a Next-generation Sequencing (NGS) method). "Indirectly acquiring" a sequence or read refers to receiving information or knowledge of, or receiving, the sequence from another party or source (*e.g.,* a third party laboratory that directly acquired the sequence). The sequence or read acquired need not be a full sequence, *e.g.*, sequencing of at least one nucleotide, or obtaining information or knowledge, that identifies one or more of the alterations disclosed herein as being present in a subject constitutes acquiring a sequence.

Directly acquiring a sequence or read includes performing a process that includes a physical change in a physical substance, *e.g.*, a starting material, such as a tissue or cellular sample, *e.g.*, a biopsy, or an isolated nucleic acid (*e.g.*, DNA or RNA) sample. Exemplary changes include making a physical entity from two or more starting materials, shearing or fragmenting a substance, such as a genomic DNA fragment; separating or purifying a substance (*e.g.*, isolating a nucleic acid sample from a tissue); combining two or more separate entities into a mixture, performing a chemical reaction that includes breaking or forming a covalent or non-covalent bond. Directly acquiring a value includes performing a process that includes a physical change in a sample or another substance as described above.

"Acquiring a sample" as the term is used herein, refers to obtaining possession of a sample, *e.g.,* a tissue sample or nucleic acid sample, by "directly acquiring" or "indirectly acquiring" the sample. "Directly acquiring a sample" means performing a process (*e.g.,* performing a physical method such as a surgery or extraction) to obtain the sample. "Indirectly acquiring a sample" refers to receiving the sample from another party or source (*e.g.,* a third party laboratory that directly acquired the sample). Directly acquiring a sample includes performing a process that includes a physical change in a physical substance, *e.g.,* a starting material, such as a tissue, *e.g.,* a tissue in a human patient or a tissue that has was previously isolated from a patient. Exemplary changes include making a physical entity from a starting material, dissecting or scraping a tissue; separating or purifying a substance (*e.g.*, a sample tissue or a nucleic acid sample); combining two or more separate entities into a mixture; performing a chemical reaction that includes breaking or forming a covalent or non-covalent bond. Directly acquiring a sample includes performing a process that includes a physical change in a sample or another substance, *e.g.*, as described above.

"Alignment selector," as used herein, refers to a parameter that allows or directs the selection of an alignment method, *e.g.,* an alignment algorithm or parameter, that can optimize the sequencing of a preselected subgenomic interval. An alignment selector can be specific to, or selected as a function, *e.g.,* of one or more of the following:

1. The sequence context, *e.g.,* sequence context, of a subgenomic interval (*e.g.,* the preselected nucleotide position to be evaluated) that is associated with a propensity for misalignment of reads for said subgenomic interval. *E.g.*, the existence of a sequence element in or near the subgenomic interval to be evaluated that is repeated elsewhere in the genome can cause misalignment and thereby reduce performance. Performance can be enhanced by selecting an algorithm or an algorithm parameter that minimizes misalignment. In this case the value for the alignment selector can be a function of the sequence context, *e.g.,* the presence or absence of a sequence of preselected length that is repeated at least a preselected number of times in the genome (or in the portion of the genome being analyzed).

2. The tumor type being analyzed. *E.g.*, a specific tumor type can be characterized by increased rate of deletions. Thus, performance can be enhanced by selecting an algorithm or algorithm parameter that is more sensitive to indels. In this case the value for the alignment selector can be a function of the tumor type, *e.g.,* an identifier for the tumor type. In an embodiment the value is the identity of the tumor type, *e.g.,* a hematologic malignancy (or premaligancy).

3. The gene, or type of gene, being analyzed, *e.g.,* a gene, or type of gene, can be analyzed. Oncogenes, by way of example, are often characterized by substitutions or in-frame indels. Thus, performance can be enhanced by selecting an algorithm or algorithm parameter that is particularly sensitive to these variants and specific against others. Tumor suppressors are often characterized by frame-shift indels. Thus, performance can be enhanced by selecting an algorithm or algorithm parameter that is particularly sensitive to these variants. Thus, performance can be enhanced by selecting an algorithm or algorithm parameter matched with the subgenomic interval. In this case the value for the alignment selector can be a function of the gene or gene type, *e.g.,* an identifier for gene or gene type. In an embodiment the value is the identity of the gene.

4. The site (*e.g.*, nucleotide position) being analyzed. In this case the value for the alignment selector can be a function of the site or the type of site, *e.g.,* an identifier for the site or site type. In an embodiment the value is the identity of the site. (*E.g.*, if the gene containing the site is highly homologous with another gene, normal/fast short read alignment algorithms (*e.g.,* BWA) may have difficulty distinguishing between the two genes, potentially necessitating more intensive alignment methods (Smith-Waterman) or even assembly (ARACHNE). Similarly, if the gene sequence contains low-complexity regions (*e.g.,* AAAAAA), more intensive alignment methods may be necessary.

5. The variant, or type of variant, associated with the subgenomic interval being evaluated. *E.g.,* a substitution, insertion, deletion, translocation or other rearrangement. Thus, performance can be enhanced by selecting an algorithm or algorithm parameter that is more sensitive to the specific variant type. In this case the value for the alignment selector can be a function of the type of variant, *e.g.,* an identifier for the type of variant. In an embodiment the value is the identity of the type of variant, *e.g.*, a substitution.

6. The type of sample, a FFPE or other fixed sample. Sample type/quality can affect error (spurious observation of non-reference sequence) rate. Thus, performance can be enhanced by selecting an algorithm or algorithm parameter that accurately models the true error rate in the sample. In this case the value for the alignment selector can be a function of the type of sample, *e.g.*, an identifier for the sample type. In an embodiment, the value is the identity of the sample type, *e.g.,* a fixed sample.

"Alteration" or "altered structure" as used herein, of a gene or gene product (*e.g.*, a marker gene or gene product) refers to the presence of a mutation or mutations within the gene or gene product, *e.g.,* a mutation, which affects integrity, sequence, structure, amount or activity of the gene or gene product, as compared to the normal or wild-type gene. The alteration can be in amount, structure, and/or activity in a cancer tissue or cancer cell, as compared to its amount, structure, and/or activity, in a normal or healthy tissue or cell (*e.g.,* a control), and is associated with a disease state, such as cancer. For example, an alteration which is associated with cancer, or predictive of responsiveness to anti-cancer therapeutics, can have an altered nucleotide sequence (*e.g.,* a mutation), amino acid sequence, chromosomal translocation, intra-chromosomal inversion, copy number, expression level, protein level, protein activity, epigenetic modification (*e.g.,* methylation or acetylation status, or post-translational modification, in a cancer tissue or cancer cell, as compared to a normal, healthy tissue or cell. Exemplary mutations include, but are not limited to, point mutations (*e.g.,* silent, missense, or nonsense), deletions, insertions, inversions, duplications, amplification, translocations, inter- and intra-chromosomal rearrangements. Mutations can be present in the coding or non-coding region of the gene. In certain embodiments, the alteration(s) is detected as a rearrangement, *e.g.,* a genomic rearrangement comprising one or more introns or fragments thereof (*e.g.,* one or more rearrangements in the 5'- and/or 3'-UTR). In certain embodiments, the alterations are associated (or not associated) with a phenotype, *e.g.,* a cancerous phenotype (*e.g.,* one or more of cancer risk, cancer progression, cancer treatment or resistance to cancer treatment). In one embodiment, the alteration is associated with one or more of: a genetic risk factor for cancer, a positive treatment response predictor, a negative treatment response predictor, a positive prognostic factor, a negative prognostic factor, or a diagnostic factor.

As used herein, the term "indel" refers to an insertion, a deletion, or both, of one or more nucleotides in a nucleic acid of a cell. In certain embodiments, an indel includes both an insertion and a deletion of one or more nucleotides, where both the insertion and the deletion are nearby on the nucleic acid. In certain embodiments, the indel results in a net change in the total number of nucleotides. In certain embodiments, the indel results in a net change of about 1 to about 50 nucleotides.

"Clonal profile", as that term is used herein, refers to the occurrence, identity, variability, distribution, expression (the occurrence or level of transcribed copies of a subgenomic signature), or abundance, *e.g.,* the relative abundance, of one or more sequences, *e.g.,* an allele or signature, of a subject interval (or of a cell comprising the same). In an embodiment, the clonal profile is a value for the relative abundance for one sequence, allele, or signature, for a subject interval (or of a cell comprising the same) when a plurality of sequences, alleles, or signatures for that subject interval are present in a sample. *E.g.,* in an embodiment, a clonal profile comprises a value for the relative abundance, of one or more of a plurality of VDJ or VJ combinations for a subject interval. In an embodiment, a clonal profile comprises a value for the relative abundance, of a selected V segment, for a subject interval. In an embodiment, a clonal profile comprises a value for the diversity, *e.g.,* as arises from somatic hypermutation, within the sequences of a subject interval. In an embodiment, a clonal profile comprises a value for the occurrence or level of expression of a sequence, allele, or signature, *e.g.,* as evidenced by the occurrence or level of an expressed subgenomic interval comprising the sequence, allele or signature.

"Expressed subgenomic interval", as that term is used herein, refers to the transcribed sequence of a subgenomic interval. In an embodiment, the sequence of the expressed subgenomic interval will differ from the subgenomic interval from which it is transcribed, *e.g.,* as some sequence may not be transcribed.

"Signature", as that term is used herein, refers to a sequence of a subject interval. A signature can be diagnostic of the occurrence of one of a plurality of possibilities at a subject interval, *e.g.,* a signature can be diagnostic of: the occurrence of a selected V segment in a rearranged heavy or light chain variable region gene; the occurrence of a selected VJ junction, *e.g.,* the occurrence of a selected V and a selected J segment in a rearranged heavy chain variable region gene. In an embodiment, a signature comprises a plurality of a specific nucleic acid sequences. Thus, a signature is not limited to a specific nucleic acid sequence, but rather is sufficiently unique that it can distinguish between a first group of sequences or possibilities at a subject interval and a second group of possibilities at a subject interval, *e.g.,* it can distinguish between a first V segment and a second V segment, allowing *e.g.,* evaluation of the usage of various V segments. The term signature comprises the term specific signature, which is a specific nucleic acid sequence. In an embodiment the signature is indicative of, or is the product of, a specific event, *e.g.,* a rearrangement event.

"Subgenomic interval" as that term is used herein, refers to a portion of genomic sequence. In an embodiment, a subgenomic interval can be a single nucleotide position, *e.g.,* a nucleotide position variants of which are associated (positively or negatively) with a tumor phenotype. In an embodiment, a subgenomic interval comprises more than one nucleotide position. Such embodiments include sequences of at least 2, 5, 10, 50, 100, 150, or 250 nucleotide positions in length. Subgenomic intervals can comprise an entire gene, or a preselected portion thereof, *e.g.,* the coding region (or portions thereof), a preselected intron (or portion thereof) or exon (or portion thereof). A subgenomic interval can comprise all or a part of a fragment of a naturally occurring, *e.g.,* genomic DNA, nucleic acid. *E.g.,* a subgenomic interval can correspond to a fragment of genomic DNA which is subjected to a sequencing reaction. In embodiments, a subgenomic interval is continuous sequence from a genomic source. In embodiments, a subgenomic interval includes sequences that are not contiguous in the genome, *e.g.,* it can include junctions formed found at exon-exon junctions in cDNA.

In an embodiment, a subgenomic interval corresponds to a rearranged sequence, *e.g.,* a sequence in a B or T cell that arises as a result of the joining of, a V segment to a D segment, a D segment to a J segment, a V segment to a J segment, or a J segment to a class segment.

In an embodiment, there is no diversity at a subgenomic interval.

In an embodiment, there is diversity at a subgenomic interval, *e.g.,* the subgenomic interval is represented by more than one sequence, *e.g.,* the subgenomic interval that covers a VD sequence can be represented by more than one signature.

In an embodiment, a subgenomic interval comprises or consists of: a single nucleotide position; an intragenic region or an intergenic region; an exon or an intron, or a fragment thereof, typically an exon sequence or a fragment thereof; a coding region or a non-coding region, *e.g.,* a promoter, an enhancer, a 5' untranslated region (5' UTR), or a 3' untranslated region (3' UTR), or a fragment thereof; a cDNA or a fragment thereof; an SNP; a somatic mutation, a germline mutation or both; an alteration, *e.g.,* a point or a single mutation; a deletion mutation (*e.g.,* an in-frame deletion, an intragenic deletion, a full gene deletion); an insertion mutation (*e.g.,* intragenic insertion); an inversion mutation (*e.g.,* an intra-chromosomal inversion); a linking mutation; a linked insertion mutation; an inverted duplication mutation; a tandem duplication (*e.g.,* an intrachromosomal tandem duplication); a translocation (*e.g.,* a chromosomal translocation, a non-reciprocal translocation); a rearrangement (*e.g.,* a genomic rearrangement (*e.g.,* a rearrangement of one or more introns, or a fragment thereof; a rearranged intron can include a 5'- and/or 3'- UTR)); a change in gene copy number; a change in gene expression; a change in RNA levels; or a combination thereof. The "copy number of a gene" refers to the number of DNA sequences in a cell encoding a particular gene product. Generally, for a given gene, a mammal has two copies of each gene. The copy number can be increased, *e.g.,* by gene amplification or duplication, or reduced by deletion.

"Subject interval", as that term is used herein, refers to a subgenomic interval or an expressed subgenomic interval. In an embodiment, a subgenomic interval and an expressed subgenomic interval correspond, meaning that the expressed subgenomic interval comprises sequence expressed from the corresponding subgenomic interval. In an embodiment, a subgenomic interval and an expressed subgenomic interval are non-corresponding, meaning that the expressed subgenomic interval does not comprise sequence expressed from the non-corresponding subgenomic interval, but rather corresponds to a different subgenomic interval. In an embodiment, a subgenomic interval and an expressed subgenomic interval partially correspond, meaning that the expressed subgenomic interval comprises sequence expressed from the corresponding subgenomic interval and sequence expressed from a different corresponding subgenomic interval.

As used herein, the term "library" refers to a collection of members. In one embodiment, the library includes a collection of nucleic acid members, *e.g.,* a collection of whole genomic, subgenomic fragments, cDNA, cDNA fragments, RNA, *e.g.,* mRNA, RNA fragments, or a combination thereof. In one embodiment, a portion or all of the library members comprises an adapter sequence. The adapter sequence can be located at one or both ends. The adapter sequence can be useful, *e.g.,* for a sequencing method (*e.g.,* an NGS method), for amplification, for reverse transcription, or for cloning into a vector.

The library can comprise a collection of members, *e.g.,* a target member (*e.g.,* a tumor member, a reference member, a PGx member, or a combination thereof). The members of the library can be from a single individual. In embodiments, a library can comprise members from more than one subject (*e.g.,* 2, 3, 4, 5, 6, 7, 8, 9, 10, 20, 30 or more subjects), *e.g.,* two or more libraries from different subjects can be combined to form a library comprising members from more than one subject. In one embodiment, the subject is a human having, or at risk of having, a cancer or tumor.

"Library catch" refers to a subset of a library, *e.g.,* a subset enriched for preselected subgenomic intervals, *e.g.,* product captured by hybridization with preselected baits.

"Member" or "library member" or other similar term, as used herein, refers to a nucleic acid molecule, *e.g.,* a DNA, RNA, or a combination thereof, that is the member of a library. Typically, a member is a DNA molecule, *e.g.,* genomic DNA or cDNA. A member can be fragmented, *e.g.,* sheared or enzymatically prepared, genomic DNA. Members comprise sequence from a subject and can also comprise sequence not derived from the subject, *e.g.,* an adapter sequence, a primer sequence, or other sequences that allow for identification, *e.g.,* "barcode" sequences.

"Bait," as used herein, is type of hybrid capture reagent. A bait can be a nucleic acid molecule, *e.g.,* a DNA or RNA molecule, which can hybridize to (*e.g.,* be complementary to), and thereby allow capture of a target nucleic acid. In one embodiment, a bait is an RNA molecule (*e.g.,* a naturally-occurring or modified RNA molecule); a DNA molecule (*e.g.,* a naturally-occurring or modified DNA molecule), or a combination thereof. In other embodiments, a bait includes a binding entity, *e.g.,* an affinity tag, that allows capture and separation, *e.g.,* by binding to a binding entity, of a hybrid formed by a bait and a nucleic acid hybridized to the bait. In one embodiment, a bait is suitable for solution phase hybridization. In one embodiment, a bait is a bicyclic nuclei acid (BNA) molecule.

"Bait set," as used herein, refers to one or a plurality of bait molecules.

"Binding entity" means any molecule to which molecular tags can be directly or indirectly attached that is capable of specifically binding to an analyte. The binding entity can be an affinity tag on each bait sequence. In certain embodiments, the binding entity allows for separation of the bait/member hybrids from the hybridization mixture by binding to a partner, such as an avidin molecule, or an antibody that binds to the hapten or an antigen-binding fragment thereof. Exemplary binding entities include, but are not limited to, a biotin molecule, a hapten, an antibody, an antibody binding fragment, a peptide, and a protein.

"Complementary" refers to sequence complementarity between regions of two nucleic acid strands or between two regions of the same nucleic acid strand. It is known that an adenine residue of a first nucleic acid region is capable of forming specific hydrogen bonds ("base pairing") with a residue of a second nucleic acid region which is antiparallel to the first region if the residue is thymine or uracil. Similarly, it is known that a cytosine residue of a first nucleic acid strand is capable of base pairing with a residue of a second nucleic acid strand which is antiparallel to the first strand if the residue is guanine. A first region of a nucleic acid is complementary to a second region of the same or a different nucleic acid if, when the two regions are arranged in an antiparallel fashion, at least one nucleotide residue of the first region is capable of base pairing with a residue of the second region. In certain embodiments, the first region comprises a first portion and the second region comprises a second portion, whereby, when the first and second portions are arranged in an antiparallel fashion, at least about 50%, at least about 75%, at least about 90%, or at least about 95% of the nucleotide residues of the first portion are capable of base pairing with nucleotide residues in the second portion. In other embodiments, all nucleotide residues of the first portion are capable of base pairing with nucleotide residues in the second portion.

The term "cancer" or "tumor" is used interchangeably herein. These terms refer to the presence of cells possessing characteristics typical of cancer-causing cells, such as uncontrolled proliferation, immortality, metastatic potential, rapid growth and proliferation rate, and certain characteristic morphological features. Cancer cells are often in the form of a tumor, but such cells can exist alone within an animal, or can be a non-tumorigenic cancer cell, such as a leukemia cell. These terms include a solid tumor, a soft tissue tumor, or a metastatic lesion. As used herein, the term "cancer" includes premalignant, as well as malignant cancers.

"Likely to" or "increased likelihood," as used herein, refers to an increased probability that an item, object, thing or person will occur. Thus, in one example, a subject that is likely to respond to treatment has an increased probability of responding to treatment relative to a reference subject or group of subjects.

"Unlikely to" refers to a decreased probability that an event, item, object, thing or person will occur with respect to a reference. Thus, a subject that is unlikely to respond to treatment has a decreased probability of responding to treatment relative to a reference subject or group of subjects.

"Control member" refers to a member having sequence from a non-tumor cell.

"Indel alignment sequence selector," as used herein, refers to a parameter that allows or directs the selection of a sequence to which a read is to be aligned with in the case of a preselected indel. Use of such a sequence can optimize the sequencing of a preselected subgenomic interval comprising an indel. The value for an indel alignment sequence selector is a function of a preselected indel, *e.g.,* an identifier for the indel. In an embodiment the value is the identity of the indel.

"Next-generation sequencing or NGS or NG sequencing" as used herein, refers to any sequencing method that determines the nucleotide sequence of either individual nucleic acid molecules (*e.g.*, in single molecule sequencing) or clonally expanded proxies for individual nucleic acid molecules in a high throughput fashion (*e.g.,* greater than $10^3$, $10^4$, $10^5$ or more molecules are sequenced simultaneously). In one embodiment, the relative abundance of the nucleic acid species in the library can be estimated by counting the relative number of occurrences of their cognate sequences in the data generated by the sequencing experiment. Next-generation sequencing methods are known in the art, and are described, *e.g..* in Metzker, M. (2010) Nature Biotechnology Reviews 11:31-46. Next-generation sequencing can detect a variant present in less than 5% of the nucleic acids in a sample.

"Nucleotide value" as referred herein, represents the identity of the nucleotide(s) occupying or assigned to a preselected nucleotide position. Typical nucleotide values include: missing (*e.g.*, deleted); additional (*e.g.*, an insertion of one or more nucleotides, the identity of which may or may not be included); or present (occupied); A; T; C; or G. Other values can be, *e.g.*, not Y, wherein Y is A. T, G, or C; A or X, wherein X is one or two of T, G, or C; T or X, wherein X is one or two of A, G, or C; G or X, wherein X is one or two of T, A, or C; C or X, wherein X is one or two of T, G, or A; a pyrimidine nucleotide; or a purine nucleotide. A nucleotide value can be a frequency for 1 or more, *e.g.*, 2, 3, or 4, bases (or other value described herein, *e.g.,* missing or additional) at a nucleotide position. E.g., a nucleotide value can comprise a frequency for A, and a frequency for G, at a nucleotide position.

"Or" is used herein to mean, and is used interchangeably with, the term "and/or", unless context clearly indicates otherwise. The use of the term "and/or" in some places herein does not mean that uses of the term "or" are not interchangeable with the term "and/or" unless the context clearly indicates otherwise.

"Primary control" refers to a non tumor tissue other than NAT tissue in a tumor sample. Blood is a typical primary control.

"Rearrangement alignment sequence selector," as used herein, refers to a parameter that allows or directs the selection of a sequence to which a read is to be aligned with in the case of a preselected rearrangement. Use of such a sequence can optimize the sequencing of a preselected subgenomic interval comprising a rearrangement. The value for a rearrangement alignment sequence selector is a function of a preselected rearrangement, *e.g.*, an identifier for the rearrangement. In an embodiment the value is the identity of the rearrangement. An "indel alignment sequence selector" (also defined elsewhere herein) is an example of a rearrangement alignment sequence selector.

"Sample," "tissue sample," "patient sample," "patient cell or tissue sample" or "specimen" comprises a tissue, a cell, *e.g.,* a circulating cell, obtained from a subject or patient. The source of the tissue sample can be solid tissue as from a fresh, frozen and/or preserved organ, tissue sample, biopsy, or aspirate; blood or any blood constituents; bodily fluids such as cerebral spinal fluid, amniotic fluid, peritoneal fluid or interstitial fluid; or cells from any time in gestation or development of the subject. The tissue sample can contain compounds that are not naturally intermixed with the tissue in nature such as preservatives, anticoagulants, buffers, fixatives, nutrients, antibiotics or the like. In one embodiment, the sample is preserved as a frozen sample or as formaldehyde- or paraformaldehyde-fixed paraffin-embedded (FFPE) tissue preparation. For example, the sample can be embedded in a matrix, *e.g.*, an FFPE block or a frozen sample. In another embodiment, the sample is a blood sample. In yet another embodiment, the sample is a bone marrow aspirate sample. In another embodiment, the sample comprises circulating tumor DNA (ctDNA). In another embodiment, the sample comprises circulating tumor cells (CTCs).

In an embodiment, the sample is a cell associated with a tumor, *e.g.,* a tumor cell or a tumor-infiltrating lymphocyte (TIL). In one embodiment, the sample is a tumor sample, *e.g.,* includes one or more premalignant or malignant cells. In an embodiment, the sample is acquired from a hematologic malignancy (or premaligancy), *e.g.*, a hematologic malignancy (or premaligancy) described herein. In certain embodiments, the sample, *e.g.,* the tumor sample, is acquired from a solid tumor, a soft tissue tumor or a metastatic lesion. In other embodiments, the sample, *e.g.*, the tumor sample, includes tissue or cells from a surgical margin. In another embodiment, the sample, *e.g.*, tumor sample, includes one or more circulating tumor cells (CTC) (*e.g.,* a CTC acquired from a blood sample). In an embodiment, the sample is a cell not associated with a tumor, *e.g..* a non-tumor cell or a peripheral blood lymphocyte.

"Sensitivity," as used herein, is a measure of the ability of a method to detect a preselected sequence variant in a heterogeneous population of sequences. A method has a sensitivity of S% for variants of F% if, given a sample in which the preselected sequence variant is present as at least F% of the sequences in the sample, the method can detect the preselected sequence at a preselected confidence of C%, S% of the time. By way of example, a method has a sensitivity of 90% for variants of 5% if, given a sample in which the preselected variant sequence is present as at least 5% of the sequences in the sample, the method can detect the preselected sequence at a preselected confidence of 99%, 9 out of 10 times (F=5%; C=99%; S=90%). Exemplary sensitivities include those of S=90%, 95%, 99% for sequence variants at F=1%, 5%, 10%, 20%, 50%, 100% at confidence levels of C= 90%, 95%, 99%, and 99.9%.

"Specificity," as used herein, is a measure of the ability of a method to distinguish a truly occurring preselected sequence variant from sequencing artifacts or other closely related sequences. It is the ability to avoid false positive detections. False positive detections can arise from errors introduced into the sequence of interest during sample preparation, sequencing error, or inadvertent sequencing of closely related sequences like pseudo-genes or members of a gene family. A method has a specificity of X% if, when applied to a sample set of $N_{Total}$ sequences, in which $X_{True}$ sequences are truly variant and $X_{Not\,true}$ are not truly variant, the method selects at least X % of the not truly variant as not variant. E.g., a method has a specificity of 90% if, when applied to a sample set of 1,000 sequences, in which 500 sequences are truly variant and 500 are not truly variant, the method selects 90% of the 500 not truly variant sequences as not variant. Exemplary specificities include 90, 95, 98, and 99%.

A "tumor nucleic acid sample" as used herein, refers to nucleic acid molecules from a tumor or cancer sample. Typically, it is DNA, *e.g.*, genomic DNA, or cDNA derived from RNA, from a tumor or cancer sample. In certain embodiments, the tumor nucleic acid sample is purified or isolated (*e.g.,* it is removed from its natural state).

A "control" or "reference" "nucleic acid sample" as used herein, refers to nucleic acid molecules from a control or reference sample. Typically, it is DNA, *e.g.*, genomic DNA, or cDNA derived from RNA, not containing the alteration or variation in the gene or gene product. In certain embodiments, the reference or control nucleic acid sample is a wild-type or a non-mutated sequence. In certain embodiments, the reference nucleic acid sample is purified or isolated (*e.g.,* it is removed from its natural state). In other embodiments, the reference nucleic acid sample is from a non-tumor sample, *e.g.,* a blood control, a normal adjacent tissue (NAT), or any other non-cancerous sample from the same or a different subject.

"Sequencing" a nucleic acid molecule requires determining the identity of at least 1 nucleotide in the molecule (*e.g.*, a DNA molecule, an RNA molecule, or a cDNA molecule derived from an RNA molecule). In embodiments the identity of less than all of the nucleotides in a molecule are determined. In other embodiments, the identity of a majority or all of the nucleotides in the molecule is determined.

"Threshold value," as used herein, is a value that is a function of the number of reads required to be present to assign a nucleotide value to a subject interval (*e.g.*, a subgenomic interval or an expressed subgenomic interval). E.g., it is a function of the number of reads having a specific nucleotide value, *e.g.*, "A," at a nucleotide position, required to assign that nucleotide value to that nucleotide position in the subgenomic interval. The threshold value can, *e.g.,* be expressed as (or as a function of) a number of reads, *e.g.,* an integer, or as a proportion of reads having the preselected value. By way of example, if the threshold value is X, and X+1 reads having the nucleotide value of "A" are present, then the value of "A" is assigned to the preselected position in the subject interval (*e.g.*, subgenomic interval or expressed subgenomic interval). The threshold value can also be expressed as a function of a mutation or variant expectation, mutation frequency, or of Bayesian prior. In an embodiment, a preselected mutation frequency would require a preselected number or proportion of reads having a nucleotide value, *e.g.,* A or G, at a preselected position, to call that nucleotide value. In embodiments the threshold value can be a function of mutation expectation, *e.g.*, mutation frequency, and tumor type. *E.g.,* a preselected variant at a preselected nucleotide position could have a first threshold value if the patient has a first tumor type and a second threshold value if the patient has a second tumor type.

As used herein, "target member" refers to a nucleic acid molecule that one desires to isolate from the nucleic acid library. In one embodiment, the target members can be a tumor member, a reference member, a control member, or a PGx member as described herein.

"Tumor member," or other similar term (*e.g.,* a "tumor or cancer-associated member"), as used herein refers to a member having sequence from a tumor cell. In one embodiment, the tumor member includes a subject interval (*e.g.,* a subgenomic interval or an expressed subgenomic interval) having a sequence (*e.g.,* a nucleotide sequence) that has an alteration (*e.g.,* a mutation) associated with a cancerous phenotype. In other embodiments, the tumor member includes a subject interval (*e.g.,* a subgenomic interval or an expressed subgenomic interval) having a wild-type sequence (*e.g.,* a wild-type nucleotide sequence). For example, a subject interval (*e.g.,* a subgenomic interval or an expressed subgenomic interval) from a heterozygous or homozygous wild-type allele present in a cancer cell. A tumor member can include a reference member or a PGx member.

"Reference member," or other similar term (*e.g.,* a "control member"), as used herein, refers to a member that comprises a subject interval (*e.g.,* a subgenomic interval or an expressed subgenomic interval) having a sequence (*e.g.,* a nucleotide sequence) that is not associated with the cancerous phenotype. In one embodiment, the reference member includes a wild-type or a non-mutated nucleotide sequence of a gene or gene product that when mutated is associated with the cancerous phenotype. The reference member can be present in a cancer cell or non-cancer cell.

"PGx member" or other similar term, as used herein, refers to a member that comprises a subject interval (*e.g.*, a subgenomic interval or an expressed subgenomic interval) that is associated with the pharmacogenetic or pharmacogenomic profile of a gene. In one embodiment, the PGx member includes an SNP (*e.g.,* an SNP as described herein). In other embodiments, the PGx member includes a subject interval (*e.g.*. a subgenomic interval or an expressed subgenomic interval) according to **Tables 1-4 or FIGs. 3A-4D.**

"Variant," as used herein, refers to a structure that can be present at a subgenomic interval that can have more than one structure, *e.g.,* an allele at a polymorphic locus.

As used herein, "X is a function of Y" means, *e.g.,* one variable X is associated with another variable Y. In one embodiment, if X is a function of Y, a causal relationship between X and Y may be implied, but does not necessarily exist.

Headings, *e.g.*, (a), (b), (i) etc., are presented merely for ease of reading the specification and claims. The use of headings in the specification or claims does not require the steps or elements be performed in alphabetical or numerical order or the order in which they are presented.

## MUTATION LOAD

As used herein, the term "mutation load" or "mutational load" refers to the level, *e.g.*, number, of an alteration (*e.g.,* one or more alterations, *e.g.,* one or more somatic alterations) per a preselected unit (*e.g.,* per megabase) in a predetermined set of genes (*e.g.,* in the coding regions of the predetermined set of genes). Mutation load can be measured, *e.g.*, on a whole genome or exome basis, or on the basis of a subset of genome or exome. In certain embodiments, the mutation load measured on the basis of a subset of genome or exome can be extrapolated to determine a whole genome or exome mutation load.

In certain embodiments, the mutation load is measured in a sample, *e.g.*, a tumor sample (*e.g.*, a tumor sample or a sample derived from a tumor), from a subject. *e.g.,* a subject described herein. In certain embodiments, the mutation load is expressed as a percentile, *e.g.*, among the mutation loads in samples from a reference population. In certain embodiments, the reference population includes patients having the same type of cancer as the subject. In other embodiments, the reference population includes patients who are receiving, or have received, the same type of therapy, as the subject. In certain embodiments, the mutation load obtained by a method described herein, *e.g.,* by evaluating the level of an alteration *(e.g.,* a somatic alteration) in a predetermined set of genes set forth in **Tables 1-4 or FIGs. 3A-4D,** correlates with the whole genome or exome mutation load.

The terms "mutation load," "mutational load," "mutation burden," and "mutational burden" are used interchangeably herein. In the context of a tumor, a mutational load is also referred to herein as "tumor mutational burden," "tumor mutation burden," or "TMB."

*Gene Selection*

The selected genes or gene products (also referred to herein as the "target genes or gene products") can include subject intervals (*e.g*., subgenomic intervals, expressed subgenomic intervals, or both) comprising intragenic regions or intergenic regions. For example, the subject intervals (*e.g.,* subgenomic interval or expressed subgenomic interval) can include an exon or an intron, or a fragment thereof, typically an exon sequence or a fragment thereof. The subject interval (*e.g..* subgenomic interval or expressed subgenomic interval) can include a coding region or a non-coding region, *e.g.,* a promoter, an enhancer, a 5' untranslated region (5' UTR), or a 3' untranslated region (3' UTR), or a fragment thereof. In other embodiments, the subject interval includes a cDNA or a fragment thereof. In other embodiments, the subject interval includes an SNP, *e.g.,* as described herein.

In other embodiments, the subject intervals (*e.g.,* subgenomic intervals, expressed subgenomic intervals, or both) include substantially all exons in a genome, *e.g.,* one or more of the subject intervals (*e.g.,* subgenomic intervals, expressed subgenomic intervals, or both) as described herein (*e.g.,* exons from selected genes or gene products of interest (*e.g.,* genes or gene products associated with a cancerous phenotype as described herein)). In one embodiment, the subject interval (*e.g.,* subgenomic interval or expressed subgenomic interval) includes a somatic mutation, a germline mutation or both. In one embodiment, the subject interval (*e.g.,* subgenomic interval or expressed subgenomic interval) includes an alteration, *e.g.,* a point or a single mutation, a deletion mutation (*e.g.,* an in-frame deletion, an intragenic deletion, a full gene deletion), an insertion mutation (*e.g.,* intragenic insertion), an inversion mutation (*e.g.,* an intra-chromosomal inversion), a linking mutation, a linked insertion mutation, an inverted duplication mutation, a tandem duplication (*e.g.,* an intrachromosomal tandem duplication), a translocation (*e.g.,* a chromosomal translocation, a non-reciprocal translocation), a rearrangement, a change in gene copy number, or a combination thereof. In certain embodiments, the subject interval (*e.g.,* subgenomic interval or expressed subgenomic interval) constitutes less than 5%, 1%, 0.5%, 0.1%, 0.01%, 0.001% of the coding region of the genome of the tumor cells in a sample. In other embodiments, the subject intervals (*e.g.,* subgenomic intervals, expressed subgenomic intervals, or both) are not involved in a disease, *e.g.,* are not associated with a cancerous phenotype as described herein.

In one embodiment, the target gene or gene product is a biomarker. As used herein, a "biomarker" or "marker" is a gene, mRNA, or protein which can be altered, wherein said alteration is associated with cancer. The alteration can be in amount, structure, and/or activity in a cancer tissue or cancer cell, as compared to its amount, structure, and/or activity, in a normal or healthy tissue or cell (*e.g.,* a control), and is associated with a disease state, such as cancer. For example, a marker associated with cancer, or predictive of responsiveness to anti-cancer therapeutics, can have an altered nucleotide sequence, amino acid sequence, chromosomal translocation, intra-chromosomal inversion, copy number, expression level, protein level, protein activity, epigenetic modification (*e.g.,* methylation or acetylation status, or post-translational modification, in a cancer tissue or cancer cell as compared to a normal, healthy tissue or cell. Furthermore, a "marker" includes a molecule whose structure is altered, *e.g.,* mutated (contains a mutation), *e.g.,* differs from the wild-type sequence at the nucleotide or amino acid level, *e.g.,* by substitution, deletion, or insertion, when present in a tissue or cell associated with a disease state, such as cancer.

In one embodiment, the target gene or gene product includes a single nucleotide polymorphism (SNP). In another embodiment, the gene or gene product has a small deletion, *e.g.,* a small intragenic deletion (*e.g.,* an in-frame or frame-shift deletion). In yet another embodiment, the target sequence results from the deletion of an entire gene. In still another embodiment, the target sequence has a small insertion, *e.g.,* a small intragenic insertion. In one embodiment, the target sequence results from an inversion, *e.g.,* an intrachromosal inversion. In another embodiment, the target sequence results from an interchromosal translocation. In yet another embodiment, the target sequence has a tandem duplication. In one embodiment, the target sequence has an undesirable feature (*e.g.,* high GC content or repeat element). In another embodiment, the target sequence has a portion of nucleotide sequence that cannot itself be successfully targeted, *e.g.,* because of its repetitive nature. In one embodiment, the target sequence results from alternative splicing. In another embodiment, the target sequence is chosen from a gene or gene product, or a fragment thereof according to **Tables 1-4 or FIGs. 3A-4D.**

In an embodiment, the target gene or gene product, or a fragment thereof, is an antibody gene or gene product, an immunoglobulin superfamily receptor (*e.g.,* B-cell receptor (BCR) or T-cell receptor (TCR)) gene or gene product, or a fragment thereof.

Human antibody molecules (and B cell receptors) are composed of heavy and light chains with both constant (C) and variable (V) regions that are encoded by genes on at least the following three loci.

1. Immunoglobulin heavy locus (IGH@) on chromosome 14, containing gene segments for the immunoglobulin heavy chain;
2. Immunoglobulin kappa (κ) locus (IGK@) on chromosome 2, containing gene segments for the immunoglobulin light chain;
3. Immunoglobulin lambda (λ) locus (IGL@) on chromosome 22, containing gene segments for the immunoglobulin light chain.

Each heavy chain and light chain gene contains multiple copies of three different types of gene segments for the variable regions of the antibody proteins. For example, the immunoglobulin heavy chain region can contain one of five different classes γ, δ, α, μ and ε, 44 Variable (V) gene segments, 27 Diversity (D) gene segments, and 6 Joining (J) gene segments. The light chains can also possess numerous V and J gene segments, but do not have D gene segments. The lambda light chain has 7 possible C regions and the kappa light chain has 1.

Immunoglobulin heavy locus (IGH@) is a region on human chromosome 14 that contains genes for the heavy chains of human antibodies (or immunoglobulins). For example, the IGH locus includes IGHV (variable), IGHD (diversity), IGHJ (joining), and IGHC (constant) genes. Exemplary genes encoding the immunoglobulin heavy chains include, but are not limited to IGHV1-2, IGHV1-3, IGHV1-8, IGHV1-12, IGHVI1-14, IGHV1-17, IGHV1-18, IGHV1-24, IGHV1-45, IGHV1-46, IGHV1-58, IGHV1-67, IGHV1-68, IGHV1-69, IGHV1-38-4, 1GHV1-69-2, IGHV2-5, IGHV2-10, IGHV2-26, IGHV2-70, IGHV3-6, IGHV3-7, IGHV3-9, IGHV3-11, IGHV3-13, IGHV3-15, IGHV3-16, IGHV3-19, IGHV3-20, IGHV3-21, IGHV3-22, IGHV3-23, IGHV3-25, IGHV3-29, IGHV3-30, IGHV3-30-2, IGHV3-30-3, IGHV3-30-5, IGHV3-32, IGHV3-33, IGHV3-33-2, IGHV3-35, IGHV3-36, IGHV3-37, IGHV3-38, IGHV3-41, IGHV3-42, IGHV3-43, IGHV3-47, IGHV3-48, IGHV3-49, IGHV3-50, IGHV3-52, IGHV3-53, IGHV3-54, IGHV3-57, IGHV3-60, IGHV3-62, IGHV3-63, IGHV3-64, IGHV3-65, IGHV3-66, IGHV3-71, IGHV3-72, IGHV3-73, IGHV3-74, IGHV3-75, IGHV3-76, IGHV3-79, IGHV3-38-3, IGHV3-69-1, IGHV4-4, IGHV4-28, IGHV4-30-1, IGHV4-30-2, IGHV4-30-4, IGHV4-31, IGHV4-34, IGHV4-39, IGHV4-55, IGHV4-59, IGHV4-61, IGHV4-80, IGHV4-38-2, IGHV5-51, IGHV5-78, IGHV5-10-1, IGHV6-1, IGHV7-4-1, IGHV7-27, IGHV7-34-1, IGHV7-40, IGHV7-56. IGHV7-81, IGHVII-1-1, IGHVII-15-1, IGHVII-20-1, IGHVII-22-1, IGHVII-26-2, IGHVII-28-1, IGHVII-30-1, IGHVII-31-1, IGHVII-33-1, IGHVII-40-1, IGHVII-43-1, IGHVII-44-2, IGHVII-46-1, IGHVII-49-1, IGHVII-51-2, IGHVII-53-1, IGHVII-60-1, IGHVII-62-1, IGHVII-65-1, IGHVII-67-1, IGHVII-74-1, IGHVII-78-1, IGHVIII-2-1, IGHVIII-5-1, IGHVIII-5-2, IGHVIII-11-1, IGHVIII-13-1, IGHVIII-16-1, IGHVIII-22-2, IGHVIII-25-1, IGHVIII-26-1, IGHVIII-38-1, IGHVIII-44, IGHVIII-47-1, IGHVIII-51-1, IGHVIII-67-2, IGHVIII-67-3, IGHVIII-67-4, IGHVIII-76-1, IGHVIII-82, IGHVIV-44-1, IGHD1-1, IGHD1-7, IGHD1-14, IGHD1-20, IGHD1-26, IGHD2-2, IGHD2-8, IGHD2-15, IGHD2-21, IGHD3-3, IGHD3-9, IGHD3-10, IGHD3-16, IGHD3-22, IGHD4-4, IGHD4-11, IGHD4-17, IGHD4-23, IGHD5-5, IGHD5-12, IGHD5-18, IGHD5-24, IGHD6-6, IGHD6-13, IGHD6-19, IGHD6-25, IGHD7-27, IGHJ1, IGHJ1P, IGHJ2, IGHJ2P, IGHJ3, IGHJ3P, IGHJ4, IGHJ5, IGHJ6, IGHA1, IGHA2, IGHG1, IGHG2, IGHG3, IGHG4, IGHGP, IGHD, IGHE, IGHEP1, IGHM, and IGHV1-69D.

Immunoglobulin kappa locus (IGK@) is a region on human chromosome 2 that contains genes for the kappa ($\kappa$) light chains of antibodies (or immunoglobulins). For example, the IGK locus includes IGKV (variable), IGKJ (joining), and IGKC (constant) genes. Exemplary genes encoding the immunoglobulin kappa light chains include, but are not limited to, 1GKV1-5, IGKV1-6, IGKV1-8, IGKVI-9, IGKVI-12, IGKV1-13, IGKV1-16, IGKV1-17, IGKV1-22, IGKV1-27, IGKV1-32, IGKV1-33, IGKV1-35, IGKV1-37, IGKV1-39, IGKV1D-8, IGKV1D-12, IGKV1D-13, IGKV1D-16 IGKV1D-17, IGKV1D-22, IGKV1D-27, IGKV1D-32, IGKV1D-33, IGKV1D-35, IGKV1U-37, IGKV1D-39, IGKV1D-42, IGKV1D-43, IGKV2-4, IGKV2-10, IGKV2-14, IGKV2-18, IGKV2-19, IGKV2-23, IGKV2-24, IGKV2-26, IGKV2-28, IGKV2-29, IGKV2-30, IGKV2-36, IGKV2-38, IGKV2-40, IGKV2D-10, IGKV2D-14, IGKV2D-18, IGKV2D-19, IGKV2D-23, IGKV2D-24, IGKV2D-26, IGKV2D-28, IGKV2D-29, IGKV2D-30, IGKV2D-36, IGKV2D-38, IGKV2D-40, IGKV3-7, IGKV3-11, IGKV3-15, IGKV3-20, IGKV3-25, IGKV3-31, IGKV3-34, IGKV3D-7, IGKV3D-11, IGKV3D-15, IGKV3D-20, IGKV3D-25, IGKV3D-31. IGKV3D-34, IGKV4-1, IGKV5-2, IGKV6-21, IGKV6D-21, IGKV6D-41, IGKV7-3, IGKJ1, IGKJ2, IGKJ3, IGKJ4, IGKJ5, and IGKC.

Immunoglobulin lambda locus (IGL@) is a region on human chromosome 22 that contains genes for the lambda light chains of antibody (or immunoglobulins). For example, the IGL locus includes IGLV (variable), IGLJ (joining), and IGLC (constant) genes. Exemplary genes encoding the immunoglobulin lambda light chains include, but are not limited to, IGLV1-36, IGLV1-40, IGLV1-41, IGLV1-44, IGLV1-47, IGLV1-50, IGLV1-51, IGLV1-62, IGLV2-5, IGLV2-8, IGLV2-11, IGLV2-14, IGLV2-18, IGLV2-23, IGLV2-28, IGLV2-33, IGLV2-34, IGLV3-1, IGLV3-2, IGLV3-4, IGLV3-6, IGLV3-7, IGLV3-9, IGLV3-10, IGLV3-12, IGLV3-13, IGLV3-15, IGLV3-16, IGLV3-17, IGLV3-19, IGLV3-21, 1GLV3-22, IGLV3-24, IGLV3-25, IGLV3-26, IGLV3-27, IGLV3-29, IGLV3-30, IGLV3-31, IGLV3-32, IGLV4-3, IGLV4-60, IGLV4-69, IGLV5-37, IGLV5-39, IGLV5-45, IGLV5-48, IGLV5-52, IGLV6-57, IGLV7-35, IGLV7-43, IGLV7-46, IGLV8-61, IGLV9-49, IGLV10-54, IGLV10-67, IGLV11-55, IGLVI-20, IGLVI-38, IGLVI-42, IGLVI-56, IGLVI-63, IGLVI-68, IGLVI-70, IGLVIV-53, IGLVIV-59, IGLVIV-64, IGLVIV-65, IGLVIV-66-1, IGLVV-58, IGLVV-66, IGLVVI-22-1, IGLVVI-25-1, IGLVVII-41-1, IGLJ1, IGLJ2, IGLJ3, IGLJ4, IGLJ5, IGLJ6, IGLJ7, IGLC1, IGLC2, IGLC3, IGLC4, IGLC5, IGLC6, and IGLC7.

The B-cell receptor (BCR) is composed of two parts: i) a membrane-bound immunoglobulin molecule of one isotype (*e.g.*, IgD or IgM). With the exception of the presence of an integral membrane domain, these can be identical to their secreted forms and ii) a signal transduction moiety: a heterodimer called Ig-$\alpha$/Ig-$\beta$ (CD79), bound together by disulfide bridges. Each member of the dimer spans the plasma membrane and has a cytoplasmic tail bearing an immunoreceptor tyrosine-based activation motif (ITAM).

The T-cell receptor (TCR) is composed of two different protein chains (*i.e.,* a heterodimer). In 95% of T cells, this consists of an alpha ($\alpha$) and beta ($\beta$) chain, whereas in 5% of T cells this consists of gamma ($\gamma$) and delta ($\delta$) chains. This ratio can change during ontogeny and in diseased states. The T cell receptor genes are similar to immunoglobulin genes in that they too contain multiple V, D and J gene segments in their beta and delta chains (and V and J gene segments in their alpha and gamma chains) that are rearranged during the development of the lymphocyte to provide each cell with a unique antigen receptor.

T-cell receptor alpha locus (TRA) is a region on human chromosome 14 that contains genes for the TCR alpha chains. For example, the TRA locus includes, *e.g.*, TRAV (variable), TRAJ (joining), and TRAC (constant) genes. Exemplary genes encoding the T-cell receptor alpha chains include, but are not limited to, TRAV1-1, TRAV1-2, TRAV2, TRAV3, TRAV4, TRAV5, TRAV6, TRAV7, TRAV8-1, TRAV8-2, TRAV8-3, TRAV8-4, TRAV8-5, TRAV8-6, TRAV8-7, TRAV9-1, TRAV9-2, TRAV10, TRAV11, TRAV12-1, TRAV12-2, TRAV12-3, TRAV13-1, TRAV13-2, TRAV14UV4, TRAV15, TRAV16, TRAV17, TRAV18, TRAV19, TRAV20, TRAV21, TRAV22, TRAV23DV6, TRAV24, TRAV25, TRAV26-1, TRAV26-2, TRAV27, TRAV28, TRAV29DV5, TRAV30, TRAV31, TRAV32, TRAV33, TRAV34, TRAV35, TRAV36DV7, TRAV37, TRAV38-1, TRAV38-2DV8, TRAV39, TRAV40, TRAV41, TRAJ1, TRAJ2, TRAJ3, TRAJ4, TRAJ5, TRAJ6, TRAJ7, TRAJ8, TRAJ9, TRAJ10, TRAJ11, TRAJ12, TRAJ13, TRAJ14, TRAJ15, TRAJ16, TRAJ17, TRAJ18, TRAJ19, TRAJ20, TRAJ21, TRAJ22, TRAJ23, TRAJ24, TRAJ25, TRAJ26, TRAJ27, TRAJ28, TRAJ29, TRAJ30, TRAJ31, TRAJ32, TRAJ33, TRAJ34, TRAJ35, TRAJ36, TRAJ37, TRAJ38, TRAJ39, TRAJ40, TRAJ41, TRAJ42, TRAJ43, TRAJ44, TRAJ45, TRAJ46, TRAJ47, TRAJ48, TRAJ49, TRAJ50, TRAJ51, TRAJ52, TRAJ53, TRAJ54, TRAJ55, TRAJ56, TRAJ57, TRAJ58, TRAJ59, TRAJ60, TRAJ61, and TRAC.

T-cell receptor beta locus (TRB) is a region on human chromosome 7 that contains genes for the TCR beta chains. For example, the TRB locus includes, *e.g.*, TRBV (variable), TRBD (diversity), TRBJ (joining), and TRBC (constant) genes. Exemplary genes encoding the T-cell receptor beta chains include, but are not limited to, TRBV1, TRBV2, TRBV3-1, TRBV3-2, TRBV4-1, TRBV4-2, TRBV4-3, TRBV5-1, TRBV5-2, TRBV5-3, TRBV5-4, TRBV5-5, TRBV5-6, TRBV5-7, TRBV6-2, TRBV6-3, TRBV6-4, TRBV6-5, TRBV6-6, TRBV6-7, TRBV6-8, TRBV6-9, TRBV7-1, TRBV7-2, TRBV7-3, TRBV7-4, TRBV7-5, TRBV7-6, TRBV7-7, TRBV7-8, TRBV7-9, TRBV8-1, TRBV8-2, TRBV9, TRBV10-1, TRBV10-2, TRBV10-3, TRBV11-1, TRBV11-2, TRBV11-3, TRBV12-1, TRBV12-2, TRBV12-3, TRBV12-4, TRBV12-5, TRBV13, TRBV14, TRBV15, TRBV16, TRBV17, TRBV18, TRBV19, TRBV20-1, TRBV21-1, TRBV22-1, TRBV23-1, TRBV24-1, TRBV25-1, TRBV26, TRBV27, TRBV28, TRBV29-1, TRBV30, TRBVA, TRBVB, TRBV5-8, TRBV6-1, TRBDI1, TRBD2, TRBJ1-1, TRBJ1-2, TRBJ1-3, TRBJ1-4, TRBJ1-5, TRBJ1-6, TRBJ2-1, TRBJ2-2, TRBJ2-2P, TRBJ2-3, TRBJ2-4, TRBJ2-5, TRBJ2-6, TRBJ2-7, TRBC1, and TRBC2.

T-cell receptor delta locus (TRD) is a region on human chromosome 14 that contains genes for the TCR delta chains. For example, the TRD locus includes, *e.g.,* TRDV (variable), TRDJ (joining), and TRDC (constant) genes. Exemplary genes encoding the T-cell receptor delta chains include, but are not limited to, TRDV1, TRDV2, TRDV3, TRDD1, TRDD2, TRDD3, TRDJ1, TRDJ2, TRDJ3, TRDJ4, and TRDC.

T-cell receptor gamma locus (TRG) is a region on human chromosome 7 that contains genes for the TCR gamma chains. For example, the TRG locus includes, e.g.. TRGV (variable), TRGJ (joining), and TRGC (constant) genes. Exemplary genes encoding the T-cell receptor gamma chains include, but are not limited to, TRGV1, TRGV2, TRGV3, TRGV4, TRGV5, TRGV5P, TRGV6, TRGV7, TRGV8, TRGV9, TRGV10, TRGV11, TRGVA, TRGVB, TRGJ1, TRGJ2, TRGJP, TRGJP1, TRGJP2, TRGC1, and TRGC2.

Exemplary cancers include, but are not limited to, B cell cancer, *e.g.,* multiple myeloma, melanomas, breast cancer, lung cancer (such as non-small cell lung carcinoma or NSCLC), bronchus cancer, colorectal cancer, prostate cancer, pancreatic cancer, stomach cancer, ovarian cancer, urinary bladder cancer, brain or central nervous system cancer, peripheral nervous system cancer, esophageal cancer, cervical cancer, uterine or endometrial cancer, cancer of the oral cavity or pharynx, liver cancer, kidney cancer, testicular cancer, biliary tract cancer, small bowel or appendix cancer, salivary gland cancer, thyroid gland cancer, adrenal gland cancer, osteosarcoma, chondrosarcoma, cancer of hematological tissues, adenocarcinomas, inflammatory myofibroblastic tumors, gastrointestinal stromal tumor (GIST), colon cancer, multiple myeloma (MM), myelodysplastic syndrome (MDS), myeloproliferative disorder (MPD), acute lymphocytic leukemia (ALL), acute myelocytic leukemia (AML), chronic myelocytic leukemia (CML), chronic lymphocytic leukemia (CLL), polycythemia Vera, Hodgkin lymphoma, non-Hodgkin lymphoma (NHL), soft-tissue sarcoma, fibrosarcoma, myxosarcoma, liposarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, squamous cell carcinoma, basal cell carcinoma, adenocarcinoma, sweat gland carcinoma, sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, seminoma, embryonal carcinoma, Wilms' tumor, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, neuroblastoma, retinoblastoma, follicular lymphoma, diffuse large B-cell lymphoma, mantle cell lymphoma, hepatocellular carcinoma, thyroid cancer, gastric cancer, head and neck cancer, small cell cancers, essential thrombocythemia, agnogenic myeloid metaplasia, hypereosinophilic syndrome, systemic mastocytosis, familiar hypereosinophilia, chronic eosinophilic leukemia, neuroendocrine cancers, carcinoid tumors, and the like.

Additional exemplary cancers are described in **Table 6**.

In an embodiment, the cancer is a hematologic malignancy (or premaligancy). As used herein, a hematologic malignancy refers to a tumor of the hematopoietic or lymphoid tissues, *e.g.,* a tumor that affects blood, bone marrow, or lymph nodes. Exemplary hematologic malignancies include, but are not limited to, leukemia (*e.g..* acute lymphoblastic leukemia (ALL), acute myeloid leukemia (AML), chronic lymphocytic leukemia (CLL), chronic myelogenous leukemia (CML), hairy cell leukemia, acute monocytic leukemia (AMoL), chronic myelomonocytic leukemia (CMML), juvenile myelomonocytic leukemia (JMML), or large granular lymphocytic leukemia), lymphoma (*e.g.,* AIDS-related lymphoma, cutaneous T-cell lymphoma, Hodgkin lymphoma (*e.g.,* classical Hodgkin lymphoma or nodular lymphocyte-predominant Hodgkin lymphoma), mycosis fungoides, non-Hodgkin lymphoma (*e.g.,* B-cell non-Hodgkin lymphoma (*e.g..* Burkitt lymphoma, small lymphocytic lymphoma (CLL/SLL), diffuse large B-cell lymphoma, follicular lymphoma, immunoblastic large cell lymphoma, precursor B-lymphoblastic lymphoma, or mantle cell lymphoma) or T-cell non-Hodgkin lymphoma (mycosis fungoides, anaplastic large cell lymphoma, or precursor T-lymphoblastic lymphoma)), primary central nervous system lymphoma, Sézary syndrome, Waldenström macroglobulinemia), chronic myeloproliferative neoplasm, Langerhans cell histiocytosis, multiple myeloma/plasma cell neoplasm, myelodysplastic syndrome, or myclodysplastic/myeloproliferative neoplasm. Premaligancy, as used herein, refers to a tissue that is not yet malignant but is poised to become malignant.

In one embodiment, the target gene or gene product, or a fragment thereof, is selected from any of the genes or gene products described in **Tables 1-4 or FIGs. 3A-4D.**

**Table 1**. Exemplary genes with complete exonic coverage in an exemplary DNA-seq baitset

| | | | | | | | | | |
|---|---|---|---|---|---|---|---|---|---|
| ABL1 | BTK | CTNNB1 | FAS (TNFRSF6) | HIST1H1C | KDR | MYCN | PDK1 | RPL13 | SUFU |
| ACTB | BTLA | CUL4A | FAT3 | HIST1H1D | KEAP1 | MYD88 | PHF6 | RPL15 | SUZ12 |
| AKT1 | c11orf30 (EMSY) | CUL4B | FBXO11 | HIST1H1E | KIT | MYO18A | PIK3C2G | RPL35A | SYK |
| AKT2 | CAD | CUX1 | FBXO31 | HIST1H2AC | KLHL6 | NBN | PIK3C3 | RPS14 | TAF1 |
| AKT3 | CARD11 | CXCR4 | FBXW7 | HIST1H2AG | KMT2A (MLL) | NCOR1 | PIK3CA | RPS19 | TBL1XR1 |
| ALK | CASP8 | CYP17A1 | FGF10 | HIST1H2AL | KMT2B (MLL2) | NCOR2 | PIK3CG | RPS26 | TBX3 |
| ALOX12B | CBFB | DAXX | FGF12 | HIST1H2AM | KMT2C (MLL3) | NCSTN | PIK3R1 | RPTOR | TCF3 |
| AMER1 (FAM123B or WTX) | CBL | DDR2 | FGF14 | HIST1H2BC | KRAS | NF1 | PIK3R2 | RUNX1 | TCL1A |
| APC | CCND1 | DDX3X | FGF19 | HIST1H2BJ | LEF1 | NF2 | PIM1 | RUNX1T1 | TET2 |
| APCDD1 | CCND2 | DIS3 | FGF23 | HIST1H2BK | LMO1 | NFE2L2 | PLCG2 | S1PR2 | TGFBR2 |
| APH1A | CCND3 | DKC1 | FGF3 | HIST1H2BO | LRP1B | NFKBIA | PMS2 | SBDS | TIPARP |
| AR | CCNE1 | DNM2 | FGF4 | HIST1H3B | LRRK2 | NKX2-1 | PNRC1 | SDHA | TLL2 |
| ARAF | CCT6B | DNMT3A | FGF6 | HLA-A | MAF | NOD1 | POT1 | SDHB | TMEM30A |
| ARFRP1 | CD22 | DOT1L | FGF7 | HNF1A | MAFB | NOTCH1 | PPP2R1A | SDHC | TMSB4XP8 (TMSL3) |
| ARHGAP26 (GRAF) | CD274 (PDL1) | DTX1 | FGFR1 | HRAS | MAGED1 | NOTCH2 | PRDM1 | SDHD | TNFAIP3 |
| ARID1A | CD36 | DUSP2 | FGFR2 | HSP90AA1 | MALT1 | NOTCH3 | PRKAR1A | SERP2 | TNFRSF11A |
| ARID2 | CD58 | DUSP9 | FGFR3 | ICK | MAP2K1 | NOTCH4 | PRKDC | SETBP1 | TNFRSF14 |
| ASMTL | CD70 | EBF1 | FGFR4 | ID3 | MAP2K2 | NPM1 | PRSS8 | SETD2 | TNFRSF17 |
| ASXL1 | CD79A | ECT2L | FHIT | IDH1 | MAP2K4 | NRAS | PTCH1 | SF3B1 | TOP1 |
| ATM | CD79B | EED | FLCN | IDH2 | MAP3K1 | NSD1 | PTEN | SGK1 | TP53 |
| ATR | CDC73 | EGFR | FLT1 | IGF1 | MAP3K13 | NT5C2 | PTPN11 | SH2B3 | TP63 |

(continued)

| | | | | | | | | | |
|---|---|---|---|---|---|---|---|---|---|
| ATRX | CDH1 | ELP2 | FLT3 | IGF1R | MAP3KI4 | NTRK1 | PTPN2 | SMAD2 | TRAF2 |
| AURKA | CDK12 | EP300 | FLT4 | IGF2 | MAP3K6 | NTRK2 | PTPN6 (SHP-1) | SMAD4 | TRAF3 |
| AURKB | CDK4 | EPHA3 | FLYWCH1 | IKBKE | MAP3K7 | NTRK3 | PTPRO | SMARCA1 | TRAF5 |
| AXIN1 | CDK6 | EPHA5 | FOXL2 | IKZF1 | MAPK1 | NUP93 | RAD21 | SMARCA4 | TRRAP |
| AXL | CDK8 | EPHA7 | FOXO1 | IKZF2 | MCL1 | NUP98 | RAD50 | SMARCB1 | TSC1 |
| B2M | CDKN1B | EPHB1 | FOXO3 | IKZF3 | MDM2 | P2RY8 | RAD51 | SMARCD1 | TSC2 |
| BAP1 | CDKN2A | ERBB2 | FOXP1 | IL7R | MDM4 | PAG1 | RAD51B | SMC1A | TSHR |
| BARD1 | CDKN2B | ERBB3 | FRS2 | INHBA | MED12 | PAK3 | RAD51C | SMC3 | TUSC3 |
| BCL10 | CDKN2C | ERBB4 | GADD45B | INPP4B | MEF2B | PAK7 | RAD51D | SMO | TYK2 |
| BCL11B | CEBPA | ERG | GATA1 | INPP5D (SHIP) | MEF2C | PALB2 | RAD52 | SOCS1 | U2AF1 |
| BCL2 | CHD2 | ESR1 | GATA2 | IRF1 | MEN1 | PARP1 | RAD54L | SOCS2 | U2AF2 |
| BCL2L2 | CHEK1 | ETS1 | GATA3 | IRF4 | MET | PARP2 | RAF1 | SOCS3 | VHL |
| BCL6 | CHEK2 | ETV6 | GID4 (c17orf39) | IRF8 | MIB1 | PARP3 | RARA | SOX10 | WDR90 |
| BCL7A | CHUK | EXOSC6 | GNA11 | IRS2 | MITF | PARP4 | RASGEF1A | SOX2 | WHSC1 (MMSET or NSD2) |
| BCOR | CIC | EZH2 | GNA12 | JAK1 | MKI67 | PASK | RB1 | SPEN | WISP3 |
| BCORL1 | CIITA | FAF1 | GNA13 | JAK2 | MLH1 | PAX5 | REL | SPOP | WT1 |
| BIRC3 | CKS1B | FAM46C | GNAQ | JAK3 | MPL | PBRM1 | RELN | SRC | XBP1 |
| BLM | CPS1 | FANCA | GNAS | JARID2 | MRE11A | PC | RET | SRSF2 | XPO1 |
| BRAF | CRBN | FANCC | GPR124 | JUN | MSH2 | PCBP1 | RHOA | STAG2 | XRCC3 |
| BRCA1 | CREBBP | FANCD2 | GRIN2A | KAT6A (MYST3) | MSH3 | PCLO | RICTOR | STAT3 | YY1AP1 |
| BRCA2 | CRKL | FANCE | GSK3B | KDM2B | MSH6 | PDCD1 | RMRP | STAT4 | ZMYM3 |
| BRD4 | CRLF2 | FANCF | GTSE1 | KDM4C | MTOR | PDCD11 | RNF43 | STAT5A | ZNF217 |
| BRIP1 (BACH1) | CSF1R | FANCG | HDAC1 | KDM5A | MUTYH | PDCU1LG2 (PDL2) | ROS1 | STAT5B | ZNF24 (ZSCAN3) |

(continued)

| BRSK1 | CSF3R | FANCI | HDAC4 | KDM5C | MYC | PDGFRA | RPA1 | STAT6 | ZNF703 |
|---|---|---|---|---|---|---|---|---|---|
| BTG1 | CTCF | FANCL | HDAC7 | KDM6A | MYCL (MYCL1) | PDGFRB | RPL11 | STK11 | ZRSR2 |
| BTG2 | CTNNA1 | FANCM | HGF | | | | | | |

**Table 2.** Exemplary genes with select introns covered in an exemplary DNA-seq baitset

| ALK | BRAF | EGFR | ETV4 | EWSR1 | IGK | JAK2 | NTRK1 | RAF1 | ROS1 |
|------|-------|------|------|-------|------|-------------|--------|------|---------|
| BCL2 | CCND1 | EPOR | ETV5 | FGFR2 | IGL | KMT2A (MLL) | PDGFRA | RARA | TMPRSS2 |
| BCL6 | CRLF2 | ETV1 | ETV6 | IGH | JAK1 | MYC | PDGFRB | RET | TRG |
| BCR | | | | | | | | | |

**Table 3.** Exemplary genes targeted in an exemplary RNA-seq baitset

| | | | | | | | | | |
|---|---|---|---|---|---|---|---|---|---|
| ABI1 | BRCA2 | CTNNB1 | FGFR3 | IGH | MDS2 | NKX2-1 | PIK3R1 | RPS14 | TET1 |
| ABL1 | BTG1 | DDIT3 | FLI1 | IGK | MECOM | NOTCH1 | PIK3R2 | RPS15 | TFE3 |
| ABL2 | CAMTA1 | DDR1 | FNBP1 | IGL | MEF2C | NPM1 | PIM1 | RPS19 | TFG |
| ACSL6 | CARS | DDX10 | FOXO1 | IKZF1 | MKL1 | NR4A3 | PLAG1 | RPS26 | TFPT |
| AFF1 | CBFA2T3 | DDX6 | FOXO3 | IKZF3 | MKL2 | NSD1 | PML | RUNX1 | TFRC |
| AFF4 | CBFB | DEK | FOX04 | IL21R | MLF1 | NTRK1 | POU2AF1 | RUNX1T1 (ETO) | TLX1 |
| ALK | CBL | DLEU2 | FOXP1 | IL3 | MLLT1 (ENL) | NTRK2 | PPP1CB | RUNX2 | TLX3 |
| ARHGAP26 | CCND1 | DNMT3A | FSTL3 | INSR | MLLT10 (AF10) | NTRK3 | PRDM1 | SEC31A | TMPRSS2 |
| ARHGEF12 | CCND2 | DUSP22 | FUS | IRF4 | MLLT3 | NUMA1 | PRDM16 | SEPT5 | TNFRSF11A |
| ARID1A | CCND3 | EGFR | GAS7 | ITK | MLLT4 (AF6) | NUP214 | PRRX1 | SEPT6 | TNFSF9 |
| ARID1B | CD247 | EIF4A2 | GLI1 | JAK1 | MLLT6 | NUP98 | PSIP1 | SEPT9 | TOP1 |
| ARNT | CD274 (PDL1) | ELF4 | GLIS2 | JAK2 | MN1 | NUTM2A | PTCH1 | SET | TP53 |
| ASXL1 | CD70 | ELL | GMPS | JAK3 | MNX1 | OLIG2 | PTEN | SH3GL1 | TP63 |
| ATF1 | CDC73 | ELN | GPHN | JAZF1 | MSH2 | OMD | PTK7 | SLC1A2 | TPM3 |
| ATG5 | CDK6 | EMLA4 | HDAC4 | KAT6A (MYST3) | MSH6 | P2RY8 | RABEP1 | SMARCB1 | TPM4 |
| ATIC | CDKN2A | EP300 | HERPUD1 | KDM4C | MSI2 | PAFAH1B2 | RAF1 | SNX29 (RUNDC2A) | TRAF2 |
| ATM | CDX2 | EPHA7 | HEY1 | KDSR | MSN | PALB2 | RALGDS | SRSF3 | TRAF3 |
| ATR | CEBPA | EPOR | HIP1 | KIF5B | MTAP | PAX3 | RANBP17 | SS18 | TRAF5 |
| ATXN1 | CHIC2 | EPS15 | HIST1H1A | KMT2A (MLL) | MTCP1 | PAX5 | RAP1GDS1 | SSX1 | TRG |
| AXL | CHN1 | ERBB2 | HIST1H4I | LASP1 | MUC1 | PAX7 | RARA | SSX2 | TRIM24 |
| BAP1 | CHTOP (C1orf77) | ERG | HLF | LCK | MYB | PBX1 | RB1 | SSX4 | TRIP11 |
| BCL10 | CIC | ETS1 | HMGA1 | LCP1 | MYC | PCM1 | RBM15 | STAT6 | TSC1 |
| BCL11A | CIITA | ETV1 | HMGA2 | LEF1 | MYH11 | PCSK7 | RCOR1 | STK11 | TSC2 |
| BCL11B | CKS1B | ETV4 | HOXA11 | LMO1 | MYH9 | POCD1LG2 (PDL2) | RET | STL | TTL |

(continued)

| BCL2 | CLP1 | ETV5 | HOXA13 | LMO2 | NACA | PDE4DIP | RHOH | SYK | TYK2 |
|---|---|---|---|---|---|---|---|---|---|
| BCL3 | CLTC | ETV6 | HOXA3 | LPP | NBEAP1 (BCL8) | PDGFB | RNF213 | TAF15 | USP6 |
| BCL6 | CLTCL1 | EWSR1 | HOXA9 | LTK | NCOA2 | PDGFRA | ROS1 | TAL1 | WHSC1 |
| BCL7A | CNTRL (CEP110) | FBXW7 | HOXC11 | LYL1 | NDRG1 | PDGFRB | RPA1 | TAL2 | WHSC1L1 |
| BCL9 | COL1A1 | FCGR2B | HOXC13 | MAF | NF1 | PDK1 | RPL13 | TBL1XR1 | YPEL5 |
| BCOR | CREB3L1 | FCRL4 | HOXD11 | MAFB | NF2 | PER1 | RPL15 | TCF3 (E2A) | ZBTB16 |
| BCR | CREB3L2 | FEV | HOXD13 | MAGEA5 | NFKB2 | PGAM5 | RPL22 | TCL1A (TCL1) | ZMYM2 |
| BIRC3 | CREBBP | FGFR1 | HSP90AA1 | MALT1 | NFKBIE | PHF1 | RPL35A | TCL6 | ZNF384 |
| BRAF | CRLF2 | FGFR1OP | HSP90AB1 | MAP3K7 | NIN | PICALM | RPN1 | TEC | ZNF521 |
| BRCA1 | CSF1 | FGFR2 | | | | | | | |

**Table 4.** Additional exemplary genes with complete exonic coverage in an exemplary DNA-seq baitset

| | | | | | | | | | |
|---|---|---|---|---|---|---|---|---|---|
| ASMTL | CKS1B | EXOSC6 | GNA12 | IKZF2 | MIB1 | PDCD1 | RPL15 | SDHC | TCL1A |
| AXIN1 | CPS1 | FAF1 | GTSE1 | IKZF3 | MKI67 | PDCD11 | RPL35A | SDHD | TLL2 |
| BRD4 | CSF3R | FBXO11 | HDAC1 | INPP4B | MSH3 | PLCG2 | RPS14 | SERP2 | TMEM30A |
| BRSK1 | CXCR4 | FBXO31 | HIST1H1D | KMT2C (MLL3) | MYO18A | POT1 | RPS19 | SOCS2 | TP63 |
| BTLA | DDX3X | FHIT | HIST1H2AC | MAF | NCOR2 | RASGEF1A | RPS26 | SOCS3 | TUSC3 |
| CAD | DKC1 | FLYWCH1 | HIST1H2AM | MAFB | NOD1 | RHOA | S1PR2 | STAT5A | WDR90 |
| CCT6B | EBF1 | FOXP1 | HIST1H2BJ | MALT1 | PASK | RMRP | SBDS | STAT5B | WHSC1 (MMSET or NSD2) |
| CD36 | ELP2 | FRS2 | HNF1A | MAP3K6 | PC | RPL11 | SDHA | TAF1 | YY1AP1 |
| CHD2 | ETS1 | GADD45B | ICK | MAPK1 | PCBP1 | RPL13 | SDHB | TBL1XR1 | ZNF24 (ZSCAN3) |
| CALR | | | | | | | | | |

Additional exemplary genes are shown in **FIGs. 3A-4D.**

In one embodiment, the target gene or gene product, or a fragment thereof, has one or more of substitutions, indels, or copy number alterations that are associated with cancer, *e.g.,* a hematologic malignancy (or premaligancy). Exemplary genes or gene products include, but are not limited to, ABL1, ACTB, AKT1, AKT2, AKT3, ALK, AMER1 (FAM123B or WTX), APC, APH1A, AR, ARAF, ARFRP1, ARHGAP26 (GRAF) ARID1A, ARID2, ASMTL. ASXL1, ATM, ATR, ATRX. AURKA, AURKB. AXIN1, AXL, B2M, BAP1, BARD1, BCL10, BCL11B, BCL2. BCL2L2, BCL6, BCL7A, BCOR, BCORL1, BIRC3, BLM, BRAF, BRCA1, BRCA2, BRD4, BRIP1 (BACH1), BRSK1, BTG2, BTK, BTLA, c11 or, f30 (EMSY), CAD, CARD11, CBFB, CBL, CCND1, CCND2, CCND3, CCNE1, CCT6B, CD22, CD274, (PDL 1). CD36, CD58. CD70, CD79A, CD79B, CDC73. CDH1, CDK12, CDK4, CDK6, CDK8, CDKN1B, CDKN2A, CDKN2B, CDKN2C, CEBPA, CHD2, CHEK1, CHEK2, CIC, CIITA, CKSIB, CPS1, CREBBP, CRKL, CRLF2, CSFIR, CSF3R, CTCF, CTNNA1, CTNNB1, CUX1, CXCR4, DAXX, DDR2, DDX3X, DNM2, DNMT3A, DOT1L, DTX1, DUSP2, DUSP9. EBF1, ECT2L, EED, EGFR, ELP2, EP300, EPHA3, EPHA5, EPHA7, EPHBI, ERBB2, ERBB3, ERBB4, ERG, ESR1, ETS1, ETV6. EXOSC6, EZH2, FAF1, FAM46C, FANCA, FANCC, FANCD2, FANCE, FANCF, FANCG, FANCL. FAS (TNFRSF6), FBXO11, FBXO31, FBXW7, FGF10, FGF14, FGF19, FGF23, FGF3, FGF4, FGF6, FGFR1, FGFR2, FGFR3, FGFR4, FHIT, FLCN, FLT1, FLT3, FLT4, FLYWCH1, FOXL2, FOXO1, FOXO3, FOXY1, FRS2, GADD45B, GATA1, GATA2, GATA3, GID4 (CI7orf39), GNA11, GNA12, GNA13, GNAQ. GNAS, GPR124, GRIN2A, GSK3B, GTSE1, HDAC1, HDAC4, HDAC7, HGF, HIST1H1C, HIST1H1D, HIST1H1E, HIST1H2AC, HIST1H2AG, HIST1H2AL, HIST1H2AM, HIST1H2BC. HIST1H2BJ, HIST1H2BK, HIST1H2BO, HIST1H3B, HNF1A, HRAS, HSP90AA1, ICK, ID3, IDH1, IDH2, IGF1R, IKBKE, IKZF1, IKZF2, IKZF3, IL7R, INHBA, INPP4B, INPP5D (SHIP). IRF1, IRF4, IRF8, IRS2, JAK1, JAK2. JAK3, JARID2, JUN, KAT6A (MYST3), KDM2B, KDM4C, KDM5A. KDM5C. KDM6A, KDR, KEAP1, KIT, KLHL6, KMT2A (MLL), KMT2B (MLL2), KMT2C (MLL3), KRAS. LEF1, LRP1B, LRRK2. MAF, MAFB, MAGED1, MALT1, MAP2K1, MAP2K2, MAP2K4, MAP3K1, MAP3K14, MAP3K6, MAP3K7, MAPK1, MCL1, MDM2, MDM4, MED12, MEF2B, MEF2C, MEN1, MET, MIB1, MITF. MKI67, MLH1, MPL, MRE11A, MSH2, MSH3, MSH6. MTOR, MUTYH, MYC, MYCL (MYCL1), MYCN, MYD88, MYO18A, NCOR2, NCSTN, NF1, NF2, NFE2L2, NFKBIA, NKX2-1, NOD1, NOTCH1, NOTCH2, NPM1, NRAS, NT5C2, NTRK1, NTRK2, NTRK3, NUP93, NUP98, P2RY8, PAG1, PAK3, PALB2, PASK, PAX5, PBRM1, PC, PCBP1, PCLO, PDCD1, PDCD11, PDCD1LG2 (PDL2), PDGFRA, PDGFRB, PDK1, PHF6, PIK3CA, PIK3CG. PIK3R1, PIK3R2, PIM1, PLCG2, POT1, PPP2R1A, PRDM1, PRKAR1A, PRKDC. PRSS8. PTCH1, PTEN, PTPN11, PTPN2, PTPN6 (SHP-1), PTPRO. RAD21, RAD50, RAD51. RAF1, RARA, RASGEF1A, RB1, RELN, RET. RHOA, RICTOR. RNF43, ROS1, RPTOR. RUNX1, S1PR2, SDHA, SDHB. SDHC. SDHD, SERP2, SETBP1, SETD2. SF3B1, SGK1. SMAD2, SMAD4, SMARCA1, SMARCA4, SMARCB1, SMC1A, SMC3, SMO. SOCS1, SOCS2, SOCS3, SOX10, SOX2, SPEN, SPOP, SRC, SRSF2, STAG2, STAT3, STAT4, STAT5A, STAT5B, STAT6, STK11, SUFU, SUZ12, TAF1, TBL1XR1, TCF3, TCL1A, TET2, TGFBR2, TLL2, TMEM30A, TMSB4XP8 (TMSL3). TNFAIP3, TNFRSF11A, TNFRSF14, TNFRSF17, TOP1, TP53, TP63, TRAF2, TRAF3, TRAF5, TSC1, TSC2, TSHR, TUSC3, TYK2, U2AF1, U2AF2, VHL, WDR90, WHSC1 (MMSET, or, NSD2), WISP3, WT1, XBP1, XPO1, YY1AP1, ZMYM3, ZNF217, ZNF24 (ZSCAN3), ZNF703, or ZRSR2.

In one embodiment, the target gene or gene product, or a fragment thereof, has one or more rearrangements that are associated with cancer, *e.g..* a hematologic malignancy (or premaligancy). Exemplary genes or gene products include, but are not limited to, ALK, BCL6, BRAF, CRLF2, EPOR, ETV4, ETV6, FGFR2, IGK, BCL2, BCR, CCND1, EGFR, ETV1, ETV5, EWSR1, IGH, IGL, JAK1, KMT2A, (MLL), NTRK1, PDGFRB. RARA, ROS1, TRG. JAK2, MYC, PDGFRA, RAF1, RET, or TMPRSS2.

In another embodiment, the target gene or gene product, or a fragment thereof, has one or more fusions that are associated with cancer. Exemplary genes or gene products include, but are not limited to, ABII, CBFA2T3, EIF4A2, FUS. JAK1, MUC1, PBX1, RNF213, TET1, ABL1, CBFB, ELF4, GAS7, JAK2, MYB, PCM1, ROS1, TFE3, ABL2, CBL, ELL, GLI1, JAK3, MYC, PCSK7, RPL22, TFG, ACSL6, CCND1, ELN, GMPS, JAZF1, MYH11, PDCD1LG2 (PDL2), RPN1, TFPT, AFF1, CCND2. EML4, GPHN, KAT6A (MYST3), MYH9, PDE4DIP, RUNX1, TFRC, AFF4, CCND3, EP300, HERPUD1, KDSR. NACA, PDGFB, RUNX1T1 (ETO), TLX1, ALK, CD274 (PDL1), EPOR, HEY1, KIF5B, NBEAP1 (BCLS), PDGFRA, RUNX2, TLX3, ARHGAP26 (GRAF), CDK6, EPS15, HIP1, KMT2A (MLL), NCOA2. PDGFRB, SEC31A, TMPRSS2, ARHGEF12, CDX2, ERBB2, HIST1H4I, LASP1, NDRG1, PER1, SEPT5. TNFRSF11A, ARID1A, CHIC2, ERG, HLF. LCP1, NF1, PHF1, SEPT6, TOP1, ARNT, CHN1, ETS1, HMGA1. LMO1, NF2, PICALM. SEPT9, TP63, ASXL1, CIC, ETV1, HMGA2, LMO2, NFKB2, PIM1, SET, TPM3, ATF1, CIITA, ETV4, HOXA11, LPP, NIN, PLAG1, SH3GL1, TPM4. ATG5, CLP1, ETV5, HOXA13, LYL1, NOTCH1, PML, SLC1A2, TRIM24, ATIC, CLTC, ETV6. HOXA3, MAF, NPM1, POU2AF1, SNX29 (RUNDC2A), TRIP11, BCL10, CLTCL1, EWSR1. HOXA9, MAFB, NR4A3, PPP1CB, SRSF3, TTL, BCL11A, CNTRL (CEP110), FCGR2B, HOXC11, MALT1, NSDI1, PRDM1, SS18, TYK2, BCL11B, COL1A1, FCRL4, HOXC13, MDS2, NTRK1, PRDM16, SSX1, USP6, BCL2, CREB3L1, FEV, HOXD11, MECOM, NTRK2, PRRX1, SSX2. WHSC1 (MMSET, or, NSD2), BCL3, CREB3L2, FGFR1, HOXD13, MKL1, NTRK3, PSIP1, SSX4, WHSC1L1, BCL6. CREBBP, FGFR1OP, HSP90AA1, MLF1, NUMA1, PTCH1, STAT6, YPEL5, BCL7A. CRLF2, FGFR2, HSP90AB1. MLLT1 (ENL), NUP214, PTK7, STL, ZBTB16, BCL9, CSFI, FGFR3, IGH, MLLT10 (AF10), NUP98, RABEP1, SYK, ZMYM2, BCOR, CTNNB1, FLI1, IGK, MLLT3. NUTM2A, RAF1, TAF15, ZNF384, BCR, DDIT3, FNBP1, IGL, MLLT4, (AF6), OMD, RALGDS, TAL1, ZNF521, BIRC3, DDX10, FOXO1, IKZF1, MLLT6, P2RY8, RAP1GDSI1, TAL2, BRAF. DDX6, FOXO3, IL21R, MN1, PAFAH1B2, RARA, TBL1XR1, BTG1, DEK, FOXO4, IL3, MNX1, PAX3, RBM15, TCF3 (E2A), CAMTA1, DUSP22, FOXP1, IRF4, MSI2, PAX5, RET, TCL1A (TCL1), CARS, EGFR, FSTL3, ITK, MSN, PAX7, RHOH, or TEC.

Additional exemplary genes are described, *e.g.*, in Tables 1-11 of International Application Publication No. WO2012/092426, the content of which is incorporated by reference in its entirety.

Applications of the foregoing methods include using a library of oligonucleotides containing all known sequence variants (or a subset thereof) of a particular gene or genes for sequencing in medical specimens.

In certain embodiments, the method or assay further includes one or more of:

(i) fingerprinting the nucleic acid sample;
(ii) quantifying the abundance of a gene or gene product (*e.g.,* a gene or gene product as described herein) in the nucleic acid sample;
(iii) quantifying the relative abundance of a transcript in the sample;
(iv) identifying the nucleic acid sample as belonging to a particular subject (*e.g.,* a normal control or a cancer patient);
(v) identifying a genetic trait in the nucleic acid sample (*e.g.* one or more subject's genetic make-up (*e.g.*, ethnicity, race, familial traits));
(vi) determining the ploidy in the nucleic acid sample; determining a loss of heterozygosity in the nucleic acid sample;
(vii) determining the presence or absence of a gene duplication event in the nucleic acid sample;
(viii) determining the presence or absence of a gene amplification event in the nucleic acid sample; or
(ix) determining the level of tumor/normal cellular admixture in the nucleic acid sample.

**NUCLEIC ACID SAMPLES**

A variety of tissue samples can be the source of the nucleic acid samples used in the present methods. Genomic or subgenomic nucleic acid (*e.g.,* DNA or RNA) can be isolated from a subject's sample (*e.g.*, a tumor sample, a normal adjacent tissue (NAT), a blood sample), a sample containing circulating tumor cells (CTC) or any normal control). In certain embodiments, the tissue sample is preserved as a frozen sample or as formaldehyde- or paraformaldehyde-fixed paraffin-embedded (FFPE) tissue preparation. For example, the sample can be embedded in a matrix, *e.g.*, an FFPE block or a frozen sample. In certain embodiments, the tissue sample is a blood sample. In other embodiments, the tissue sample is a bone marrow aspirate (BMA) sample. The isolating step can include flow-sorting of individual chromosomes; and/or micro-dissecting a subject's sample (*e.g.,* a tumor sample, a NAT, a blood sample).

An "isolated" nucleic acid molecule is one which is separated from other nucleic acid molecules which are present in the natural source of the nucleic acid molecule. In certain embodiments, an "isolated" nucleic acid molecule is free of sequences (such as protein-encoding sequences) which naturally flank the nucleic acid (*i.e.,* sequences located at the 5' and 3' ends of the nucleic acid) in the genomic DNA of the organism from which the nucleic acid is derived. For example, in various embodiments, the isolated nucleic acid molecule can contain less than about 5 kB, less than about 4 kB, less than about 3 kB, less than about 2 kB, less than about 1 kB, less than about 0.5 kB or less than about 0.1 kB of nucleotide sequences which naturally flank the nucleic acid molecule in genomic DNA of the cell from which the nucleic acid is derived. Moreover, an "isolated" nucleic acid molecule, such as an RNA molecule or a cDNA molecule, can be substantially free of other cellular material or culture medium, *e.g.* when produced by recombinant techniques, or substantially free of chemical precursors or other chemicals, *e.g.*, when chemically synthesized.

The language "substantially free of other cellular material or culture medium" includes preparations of nucleic acid molecule in which the molecule is separated from cellular components of the cells from which it is isolated or recombinantly produced. Thus, nucleic acid molecule that is substantially free of cellular material includes preparations of nucleic acid molecule having less than about 30%, less than about 20%, less than about 10%, or less than about 5% (by dry weight) of other cellular material or culture medium.

In certain embodiments, the nucleic acid is isolated from an aged sample, *e.g.,* an aged FFPE sample. The aged sample, can be, for example, years old, *e.g.,* 1 year, 2 years, 3 years. 4 years, 5 years, 10 years, 15 years, 20 years. 25 years. 50 years. 75 years. or 100 years old or older.

A nucleic acid sample can be obtained from tissue samples (*e.g.,* a biopsy, a FFPE sample, a blood sample, or a bone marrow aspirate sample) of various sizes. For example, the nucleic acid can be isolated from a tissue sample from 5 to 200 $\mu$m, or larger. For example, the tissue sample can measure 5 $\mu$m, 10 $\mu$m, 20 $\mu$m, 30 $\mu$m, 40 $\mu$m, 50 $\mu$m, 70 $\mu$m, 100 $\mu$m, 110 $\mu$m, 120 $\mu$m, 150 $\mu$m or 200 $\mu$m or larger.

Protocols for DNA isolation from a tissue sample are known in the art, *e.g.,* as provided in Example 1 of International Patent Application Publication No. WO 2012/092426. Additional methods to isolate nucleic acids (*e.g.*, DNA) from formaldehyde- or paraformaldehyde-fixed, paraffin-embedded (FFPE) tissues are disclosed, *e.g.,* in Cronin M. et al., (2004) Am J Pathol. 164(1):35-42; Masuda N. et al., (1999) Nucleic Acids Res. 27(22):44364443; Specht K. et al., (2001) Am J Pathol. 158(2):419-429, Ambion RecoverAll™ Total Nucleic Acid Isolation Protocol (Ambion, Cat. No. AM1975, September 2008), Maxwell® 16 FFPE Plus LEV DNA Purification Kit Technical Manual (Promega Literature #TM349. February 2011), E.Z.N.A.® FFPE DNA Kit Handbook (OMEGA bio-tek, Norcross, GA, product numbers D3399-00, D3399-01. and D3399-02; June 2009), and QIAamp® DNA FFPE Tissue Handbook (Qiagen, Cat. No. 37625, October 2007). RecoverAll™ Total Nucleic Acid Isolation Kit uses xylene at elevated temperatures to solubilize paraffin-embedded samples and a glass-fiber filter to capture nucleic acids. Maxwell® 16 FFPE Plus LEV DNA Purification Kit is used with the Maxwell® 16 Instrument for purification of genomic DNA from 1 to 10 μm sections of FFPE tissue. DNA is purified using silica-clad paramagnetic particles (PMPs), and eluted in low elution volume. The E.Z.N.A.® FFPE DNA Kit uses a spin column and buffer system for isolation of genomic DNA. QIAamp® DNA FFPE Tissue Kit uses QIAamp® DNA Micro technology for purification of genomic and mitochondrial DNA. Protocols for DNA isolation from blood are disclosed, *e.g.,* in the Maxwell® 16 LEV Blood DNA Kit and Maxwell 16 Buccal Swab LEV DNA Purification Kit Technical Manual (Promega Literature #TM333, January 1, 2011).

Protocols for RNA isolation are disclosed, *e.g.*, in the Maxwell® 16 Total RNA Purification Kit Technical Bulletin (Promega Literature #TB351, August 2009).

The isolated nucleic acid samples (*e.g.*, genomic DNA samples) can be fragmented or sheared by practicing routine techniques. For example, genomic DNA can be fragmented by physical shearing methods, enzymatic cleavage methods, chemical cleavage methods, and other methods well known to those skilled in the art. The nucleic acid library can contain all or substantially all of the complexity of the genome. The term "substantially all" in this context refers to the possibility that there can in practice be some unwanted loss of genome complexity during the initial steps of the procedure. The methods described herein also are useful in cases where the nucleic acid library is a portion of the genome, *i.e.,* where the complexity of the genome is reduced by design. In some embodiments, any selected portion of the genome can be used with the methods described herein. In certain embodiments, the entire exome or a subset thereof is isolated.

Methods featured in the invention can further include isolating a nucleic acid sample to provide a library (*e.g.,* a nucleic acid library as described herein). In certain embodiments, the nucleic acid sample includes whole genomic, subgenomic fragments, or both. The isolated nucleic acid samples can be used to prepare nucleic acid libraries. Thus, in one embodiment, the methods featured in the invention further include isolating a nucleic acid sample to provide a library (e.g., a nucleic acid library as described herein). Protocols for isolating and preparing libraries from whole genomic or subgenomic fragments are known in the art (e.g., Illumina's genomic DNA sample preparation kit). In certain embodiments, the genomic or subgenomic DNA fragment is isolated from a subject's sample *(e.g.,* a tumor sample, a normal adjacent tissue (NAT), a blood sample or any normal control)). In one embodiment, the sample *(e.g.,* the tumor or NAT sample) is a preserved specimen. For example, the sample is embedded in a matrix, e.g., an FFPE block or a frozen sample. In certain embodiments, the isolating step includes flow-sorting of individual chromosomes; and/or microdissecting a subject's sample (e.g., a tumor sample, a NAT, a blood sample). In certain embodiments, the nucleic acid sample used to generate the nucleic acid library is less than 5 microgram, less than 1 microgram, or less than 500ng, less than 200ng, less than 100ng, less than 50ng, less than 10ng, less than 5 ng, or less than 1 ng.

In still other embodiments, the nucleic acid sample used to generate the library includes RNA or cDNA derived from RNA. In some embodiments, the RNA includes total cellular RNA. In other embodiments, certain abundant RNA sequences (e.g., ribosomal RNAs) have been depleted. In some embodiments, the poly(A)-tailed mRNA fraction in the total RNA preparation has been enriched. In some embodiments, the cDNA is produced by random-primed cDNA synthesis methods. In other embodiments, the cDNA synthesis is initiated at the poly(A) tail of mature mRNAs by priming by oligo(dT)-containing oligonucleotides. Methods for depletion, poly(A) enrichment, and cDNA synthesis are well known to those skilled in the art.

The method can further include amplifying the nucleic acid sample by specific or non-specific nucleic acid amplification methods that are well known to those skilled in the art. In some embodiments, the nucleic acid sample is amplified, e.g., by whole-genome amplification methods such as random-primed strand-displacement amplification.

In other embodiments, the nucleic acid sample is fragmented or sheared by physical or enzymatic methods and ligated to synthetic adapters, size-selected (e.g., by preparative gel electrophoresis) and amplified (e.g., by PCR). In other embodiments, the fragmented and adapter-ligated group of nucleic acids is used without explicit size selection or amplification prior to hybrid selection.

In other embodiments, the isolated DNA *(e.g.,* the genomic DNA) is fragmented or sheared. In some embodiments, the library includes less than 50% of genomic DNA, such as a subfraction of genomic DNA that is a reduced representation or a defined portion of a genome, *e.g.,* that has been subfractionated by other means. In other embodiments, the library includes all or substantially all genomic DNA.

In some embodiments, the library includes less than 50% of genomic DNA, such as a subfraction of genomic DNA that is a reduced representation or a defined portion of a genome, e.g., that has been subfractionated by other means. In other embodiments, the library includes all or substantially all genomic DNA. Protocols for isolating and preparing libraries from whole genomic or subgenomic fragments are known in the art (e.g., Illumina's genomic DNA sample preparation kit), and are described herein in the Examples. Alternative methods for DNA shearing are known in the art, *e.g..* as described in Example 4 in International Patent Application Publication No. WO 2012/092426. for example, alternative DNA shearing methods can be more automatable and/or more efficient (e.g., with degraded FFPE samples). Alternatives to DNA shearing methods can also be used to avoid a ligation step during library preparation.

The methods described herein can be performed using a small amount of nucleic acids, e.g., when the amount of source DNA or RNA is limiting (e.g., even after whole-genome amplification). In one embodiment, the nucleic acid comprises less than about 5 $\mu$g, 4 $\mu$g, 3 $\mu$g, 2 $\mu$g, 1 $\mu$g, 0.8 $\mu$g, 0.7 $\mu$g, 0.6 $\mu$g, 0.5 $\mu$g, or 400 ng, 300 ng, 200 ng, 100 ng, 50 ng, 10 ng. 5 ng, 1 ng, or less of nucleic acid sample. For example, one can typically begin with 50-100 ng of genomic DNA. One can start with less, however, if one amplifies the genomic DNA (e.g., using PCR) before the hybridization step, e.g., solution hybridization. Thus it is possible, but not essential, to amplify the genomic DNA before hybridization, e.g., solution hybridization.

The nucleic acid sample used to generate the library can also include RNA or cDNA derived from RNA. In some embodiments, the RNA includes total cellular RNA. In other embodiments, certain abundant RNA sequences (e.g., ribosomal RNAs) have been depleted. In other embodiments, the poly(A)-tailed mRNA fraction in the total RNA preparation has been enriched. In some embodiments, the cDNA is produced by random-primed cDNA synthesis methods. In other embodiments, the cDNA synthesis is initiated at the poly(A) tail of mature mRNAs by priming by oligo(dT)-containing oligonucleotides. Methods for depletion, poly(A) enrichment, and cDNA synthesis are well known to those skilled in the art.

The method can further include amplifying the nucleic acid sample by specific or non-specific nucleic acid amplification methods that are known to those skilled in the art. The nucleic acid sample can be amplified, e.g., by whole-genome amplification methods such as random-primed strand-displacement amplification.

The nucleic acid sample can be fragmented or sheared by physical or enzymatic methods as described herein, and ligated to synthetic adapters, size-selected (e.g., by preparative gel electrophoresis) and amplified (e.g., by PCR). The fragmented and adapter-ligated group of nucleic acids is used without explicit size selection or amplification prior to hybrid selection.

In an embodiment, the nucleic acid sample comprises DNA, RNA (or cDNA derived from RNA), or both, from a non-cancer cell or a non-malignant cell, e.g., a tumor-infiltrating lymphocyte. In an embodiment, the nucleic acid sample comprises DNA, RNA (or cDNA derived from RNA), or both, from a non-cancer cell or a non-malignant cell, e.g., a tumor-infiltrating lymphocyte, and does not comprise, or is essentially free of, DNA, RNA (or cDNA derived from RNA), or both, from a cancer cell or a malignant cell.

In an embodiment, the nucleic acid sample comprises DNA, RNA (or cDNA derived from RNA) from a cancer cell or a malignant cell. In an embodiment, the nucleic acid sample comprises DNA, RNA (or cDNA derived from RNA) from a cancer cell or a malignant cell, and does not comprise, or is essentially free of, DNA, RNA (or cDNA derived from RNA), or both, from a non-cancer cell or a non-malignant cell, e.g., a tumor-infiltrating lymphocyte.

In an embodiment, the nucleic acid sample comprises DNA, RNA (or cDNA derived from RNA), or both, from a non-cancer cell or a non-malignant cell, e.g., a tumor-infiltrating lymphocyte, and DNA, RNA (or cDNA derived from RNA), or both, from a cancer cell or a malignant cell.

*DESIGN AND CONSTRUCTION OF BAITS*

A bait can be a nucleic acid molecule, e.g., a DNA or RNA molecule, which can hybridize to (e.g., be complementary to), and thereby allow capture of a target nucleic acid. In certain embodiments, the target nucleic acid is a genomic DNA molecule. In other embodiments, the target nucleic acid is an RNA molecule or a cDNA molecule derived from an RNA molecule. In one embodiment, a bait is an RNA molecule. In other embodiments, a bait includes a binding entity, e.g., an affinity tag, that allows capture and separation, e.g., by binding to a binding entity, of a hybrid formed by a bait and a nucleic acid hybridized to the bait. In one embodiment, a bait is suitable for solution phase hybridization.

Typically, RNA molecules are used as bait sequences. A RNA-DNA duplex is more stable than a DNA-DNA duplex, and therefore provides for potentially better capture of nucleic acids.

RNA baits can be made as described elsewhere herein, using methods known in the art including, but not limited to, *de novo* chemical synthesis and transcription of DNA molecules using a DNA-dependent RNA polymerase. In one embodiment, the bait sequence is produced using known nucleic acid amplification methods, such as PCR, e.g., using human DNA or pooled human DNA samples as the template. The oligonucleotides can then be converted to RNA baits. In one embodiment, *in vitro* transcription is used, for example, based on adding an RNA polymerase promoter sequence to one end of the oligonucleotide. In one embodiment, the RNA polymerase promoter sequence is added at the end of the bait by amplifying or reamplifying the bait sequence, e.g., using PCR or other nucleic acid amplification methods, e.g., by tailing one primer of each target-specific primer pairs with an RNA promoter sequence. In one embodiment, the RNA polymerase is a T7 polymerase, a SP6 polymerase, or a T3 polymerase. In one embodiment, RNA bait is labeled with a tag, e.g.. an affinity tag. In one embodiment, RNA bait is made by *in vitro* transcription, e.g., using biotinylated UTP. In another embodiment, RNA bait is produced without biotin and then biotin is crosslinked to the RNA molecule using methods well known in the art, such as psoralen crosslinking. In one embodiment, the RNA bait is an RNase-resistant RNA molecule, which can be made, e.g., by using modified nucleotides during transcription to produce a RNA molecule that resists RNase degradation. In one embodiment, the RNA bait corresponds to only one strand of the double-stranded DNA target. Typically, such RNA baits are not self-complementary and are more effective as hybridization drivers.

The bait sets can be designed from reference sequences, such that the baits are optimal for selecting targets of the reference sequences. In some embodiments, bait sequences are designed using a mixed base (e.g., degeneracy). For example, the mixed base(s) can be included in the bait sequence at the position(s) of a common SNP or mutation, to optimize the bait sequences to catch both alleles (e.g., SNP and non-SNP; mutant and non-mutant). In some embodiments, all known sequence variations (or a subset thereof) can be targeted with multiple oligonucleotide baits, rather than by using mixed degenerate oligonucleotides.

In certain embodiments, the bait set includes an oligonucleotide (or a plurality of oligonucleotides) between about 100 nucleotides and 300 nucleotides in length. Typically, the bait set includes an oligonucleotide (or a plurality of oligonucleotides) between about 130 nucleotides and 230 nucleotides, or about 150 and 200 nucleotides, in length. In other embodiments, the bait set includes an oligonucleotide (or a plurality of oligonucleotides) between about 300 nucleotides and 1000 nucleotides in length.

In some embodiments, the target member-specific sequences in the oligonucleotide are between about 40 and 1000 nucleotides, about 70 and 300 nucleotides, about 100 and 200 nucleotides in length, typically between about 120 and 170 nucleotides in length.

In some embodiments, the bait set includes a binding entity. The binding entity can be an affinity tag on each bait sequence. In some embodiments, the affinity tag is a biotin molecule or a hapten. In certain embodiments, the binding entity allows for separation of the bait/member hybrids from the hybridization mixture by binding to a partner, such as an avidin molecule, or an antibody that binds to the hapten or an antigen-binding fragment thereof.

In other embodiments, the oligonucleotides in the bait set contain forward and reverse complement sequences for the same target member sequence whereby the oligonucleotides with reverse-complemented member-specific sequences also carry reverse complement universal tails. This can lead to RNA transcripts that are the same strand, i.e., not complementary to each other.

In other embodiments, the bait set includes oligonucleotides that contain degenerate or mixed bases at one or more positions. In still other embodiments, the bait set includes multiple or substantially all known sequence variants present in a population of a single species or community of organisms. In one embodiment, the bait set includes multiple or substantially all known sequence variants present in a human population.

In other embodiments, the bait set includes cDNA sequences or is derived from cDNA sequences. In other embodiments, the bait set includes amplification products (e.g., PCR products) that are amplified from genomic DNA, cDNA or cloned DNA.

In other embodiments, the bait set includes RNA molecules. In some embodiments, the set includes chemically, enzymatically modified, or in vitro transcribed RNA molecules, including but not limited to, those that are more stable and resistant to RNase.

In yet other embodiments, the baits are produced by methods described in US 2010/0029498 and Gnirke, A. et al. (2009) Nat Biotechnol. 27(2):182-189. For example, biotinylated RNA baits can be produced by obtaining a pool of synthetic long oligonucleotides, originally synthesized on a microarray, and amplifying the oligonucleotides to produce the bait sequences. In some embodiments, the baits are produced by adding an RNA polymerase promoter sequence at one end of the bait sequences, and synthesizing RNA sequences using RNA polymerase. In one embodiment, libraries of synthetic oligodeoxynucleotides can be obtained from commercial suppliers, such as Agilent Technologies, Inc., and amplified using known nucleic acid amplification methods.

Accordingly, a method of making the aforesaid bait set is provided. The method includes selecting one or more target-specific bait oligonucleotide sequences (e.g., one or more mutation capturing, reference or control oligonucleotide sequences as described herein); obtaining a pool of target-specific bait oligonucleotide sequences (e.g., synthesizing the pool of target-specific bait oligonucleotide sequences, e.g., by microarray synthesis); and optionally, amplifying the oligonucleotides to produce the bait set.

In other embodiments, the methods further include amplifying (e.g., by PCR) the oligonucleotides using one or more biotinylated primers. In some embodiments, the oligonucleotides include a universal sequence at the end of each oligonucleotide attached to the microarray. The methods can further include removing the universal sequences from the oligonucleotides. Such methods can also include removing the complementary strand of the oligonucleotides, annealing the oligonucleotides, and extending the oligonucleotides. In some of these embodiments, the methods for amplifying (e.g., by PCR) the oligonucleotides use one or more biotinylated primers. In some embodiments, the method further includes size selecting the amplified oligonucleotides.

In one embodiment, an RNA bait set is made. The methods include producing a set of bait sequences according to the methods described herein, adding an RNA polymerase promoter sequence at one end of the bait sequences, and synthesizing RNA sequences using RNA polymerase. The RNA polymerase can be chosen from a T7 RNA polymerase, an SP6 RNA polymerase, or a T3 RNA polymerase. In other embodiments, the RNA polymerase promoter sequence is added at the ends of the bait sequences by amplifying (e.g., by PCR) the bait sequences. In embodiments where the bait sequences are amplified by PCR with specific primer pairs out of genomic DNA or cDNA, adding an RNA promoter sequence to the 5' end of one of the two specific primers in each pair will lead to a PCR product that can be transcribed into an RNA bait using standard methods.

In other embodiments, bait sets can be produced using human DNA or pooled human DNA samples as the template. In such embodiments, the oligonucleotides are amplified by polymerase chain reaction (PCR). In other embodiments, the amplified oligonucleotides are reamplified by rolling circle amplification or hyperbranched rolling circle amplification. The same methods also can be used to produce bait sequences using human DNA or pooled human DNA samples as the template. The same methods can also be used to produce bait sequences using subfractions of a genome obtained by other methods, including but not limited to restriction digestion, pulsed-field gel electrophoresis, flow-sorting, CsCl density gradient centrifugation, selective kinetic reassociation, microdissection of chromosome preparations, and other fractionation methods known to those skilled in the art.

In certain embodiments, the number of baits in the bait set is less than 1,000. In other embodiments, the number of baits in the bait set is greater than 1,000, greater than 5,000, greater than 10,000, greater than 20,000, greater than 50,000, greater than 100,000, or greater than 500.000.

The length of the bait sequence can be between about 70 nucleotides and 1000 nucleotides. In one embodiment, the bait length is between about 100 and 300 nucleotides, 110 and 200 nucleotides, or 120 and 170 nucleotides, in length. In addition to those mentioned above, intermediate oligonucleotide lengths of about 70, 80, 90, 100, 110, 120, 130, 140, 150, 160, 170, 180, 190, 200, 210, 220, 230, 240, 250, 300, 400, 500, 600, 700, 800, and 900 nucleotides in length can be used in the methods described herein. In some embodiments, oligonucleotides of about 70, 80, 90, 100, 110, 120, 130, 140, 150. 160, 170, 180, 190, 200, 210, 220, or 230 bases can be used.

Each bait sequence can include a target-specific (e.g., a member-specific) bait sequence and universal tails on one or both ends. As used herein, the term "bait sequence" can refer to the target-specific bait sequence or the entire oligonucleotide including the target-specific "bait sequence" and other nucleotides of the oligonucleotide. The target-specific sequences in the baits are between about 40 nucleotides and 1000 nucleotides in length. In one embodiment, the target-specific sequence is between about 70 nucleotides and 300 nucleotides in length. In another embodiment, the target-specific sequence is between about 100 nucleotides and 200 nucleotides in length. In yet another embodiment, the target-specific sequence is between about 120 nucleotides and 170 nucleotides in length, typically 120 nucleotides in length. Intermediate lengths in addition to those mentioned above also can be used in the methods described herein, such as target-specific sequences of about 40, 50, 60. 70, 80, 90, 100, 110, 120, 130. 140, 150, 160, 170, 180, 190, 200, 210, 220, 230, 240. 250, 300, 400, 500, 600, 700, 800, and 900 nucleotides in length, as well as target-specific sequences of lengths between the above-mentioned lengths.

In one embodiment, the bait is an oligomer (e.g., comprised of RNA oligomers, DNA oligomers, or a combination thereof) about 50 to 200 nucleotides in length (e.g., about 50, 60, 80, 90, 100, 110, 120, 130, 140, 150, 160, 170, 190, or 200 nucleotides in length). In one embodiment, each bait oligomer includes about 120 to 170, or typically, about 120 nucleotides, which are a target-specific bait sequence. The bait can comprise additional non-target-specific nucleotide sequences at one or both ends. The additional nucleotide sequences can be used, e.g., for PCR amplification or as a bait identifier. In certain embodiments, the bait additionally comprises a binding entity as described herein (e.g., a capture tag such as a biotin molecule). The binding entity, e.g., biotin molecule, can be attached to the bait. e.g., at the 5'-end, 3'-end, or internally (e.g., by incorporating a biotinylated nucleotide), of the bait. In one embodiment, the biotin molecule is attached at the 5'-end of the bait.

In one exemplary embodiment, the bait is an oligonucleotide about 150 nucleotides in length, of which 120 nucleotides are target-specific "bait sequence". The other 30 nucleotides (e.g.. 15 nucleotides on each end) are universal arbitrary tails used for PCR amplification. The tails can be any sequence selected by the user. For example, the pool of synthetic oligonucleotides can include oligonucleotides of the sequence of 5'-ATCGCAC-CAGCGTGTN$_{120}$CACTGCGGCTCCTCA-3' (SEQ ID NO: 1) with N$_{120}$ indicating the target-specific bait sequences.

The bait sequences described herein can be used for selection of exons and short target sequences. In one embodiment, the bait is between about 100 nucleotides and 300 nucleotides in length. In another embodiment, the bait is between about 130 nucleotides and 230 nucleotides in length. In yet another embodiment, the bait is between about 150 nucleotides and 200 nucleotides in length. The target-specific sequences in the baits, e.g., for selection of exons and short target sequences, are between about 40 nucleotides and 1000 nucleotides in length. In one embodiment, the target-specific sequence is between about 70 nucleotides and 300 nucleotides in length. In another embodiment, the target-specific sequence is between about 100 nucleotides and 200 nucleotides in length. In yet another embodiment, the target-specific sequence is between about 120 nucleotides and 170 nucleotides in length.

In some embodiments, long oligonucleotides can minimize the number of oligonucleotides necessary to capture the target sequences. For example, one oligonucleotide can be used per exon. It is known in the art that the mean and median lengths of the protein-coding exons in the human genome are about 164 and 120 base pairs, respective. Longer baits can be more specific and capture better than shorter ones. As a result, the success rate per oligonucleotide bait sequence is higher than with short oligonucleotides. In one embodiment, the minimum bait-covered sequence is the size of one bait (e.g., 120-170 bases), e.g., for capturing exon-sized targets. In determining the length of the bait sequences, one also can take into consideration that unnecessarily long baits catch more unwanted DNA directly adjacent to the target. Longer oligonucleotide baits can also be more tolerant to polymorphisms in the targeted region in the DNA samples than shorter ones. Typically, the bait sequences are derived from a reference genome sequence. If the target sequence in the actual DNA sample deviates from the reference sequence, for example if it contains a single nucleotide polymorphism (SNP), it can hybridize less efficiently to the bait and may therefore be under-represented or completely absent in the sequences hybridized to the bait sequences. Allelic drop-outs due to SNPs can be less likely with the longer synthetic bait molecules for the reason that a single mismatch in, e.g., 120 to 170 bases can have less of an effect on hybrid stability than a single mismatch in, 20 or 70 bases, which are the typical bait or primer lengths in multiplex amplification and microarray capture, respectively.

For selection of targets that are long compared to the length of the capture baits, such as genomic regions, bait sequence lengths are typically in the same size range as the baits for short targets mentioned above, except that there is no need to limit the maximum size of bait sequences for the sole purpose of minimizing targeting of adjacent sequences. Alternatively, oligonucleotides can be titled across a much wider window (typically 600 bases). This method can be used to capture DNA fragments that are much larger (e.g., about 500 bases) than a typical exon. As a result, much more unwanted flanking non-target sequences are selected.

*Bait Synthesis*

The baits can be any type of oligonucleotide, e.g., DNA or RNA. The DNA or RNA baits ("oligo baits") can be synthesized individually, or can be synthesized in an array, as a DNA or RNA bait set ("array baits"). An oligo bait, whether provided in an array format, or as an isolated oligo, is typically single stranded. The bait can additionally comprise a binding entity as described herein (e.g., a capture tag such as a biotin molecule). The binding entity, e.g., biotin molecule, can be attached to the bait, e.g., at the 5' or 3'-end of the bait, typically, at the 5'-end of the bait. Bait sets can be synthesized by methods described in the art. e.g., as described in International Patent Application Publication No. WO 2012/092426.

*HYBRIDIZATION CONDITIONS*

The methods featured in the invention include the step of contacting the library (e.g., the nucleic acid library) with a plurality of baits to provide a selected library catch. The contacting step can be effected in solution hybridization. In certain embodiments, the method includes repeating the hybridization step by one or more additional rounds of solution hybridization. In some embodiments, the methods further include subjecting the library catch to one or more additional rounds of solution hybridization with the same or different collection of baits. Hybridization methods that can be adapted for use in the methods herein are described in the art, e.g., as described in International Patent Application Publication No. WO 2012/092426.

Additional embodiments or features of the present invention are as follows:
In another aspect, the invention features a method of making the aforesaid bait sets. The method includes selecting one or more target-specific bait oligonucleotide sequences (e.g., any of the bait sequences corresponding to the subject intervals (e.g., subgenomic intervals, expressed subgenomic intervals, or both) of the gene or gene products as described herein); obtaining a pool of target-specific bait oligonucleotide sequences (e.g., synthesizing the pool of target-specific bait oligonucleotide sequences, e.g., by microarray synthesis); and optionally, amplifying the oligonucleotides to produce the bait sets.

In yet another aspect, the invention features a method for determining the presence or absence of an alteration associated, e.g., positively or negatively, with a cancerous phenotype (e.g., at least 10, 20, 30, 50 or more of the alterations in the genes or gene products described herein) in a nucleic acid sample. The method includes contacting the nucleic acids in the sample in a solution-based reaction according to any of the methods and baits described herein to obtain a nucleic acid catch; and sequencing (e.g., by next-generation sequencing) all or a subset of the nucleic acid catch, thereby determining the presence or absence of the alteration in the genes or gene products described herein).

In certain embodiments, the bait set includes an oligonucleotide (or a plurality of oligonucleotides) between about 100 nucleotides and 300 nucleotides in length. Typically, the bait set includes an oligonucleotide (or a plurality of oligonucleotides) between about 130 nucleotides and 230 nucleotides, or about 150 and 200 nucleotides, in length. In other embodiments, the bait set includes an oligonucleotide (or a plurality of oligonucleotides) between about 300 nucleotides and 1000 nucleotides in length.

In some embodiments, the target member-specific sequences in the oligonucleotide are between about 40 and 1000 nucleotides, about 70 and 300 nucleotides, about 100 and 200 nucleotides in length, typically between about 120 and 170 nucleotides in length.

In some embodiments, the bait set includes a binding entity. The binding entity can be an affinity tag on each bait sequence. In some embodiments, the affinity tag is a biotin molecule or a hapten. In certain embodiments, the binding entity allows for separation of the bait/member hybrids from the hybridization mixture by binding to a partner, such as an avidin molecule, or an antibody that binds to the hapten or an antigen-binding fragment thereof.

In other embodiments, the oligonucleotides in the bait set contain forward and reverse complement sequences for the same target member sequence whereby the oligonucleotides with reverse-complemented member-specific sequences also carry reverse complement universal tails. This can lead to RNA transcripts that are the same strand, *i.e.,* not complementary to each other.

In other embodiments, the bait set includes oligonucleotides that contain degenerate or mixed bases at one or more positions. In still other embodiments, the bait set includes multiple or substantially all known sequence variants present in a population of a single species or community of organisms. In one embodiment, the bait set includes multiple or substantially all known sequence variants present in a human population.

In other embodiments, the bait set includes cDNA sequences or are derived from cDNAs sequences. In one embodiment, the cDNA is prepared from an RNA sequence, *e.g.,* a tumor- or cancer cell-derived RNA, *e.g.,* an RNA obtained from a tumor-FFPE sample, a blood sample, or a bone marrow aspirate sample. In other embodiments, the bait set includes amplification products (e.g., PCR products) that are amplified from genomic DNA, cDNA or cloned DNA.

In other embodiments, the bait set includes RNA molecules. In some embodiments, the set includes are chemically, enzymatically modified, or *in vitro* transcribed RNA molecules, including but not limited to, those that are more stable and resistant to RNase.

In yet other embodiments, the baits are produced by methods described in US 2010/0029498 and Gnirke, A. et al. (2009) Nat Biotechnol. 27(2):182-189. For example, biotinylated RNA baits can be produced by obtaining a pool of synthetic long oligonucleotides, originally synthesized on a microarray, and amplifying the oligonucleotides to produce the bait sequences. In some embodiments, the baits are produced by adding an RNA polymerase promoter sequence at one end of the bait sequences, and synthesizing RNA sequences using RNA polymerase. In one embodiment, libraries of synthetic oligodeoxynucleotides can be obtained from commercial suppliers, such as Agilent Technologies, Inc., and amplified using known nucleic acid amplification methods.

Accordingly, a method of making the aforesaid bait set is provided. The method includes selecting one or more target-specific bait oligonucleotide sequences (e.g., one or more mutation capturing, reference or control oligonucleotide sequences as described herein); obtaining a pool of target-specific bait oligonucleotide sequences (e.g., synthesizing the pool of target-specific bait oligonucleotide sequences, *e.g.,* by microarray synthesis); and optionally, amplifying the oligonucleotides to produce the bait set.

In other embodiments, the methods further include amplifying (e.g., by PCR) the oligonucleotides using one or more biotinylated primers. In some embodiments, the oligonucleotides include a universal sequence at the end of each oligonucleotide attached to the microarray. The methods can further include removing the universal sequences from the oligonucleotides. Such methods can also include removing the complementary strand of the oligonucleotides, annealing the oligonucleotides, and extending the oligonucleotides. In some of these embodiments, the methods for amplifying (e.g., by PCR) the oligonucleotides use one or more biotinylated primers. In some embodiments, the method further includes size selecting the amplified oligonucleotides.

In one embodiment, an RNA bait set is made. The methods include producing a set of bait sequences according to the methods described herein, adding an RNA polymerase promoter sequence at one end of the bait sequences, and synthesizing RNA sequences using RNA polymerase. The RNA polymerase can be chosen from a T7 RNA polymerase, an SP6 RNA polymerase, or a T3 RNA polymerase. In other embodiments, the RNA polymerase promoter sequence is added at the ends of the bait sequences by amplifying *(e.g.,* by PCR) the bait sequences. In embodiments where the bait sequences are amplified by PCR with specific primer pairs out of genomic DNA or cDNA. adding an RNA promoter sequence to the 5' end of one of the two specific primers in each pair will lead to a PCR product that can be transcribed into an RNA bait using standard methods.

In other embodiments, bait sets can be produced using human DNA or pooled human DNA samples as the template. In such embodiments, the oligonucleotides are amplified by polymerase chain reaction (PCR). In other embodiments, the amplified oligonucleotides are reamplified by rolling circle amplification or hyperbranched rolling circle amplification. The same methods also can be used to produce bait sequences using human DNA or pooled human DNA samples as the template. The same methods can also be used to produce bait sequences using subfractions of a genome obtained by other methods, including but not limited to restriction digestion, pulsed-field gel electrophoresis, flow-sorting, CsCl density gradient centrifugation, selective kinetic reassociation, microdissection of chromosome preparations, and other fractionation methods known to those skilled in the art.

In certain embodiments, the number of baits in the bait set is less than 1,000, *e.g.,* 2, 3, 4, 5, 10, 50, 100, 500 baits. In other embodiments, the number of baits in the bait set is greater than 1,000, greater than 5,000, greater than 10,000, greater than 20,000, greater than 50,000, greater than 100,000, or greater than 500,000.

In certain embodiments, a library *(e.g.,* a nucleic acid library) includes a collection of members. As described herein, the library members can include a target member (e.g., a tumor member, a reference member and/or a control member; also referred to herein as a first, second and/or third member, respectively). The members of the library can be from a single individual. In embodiments a library can comprise members from more than one subject (e.g., 2, 3, 4, 5, 6, 7, 8, 9, 10, 20, 30 or more subjects), e.g., two or more libraries from different subjects can be combined to form a library having members from more than one subject. In one embodiment, the subject is a human having, or at risk of having, a cancer or tumor.

"Member" or "library member" or other similar term, as used herein, refers to a nucleic acid molecule, e.g., DNA or RNA. that is a member of a library. Typically, a member is a DNA molecule, e.g., genomic DNA or cDNA. A member can be sheared genomic DNA. In other embodiments, the member can be a cDNA. In other embodiments, the member can be an RNA. Members comprise sequence from a subject and can also comprise a sequence not derived from the subject, e.g., primers or sequences that allow for identification, e.g., "barcode" sequences.

In yet another embodiment, the methods featured in the invention further include isolating a nucleic acid sample to provide a library (e.g., a nucleic acid library as described herein). In certain embodiments, the nucleic acid sample includes whole genomic, subgenomic fragments, or both. Protocols for isolating and preparing libraries from whole genomic or subgenomic fragments are known in the art (e.g., Illumina's genomic DNA sample preparation kit). In certain embodiments, the genomic or subgenomic DNA fragment is isolated from a subject's sample (e.g., a tumor sample, a normal adjacent tissue (NAT), a blood sample or any normal control)). In one embodiment, the sample (e.g., the tumor or NAT sample) is a preserved. For example, the sample is embedded in a matrix, e.g., an FFPE block or a frozen sample. In certain embodiments, the isolating step includes flow-sorting of individual chromosomes; and/or microdissecting a subject's sample (e.g., a tumor sample, a NAT, a blood sample). In certain embodiments, the nucleic acid sample used to generate the nucleic acid library is less than 5 micrograms, less than 1 microgram, or less than 500ng (e.g., 200 ng or less).

In still other embodiments, the nucleic acid sample used to generate the library includes RNA or cDNA derived from RNA. In some embodiments, the RNA includes total cellular RNA. In other embodiments, certain abundant RNA sequences (e.g., ribosomal RNAs) have been depleted. In some embodiments, the poly(A)-tailed mRNA fraction in the total RNA preparation has been enriched. In some embodiments, the cDNA is produced by random-primed cDNA synthesis methods. In other embodiments, the cDNA synthesis is initiated at the poly(A) tail of mature mRNAs by priming by oligo(dT)-containing oligonucleotides. Methods for depletion, poly(A) enrichment, and cDNA synthesis are well known to those skilled in the art.

The method can further include amplifying the nucleic acid sample by specific or non-specific nucleic acid amplification methods that are well known to those skilled in the art.

In some embodiments, the nucleic acid sample is amplified, e.g., by whole-genome amplification methods such as random-primed strand-displacement amplification.

In other embodiments, the nucleic acid sample is fragmented or sheared by physical or enzymatic methods and ligated to synthetic adapters, size-selected (e.g., by preparative gel electrophoresis) and amplified (e.g., by PCR). In other embodiments, the fragmented and adapter-ligated group of nucleic acids is used without explicit size selection or amplification prior to hybrid selection.

In other embodiments, the isolated DNA (e.g., the genomic DNA) is fragmented or sheared. In some embodiments, the library includes less than 50% of genomic DNA, such as a subfraction of genomic DNA that is a reduced representation or a defined portion of a genome, e.g., that has been subfractionated by other means. In other embodiments, the library includes all or substantially all genomic DNA.

In certain embodiments, the members of the library include a subgenomic interval that includes an intragenic region or an intergenic region. In another embodiment, the subgenomic interval includes an exon or an intron, or a fragment thereof, typically an exon sequence or a fragment thereof. In one embodiment, the subgenomic interval includes a coding region or a non-coding region, e.g., a promoter, an enhancer, a 5' untranslated region (5' UTR), or a 3' untranslated region (3' UTR), or a fragment thereof. In other embodiments, the subgenomic interval includes a cDNA or a fragment thereof (e.g., cDNA obtained from a tumor RNA (e.g., RNA extracted from a tumor sample, e.g., FFPE-tumor sample). In other embodiments, the subgenomic interval includes an SNP, e.g., as described herein. In other embodiments, the target members include substantially all exons in a genome. In other embodiments, the target members include a subgenomic interval as described herein, e.g., subgenomic intervals, e.g., exons from selected genes or gene products of interest (e.g., genes or gene products associated with a cancerous phenotype as described herein).

In one embodiment, the subgenomic interval includes a somatic mutation, a germline mutation or both. In one embodiment, the subgenomic interval includes an alteration, e.g., a point or a single mutation, a deletion mutation (e.g., an in-frame deletion, an intragenic deletion, a full gene deletion), an insertion mutation (e.g., intragenic insertion), an inversion mutation (e.g., an intra-chromosomal inversion), a linking mutation, a linked insertion mutation, an inverted duplication mutation, a tandem duplication (e.g., an intrachromosomal tandem duplication), a translocation (e.g., a chromosomal translocation, a non-reciprocal translocation), a rearrangement (e.g., a genomic rearrangement), a change in gene copy number, or a combination thereof. In certain embodiments, the subgenomic interval constitutes less than 5%, 1%, 0.5%, 0.1%, 0.01%, 0.001% of the coding region of the genome of the tumor cells in a sample. In other embodiments, the subgenomic intervals are not involved in a disease, *e.g.,* are not associated with a cancerous phenotype as described herein.

The methods featured in the invention include the step of contacting one or a plurality of libraries *(e.g.,* one or a plurality of nucleic acid libraries) with a plurality of baits to provide a selected subgroup of nucleic acids, *e.g.,* a library catch. In one embodiment, the contacting step is effected in a solid support, *e.g.,* an array. Suitable solid supports for hybridization are described in, *e.g.,* Albert, T.J. et al. (2007) Nat. Methods 4(11):903-5; Hodges, E. et al. (2007) Nat. Genet. 39(12):1522-7; and Okou, D.T. et al. (2007) Nat. Methods 4(11):907-9. In other embodiments, the contacting step is effected in solution hybridization. In certain embodiments, the method includes repeating the hybridization step by one or more additional rounds of hybridization. In some embodiments, the methods further include subjecting the library catch to one or more additional rounds of hybridization with the same or different collection of baits.

In other embodiments, the methods featured in the invention further include amplifying the library catch *(e.g.,* by PCR). In other embodiments, the library catch is not amplified.

In yet other embodiments, the methods further include analyzing the library catch. In one embodiment, the library catch is analyzed by a sequencing method, *e.g..* a next-generation sequencing method as described herein. The methods include isolating a library catch by solution hybridization, and subjecting the library catch by nucleic acid sequencing. In certain embodiments, the library catch can be re-sequenced. Next-generation sequencing methods are known in the art, and are described, e.g., in Metzker, M. (2010) Nature Biotechnology Reviews 11:31-46.

In yet other embodiments, the methods further include the step of subjecting the library catch to genotyping, thereby identifying the genotype of the selected nucleic acids.

In certain embodiments, the method further includes one or more of:

i) fingerprinting the nucleic acid sample;
ii) quantifying the abundance of a gene or gene product *(e.g.,* a gene or gene product as described herein) in the nucleic acid sample (e.g., quantifying the relative abundance of a transcript in the sample);
iii) identifying the nucleic acid sample as belonging to a particular subject (e.g., a normal control or a cancer patient);
iv) identifying a genetic trait in the nucleic acid sample (e.g., one or more subject's genetic make-up (e.g., ethnicity, race, familial traits));
v) determining the ploidy in the nucleic acid sample; determining a loss of heterozygosity in the nucleic acid sample;
vi) determining the presence or absence of a gene duplication event in the nucleic acid sample;
vii) determining the presence or absence of a gene amplification event in the nucleic acid sample; or
viii) determining the level of tumor/normal cellular admixture in the nucleic acid sample.

Any of the methods described herein can be combined with one or more of the embodiments below.

In an embodiment, the method comprises acquiring a nucleotide sequence read obtained from a tumor and/or control nucleic acid sample (e.g., an FFPE-derived nucleic acid sample, or a nucleic acid sample derived from a blood sample or a bone marrow aspirate sample).

In an embodiment, the reads are provided by a next-generation sequencing method.

In an embodiment, the method includes providing a library of nucleic acid members and sequencing a preselected subgenomic interval from a plurality of members of said library. In embodiments, the method can include a step of selecting a subset of said library for sequencing, *e.g.,* a solution-based selection.

In certain embodiments, a method comprises hybrid capture methods which are designed to capture two or more different target categories, each with a different bait design strategies. The hybrid capture methods and compositions are intended to capture a defined subset of target sequences (e.g., target members) and provide homogenous coverage of the target sequence, while minimizing coverage outside of that subset. In one embodiment, the target sequences include the entire exome out of genomic DNA. or a selected subset thereof. The methods and compositions disclosed herein provide different bait sets for achieving different depths and patterns of coverage for complex target nucleic acid sequences (e.g., libraries).

In certain embodiments, the different categories of bait sets and targets are as follows.

A. A first bait set that selects a high-level target *(e.g.,* one or more tumor members and/or reference members, such as genes, exons, or bases) for which the deepest coverage is required to enable a high level of sensitivity for mutations that appear at low frequencies. For example, detection of point mutations that appear at a frequency of about 5% or less *(i.e.* 5% of the cells from which the sample was prepared harbor this mutation in their genome). The first bait set typically requires about 500X or higher sequencing depth to ensure high detection reliability. In one embodiment, the first bait set selects one or more subgenomic intervals *(e.g.,* exons) that are frequently mutated in certain types of cancer, *e.g.,* a gene or gene product according to **Tables 1-4 or FIGs. 3A-4D.**

B. A second bait set that selects a mid-level target *(e.g.,* one or more tumor members and/or reference members, such as genes, exons, or bases) for which high coverage is required to enable high level of sensitivity for mutations that appear at a higher frequency than the high level target, *e.g.,* a frequency of about 10%. For example, detection of an alteration (e.g., a point mutation) that appears at a frequency of 10% requires about 200X or higher sequencing depth to ensure high detection reliability. In one embodiment, the second bait set selects one or more subgenomic intervals (e.g., exons) that are chosen from the genes or gene products according to **Tables 1-4 or FIGs. 3A-4D.**

C. A third bait set that selects a low-level target (e.g., one or more PGx members, such as genes, exons, or bases) for which low-medium coverage is required to enable high level of sensitivity, *e.g.*, to detect heterozygous alleles. For example, detection of heterozygous alleles requires 10-100X sequencing depth to ensure high detection reliability. In one embodiment, the third bait set selects one or more subgenomic intervals (e.g., exons) that are chosen from: a) pharmacogenomic SNPs that may explain the ability of patient to metabolize different drugs, b) genomic SNPs that may be used to uniquely identify (fingerprint) a patient, and c) genomic SNPs/loci that may be used to assess copy number gains/losses of genomic DNA and loss-of-heterozygosity (LOH).

D. A fourth bait set that selects an intron target *(e.g.,* an intron member) for which low-medium coverage is required to detect structural breakpoints such as genomic translocations or indels. For example, detection of an intronic breakpoint requires 5-50X sequence-pair spanning depth to ensure high detection reliability. Said fourth bait sets can be used to detect, for example, translocation/indel-prone cancer genes.

E. A fifth bait set that selects an intron target *(e.g.,* an intron member) for which sparse coverage is required to improve the ability to detect copy number changes. For example, detection of a 1 copy deletion of several terminal exons requires 0.1-10X coverage to ensure high detection reliability. Said fifth bait sets can be used to detect, for example, amplification/deletion-prone cancer genes.

The methods and compositions featured in the invention involve tuning the relative sequence coverage of each bait set/target category. Methods for implementing differences in relative sequence coverage in bait design include one or more of:

(i) Differential representation of different bait sets - The bait set design to capture a given target *(e.g.,* a target member) can be included in more/fewer number of copies to enhance/reduce relative target coverage depths;

(ii) Differential overlap of bait subsets - The bait set design to capture a given target *(e.g.,* a target member) can include a longer or shorter overlap between neighboring baits to enhance/reduce relative target coverage depths;

(iii) Differential bait parameters - The bait set design to capture a given target *(e.g.,* a target member) can include sequence modifications/shorter length to reduce capture efficiency and lower the relative target coverage depths;

(iv) Mixing of different bait sets - Bait sets that are designed to capture different target sets can be mixed at different molar ratios to enhance/reduce relative target coverage depths;

(v) Using different types of oligonucleotide bait sets -In certain embodiments, the bait set can include:

(a) one or more chemically *(e.g.,* non-enzymatically) synthesized (*e.g.*, individually synthesized) baits,
(b) one or more baits synthesized in an array.
(c) one or more enzymatically prepared, *e.g., in vitro* transcribed, baits;
(d) any combination of (a), (b) and/or (c),
(e) one or more DNA oligonucleotides (e.g., a naturally or non-naturally occurring DNA oligonucleotide),
(f) one or more RNA oligonucleotides *(e.g.,* a naturally or non-naturally occurring RNA oligonucleotide).
(g) a combination of (e) and (f), or
(h) a combination of any of the above.

The different oligonucleotide combinations can be mixed at different ratios, *e.g.,* a ratio chosen from 1:1, 1:2. 1:3, 1:4, 1:5, 1:10, 1:20, 1:50; 1:100, 1:1000, or the like. In one embodiment, the ratio of chemically-synthesized bait to array-generated bait is chosen from 1:5, 1:10, or 1:20. The DNA or RNA oligonucleotides can be naturally- or nonnaturally-occurring. In certain embodiments, the baits include one or more non-naturally-occurring nucleotides to, *e.g.,* increase melting temperature. Exemplary non-naturally occurring oligonucleotides include modified DNA or RNA nucleotides. An exemplary modified RNA nucleotide is a locked nucleic acid (LNA), wherein the ribose moiety of an LNA nucleotide is modified with an extra bridge connecting the 2' oxygen and 4' carbon (Kaur, H; Arora, A; Wengel. J; Maiti, S; Arora, A.; Wengel, J.; Maiti, S. (2006). "Thermodynamic, Counterion, and Hydration Effects for the Incorporation of Locked Nucleic Acid Nucleotides into DNA Duplexes". Biochemistry 45 (23): 7347-55). Other modified exemplary DNA and RNA nucleotides include, but are not limited to, peptide nucleic acid (PNA) composed of repeating N-(2-aminoethyl)-glycine units linked by peptide bonds (Egholm, M. et al. (1993) Nature 365 (6446): 566-8); a DNA or RNA oligonucleotide modified to capture low GC regions; a bicyclic nucleic acid (BNA) or a crosslinked oligonucleotide; a modified 5-methyl deoxycytidine; and 2.6-diaminopurine. Other modified DNA and RNA nucleotides are known in the art.

In certain embodiments, a substantially uniform or homogeneous coverage of a target sequence *(e.g.,* a target member) is obtained. For example, within each bait set/target category, uniformity of coverage can be optimized by modifying bait parameters, for example, by one or more of:

(i) Increasing/decreasing bait representation or overlap can be used to enhance/reduce coverage of targets (e.g., target members), which are under/over-covered relative to other targets in the same category;

(ii) For low coverage, hard to capture target sequences (e.g., high GC content sequences), expand the region being targeted with the bait sets to cover, *e.g.,* adjacent sequences *(e.g.,* less GC-rich adjacent sequences);

(iii) Modifying a bait sequence can be used to reduce secondary structure of the bait and enhance its efficiency of selection;

(iv) Modifying a bait length can be used to equalize melting hybridization kinetics of different baits within the same category. Bait length can be modified directly (by producing baits with varying lengths) or indirectly (by producing baits of consistent length, and replacing the bait ends with arbitrary sequence);

(v) Modifying baits of different orientation for the same target region (i.e. forward and reverse strand) may have different binding efficiencies. The bait set with either orientation providing optimal coverage for each target may be selected;

(vi) Modifying the amount of a binding entity, *e.g.,* a capture tag *(e.g.* biotin), present on each bait may affect its binding efficiency. Increasing/decreasing the tag level of baits targeting a specific target may be used to enhance/reduce the relative target coverage;

(vii) Modifying the type of nucleotide used for different baits can be used to affect binding affinity to the target, and enhance/reduce the relative target coverage; or

(viii) Using modified oligonucleotide baits, *e.g.,* having more stable base pairing, can be used to equalize melting hybridization kinetics between areas of low or normal GC content relative to high GC content.

For example, different types of oligonucleotide bait sets can be used.

In one embodiment, the value for efficiency of selection is modified by using different types of bait oligonucleotides to encompass pre-selected target regions. For example, a first bait set *(e.g.,* an array-based bait set comprising 10,000-50,000 RNA or DNA baits) can be used to cover a large target area *(e.g.,* 1-2MB total target area). The first bait set can be spiked with a second bait set *(e.g.,* individually synthesized RNA or DNA bait set comprising less than 5,000 baits) to cover a pre-selected target region *(e.g.,* selected subgenomic intervals of interest spanning, *e.g.,* 250kb or less, of a target area) and/or regions of higher secondary structure, *e.g.,* higher GC content. Selected subgenomic intervals of interest may correspond to one or more of the genes or gene products described herein, or a fragment thereof. The second bait set may include about 2,000-5,000 baits depending on the bait overlap desired. In yet other embodiments, the second bait set can include selected oligo baits *(e.g.,* less than 400, 200, 100, 50, 40, 30, 20, 10 baits) spiked into the first bait set. The second bait set can be mixed at any ratio of individual oligo baits. For example, the second bait set can include individual baits present as a 1:1 equimolar ratio. Alternatively, the second bait set can include individual baits present at a different ratio *(e.g.,* 1:5, 1:10, 1:20), for example, to optimize capture of certain targets *(e.g..* certain targets can have a 5-10X of the second bait compared to other targets).

*SEQUENCING*

The invention also includes methods of sequencing nucleic acids. In these methods, nucleic acid library members are isolated by using the methods described herein. *e.g.,* using solution hybridization, thereby providing a library catch. The library catch or a subgroup thereof can be sequenced. Accordingly, the methods featured in the invention further include analyzing the library catch. In one embodiment, the library catch is analyzed by a sequencing method, *e.g.,* a next-generation sequencing method as described herein. The methods include isolating a library catch by solution hybridization, and subjecting the library catch by nucleic acid sequencing. In certain embodiments, the library catch can be re-sequenced.

Any method of sequencing known in the art can be used. Sequencing of nucleic acids isolated by selection methods are typically carried out using next-generation sequencing (NGS). Sequencing methods suitable for use herein are described in the art, *e.g..* as described in International Patent Application Publication No. WO 2012/092426.

After NGS reads have been generated, they can be aligned to a known reference sequence or assembled *de novo.* For example, identifying genetic variations such as single nucleotide polymorphisms and structural variants in a sample *(e.g.,* a tumor sample) can be accomplished by aligning NGS reads to a reference sequence *(e.g.,* a wild-type sequence). Methods of sequence alignment for NGS are described *e.g.,* in Trapnell C. and Salzberg S.L. Nature Biotech., 2009, 27:455-457. Examples of *de novo* assemblies are described, *e.g.,* in Warren R. et al., Bioinformatics, 2007, 23:500-501; Butler J. et al., Genome Res., 2008, 18:810-820; and Zerbino D.R. and Birney E., Genome Res., 2008, 18:821-829. Sequence alignment or assembly can be performed using read data from one or more NGS platforms, *e.g.,* mixing Roche/454 and Illumina/Solexa read data.

*ALIGNMENT*

Alignment is the process of matching a read with a location, *e.g.,* a genomic location. Misalignment (e.g., the placement of base-pairs from a short read on incorrect locations in the genome)., *e.g.,* misalignment due to sequence context *(e.g.,* presence of repetitive sequence) of reads around an actual cancer mutation can lead to reduction in sensitivity of mutation detection, as reads of the alternate allele may be shifted off the main pile-up of alternate allele reads. If the problematic sequence context occurs where no actual mutation is present, misalignment may introduce artifactual reads of "mutated" alleles by placing actual reads of reference genome bases onto the wrong location. Because mutation-calling algorithms for multiplied multigene analysis should be sensitive to even low-abundance mutations, these misalignments may increase false positive discovery rates/reduce specificity.

As discussed herein, reduced sensitivity for actual mutations may be addressed by evaluating the quality of alignments (manually or in an automated fashion) around expected mutation sites in the genes being analyzed. The sites to be evaluated can be obtained from databases of cancer mutations (*e.g.* COSMIC). Regions that are identified as problematic can be remedied with the use of an algorithm selected to give better performance in the relevant sequence context, *e.g.,* by alignment optimization (or re-alignment) using slower, but more accurate alignment algorithms such as Smith-Waterman alignment. In cases where general alignment algorithms cannot remedy the problem, customized alignment approaches may be created by, *e.g.*, adjustment of maximum difference mismatch penalty parameters for genes with a high likelihood of containing substitutions; adjusting specific mismatch penalty parameters based on specific mutation types that are common in certain tumor types (*e.g.* C→T in melanoma); or adjusting specific mismatch penalty parameters based on specific mutation types that are common in certain sample types (*e.g.* substitutions that are common in FFPE).

Reduced specificity (increased false positive rate) in the evaluated gene regions due to misalignment can be assessed by manual or automated examination of all mutation calls in samples sequenced. Those regions found to be prone to spurious mutation calls due to misalignment can be subjected to same alignment remedies as above. In cases where no algorithmic remedy is found possible, "mutations" from the problem regions can be classified or screened out from the test panel.

Methods disclosed herein allow the use of multiple, individually tuned, alignment methods or algorithms to optimize performance in the sequencing of subgenomic intervals associated with rearrangements, *e.g.*, indels, particularly in methods that rely on massively parallel sequencing of a large number of diverse genetic events in a large number of diverse genes, *e.g.,* from tumor samples. In embodiments multiple alignment methods that are individually customized or tuned to each of a number of rearrangements in different genes are used to analyze reads. In embodiments tuning can be a function of (one or more of) the gene (or other subgenomic interval) being sequenced, the tumor type in the sample, the variant being sequenced, or a characteristic of the sample or the subject. This selection or use of alignment conditions finely tuned to a number of subgenomic intervals to be sequenced allows optimization of speed, sensitivity and specificity. The method is particularly effective when the alignment of reads for a relatively large number of diverse subgenomic intervals is optimized. In embodiments the method includes the use of alignment methods optimized for rearrangements and others optimized for subgenomic intervals not associated with rearrangements.

Thus, in an embodiment, a method described herein, *e.g.,* a method of analyzing a tumor sample comprises an alignment method for rearrangements described herein.

Generally, the accurate detection of indel mutations is an exercise in alignment, as the spurious indel rate on the sequencing platforms disabled herein is relatively low (thus, even a handful of observations of correctly aligned indels can be strong evidence of mutation). Accurate alignment in the presence of indels can be difficult however (especially as indel length increases). In addition to the general issues associated with alignment, *e.g.,* of substitutions, the indel itself can cause problems with alignment. (For instance, a deletion of 2bp of a dinucleotide repeat cannot be readily definitively placed.) Both sensitivity and specificity can be reduced by incorrect placement of shorter (<15bp) apparent indel-containing reads. Larger indels (getting closer in magnitude to the length of individual reads, *e.g.,* reads of 36bp) can cause failure to align the read at all, making detection of the indel impossible in the standard set of aligned reads.

Databases of cancer mutations can be used to address these problems and improve performance. To reduce false positive indel discovery (improve specificity), regions around commonly expected indels can be examined for problematic alignments due to sequence context and addressed similarly to substitutions above. To improve sensitivity of indel detection, several different approaches of using information on the indels expected in cancer can be used. E.g., short-reads contained expected indels can be simulated and alignment attempted. The alignments can be studied and problematic indel regions can have alignment parameters adjusted, for instance by reducing gap open/extend penalties or by aligning partial reads (*e.g.* the first or second half of a read).

Alternatively, initial alignment can be attempted not just with the normal reference genome, but also with alternate versions of the genome, containing each of the known or likely cancer indel mutations. In this approach, reads of indels that initially failed to align or aligned incorrectly are placed successfully on the alternate (mutated) version of the genome.

In this way, indel alignment (and thus calling) can be optimized for the expected cancer genes/sites. As used herein, a sequence alignment algorithm embodies a computational method or approach used to identify from where in the genome a read sequence *(e.g.,* a short-read sequence, *e.g.,* from next-generation sequencing) most likely originated by assessing the similarity between the read sequence and a reference sequence. A variety of algorithms can be applied to the sequence alignment problem. Some algorithms are relatively slow, but allow relatively high specificity. These include, *e.g.,* dynamic programming-based algorithms. Dynamic programming is a method for solving complex problems by breaking them down into simpler steps. Other approaches are relatively more efficient, but are typically not as thorough. These include, *e.g.,* heuristic algorithms and probabilistic methods designed for large-scale database search.

Alignment parameters are used in alignment algorithms to adjust performance of an algorithm, *e.g.,* to produce an optimal global or local alignment between a read sequence and a reference sequence. Alignment parameters can give weights for match, mismatch, and indels. For example, lower weights allow alignments with more mismatches and indels.

Sequence context, *e.g.,* presence of repetitive sequences *(e.g.,* tandem repeats, interspersed repeats), low-complexity regions, indels, pseudogenes, or paralogs can affect the alignment specificity (e.g., cause misalignment). As used herein, misalignment refers to the placement of base-pairs from the short read on incorrect locations in the genome.

The sensitivity of alignment can be increased when an alignment algorithm is selected or an alignment parameter is adjusted based on tumor type. *e.g.,* a tumor type that tends to have a particular mutation or mutation type.

The sensitivity of alignment can be increased when an alignment algorithm is selected or an alignment parameter is adjusted based on a particular gene type (e.g., oncogene, tumor suppressor gene). Mutations in different types of cancer-associated genes can have different impacts on cancer phenotype. For example, mutant oncogene alleles are typically dominant. Mutant tumor suppressor alleles are typically recessive, which means that in most cases both alleles of a tumor suppressor genes must be affected before an effect is manifested.

The sensitivity of alignment can be adjusted *(e.g.,* increased) when an alignment algorithm is selected or an alignment parameter is adjusted based on mutation type *(e.g.,* single nucleotide polymorphism, indel (insertion or deletion), inversion, translocation, tandem repeat).

The sensitivity of alignment can be adjusted (e.g., increased) when an alignment algorithm is selected or an alignment parameter is adjusted based on mutation site *(e.g.,* a mutation hotspot). A mutation hotspot refers to a site in the genome where mutations occur up to 100 times more frequently than the normal mutation rate.

The sensitivity/specificity of alignment can be adjusted (e.g., increased) when an alignment algorithm is selected or an alignment parameter is adjusted based on sample type *(e.g.,* an FFPE sample).

Alignment algorithms can be selected to adjust (e.g., increase) the alignment sensitivity/specificity, based on sample type *(e.g.,* an FFPE sample, a blood sample, or a bone marrow aspirate sample).

Optimization of alignment is described in the art, *e.g.,* as set out in International Patent Application Publication No. WO 2012/092426.

*MUTATION CALLING*

Base calling refers to the raw output of a sequencing device. Mutation calling refers to the process of selecting a nucleotide value, *e.g.,* A, G, T, or C, for a nucleotide position being sequenced. Typically, the sequencing reads (or base calling) for a position will provide more than one value, *e.g.,* some reads will give a T and some will give a G. Mutation calling is the process of assigning a nucleotide value, *e.g.,* one of those values to the sequence. Although it is referred to as "mutation" calling it can be applied to assign a nucleotide value to any nucleotide position, *e.g.,* positions corresponding to mutant alleles, wild-type alleles, alleles that have not been characterized as either mutant or wild-type, or to positions not characterized by variability. Methods for mutation calling can include one or more of the following: making independent calls based on the information at each position in the reference sequence (e.g., examining the sequence reads; examining the base calls and quality scores; calculating the probability of observed bases and quality scores given a potential genotype; and assigning genotypes (e.g., using Bayes rule)); removing false positives (e.g., using depth thresholds to reject SNPs with read depth much lower or higher than expected; local realignment to remove false positives due to small indels); and performing linkage disequilibrium (LD)/imputation based analysis to refine the calls.

Equations to calculate the genotype likelihood associated with a specific genotype and position are described, *e.g.,* in Li H. and Durbin R. Bioinformatics, 2010; 26(5): 589-95. The prior expectation for a particular mutation in a certain cancer type can be used when evaluating samples from that cancer type. Such likelihood can be derived from public databases of cancer mutations, *e.g.,* Catalogue of Somatic Mutation in Cancer (COSMIC), HGMD (Human Gene Mutation Database), The SNP Consortium, Breast Cancer Mutation Data Base (BIC), and Breast Cancer Gene Database (BCGD).

Examples of LD/imputation based analysis are described, *e.g.,* in Browning B.L. and Yu Z. Am. J. Hum. Genet. 2009. 85(6):847-61. Examples of low-coverage SNP calling methods are described, *e.g.,* in Li Y. et al., Annu. Rev. Genomics Hum. Genet. 2009, 10:387-406.

After alignment, detection of substitutions can be performed using a calling method, *e.g.,* Bayesian mutation calling method: which is applied to each base in each of the subgenomic intervals, *e.g.,* exons of the gene to be evaluated, where presence of alternate alleles is observed. This method will compare the probability of observing the read data in the presence of a mutation with the probability of observing the read data in the presence of base-calling error alone. Mutations can be called if this comparison is sufficiently strongly supportive of the presence of a mutation.

Methods have been developed that address limited deviations from frequencies of 50% or 100% for the analysis of cancer DNA. (e.g., SNVMix -Bioinformatics. 2010 March 15; 26(6): 730-736.) Methods disclosed herein however allow consideration of the possibility of the presence of a mutant allele in anywhere between 1% and 100% of the sample DNA, and especially at levels lower than 50%. This approach is particularly important for the detection of mutations in low-purity FFPE samples of natural (multi-clonal) tumor DNA.

An advantage of a Bayesian mutation-detection approach is that the comparison of the probability of the presence of a mutation with the probability of base-calling error alone can be weighted by a prior expectation of the presence of a mutation at the site. If some reads of an alternate allele are observed at a frequently mutated site for the given cancer type, then presence of a mutation may be confidently called even if the amount of evidence of mutation does not meet the usual thresholds. This flexibility can then be used to increase detection sensitivity for even rarer mutations/lower purity samples, or to make the test more robust to decreases in read coverage. The likelihood of a random base-pair in the genome being mutated in cancer is ~1e-6. The likelihood of specific mutations at many sites in a typical multigenic cancer genome panel can be orders of magnitude higher. These likelihoods can be derived from public databases of cancer mutations (e.g., COSMIC). Indel calling is a process of finding bases in the sequencing data that differ from the reference sequence by insertion or deletion, typically including an associated confidence score or statistical evidence metric.

Methods of indel calling can include the steps of identifying candidate indels, calculating genotype likelihood through local re-alignment, and performing LD-based genotype inference and calling. Typically, a Bayesian approach is used to obtain potential indel candidates, and then these candidates are tested together with the reference sequence in a Bayesian framework.

Algorithms to generate candidate indels are described. *e.g.,* in McKenna A. et al., Genome Res. 2010; 20(9):1297-303; Ye K. et al., Bioinformatics, 2009; 25(21):2865-71; Lunter G. and Goodson M. Genome Res. 2010, epub ahead of print; and Li H. et al., Bioinformatics 2009, Bioinformatics 25(16):2078-9.

Methods for generating indel calls and individual-level genotype likelihoods include, *e.g.,* the Dindel algorithm (Albers C.A. et al., Genome Res. 2011;21(6):961-73). For example, the Bayesian EM algorithm can be used to analyze the reads, make initial indel calls, and generate genotype likelihoods for each candidate indel, followed by imputation of genotypes using, *e.g.,* QCALL (Le S.Q. and Durbin R. Genome Res. 2011;21(6):952-60). Parameters, such as prior expectations of observing the indel can be adjusted (e.g., increased or decreased), based on the size or location of the indels.

Optimization of mutation calling is described in the art, *e.g.*, as set out in International Patent Application Publication No. WO 2012/092426.

*SGZ ALGORITHM*

Various types of alterations, *e.g.*, somatic alterations and germline mutations, can be detected by a method (e.g., a sequencing, alignment, or mutation calling method) described herein. In certain embodiments, a germline mutation is further identified by a method using the SGZ algorithm. The SGZ algorithm is described in Sun et al. Cancer Research 2014; 74(19S):1893-1893; International Application Publication No. WO2014/183078 and U.S. Application Publication No. 2014/0336996.

*OTHER EMBODIMENTS*

In embodiments of a method described herein a step or parameter in the method is used to modify a downstream step or parameter in the method.

In an embodiment, a characteristic of the tumor sample is used to modify a downstream step or parameter in one or more or all of: isolation of nucleic acid from said sample; library construction; bait design or selection; hybridization conditions; sequencing; read mapping; selection of a mutation calling method; mutation calling; or mutation annotation.

In an embodiment, a characteristic of an isolated tumor, or control, nucleic acid is used to modify a downstream step or parameter in one or more or all of: isolation of nucleic acid from said sample; library construction; bait design or selection; hybridization conditions; sequencing; read mapping; selection of a mutation calling method; mutation calling; or mutation annotation.

In an embodiment, a characteristic of a library is used to modify a downstream step or parameter in one or more or all of: re-isolation of nucleic acid from said sample; subsequent library construction; bait design or selection; hybridization conditions; sequencing; read mapping; selection of a mutation calling method; mutation calling; or mutation annotation.

In an embodiment, a characteristic of a library catch is used to modify a downstream step or parameter in one or more or all of: re-isolation of nucleic acid from said sample; subsequent library construction; bait design or selection; hybridization conditions; sequencing; read mapping; selection of a mutation calling method; mutation calling; or mutation annotation.

In an embodiment, a characteristic of the sequencing method is used to modify a downstream step or parameter in one or more or all of: re-isolation of nucleic acid from said sample; subsequent library construction; bait design or selection; subsequent determination of hybridization conditions subsequent sequencing; read mapping; selection of a mutation calling method; mutation calling; or mutation annotation.

In an embodiment, characteristic of the collection of mapped reads is used to modify a downstream step or parameter in one or more or all of: re-isolation of nucleic acid from said sample; subsequent library construction; bait design or selection; subsequent determination of hybridization conditions subsequent sequencing; subsequent read mapping; selection of a mutation calling method; mutation calling; or mutation annotation.

In an embodiment, the method comprises acquiring a value for a tumor sample characteristic, *e.g.*, acquiring a value: for the proportion of tumor cells in said sample; for the cellularity of said tumor sample; or from an image of the tumor sample.

In embodiments, the method includes, responsive to said acquired value for a tumor sample characteristic, selecting a parameter for: isolation of nucleic acid from a tumor sample, library construction; bait design or selection; bait/library member hybridization; sequencing; or mutation calling.

In an embodiment, a method further comprising acquiring a value for the amount of tumor tissue present in said tumor sample, comparing said acquired value with a reference criterion, and if said reference criterion is met, accepting said tumor sample, *e.g.*, accepting said tumor sample if said tumor sample contains greater than 30, 40 or 50% tumor cells.

In an embodiment, a method further comprises acquiring a sub-sample enriched for tumor cells. *e.g.*, by macrodissecting tumor tissue from said tumor sample, from a tumor sample that fails to meet the reference criterion.

In an embodiment, a method further comprises determining if a primary control, *e.g.*, a blood sample, is available and if so isolating a control nucleic acid *(e.g.*, DNA) from said primary control.

In an embodiment, a method further comprises determining if NAT is present in said tumor sample *(e.g.*, where no primary control sample is available).

In an embodiment, a method further comprises acquiring a sub-sample enriched for non-tumor cells, *e.g.*, by macrodissecting non-tumor tissue from said NAT in a tumor sample not accompanied by a primary control.

In an embodiment, a method further comprises determining that no primary control and no NAT is available and marking said tumor sample for analysis without a matched control.

In an embodiment, a method further comprises isolating nucleic acid from said tumor sample to provide an isolated tumor nucleic acid sample.

In an embodiment, a method further comprises isolating a nucleic acid from a control to provide an isolated control nucleic acid sample.

In an embodiment, a method further comprises rejecting a sample with no detectable nucleic acid.

In an embodiment, a method further comprises acquiring a value for nucleic acid yield in said isolated nucleic acid sample and comparing the acquired value to a reference criterion, *e.g.*, wherein if said acquired value is less than said reference criterion, then amplifying said isolated nucleic acid sample prior to library construction.

In an embodiment, a method further comprises acquiring a value for the size of nucleic acid fragments in said isolated nucleic acid sample and comparing the acquired value to a reference criterion, *e.g.*, a size. *e.g.*, average size, of at least 300, 600. or 900 bps. A parameter described herein can be adjusted or selected in response to this determination.

In an embodiment, a method further comprises acquiring a library wherein the size of said nucleic acid fragments in the library are less than or equal to a reference value, and said library is made without a fragmentation step between DNA isolation and making the library.

In an embodiment, a method further comprises acquiring nucleic acid fragments and if the size of said nucleic acid fragments are equal to or greater than a reference value and are fragmented and then such nucleic acid fragments are made into a library.

In an embodiment, a method further comprises labeling each of a plurality of library members, *e.g.*, by addition of an identifiable distinct nucleic acid sequence (a barcode), to each of a plurality of members.

In an embodiment, a method further comprises attaching a primer to each of a plurality of library members.

In an embodiment, a method further comprises providing a plurality of baits and selecting a plurality of baits, said selection being responsive to: 1) a patient characteristic, *e.g.*, age, stage of tumor, prior treatment, or resistance; 2) tumor type; 3) a characteristic of the tumor sample; 4) a characteristic of a control sample; 5) presence or type of control; 6) a characteristic of the isolated tumor (or control) nucleic acid sample; 7) a library characteristic; 8) a mutation known to be associated with the type of tumor in the tumor sample; 9) a mutation not known to be associated with the type of tumor in the tumor sample; 10) the ability to sequence (or hybridized to or recover) a preselected sequence or identify a preselected mutation, *e.g.*, the difficulty associated with sequence having a high GC region or a rearrangement; or 11) the genes being sequenced.

In an embodiment, a method further comprises responsive, *e.g.*, to a determination of a low number of tumor cells in said tumor sample, selecting a bait, or plurality of baits, giving relatively highly efficient capture of members from a first gene as compared with members of a second gene, *e.g.*, wherein a mutation in the first gene is associated the tumor phenotype for the tumor type of the tumor sample.

In an embodiment, a method further comprises acquiring a value for a library catch characteristic, *e.g.,* the nucleic acid concentration or representation, and comparing the acquired value with a reference criterion for nucleic acid concentration, or for representation.

In an embodiment, a method further comprises selecting a library with a value for a library characteristic that does not meet the reference criterion for reworking (*e.g.*, for changing the value to meet the reference criterion).

In an embodiment, a method further comprises selecting a library with a value for a library characteristic that meets the reference criterion for library quantitation.

In an embodiment, a method further comprises providing an association of a tumor type, a gene, and a genetic alteration (a TGA) for a subject.

In an embodiment, a method further comprises providing a preselected database having a plurality of elements, wherein each element comprises a TGA.

In an embodiment, a method further comprises characterizing a TGA of a subject comprising: determining if said TGA is present in a preselected database, *e.g.*, a database of validated TGAs; associating information for the TGA from the preselected database with said TGA (annotating) from said subject; and optionally, determining if a second or subsequent TGA for said subject is present in said preselected database and if so associating information for the second or subsequent TGA from the preselected database with said second TGA present in said patient.

In an embodiment, a method further comprises memorializing the presence or absence of a TGA. and optionally an associated annotation, of a subject to form a report.

In an embodiment, a method further comprises transmitting said report to a recipient party.

In an embodiment, a method further comprises characterizing a TGA of a subject comprising: determining if said TGA is present in a preselected database, *e.g.,* a database of validated TGAs; or determining if a TGA not in said preselected database has a known clinically relevant G or A and if so providing an entry for said TGA in said preselected database.

In an embodiment, a method further comprises memorializing the presence or absence of a mutation found in the DNA of the tumor sample from a subject to form a report.

In an embodiment, a method further comprises memorializing the presence or absence of a TGA. and optionally an associated annotation, of a subject to form a report.

In an embodiment, a method further comprises transmitting said report to a recipient party.

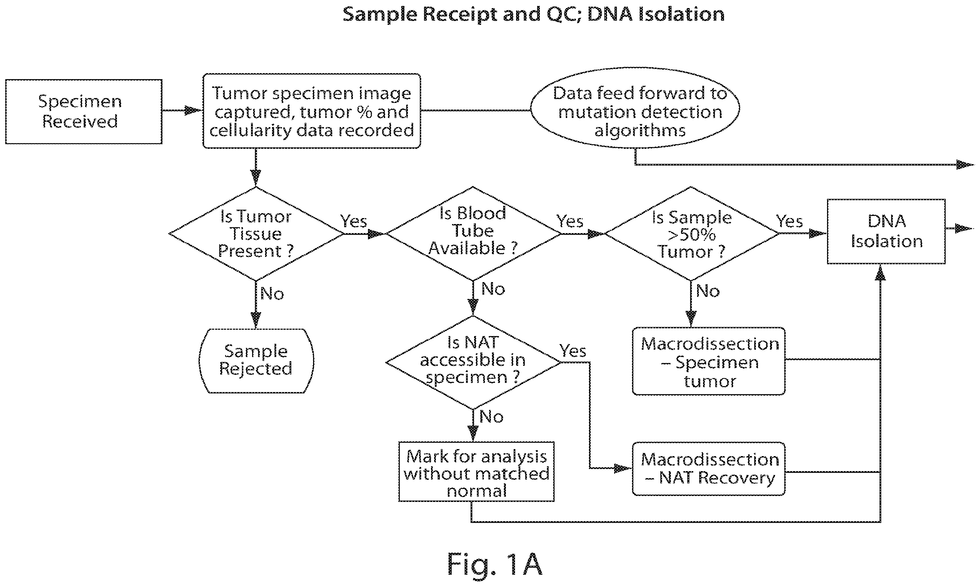

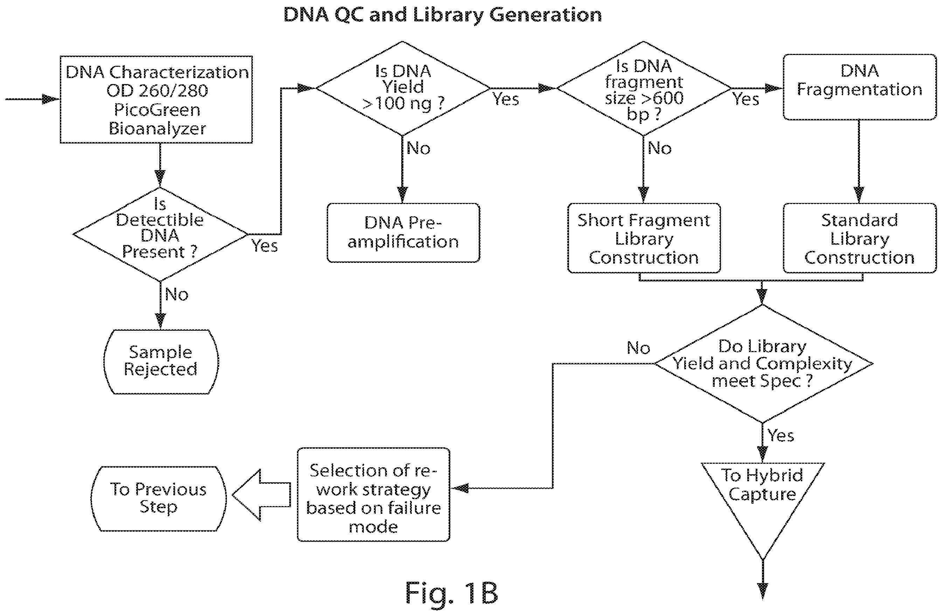

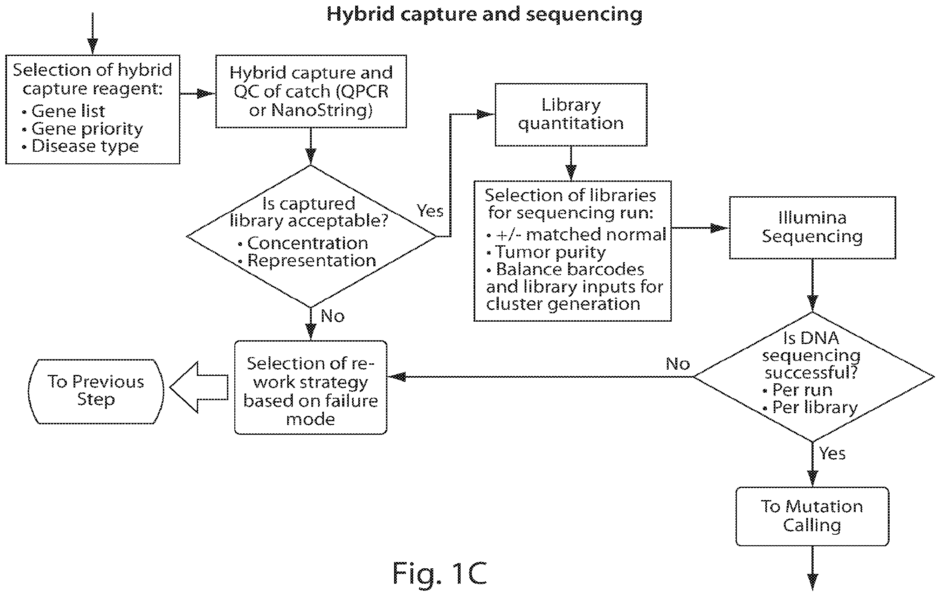

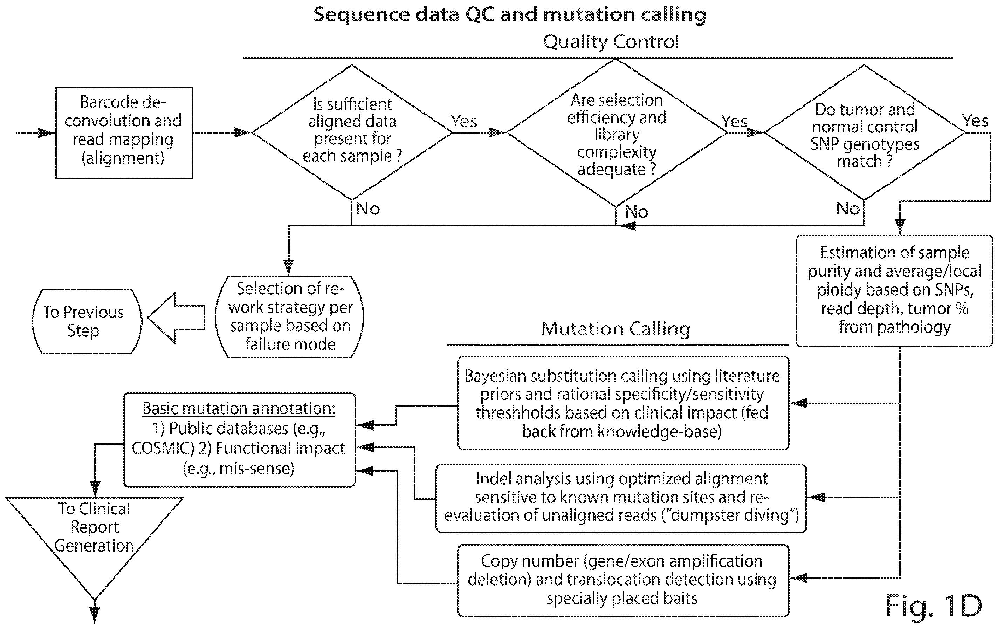

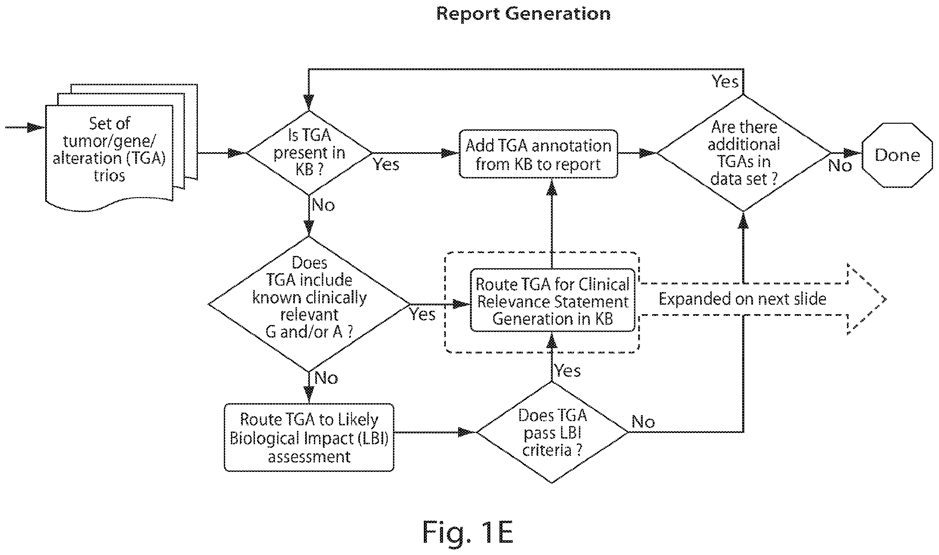

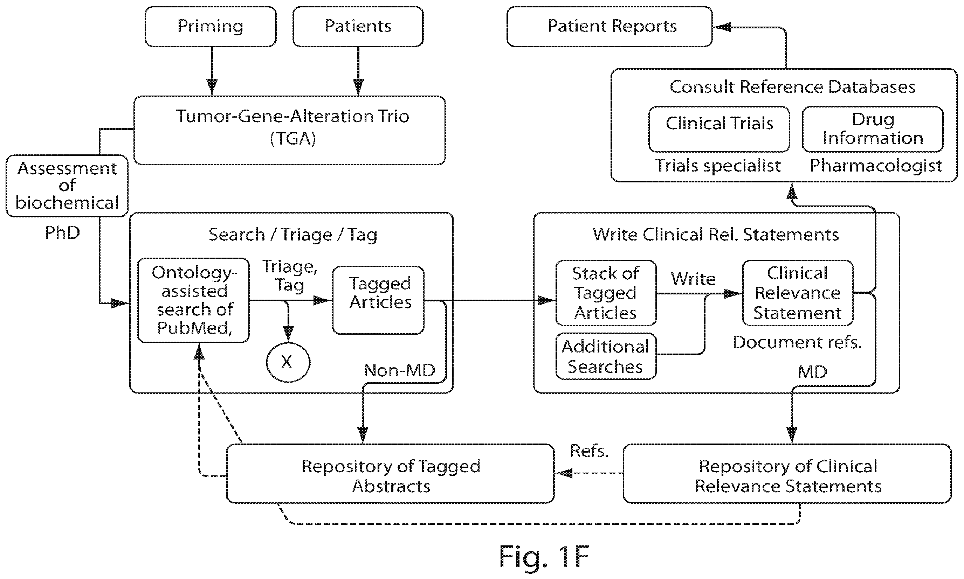

A flowchart depiction of an embodiment of a method for multigene analysis of a tumor sample is provided in **FIGs. 1A-1F**.

The disclosure includes **Table 5** (Appendix A), which is part of the specification.

## EXAMPLES

This invention is further illustrated by the following examples which should not be construed as limiting.

EXAMPLE 1: COMPARISON OF WHOLE GENOME MUTATIONAL BURDEN WITH MUTATIONAL BURDEN MEASURED BY TARGETED GENES

In this example, whether TMB, as measured by a comprehensive genomic profiling (CGP) test targeting 315 genes (1.1 Mb of coding genome), could provide an accurate assessment of whole exome TMB, was determined. Accurate measurement of TMB by a targeted comprehensive genomic profiling test was demonstrated.

*Methods*

*Analysis of TCGA data*

TCGA data was obtained from public repositories (Cancer Genome Atlas Research Network et al. Nat Genet 2013; 45:1113-20). For this analysis, the somatic called variants as determined by TCGA were used as the raw mutation count. 38 Mb was used as the estimate of the exome size. For the downsampling analysis, the observed number of mutations/Mb was simulated 1000 times using the binomial distribution at whole exome TMB = 100 mutations/Mb, 20 mutations/Mb, and 10 mutations/Mb for various portions of the exome ranging from 0-10 Mb per portion. Melanoma TCGA data was obtained from dbGap accession number phs000452.v1.p1 (Berger et al. Nature 2012; 485:502-6).

*Tumor Mutational Burden*

Without wishing to be bound by theory, in this example, tumor mutational burden was determined as follows. The tumor mutational burden was measured as the number of somatic, coding, base substitution and indel mutations, per megabase of genome examined. All base substitutions and indels in the coding region of targeted genes, including synonymous alterations, were initially counted before filtering as described below. Synonymous mutations were counted in order to reduce sampling noise. While synonymous mutations are not likely to be directly involved in creating immunogenicity, their presence is a signal of mutational processes that also result in nonsynonymous mutations and neoantigens elsewhere in the genome. Non-coding alterations were not counted. Alterations listed as known somatic alterations in COSMIC and truncations in tumor suppressor genes were not counted, since the tested genes are biased toward genes with functional mutations in cancer (Bamford et al. Br J Cancer 2004; 91:355-8). Alterations predicted to be germline by the somatic-germline-zygosity (SGZ) algorithm were not counted (Sun et al. Cancer Research 2014; 74(19S):1893-1893). Alterations that were recurrently predicted to be germline in the cohort of clinical specimens were not counted. Known germline alterations in dbSNP were not counted. Germline alterations occurring with two or more counts in the ExAC database were not counted (Lek et al. Nature 2016; 536:285-91). To calculate the TMB per megabase, the total number of mutations counted was divided by the size of the coding region of the targeted territory. The nonparametric Mann-Whitney U-test was subsequently used to test for significance in difference of means between two populations.

*Results*

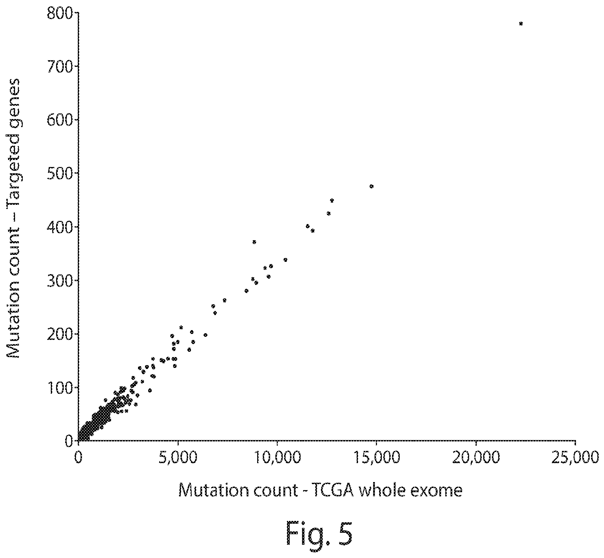

An initial analysis of publicly available TCGA whole exome sequencing dataset (The Cancer Genome Atlas; cancergenome.nih.gov) was performed to determine whether mutational burden measured using targeted genes (*e.g.,* the genes set forth in **FIGs. 3A-3B**) would provide an accurate assessment of whole exome mutational burden. Full mutation call data for 7,001 specimens from 35 distinct studies/diseases were downloaded from TCGA. The number of somatic coding mutations was counted for the whole exome dataset and the number of these mutations occurring in the genes targeted by the test using the genes set forth in **FIGs 3A-3B.** These data are presented in **Table 5** (Appendix A) and/or scatter plot shown in **FIGs. 5-6**. Mutational burden from whole exome is correlated with mutational burden from the genes set forth in **FIGs. 3A-3B** only with a coefficient of determination (R squared) of 0.974.

Further analysis included whole-exome sequencing data from 35 studies, published as part of The Cancer Genome Atlas, examining a total of 8,917 cancer specimens (Cancer Genome Atlas Research Network et al. Nat Genet 2013; 45:1113-20). The number of mutations was determined in total and compared to the number of mutations in the 315 genes targeted by the test. These results were also highly correlated as well ($R^2$=0.98).

These results demonstrate that whole exome mutational burden can be accurately assessed using CGP targeting the entire coding region of several hundred genes (e.g., using only the data from genes targeted by the test using the genes set forth in **FIGs. 3A-3B).**

To summarize, this study shows that tumor mutation burden calculated using a 1.1 Mb comprehensive genomic profiling assay agrees well with whole exome measures of mutation burden. This indicates that CGP, targeting the entire coding region of several hundred genes, covers sufficient genomic space to accurately assess whole exome mutational burden. It was found that filtering out germline alterations and rare variants could be used to obtain accurate measurements of TMB, and this can especially be useful in patients from ethnic backgrounds not well represented in sequencing datasets. These findings indicate that CGP is an accurate, cost-effective, and clinically available tool for measuring TMB. The results of the downsampling analysis show that the variation in measurement due to sampling when sequencing 1.1 Mb is acceptably low, resulting in highly accurate calling of TMB at a range of TMB levels. This sampling variation increases as the number of Mb sequenced decreases, especially at lower levels of TMB.

EXAMPLE 2: THE LANDSCAPE OF MUTATION BURDEN ACROSS CANCER TYPES

In this example, the distribution of TMB was described across a diverse cohort of ≥100,000 cancer specimens, and association between somatic alterations and TMB was tested for in over 100 tumor types. A subset of patients were found to exhibit high TMB across almost all cancer disease types, including many rare tumor types. It was found that TMB increases significantly with age, showing a 2.4-fold difference between age 10 and age 90. Using a CGP assay targeting ~1.1 Mb of coding genome, it was found that there are many disease types with a substantial portion of patients with high TMB who might benefit from immunotherapy.

This study provides better understanding of the landscape of TMB across the spectrum of human cancer based on data from comprehensive genomic profiling (CGP) of >100,000 patient tumors of diverse type. The analysis described in this Example expands significantly upon existing data that quantifies mutation burden in cancer, providing data for many previously undescribed cancer types. New data were provided to support rational expansion of the patient population that could benefit from immunotherapy and allow informed design of clinical trials of immunotherapy agents in untested cancer types.

*Methods*

*Comprehensive Genomic Profiling*

CGP was performed as previously described in detail (Frampton et al. Nat Biotech 2013; 31:1023-1031; He et al. Blood 2016; 127:3004-14; FoundationOne assay (Cambridge, MA. USA)). Briefly, the pathologic diagnosis of each case was confirmed by review of hematoxylin and eosin (H&E) stained slides and all samples that advanced to DNA extraction contained a minimum of 20% tumor cells. Hybridization capture of exonic regions from 185, 236. 315, or 405 cancer-related genes and select introns from 19, 28, or 31 genes commonly rearranged in cancer was applied to $\geq$ 50 ng of DNA extracted from formalin-fixed paraffin-embedded clinical cancer specimens. These libraries were sequenced to high, uniform median coverage (>500x) and assessed for base substitutions, short insertions and deletions, copy number alterations and gene fusions/rearrangements (Frampton et al. Nat Biotech 2013; 31:1023-1031). Data from each of three versions of the assay was used in the analysis.

*Tumor Mutational Burden*

Without wishing to be bound by theory, in this example, tumor mutational burden was determined as described in Example 1.

*Cohort Selection*

From an initial clinical cohort of 102,292 samples, duplicate assay results from the same patient were excluded, and samples with less than 300x median exon coverage were excluded to make an analysis set of 92,439 samples. For analyses by cancer type, they had to contain a minimum of 50 unique specimens following sample level filtering.

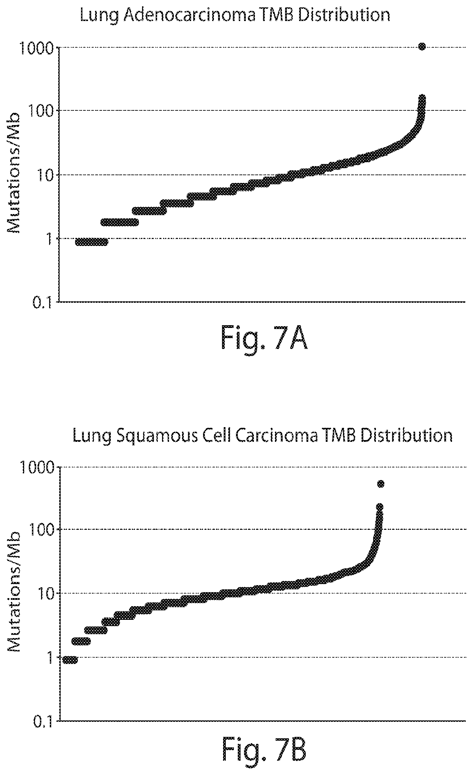

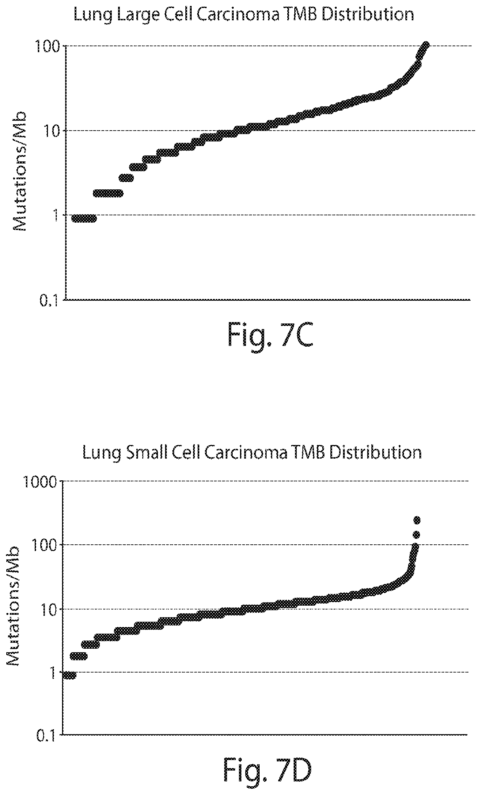

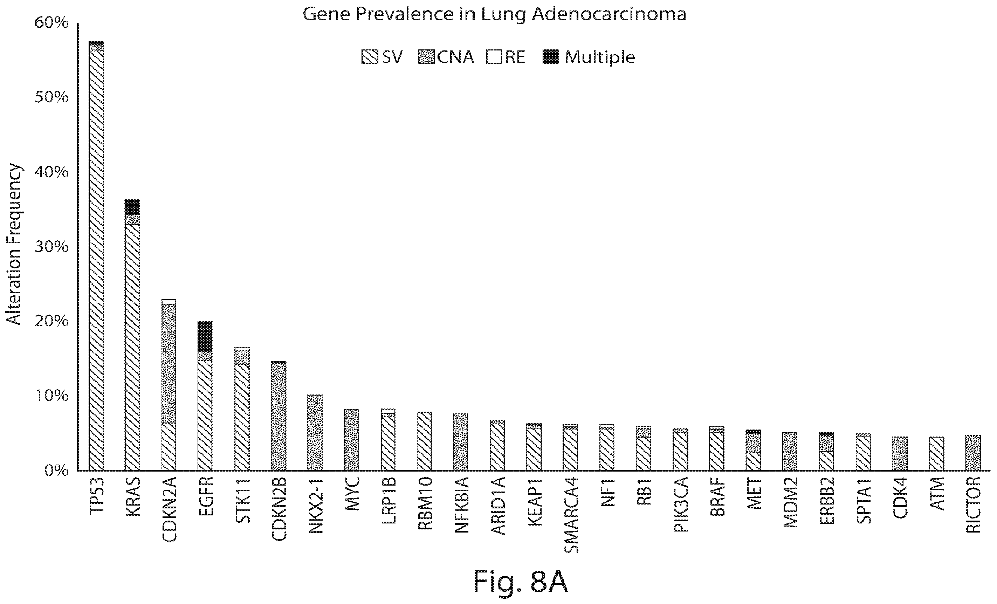

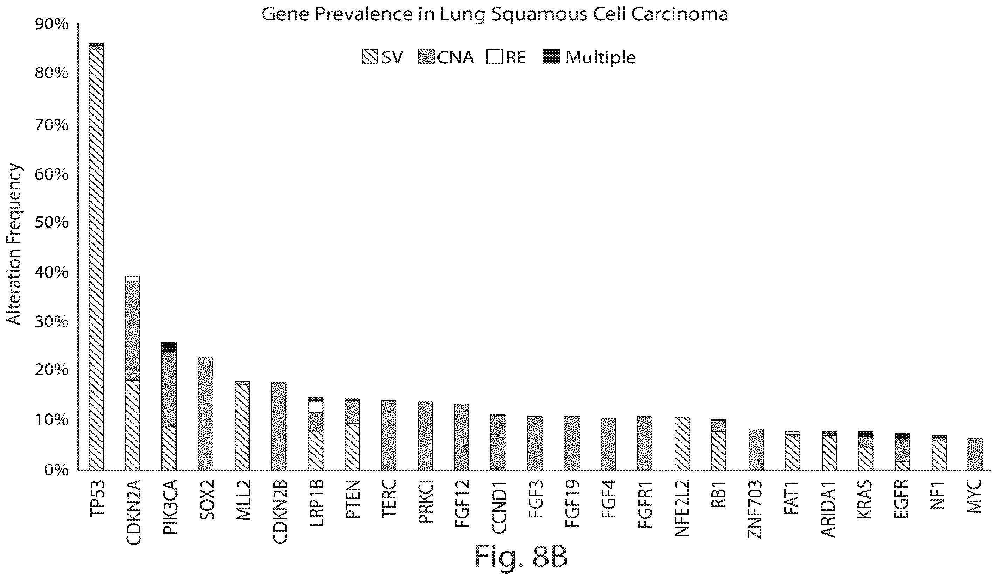

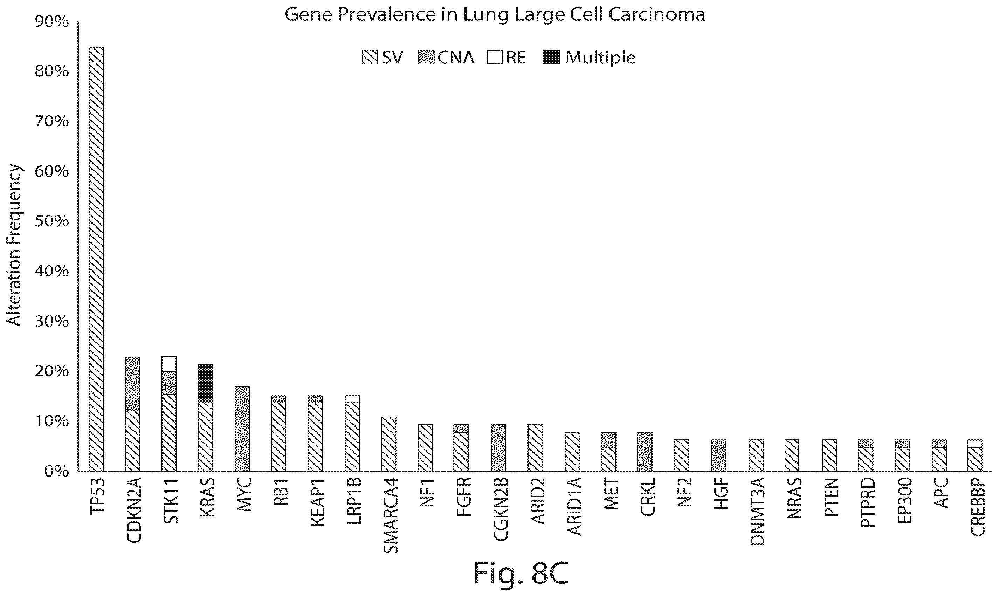

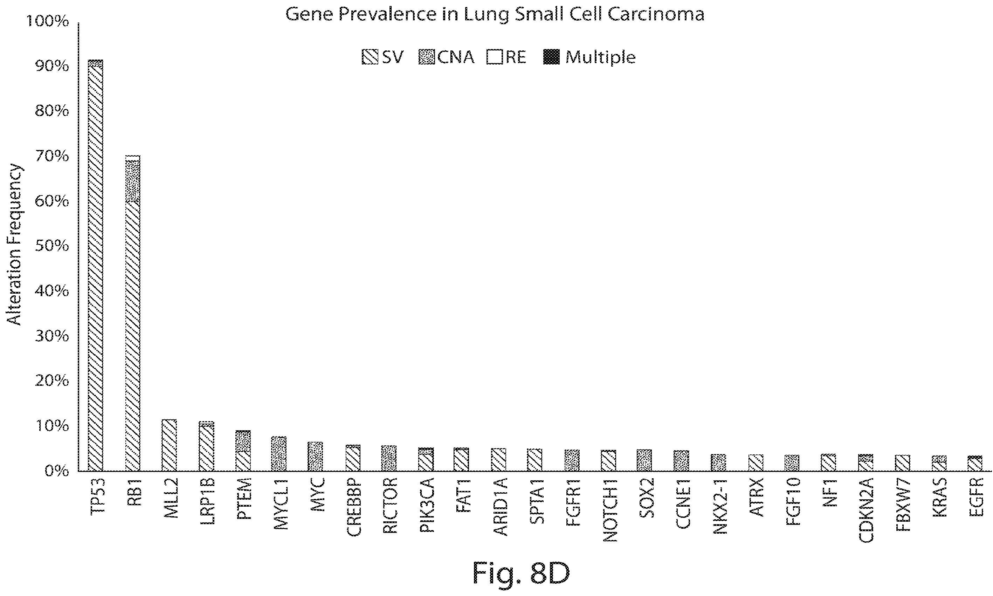

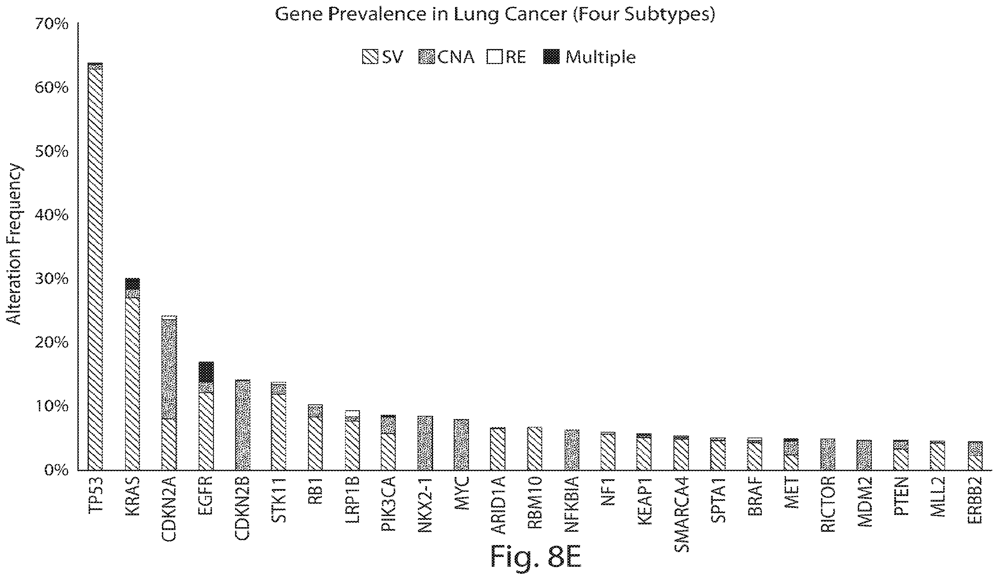

The landscape of TMB was examined across the cohort of patients profiled in the laboratory. CGP was performed in the course of routine clinical care for 102,292 cancer patients (*see* the "Methods" section of this Example). The unique patient cohort contained 41,964 male and 50.376 female patients. Median patient age at the time of specimen collection was 60 years (range: <1 year to >89 years), and 2.5 percent of cases were from pediatric patients under 18 years old. This body of data provided 541 distinct cancer types for analysis. Notably, the majority of specimens were from patients with significantly pretreated, advanced and metastatic disease. Across the entire dataset, the median mutation burden was 3.6 mutations/Mb, with a range of 0-1,241 mutations/Mb. This agrees well with previous estimates of mutation burden from whole exome studies (Alexandrov et al. Nature 2013; 500:415-21; Lawrence et al. Nature 2013; 499:214-8). A significant increase in TMB associated with increased age $(p < 1 \times 10^{-16})$ was found, though the effect size was small (FIG. 7). Median TMB at age 10 was 1.67 mutations/Mb, and median TMB at age 88 was 4.50 mutations/Mb. A linear model fit to the data predicted a 2.4-fold difference in TMB between age 10 and age 90, consist with the median TMB differences at these ages. There was no statistically significant difference in median mutation burden between female and male patients (FIG. 8A).

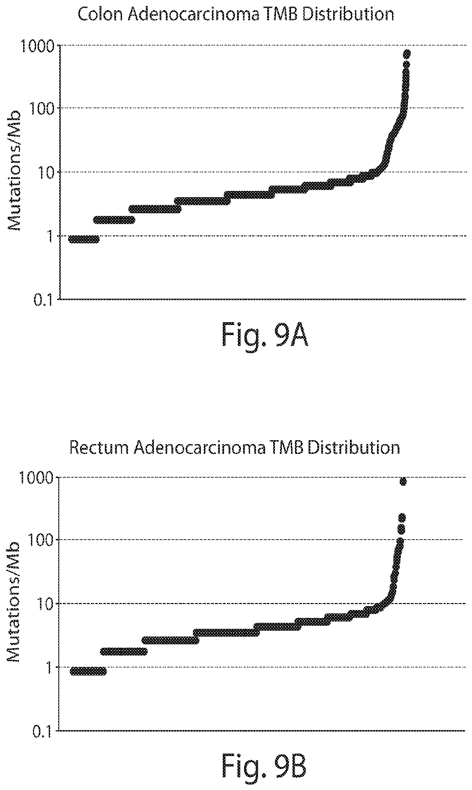

TMB was examined for 167 distinct cancer types for which more than 50 specimens had been tested **(FIG. 9, Table 6).** The median TMB ranged widely, from 0.8 mutations/Mb in bone marrow myelodysplastic syndrome to 45.2 mutations/Mb in skin squamous cell carcinoma. It was found that pediatric malignancies (patient age less than 18 years) had lower TMB (median 1.7 mutations/Mb) than adult malignancies (median 3.6 mutations/Mb). Disease types common in pediatric patients such as leukemia, lymphoma, and neuroblastoma had low TMB, as did sarcomas **(Table 6).**

**Table 6**. Summary of TMB properties by disease

| Disease type | Specimen count | Median TMB | Maximum TMB | Percent cases with >20 mutations/Mb | 95% CI* |
|---|---|---|---|---|---|
| skin basal cell carcinoma | 92 | 47.3 | 215.3 | 70.7 | 59.5 - 78 |
| skin squamous cell carcinoma (scc) | 266 | 45.2 | 409.0 | 67.3 | 61.1-72.3 |
| skin melanoma | 879 | 14.4 | 632.4 | 39.7 | 36.5 - 43 |
| unknown primary melanoma | 1324 | 12.6 | 469.4 | 37.6 | 35 - 40.3 |
| lung large cell carcinoma | 74 | 12.2 | 56.8 | 24.3 | 14.9 - 33.7 |

(continued)

| Disease type | Specimen count | Median TMB | Maximum TMB | Percent cases with >20 mutations/Mb | 95% CI* |
|---|---|---|---|---|---|
| lymph node lymphoma diffuse large b cell | 348 | 10.0 | 251.2 | 18.4 | 14.4 - 22.5 |
| lung small cell undifferentiated carcinoma | 913 | 9.9 | 227.9 | 9.0 | 7.3 - 11 |
| lung large cell neuroendocrine carcinoma | 288 | 9.9 | 98.2 | 19.8 | 15.6 - 24.8 |
| lung squamous cell carcinoma (scc) | 2102 | 9.0 | 521.6 | 11.3 | 10 - 12.7 |
| lymph node lymphoma follicular lymphoma | 107 | 8.3 | 26.7 | 3.7 | 1.5 - 9.2 |
| bladder carcinoma (nos) | 77 | 8.1 | 36.9 | 14.3 | 7.2 - 22.3 |
| lung non-small cell lung carcinoma (nos) | 2636 | 8.1 | 173.9 | 17.0 | 15.6 - 18.5 |
| unknown primary squamous cell carcinoma (sec) | 606 | 7.6 | 344.1 | 21.6 | 18.5 - 25.1 |
| bladder urothelial (transitional cell) carcinoma | 1218 | 7.2 | 119.8 | 11.9 | 10.2 - 13.8 |
| unknown primary urothelial carcinoma | 188 | 7.2 | 107.2 | 13.8 | 9.2 - 18.9 |
| lung sarcomatoid carcinoma | 130 | 7.2 | 165.2 | 19.2 | 13.4 - 26.8 |
| unknown primary undifferentiated small cell carcinoma | 117 | 6.3 | 56.8 | 8.5 | 4.7 - 15 |
| head and neck melanoma | 59 | 6.3 | 170.3 | 25.4 | 16.1 - 37.8 |
| lung adenocarcinoma | 11855 | 6.3 | 755.0 | 12.3 | 11.8 - 12.9 |
| lymph node lymphoma b-cell (nos) | 88 | 6.3 | 37.6 | 11.4 | 5.5 - 18.3 |
| rectum squamous cell carcinoma (scc) | 52 | 5.9 | 33.3 | 3.8 | 0.1 - 10.1 |
| lung adenosquamous carcinoma | 154 | 5.4 | 73.0 | 12.3 | 8 - 18.5 |
| anus squamous cell carcinoma (scc) | 232 | 5.4 | 55.9 | 5.6 | 3 - 8.8 |
| cervix squamous cell carcinoma (sec) | 284 | 5.4 | 73.0 | 6.7 | 4 - 9.8 |
| kidney urothelial carcinoma | 224 | 5.4 | 499.1 | 6.3 | 3.8 - 10.2 |
| unknown primary sarcomatoid carcinoma | 64 | 5.4 | 123.6 | 15.6 | 8.7 - 26.4 |
| ureter urothelial carcinoma | 88 | 5.4 | 45.9 | 6.8 | 2.5 - 12.6 |
| vulva squamous cell carcinoma (scc) | 72 | 5.2 | 41.6 | 4.2 | 1.4 - 11.5 |
| stomach adenocarcinoma intestinal type | 58 | 5.0 | 82.0 | 19.0 | 10.9 - 30.9 |
| gastroesophageal junction adenocarcinoma | 1498 | 5.0 | 865.8 | 2.5 | 1.8 - 3.4 |
| esophagus carcinoma (nos) | 67 | 5.0 | 38.7 | 3.0 | 0.8 - 10.2 |

(continued)

| Disease type | Specimen count | Median TMB | Maximum TMB | Percent cases with >20 mutations/Mb | 95% CI* |
|---|---|---|---|---|---|
| head and neck squamous cell carcinoma (hnscc) | 1184 | 5.0 | 334.2 | 10.1 | 8.5 - 11.9 |
| esophagus squamous cell carcinoma (see) | 219 | 5.0 | 53.2 | 1.8 | 0.5 - 3.9 |
| uterus endometrial adenocarcinoma endometrioid | 459 | 4.5 | 615.3 | 18.5 | 15 - 22.1 |
| nasopharynx and paranasal sinuses squamous cell carcinoma (scc) | 67 | 4.5 | 48.6 | 9.0 | 4.2 - 18.2 |
| bladder adenocarcinoma | 80 | 4.5 | 36.9 | 2.5 | 0.7 - 8.7 |
| colon adenocarcinoma (crc) | 7758 | 4.5 | 752.3 | 5.3 | 4.8 - 5.8 |
| penis squamous cell carcinoma (scc) | 60 | 4.5 | 36.6 | 6.7 | 2.6 - 15.9 |
| small intestine adenocarcinoma | 277 | 4.5 | 278.4 | 8.3 | 5.3 - 11.7 |
| unknown primary carcinoma (nos) | 1405 | 4.5 | 445.9 | 10.7 | 9.2 - 12.5 |
| uterus endometrial adenocarcinoma (nos) | 743 | 4.5 | 636.0 | 14.7 | 12.3 - 17.4 |
| skin merkel cell carcinoma | 206 | 4.3 | 228.8 | 37.9 | 31.1 - 44.2 |
| head and neck carcinoma (nos) | 69 | 3.8 | 95.8 | 5.8 | 2.3 - 14 |
| unknown primary malignant neoplasm (nos) | 491 | 3.8 | 607.2 | 14.9 | 11.8 - 18.1 |
| breast carcinoma (nos) | 4722 | 3.8 | 135.1 | 3.1 | 2.6 - 3.6 |
| colon neuroendocrine carcinoma | 140 | 3.7 | 63.1 | 4.3 | 1.5 - 8.1 |
| uterus carcinosarcoma | 245 | 3.6 | 311.7 | 3.3 | 1.7 - 6.3 |
| skin adnexal carcinoma | 74 | 3.6 | 238.3 | 12.2 | 6.5 - 21.5 |
| unknown primary adenocarcinoma | 2751 | 3.6 | 418.6 | 6.9 | 6 - 7.9 |
| ovary epithelial carcinoma (nos) | 823 | 3.6 | 308.1 | 2.1 | 1.3 - 3.3 |
| stomach adenocarcinoma (nos) | 962 | 3.6 | 131.5 | 5.5 | 4.2 - 7.1 |
| duodenum adenocarcinoma | 249 | 3.6 | 126.1 | 6.0 | 3.4 - 9.2 |
| prostate undifferentiated carcinoma | 91 | 3.6 | 83.8 | 7.7 | 3.8 - 15 |
| breast invasive ductal carcinoma (idc) | 4297 | 3.6 | 261.3 | 1.4 | 1 - 1.7 |
| cervix adenocarcinoma | 195 | 3.6 | 60.4 | 3.6 | 1.4 - 6.5 |
| liver hepatocellular carcinoma (hcc) | 602 | 3.6 | 65.6 | 1.0 | 0.5 - 2.2 |
| ovary carcinosarcoma | 134 | 3.6 | 650.5 | 2.2 | 0.8 - 6.4 |
| ovary high grade serous carcinoma | 348 | 3.6 | 12.6 | 0.0 | 0 - 1.1 |
| brain gliosarcoma | 51 | 3.6 | 157.7 | 2.0 | 0.1 - 10.3 |
| fallopian tube serous carcinoma | 188 | 3.6 | 17.1 | 0.0 | 0 - 2 |
| gallbladder adenocarcinoma | 511 | 3.6 | 418.6 | 2.2 | 1.2 - 3.8 |

(continued)

| Disease type | Specimen count | Median TMB | Maximum TMB | Percent cases with >20 mutations/Mb | 95% CI* |
|---|---|---|---|---|---|
| ovary endometrioid adenocarcinoma | 105 | 3.6 | 69.4 | 8.6 | 4.6 - 15.5 |
| rectum adenocarcinoma (crc) | 1318 | 3.6 | 851.4 | 2.2 | 1.5 - 3.1 |
| salivary gland carcinoma (nos) | 160 | 3.6 | 218.0 | 6.3 | 3.4 - 11.1 |
| uterus endometrial adenocarcinoma clear cell | 62 | 3.6 | 72.1 | 8.1 | 2.5 - 15.4 |
| prostate neuroendocrine carcinoma | 99 | 3.6 | 370.3 | 5.1 | 2.2 - 11.3 |
| lymph node lymphoma mantle cell | 75 | 3.3 | 14.2 | 0.0 | 0 - 4.9 |
| lymph node lymphoma t-cell (nos) | 91 | 3.3 | 145.2 | 4.4 | 1.1 - 9.2 |
| soft tissue angiosarcoma | 157 | 3.3 | 157.7 | 13.4 | 8.4 - 18.9 |
| bone marrow multiple myeloma | 1580 | 3.3 | 60.9 | 1.2 | 0.8 - 1.9 |
| unknown primary serous carcinoma | 64 | 3.2 | 24.3 | 3.1 | 0.9 - 10.7 |
| stomach adenocarcinoma diffuse type | 230 | 2.7 | 159.5 | 3.0 | 1.5 - 6.1 |
| kidney clear cell carcinoma | 537 | 2.7 | 29.7 | 0.2 | 0 - 0.7 |
| salivary gland mucoepidermoid carcinoma | 55 | 2.7 | 159.5 | 7.3 | 2.9 - 17.3 |
| breast metaplastic carcinoma | 142 | 2.7 | 39.6 | 2.1 | 0.7 - 6 |
| breast invasive lobular carcinoma (ilc) | 520 | 2.7 | 76.6 | 5.4 | 3.8 - 7.7 |
| brain glioblastoma (gbm) | 2729 | 2.7 | 660.4 | 4.2 | 3.5 - 5 |
| ovary serous carcinoma | 2100 | 2.7 | 511.9 | 0.4 | 0.2 - 0.7 |
| brain oligodendroglioma | 321 | 2.7 | 807.2 | 8.4 | 5.6 - 11.6 |
| pancreas acinar cell carcinoma | 65 | 2.7 | 14.4 | 0.0 | 0 - 5.6 |
| salivary gland adenocarcinoma | 139 | 2.7 | 152.3 | 2.2 | 0.4 - 5.1 |
| kidney renal papillary carcinoma | 152 | 2.7 | 13.5 | 0.0 | 0 - 2.5 |
| uterus endometrial adenocarcinoma papillary serous | 395 | 2.7 | 484.1 | 1.5 | 0.5 - 2.9 |
| ovary mucinous carcinoma | 57 | 2.7 | 20.7 | 1.8 | 0 - 6.3 |
| ovary clear cell carcinoma | 236 | 2.7 | 105.4 | 1.7 | 0.7 - 4.3 |
| kidney renal cell carcinoma (nos) | 543 | 2.7 | 20.7 | 0.2 | 0 - 0.7 |
| adrenal gland cortical carcinoma | 204 | 2.7 | 82.0 | 2.5 | 1.1 - 5.6 |
| testis germ cell tumor (non-seminoma) | 71 | 2.7 | 15.3 | 0.0 | 0 - 5.1 |
| soft tissue neuroblastoma | 265 | 2.7 | 56.8 | 0.4 | 0 - 1.4 |
| nasopharynx and paranasal sinuses undifferentiated carcinoma | 105 | 2.7 | 31.5 | 1.9 | 0 - 5.2 |
| kidney carcinoma (nos) | 59 | 2.7 | 29.7 | 1.7 | 0.1 - 9 |
| kidney sarcomatoid carcinoma | 70 | 2.7 | 46.6 | 1.4 | 0 - 5.2 |

(continued)

| Disease type | Specimen count | Median TMB | Maximum TMB | Percent cases with >20 mutations/Mb | 95% CI* |
|---|---|---|---|---|---|
| pancreatobiliary carcinoma (nos) | 594 | 2.7 | 54.1 | 2.2 | 1.2 - 3.5 |
| unknown primary undifferentiated neuroendocrine carcinoma | 674 | 2.7 | 401.8 | 6.1 | 4.4 - 8 |
| pancreas neuroendocrine carcinoma | 233 | 2.7 | 559.5 | 1.7 | 0.4 - 3.7 |
| prostate acinar adenocarcinoma | 1448 | 2.7 | 1241.5 | 3.4 | 2.5 - 4.4 |
| uterus sarcoma (nos) | 54 | 2.6 | 10.8 | 0.0 | 0 - 6.6 |
| kidney wilms tumor | 62 | 2.5 | 16.2 | 0.0 | 0 - 5.8 |
| thymus carcinoma (nos) | 168 | 2.5 | 30.6 | 3.6 | 1.6 - 7.6 |
| soft tissue rhabdomyosarcoma (nos) | 89 | 2.5 | 55.1 | 1.1 | 0.1 - 6.1 |
| soft tissue leiomyosarcoma | 567 | 2.5 | 53.4 | 0.7 | 0.2 - 1.5 |
| liver cholangiocarcinoma | 1456 | 2.5 | 122.5 | 1.9 | 1.3 - 2.7 |
| appendix adenocarcinoma | 400 | 2.5 | 64.9 | 2.0 | 1 - 3.9 |
| thyroid anaplastic carcinoma | 147 | 2.5 | 100.9 | 1.4 | 0.4 - 4.8 |
| pancreas carcinoma (nos) | 653 | 2.5 | 136.2 | 1.4 | 0.6 - 2.4 |
| peritoneum serous carcinoma | 194 | 2.5 | 12.6 | 0.0 | 0 - 1.9 |
| bile duct adenocarcinoma | 227 | 2.5 | 39.1 | 2.6 | 1.2 - 5.6 |
| soft tissue malignant peripheral nerve sheath tumor (mpnst) | 134 | 2.5 | 141.2 | 8.2 | 4.1 - 13.2 |
| soft tissue sarcoma undifferentiated | 260 | 2.5 | 401.4 | 8.1 | 5 - 11.6 |
| bone osteosarcoma | 283 | 2.5 | 26.7 | 0.4 | 0 - 1.3 |
| soft tissue sarcoma (nos) | 636 | 2.5 | 167.6 | 5.0 | 3.5 - 6.8 |
| bone marrow leukemia t cell acute (tall) | 75 | 2.5 | 11.7 | 0.0 | 0 - 4.9 |
| soft tissue rhabdomyosarcoma embryonal | 54 | 2.5 | 8.3 | 0.0 | 0 - 6.6 |
| unknown primary gist | 132 | 2.5 | 10.8 | 0.0 | 0 - 2.8 |
| uterus leiomyosarcoma | 349 | 2.5 | 63.0 | 0.9 | 0.2 - 2.1 |
| soft tissue myxofibrosarcoma | 58 | 2.2 | 145.2 | 1.7 | 0 - 6.2 |
| ovary granulosa cell tumor | 144 | 1.8 | 9.9 | 0.0 | 0 - 2.6 |
| brain ependymoma | 108 | 1.8 | 37.8 | 0.9 | 0 - 5.1 |
| lung atypical carcinoid | 83 | 1.8 | 180.2 | 1.2 | 0.1 - 6.5 |
| brain meningioma | 326 | 1.8 | 152.6 | 0.9 | 0.2 - 2.2 |
| salivary gland acinic cell tumor | 81 | 1.8 | 668.5 | 1.2 | 0 - 4.5 |
| thyroid carcinoma (nos) | 123 | 1.8 | 20.7 | 0.8 | 0 - 3 |
| thyroid medullary carcinoma | 96 | 1.8 | 26.5 | 1.0 | 0.1 - 5.7 |
| thyroid follicular carcinoma | 68 | 1.8 | 18.9 | 0.0 | 0 - 5.3 |
| small intestine gist | 114 | 1.8 | 8.8 | 0.0 | 0 - 3.3 |

(continued)

| Disease type | Specimen count | Median TMB | Maximum TMB | Percent cases with >20 mutations/Mb | 95% CI* |
|---|---|---|---|---|---|
| appendix mucinous neoplasm (nos) | 106 | 1.8 | 51.7 | 0.9 | 0 - 3.5 |
| salivary gland adenoid cystic carcinoma | 184 | 1.8 | 7.6 | 0.0 | 0 - 2 |
| pancreas ductal adenocarcinoma | 2483 | 1.8 | 318.0 | 1.0 | 0.6 - 1.4 |
| unknown primary carcinoid | 70 | 1.8 | 25.2 | 1.4 | 0 - 5.2 |
| stomach gist | 163 | 1.8 | 9.9 | 0.0 | 0 - 2.3 |
| thyroid papillary carcinoma | 350 | 1.8 | 15.3 | 0.0 | 0 - 1.1 |
| brain glioma (nos) | 220 | 1.8 | 314.4 | 2.7 | 1 - 5.2 |
| brain medulloblastoma | 118 | 1.8 | 12.6 | 0.0 | 0 - 3.2 |
| head and neck adenoid cystic carcinoma | 231 | 1.8 | 10.8 | 0.0 | 0 - 1.6 |
| pleura mesothelioma | 414 | 1.8 | 30.6 | 0.5 | 0.1 - 1.7 |
| brain oligoastrocytoma | 73 | 1.8 | 85.6 | 1.4 | 0.1 - 7.4 |
| peritoneum mesothelioma | 135 | 1.8 | 167.6 | 2.2 | 0.4 - 5.2 |
| brain astrocytoma | 351 | 1.8 | 87.4 | 1.4 | 0.4 - 2.9 |
| brain anaplastic astrocytoma | 396 | 1.8 | 191.0 | 2.0 | 0.9 - 3.6 |
| soft tissue chondrosarcoma | 124 | 1.7 | 49.2 | 0.8 | 0 - 3 |
| soft tissue solitary fibrous tumor | 86 | 1.7 | 28.4 | 1.2 | 0 - 4.3 |
| bone marrow leukemia non-lymphocytic acute myelocytic (aml) | 888 | 1.7 | 15.0 | 0.0 | 0 - 0.4 |
| soft tissue fibrosarcoma | 68 | 1.7 | 105.2 | 1.5 | 0 - 5.3 |
| uterus endometrial stromal sarcoma | 85 | 1.7 | 40.1 | 1.2 | 0.1 - 6.4 |
| bone chondrosarcoma | 93 | 1.7 | 69.3 | 2.2 | 0.6 - 7.5 |
| soft tissue paraganglioma | 53 | 1.7 | 316.5 | 1.9 | 0 - 6.8 |
| soft tissue ewing sarcoma | 191 | 1.7 | 23.4 | 0.5 | 0 - 2 |
| bone marrow leukemia lymphocytic acute (all) | 155 | 1.7 | 64.3 | 1.3 | 0.4 - 4.6 |
| soft tissue liposarcoma | 451 | 1.7 | 26.7 | 0.2 | 0 - 0.8 |
| bone marrow leukemia b cell acute (ball) | 204 | 1.7 | 32.5 | 1.0 | 0.3 - 3.5 |
| soft tissue desmoplastic small round cell tumor | 65 | 1.7 | 10.8 | 0.0 | 0 - 5.6 |
| adrenal gland neuroblastoma | 122 | 1.7 | 12.6 | 0.0 | 0 - 3.1 |
| soft tissue synovial sarcoma | 208 | 1.7 | 24.2 | 1.0 | 0 - 2.7 |
| soft tissue rhabdomyosarcoma alveolar | 61 | 1.7 | 7.5 | 0.0 | 0 - 5.9 |
| bone marrow leukemia non-lymphocytic myelomonocytic (mml) | 55 | 1.7 | 6.7 | 0.0 | 0 - 6.5 |

(continued)

| Disease type | Specimen count | Median TMB | Maximum TMB | Percent cases with >20 mutations/Mb | 95% CI* |
|---|---|---|---|---|---|
| bone marrow leukemia lymphocytic chronic (cll) | 176 | 1.7 | 15.9 | 0.0 | 0 - 2.1 |
| unknown primary adenoid cystic carcinoma | 76 | 1.5 | 55.0 | 1.3 | 0 - 4.8 |
| lung adenoid cystic carcinoma | 57 | 1.3 | 171.2 | 1.8 | 0 - 6.3 |
| thymus thymoma (nos) | 108 | 1.3 | 13.4 | 0.0 | 0 - 3.4 |
| eye intraocular melanoma | 113 | 1.3 | 21.4 | 0.9 | 0 - 3.3 |
| bone chordoma | 110 | 1.3 | 16.2 | 0.0 | 0 - 3.4 |
| small intestine carcinoid | 50 | 0.9 | 5.4 | 0.0 | 0 - 7.1 |
| soft tissue tibromatosis | 104 | 0.9 | 8.3 | 0.0 | 0 - 3.6 |
| brain astrocytoma pilocytic | 95 | 0.9 | 508.1 | 1.1 | 0.1 - 5.7 |
| bone marrow myeloproliferative neoplasm (mpn) | 138 | 0.8 | 7.5 | 0.0 | 0 - 2.7 |
| bone marrow myeloproliferative disorder (mpd) | 57 | 0.8 | 25.9 | 1.8 | 0 - 6.3 |
| bone marrow myelodysplastic syndrome (mds) | 513 | 0.8 | 10.0 | 0.0 | 0 - 0.7 |
| *CI: Confidence Interval | | | | | |

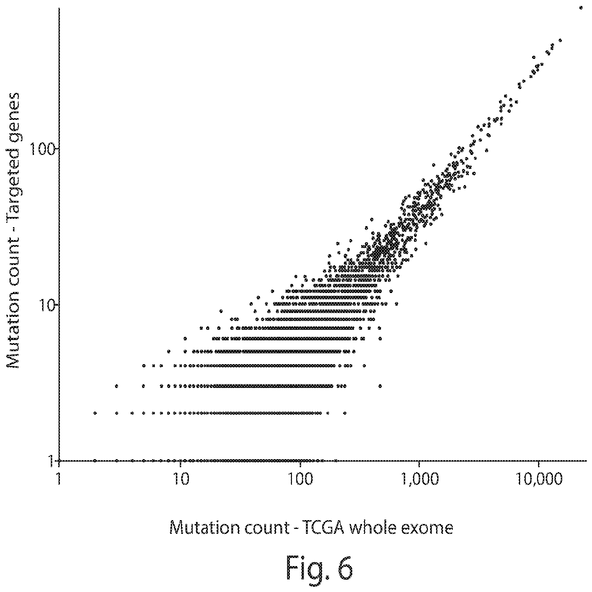

Diseases known to have significant mutagen exposure, such as lung and skin cancers, were more highly mutated (median TMB 7.2 mutations/Mb and 13.5 mutations/Mb, respectively). Disease indications currently approved for immunotherapies, including melanoma, non-small cell lung cancer (NSCLC), and bladder, had high TMB (see **Table** 6). Identifying additional cancer types with high TMB may represent an opportunity to expand the list of indications that respond favorably to checkpoint inhibitor blockade. These include skin squamous cell carcinoma, lung small cell undifferentiated carcinoma, diffuse large B cell lymphoma, as well many other types of cancer **(FIGS. 6A-6C).** In addition to identifying additional cancer types with high overall TMB, cases with high TMB were found across nearly every cancer type (see **Tables 6-7).** This raises the possibility that patients with high TMB who may benefit from immunotherapy can be identified in nearly every type of cancer. For example, in soft tissue angiosarcoma, while the median mutation burden was 3.8 mutations/Mb, 13.4% of cases had more than 20 mutations/Mb. Overall, 20 tumor types affecting 8 tissues were identified with greater than 10% of patients who had high TMB and 38 tumor type affecting 19 tissues with greater than 5% of patients with high TMB (see **Table** 7).

**Table 7.** Disease indications with greater than 5% of specimens showing high TMB (>20 mutations/Mb).

| Disease type | Specimen count | Median mutations/Mb | Percent cases with >20 mutations/Mb (95% CI) |
|---|---|---|---|
| skin basal cell carcinoma | 92 | 47.3 | 70.7 (60.7 - 79) |
| skin squamous cell carcinoma (scc) | 266 | 45.2 | 67.3 (61.4 - 72.7) |
| skin melanoma | 879 | 14.4 | 39.7 (36.4 - 42.9) |
| skin merkel cell carcinoma | 206 | 4.3 | 37.9 (31.5 - 44.7) |
| unknown primary melanoma | 1324 | 12.6 | 37.6 (35 - 40.2) |
| head and neck melanoma | 59 | 6.3 | 25.4 (14.7 - 36) |
| lung large cell carcinoma | 74 | 12.2 | 24.3 (14.9 - 33.7) |

(continued)

| Disease type | Specimen count | Median mutations/Mb | Percent cases with >20 mutations/Mb (95% CI) |
|---|---|---|---|
| unknown primary squamous cell carcinoma (scc) | 606 | 7.6 | 21.6 (18.4 - 24.9) |
| lung large cell neuroendocrine carcinoma | 288 | 9.9 | 19.8 (15.6 - 24.8) |
| lung sarcomatoid carcinoma | 130 | 7.2 | 19.2 (12.7 - 26) |
| stomach adenocarcinoma intestinal type | 58 | 5.0 | 19 (10.9 - 30.9) |
| uterus endometrial adenocarcinoma endometrioid | 459 | 4.5 | 18.5 (15 - 22.1) |
| lymph node lymphoma diffuse large b cell | 348 | 10.0 | 18.4 (14.7 - 22.8) |
| lung non-small cell lung carcinoma (nos) | 2636 | 8.1 | 17 (15.6 - 18.5) |
| unknown primary sarcomatoid carcinoma | 64 | 5.4 | 15.6 (7.6 - 24.6) |
| unknown primary malignant neoplasm (nos) | 491 | 3.8 | 14.9 (12 - 18.3) |
| uterus endometrial adenocarcinoma (nos) | 743 | 4.5 | 14.7 (12.3 - 17.4) |
| bladder carcinoma (nos) | 77 | 8.1 | 14.3 (8.2 - 23.8) |
| unknown primary urothelial carcinoma | 188 | 7.2 | 13.8 (9.2 - 18.9) |
| soft tissue angiosarcoma | 157 | 3.3 | 13.4 (8.9 - 19.6) |
| lung adenocarcinoma | 11855 | 6.3 | 12.3 (11.7 - 12.9) |
| lung adenosquamous carcinoma | 154 | 5.4 | 12.3 (7.5 - 17.7) |
| skin adnexal carcinoma | 74 | 3.6 | 12.2 (6.5 - 21.5) |
| bladder urothelial (transitional cell) carcinoma | 1218 | 7.2 | 11.9 (10.1 - 13.8) |
| lymph node lymphoma b-cell (nos) | 88 | 6.3 | 11.4 (6.3 - 19.7) |
| lung squamous cell carcinoma (scc) | 2102 | 9.0 | 11.3 (10 - 12.7) |
| unknown primary carcinoma (nos) | 1405 | 4.5 | 10.7 (9.2 - 12.4) |
| head and neck squamous cell carcinoma (hnscc) | 1184 | 5.0 | 10.1 (8.5 - 11.9) |
| lung small cell undifferentiated carcinoma | 913 | 9.9 | 9 (7.3 - 11) |
| nasopharynx and paranasal sinuses squamous cell carcinoma (scc) | 67 | 4.5 | 9 (4.2 - 18.2) |
| ovary endometrioid adenocarcinoma | 105 | 3.6 | 8.6 (4.6 - 15.5) |

(continued)

| Disease type | Specimen count | Median mutations/Mb | Percent cases with >20 mutations/Mb (95% CI) |
|---|---|---|---|
| unknown primary undifferentiated small cell carcinoma | 117 | 6.3 | 8.5 (4.1 - 14) |
| brain oligodendroglioma | 321 | 2.7 | 8.4 (5.6 - 11.6) |
| small intestine adenocarcinoma | 277 | 4.5 | 8.3 (5.3 - 11.7) |
| soft tissue malignant peripheral nerve sheath tumor (mpnst) | 134 | 2.5 | 8.2 (4.1 - 13.2) |
| soft tissue sarcoma undifferentiated | 260 | 2.5 | 8.1 (5.3 - 12) |
| uterus endometrial adenocarcinoma clear cell | 62 | 3.6 | 8.1 (3.5 - 17.5) |
| prostate undifferentiated carcinoma | 91 | 3.6 | 7.7 (3.8 - 15) |
| salivary gland mucoepidermoid carcinoma | 55 | 2.7 | 7.3 (2.9 - 17.3) |
| unknown primary adenocarcinoma | 2751 | 3.6 | 6.9 (6 - 7.9) |
| ureter urothelial carcinoma | 88 | 5.4 | 6.8 (2.5 - 12.6) |
| cervix squamous cell carcinoma (scc) | 284 | 5.4 | 6.7 (4.3 - 10.2) |
| penis squamous cell carcinoma (scc) | 60 | 4.5 | 6.7 (2.6 - 15.9) |
| salivary gland carcinoma (nos) | 160 | 3.6 | 6.3 (3.4 - 11.1) |
| kidney urothelial carcinoma | 224 | 5.4 | 6.3 (3.8 - 10.2) |
| unknown primary undifferentiated | 674 | 2.7 | 6.1 (4.5 - 8.1) |
| neuroendocrine carcinoma | | | |
| duodenum adenocarcinoma | 249 | 3.6 | 6 (3.4 - 9.2) |

To summarize, this study characterizes and provides extensive data describing tumor mutational burden across more than 100,000 clinical cancer specimens from advanced disease, including many previously undescribed types of cancer. These data can be used to guide design of immunotherapy clinical trials across a broader range of indications. Currently, immunotherapies targeting CTLA-4, PD-1, and PD-L1 are approved in a small number of indications, melanoma, bladder, NSCLC, and renal cell carcinoma. It was observed that melanoma and NSCLC represent some of the highest mutation burden indications. Several novel disease types were identified with high mutation burden which may be good targets for immuno-oncology treatment development. In addition, a wide range of TMB was observed across many cancer types. It was found that there may be many disease types with a substantial portion of patients who might benefit from these therapies. Overall, 22 tumor types affecting 8 tissues were identified, where greater than 10% of patients had high TMB.

## EXAMPLE 3: COMPREHENSIVE GENOMIC PROFILING TO ASSESS MUTATIONAL LOAD IN LUNG CANCER

Lung cancer presents a management challenge, particularly when *EGFR, ALK* or *ROS1* mutations cannot be detected and cytotoxic therapy has failed. To study the association of mutation load with the efficacy of novel immunotherapeutic agents (e.g., PD-1/PD-L1 and CTLA4 inhibitors), mutational load was assessed via genomic profiling performed in the course of clinical care for patients with lung cancer.

*Methods*

Briefly, DNA was extracted from 40 microns of FFPE sections from patients with lung cancer. CGP was performed on hybridization-captured, adaptor ligation based libraries to a median coverage depth of 663× for 315 cancer-related genes plus introns from 28 genes frequently rearranged in cancer. Without wishing to be bound by theory, in this Example, mutational load was characterized as the number of base substitutions or indels per megabase (Mb) after filtering to remove known somatic and functional alterations as described herein, given that these are selected for with hybrid capture.

*FFPE Tumor Samples*

The sample requirements are as follows: Surface area: $\geq 25$ mm$^2$; Sample volume: $\geq 1$ mm$^3$; Nucleated cellularity: $\geq 80\%$ or $\geq 30,000$ cells; Tumor content: $\geq 20\%$; Fraction of patients with tissue insufficient for analysis: 10-15%.

*Sequencing Library Preparation*

The laboratory process required $\geq 50$ng of dsDNA (quantified by PicoGreen). The DNA was fragmented by sonication (Covaris) and used in "with-bead" library construction. The DNA fragments were captured by hybridization with biotinylated DNA oligonucleotides. 49 × 49 paired-end sequencing was performed on the Illumina HiSeq platform to >500× average unique coverage, with >100× at >99% of exons.

*Analysis pipeline*

Base substitutions were analyzed by Bayesian algorithm. Short insertions/deletions were evaluated by local assembly. Copy number alterations were analyzed by comparison with process-matched normal control. Gene fusions were examined by analysis of chimeric read pairs.

The analysis methods had sensitivity to variants present at any mutant allele frequency and were able to detect long (1-40 bp) indel variants using de Bruijn graph-based local assembly. The analysis method also used comparative genomic hybridization (CGH)-like analysis of read-depth for assessment of copy number alterations (CNAs).

*Clinical Report*

The reporting approach provided interpretation without a matched normal. Germline variants from 1000 Genomes Project (dbSNP135) were removed. Known driver alterations (COSMIC v62) were highlighted as biologically significant. A concise summary of the biomedical literature and current clinical trials was provided for each alteration

*Mutation Load Analysis Methods*

The goal of the mutation load algorithm is to quantify the number of somatic mutations detected on the FoundationOne® test, and to extrapolate that value to the exome or genome as a whole.

All short variant alterations (base substitutions and indels) detected on the FoundationOne test are counted. All coding alterations, including silent alterations, are counted. Non-coding alterations are not counted. Alterations with known functional status (occurring as known somatic alterations in the COSMIC database; cancer.sanger.ac.uk/cosmic) and likely functional status (truncations in tumor suppressor genes) are not counted. Known germline alterations in the dbSNP database (www.ncbi.nlm.nih.gov/SNP) are not counted. Germline alterations occurring with two or more counts in the ExAC database (exac.broadinstitute.org) are not counted. Alterations that are predicted to be germline, in the specimen being assessed, by the somatic-germline-zygosity (SGZ) algorithm (*e.g.*, as described in International Application Publication No. WO2014/183078, U.S. Application Publication No. 2014/0336996, and Sun *et al. Cancer Research* 2014; 74(19S):1893-1893) are not counted. Alterations that are predicted to be germline, with high confidence, in the cohort of >60,000 clinical specimens, by the SGZ algorithm, are not counted. To calculate the mutation load per megabase, the total number of mutations counted is divided by the coding region target territory of the test, which is 1.252 megabases for the current version of the test.

***Results***

The genomic profiles from a total of 10,676 lung adenocarcinoma, 1.960 lung squamous cell carcinoma, 220 lung large cell carcinoma, and 784 lung small cell carcinoma were assessed. The median age of lung cancer patients was 66 years old with a 0.9:1 male:female ratio. Mean mutations per megabase were assessed as a range of 0 to 984, and the 25th, median, and 75th quartile thresholds were 2.7. 7.2, and 22.5.

The clinical characteristics of lung cancer patient cohorts are shown in **Table 8.** The mutational load characteristics of lung cancer are shown in **Table 9.**

**Table 8.** Clinical characteristics of lung cancer patient cohorts

| | Number of Cases | Gender Ratio (M:F) | Patient Age (yr) | | |
|---|---|---|---|---|---|
| | | | 10th Percentile | Median | 90th Percentile |
| Lung adenocarcinoma | 10676 | 0.8 : 1 | 50 | 65 | 79 |
| Lung squamous cell carcinoma | 1960 | 1.6 : 1 | 54 | 68 | 79 |
| Lung large cell carcinoma | 220 | 1.1 : 1 | 49 | 62 | 75 |
| Lung small cell carcinoma | 784 | 1 : 1 | 50 | 62 | 75 |

**Table 9.** Mutational load characteristics of lung cancer

| | Tumor Mutational Burden (mutations/Mb) | | | | | Fraction TMB≥10 | Fraction TMB≥20 |
|---|---|---|---|---|---|---|---|
| | Minimum | 10th Percentile | Median | 90th Percentile | Maximum | | |
| Lung adenocarcinoma | 0 | 0.9 | 6.3 | 23 | 984 | 36% | 14% |
| Lung squamous cell carcinoma | 0 | 2.7 | 9.0 | 22 | 522 | 49% | 13% |
| Lung large cell carcinoma | 0 | 1.8 | 9.9 | 31 | 98 | 53% | 23% |
| Lung small cell carcinoma | 0 | 3.6 | 9.0 | 19 | 234 | 49% | 10% |

The mutational load distribution in the clinical cohort is shown in **FIGs. 7A-7D.** The mutation prevalence in lung cancer is shown in **FIGs. 8A-8E.**

To summarize, a highly variable mutational load was seen in patients with lung cancer. The ability to accurately discriminate somatic vs normal mutations computationally is essential when a patient matched normal specimen is unavailable. A substantial fraction of lung cancer cases have a high mutation load (39% ≥10 per Mb; 13% ≥20 per Mb) and are potential candidates for clinical trials of immunotherapeutic agents.

## EXAMPLE 4: COMPREHENSIVE GENOMIC PROFILING TO ASSESS MUTATIONAL LOAD IN COLORECTAL ADENOCARCINOMAS

Colorectal adenocarcinoma remains a clinical challenge, particularly when *KRAS* or *NRAS* gene is mutated and cytotoxic therapy has failed. To study the association of tumor mutational load with predicted benefit from immune checkpoint inhibitors, the relationship between mutational burden and clinically relevant genomic alterations in colorectal adenocarcinoma samples were assessed in the course of routine clinical care using genomic profiling.

### *Methods*

DNA was extracted from 40 microns of FFPE sections from patients with colorectal adenocarcinoma. CGP was performed on hybridization-captured, adaptor ligation based libraries to a mean coverage depth of 698× for 315 cancer-related genes plus introns from 28 genes frequently rearranged in cancer. Without wishing to be bound by theory, in this Example, mutational load was characterized as the number of base substitutions or indels per megabase (Mb) after filtering to remove known somatic and functional alterations as described herein, given that these are selected for with hybrid capture.

Sample requirements, sequencing library preparation, analysis pipeline, clinical report, and mutational load analysis methods are as described in Example 3.

*Results*

The genomic profiles from a total of 6,742 colon and 1,176 rectum adenocarcinomas were assessed. The median age of colorectal adenocarcinoma patients was 57 years old with a 1.2:1 male:female ratio. Mean mutations per megabase were assessed as a range of 0 to 866. and the 25th, median, and 75th quartile thresholds were 2.7. 4.5. and 6.3.

Genetic alterations in mismatch repair genes *MLH1, MSH2, MSH6,* or DNA polymerase gene *POLD1* were detected in 174 (2.2%), 191 (2.4%), 315 (3.9%) or 283 (3.6%) cases of colorectal adenocarcinoma, which was associated with a median tumor mutational load of 30, 23, 29, or 15, respectively. However, the ten most frequently altered gens in this cohort - *APC* (76%), *TP53* (76%), *KRAS* (51%), *PIK3CA* (18%), *SMAD4* (15%), *FBXW7* (10%), *SOX9* (10%), *MYC* (8%), *BRAF* (8%), and *PTEN* (8%) - were not associated with differences in tumor mutational load.

The clinical characteristics of colorectal adenocarcinoma patient cohorts are shown in **Table 10.** The mutational load characteristics of colorectal adenocarcinoma are described in **Table 11.**

**Table 10.** Clinical characteristics of colorectal adenocarcinoma patient cohorts

| Number of Cases | Gender Ratio (M:F) | Patient Age (yr) | | |
|---|---|---|---|---|
| | | 10th | Median Percentile | 90th Percentile |
| Colon adenocarcinoma | 6742 | 1.1 : 1 | 41 | 57 | 73 |
| Rectum adenocarcinoma | 1176 | 1.5 : 1 | 40 | 55 | 72 |

**Table 11.** Mutational load characteristics of colorectal adenocarcinoma

| | Tumor Mutational Burden (mutations/Mb) | | | | | Fraction TMB≥10 | Fraction TMB≥20 |
|---|---|---|---|---|---|---|---|
| | Minimum | 10th Percentile | Median | 90th Percentile | Maximum | | |
| Colon adenocarcinoma | 0 | 1.8 | 4.5 | 10 | 751 | 10% | 6% |
| Rectum adenocarcinoma | 0 | 0.9 | 3.6 | 8 | 866 | 6% | 3% |

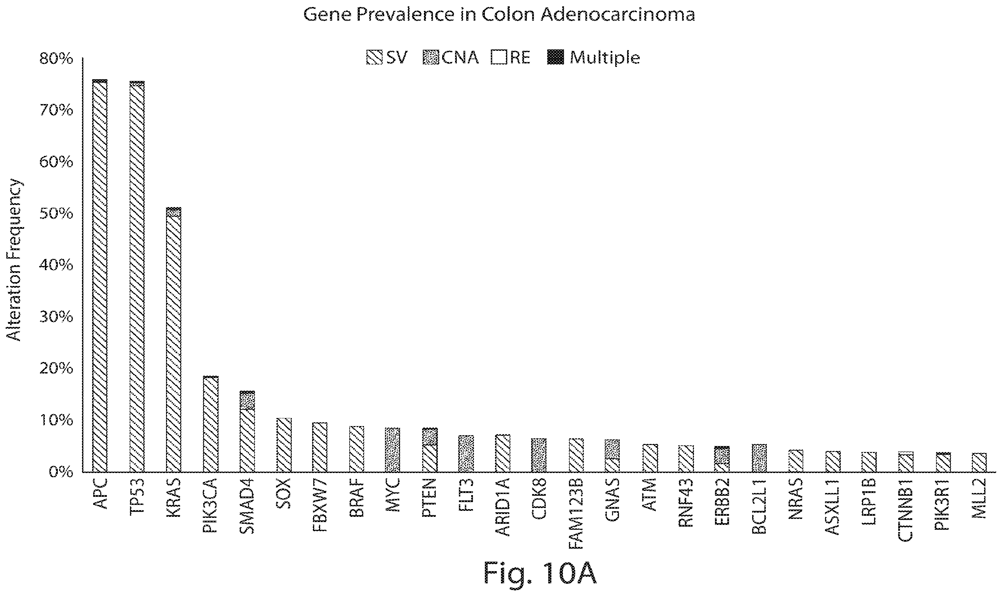

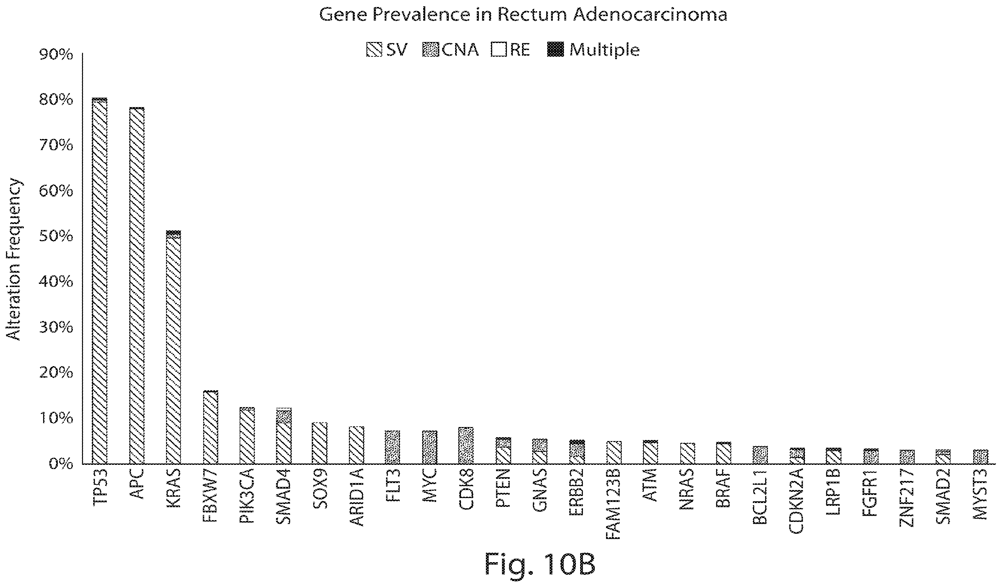

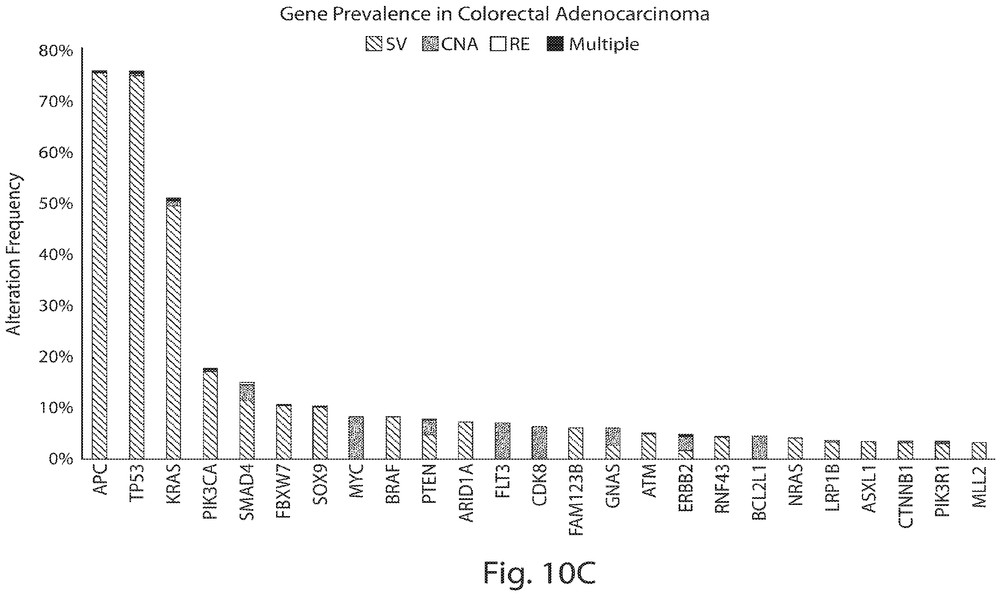

The mutational load distribution in the clinical cohort is shown in **FIGs. 9A-9B.** The mutation prevalence in colorectal adenocarcinoma is shown in **FIGs. 10A-10C.**

To summarize, CGP in the course of clinical care can be used to assess mutational load in colorectal adenocarcinoma. Mutations in DNA mismatch repair genes were associated with higher mutation burden as expected. A substantial fraction of colorectal adenocarcinoma cases have a high mutation load (9% ≥10 per Mb; 5% ≥20 per Mb) and are potential candidates for clinical trials of immunotherapeutic agents. Incorporation of CGP into ongoing prospective immunotherapy trials and clinical practice is needed to refine these relationships.

**EXAMPLE 5: COMPREHENSIVE GENOMIC PROFILING TO ASSESS MUTATIONAL LOAD IN TWENTY-FOUR TYPES OF HUMAN NEOPLASMS**

To study the association of tumor mutational load with predicted benefit from immune checkpoint inhibitors, the distribution of mutational burden in 24 types of neoplasms were assessed in the course of routine clinical care using genomic profiling.

*Methods*

DNA was extracted from 40 microns of FFPE sections from patients with one of twenty-four types of neoplasms. CGP was performed on hybridization-captured, adaptor ligation based libraries to a mean coverage depth of greater than 500× for 315 cancer-related genes plus introns from 28 genes frequently rearranged in cancer. Without wishing to be bound by theory, in this Example, mutational load was characterized as the number of base substitutions or indels per megabase (Mb) after filtering to remove known somatic and functional alterations as described herein, given that these are selected for with hybrid capture.

Sample requirements, sequencing library preparation, analysis pipeline, clinical report, and mutational load analysis methods are as described in Example 3.

*Results*

The genomic profiles from a total of 15.508 neoplasm specimens were assessed. The median age of patient cohort was 60 years old with a 0.6:1 male:female ratio. Mean mutations per megabase were assessed as a range of 0 to 689, and the 25th, median, and 75th quartile thresholds were 1.8, 3.6, and 5.4.

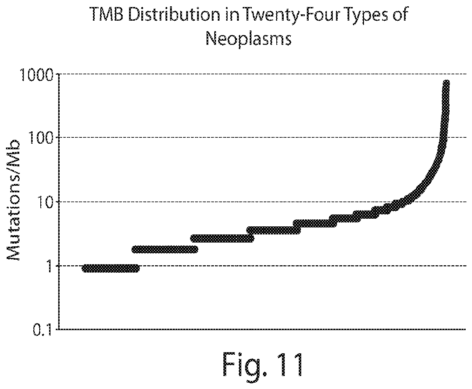

The clinical characteristics of patient cohorts are shown in **Table 12.** The mutational load characteristics of twenty-four types of neoplasms are described in **Table 13.** The TMB distribution in 24 different neoplasms is shown in **FIG. 11.**

**Table 12.** Clinical characteristics of cancer patient cohorts

| | Number of Cases | Gender Ratio (M:F ) | Patient Age (yr) | | |
|---|---|---|---|---|---|
| | | | 10th Percentile | Median | 90th Percentile |
| Bladder urothelial transitional cell carcinoma | 963 | 2.7 : 1 | 52 | 67 | 79 |
| Brain astrocytoma | 257 | 1.5 : 1 | 17 | 39 | 64 |
| Brain glioblastoma | 2248 | 1.5 : 1 | 31 | 56 | 71 |
| Breast invasive ductal carcinoma | 3291 | 0 : 1 | 37 | 53 | 69 |
| Breast invasive lobular carcinoma | 419 | 0 : 1 | 45 | 60 | 74 |
| Cervix adenocarcinoma | 146 | n/a | 32 | 50 | 67 |
| Cervix adenosquamous carcinoma | 17 | n/a | 27 | 42 | 65 |
| Cervix squamous cell carcinoma | 231 | n/a | 32 | 46 | 65 |
| Head & Neck adenocarcinoma | 15 | 2 : 1 | 34 | 58 | 80 |
| Liver hepatocellular carcinoma | 484 | 2.6 : 1 | 43 | 62 | 74 |
| Ovary serous carcinoma | 1626 | n/a | 45 | 60 | 73 |
| Ovary clear cell carcinoma | 198 | n/a | 40 | 54 | 68 |
| Ovary endometrioid adenocarcinoma | 88 | n/a | 39 | 56 | 68 |
| Ovary mucinous carcinoma | 38 | n/a | 26 | 49 | 69 |
| Ovary germ cell tumor | 24 | n/a | 8 | 28 | 71 |
| Pancreas ductal adenocarcinoma | 2047 | 1.2 : 1 | 49 | 63 | 75 |
| Prostate acinar adenocarcinoma | 1366 | n/a | 53 | 65 | 76 |
| Prostate ductal adenocarcinoma | 17 | n/a | 57 | 69 | 79 |
| Skin melanoma | 759 | 1.8 : 1 | 39 | 62 | 80 |
| Stomach adenocarcinoma diffuse type | 165 | 0.7 : 1 | 35 | 55 | 70 |
| Stomach adenocarcinoma intestinal type | 42 | 2.8 : 1 | 37 | 57 | 76 |
| Stomach gastrointestinal stromal tumor | 103 | 1.5 : 1 | 33 | 60 | 76 |
| Uterus endometrial adenocarcinoma (NOS) | 632 | n/a | 51 | 64 | 75 |
| Uterus endometrial adenocarcinoma papillary serous | 334 | n/a | 56 | 66 | 76 |

**Table 13.** Mutational load characteristics of twenty-four types of neoplasms

| | Tumor Mutational Burden (mutations/Mb) | | | | | Fraction TMB≥10 | Fraction TMB≥20 |
|---|---|---|---|---|---|---|---|
| | Minimum | 10th Percentile | Median | 90th Percentile | Maximum | | |
| Bladder urothelial transitional cell carcinoma | 0 | 2.16 | 7.2 | 22 | 108 | 36% | 13% |
| Brain astrocytoma | 0 | 0 | 1.8 | 5 | 87 | 3% | 1% |
| Brain glioblastoma | 0 | 0.9 | 2.7 | 6 | 689 | 6% | 4% |

(continued)

| | Tumor Mutational Burden (mutations/Mb) | | | | | Fraction TMB≥10 | Fraction TMB≥20 |
|---|---|---|---|---|---|---|---|
| | Minimum | 10th Percentile | Median | 90th Percentile | Maximum | | |
| Breast invasive ductal carcinoma | 0 | 0.9 | 3.6 | 8 | 261 | 7% | 2% |
| Breast invasive lobular carcinoma | 0 | 0.9 | 2.7 | 14 | 78 | 14% | 7% |
| Cervix adenocarcinoma | 0 | 0.9 | 3.6 | 10 | 62 | 12% | 3% |
| Cervix adenosquamous carcinoma | 0 | 1.44 | 3.6 | 35 | 35 | 12% | 12% |
| Cervix squamous cell carcinoma | 0 | 1.8 | 5.4 | 17 | 73 | 21% | 7% |
| Head & Neck adenocarcinoma | 0 | 0.54 | 2.7 | 8 | 10 | 7% | 0% |
| Liver hepatocellular carcinoma | 0 | 0.9 | 3.6 | 8 | 42 | 6% | 1% |
| Ovary serous carcinoma | 0 | 0.9 | 2.7 | 7 | 21 | 3% | 0% |
| Ovary clear cell carcinoma | 0 | 0.9 | 2.7 | 7 | 107 | 6% | 3% |
| Ovary endometrioid adenocarcinoma | 0 | 0.9 | 3.6 | 22 | 70 | 16% | 10% |
| Ovary mucinous carcinoma | 0 | 0 | 2.7 | 6 | 21 | 3% | 3% |
| Ovary germ cell tumor | 0 | 0 | 3.6 | 23 | 36 | 21% | *13%* |
| Pancreas ductal adenocarcinoma | 0 | 0 | 1.8 | 5 | 322 | 2% | 1% |
| Prostate acinar adenocarcinoma | 0 | 0.9 | 2.7 | 7 | 319 | 6% | 3% |
| Prostate ductal adenocarcinoma | 0.9 | 0.9 | 2.7 | 5 | 5 | 0% | 0% |
| Skin melanoma | 0 | 1.8 | 14.4 | 82 | 632 | 62% | 41% |
| Stomach adenocarcinoma diffuse type | 0 | 0 | 2.7 | 7 | 160 | 6% | 4% |
| Stomach adenocarcinoma intestinal type | 0 | 0.54 | 4.5 | 37 | 64 | 14% | 10% |
| Stomach gastrointestinal stromal tumor | 0 | 0 | 1.8 | 5 | 10 | 2% | 0% |
| Uterus endometrial adenocarcinoma (NOS) | 0 | 0.9 | 4.5 | 28 | 636 | 26% | 16% |

(continued)

| | Tumor Mutational Burden (mutations/Mb) | | | | | Fraction TMB≥10 | Fraction TMB≥20 |
|---|---|---|---|---|---|---|---|
| | Minimum | 10th Percentile | Median | 90th Percentile | Maximum | | |
| Uterus endometrial adenocarcinoma papillary serous | 0 | 0.9 | 3.6 | 8 | 65 | 5% | 1% |

Additional examples relevant to the methods and systems described herein are described, *e.g.,* in Examples 1-17 of International Application Publication No. WO2012/092426, Examples 16 and 17 of International Application Publication No. WO2016/090273.

## TABLE 5 (APPENDIX A)

| TCGA STUDY | TGCA SPECIMEN NAME | TCGA WHOLE EXOME MUTATION COUNT | TCGA TARGETED GENE MUTATION COUNT |
|---|---|---|---|
| ACC | TCGA-OR-A5J1-01 | 66 | 2 |
| ACC | TCGA-OR-A5J2-01 | 122 | 6 |
| ACC | TCGA-OR-A5J3-01 | 116 | 2 |
| ACC | TCGA-OR-A5J4-01 | 230 | 6 |
| ACC | TCGA-OR-A5J5-01 | 733 | 27 |
| ACC | TCGA-OR-A5J6-01 | 162 | 6 |
| ACC | TCGA-OR-A5J7-01 | 137 | 5 |
| ACC | TCGA-OR-A5J8-01 | 238 | 10 |
| ACC | TCGA-OR-A5J9-01 | 183 | 5 |
| ACC | TCGA-OR-A5JA-01 | 782 | 34 |
| ACC | TCGA-OR-A5JB-01 | 490 | 23 |
| ACC | TCGA-OR-A5JC-01 | 116 | 4 |
| ACC | TCGA-OR-A5JD-01 | 95 | 1 |
| ACC | TCGA-OR-A5JE-01 | 170 | 8 |
| ACC | TCGA-OR-A5JF-01 | 181 | 5 |
| ACC | TCGA-OR-A5JG-01 | 172 | 4 |
| ACC | TCGA-OR-A5JH-01 | 84 | 4 |
| ACC | TCGA-OR-A5JI-01 | 89 | 1 |
| ACC | TCGA-OR-A5JJ-01 | 139 | 8 |
| ACC | TCGA-OR-A5JK-01 | 127 | 3 |
| ACC | TCGA-OR-A5JL-01 | 120 | 4 |
| ACC | TCGA-OR-A5JM-01 | 191 | 10 |
| ACC | TCGA-OR-A5JO-01 | 155 | 3 |
| ACC | TCGA-OR-A5JP-01 | 198 | 8 |
| ACC | TCGA-OR-A5JQ-01 | 107 | 1 |
| ACC | TCGA-OR-A5JR-01 | 89 | 2 |
| ACC | TCGA-OR-A5JS-01 | 215 | 7 |
| ACC | TCGA-OR-A5JT-01 | 103 | 4 |
| ACC | TCGA-OR-A5JU-01 | 147 | 3 |
| ACC | TCGA-OR-A5JV-01 | 87 | 1 |
| ACC | TCGA-OR-A5JW-01 | 136 | 3 |
| ACC | TCGA-OR-A5JX-01 | 132 | 9 |
| ACC | TCGA-OR-A5JY-01 | 168 | 7 |
| ACC | TCGA-OR-A5JZ-01 | 129 | 3 |
| ACC | TCGA-OR-A5K0-01 | 217 | 7 |
| ACC | TCGA-OR-A5K1-01 | 129 | 2 |
| ACC | TCGA-OR-A5K2-01 | 225 | 7 |
| ACC | TCGA-OR-A5K3-01 | 246 | 5 |
| ACC | TCGA-OR-A5K4-01 | 419 | 16 |
| ACC | TCGA-OR-A5K5-01 | 239 | 9 |
| ACC | TCGA-OR-A5K6-01 | 168 | 6 |
| ACC | TCGA-OR-A5K8-01 | 118 | 1 |
| ACC | TCGA-OR-A5K9-01 | 305 | 11 |
| ACC | TCGA-OR-A5KB-01 | 2650 | 101 |
| ACC | TCGA-OR-A5KO-01 | 186 | 4 |
| ACC | TCGA-OR-A5KP-01 | 168 | 3 |
| ACC | TCGA-OR-A5KQ-01 | 127 | 6 |
| ACC | TCGA-OR-A5KS-01 | 146 | 4 |
| ACC | TCGA-OR-A5KT-01 | 97 | 3 |
| ACC | TCGA-OR-A5KU-01 | 135 | 3 |
| ACC | TCGA-OR-A5KV-01 | 128 | 3 |
| ACC | TCGA-OR-A5KW-01 | 124 | 4 |
| ACC | TCGA-OR-A5KX-01 | 126 | 3 |
| ACC | TCGA-OR-A5KY-01 | 161 | 12 |
| ACC | TCGA-OR-A5KZ-01 | 118 | 5 |
| ACC | TCGA-OR-A5L1-01 | 91 | 1 |
| ACC | TCGA-OR-A5L2-01 | 278 | 11 |
| ACC | TCGA-OR-A5L3-01 | 109 | 5 |
| ACC | TCGA-OR-A5L4-01 | 95 | 1 |
| ACC | TCGA-OR-A5L5-01 | 152 | 4 |
| ACC | TCGA-OR-A5L6-01 | 127 | 2 |
| ACC | TCGA-OR-A5L8-01 | 123 | 2 |
| ACC | TCGA-OR-A5L9-01 | 108 | 3 |
| ACC | TCGA-OR-A5LA-01 | 88 | 2 |
| ACC | TCGA-OR-A5LB-01 | 429 | 14 |
| ACC | TCGA-OR-A5LC-01 | 170 | 2 |
| ACC | TCGA-OR-A5LD-01 | 126 | 2 |
| ACC | TCGA-OR-A5LE-01 | 150 | 6 |
| ACC | TCGA-OR-A5LF-01 | 145 | 5 |
| ACC | TCGA-OR-A5LG-01 | 164 | 5 |
| ACC | TCGA-OR-A5LH-01 | 127 | 2 |
| ACC | TCGA-OR-A5LI-01 | 219 | 6 |
| ACC | TCGA-OR-A5LJ-01 | 757 | 30 |
| ACC | TCGA-OR-A5LK-01 | 150 | 7 |
| ACC | TCGA-OR-A5LL-01 | 112 | 3 |
| ACC | TCGA-OR-A5LN-01 | 150 | 2 |
| ACC | TCGA-OR-A5LO-01 | 189 | 6 |
| ACC | TCGA-OR-A5LP-01 | 97 | 4 |
| ACC | TCGA-OR-A5LR-01 | 78 | 1 |
| ACC | TCGA-OR-A5LS-01 | 182 | 3 |
| ACC | TCGA-OR-A5LT-01 | 133 | 5 |
| ACC | TCGA-OU-A5PI-01 | 159 | 4 |
| ACC | TCGA-P6-A5OF-01 | 89 | 2 |
| ACC | TCGA-P6-A5OH-01 | 310 | 10 |
| ACC | TCGA-PA-A5YG-01 | 61 | 1 |
| ACC | TCGA-PK-A5H8-01 | 81 | 2 |
| ACC | TCGA-PK-A5H9-01 | 158 | 3 |
| ACC | TCGA-PK-A5HA-01 | 195 | 4 |
| ACC | TCGA-PK-A5HB-01 | 1641 | 56 |
| ACC | TCGA-PK-A5HC-01 | 263 | 14 |
| BLCA | TCGA-BL-A0C8-01 | 174 | 7 |
| BLCA | TCGA-BL-A13I-01 | 122 | 6 |
| BLCA | TCGA-BL-A13J-01 | 132 | 6 |
| BLCA | TCGA-BL-A3JM-01 | 247 | 14 |
| BLCA | TCGA-BT-A0S7-01 | 56 | 6 |
| BLCA | TCGA-BT-A0YX-01 | 759 | 33 |
| BLCA | TCGA-BT-A20J-01 | 580 | 30 |
| BLCA | TCGA-BT-A20N-01 | 339 | 8 |
| BLCA | TCGA-BT-A20O-01 | 220 | 13 |
| BLCA | TCGA-BT-A20P-01 | 124 | 7 |
| BLCA | TCGA-BT-A20Q-01 | 126 | 6 |
| BLCA | TCGA-BT-A20R-01 | 353 | 19 |
| BLCA | TCGA-BT-A20T-01 | 326 | 18 |
| BLCA | TCGA-BT-A20U-01 | 102 | 8 |
| BLCA | TCGA-BT-A20W-01 | 113 | 6 |
| BLCA | TCGA-BT-A2LB-01 | 724 | 30 |
| BLCA | TCGA-BT-A2LD-01 | 134 | 10 |
| BLCA | TCGA-BT-A3PH-01 | 541 | 27 |
| BLCA | TCGA-BT-A3PJ-01 | 599 | 17 |
| BLCA | TCGA-BT-A3PK-01 | 419 | 21 |
| BLCA | TCGA-BT-A42B-01 | 78 | 5 |
| BLCA | TCGA-BT-A42C-01 | 420 | 20 |
| BLCA | TCGA-C4-A0F0-01 | 117 | 6 |
| BLCA | TCGA-C4-A0F1-01 | 201 | 12 |
| BLCA | TCGA-C4-A0F6-01 | 251 | 12 |
| BLCA | TCGA-C4-A0F7-01 | 94 | 2 |
| BLCA | TCGA-CF-A1HR-01 | 382 | 13 |
| BLCA | TCGA-CF-A1HS-01 | 369 | 15 |
| BLCA | TCGA-CF-A27C-01 | 191 | 6 |
| BLCA | TCGA-CF-A3MF-01 | 38 | 2 |
| BLCA | TCGA-CF-A3MG-01 | 177 | 8 |
| BLCA | TCGA-CF-A3MH-01 | 61 | 2 |
| BLCA | TCGA-CF-A3MI-01 | 89 | 4 |
| BLCA | TCGA-CU-A0YN-01 | 178 | 5 |
| BLCA | TCGA-CU-A0YO-01 | 129 | 8 |
| BLCA | TCGA-CU-A0YR-01 | 178 | 9 |
| BLCA | TCGA-CU-A3KJ-01 | 276 | 16 |
| BLCA | TCGA-CU-A3QU-01 | 119 | 6 |
| BLCA | TCGA-CU-A3YL-01 | 278 | 17 |
| BLCA | TCGA-DK-A1A3-01 | 748 | 37 |
| BLCA | TCGA-DK-A1A5-01 | 328 | 17 |
| BLCA | TCGA-DK-A1A6-01 | 377 | 22 |
| BLCA | TCGA-DK-A1A7-01 | 258 | 13 |
| BLCA | TCGA-DK-A1AA-01 | 100 | 5 |
| BLCA | TCGA-DK-A1AB-01 | 236 | 8 |
| BLCA | TCGA-DK-A1AC-01 | 1789 | 67 |
| BLCA | TCGA-DK-A1AD-01 | 284 | 13 |
| BLCA | TCGA-DK-A1AE-01 | 150 | 11 |
| BLCA | TCGA-DK-A1AF-01 | 154 | 8 |
| BLCA | TCGA-DK-A1AG-01 | 151 | 12 |
| BLCA | TCGA-DK-A2HX-01 | 148 | 14 |
| BLCA | TCGA-DK-A2I1-01 | 299 | 8 |
| BLCA | TCGA-DK-A2I2-01 | 142 | 8 |
| BLCA | TCGA-DK-A2I4-01 | 1122 | 51 |
| BLCA | TCGA-DK-A2I6-01 | 388 | 13 |
| BLCA | TCGA-DK-A3IK-01 | 250 | 8 |
| BLCA | TCGA-DK-A3IL-01 | 212 | 12 |
| BLCA | TCGA-DK-A3IM-01 | 120 | 6 |
| BLCA | TCGA-DK-A3IN-01 | 470 | 27 |
| BLCA | TCGA-DK-A3IQ-01 | 241 | 7 |
| BLCA | TCGA-DK-A3IS-01 | 432 | 20 |
| BLCA | TCGA-DK-A3IT-01 | 472 | 20 |
| BLCA | TCGA-DK-A3IU-01 | 535 | 14 |
| BLCA | TCGA-DK-A3IV-01 | 128 | 4 |
| BLCA | TCGA-DK-A3WW-01 | 1432 | 49 |
| BLCA | TCGA-DK-A3WX-01 | 49 | 2 |
| BLCA | TCGA-DK-A3WY-01 | 19 | 1 |
| BLCA | TCGA-DK-A3X1-01 | 1043 | 38 |
| BLCA | TCGA-E5-A2PC-01 | 276 | 18 |
| BLCA | TCGA-E7-A3X6-01 | 144 | 2 |
| BLCA | TCGA-E7-A3Y1-01 | 195 | 8 |
| BLCA | TCGA-FD-A3B3-01 | 219 | 16 |
| BLCA | TCGA-FD-A3B5-01 | 176 | 12 |
| BLCA | TCGA-FD-A3B6-01 | 319 | 13 |
| BLCA | TCGA-FD-A3B7-01 | 92 | 7 |
| BLCA | TCGA-FD-A3B8-01 | 78 | 7 |
| BLCA | TCGA-FD-A3N5-01 | 389 | 22 |
| BLCA | TCGA-FD-A3NA-01 | 204 | 11 |
| BLCA | TCGA-FD-A3SJ-01 | 279 | 9 |
| BLCA | TCGA-FD-A3SL-01 | 261 | 17 |
| BLCA | TCGA-FD-A3SM-01 | 243 | 20 |
| BLCA | TCGA-FD-A3SN-01 | 462 | 18 |
| BLCA | TCGA-FD-A3SO-01 | 434 | 22 |
| BLCA | TCGA-FD-A3SP-01 | 148 | 12 |
| BLCA | TCGA-FD-A3SQ-01 | 100 | 3 |
| BLCA | TCGA-FD-A3SR-01 | 152 | 9 |
| BLCA | TCGA-FD-A3SS-01 | 530 | 17 |
| BLCA | TCGA-FJ-A3Z7-01 | 386 | 23 |
| BLCA | TCGA-FJ-A3ZE-01 | 487 | 21 |
| BLCA | TCGA-FJ-A3ZF-01 | 303 | 17 |
| BLCA | TCGA-FT-A3EE-01 | 143 | 9 |
| BLCA | TCGA-G2-A2EC-01 | 214 | 8 |
| BLCA | TCGA-G2-A2EF-01 | 318 | 12 |
| BLCA | TCGA-G2-A2EJ-01 | 368 | 24 |
| BLCA | TCGA-G2-A2EK-01 | 86 | 4 |
| BLCA | TCGA-G2-A2EO-01 | 1015 | 51 |
| BLCA | TCGA-G2-A2ES-01 | 547 | 22 |
| BLCA | TCGA-G2-A3IB-01 | 122 | 9 |
| BLCA | TCGA-G2-A3IE-01 | 471 | 24 |
| BLCA | TCGA-G2-A3VY-01 | 855 | 34 |
| BLCA | TCGA-GC-A3BM-01 | 154 | 14 |
| BLCA | TCGA-GC-A3I6-01 | 243 | 10 |
| BLCA | TCGA-GC-A3OO-01 | 137 | 5 |
| BLCA | TCGA-GC-A3RB-01 | 201 | 8 |
| BLCA | TCGA-GC-A3RC-01 | 450 | 21 |
| BLCA | TCGA-GC-A3RD-01 | 117 | 6 |
| BLCA | TCGA-GC-A3WC-01 | 176 | 8 |
| BLCA | TCGA-GC-A3YS-01 | 302 | 9 |
| BLCA | TCGA-GD-A2C5-01 | 344 | 21 |
| BLCA | TCGA-GD-A3OP-01 | 447 | 21 |
| BLCA | TCGA-GD-A3OQ-01 | 133 | 7 |
| BLCA | TCGA-GD-A3OS-01 | 115 | 6 |
| BLCA | TCGA-GU-A42R-01 | 199 | 17 |
| BLCA | TCGA-GV-A3JV-01 | 148 | 9 |
| BLCA | TCGA-GV-A3JW-01 | 116 | 2 |
| BLCA | TCGA-GV-A3JX-01 | 364 | 30 |
| BLCA | TCGA-GV-A3JZ-01 | 579 | 20 |
| BLCA | TCGA-GV-A3QF-01 | 164 | 7 |
| BLCA | TCGA-GV-A3QG-01 | 111 | 6 |
| BLCA | TCGA-GV-A3QH-01 | 225 | 13 |
| BLCA | TCGA-GV-A3QI-01 | 572 | 14 |
| BLCA | TCGA-GV-A3QK-01 | 181 | 4 |
| BLCA | TCGA-GV-A40E-01 | 216 | 14 |
| BLCA | TCGA-GV-A40G-01 | 208 | 9 |
| BLCA | TCGA-H4-A2HO-01 | 115 | 6 |
| BLCA | TCGA-H4-A2HQ-01 | 556 | 17 |
| BLCA | TCGA-HQ-A2OE-01 | 189 | 10 |
| BLCA | TCGA-K4-A3WS-01 | 334 | 23 |
| BLCA | TCGA-K4-A3WU-01 | 263 | 10 |
| BLCA | TCGA-K4-A3WV-01 | 83 | 5 |
| BRCA | TCGA-A1-A0SB-01 | 19 | 0 |
| BRCA | TCGA-A1-A0SD-01 | 27 | 1 |
| BRCA | TCGA-A1-A0SE-01 | 26 | 2 |
| BRCA | TCGA-A1-A0SF-01 | 40 | 2 |
| BRCA | TCGA-A1-A0SG-01 | 34 | 3 |
| BRCA | TCGA-A1-A0SH-01 | 94 | 1 |
| BRCA | TCGA-A1-A0SI-01 | 194 | 12 |
| BRCA | TCGA-A1-A0SJ-01 | 32 | 2 |
| BRCA | TCGA-A1-A0SK-01 | 173 | 6 |
| BRCA | TCGA-A1-A0SM-01 | 32 | 0 |
| BRCA | TCGA-A1-A0SN-01 | 50 | 1 |
| BRCA | TCGA-A1-A0SP-01 | 46 | 3 |
| BRCA | TCGA-A1-A0SQ-01 | 27 | 4 |
| BRCA | TCGA-A2-A04N-01 | 42 | 7 |
| BRCA | TCGA-A2-A04P-01 | 112 | 5 |
| BRCA | TCGA-A2-A04Q-01 | 14 | 2 |
| BRCA | TCGA-A2-A04R-01 | 80 | 2 |
| BRCA | TCGA-A2-A04T-01 | 93 | 7 |
| BRCA | TCGA-A2-A04U-01 | 56 | 4 |
| BRCA | TCGA-A2-A04V-01 | 34 | 2 |
| BRCA | TCGA-A2-A04W-01 | 108 | 6 |
| BRCA | TCGA-A2-A04X-01 | 30 | 1 |
| BRCA | TCGA-A2-A04Y-01 | 50 | 0 |
| BRCA | TCGA-A2-A0CK-01 | 26 | 2 |
| BRCA | TCGA-A2-A0CL-01 | 24 | 1 |
| BRCA | TCGA-A2-A0CM-01 | 54 | 2 |
| BRCA | TCGA-A2-A0CO-01 | 37 | 2 |
| BRCA | TCGA-A2-A0CP-01 | 85 | 8 |
| BRCA | TCGA-A2-A0CQ-01 | 39 | 1 |

## TABLE 5 (APPENDIX A)

| | | | |
|---|---|---|---|
| BRCA | TCGA-A2-A0CR-01 | 121 | 9 |
| BRCA | TCGA-A2-A0CS-01 | 34 | 4 |
| BRCA | TCGA-A2-A0CT-01 | 79 | 2 |
| BRCA | TCGA-A2-A0CU-01 | 45 | 1 |
| BRCA | TCGA-A2-A0CV-01 | 23 | 1 |
| BRCA | TCGA-A2-A0CW-01 | 61 | 5 |
| BRCA | TCGA-A2-A0CX-01 | 204 | 8 |
| BRCA | TCGA-A2-A0CZ-01 | 19 | 0 |
| BRCA | TCGA-A2-A0D0-01 | 66 | 5 |
| BRCA | TCGA-A2-A0D1-01 | 75 | 3 |
| BRCA | TCGA-A2-A0D2-01 | 80 | 4 |
| BRCA | TCGA-A2-A0D3-01 | 27 | 4 |
| BRCA | TCGA-A2-A0D4-01 | 31 | 0 |
| BRCA | TCGA-A2-A0EM-01 | 28 | 2 |
| BRCA | TCGA-A2-A0EN-01 | 36 | 5 |
| BRCA | TCGA-A2-A0EO-01 | 33 | 2 |
| BRCA | TCGA-A2-A0EP-01 | 9 | 0 |
| BRCA | TCGA-A2-A0EQ-01 | 84 | 6 |
| BRCA | TCGA-A2-A0ER-01 | 29 | 2 |
| BRCA | TCGA-A2-A0ES-01 | 5 | 0 |
| BRCA | TCGA-A2-A0ET-01 | 33 | 0 |
| BRCA | TCGA-A2-A0EU-01 | 40 | 3 |
| BRCA | TCGA-A2-A0EV-01 | 62 | 5 |
| BRCA | TCGA-A2-A0EW-01 | 14 | 1 |
| BRCA | TCGA-A2-A0EX-01 | 30 | 2 |
| BRCA | TCGA-A2-A0EY-01 | 212 | 11 |
| BRCA | TCGA-A2-A0ST-01 | 9 | 1 |
| BRCA | TCGA-A2-A0SU-01 | 23 | 1 |
| BRCA | TCGA-A2-A0SV-01 | 36 | 4 |
| BRCA | TCGA-A2-A0SW-01 | 54 | 4 |
| BRCA | TCGA-A2-A0SX-01 | 27 | 3 |
| BRCA | TCGA-A2-A0SY-01 | 131 | 3 |
| BRCA | TCGA-A2-A0T0-01 | 334 | 13 |
| BRCA | TCGA-A2-A0T1-01 | 33 | 2 |
| BRCA | TCGA-A2-A0T2-01 | 47 | 3 |
| BRCA | TCGA-A2-A0T3-01 | 38 | 0 |
| BRCA | TCGA-A2-A0T4-01 | 33 | 4 |
| BRCA | TCGA-A2-A0T5-01 | 1574 | 54 |
| BRCA | TCGA-A2-A0T6-01 | 124 | 5 |
| BRCA | TCGA-A2-A0T7-01 | 23 | 4 |
| BRCA | TCGA-A2-A0YC-01 | 18 | 4 |
| BRCA | TCGA-A2-A0YD-01 | 64 | 3 |
| BRCA | TCGA-A2-A0YE-01 | 56 | 4 |
| BRCA | TCGA-A2-A0YF-01 | 30 | 2 |
| BRCA | TCGA-A2-A0YG-01 | 54 | 3 |
| BRCA | TCGA-A2-A0YH-01 | 60 | 3 |
| BRCA | TCGA-A2-A0YI-01 | 14 | 2 |
| BRCA | TCGA-A2-A0YJ-01 | 55 | 3 |
| BRCA | TCGA-A2-A0YK-01 | 197 | 11 |
| BRCA | TCGA-A2-A0YL-01 | 13 | 1 |
| BRCA | TCGA-A2-A0YM-01 | 71 | 3 |
| BRCA | TCGA-A2-A0YT-01 | 53 | 4 |
| BRCA | TCGA-A2-A1FV-01 | 43 | 1 |
| BRCA | TCGA-A2-A1FW-01 | 70 | 3 |
| BRCA | TCGA-A2-A1FX-01 | 20 | 4 |
| BRCA | TCGA-A2-A1FZ-01 | 32 | 1 |
| BRCA | TCGA-A2-A1G0-01 | 24 | 3 |
| BRCA | TCGA-A2-A1G1-01 | 32 | 3 |
| BRCA | TCGA-A2-A1G4-01 | 36 | 2 |
| BRCA | TCGA-A2-A1G6-01 | 6 | 0 |
| BRCA | TCGA-A2-A259-01 | 16 | 2 |
| BRCA | TCGA-A2-A25A-01 | 103 | 4 |
| BRCA | TCGA-A2-A25B-01 | 88 | 5 |
| BRCA | TCGA-A2-A25C-01 | 25 | 1 |
| BRCA | TCGA-A2-A25D-01 | 56 | 3 |
| BRCA | TCGA-A2-A25E-01 | 57 | 3 |
| BRCA | TCGA-A2-A25F-01 | 8 | 1 |
| BRCA | TCGA-A2-A3KC-01 | 41 | 4 |
| BRCA | TCGA-A2-A3KD-01 | 19 | 0 |
| BRCA | TCGA-A2-A3X5-01 | 34 | 4 |
| BRCA | TCGA-A2-A3XT-01 | 103 | 4 |
| BRCA | TCGA-A2-A3XU-01 | 31 | 1 |
| BRCA | TCGA-A2-A3XV-01 | 57 | 7 |
| BRCA | TCGA-A2-A3XW-01 | 12 | 0 |
| BRCA | TCGA-A2-A3XX-01 | 42 | 2 |
| BRCA | TCGA-A2-A3XY-01 | 60 | 1 |
| BRCA | TCGA-A2-A3XZ-01 | 53 | 0 |
| BRCA | TCGA-A2-A3Y0-01 | 198 | 6 |
| BRCA | TCGA-A2-A4RW-01 | 126 | 4 |
| BRCA | TCGA-A2-A4RX-01 | 25 | 1 |
| BRCA | TCGA-A2-A4RY-01 | 35 | 1 |
| BRCA | TCGA-A2-A4S0-01 | 17 | 1 |
| BRCA | TCGA-A2-A4S1-01 | 80 | 7 |
| BRCA | TCGA-A2-A4S2-01 | 45 | 6 |
| BRCA | TCGA-A2-A4S3-01 | 90 | 3 |
| BRCA | TCGA-A7-A0CD-01 | 35 | 2 |
| BRCA | TCGA-A7-A0CE-01 | 208 | 6 |
| BRCA | TCGA-A7-A0CG-01 | 35 | 2 |
| BRCA | TCGA-A7-A0CH-01 | 50 | 5 |
| BRCA | TCGA-A7-A0CJ-01 | 51 | 2 |
| BRCA | TCGA-A7-A0D9-01 | 50 | 4 |
| BRCA | TCGA-A7-A0DA-01 | 133 | 3 |
| BRCA | TCGA-A7-A0DB-01 | 135 | 10 |
| BRCA | TCGA-A7-A0DC-01 | 1 | 1 |
| BRCA | TCGA-A7-A13D-01 | 220 | 7 |
| BRCA | TCGA-A7-A13E-01 | 297 | 11 |
| BRCA | TCGA-A7-A13F-01 | 64 | 4 |
| BRCA | TCGA-A7-A13G-01 | 27 | 1 |
| BRCA | TCGA-A7-A13H-01 | 27 | 3 |
| BRCA | TCGA-A7-A26E-01 | 379 | 23 |
| BRCA | TCGA-A7-A26F-01 | 101 | 5 |
| BRCA | TCGA-A7-A26G-01 | 56 | 4 |
| BRCA | TCGA-A7-A26H-01 | 102 | 9 |
| BRCA | TCGA-A7-A26I-01 | 36 | 4 |
| BRCA | TCGA-A7-A26J-01 | 215 | 9 |
| BRCA | TCGA-A7-A2KD-01 | 49 | 3 |
| BRCA | TCGA-A7-A3IY-01 | 26 | 2 |
| BRCA | TCGA-A7-A3IZ-01 | 130 | 4 |
| BRCA | TCGA-A7-A3J0-01 | 87 | 0 |
| BRCA | TCGA-A7-A3J1-01 | 33 | 2 |
| BRCA | TCGA-A7-A3RF-01 | 73 | 5 |
| BRCA | TCGA-A7-A425-01 | 49 | 2 |
| BRCA | TCGA-A7-A426-01 | 46 | 3 |
| BRCA | TCGA-A7-A4SA-01 | 169 | 12 |
| BRCA | TCGA-A7-A4SB-01 | 43 | 5 |
| BRCA | TCGA-A7-A4SC-01 | 81 | 4 |
| BRCA | TCGA-A7-A4SD-01 | 110 | 4 |
| BRCA | TCGA-A7-A4SE-01 | 208 | 10 |
| BRCA | TCGA-A7-A4SF-01 | 88 | 3 |
| BRCA | TCGA-A7-A56D-01 | 288 | 13 |
| BRCA | TCGA-A7-A5ZV-01 | 215 | 8 |
| BRCA | TCGA-A7-A5ZW-01 | 31 | 5 |
| BRCA | TCGA-A7-A5ZX-01 | 60 | 8 |
| BRCA | TCGA-A8-A06N-01 | 34 | 1 |
| BRCA | TCGA-A8-A06O-01 | 66 | 6 |
| BRCA | TCGA-A8-A06P-01 | 44 | 6 |
| BRCA | TCGA-A8-A06Q-01 | 216 | 7 |
| BRCA | TCGA-A8-A06R-01 | 59 | 7 |
| BRCA | TCGA-A8-A06T-01 | 28 | 3 |
| BRCA | TCGA-A8-A06U-01 | 59 | 4 |
| BRCA | TCGA-A8-A06X-01 | 192 | 12 |
| BRCA | TCGA-A8-A06Y-01 | 33 | 3 |
| BRCA | TCGA-A8-A06Z-01 | 61 | 5 |
| BRCA | TCGA-A8-A075-01 | 74 | 5 |
| BRCA | TCGA-A8-A076-01 | 56 | 5 |
| BRCA | TCGA-A8-A079-01 | 53 | 3 |
| BRCA | TCGA-A8-A07B-01 | 68 | 5 |
| BRCA | TCGA-A8-A07C-01 | 138 | 5 |
| BRCA | TCGA-A8-A07E-01 | 76 | 4 |
| BRCA | TCGA-A8-A07F-01 | 31 | 3 |
| BRCA | TCGA-A8-A07G-01 | 51 | 3 |
| BRCA | TCGA-A8-A07I-01 | 41 | 3 |
| BRCA | TCGA-A8-A07J-01 | 46 | 1 |
| BRCA | TCGA-A8-A07L-01 | 100 | 7 |
| BRCA | TCGA-A8-A07O-01 | 41 | 6 |
| BRCA | TCGA-A8-A07P-01 | 53 | 3 |
| BRCA | TCGA-A8-A07R-01 | 472 | 16 |
| BRCA | TCGA-A8-A07U-01 | 38 | 2 |
| BRCA | TCGA-A8-A07W-01 | 81 | 4 |
| BRCA | TCGA-A8-A07Z-01 | 25 | 3 |
| BRCA | TCGA-A8-A081-01 | 84 | 3 |
| BRCA | TCGA-A8-A082-01 | 25 | 2 |
| BRCA | TCGA-A8-A083-01 | 31 | 2 |
| BRCA | TCGA-A8-A084-01 | 66 | 4 |
| BRCA | TCGA-A8-A085-01 | 70 | 3 |
| BRCA | TCGA-A8-A086-01 | 24 | 1 |
| BRCA | TCGA-A8-A08A-01 | 32 | 2 |
| BRCA | TCGA-A8-A08B-01 | 34 | 3 |
| BRCA | TCGA-A8-A08C-01 | 3 | 1 |
| BRCA | TCGA-A8-A08F-01 | 117 | 4 |
| BRCA | TCGA-A8-A08G-01 | 36 | 0 |
| BRCA | TCGA-A8-A08H-01 | 56 | 2 |
| BRCA | TCGA-A8-A08I-01 | 41 | 1 |
| BRCA | TCGA-A8-A08J-01 | 31 | 1 |
| BRCA | TCGA-A8-A08L-01 | 203 | 5 |
| BRCA | TCGA-A8-A08O-01 | 26 | 2 |
| BRCA | TCGA-A8-A08P-01 | 38 | 2 |
| BRCA | TCGA-A8-A08R-01 | 172 | 6 |
| BRCA | TCGA-A8-A08S-01 | 53 | 3 |
| BRCA | TCGA-A8-A08T-01 | 50 | 5 |
| BRCA | TCGA-A8-A08X-01 | 18 | 1 |
| BRCA | TCGA-A8-A08Z-01 | 33 | 6 |
| BRCA | TCGA-A8-A090-01 | 36 | 1 |
| BRCA | TCGA-A8-A091-01 | 29 | 2 |
| BRCA | TCGA-A8-A092-01 | 87 | 6 |
| BRCA | TCGA-A8-A093-01 | 102 | 7 |
| BRCA | TCGA-A8-A094-01 | 178 | 9 |
| BRCA | TCGA-A8-A095-01 | 125 | 8 |
| BRCA | TCGA-A8-A096-01 | 30 | 2 |
| BRCA | TCGA-A8-A097-01 | 120 | 4 |
| BRCA | TCGA-A8-A099-01 | 22 | 1 |
| BRCA | TCGA-A8-A09A-01 | 105 | 5 |
| BRCA | TCGA-A8-A098-01 | 41 | 2 |
| BRCA | TCGA-A8-A09C-01 | 30 | 1 |
| BRCA | TCGA-A8-A09D-01 | 49 | 2 |
| BRCA | TCGA-A8-A09E-01 | 47 | 2 |
| BRCA | TCGA-A8-A09G-01 | 244 | 5 |
| BRCA | TCGA-A8-A09I-01 | 133 | 9 |
| BRCA | TCGA-A8-A09K-01 | 37 | 2 |
| BRCA | TCGA-A8-A09M-01 | 95 | 6 |
| BRCA | TCGA-A8-A09N-01 | 81 | 4 |
| BRCA | TCGA-A8-A09Q-01 | 98 | 4 |
| BRCA | TCGA-A8-A09R-01 | 50 | 2 |
| BRCA | TCGA-A8-A09T-01 | 27 | 2 |
| BRCA | TCGA-A8-A09V-01 | 25 | 2 |
| BRCA | TCGA-A8-A09W-01 | 81 | 9 |
| BRCA | TCGA-A8-A09X-01 | 58 | 4 |
| BRCA | TCGA-A8-A09Z-01 | 1438 | 52 |
| BRCA | TCGA-A8-A0A1-01 | 37 | 3 |
| BRCA | TCGA-A8-A0A2-01 | 35 | 1 |
| BRCA | TCGA-A8-A0A4-01 | 35 | 2 |
| BRCA | TCGA-A8-A0A6-01 | 3175 | 110 |
| BRCA | TCGA-A8-A0A7-01 | 91 | 8 |
| BRCA | TCGA-A8-A0A9-01 | 50 | 4 |
| BRCA | TCGA-A8-A0AB-01 | 27 | 3 |
| BRCA | TCGA-A8-A0AD-01 | 32 | 4 |
| BRCA | TCGA-AC-A23C-01 | 99 | 6 |
| BRCA | TCGA-AC-A23E-01 | 18 | 3 |
| BRCA | TCGA-AC-A23G-01 | 30 | 1 |
| BRCA | TCGA-AC-A23H-01 | 4714 | 170 |
| BRCA | TCGA-AC-A2B8-01 | 103 | 6 |
| BRCA | TCGA-AC-A2BK-01 | 99 | 0 |
| BRCA | TCGA-AC-A2BM-01 | 26 | 1 |
| BRCA | TCGA-AC-A2FB-01 | 19 | 3 |
| BRCA | TCGA-AC-A2FE-01 | 35 | 1 |
| BRCA | TCGA-AC-A2FF-01 | 16 | 5 |
| BRCA | TCGA-AC-A2FG-01 | 54 | 4 |
| BRCA | TCGA-AC-A2FK-01 | 1 | 0 |
| BRCA | TCGA-AC-A2FM-01 | 61 | 3 |
| BRCA | TCGA-AC-A2FO-01 | 25 | 2 |
| BRCA | TCGA-AC-A2QH-01 | 88 | 5 |
| BRCA | TCGA-AC-A2QI-01 | 91 | 5 |
| BRCA | TCGA-AC-A2QJ-01 | 99 | 5 |
| BRCA | TCGA-AC-A3BB-01 | 143 | 8 |
| BRCA | TCGA-AC-A3EH-01 | 66 | 2 |
| BRCA | TCGA-AC-A3HN-01 | 21 | 2 |
| BRCA | TCGA-AC-A3OD-01 | 154 | 11 |
| BRCA | TCGA-AC-A3QP-01 | 35 | 2 |
| BRCA | TCGA-AC-A3TM-01 | 106 | 6 |
| BRCA | TCGA-AC-A3TN-01 | 233 | 7 |
| BRCA | TCGA-AC-A3W5-01 | 160 | 3 |
| BRCA | TCGA-AC-A3W6-01 | 272 | 10 |
| BRCA | TCGA-AC-A3W7-01 | 90 | 3 |
| BRCA | TCGA-AC-A3YI-01 | 20 | 2 |
| BRCA | TCGA-AC-A3YJ-01 | 35 | 2 |
| BRCA | TCGA-AC-A5EH-01 | 160 | 9 |
| BRCA | TCGA-AC-A5EI-01 | 36 | 1 |
| BRCA | TCGA-AC-A5XS-01 | 1079 | 61 |
| BRCA | TCGA-AC-A5XU-01 | 74 | 5 |
| BRCA | TCGA-AC-A62X-01 | 68 | 4 |
| BRCA | TCGA-AC-A62Y-01 | 73 | 3 |
| BRCA | TCGA-AN-A03X-01 | 43 | 5 |
| BRCA | TCGA-AN-A03Y-01 | 29 | 3 |
| BRCA | TCGA-AN-A041-01 | 24 | 1 |
| BRCA | TCGA-AN-A046-01 | 5702 | 184 |
| BRCA | TCGA-AN-A049-01 | 36 | 4 |
| BRCA | TCGA-AN-A04A-01 | 31 | 4 |
| BRCA | TCGA-AN-A04C-01 | 79 | 3 |
| BRCA | TCGA-AN-A04D-01 | 98 | 2 |
| BRCA | TCGA-AN-A0AJ-01 | 80 | 3 |
| BRCA | TCGA-AN-A0AK-01 | 1317 | 76 |
| BRCA | TCGA-AN-A0AL-01 | 79 | 4 |
| BRCA | TCGA-AN-A0AM-01 | 51 | 6 |
| BRCA | TCGA-AN-A0AR-01 | 118 | 5 |
| BRCA | TCGA-AN-A0AS-01 | 41 | 3 |
| BRCA | TCGA-AN-A0AT-01 | 112 | 6 |
| BRCA | TCGA-AN-A0FD-01 | 42 | 1 |
| BRCA | TCGA-AN-A0FF-01 | 29 | 2 |
| BRCA | TCGA-AN-A0FJ-01 | 86 | 4 |
| BRCA | TCGA-AN-A0FK-01 | 45 | 0 |
| BRCA | TCGA-AN-A0FL-01 | 181 | 7 |
| BRCA | TCGA-AN-A0FN-01 | 33 | 2 |
| BRCA | TCGA-AN-A0FS-01 | 68 | 5 |
| BRCA | TCGA-AN-A0FT-01 | 98 | 4 |

## TABLE 5 (APPENDIX A)

| | | | | | | | | | | | |
|---|---|---|---|---|---|---|---|---|---|---|---|
| BRCA | TCGA-AN-A0FV-01 | 86 | 3 | BRCA | TCGA-AR-A1AT-01 | 30 | 2 | BRCA | TCGA-B6-A2IU-01 | 44 | 4 |
| BRCA | TCGA-AN-A0FW-01 | 104 | 4 | BRCA | TCGA-AR-A1AU-01 | 21 | 1 | BRCA | TCGA-B6-A3ZX-01 | 57 | 2 |
| BRCA | TCGA-AN-A0FX-01 | 148 | 12 | BRCA | TCGA-AR-A1AV-01 | 40 | 3 | BRCA | TCGA-B6-A400-01 | 134 | 6 |
| BRCA | TCGA-AN-A0FY-01 | 60 | 3 | BRCA | TCGA-AR-A1AW-01 | 20 | 5 | BRCA | TCGA-B6-A401-01 | 27 | 3 |
| BRCA | TCGA-AN-A0FZ-01 | 46 | 3 | BRCA | TCGA-AR-A1AX-01 | 15 | 1 | BRCA | TCGA-B6-A402-01 | 68 | 3 |
| BRCA | TCGA-AN-A0G0-01 | 41 | 5 | BRCA | TCGA-AR-A1AY-01 | 51 | 2 | BRCA | TCGA-B6-A408-01 | 54 | 6 |
| BRCA | TCGA-AN-A0XL-01 | 30 | 2 | BRCA | TCGA-AR-A24H-01 | 126 | 3 | BRCA | TCGA-B6-A409-01 | 39 | 2 |
| BRCA | TCGA-AN-A0XN-01 | 121 | 6 | BRCA | TCGA-AR-A24K-01 | 29 | 3 | BRCA | TCGA-B6-A40B-01 | 64 | 3 |
| BRCA | TCGA-AN-A0XO-01 | 29 | 2 | BRCA | TCGA-AR-A24L-01 | 47 | 2 | BRCA | TCGA-B6-A40C-01 | 41 | 3 |
| BRCA | TCGA-AN-A0XP-01 | 27 | 3 | BRCA | TCGA-AR-A24M-01 | 24 | 3 | BRCA | TCGA-BH-A0AU-01 | 20 | 1 |
| BRCA | TCGA-AN-A0XR-01 | 35 | 5 | BRCA | TCGA-AR-A24N-01 | 34 | 2 | BRCA | TCGA-BH-A0AV-01 | 68 | 1 |
| BRCA | TCGA-AN-A0XS-01 | 27 | 2 | BRCA | TCGA-AR-A24O-01 | 18 | 1 | BRCA | TCGA-BH-A0AW-01 | 147 | 4 |
| BRCA | TCGA-AN-A0XT-01 | 18 | 2 | BRCA | TCGA-AR-A24P-01 | 22 | 3 | BRCA | TCGA-BH-A0AY-01 | 104 | 2 |
| BRCA | TCGA-AN-A0XU-01 | 99 | 6 | BRCA | TCGA-AR-A24Q-01 | 145 | 9 | BRCA | TCGA-BH-A0AZ-01 | 58 | 2 |
| BRCA | TCGA-AN-A0XV-01 | 29 | 2 | BRCA | TCGA-AR-A24R-01 | 26 | 2 | BRCA | TCGA-BH-A0B0-01 | 51 | 5 |
| BRCA | TCGA-AN-A0XW-01 | 224 | 10 | BRCA | TCGA-AR-A24S-01 | 44 | 4 | BRCA | TCGA-BH-A0B1-01 | 59 | 0 |
| BRCA | TCGA-AO-A03L-01 | 25 | 3 | BRCA | TCGA-AR-A24T-01 | 24 | 4 | BRCA | TCGA-BH-A0B3-01 | 71 | 8 |
| BRCA | TCGA-AO-A03M-01 | 783 | 25 | BRCA | TCGA-AR-A24U-01 | 21 | 2 | BRCA | TCGA-BH-A0B4-01 | 64 | 0 |
| BRCA | TCGA-AO-A03N-01 | 103 | 4 | BRCA | TCGA-AR-A24V-01 | 29 | 0 | BRCA | TCGA-BH-A0B5-01 | 64 | 7 |
| BRCA | TCGA-AO-A03O-01 | 64 | 3 | BRCA | TCGA-AR-A24W-01 | 7 | 0 | BRCA | TCGA-BH-A0B6-01 | 830 | 29 |
| BRCA | TCGA-AO-A03P-01 | 49 | 1 | BRCA | TCGA-AR-A24X-01 | 31 | 2 | BRCA | TCGA-BH-A0B7-01 | 25 | 2 |
| BRCA | TCGA-AO-A03R-01 | 44 | 1 | BRCA | TCGA-AR-A24Z-01 | 40 | 0 | BRCA | TCGA-BH-A0B8-01 | 78 | 8 |
| BRCA | TCGA-AO-A03T-01 | 93 | 6 | BRCA | TCGA-AR-A250-01 | 69 | 2 | BRCA | TCGA-BH-A0B9-01 | 39 | 6 |
| BRCA | TCGA-AO-A03U-01 | 9 | 0 | BRCA | TCGA-AR-A251-01 | 153 | 6 | BRCA | TCGA-BH-A0BA-01 | 58 | 6 |
| BRCA | TCGA-AO-A03V-01 | 54 | 3 | BRCA | TCGA-AR-A252-01 | 9 | 2 | BRCA | TCGA-BH-A0BC-01 | 135 | 8 |
| BRCA | TCGA-AO-A0J2-01 | 71 | 2 | BRCA | TCGA-AR-A254-01 | 31 | 3 | BRCA | TCGA-BH-A0BD-01 | 20 | 1 |
| BRCA | TCGA-AO-A0J3-01 | 79 | 4 | BRCA | TCGA-AR-A255-01 | 59 | 4 | BRCA | TCGA-BH-A0BF-01 | 32 | 3 |
| BRCA | TCGA-AO-A0J4-01 | 91 | 6 | BRCA | TCGA-AR-A256-01 | 187 | 3 | BRCA | TCGA-BH-A0BG-01 | 45 | 1 |
| BRCA | TCGA-AO-A0J5-01 | 60 | 2 | BRCA | TCGA-AR-A2LE-01 | 315 | 13 | BRCA | TCGA-BH-A0BJ-01 | 53 | 5 |
| BRCA | TCGA-AO-A0J6-01 | 106 | 3 | BRCA | TCGA-AR-A2LH-01 | 22 | 1 | BRCA | TCGA-BH-A0BL-01 | 97 | 4 |
| BRCA | TCGA-AO-A0J7-01 | 25 | 2 | BRCA | TCGA-AR-A2LJ-01 | 16 | 3 | BRCA | TCGA-BH-A0BM-01 | 67 | 8 |
| BRCA | TCGA-AO-A0J8-01 | 28 | 2 | BRCA | TCGA-AR-A2LK-01 | 38 | 2 | BRCA | TCGA-BH-A0BO-01 | 44 | 5 |
| BRCA | TCGA-AO-A0J9-01 | 98 | 2 | BRCA | TCGA-AR-A2LL-01 | 30 | 2 | BRCA | TCGA-BH-A0BP-01 | 136 | 7 |
| BRCA | TCGA-AO-A0JA-01 | 32 | 2 | BRCA | TCGA-AR-A2LM-01 | 15 | 2 | BRCA | TCGA-BH-A0BQ-01 | 37 | 0 |
| BRCA | TCGA-AO-A0JB-01 | 86 | 3 | BRCA | TCGA-AR-A2LN-01 | 38 | 3 | BRCA | TCGA-BH-A0BR-01 | 65 | 3 |
| BRCA | TCGA-AO-A0JC-01 | 11 | 0 | BRCA | TCGA-AR-A2LO-01 | 12 | 0 | BRCA | TCGA-BH-A0BS-01 | 14 | 1 |
| BRCA | TCGA-AO-A0JD-01 | 106 | 7 | BRCA | TCGA-AR-A2LQ-01 | 8 | 0 | BRCA | TCGA-BH-A0BT-01 | 35 | 5 |
| BRCA | TCGA-AO-A0JE-01 | 40 | 3 | BRCA | TCGA-AR-A2LR-01 | 61 | 6 | BRCA | TCGA-BH-A0BW-01 | 64 | 2 |
| BRCA | TCGA-AO-A0JF-01 | 30 | 7 | BRCA | TCGA-AR-A5QM-01 | 35 | 2 | BRCA | TCGA-BH-A0BW-01 | 142 | 6 |
| BRCA | TCGA-AO-A0JI-01 | 16 | 0 | BRCA | TCGA-AR-A5QN-01 | 45 | 0 | BRCA | TCGA-BH-A0BZ-01 | 224 | 3 |
| BRCA | TCGA-AO-A0JJ-01 | 15 | 2 | BRCA | TCGA-AR-A5QP-01 | 28 | 1 | BRCA | TCGA-BH-A0C0-01 | 140 | 4 |
| BRCA | TCGA-AO-A0JL-01 | 62 | 3 | BRCA | TCGA-AR-A5QQ-01 | 61 | 2 | BRCA | TCGA-BH-A0C1-01 | 33 | 3 |
| BRCA | TCGA-AO-A0JM-01 | 34 | 4 | BRCA | TCGA-B6-A0I1-01 | 65 | 4 | BRCA | TCGA-BH-A0C3-01 | 15 | 3 |
| BRCA | TCGA-AO-A124-01 | 138 | 7 | BRCA | TCGA-B6-A0I2-01 | 51 | 2 | BRCA | TCGA-BH-A0C7-01 | 38 | 6 |
| BRCA | TCGA-AO-A125-01 | 41 | 4 | BRCA | TCGA-B6-A0I5-01 | 24 | 3 | BRCA | TCGA-BH-A0DD-01 | 22 | 1 |
| BRCA | TCGA-AO-A126-01 | 21 | 1 | BRCA | TCGA-B6-A0I6-01 | 106 | 5 | BRCA | TCGA-BH-A0DE-01 | 55 | 8 |
| BRCA | TCGA-AO-A128-01 | 998 | 37 | BRCA | TCGA-B6-A0I8-01 | 108 | 6 | BRCA | TCGA-BH-A0DG-01 | 25 | 2 |
| BRCA | TCGA-AO-A129-01 | 87 | 3 | BRCA | TCGA-B6-A0I9-01 | 75 | 9 | BRCA | TCGA-BH-A0DH-01 | 61 | 3 |
| BRCA | TCGA-AO-A12A-01 | 16 | 4 | BRCA | TCGA-B6-A0IA-01 | 50 | 1 | BRCA | TCGA-BH-A0DI-01 | 32 | 2 |
| BRCA | TCGA-AO-A12B-01 | 17 | 1 | BRCA | TCGA-B6-A0IB-01 | 60 | 2 | BRCA | TCGA-BH-A0DK-01 | 172 | 4 |
| BRCA | TCGA-AO-A12D-01 | 36 | 3 | BRCA | TCGA-B6-A0IC-01 | 48 | 1 | BRCA | TCGA-BH-A0DL-01 | 82 | 7 |
| BRCA | TCGA-AO-A12E-01 | 644 | 10 | BRCA | TCGA-B6-A0IE-01 | 32 | 2 | BRCA | TCGA-BH-A0DO-01 | 22 | 1 |
| BRCA | TCGA-AO-A12F-01 | 32 | 1 | BRCA | TCGA-B6-A0IG-01 | 45 | 2 | BRCA | TCGA-BH-A0DP-01 | 73 | 3 |
| BRCA | TCGA-AO-A12G-01 | 36 | 4 | BRCA | TCGA-B6-A0IH-01 | 24 | 1 | BRCA | TCGA-BH-A0DQ-01 | 53 | 4 |
| BRCA | TCGA-AO-A12H-01 | 21 | 2 | BRCA | TCGA-B6-A0IJ-01 | 136 | 7 | BRCA | TCGA-BH-A0DS-01 | 36 | 4 |
| BRCA | TCGA-AO-A1KO-01 | 35 | 0 | BRCA | TCGA-B6-A0IK-01 | 151 | 10 | BRCA | TCGA-BH-A0DT-01 | 14 | 5 |
| BRCA | TCGA-AO-A1KP-01 | 22 | 1 | BRCA | TCGA-B6-A0IM-01 | 39 | 5 | BRCA | TCGA-BH-A0DV-01 | 5 | 2 |
| BRCA | TCGA-AO-A1KR-01 | 70 | 5 | BRCA | TCGA-B6-A0IN-01 | 41 | 4 | BRCA | TCGA-BH-A0DX-01 | 61 | 5 |
| BRCA | TCGA-AO-A1KS-01 | 43 | 0 | BRCA | TCGA-B6-A0IO-01 | 72 | 7 | BRCA | TCGA-BH-A0DZ-01 | 520 | 22 |
| BRCA | TCGA-AO-A1KT-01 | 42 | 1 | BRCA | TCGA-B6-A0IP-01 | 27 | 4 | BRCA | TCGA-BH-A0E0-01 | 59 | 4 |
| BRCA | TCGA-AQ-A04H-01 | 69 | 1 | BRCA | TCGA-B6-A0IQ-01 | 60 | 2 | BRCA | TCGA-BH-A0E1-01 | 64 | 6 |
| BRCA | TCGA-AQ-A04J-01 | 57 | 1 | BRCA | TCGA-B6-A0RE-01 | 104 | 6 | BRCA | TCGA-BH-A0E2-01 | 37 | 2 |
| BRCA | TCGA-AQ-A04L-01 | 51 | 2 | BRCA | TCGA-B6-A0RG-01 | 63 | 10 | BRCA | TCGA-BH-A0E6-01 | 56 | 3 |
| BRCA | TCGA-AQ-A0Y5-01 | 48 | 1 | BRCA | TCGA-B6-A0RH-01 | 41 | 4 | BRCA | TCGA-BH-A0E7-01 | 50 | 4 |
| BRCA | TCGA-AQ-A1H2-01 | 28 | 4 | BRCA | TCGA-B6-A0RI-01 | 18 | 3 | BRCA | TCGA-BH-A0E9-01 | 12 | 3 |
| BRCA | TCGA-AQ-A1H3-01 | 30 | 7 | BRCA | TCGA-B6-A0RN-01 | 38 | 4 | BRCA | TCGA-BH-A0EA-01 | 21 | 4 |
| BRCA | TCGA-AQ-A54N-01 | 82 | 4 | BRCA | TCGA-B6-A0RO-01 | 69 | 8 | BRCA | TCGA-BH-A0EB-01 | 38 | 3 |
| BRCA | TCGA-AQ-A54O-01 | 33 | 2 | BRCA | TCGA-B6-A0RP-01 | 21 | 2 | BRCA | TCGA-BH-A0EE-01 | 82 | 8 |
| BRCA | TCGA-AR-A0TS-01 | 41 | 4 | BRCA | TCGA-B6-A0RQ-01 | 17 | 3 | BRCA | TCGA-BH-A0EI-01 | 20 | 1 |
| BRCA | TCGA-AR-A0TU-01 | 79 | 8 | BRCA | TCGA-B6-A0RT-01 | 13 | 0 | BRCA | TCGA-BH-A0GY-01 | 47 | 3 |
| BRCA | TCGA-AR-A0TX-01 | 289 | 16 | BRCA | TCGA-B6-A0RU-01 | 43 | 1 | BRCA | TCGA-BH-A0GZ-01 | 28 | 1 |
| BRCA | TCGA-AR-A0TY-01 | 115 | 3 | BRCA | TCGA-B6-A0RV-01 | 46 | 5 | BRCA | TCGA-BH-A0H0-01 | 21 | 1 |
| BRCA | TCGA-AR-A0U0-01 | 50 | 4 | BRCA | TCGA-B6-A0WS-01 | 23 | 2 | BRCA | TCGA-BH-A0H3-01 | 17 | 4 |
| BRCA | TCGA-AR-A0U1-01 | 68 | 2 | BRCA | TCGA-B6-A0WT-01 | 21 | 3 | BRCA | TCGA-BH-A0H5-01 | 15 | 0 |
| BRCA | TCGA-AR-A0U2-01 | 57 | 8 | BRCA | TCGA-B6-A0WV-01 | 29 | 2 | BRCA | TCGA-BH-A0H6-01 | 18 | 1 |
| BRCA | TCGA-AR-A0U3-01 | 37 | 2 | BRCA | TCGA-B6-A0WW-01 | 37 | 2 | BRCA | TCGA-BH-A0H7-01 | 83 | 3 |
| BRCA | TCGA-AR-A1AH-01 | 94 | 2 | BRCA | TCGA-B6-A0WX-01 | 30 | 4 | BRCA | TCGA-BH-A0H9-01 | 75 | 5 |
| BRCA | TCGA-AR-A1AI-01 | 63 | 2 | BRCA | TCGA-B6-A0WY-01 | 15 | 1 | BRCA | TCGA-BH-A0HA-01 | 303 | 12 |
| BRCA | TCGA-AR-A1AJ-01 | 25 | 3 | BRCA | TCGA-B6-A0WZ-01 | 39 | 3 | BRCA | TCGA-BH-A0HB-01 | 34 | 2 |
| BRCA | TCGA-AR-A1AK-01 | 64 | 4 | BRCA | TCGA-B6-A0X0-01 | 22 | 3 | BRCA | TCGA-BH-A0HF-01 | 916 | 37 |
| BRCA | TCGA-AR-A1AL-01 | 25 | 4 | BRCA | TCGA-B6-A0X1-01 | 64 | 2 | BRCA | TCGA-BH-A0HI-01 | 26 | 1 |
| BRCA | TCGA-AR-A1AM-01 | 30 | 3 | BRCA | TCGA-B6-A0X4-01 | 19 | 3 | BRCA | TCGA-BH-A0HK-01 | 207 | 0 |
| BRCA | TCGA-AR-A1AN-01 | 18 | 3 | BRCA | TCGA-B6-A0X5-01 | 36 | 2 | BRCA | TCGA-BH-A0HL-01 | 38 | 3 |
| BRCA | TCGA-AR-A1AO-01 | 8 | 0 | BRCA | TCGA-B6-A0X7-01 | 22 | 1 | BRCA | TCGA-BH-A0HN-01 | 27 | 2 |
| BRCA | TCGA-AR-A1AP-01 | 35 | 3 | BRCA | TCGA-B6-A1KC-01 | 27 | 1 | BRCA | TCGA-BH-A0HO-01 | 23 | 1 |
| BRCA | TCGA-AR-A1AQ-01 | 45 | 5 | BRCA | TCGA-B6-A1KF-01 | 53 | 2 | BRCA | TCGA-BH-A0HP-01 | 450 | 23 |
| BRCA | TCGA-AR-A1AR-01 | 54 | 1 | BRCA | TCGA-B6-A1KI-01 | 20 | 4 | BRCA | TCGA-BH-A0HQ-01 | 33 | 2 |
| BRCA | TCGA-AR-A1AS-01 | 42 | 7 | BRCA | TCGA-B6-A1KN-01 | 57 | 4 | BRCA | TCGA-BH-A0HU-01 | 37 | 0 |

## TABLE 5 (APPENDIX A)

| | | | |
|---|---|---|---|
| BRCA | TCGA-BH-A0HW-01 | 40 | 4 |
| BRCA | TCGA-BH-A0HX-01 | 57 | 1 |
| BRCA | TCGA-BH-A0HY-01 | 55 | 5 |
| BRCA | TCGA-BH-A0RX-01 | 18 | 2 |
| BRCA | TCGA-BH-A0W3-01 | 20 | 1 |
| BRCA | TCGA-BH-A0W4-01 | 32 | 4 |
| BRCA | TCGA-BH-A0W5-01 | 25 | 5 |
| BRCA | TCGA-BH-A0W7-01 | 177 | 8 |
| BRCA | TCGA-BH-A0WA-01 | 89 | 4 |
| BRCA | TCGA-BH-A18F-01 | 54 | 6 |
| BRCA | TCGA-BH-A18G-01 | 1516 | 48 |
| BRCA | TCGA-BH-A18H-01 | 29 | 3 |
| BRCA | TCGA-BH-A18I-01 | 24 | 1 |
| BRCA | TCGA-BH-A18J-01 | 63 | 4 |
| BRCA | TCGA-BH-A18K-01 | 43 | 6 |
| BRCA | TCGA-BH-A18L-01 | 41 | 0 |
| BRCA | TCGA-BH-A18M-01 | 13 | 1 |
| BRCA | TCGA-BH-A18N-01 | 31 | 3 |
| BRCA | TCGA-BH-A18P-01 | 315 | 14 |
| BRCA | TCGA-BH-A18Q-01 | 79 | 3 |
| BRCA | TCGA-BH-A18R-01 | 26 | 0 |
| BRCA | TCGA-BH-A18S-01 | 25 | 1 |
| BRCA | TCGA-BH-A18T-01 | 72 | 1 |
| BRCA | TCGA-BH-A18U-01 | 96 | 7 |
| BRCA | TCGA-BH-A18V-01 | 183 | 8 |
| BRCA | TCGA-BH-A18V-06 | 107 | 3 |
| BRCA | TCGA-BH-A1EN-01 | 47 | 1 |
| BRCA | TCGA-BH-A1EO-01 | 33 | 3 |
| BRCA | TCGA-BH-A1ES-01 | 18 | 1 |
| BRCA | TCGA-BH-A1ES-06 | 50 | 1 |
| BRCA | TCGA-BH-A1ET-01 | 23 | 2 |
| BRCA | TCGA-BH-A1EU-01 | 16 | 1 |
| BRCA | TCGA-BH-A1EV-01 | 84 | 6 |
| BRCA | TCGA-BH-A1EW-01 | 14 | 0 |
| BRCA | TCGA-BH-A1EX-01 | 16 | 1 |
| BRCA | TCGA-BH-A1EY-01 | 48 | 4 |
| BRCA | TCGA-BH-A1F0-01 | 21 | 1 |
| BRCA | TCGA-BH-A1F2-01 | 51 | 2 |
| BRCA | TCGA-BH-A1F5-01 | 32 | 4 |
| BRCA | TCGA-BH-A1F6-01 | 68 | 5 |
| BRCA | TCGA-BH-A1F8-01 | 137 | 8 |
| BRCA | TCGA-BH-A1FC-01 | 74 | 3 |
| BRCA | TCGA-BH-A1FD-01 | 28 | 2 |
| BRCA | TCGA-BH-A1FE-01 | 14 | 3 |
| BRCA | TCGA-BH-A1FG-01 | 19 | 0 |
| BRCA | TCGA-BH-A1FH-01 | 4 | 1 |
| BRCA | TCGA-BH-A1FJ-01 | 28 | 3 |
| BRCA | TCGA-BH-A1FL-01 | 25 | 1 |
| BRCA | TCGA-BH-A1FM-01 | 37 | 1 |
| BRCA | TCGA-BH-A1FN-01 | 111 | 4 |
| BRCA | TCGA-BH-A1FR-01 | 14 | 1 |
| BRCA | TCGA-BH-A1FU-01 | 27 | 2 |
| BRCA | TCGA-BH-A201-01 | 18 | 1 |
| BRCA | TCGA-BH-A202-01 | 28 | 5 |
| BRCA | TCGA-BH-A203-01 | 59 | 5 |
| BRCA | TCGA-BH-A204-01 | 40 | 4 |
| BRCA | TCGA-BH-A208-01 | 58 | 3 |
| BRCA | TCGA-BH-A209-01 | 75 | 3 |
| BRCA | TCGA-BH-A28O-01 | 25 | 0 |
| BRCA | TCGA-BH-A28Q-01 | 24 | 4 |
| BRCA | TCGA-BH-A2L8-01 | 416 | 21 |
| BRCA | TCGA-BH-A42T-01 | 38 | 2 |
| BRCA | TCGA-BH-A42U-01 | 16 | 0 |
| BRCA | TCGA-BH-A42V-01 | 38 | 4 |
| BRCA | TCGA-BH-A5IZ-01 | 156 | 9 |
| BRCA | TCGA-BH-A5J0-01 | 67 | 5 |
| BRCA | TCGA-C8-A12K-01 | 107 | 5 |
| BRCA | TCGA-C8-A12L-01 | 72 | 6 |
| BRCA | TCGA-C8-A12M-01 | 59 | 3 |
| BRCA | TCGA-C8-A12N-01 | 25 | 6 |
| BRCA | TCGA-C8-A12O-01 | 32 | 4 |
| BRCA | TCGA-C8-A12P-01 | 156 | 4 |
| BRCA | TCGA-C8-A12Q-01 | 102 | 3 |
| BRCA | TCGA-C8-A12T-01 | 155 | 10 |
| BRCA | TCGA-C8-A12U-01 | 38 | 2 |
| BRCA | TCGA-C8-A12V-01 | 15 | 0 |
| BRCA | TCGA-C8-A12W-01 | 46 | 2 |
| BRCA | TCGA-C8-A12X-01 | 45 | 1 |
| BRCA | TCGA-C8-A12Y-01 | 32 | 3 |
| BRCA | TCGA-C8-A12Z-01 | 48 | 3 |
| BRCA | TCGA-C8-A130-01 | 39 | 3 |
| BRCA | TCGA-C8-A131-01 | 56 | 5 |
| BRCA | TCGA-C8-A132-01 | 50 | 1 |
| BRCA | TCGA-C8-A133-01 | 22 | 2 |
| BRCA | TCGA-C8-A134-01 | 96 | 5 |
| BRCA | TCGA-C8-A135-01 | 50 | 4 |
| BRCA | TCGA-C8-A137-01 | 47 | 2 |
| BRCA | TCGA-C8-A138-01 | 49 | 2 |
| BRCA | TCGA-C8-A1HE-01 | 40 | 5 |
| BRCA | TCGA-C8-A1HF-01 | 33 | 4 |
| BRCA | TCGA-C8-A1HG-01 | 45 | 2 |
| BRCA | TCGA-C8-A1HI-01 | 37 | 1 |
| BRCA | TCGA-C8-A1HJ-01 | 116 | 6 |
| BRCA | TCGA-C8-A1HK-01 | 50 | 2 |
| BRCA | TCGA-C8-A1HL-01 | 29 | 2 |
| BRCA | TCGA-C8-A1HM-01 | 300 | 11 |
| BRCA | TCGA-C8-A1HN-01 | 49 | 7 |
| BRCA | TCGA-C8-A1HO-01 | 26 | 2 |
| BRCA | TCGA-C8-A26V-01 | 75 | 5 |
| BRCA | TCGA-C8-A26W-01 | 51 | 4 |
| BRCA | TCGA-C8-A26X-01 | 48 | 3 |
| BRCA | TCGA-C8-A26Y-01 | 696 | 21 |
| BRCA | TCGA-C8-A26Z-01 | 42 | 1 |
| BRCA | TCGA-C8-A273-01 | 20 | 1 |
| BRCA | TCGA-C8-A274-01 | 193 | 8 |
| BRCA | TCGA-C8-A275-01 | 123 | 2 |
| BRCA | TCGA-C8-A278-01 | 28 | 3 |
| BRCA | TCGA-C8-A27A-01 | 25 | 2 |
| BRCA | TCGA-C8-A27B-01 | 139 | 9 |
| BRCA | TCGA-C8-A3M7-01 | 180 | 13 |
| BRCA | TCGA-C8-A3M8-01 | 59 | 4 |
| BRCA | TCGA-D8-A13Y-01 | 69 | 1 |
| BRCA | TCGA-D8-A13Z-01 | 64 | 6 |
| BRCA | TCGA-D8-A140-01 | 93 | 4 |
| BRCA | TCGA-D8-A141-01 | 17 | 1 |
| BRCA | TCGA-D8-A142-01 | 66 | 3 |
| BRCA | TCGA-D8-A143-01 | 43 | 2 |
| BRCA | TCGA-D8-A145-01 | 27 | 3 |
| BRCA | TCGA-D8-A146-01 | 32 | 1 |
| BRCA | TCGA-D8-A147-01 | 120 | 7 |
| BRCA | TCGA-D8-A1J8-01 | 740 | 30 |
| BRCA | TCGA-D8-A1J9-01 | 171 | 9 |
| BRCA | TCGA-D8-A1JA-01 | 943 | 35 |
| BRCA | TCGA-D8-A1JB-01 | 27 | 3 |
| BRCA | TCGA-D8-A1JC-01 | 83 | 4 |
| BRCA | TCGA-D8-A1JD-01 | 82 | 8 |
| BRCA | TCGA-D8-A1JE-01 | 34 | 2 |
| BRCA | TCGA-D8-A1JF-01 | 45 | 4 |
| BRCA | TCGA-D8-A1JG-01 | 113 | 6 |
| BRCA | TCGA-D8-A1JH-01 | 21 | 4 |
| BRCA | TCGA-D8-A1JI-01 | 37 | 2 |
| BRCA | TCGA-D8-A1JJ-01 | 74 | 5 |
| BRCA | TCGA-D8-A1JK-01 | 180 | 7 |
| BRCA | TCGA-D8-A1JL-01 | 107 | 4 |
| BRCA | TCGA-D8-A1JM-01 | 41 | 2 |
| BRCA | TCGA-D8-A1JN-01 | 174 | 13 |
| BRCA | TCGA-D8-A1JP-01 | 106 | 6 |
| BRCA | TCGA-D8-A1JS-01 | 21 | 2 |
| BRCA | TCGA-D8-A1JT-01 | 33 | 2 |
| BRCA | TCGA-D8-A1JU-01 | 13 | 3 |
| BRCA | TCGA-D8-A1X5-01 | 50 | 2 |
| BRCA | TCGA-D8-A1X6-01 | 41 | 6 |
| BRCA | TCGA-D8-A1X7-01 | 26 | 0 |
| BRCA | TCGA-D8-A1X8-01 | 30 | 0 |
| BRCA | TCGA-D8-A1X9-01 | 88 | 5 |
| BRCA | TCGA-D8-A1XA-01 | 27 | 2 |
| BRCA | TCGA-D8-A1XB-01 | 31 | 2 |
| BRCA | TCGA-D8-A1XC-01 | 31 | 1 |
| BRCA | TCGA-D8-A1XF-01 | 40 | 1 |
| BRCA | TCGA-D8-A1XG-01 | 27 | 0 |
| BRCA | TCGA-D8-A1XI-01 | 80 | 3 |
| BRCA | TCGA-D8-A1XK-01 | 968 | 30 |
| BRCA | TCGA-D8-A1XL-01 | 95 | 4 |
| BRCA | TCGA-D8-A1XM-01 | 59 | 3 |
| BRCA | TCGA-D8-A1XO-01 | 18 | 5 |
| BRCA | TCGA-D8-A1XQ-01 | 743 | 31 |
| BRCA | TCGA-D8-A1XR-01 | 28 | 1 |
| BRCA | TCGA-D8-A1XS-01 | 22 | 2 |
| BRCA | TCGA-D8-A1XT-01 | 47 | 2 |
| BRCA | TCGA-D8-A1XU-01 | 27 | 3 |
| BRCA | TCGA-D8-A1XV-01 | 29 | 0 |
| BRCA | TCGA-D8-A1XW-01 | 90 | 3 |
| BRCA | TCGA-D8-A1XY-01 | 43 | 3 |
| BRCA | TCGA-D8-A1XZ-01 | 61 | 5 |
| BRCA | TCGA-D8-A1Y0-01 | 67 | 3 |
| BRCA | TCGA-D8-A1Y1-01 | 217 | 10 |
| BRCA | TCGA-D8-A1Y2-01 | 23 | 2 |
| BRCA | TCGA-D8-A1Y3-01 | 55 | 1 |
| BRCA | TCGA-D8-A27E-01 | 8 | 0 |
| BRCA | TCGA-D8-A27F-01 | 57 | 3 |
| BRCA | TCGA-D8-A27G-01 | 985 | 43 |
| BRCA | TCGA-D8-A27H-01 | 115 | 2 |
| BRCA | TCGA-D8-A27I-01 | 34 | 1 |
| BRCA | TCGA-D8-A27K-01 | 23 | 2 |
| BRCA | TCGA-D8-A27L-01 | 37 | 6 |
| BRCA | TCGA-D8-A27M-01 | 61 | 3 |
| BRCA | TCGA-D8-A27N-01 | 48 | 2 |
| BRCA | TCGA-D8-A27P-01 | 24 | 3 |
| BRCA | TCGA-D8-A27R-01 | 27 | 2 |
| BRCA | TCGA-D8-A27T-01 | 22 | 2 |
| BRCA | TCGA-D8-A27V-01 | 229 | 11 |
| BRCA | TCGA-D8-A27W-01 | 21 | 0 |
| BRCA | TCGA-D8-A3Z5-01 | 30 | 2 |
| BRCA | TCGA-D8-A3Z6-01 | 34 | 5 |
| BRCA | TCGA-D8-A4Z1-01 | 17 | 0 |
| BRCA | TCGA-E2-A105-01 | 59 | 4 |
| BRCA | TCGA-E2-A107-01 | 31 | 2 |
| BRCA | TCGA-E2-A108-01 | 53 | 1 |
| BRCA | TCGA-E2-A109-01 | 82 | 1 |
| BRCA | TCGA-E2-A10A-01 | 21 | 5 |
| BRCA | TCGA-E2-A10B-01 | 39 | 2 |
| BRCA | TCGA-E2-A10C-01 | 357 | 19 |
| BRCA | TCGA-E2-A10E-01 | 25 | 1 |
| BRCA | TCGA-E2-A10F-01 | 28 | 4 |
| BRCA | TCGA-E2-A14N-01 | 57 | 3 |
| BRCA | TCGA-E2-A14O-01 | 36 | 0 |
| BRCA | TCGA-E2-A14P-01 | 82 | 2 |
| BRCA | TCGA-E2-A14Q-01 | 24 | 0 |
| BRCA | TCGA-E2-A14R-01 | 112 | 4 |
| BRCA | TCGA-E2-A14S-01 | 37 | 1 |
| BRCA | TCGA-E2-A14T-01 | 33 | 1 |
| BRCA | TCGA-E2-A14U-01 | 20 | 2 |
| BRCA | TCGA-E2-A14V-01 | 75 | 2 |
| BRCA | TCGA-E2-A14W-01 | 84 | 2 |
| BRCA | TCGA-E2-A14X-01 | 25 | 1 |
| BRCA | TCGA-E2-A14Y-01 | 64 | 4 |
| BRCA | TCGA-E2-A14Z-01 | 62 | 1 |
| BRCA | TCGA-E2-A150-01 | 47 | 4 |
| BRCA | TCGA-E2-A152-01 | 121 | 5 |
| BRCA | TCGA-E2-A153-01 | 44 | 6 |
| BRCA | TCGA-E2-A154-01 | 42 | 1 |
| BRCA | TCGA-E2-A155-01 | 55 | 4 |
| BRCA | TCGA-E2-A156-01 | 17 | 3 |
| BRCA | TCGA-E2-A158-01 | 95 | 4 |
| BRCA | TCGA-E2-A159-01 | 148 | 11 |
| BRCA | TCGA-E2-A15A-01 | 40 | 1 |
| BRCA | TCGA-E2-A15A-06 | 31 | 1 |
| BRCA | TCGA-E2-A15C-01 | 29 | 5 |
| BRCA | TCGA-E2-A15D-01 | 28 | 2 |
| BRCA | TCGA-E2-A15E-01 | 28 | 2 |
| BRCA | TCGA-E2-A15E-06 | 36 | 2 |
| BRCA | TCGA-E2-A15F-01 | 21 | 0 |
| BRCA | TCGA-E2-A15G-01 | 59 | 7 |
| BRCA | TCGA-E2-A15H-01 | 32 | 4 |
| BRCA | TCGA-E2-A15I-01 | 193 | 8 |
| BRCA | TCGA-E2-A15J-01 | 24 | 2 |
| BRCA | TCGA-E2-A15K-01 | 73 | 8 |
| BRCA | TCGA-E2-A15K-06 | 92 | 12 |
| BRCA | TCGA-E2-A15L-01 | 38 | 2 |
| BRCA | TCGA-E2-A15M-01 | 124 | 3 |
| BRCA | TCGA-E2-A15O-01 | 36 | 2 |
| BRCA | TCGA-E2-A15P-01 | 46 | 2 |
| BRCA | TCGA-E2-A15R-01 | 43 | 0 |
| BRCA | TCGA-E2-A15S-01 | 45 | 2 |
| BRCA | TCGA-E2-A15T-01 | 44 | 2 |
| BRCA | TCGA-E2-A1AZ-01 | 53 | 0 |
| BRCA | TCGA-E2-A1B0-01 | 40 | 2 |
| BRCA | TCGA-E2-A1B1-01 | 14 | 1 |
| BRCA | TCGA-E2-A1B4-01 | 36 | 5 |
| BRCA | TCGA-E2-A1B5-01 | 27 | 8 |
| BRCA | TCGA-E2-A1B6-01 | 11 | 3 |
| BRCA | TCGA-E2-A1BC-01 | 68 | 8 |
| BRCA | TCGA-E2-A1BD-01 | 29 | 4 |
| BRCA | TCGA-E2-A1IE-01 | 21 | 2 |
| BRCA | TCGA-E2-A1IF-01 | 17 | 4 |
| BRCA | TCGA-E2-A1IG-01 | 42 | 3 |
| BRCA | TCGA-E2-A1IH-01 | 62 | 5 |
| BRCA | TCGA-E2-A1II-01 | 28 | 1 |
| BRCA | TCGA-E2-A1IJ-01 | 21 | 3 |
| BRCA | TCGA-E2-A1IK-01 | 18 | 2 |
| BRCA | TCGA-E2-A1IL-01 | 21 | 1 |
| BRCA | TCGA-E2-A1IN-01 | 193 | 8 |
| BRCA | TCGA-E2-A1IO-01 | 14 | 3 |
| BRCA | TCGA-E2-A1IU-01 | 24 | 0 |
| BRCA | TCGA-E2-A1L6-01 | 20 | 1 |
| BRCA | TCGA-E2-A1L7-01 | 67 | 2 |
| BRCA | TCGA-E2-A1L8-01 | 32 | 2 |
| BRCA | TCGA-E2-A1L9-01 | 23 | 5 |
| BRCA | TCGA-E2-A1LA-01 | 85 | 1 |
| BRCA | TCGA-E2-A1LB-01 | 39 | 5 |
| BRCA | TCGA-E2-A1LE-01 | 80 | 4 |
| BRCA | TCGA-E2-A1LG-01 | 130 | 9 |
| BRCA | TCGA-E2-A1LH-01 | 150 | 3 |
| BRCA | TCGA-E2-A1LI-01 | 15 | 0 |

# TABLE 5 (APPENDIX A)

| | | | | | | | | | | | |
|---|---|---|---|---|---|---|---|---|---|---|---|
| BRCA | TCGA-E2-A1LK-01 | 46 | 4 | BRCA | TCGA-EW-A1P6-01 | 23 | 2 | CESC | TCGA-C5-A1M7-01 | 119 | 4 |
| BRCA | TCGA-E2-A1LL-01 | 25 | 0 | BRCA | TCGA-EW-A1P7-01 | 9 | 1 | CESC | TCGA-C5-A1M8-01 | 225 | 6 |
| BRCA | TCGA-E2-A1LS-01 | 76 | 4 | BRCA | TCGA-EW-A1P8-01 | 46 | 3 | CESC | TCGA-C5-A1M9-01 | 201 | 10 |
| BRCA | TCGA-E2-A2P5-01 | 105 | 7 | BRCA | TCGA-EW-A1PA-01 | 36 | 3 | CESC | TCGA-C5-A1ME-01 | 68 | 3 |
| BRCA | TCGA-E2-A2P6-01 | 166 | 3 | BRCA | TCGA-EW-A1PB-01 | 138 | 2 | CESC | TCGA-C5-A1MF-01 | 94 | 5 |
| BRCA | TCGA-E2-A3DX-01 | 26 | 2 | BRCA | TCGA-EW-A1PC-01 | 154 | 6 | CESC | TCGA-C5-A1MH-01 | 315 | 16 |
| BRCA | TCGA-E2-A56Z-01 | 54 | 1 | BRCA | TCGA-EW-A1PD-01 | 73 | 3 | CESC | TCGA-C5-A1MI-01 | 265 | 13 |
| BRCA | TCGA-E2-A570-01 | 34 | 3 | BRCA | TCGA-EW-A1PE-01 | 35 | 6 | CESC | TCGA-C5-A1MJ-01 | 114 | 3 |
| BRCA | TCGA-E2-A573-01 | 61 | 2 | BRCA | TCGA-EW-A1PG-01 | 7 | 0 | CESC | TCGA-C5-A1MK-01 | 368 | 19 |
| BRCA | TCGA-E2-A574-01 | 121 | 6 | BRCA | TCGA-EW-A1PH-01 | 43 | 2 | CESC | TCGA-C5-A1ML-01 | 343 | 8 |
| BRCA | TCGA-E9-A1N3-01 | 28 | 2 | BRCA | TCGA-EW-A2FR-01 | 56 | 3 | CESC | TCGA-C5-A1MN-01 | 182 | 9 |
| BRCA | TCGA-E9-A1N4-01 | 29 | 3 | BRCA | TCGA-EW-A2FS-01 | 29 | 1 | CESC | TCGA-C5-A1MP-01 | 47 | 1 |
| BRCA | TCGA-E9-A1N5-01 | 30 | 2 | BRCA | TCGA-EW-A2FV-01 | 23 | 3 | CESC | TCGA-C5-A1MQ-01 | 188 | 6 |
| BRCA | TCGA-E9-A1N8-01 | 29 | 6 | BRCA | TCGA-EW-A2FW-01 | 21 | 2 | CESC | TCGA-C5-A2LS-01 | 84 | 3 |
| BRCA | TCGA-E9-A1N9-01 | 52 | 3 | BRCA | TCGA-EW-A3E8-01 | 101 | 6 | CESC | TCGA-C5-A2LT-01 | 7 | 0 |
| BRCA | TCGA-E9-A1NA-01 | 93 | 3 | BRCA | TCGA-EW-A3U0-01 | 147 | 5 | CESC | TCGA-C5-A2LV-01 | 5 | 0 |
| BRCA | TCGA-E9-A1NC-01 | 149 | 5 | BRCA | TCGA-EW-A423-01 | 72 | 4 | CESC | TCGA-C5-A2LX-01 | 240 | 12 |
| BRCA | TCGA-E9-A1ND-01 | 68 | 4 | BRCA | TCGA-GI-A2C8-01 | 109 | 6 | CESC | TCGA-C5-A2LY-01 | 4 | 0 |
| BRCA | TCGA-E9-A1NE-01 | 34 | 0 | BRCA | TCGA-GI-A2C9-01 | 160 | 4 | CESC | TCGA-C5-A2LZ-01 | 530 | 22 |
| BRCA | TCGA-E9-A1NF-01 | 45 | 3 | BRCA | TCGA-GM-A2D9-01 | 396 | 20 | CESC | TCGA-C5-A2M1-01 | 138 | 5 |
| BRCA | TCGA-E9-A1NG-01 | 20 | 4 | BRCA | TCGA-GM-A2DA-01 | 34 | 0 | CESC | TCGA-C5-A2M2-01 | 86 | 9 |
| BRCA | TCGA-E9-A1NH-01 | 27 | 7 | BRCA | TCGA-GM-A2DB-01 | 99 | 5 | CESC | TCGA-C5-A3HD-01 | 165 | 7 |
| BRCA | TCGA-E9-A1NI-01 | 137 | 8 | BRCA | TCGA-GM-A2DC-01 | 20 | 0 | CESC | TCGA-C5-A3HE-01 | 523 | 10 |
| BRCA | TCGA-E9-A1QZ-01 | 10 | 0 | BRCA | TCGA-GM-A2DD-01 | 49 | 5 | CESC | TCGA-C5-A3HF-01 | 49 | 1 |
| BRCA | TCGA-E9-A1R0-01 | 25 | 3 | BRCA | TCGA-GM-A2DF-01 | 34 | 3 | CESC | TCGA-C5-A3HL-01 | 180 | 7 |
| BRCA | TCGA-E9-A1R2-01 | 55 | 2 | BRCA | TCGA-GM-A2DH-01 | 119 | 7 | CESC | TCGA-C5-A7CG-01 | 72 | 6 |
| BRCA | TCGA-E9-A1R3-01 | 25 | 3 | BRCA | TCGA-GM-A2DI-01 | 27 | 4 | CESC | TCGA-C5-A7CH-01 | 83 | 3 |
| BRCA | TCGA-E9-A1R4-01 | 98 | 4 | BRCA | TCGA-GM-A2DK-01 | 24 | 0 | CESC | TCGA-C5-A7CJ-01 | 105 | 4 |
| BRCA | TCGA-E9-A1R5-01 | 34 | 2 | BRCA | TCGA-GM-A2DL-01 | 43 | 2 | CESC | TCGA-C5-A7CK-01 | 184 | 5 |
| BRCA | TCGA-E9-A1R6-01 | 26 | 2 | BRCA | TCGA-GM-A2DM-01 | 20 | 3 | CESC | TCGA-C5-A7CL-01 | 197 | 7 |
| BRCA | TCGA-E9-A1R7-01 | 55 | 5 | BRCA | TCGA-GM-A2DN-01 | 21 | 2 | CESC | TCGA-C5-A7CN-01 | 46 | 1 |
| BRCA | TCGA-E9-A1RA-01 | 22 | 1 | BRCA | TCGA-GM-A2DO-01 | 93 | 4 | CESC | TCGA-C5-A7CO-01 | 142 | 11 |
| BRCA | TCGA-E9-A1RB-01 | 31 | 2 | BRCA | TCGA-GM-A3NW-01 | 95 | 4 | CESC | TCGA-C5-A7UC-01 | 86 | 3 |
| BRCA | TCGA-E9-A1RC-01 | 48 | 4 | BRCA | TCGA-GM-A3NY-01 | 125 | 4 | CESC | TCGA-C5-A7UE-01 | 173 | 8 |
| BRCA | TCGA-E9-A1RD-01 | 25 | 5 | BRCA | TCGA-GM-A3XG-01 | 8 | 0 | CESC | TCGA-C5-A7UH-01 | 293 | 16 |
| BRCA | TCGA-E9-A1RE-01 | 51 | 3 | BRCA | TCGA-GM-A3XL-01 | 96 | 2 | CESC | TCGA-C5-A7X3-01 | 84 | 6 |
| BRCA | TCGA-E9-A1RF-01 | 130 | 5 | BRCA | TCGA-GM-A3XN-01 | 27 | 3 | CESC | TCGA-DG-A2KH-01 | 79 | 6 |
| BRCA | TCGA-E9-A1RG-01 | 39 | 0 | BRCA | TCGA-GM-A4E0-01 | 29 | 2 | CESC | TCGA-DG-A2KJ-01 | 3 | 0 |
| BRCA | TCGA-E9-A1RH-01 | 81 | 3 | BRCA | TCGA-GM-A5PV-01 | 26 | 4 | CESC | TCGA-DG-A2KK-01 | 386 | 15 |
| BRCA | TCGA-E9-A1RI-01 | 21 | 1 | BRCA | TCGA-GM-A5PX-01 | 33 | 1 | CESC | TCGA-DG-A2KL-01 | 245 | 9 |
| BRCA | TCGA-E9-A226-01 | 33 | 2 | BRCA | TCGA-HN-A2NL-01 | 71 | 0 | CESC | TCGA-DG-A2KM-01 | 166 | 8 |
| BRCA | TCGA-E9-A227-01 | 16 | 3 | BRCA | TCGA-HN-A2OB-01 | 13 | 2 | CESC | TCGA-DR-A0ZL-01 | 64 | 0 |
| BRCA | TCGA-E9-A228-01 | 44 | 3 | BRCA | TCGA-JL-A3YW-01 | 99 | 3 | CESC | TCGA-DR-A0ZM-01 | 1493 | 47 |
| BRCA | TCGA-E9-A229-01 | 22 | 2 | BRCA | TCGA-JL-A3YX-01 | 41 | 5 | CESC | TCGA-DS-A0VK-01 | 147 | 11 |
| BRCA | TCGA-E9-A22A-01 | 33 | 0 | BRCA | TCGA-LL-A440-01 | 12 | 2 | CESC | TCGA-DS-A0VL-01 | 244 | 12 |
| BRCA | TCGA-E9-A22B-01 | 55 | 1 | BRCA | TCGA-LL-A441-01 | 79 | 4 | CESC | TCGA-DS-A0VM-01 | 354 | 14 |
| BRCA | TCGA-E9-A22D-01 | 19 | 2 | BRCA | TCGA-LL-A50Y-01 | 43 | 4 | CESC | TCGA-DS-A0VN-01 | 167 | 7 |
| BRCA | TCGA-E9-A22E-01 | 87 | 3 | BRCA | TCGA-LL-A5YL-01 | 79 | 5 | CESC | TCGA-DS-A1OA-01 | 133 | 3 |
| BRCA | TCGA-E9-A22G-01 | 102 | 3 | BRCA | TCGA-LL-A5YM-01 | 30 | 4 | CESC | TCGA-DS-A3LQ-01 | 48 | 1 |
| BRCA | TCGA-E9-A22H-01 | 21 | 3 | BRCA | TCGA-LL-A5YN-01 | 28 | 2 | CESC | TCGA-DS-A5RQ-01 | 149 | 4 |
| BRCA | TCGA-E9-A243-01 | 62 | 4 | BRCA | TCGA-LL-A5YO-01 | 39 | 3 | CESC | TCGA-DS-A7WF-01 | 67 | 4 |
| BRCA | TCGA-E9-A244-01 | 88 | 6 | BRCA | TCGA-LL-A5YP-01 | 96 | 7 | CESC | TCGA-DS-A7WH-01 | 155 | 3 |
| BRCA | TCGA-E9-A245-01 | 19 | 1 | BRCA | TCGA-LQ-A4E4-01 | 97 | 11 | CESC | TCGA-DS-A7WI-01 | 43 | 3 |
| BRCA | TCGA-E9-A247-01 | 34 | 1 | BRCA | TCGA-MS-A51U-01 | 19 | 3 | CESC | TCGA-EA-A1QS-01 | 47 | 2 |
| BRCA | TCGA-E9-A248-01 | 19 | 1 | BRCA | TCGA-OK-A5Q2-01 | 32 | 3 | CESC | TCGA-EA-A1QT-01 | 82 | 12 |
| BRCA | TCGA-E9-A249-01 | 16 | 3 | BRCA | TCGA-OL-A5D6-01 | 43 | 5 | CESC | TCGA-EA-A3HQ-01 | 118 | 6 |
| BRCA | TCGA-E9-A24A-01 | 19 | 1 | BRCA | TCGA-OL-A5D7-01 | 59 | 3 | CESC | TCGA-EA-A3HR-01 | 107 | 5 |
| BRCA | TCGA-E9-A295-01 | 69 | 2 | BRCA | TCGA-OL-A5D8-01 | 35 | 2 | CESC | TCGA-EA-A3HT-01 | 141 | 4 |
| BRCA | TCGA-E9-A2JS-01 | 76 | 1 | BRCA | TCGA-OL-A5DA-01 | 65 | 3 | CESC | TCGA-EA-A3HU-01 | 1383 | 46 |
| BRCA | TCGA-E9-A2JT-01 | 7 | 1 | BRCA | TCGA-OL-A5RU-01 | 30 | 0 | CESC | TCGA-EA-A3QD-01 | 130 | 8 |
| BRCA | TCGA-E9-A3HO-01 | 75 | 2 | BRCA | TCGA-OL-A5RV-01 | 17 | 3 | CESC | TCGA-EA-A3QE-01 | 88 | 10 |
| BRCA | TCGA-E9-A3Q9-01 | 74 | 1 | BRCA | TCGA-OL-A5RW-01 | 174 | 7 | CESC | TCGA-EA-A3Y4-01 | 173 | 7 |
| BRCA | TCGA-E9-A3QA-01 | 71 | 3 | BRCA | TCGA-OL-A5RX-01 | 30 | 6 | CESC | TCGA-EA-A410-01 | 100 | 0 |
| BRCA | TCGA-E9-A3X8-01 | 20 | 2 | BRCA | TCGA-OL-A5RY-01 | 13 | 1 | CESC | TCGA-EA-A411-01 | 73 | 4 |
| BRCA | TCGA-E9-A54X-01 | 39 | 3 | BRCA | TCGA-OL-A5RZ-01 | 116 | 6 | CESC | TCGA-EA-A439-01 | 161 | 6 |
| BRCA | TCGA-E9-A54Y-01 | 52 | 2 | BRCA | TCGA-OL-A5S0-01 | 43 | 1 | CESC | TCGA-EA-A43B-01 | 162 | 3 |
| BRCA | TCGA-E9-A5FK-01 | 19 | 0 | BRCA | TCGA-OL-A66H-01 | 27 | 3 | CESC | TCGA-EA-A44S-01 | 69 | 5 |
| BRCA | TCGA-E9-A5FL-01 | 98 | 7 | BRCA | TCGA-OL-A66I-01 | 40 | 3 | CESC | TCGA-EA-A4BA-01 | 65 | 7 |
| BRCA | TCGA-E9-A5UO-01 | 40 | 2 | BRCA | TCGA-OL-A66J-01 | 63 | 4 | CESC | TCGA-EA-A50E-01 | 137 | 9 |
| BRCA | TCGA-E9-A5UP-01 | 39 | 2 | BRCA | TCGA-OL-A66K-01 | 33 | 4 | CESC | TCGA-EA-A556-01 | 44 | 3 |
| BRCA | TCGA-EW-A1IW-01 | 83 | 4 | BRCA | TCGA-PE-A5DC-01 | 128 | 5 | CESC | TCGA-EA-A5FO-01 | 108 | 8 |
| BRCA | TCGA-EW-A1IX-01 | 22 | 3 | BRCA | TCGA-PE-A5DD-01 | 66 | 2 | CESC | TCGA-EA-A5O9-01 | 81 | 4 |
| BRCA | TCGA-EW-A1IY-01 | 26 | 1 | BRCA | TCGA-PE-A5DE-01 | 433 | 21 | CESC | TCGA-EA-A5ZD-01 | 30 | 4 |
| BRCA | TCGA-EW-A1IZ-01 | 327 | 14 | CESC | TCGA-BI-A0VR-01 | 114 | 6 | CESC | TCGA-EA-A5ZE-01 | 79 | 6 |
| BRCA | TCGA-EW-A1J1-01 | 22 | 2 | CESC | TCGA-BI-A0VS-01 | 136 | 6 | CESC | TCGA-EA-A5ZF-01 | 104 | 2 |
| BRCA | TCGA-EW-A1J2-01 | 30 | 3 | CESC | TCGA-BI-A20A-01 | 73 | 3 | CESC | TCGA-EA-A6QX-01 | 133 | 6 |
| BRCA | TCGA-EW-A1J3-01 | 29 | 2 | CESC | TCGA-C5-A0TN-01 | 19 | 3 | CESC | TCGA-EA-A78R-01 | 106 | 4 |
| BRCA | TCGA-EW-A1J5-01 | 413 | 17 | CESC | TCGA-C5-A1BE-01 | 93 | 2 | CESC | TCGA-EK-A2GZ-01 | 104 | 1 |
| BRCA | TCGA-EW-A1J6-01 | 44 | 2 | CESC | TCGA-C5-A1BF-01 | 312 | 19 | CESC | TCGA-EK-A2H0-01 | 227 | 6 |
| BRCA | TCGA-EW-A1OV-01 | 92 | 5 | CESC | TCGA-C5-A1BI-01 | 136 | 5 | CESC | TCGA-EK-A2H1-01 | 45 | 1 |
| BRCA | TCGA-EW-A1OX-01 | 25 | 4 | CESC | TCGA-C5-A1BJ-01 | 349 | 19 | CESC | TCGA-EK-A2IP-01 | 163 | 6 |
| BRCA | TCGA-EW-A1OY-01 | 64 | 4 | CESC | TCGA-C5-A1BK-01 | 201 | 1 | CESC | TCGA-EK-A2PG-01 | 746 | 22 |
| BRCA | TCGA-EW-A1OZ-01 | 54 | 1 | CESC | TCGA-C5-A1BL-01 | 217 | 14 | CESC | TCGA-EK-A2PI-01 | 108 | 5 |
| BRCA | TCGA-EW-A1P0-01 | 28 | 3 | CESC | TCGA-C5-A1BM-01 | 154 | 9 | CESC | TCGA-EK-A2PK-01 | 6 | 0 |
| BRCA | TCGA-EW-A1P1-01 | 3 | 0 | CESC | TCGA-C5-A1BN-01 | 250 | 4 | CESC | TCGA-EK-A2PL-01 | 241 | 11 |
| BRCA | TCGA-EW-A1P3-01 | 15 | 2 | CESC | TCGA-C5-A1BQ-01 | 1085 | 41 | CESC | TCGA-EK-A2PM-01 | 383 | 14 |
| BRCA | TCGA-EW-A1P4-01 | 60 | 2 | CESC | TCGA-C5-A1M5-01 | 59 | 0 | CESC | TCGA-EK-A2R7-01 | 165 | 6 |
| BRCA | TCGA-EW-A1P5-01 | 20 | 2 | CESC | TCGA-C5-A1M6-01 | 159 | 5 | CESC | TCGA-EK-A2R8-01 | 382 | 10 |

# TABLE 5 (APPENDIX A)

| | | | |
|---|---|---|---|
| CESC | TCGA-EK-A2R9-01 | 145 | 9 |
| CESC | TCGA-EK-A2RA-01 | 296 | 6 |
| CESC | TCGA-EK-A2RB-01 | 151 | 5 |
| CESC | TCGA-EK-A2RC-01 | 234 | 11 |
| CESC | TCGA-EK-A2RD-01 | 233 | 10 |
| CESC | TCGA-EK-A2RE-01 | 66 | 1 |
| CESC | TCGA-EK-A2RJ-01 | 445 | 11 |
| CESC | TCGA-EK-A2RK-01 | 301 | 9 |
| CESC | TCGA-EK-A2RL-01 | 77 | 6 |
| CESC | TCGA-EK-A2RM-01 | 3 | 0 |
| CESC | TCGA-EK-A2RN-01 | 199 | 7 |
| CESC | TCGA-EK-A2RO-01 | 152 | 7 |
| CESC | TCGA-EK-A3GJ-01 | 309 | 10 |
| CESC | TCGA-EK-A3GK-01 | 1690 | 72 |
| CESC | TCGA-EK-A3GM-01 | 230 | 17 |
| CESC | TCGA-EK-A3GN-01 | 108 | 3 |
| CESC | TCGA-EX-A1H5-01 | 199 | 8 |
| CESC | TCGA-EX-A1H6-01 | 114 | 2 |
| CESC | TCGA-EX-A3L1-01 | 41 | 2 |
| CESC | TCGA-EX-A449-01 | 48 | 4 |
| CESC | TCGA-EX-A69L-01 | 91 | 6 |
| CESC | TCGA-EX-A69M-01 | 76 | 2 |
| CESC | TCGA-FU-A23K-01 | 131 | 5 |
| CESC | TCGA-FU-A23L-01 | 172 | 4 |
| CESC | TCGA-FU-A2QG-01 | 125 | 12 |
| CESC | TCGA-FU-A3EO-01 | 79 | 5 |
| CESC | TCGA-FU-A3HY-01 | 240 | 9 |
| CESC | TCGA-FU-A3HZ-01 | 2519 | 67 |
| CESC | TCGA-FU-A3NI-01 | 128 | 4 |
| CESC | TCGA-FU-A3TQ-01 | 87 | 7 |
| CESC | TCGA-FU-A3TX-01 | 81 | 5 |
| CESC | TCGA-FU-A3WB-01 | 87 | 2 |
| CESC | TCGA-FU-A3YQ-01 | 94 | 2 |
| CESC | TCGA-FU-A40J-01 | 243 | 11 |
| CESC | TCGA-FU-A57G-01 | 51 | 7 |
| CESC | TCGA-FU-A5XV-01 | 133 | 5 |
| CESC | TCGA-FU-A770-01 | 85 | 3 |
| CESC | TCGA-HG-A2PA-01 | 97 | 6 |
| CESC | TCGA-HM-A3JJ-01 | 11 | 0 |
| CESC | TCGA-HM-A3JK-01 | 77 | 0 |
| CESC | TCGA-HM-A4S6-01 | 54 | 1 |
| CESC | TCGA-HM-A6W2-01 | 4 | 0 |
| CESC | TCGA-IR-A3L7-01 | 52 | 0 |
| CESC | TCGA-IR-A3LA-01 | 1417 | 54 |
| CESC | TCGA-IR-A3LB-01 | 75 | 1 |
| CESC | TCGA-IR-A3LC-01 | 68 | 0 |
| CESC | TCGA-IR-A3LF-01 | 120 | 9 |
| CESC | TCGA-IR-A3LH-01 | 1139 | 24 |
| CESC | TCGA-IR-A3LI-01 | 474 | 6 |
| CESC | TCGA-IR-A3LK-01 | 1750 | 57 |
| CESC | TCGA-IR-A3LL-01 | 501 | 23 |
| CESC | TCGA-JW-A5VG-01 | 44 | 3 |
| CESC | TCGA-JW-A5VH-01 | 94 | 8 |
| CESC | TCGA-JW-A5VI-01 | 99 | 5 |
| CESC | TCGA-JW-A5VJ-01 | 238 | 8 |
| CESC | TCGA-JW-A5VK-01 | 76 | 4 |
| CESC | TCGA-JW-A5VL-01 | 2725 | 117 |
| CESC | TCGA-JW-A69B-01 | 121 | 5 |
| CESC | TCGA-JW-A852-01 | 132 | 9 |
| CESC | TCGA-JX-A3PZ-01 | 71 | 3 |
| CESC | TCGA-JX-A3Q0-01 | 929 | 44 |
| CESC | TCGA-JX-A3Q8-01 | 79 | 1 |
| CESC | TCGA-JX-A5QV-01 | 66 | 1 |
| CESC | TCGA-LP-A4AU-01 | 49 | 3 |
| CESC | TCGA-LP-A4AV-01 | 526 | 31 |
| CESC | TCGA-LP-A4AW-01 | 128 | 2 |
| CESC | TCGA-LP-A4AX-01 | 71 | 5 |
| CESC | TCGA-LP-A5U2-01 | 284 | 6 |
| CESC | TCGA-LP-A5U3-01 | 68 | 0 |
| CESC | TCGA-LP-A7HU-01 | 218 | 4 |
| CESC | TCGA-MU-A51Y-01 | 223 | 3 |
| CESC | TCGA-MU-A5YI-01 | 161 | 4 |
| CESC | TCGA-MY-A5BD-01 | 359 | 13 |
| CESC | TCGA-MY-A5BE-01 | 64 | 2 |
| CESC | TCGA-MY-A5BF-01 | 7 | 0 |
| CESC | TCGA-Q1-A5R1-01 | 73 | 4 |
| CESC | TCGA-Q1-A5R2-01 | 374 | 12 |
| CESC | TCGA-Q1-A5R3-01 | 79 | 4 |
| CESC | TCGA-Q1-A6DT-01 | 229 | 7 |
| CESC | TCGA-Q1-A6DV-01 | 56 | 2 |
| CESC | TCGA-Q1-A6DW-01 | 171 | 8 |
| CESC | TCGA-Q1-A73O-01 | 1987 | 62 |
| CESC | TCGA-Q1-A73P-01 | 342 | 9 |
| CESC | TCGA-Q1-A73Q-01 | 116 | 3 |
| CESC | TCGA-Q1-A73R-01 | 97 | 5 |
| CESC | TCGA-Q1-A73S-01 | 66 | 3 |
| CESC | TCGA-R2-A69V-01 | 151 | 7 |
| CESC | TCGA-RA-A741-01 | 127 | 0 |
| CESC | TCGA-UC-A7PD-01 | 55 | 4 |
| CESC | TCGA-UC-A7PF-01 | 551 | 16 |
| CESC | TCGA-WL-A834-01 | 146 | 5 |
| CHOL | TCGA-3X-AAV9-01 | 199 | 5 |
| CHOL | TCGA-3X-AAVA-01 | 123 | 3 |
| CHOL | TCGA-3X-AAVB-01 | 122 | 8 |
| CHOL | TCGA-3X-AAVC-01 | 191 | 6 |
| CHOL | TCGA-3X-AAVE-01 | 149 | 6 |
| CHOL | TCGA-4G-AAZO-01 | 352 | 15 |
| CHOL | TCGA-4G-AAZT-01 | 164 | 6 |
| CHOL | TCGA-W5-AA2G-01 | 184 | 14 |
| CHOL | TCGA-W5-AA2H-01 | 103 | 7 |
| CHOL | TCGA-W5-AA2I-01 | 162 | 5 |
| CHOL | TCGA-W5-AA2O-01 | 188 | 12 |
| CHOL | TCGA-W5-AA2Q-01 | 183 | 10 |
| CHOL | TCGA-W5-AA2R-01 | 143 | 7 |
| CHOL | TCGA-W5-AA2T-01 | 138 | 4 |
| CHOL | TCGA-W5-AA2U-01 | 232 | 15 |
| CHOL | TCGA-W5-AA2W-01 | 114 | 3 |
| CHOL | TCGA-W5-AA2X-01 | 212 | 11 |
| CHOL | TCGA-W5-AA2Z-01 | 117 | 4 |
| CHOL | TCGA-W5-AA30-01 | 190 | 11 |
| CHOL | TCGA-W5-AA31-01 | 151 | 7 |
| CHOL | TCGA-W5-AA33-01 | 99 | 2 |
| CHOL | TCGA-W5-AA34-01 | 171 | 7 |
| CHOL | TCGA-W5-AA38-01 | 161 | 3 |
| CHOL | TCGA-W5-AA39-01 | 1122 | 40 |
| CHOL | TCGA-W6-AA0S-01 | 159 | 10 |
| CHOL | TCGA-WD-A7RX-01 | 164 | 12 |
| CHOL | TCGA-YR-A95A-01 | 148 | 9 |
| CHOL | TCGA-ZD-A8I3-01 | 156 | 9 |
| CHOL | TCGA-ZH-A8Y1-01 | 121 | 5 |
| CHOL | TCGA-ZH-A8Y2-01 | 176 | 8 |
| CHOL | TCGA-ZH-A8Y4-01 | 194 | 9 |
| CHOL | TCGA-ZH-A8Y5-01 | 124 | 5 |
| CHOL | TCGA-ZH-A8Y6-01 | 222 | 9 |
| CHOL | TCGA-ZH-A8Y8-01 | 166 | 8 |
| CHOL | TCGA-ZU-A854-01 | 155 | 9 |
| COAD | TCGA-A6-2672-01 | 600 | 25 |
| COAD | TCGA-A6-2674-01 | 31 | 3 |
| COAD | TCGA-A6-2676-01 | 839 | 41 |
| COAD | TCGA-A6-2677-01 | 78 | 6 |
| COAD | TCGA-A6-2678-01 | 69 | 8 |
| COAD | TCGA-A6-2683-01 | 107 | 6 |
| COAD | TCGA-A6-3807-01 | 100 | 7 |
| COAD | TCGA-A6-3808-01 | 124 | 9 |
| COAD | TCGA-A6-3810-01 | 125 | 5 |
| COAD | TCGA-AA-3514-01 | 99 | 5 |
| COAD | TCGA-AA-3516-01 | 932 | 36 |
| COAD | TCGA-AA-3517-01 | 38 | 2 |
| COAD | TCGA-AA-3518-01 | 591 | 29 |
| COAD | TCGA-AA-3519-01 | 54 | 5 |
| COAD | TCGA-AA-3520-01 | 68 | 7 |
| COAD | TCGA-AA-3521-01 | 55 | 5 |
| COAD | TCGA-AA-3522-01 | 60 | 6 |
| COAD | TCGA-AA-3524-01 | 52 | 4 |
| COAD | TCGA-AA-3525-01 | 629 | 29 |
| COAD | TCGA-AA-3526-01 | 68 | 3 |
| COAD | TCGA-AA-3527-01 | 107 | 12 |
| COAD | TCGA-AA-3529-01 | 63 | 1 |
| COAD | TCGA-AA-3530-01 | 94 | 8 |
| COAD | TCGA-AA-3531-01 | 74 | 7 |
| COAD | TCGA-AA-3532-01 | 47 | 3 |
| COAD | TCGA-AA-3534-01 | 65 | 5 |
| COAD | TCGA-AA-3538-01 | 46 | 1 |
| COAD | TCGA-AA-3542-01 | 413 | 16 |
| COAD | TCGA-AA-3543-01 | 240 | 18 |
| COAD | TCGA-AA-3544-01 | 66 | 7 |
| COAD | TCGA-AA-3548-01 | 40 | 3 |
| COAD | TCGA-AA-3549-01 | 78 | 4 |
| COAD | TCGA-AA-3552-01 | 61 | 5 |
| COAD | TCGA-AA-3553-01 | 36 | 2 |
| COAD | TCGA-AA-3554-01 | 335 | 17 |
| COAD | TCGA-AA-3555-01 | 908 | 47 |
| COAD | TCGA-AA-3556-01 | 44 | 5 |
| COAD | TCGA-AA-3558-01 | 139 | 9 |
| COAD | TCGA-AA-3560-01 | 62 | 4 |
| COAD | TCGA-AA-3561-01 | 61 | 5 |
| COAD | TCGA-AA-3562-01 | 50 | 6 |
| COAD | TCGA-AA-3664-01 | 170 | 12 |
| COAD | TCGA-AA-3666-01 | 159 | 11 |
| COAD | TCGA-AA-3667-01 | 75 | 4 |
| COAD | TCGA-AA-3672-01 | 1844 | 63 |
| COAD | TCGA-AA-3673-01 | 86 | 4 |
| COAD | TCGA-AA-3678-01 | 80 | 5 |
| COAD | TCGA-AA-3679-01 | 82 | 8 |
| COAD | TCGA-AA-3680-01 | 122 | 7 |
| COAD | TCGA-AA-3681-01 | 127 | 11 |
| COAD | TCGA-AA-3684-01 | 129 | 7 |
| COAD | TCGA-AA-3685-01 | 82 | 6 |
| COAD | TCGA-AA-3688-01 | 66 | 6 |
| COAD | TCGA-AA-3692-01 | 68 | 5 |
| COAD | TCGA-AA-3693-01 | 147 | 12 |
| COAD | TCGA-AA-3695-01 | 182 | 11 |
| COAD | TCGA-AA-3696-01 | 102 | 7 |
| COAD | TCGA-AA-3710-01 | 1357 | 53 |
| COAD | TCGA-AA-3715-01 | 2011 | 79 |
| COAD | TCGA-AA-3811-01 | 1170 | 48 |
| COAD | TCGA-AA-3812-01 | 116 | 8 |
| COAD | TCGA-AA-3814-01 | 113 | 8 |
| COAD | TCGA-AA-3818-01 | 141 | 12 |
| COAD | TCGA-AA-3819-01 | 179 | 13 |
| COAD | TCGA-AA-3821-01 | 840 | 34 |
| COAD | TCGA-AA-3831-01 | 117 | 7 |
| COAD | TCGA-AA-3833-01 | 549 | 22 |
| COAD | TCGA-AA-3837-01 | 155 | 8 |
| COAD | TCGA-AA-3842-01 | 91 | 6 |
| COAD | TCGA-AA-3844-01 | 122 | 5 |
| COAD | TCGA-AA-3845-01 | 904 | 37 |
| COAD | TCGA-AA-3846-01 | 103 | 4 |
| COAD | TCGA-AA-3848-01 | 156 | 12 |
| COAD | TCGA-AA-3850-01 | 116 | 7 |
| COAD | TCGA-AA-3851-01 | 102 | 9 |
| COAD | TCGA-AA-3852-01 | 146 | 8 |
| COAD | TCGA-AA-3854-01 | 150 | 9 |
| COAD | TCGA-AA-3855-01 | 149 | 7 |
| COAD | TCGA-AA-3856-01 | 68 | 3 |
| COAD | TCGA-AA-3858-01 | 69 | 4 |
| COAD | TCGA-AA-3860-01 | 51 | 7 |
| COAD | TCGA-AA-3864-01 | 2776 | 104 |
| COAD | TCGA-AA-3866-01 | 138 | 7 |
| COAD | TCGA-AA-3867-01 | 104 | 8 |
| COAD | TCGA-AA-3869-01 | 97 | 7 |
| COAD | TCGA-AA-3870-01 | 131 | 7 |
| COAD | TCGA-AA-3872-01 | 72 | 3 |
| COAD | TCGA-AA-3875-01 | 129 | 6 |
| COAD | TCGA-AA-3877-01 | 1118 | 32 |
| COAD | TCGA-AA-3930-01 | 176 | 10 |
| COAD | TCGA-AA-3939-01 | 138 | 8 |
| COAD | TCGA-AA-3941-01 | 121 | 8 |
| COAD | TCGA-AA-3947-01 | 1760 | 67 |
| COAD | TCGA-AA-3949-01 | 950 | 42 |
| COAD | TCGA-AA-3952-01 | 101 | 3 |
| COAD | TCGA-AA-3955-01 | 129 | 3 |
| COAD | TCGA-AA-3956-01 | 146 | 10 |
| COAD | TCGA-AA-3966-01 | 1197 | 41 |
| COAD | TCGA-AA-3971-01 | 94 | 3 |
| COAD | TCGA-AA-3972-01 | 120 | 6 |
| COAD | TCGA-AA-3973-01 | 87 | 10 |
| COAD | TCGA-AA-3975-01 | 132 | 8 |
| COAD | TCGA-AA-3976-01 | 101 | 9 |
| COAD | TCGA-AA-3977-01 | 3695 | 139 |
| COAD | TCGA-AA-3979-01 | 165 | 11 |
| COAD | TCGA-AA-3980-01 | 108 | 9 |
| COAD | TCGA-AA-3982-01 | 114 | 11 |
| COAD | TCGA-AA-3984-01 | 4113 | 149 |
| COAD | TCGA-AA-3986-01 | 90 | 6 |
| COAD | TCGA-AA-3989-01 | 136 | 6 |
| COAD | TCGA-AA-3994-01 | 201 | 14 |
| COAD | TCGA-AA-A004-01 | 73 | 2 |
| COAD | TCGA-AA-A00A-01 | 457 | 26 |
| COAD | TCGA-AA-A00D-01 | 79 | 7 |
| COAD | TCGA-AA-A00E-01 | 657 | 28 |
| COAD | TCGA-AA-A00F-01 | 46 | 3 |
| COAD | TCGA-AA-A00J-01 | 1174 | 57 |
| COAD | TCGA-AA-A00K-01 | 195 | 10 |
| COAD | TCGA-AA-A00L-01 | 58 | 4 |
| COAD | TCGA-AA-A00N-01 | 4709 | 179 |
| COAD | TCGA-AA-A00O-01 | 94 | 4 |
| COAD | TCGA-AA-A00Q-01 | 38 | 5 |
| COAD | TCGA-AA-A00R-01 | 436 | 20 |
| COAD | TCGA-AA-A00U-01 | 92 | 6 |
| COAD | TCGA-AA-A00W-01 | 30 | 4 |
| COAD | TCGA-AA-A00Z-01 | 45 | 3 |
| COAD | TCGA-AA-A010-01 | 8704 | 300 |
| COAD | TCGA-AA-A017-01 | 68 | 4 |
| COAD | TCGA-AA-A01D-01 | 196 | 8 |
| COAD | TCGA-AA-A01F-01 | 61 | 6 |
| COAD | TCGA-AA-A01G-01 | 75 | 4 |
| COAD | TCGA-AA-A01I-01 | 104 | 8 |
| COAD | TCGA-AA-A01K-01 | 188 | 10 |
| COAD | TCGA-AA-A01P-01 | 519 | 20 |
| COAD | TCGA-AA-A01Q-01 | 800 | 31 |
| COAD | TCGA-AA-A01R-01 | 1744 | 76 |
| COAD | TCGA-AA-A01S-01 | 65 | 3 |
| COAD | TCGA-AA-A01T-01 | 99 | 3 |

## TABLE 5 (APPENDIX A)

| Type | Sample | Value 1 | Value 2 |
|---|---|---|---|
| COAD | TCGA-AA-A01V-01 | 139 | 8 |
| COAD | TCGA-AA-A01X-01 | 67 | 3 |
| COAD | TCGA-AA-A01Z-01 | 109 | 9 |
| COAD | TCGA-AA-A022-01 | 1291 | 42 |
| COAD | TCGA-AA-A024-01 | 89 | 7 |
| COAD | TCGA-AA-A029-01 | 120 | 10 |
| COAD | TCGA-AA-A02F-01 | 67 | 6 |
| COAD | TCGA-AA-A02H-01 | 82 | 4 |
| COAD | TCGA-AA-A02J-01 | 152 | 11 |
| COAD | TCGA-AA-A02O-01 | 167 | 6 |
| COAD | TCGA-AA-A02W-01 | 141 | 6 |
| COAD | TCGA-AA-A02Y-01 | 126 | 5 |
| COAD | TCGA-AA-A03F-01 | 135 | 13 |
| COAD | TCGA-AA-A03J-01 | 96 | 4 |
| COAD | TCGA-AY-4070-01 | 145 | 4 |
| COAD | TCGA-AY-4071-01 | 115 | 7 |
| COADREAD | TCGA-A6-2672-01 | 600 | 25 |
| COADREAD | TCGA-A6-2674-01 | 31 | 3 |
| COADREAD | TCGA-A6-2676-01 | 839 | 41 |
| COADREAD | TCGA-A6-2677-01 | 78 | 6 |
| COADREAD | TCGA-A6-2678-01 | 69 | 8 |
| COADREAD | TCGA-A6-2683-01 | 107 | 6 |
| COADREAD | TCGA-A6-3807-01 | 100 | 7 |
| COADREAD | TCGA-A6-3808-01 | 124 | 9 |
| COADREAD | TCGA-A6-3810-01 | 125 | 5 |
| COADREAD | TCGA-AA-3514-01 | 99 | 5 |
| COADREAD | TCGA-AA-3516-01 | 932 | 36 |
| COADREAD | TCGA-AA-3517-01 | 38 | 2 |
| COADREAD | TCGA-AA-3518-01 | 591 | 29 |
| COADREAD | TCGA-AA-3519-01 | 54 | 5 |
| COADREAD | TCGA-AA-3520-01 | 68 | 7 |
| COADREAD | TCGA-AA-3521-01 | 55 | 5 |
| COADREAD | TCGA-AA-3522-01 | 60 | 6 |
| COADREAD | TCGA-AA-3524-01 | 52 | 4 |
| COADREAD | TCGA-AA-3525-01 | 629 | 29 |
| COADREAD | TCGA-AA-3526-01 | 68 | 3 |
| COADREAD | TCGA-AA-3527-01 | 107 | 12 |
| COADREAD | TCGA-AA-3529-01 | 63 | 1 |
| COADREAD | TCGA-AA-3530-01 | 94 | 8 |
| COADREAD | TCGA-AA-3531-01 | 74 | 7 |
| COADREAD | TCGA-AA-3532-01 | 47 | 3 |
| COADREAD | TCGA-AA-3534-01 | 65 | 5 |
| COADREAD | TCGA-AA-3538-01 | 46 | 1 |
| COADREAD | TCGA-AA-3542-01 | 413 | 16 |
| COADREAD | TCGA-AA-3543-01 | 240 | 18 |
| COADREAD | TCGA-AA-3544-01 | 66 | 7 |
| COADREAD | TCGA-AA-3548-01 | 40 | 3 |
| COADREAD | TCGA-AA-3549-01 | 78 | 4 |
| COADREAD | TCGA-AA-3552-01 | 61 | 5 |
| COADREAD | TCGA-AA-3553-01 | 36 | 2 |
| COADREAD | TCGA-AA-3554-01 | 335 | 17 |
| COADREAD | TCGA-AA-3555-01 | 908 | 47 |
| COADREAD | TCGA-AA-3556-01 | 44 | 5 |
| COADREAD | TCGA-AA-3558-01 | 139 | 9 |
| COADREAD | TCGA-AA-3560-01 | 62 | 4 |
| COADREAD | TCGA-AA-3561-01 | 61 | 5 |
| COADREAD | TCGA-AA-3562-01 | 50 | 6 |
| COADREAD | TCGA-AA-3664-01 | 170 | 12 |
| COADREAD | TCGA-AA-3666-01 | 159 | 11 |
| COADREAD | TCGA-AA-3667-01 | 75 | 4 |
| COADREAD | TCGA-AA-3672-01 | 1844 | 63 |
| COADREAD | TCGA-AA-3673-01 | 86 | 4 |
| COADREAD | TCGA-AA-3678-01 | 80 | 5 |
| COADREAD | TCGA-AA-3679-01 | 82 | 8 |
| COADREAD | TCGA-AA-3680-01 | 122 | 7 |
| COADREAD | TCGA-AA-3681-01 | 127 | 11 |
| COADREAD | TCGA-AA-3684-01 | 129 | 7 |
| COADREAD | TCGA-AA-3685-01 | 82 | 6 |
| COADREAD | TCGA-AA-3688-01 | 66 | 6 |
| COADREAD | TCGA-AA-3692-01 | 68 | 5 |
| COADREAD | TCGA-AA-3693-01 | 147 | 12 |
| COADREAD | TCGA-AA-3695-01 | 182 | 11 |
| COADREAD | TCGA-AA-3696-01 | 102 | 7 |
| COADREAD | TCGA-AA-3710-01 | 1357 | 53 |
| COADREAD | TCGA-AA-3715-01 | 2011 | 79 |
| COADREAD | TCGA-AA-3811-01 | 1170 | 48 |
| COADREAD | TCGA-AA-3812-01 | 116 | 8 |
| COADREAD | TCGA-AA-3814-01 | 113 | 8 |
| COADREAD | TCGA-AA-3818-01 | 141 | 12 |
| COADREAD | TCGA-AA-3819-01 | 179 | 13 |
| COADREAD | TCGA-AA-3821-01 | 840 | 34 |
| COADREAD | TCGA-AA-3831-01 | 117 | 7 |
| COADREAD | TCGA-AA-3833-01 | 549 | 22 |
| COADREAD | TCGA-AA-3837-01 | 155 | 8 |
| COADREAD | TCGA-AA-3842-01 | 91 | 6 |
| COADREAD | TCGA-AA-3844-01 | 122 | 5 |
| COADREAD | TCGA-AA-3845-01 | 904 | 37 |
| COADREAD | TCGA-AA-3846-01 | 103 | 4 |
| COADREAD | TCGA-AA-3848-01 | 156 | 12 |
| COADREAD | TCGA-AA-3850-01 | 116 | 7 |
| COADREAD | TCGA-AA-3851-01 | 102 | 9 |
| COADREAD | TCGA-AA-3852-01 | 146 | 8 |
| COADREAD | TCGA-AA-3854-01 | 150 | 9 |
| COADREAD | TCGA-AA-3855-01 | 149 | 7 |
| COADREAD | TCGA-AA-3856-01 | 68 | 3 |
| COADREAD | TCGA-AA-3858-01 | 69 | 4 |
| COADREAD | TCGA-AA-3860-01 | 51 | 7 |
| COADREAD | TCGA-AA-3864-01 | 2776 | 104 |
| COADREAD | TCGA-AA-3866-01 | 138 | 7 |
| COADREAD | TCGA-AA-3867-01 | 104 | 8 |
| COADREAD | TCGA-AA-3869-01 | 97 | 7 |
| COADREAD | TCGA-AA-3870-01 | 131 | 7 |
| COADREAD | TCGA-AA-3872-01 | 72 | 3 |
| COADREAD | TCGA-AA-3875-01 | 129 | 6 |
| COADREAD | TCGA-AA-3877-01 | 1118 | 32 |
| COADREAD | TCGA-AA-3930-01 | 176 | 10 |
| COADREAD | TCGA-AA-3939-01 | 138 | 8 |
| COADREAD | TCGA-AA-3941-01 | 121 | 8 |
| COADREAD | TCGA-AA-3947-01 | 1760 | 67 |
| COADREAD | TCGA-AA-3949-01 | 950 | 42 |
| COADREAD | TCGA-AA-3952-01 | 101 | 3 |
| COADREAD | TCGA-AA-3955-01 | 129 | 3 |
| COADREAD | TCGA-AA-3956-01 | 146 | 10 |
| COADREAD | TCGA-AA-3966-01 | 1197 | 41 |
| COADREAD | TCGA-AA-3971-01 | 94 | 3 |
| COADREAD | TCGA-AA-3972-01 | 120 | 6 |
| COADREAD | TCGA-AA-3973-01 | 87 | 10 |
| COADREAD | TCGA-AA-3975-01 | 132 | 8 |
| COADREAD | TCGA-AA-3976-01 | 101 | 9 |
| COADREAD | TCGA-AA-3977-01 | 3695 | 139 |
| COADREAD | TCGA-AA-3979-01 | 165 | 11 |
| COADREAD | TCGA-AA-3980-01 | 108 | 9 |
| COADREAD | TCGA-AA-3982-01 | 114 | 11 |
| COADREAD | TCGA-AA-3984-01 | 4113 | 149 |
| COADREAD | TCGA-AA-3986-01 | 90 | 6 |
| COADREAD | TCGA-AA-3989-01 | 136 | 6 |
| COADREAD | TCGA-AA-3994-01 | 201 | 14 |
| COADREAD | TCGA-AA-A004-01 | 73 | 2 |
| COADREAD | TCGA-AA-A00A-01 | 457 | 26 |
| COADREAD | TCGA-AA-A00D-01 | 79 | 7 |
| COADREAD | TCGA-AA-A00E-01 | 657 | 28 |
| COADREAD | TCGA-AA-A00G-01 | 46 | 3 |
| COADREAD | TCGA-AA-A00J-01 | 1174 | 57 |
| COADREAD | TCGA-AA-A00K-01 | 195 | 10 |
| COADREAD | TCGA-AA-A00L-01 | 58 | 4 |
| COADREAD | TCGA-AA-A00N-01 | 4709 | 179 |
| COADREAD | TCGA-AA-A00O-01 | 94 | 4 |
| COADREAD | TCGA-AA-A00Q-01 | 38 | 5 |
| COADREAD | TCGA-AA-A00R-01 | 436 | 20 |
| COADREAD | TCGA-AA-A00U-01 | 92 | 6 |
| COADREAD | TCGA-AA-A00W-01 | 30 | 4 |
| COADREAD | TCGA-AA-A00Z-01 | 45 | 3 |
| COADREAD | TCGA-AA-A010-01 | 8704 | 300 |
| COADREAD | TCGA-AA-A017-01 | 68 | 4 |
| COADREAD | TCGA-AA-A01D-01 | 196 | 8 |
| COADREAD | TCGA-AA-A01F-01 | 61 | 6 |
| COADREAD | TCGA-AA-A01G-01 | 75 | 4 |
| COADREAD | TCGA-AA-A01I-01 | 104 | 8 |
| COADREAD | TCGA-AA-A01K-01 | 188 | 10 |
| COADREAD | TCGA-AA-A01P-01 | 519 | 20 |
| COADREAD | TCGA-AA-A01Q-01 | 800 | 31 |
| COADREAD | TCGA-AA-A01R-01 | 1744 | 76 |
| COADREAD | TCGA-AA-A01S-01 | 65 | 3 |
| COADREAD | TCGA-AA-A01T-01 | 99 | 3 |
| COADREAD | TCGA-AA-A01V-01 | 139 | 8 |
| COADREAD | TCGA-AA-A01X-01 | 67 | 3 |
| COADREAD | TCGA-AA-A01Z-01 | 109 | 9 |
| COADREAD | TCGA-AA-A022-01 | 1291 | 42 |
| COADREAD | TCGA-AA-A024-01 | 89 | 7 |
| COADREAD | TCGA-AA-A029-01 | 120 | 10 |
| COADREAD | TCGA-AA-A02F-01 | 67 | 6 |
| COADREAD | TCGA-AA-A02H-01 | 82 | 4 |
| COADREAD | TCGA-AA-A02J-01 | 152 | 11 |
| COADREAD | TCGA-AA-A02O-01 | 167 | 6 |
| COADREAD | TCGA-AA-A02W-01 | 141 | 6 |
| COADREAD | TCGA-AA-A02Y-01 | 126 | 5 |
| COADREAD | TCGA-AA-A03F-01 | 135 | 13 |
| COADREAD | TCGA-AA-A03J-01 | 96 | 4 |
| COADREAD | TCGA-AF-2689-01 | 64 | 3 |
| COADREAD | TCGA-AF-2691-01 | 73 | 9 |
| COADREAD | TCGA-AF-2692-01 | 45 | 3 |
| COADREAD | TCGA-AF-3400-01 | 33 | 2 |
| COADREAD | TCGA-AF-3913-01 | 118 | 6 |
| COADREAD | TCGA-AG-3574-01 | 44 | 2 |
| COADREAD | TCGA-AG-3575-01 | 51 | 5 |
| COADREAD | TCGA-AG-3578-01 | 29 | 3 |
| COADREAD | TCGA-AG-3580-01 | 44 | 2 |
| COADREAD | TCGA-AG-3581-01 | 54 | 4 |
| COADREAD | TCGA-AG-3582-01 | 35 | 2 |
| COADREAD | TCGA-AG-3583-01 | 67 | 5 |
| COADREAD | TCGA-AG-3584-01 | 35 | 1 |
| COADREAD | TCGA-AG-3586-01 | 83 | 5 |
| COADREAD | TCGA-AG-3587-01 | 69 | 3 |
| COADREAD | TCGA-AG-3593-01 | 78 | 3 |
| COADREAD | TCGA-AG-3594-01 | 46 | 6 |
| COADREAD | TCGA-AG-3598-01 | 75 | 7 |
| COADREAD | TCGA-AG-3599-01 | 67 | 7 |
| COADREAD | TCGA-AG-3600-01 | 97 | 6 |
| COADREAD | TCGA-AG-3601-01 | 107 | 13 |
| COADREAD | TCGA-AG-3602-01 | 49 | 5 |
| COADREAD | TCGA-AG-3605-01 | 68 | 5 |
| COADREAD | TCGA-AG-3608-01 | 61 | 6 |
| COADREAD | TCGA-AG-3609-01 | 97 | 8 |
| COADREAD | TCGA-AG-3611-01 | 54 | 5 |
| COADREAD | TCGA-AG-3612-01 | 73 | 4 |
| COADREAD | TCGA-AG-3726-01 | 158 | 10 |
| COADREAD | TCGA-AG-3727-01 | 85 | 6 |
| COADREAD | TCGA-AG-3878-01 | 91 | 9 |
| COADREAD | TCGA-AG-3881-01 | 102 | 8 |
| COADREAD | TCGA-AG-3882-01 | 68 | 7 |
| COADREAD | TCGA-AG-3883-01 | 133 | 4 |
| COADREAD | TCGA-AG-3887-01 | 79 | 6 |
| COADREAD | TCGA-AG-3890-01 | 82 | 7 |
| COADREAD | TCGA-AG-3892-01 | 2267 | 97 |
| COADREAD | TCGA-AG-3893-01 | 118 | 5 |
| COADREAD | TCGA-AG-3894-01 | 106 | 2 |
| COADREAD | TCGA-AG-3896-01 | 107 | 8 |
| COADREAD | TCGA-AG-3898-01 | 111 | 6 |
| COADREAD | TCGA-AG-3901-01 | 68 | 6 |
| COADREAD | TCGA-AG-3902-01 | 136 | 7 |
| COADREAD | TCGA-AG-3909-01 | 92 | 8 |
| COADREAD | TCGA-AG-3999-01 | 110 | 4 |
| COADREAD | TCGA-AG-4001-01 | 127 | 12 |
| COADREAD | TCGA-AG-4005-01 | 127 | 8 |
| COADREAD | TCGA-AG-4007-01 | 171 | 9 |
| COADREAD | TCGA-AG-4008-01 | 81 | 4 |
| COADREAD | TCGA-AG-4015-01 | 104 | 7 |
| COADREAD | TCGA-AG-A002-01 | 12543 | 423 |
| COADREAD | TCGA-AG-A008-01 | 49 | 5 |
| COADREAD | TCGA-AG-A00C-01 | 69 | 6 |
| COADREAD | TCGA-AG-A00H-01 | 79 | 5 |
| COADREAD | TCGA-AG-A00Y-01 | 284 | 16 |
| COADREAD | TCGA-AG-A011-01 | 115 | 9 |
| COADREAD | TCGA-AG-A014-01 | 159 | 8 |
| COADREAD | TCGA-AG-A015-01 | 64 | 6 |
| COADREAD | TCGA-AG-A016-01 | 58 | 4 |
| COADREAD | TCGA-AG-A01L-01 | 77 | 9 |
| COADREAD | TCGA-AG-A01W-01 | 127 | 6 |
| COADREAD | TCGA-AG-A01Y-01 | 95 | 6 |
| COADREAD | TCGA-AG-A02O-01 | 81 | 9 |
| COADREAD | TCGA-AG-A025-01 | 100 | 7 |
| COADREAD | TCGA-AG-A026-01 | 225 | 8 |
| COADREAD | TCGA-AG-A02G-01 | 61 | 3 |
| COADREAD | TCGA-AG-A02N-01 | 1251 | 52 |
| COADREAD | TCGA-AG-A02X-01 | 154 | 10 |
| COADREAD | TCGA-AG-A032-01 | 91 | 6 |
| COADREAD | TCGA-AG-A036-01 | 154 | 13 |
| COADREAD | TCGA-AY-4070-01 | 145 | 4 |
| COADREAD | TCGA-AY-4071-01 | 115 | 7 |
| DLBC | TCGA-FA-8693-01 | 193 | 11 |
| DLBC | TCGA-FA-A4B8-01 | 90 | 8 |
| DLBC | TCGA-FA-A4XK-01 | 84 | 4 |
| DLBC | TCGA-FA-A6HN-01 | 207 | 11 |
| DLBC | TCGA-FA-A6HO-01 | 49 | 4 |
| DLBC | TCGA-FA-A7DS-01 | 99 | 7 |
| DLBC | TCGA-FA-A7Q1-01 | 169 | 11 |
| DLBC | TCGA-FA-A82F-01 | 169 | 11 |
| DLBC | TCGA-FA-A86F-01 | 85 | 8 |
| DLBC | TCGA-FF-8041-01 | 226 | 11 |
| DLBC | TCGA-FF-8042-01 | 290 | 12 |
| DLBC | TCGA-FF-8043-01 | 124 | 9 |
| DLBC | TCGA-FF-8046-01 | 96 | 12 |
| DLBC | TCGA-FF-8047-01 | 154 | 6 |
| DLBC | TCGA-FF-8061-01 | 179 | 9 |
| DLBC | TCGA-FF-8062-01 | 163 | 16 |
| DLBC | TCGA-FF-A7CQ-01 | 141 | 9 |
| DLBC | TCGA-FF-A7CR-01 | 198 | 14 |
| DLBC | TCGA-FF-A7CW-01 | 50 | 3 |
| DLBC | TCGA-FF-A7CX-01 | 132 | 11 |
| DLBC | TCGA-FM-8000-01 | 33 | 13 |
| DLBC | TCGA-G8-6324-01 | 4792 | 152 |
| DLBC | TCGA-G8-6325-01 | 819 | 48 |
| DLBC | TCGA-G8-6326-01 | 718 | 40 |
| DLBC | TCGA-G8-6906-01 | 919 | 44 |

# TABLE 5 (APPENDIX A)

| | | | | | | | | | | | |
|---|---|---|---|---|---|---|---|---|---|---|---|
| DLBC | TCGA-G8-6907-01 | 728 | 43 | ESCA | TCGA-L5-A4OS-01 | 159 | 3 | ESCA | TCGA-S8-A6BW-01 | 243 | 9 |
| DLBC | TCGA-G8-6909-01 | 1101 | 51 | ESCA | TCGA-L5-A4OT-01 | 358 | 13 | ESCA | TCGA-V5-A7RB-01 | 338 | 10 |
| DLBC | TCGA-G8-6914-01 | 770 | 47 | ESCA | TCGA-L5-A4OU-01 | 276 | 12 | ESCA | TCGA-V5-A7RC-01 | 256 | 14 |
| DLBC | TCGA-GR-7351-01 | 969 | 47 | ESCA | TCGA-L5-A4OW-01 | 362 | 15 | ESCA | TCGA-V5-A7RE-01 | 350 | 11 |
| DLBC | TCGA-GR-7353-01 | 764 | 37 | ESCA | TCGA-L5-A4OX-01 | 212 | 5 | ESCA | TCGA-V5-AASV-01 | 269 | 19 |
| DLBC | TCGA-GR-A4D4-01 | 100 | 9 | ESCA | TCGA-L5-A88S-01 | 218 | 11 | ESCA | TCGA-V5-AASW-01 | 267 | 4 |
| DLBC | TCGA-GR-A4D5-01 | 72 | 4 | ESCA | TCGA-L5-A88T-01 | 155 | 7 | ESCA | TCGA-V5-AASX-01 | 747 | 30 |
| DLBC | TCGA-GR-A4D6-01 | 48 | 3 | ESCA | TCGA-L5-A88V-01 | 257 | 12 | ESCA | TCGA-VR-A8EO-01 | 186 | 8 |
| DLBC | TCGA-GR-A4D9-01 | 74 | 5 | ESCA | TCGA-L5-A88W-01 | 234 | 15 | ESCA | TCGA-VR-A8EP-01 | 176 | 9 |
| DLBC | TCGA-GS-A9TQ-01 | 93 | 7 | ESCA | TCGA-L5-A88Y-01 | 5 | 1 | ESCA | TCGA-VR-A8EQ-01 | 392 | 14 |
| DLBC | TCGA-GS-A9TT-01 | 193 | 9 | ESCA | TCGA-L5-A88Z-01 | 253 | 19 | ESCA | TCGA-VR-A8ER-01 | 198 | 6 |
| DLBC | TCGA-GS-A9TU-01 | 62 | 1 | ESCA | TCGA-L5-A891-01 | 361 | 23 | ESCA | TCGA-VR-A8ET-01 | 73 | 2 |
| DLBC | TCGA-GS-A9TV-01 | 75 | 7 | ESCA | TCGA-L5-A893-01 | 329 | 13 | ESCA | TCGA-VR-A8EU-01 | 274 | 12 |
| DLBC | TCGA-GS-A9TW-01 | 249 | 13 | ESCA | TCGA-L5-A8NE-01 | 425 | 10 | ESCA | TCGA-VR-A8EW-01 | 217 | 13 |
| DLBC | TCGA-GS-A9TX-01 | 28 | 3 | ESCA | TCGA-L5-A8NF-01 | 317 | 16 | ESCA | TCGA-VR-A8EX-01 | 340 | 13 |
| DLBC | TCGA-GS-A9TY-01 | 148 | 6 | ESCA | TCGA-L5-A8NG-01 | 366 | 13 | ESCA | TCGA-VR-A8EY-01 | 251 | 11 |
| DLBC | TCGA-GS-A9TZ-01 | 406 | 34 | ESCA | TCGA-L5-A8NH-01 | 361 | 11 | ESCA | TCGA-VR-A8EZ-01 | 374 | 21 |
| DLBC | TCGA-GS-A9U3-01 | 82 | 3 | ESCA | TCGA-L5-A8NI-01 | 373 | 13 | ESCA | TCGA-VR-A8Q7-01 | 215 | 12 |
| DLBC | TCGA-GS-A9U4-01 | 25 | 2 | ESCA | TCGA-L5-A8NJ-01 | 425 | 17 | ESCA | TCGA-VR-AA4D-01 | 236 | 12 |
| DLBC | TCGA-RQ-A68N-01 | 214 | 14 | ESCA | TCGA-L5-A8NK-01 | 379 | 13 | ESCA | TCGA-VR-AA4G-01 | 222 | 11 |
| DLBC | TCGA-RQ-A6JB-01 | 79 | 4 | ESCA | TCGA-L5-A8NL-01 | 296 | 14 | ESCA | TCGA-VR-AA7B-01 | 288 | 10 |
| DLBC | TCGA-RQ-AAAT-01 | 151 | 7 | ESCA | TCGA-L5-A8NM-01 | 2624 | 93 | ESCA | TCGA-VR-AA7D-01 | 190 | 14 |
| DLBC | TCGA-VB-A8QN-01 | 178 | 11 | ESCA | TCGA-L5-A8NN-01 | 273 | 16 | ESCA | TCGA-VR-AA7I-01 | 151 | 3 |
| ESCA | TCGA-2H-A9GF-01 | 508 | 18 | ESCA | TCGA-L5-A8NQ-01 | 435 | 15 | ESCA | TCGA-X8-AAAR-01 | 243 | 12 |
| ESCA | TCGA-2H-A9GG-01 | 295 | 9 | ESCA | TCGA-L5-A8NR-01 | 407 | 10 | ESCA | TCGA-XP-A8T6-01 | 277 | 8 |
| ESCA | TCGA-2H-A9GH-01 | 320 | 8 | ESCA | TCGA-L5-A8NS-01 | 581 | 27 | ESCA | TCGA-XP-A8T7-01 | 288 | 13 |
| ESCA | TCGA-2H-A9GI-01 | 433 | 19 | ESCA | TCGA-L5-A8NT-01 | 265 | 11 | ESCA | TCGA-XP-A8T8-01 | 287 | 9 |
| ESCA | TCGA-2H-A9GJ-01 | 265 | 9 | ESCA | TCGA-L5-A8NU-01 | 160 | 3 | ESCA | TCGA-Z6-A8JD-01 | 337 | 12 |
| ESCA | TCGA-2H-A9GK-01 | 471 | 25 | ESCA | TCGA-L5-A8NV-01 | 309 | 15 | ESCA | TCGA-Z6-A8JE-01 | 337 | 8 |
| ESCA | TCGA-2H-A9GL-01 | 383 | 18 | ESCA | TCGA-L5-A8NW-01 | 399 | 12 | ESCA | TCGA-Z6-A9VB-01 | 249 | 13 |
| ESCA | TCGA-2H-A9GM-01 | 271 | 14 | ESCA | TCGA-L7-A56G-01 | 132 | 12 | ESCA | TCGA-Z6-AAPN-01 | 532 | 21 |
| ESCA | TCGA-2H-A9GN-01 | 267 | 11 | ESCA | TCGA-L7-A6VZ-01 | 462 | 28 | ESCA | TCGA-ZR-A9CJ-01 | 258 | 9 |
| ESCA | TCGA-2H-A9GO-01 | 309 | 13 | ESCA | TCGA-LN-A49K-01 | 145 | 6 | GBM | TCGA-02-0003-01 | 62 | 6 |
| ESCA | TCGA-2H-A9GQ-01 | 296 | 12 | ESCA | TCGA-LN-A49L-01 | 252 | 5 | GBM | TCGA-02-0033-01 | 51 | 6 |
| ESCA | TCGA-2H-A9GR-01 | 543 | 23 | ESCA | TCGA-LN-A49M-01 | 339 | 13 | GBM | TCGA-02-0047-01 | 89 | 10 |
| ESCA | TCGA-IC-A6RE-01 | 1446 | 58 | ESCA | TCGA-LN-A49N-01 | 107 | 10 | GBM | TCGA-02-0055-01 | 82 | 3 |
| ESCA | TCGA-IC-A6RF-01 | 472 | 22 | ESCA | TCGA-LN-A49O-01 | 127 | 6 | GBM | TCGA-02-2470-01 | 86 | 3 |
| ESCA | TCGA-IG-A3I8-01 | 333 | 6 | ESCA | TCGA-LN-A49P-01 | 122 | 8 | GBM | TCGA-02-2483-01 | 72 | 4 |
| ESCA | TCGA-IG-A3QL-01 | 169 | 15 | ESCA | TCGA-LN-A49R-01 | 190 | 8 | GBM | TCGA-02-2485-01 | 85 | 4 |
| ESCA | TCGA-IG-A3Y9-01 | 324 | 12 | ESCA | TCGA-LN-A49S-01 | 234 | 10 | GBM | TCGA-02-2486-01 | 72 | 3 |
| ESCA | TCGA-IG-A3YA-01 | 144 | 4 | ESCA | TCGA-LN-A49U-01 | 242 | 4 | GBM | TCGA-06-0119-01 | 80 | 4 |
| ESCA | TCGA-IG-A3YB-01 | 146 | 6 | ESCA | TCGA-LN-A49V-01 | 152 | 6 | GBM | TCGA-06-0122-01 | 100 | 2 |
| ESCA | TCGA-IG-A3YC-01 | 124 | 4 | ESCA | TCGA-LN-A49W-01 | 158 | 11 | GBM | TCGA-06-0124-01 | 79 | 3 |
| ESCA | TCGA-IG-A4P3-01 | 530 | 17 | ESCA | TCGA-LN-A49X-01 | 150 | 8 | GBM | TCGA-06-0125-02 | 89 | 6 |
| ESCA | TCGA-IG-A4QS-01 | 342 | 12 | ESCA | TCGA-LN-A49Y-01 | 361 | 11 | GBM | TCGA-06-0126-01 | 71 | 3 |
| ESCA | TCGA-IG-A4QT-01 | 48 | 0 | ESCA | TCGA-LN-A4A1-01 | 230 | 11 | GBM | TCGA-06-0128-01 | 89 | 7 |
| ESCA | TCGA-IG-A50L-01 | 204 | 11 | ESCA | TCGA-LN-A4A2-01 | 251 | 6 | GBM | TCGA-06-0129-01 | 55 | 7 |
| ESCA | TCGA-IG-A51D-01 | 254 | 7 | ESCA | TCGA-LN-A4A3-01 | 150 | 4 | GBM | TCGA-06-0130-01 | 38 | 5 |
| ESCA | TCGA-IG-A5B8-01 | 396 | 12 | ESCA | TCGA-LN-A4A4-01 | 186 | 9 | GBM | TCGA-06-0132-01 | 39 | 0 |
| ESCA | TCGA-IG-A5S3-01 | 124 | 5 | ESCA | TCGA-LN-A4A5-01 | 142 | 5 | GBM | TCGA-06-0137-01 | 107 | 6 |
| ESCA | TCGA-IG-A625-01 | 152 | 5 | ESCA | TCGA-LN-A4A6-01 | 194 | 9 | GBM | TCGA-06-0139-01 | 11 | 0 |
| ESCA | TCGA-IG-A6QS-01 | 207 | 9 | ESCA | TCGA-LN-A4A8-01 | 213 | 10 | GBM | TCGA-06-0140-01 | 56 | 4 |
| ESCA | TCGA-IG-A7DP-01 | 110 | 4 | ESCA | TCGA-LN-A4A9-01 | 297 | 11 | GBM | TCGA-06-0141-01 | 37 | 0 |
| ESCA | TCGA-IG-A8O2-01 | 268 | 7 | ESCA | TCGA-LN-A4MQ-01 | 111 | 10 | GBM | TCGA-06-0142-01 | 59 | 3 |
| ESCA | TCGA-IG-A97H-01 | 272 | 10 | ESCA | TCGA-LN-A4MR-01 | 235 | 11 | GBM | TCGA-06-0145-01 | 111 | 6 |
| ESCA | TCGA-IG-A97I-01 | 216 | 11 | ESCA | TCGA-LN-A5U5-01 | 96 | 7 | GBM | TCGA-06-0151-01 | 30 | 1 |
| ESCA | TCGA-JY-A6F8-01 | 444 | 15 | ESCA | TCGA-LN-A5U6-01 | 151 | 9 | GBM | TCGA-06-0152-01 | 81 | 5 |
| ESCA | TCGA-JY-A6FA-01 | 260 | 16 | ESCA | TCGA-LN-A5U7-01 | 226 | 8 | GBM | TCGA-06-0154-01 | 81 | 4 |
| ESCA | TCGA-JY-A6FB-01 | 316 | 9 | ESCA | TCGA-LN-A7HV-01 | 182 | 8 | GBM | TCGA-06-0155-01 | 92 | 8 |
| ESCA | TCGA-JY-A6FD-01 | 342 | 18 | ESCA | TCGA-LN-A7HW-01 | 170 | 5 | GBM | TCGA-06-0157-01 | 66 | 5 |
| ESCA | TCGA-JY-A6FE-01 | 217 | 5 | ESCA | TCGA-LN-A7HX-01 | 362 | 14 | GBM | TCGA-06-0158-01 | 74 | 4 |
| ESCA | TCGA-JY-A6FG-01 | 409 | 13 | ESCA | TCGA-LN-A7HY-01 | 278 | 9 | GBM | TCGA-06-0165-01 | 9 | 1 |
| ESCA | TCGA-JY-A6FH-01 | 345 | 15 | ESCA | TCGA-LN-A7HZ-01 | 149 | 7 | GBM | TCGA-06-0166-01 | 59 | 4 |
| ESCA | TCGA-JY-A93B-01 | 259 | 13 | ESCA | TCGA-LN-A8HZ-01 | 279 | 15 | GBM | TCGA-06-0167-01 | 7 | 0 |
| ESCA | TCGA-JY-A93D-01 | 229 | 13 | ESCA | TCGA-LN-A8I0-01 | 272 | 11 | GBM | TCGA-06-0168-01 | 60 | 5 |
| ESCA | TCGA-JY-A93C-01 | 220 | 6 | ESCA | TCGA-LN-A8I1-01 | 269 | 12 | GBM | TCGA-06-0169-01 | 73 | 1 |
| ESCA | TCGA-JY-A93D-01 | 340 | 10 | ESCA | TCGA-LN-A9FO-01 | 251 | 10 | GBM | TCGA-06-0171-02 | 70 | 3 |
| ESCA | TCGA-JY-A93E-01 | 345 | 15 | ESCA | TCGA-LN-A9FP-01 | 638 | 18 | GBM | TCGA-06-0173-01 | 106 | 4 |
| ESCA | TCGA-JY-A93F-01 | 252 | 15 | ESCA | TCGA-LN-A9FQ-01 | 224 | 9 | GBM | TCGA-06-0174-01 | 117 | 3 |
| ESCA | TCGA-KH-A6WC-01 | 254 | 9 | ESCA | TCGA-LN-A9FR-01 | 203 | 13 | GBM | TCGA-06-0178-01 | 5 | 0 |
| ESCA | TCGA-L5-A43C-01 | 226 | 10 | ESCA | TCGA-M9-A5M8-01 | 129 | 11 | GBM | TCGA-06-0184-01 | 74 | 7 |
| ESCA | TCGA-L5-A43E-01 | 491 | 16 | ESCA | TCGA-Q9-A6FU-01 | 302 | 8 | GBM | TCGA-06-0185-01 | 89 | 6 |
| ESCA | TCGA-L5-A43H-01 | 151 | 8 | ESCA | TCGA-Q9-A6FW-01 | 335 | 11 | GBM | TCGA-06-0188-01 | 62 | 7 |
| ESCA | TCGA-L5-A43I-01 | 322 | 12 | ESCA | TCGA-R6-A6DN-01 | 225 | 10 | GBM | TCGA-06-0189-01 | 31 | 1 |
| ESCA | TCGA-L5-A43J-01 | 1294 | 39 | ESCA | TCGA-R6-A6DQ-01 | 176 | 11 | GBM | TCGA-06-0190-02 | 88 | 8 |
| ESCA | TCGA-L5-A43M-01 | 93 | 0 | ESCA | TCGA-R6-A6KZ-01 | 256 | 14 | GBM | TCGA-06-0192-01 | 87 | 5 |
| ESCA | TCGA-L5-A4OE-01 | 450 | 15 | ESCA | TCGA-R6-A6L4-01 | 181 | 11 | GBM | TCGA-06-0195-01 | 102 | 5 |
| ESCA | TCGA-L5-A4OF-01 | 159 | 6 | ESCA | TCGA-R6-A6L6-01 | 271 | 11 | GBM | TCGA-06-0209-01 | 91 | 2 |
| ESCA | TCGA-L5-A4OG-01 | 260 | 14 | ESCA | TCGA-R6-A6XG-01 | 457 | 22 | GBM | TCGA-06-0210-02 | 80 | 9 |
| ESCA | TCGA-L5-A4OH-01 | 441 | 13 | ESCA | TCGA-R6-A6Y0-01 | 304 | 10 | GBM | TCGA-06-0211-02 | 85 | 3 |
| ESCA | TCGA-L5-A4OI-01 | 3225 | 128 | ESCA | TCGA-R6-A6Y0-01 | 389 | 16 | GBM | TCGA-06-0213-01 | 80 | 8 |
| ESCA | TCGA-L5-A4OJ-01 | 559 | 20 | ESCA | TCGA-R6-A6Y2-01 | 362 | 15 | GBM | TCGA-06-0214-01 | 95 | 8 |
| ESCA | TCGA-L5-A4OM-01 | 114 | 4 | ESCA | TCGA-R6-A8W5-01 | 239 | 12 | GBM | TCGA-06-0216-01 | 75 | 2 |
| ESCA | TCGA-L5-A4ON-01 | 234 | 14 | ESCA | TCGA-R6-A8W8-01 | 308 | 12 | GBM | TCGA-06-0219-01 | 55 | 1 |
| ESCA | TCGA-L5-A4OO-01 | 175 | 9 | ESCA | TCGA-R6-A8WC-01 | 319 | 11 | GBM | TCGA-06-0221-02 | 58 | 4 |
| ESCA | TCGA-L5-A4OP-01 | 180 | 10 | ESCA | TCGA-R6-A8WG-01 | 286 | 10 | GBM | TCGA-06-0237-01 | 63 | 5 |
| ESCA | TCGA-L5-A4OQ-01 | 112 | 6 | ESCA | TCGA-RE-A7BO-01 | 431 | 12 | GBM | TCGA-06-0238-01 | 46 | 6 |
| ESCA | TCGA-L5-A4OR-01 | 259 | 7 | ESCA | TCGA-S8-A6BV-01 | 263 | 6 | GBM | TCGA-06-0240-01 | 15 | 0 |

## TABLE 5 (APPENDIX A)

| | | | |
|---|---|---|---|
| GBM | TCGA-06-0241-01 | 81 | 8 |
| GBM | TCGA-06-0644-01 | 80 | 7 |
| GBM | TCGA-06-0645-01 | 75 | 2 |
| GBM | TCGA-06-0646-01 | 58 | 4 |
| GBM | TCGA-06-0648-01 | 91 | 3 |
| GBM | TCGA-06-0649-01 | 131 | 9 |
| GBM | TCGA-06-0650-01 | 47 | 6 |
| GBM | TCGA-06-0686-01 | 81 | 3 |
| GBM | TCGA-06-0743-01 | 126 | 8 |
| GBM | TCGA-06-0744-01 | 100 | 4 |
| GBM | TCGA-06-0745-01 | 60 | 2 |
| GBM | TCGA-06-0747-01 | 96 | 3 |
| GBM | TCGA-06-0749-01 | 76 | 5 |
| GBM | TCGA-06-0750-01 | 52 | 3 |
| GBM | TCGA-06-0875-01 | 79 | 5 |
| GBM | TCGA-06-0876-01 | 89 | 6 |
| GBM | TCGA-06-0877-01 | 93 | 4 |
| GBM | TCGA-06-0878-01 | 63 | 6 |
| GBM | TCGA-06-0879-01 | 73 | 7 |
| GBM | TCGA-06-0881-01 | 39 | 2 |
| GBM | TCGA-06-0882-01 | 51 | 2 |
| GBM | TCGA-06-0939-01 | 111 | 8 |
| GBM | TCGA-06-1804-01 | 101 | 4 |
| GBM | TCGA-06-1806-01 | 40 | 3 |
| GBM | TCGA-06-2557-01 | 72 | 6 |
| GBM | TCGA-06-2558-01 | 104 | 6 |
| GBM | TCGA-06-2559-01 | 92 | 10 |
| GBM | TCGA-06-2561-01 | 51 | 7 |
| GBM | TCGA-06-2562-01 | 89 | 7 |
| GBM | TCGA-06-2563-01 | 93 | 4 |
| GBM | TCGA-06-2564-01 | 82 | 2 |
| GBM | TCGA-06-2565-01 | 78 | 4 |
| GBM | TCGA-06-2567-01 | 80 | 4 |
| GBM | TCGA-06-2569-01 | 43 | 4 |
| GBM | TCGA-06-2570-01 | 54 | 6 |
| GBM | TCGA-06-5408-01 | 70 | 4 |
| GBM | TCGA-06-5410-01 | 42 | 3 |
| GBM | TCGA-06-5411-01 | 51 | 5 |
| GBM | TCGA-06-5412-01 | 76 | 6 |
| GBM | TCGA-06-5413-01 | 72 | 3 |
| GBM | TCGA-06-5414-01 | 55 | 3 |
| GBM | TCGA-06-5415-01 | 73 | 2 |
| GBM | TCGA-06-5417-01 | 72 | 6 |
| GBM | TCGA-06-5418-01 | 67 | 4 |
| GBM | TCGA-06-5856-01 | 75 | 2 |
| GBM | TCGA-06-5858-01 | 269 | 10 |
| GBM | TCGA-06-5859-01 | 58 | 3 |
| GBM | TCGA-06-6388-01 | 71 | 5 |
| GBM | TCGA-06-6389-01 | 51 | 3 |
| GBM | TCGA-06-6390-01 | 57 | 3 |
| GBM | TCGA-06-6391-01 | 82 | 5 |
| GBM | TCGA-06-6693-01 | 74 | 3 |
| GBM | TCGA-06-6694-01 | 117 | 8 |
| GBM | TCGA-06-6695-01 | 75 | 6 |
| GBM | TCGA-06-6697-01 | 82 | 4 |
| GBM | TCGA-06-6698-01 | 54 | 8 |
| GBM | TCGA-06-6699-01 | 83 | 4 |
| GBM | TCGA-06-6700-01 | 66 | 2 |
| GBM | TCGA-06-6701-01 | 56 | 4 |
| GBM | TCGA-08-0386-01 | 17 | 0 |
| GBM | TCGA-12-0615-01 | 80 | 2 |
| GBM | TCGA-12-0616-01 | 58 | 3 |
| GBM | TCGA-12-0618-01 | 69 | 6 |
| GBM | TCGA-12-0688-01 | 76 | 6 |
| GBM | TCGA-12-0692-01 | 99 | 4 |
| GBM | TCGA-12-0821-01 | 120 | 7 |
| GBM | TCGA-12-1597-01 | 97 | 6 |
| GBM | TCGA-12-3649-01 | 107 | 5 |
| GBM | TCGA-12-3650-01 | 73 | 3 |
| GBM | TCGA-12-3652-01 | 77 | 3 |
| GBM | TCGA-12-3653-01 | 49 | 4 |
| GBM | TCGA-12-5295-01 | 95 | 2 |
| GBM | TCGA-12-5299-01 | 55 | 1 |
| GBM | TCGA-12-5301-01 | 91 | 6 |
| GBM | TCGA-14-0740-01 | 62 | 4 |
| GBM | TCGA-14-0781-01 | 44 | 4 |
| GBM | TCGA-14-0786-01 | 57 | 2 |
| GBM | TCGA-14-0787-01 | 53 | 1 |
| GBM | TCGA-14-0789-01 | 95 | 8 |
| GBM | TCGA-14-0790-01 | 84 | 7 |
| GBM | TCGA-14-0813-01 | 121 | 8 |
| GBM | TCGA-14-0817-01 | 91 | 2 |
| GBM | TCGA-14-0862-01 | 55 | 3 |
| GBM | TCGA-14-0871-01 | 72 | 5 |
| GBM | TCGA-14-1034-02 | 93 | 6 |
| GBM | TCGA-14-1043-01 | 32 | 5 |
| GBM | TCGA-14-1395-01 | 69 | 5 |
| GBM | TCGA-14-1450-01 | 77 | 5 |
| GBM | TCGA-14-1456-01 | 36 | 7 |
| GBM | TCGA-14-1823-01 | 70 | 3 |
| GBM | TCGA-14-1825-01 | 66 | 3 |
| GBM | TCGA-14-1829-01 | 69 | 3 |
| GBM | TCGA-14-2554-01 | 85 | 4 |
| GBM | TCGA-14-3476-01 | 109 | 8 |
| GBM | TCGA-14-4157-01 | 74 | 5 |
| GBM | TCGA-15-0742-01 | 88 | 7 |
| GBM | TCGA-15-1444-01 | 24 | 6 |
| GBM | TCGA-16-0846-01 | 90 | 5 |
| GBM | TCGA-16-0861-01 | 80 | 3 |
| GBM | TCGA-16-1045-01 | 125 | 3 |
| GBM | TCGA-16-1048-01 | 108 | 4 |
| GBM | TCGA-19-1390-01 | 142 | 11 |
| GBM | TCGA-19-1790-01 | 192 | 11 |
| GBM | TCGA-19-2619-01 | 67 | 2 |
| GBM | TCGA-19-2620-01 | 90 | 5 |
| GBM | TCGA-19-2623-01 | 118 | 5 |
| GBM | TCGA-19-2624-01 | 59 | 1 |
| GBM | TCGA-19-2625-01 | 72 | 6 |
| GBM | TCGA-19-2629-01 | 127 | 6 |
| GBM | TCGA-19-2631-01 | 130 | 3 |
| GBM | TCGA-19-4068-01 | 91 | 3 |
| GBM | TCGA-19-5947-01 | 32 | 2 |
| GBM | TCGA-19-5950-01 | 68 | 1 |
| GBM | TCGA-19-5951-01 | 99 | 3 |
| GBM | TCGA-19-5952-01 | 54 | 3 |
| GBM | TCGA-19-5953-01 | 81 | 4 |
| GBM | TCGA-19-5954-01 | 96 | 3 |
| GBM | TCGA-19-5955-01 | 104 | 7 |
| GBM | TCGA-19-5958-01 | 57 | 3 |
| GBM | TCGA-19-5959-01 | 107 | 1 |
| GBM | TCGA-19-5960-01 | 55 | 1 |
| GBM | TCGA-26-1439-01 | 67 | 4 |
| GBM | TCGA-26-1442-01 | 60 | 3 |
| GBM | TCGA-26-5132-01 | 61 | 4 |
| GBM | TCGA-26-5133-01 | 71 | 5 |
| GBM | TCGA-26-5134-01 | 69 | 3 |
| GBM | TCGA-26-5135-01 | 84 | 4 |
| GBM | TCGA-26-5136-01 | 72 | 4 |
| GBM | TCGA-26-5139-01 | 63 | 4 |
| GBM | TCGA-26-6173-01 | 47 | 4 |
| GBM | TCGA-26-6174-01 | 108 | 5 |
| GBM | TCGA-27-1830-01 | 69 | 2 |
| GBM | TCGA-27-1831-01 | 75 | 6 |
| GBM | TCGA-27-1832-01 | 52 | 1 |
| GBM | TCGA-27-1833-01 | 78 | 4 |
| GBM | TCGA-27-1834-01 | 73 | 5 |
| GBM | TCGA-27-1835-01 | 82 | 6 |
| GBM | TCGA-27-1836-01 | 56 | 3 |
| GBM | TCGA-27-1837-01 | 46 | 3 |
| GBM | TCGA-27-1838-01 | 121 | 9 |
| GBM | TCGA-27-2518-01 | 68 | 3 |
| GBM | TCGA-27-2519-01 | 56 | 2 |
| GBM | TCGA-27-2521-01 | 88 | 8 |
| GBM | TCGA-27-2523-01 | 68 | 2 |
| GBM | TCGA-27-2524-01 | 76 | 4 |
| GBM | TCGA-27-2526-01 | 58 | 3 |
| GBM | TCGA-27-2527-01 | 85 | 5 |
| GBM | TCGA-27-2528-01 | 61 | 5 |
| GBM | TCGA-28-1747-01 | 59 | 4 |
| GBM | TCGA-28-1753-01 | 71 | 4 |
| GBM | TCGA-28-2499-01 | 37 | 3 |
| GBM | TCGA-28-2501-01 | 65 | 1 |
| GBM | TCGA-28-2502-01 | 74 | 4 |
| GBM | TCGA-28-2509-01 | 76 | 6 |
| GBM | TCGA-28-2510-01 | 36 | 1 |
| GBM | TCGA-28-2513-01 | 101 | 4 |
| GBM | TCGA-28-2514-01 | 64 | 1 |
| GBM | TCGA-28-5204-01 | 57 | 1 |
| GBM | TCGA-28-5207-01 | 73 | 8 |
| GBM | TCGA-28-5208-01 | 78 | 7 |
| GBM | TCGA-28-5209-01 | 120 | 7 |
| GBM | TCGA-28-5211-01 | 48 | 0 |
| GBM | TCGA-28-5213-01 | 66 | 4 |
| GBM | TCGA-28-5214-01 | 62 | 5 |
| GBM | TCGA-28-5215-01 | 50 | 1 |
| GBM | TCGA-28-5216-01 | 65 | 4 |
| GBM | TCGA-28-5218-01 | 36 | 3 |
| GBM | TCGA-28-5219-01 | 67 | 6 |
| GBM | TCGA-28-5220-01 | 52 | 2 |
| GBM | TCGA-28-6450-01 | 72 | 4 |
| GBM | TCGA-32-1970-01 | 103 | 9 |
| GBM | TCGA-32-1977-01 | 122 | 6 |
| GBM | TCGA-32-1979-01 | 105 | 6 |
| GBM | TCGA-32-1980-01 | 24 | 2 |
| GBM | TCGA-32-1982-01 | 102 | 7 |
| GBM | TCGA-32-1986-01 | 64 | 3 |
| GBM | TCGA-32-1991-01 | 88 | 6 |
| GBM | TCGA-32-2491-01 | 134 | 7 |
| GBM | TCGA-32-2494-01 | 101 | 6 |
| GBM | TCGA-32-2495-01 | 120 | 9 |
| GBM | TCGA-32-2615-01 | 56 | 1 |
| GBM | TCGA-32-2632-01 | 126 | 8 |
| GBM | TCGA-32-2634-01 | 63 | 5 |
| GBM | TCGA-32-2638-01 | 78 | 2 |
| GBM | TCGA-32-4208-01 | 87 | 5 |
| GBM | TCGA-32-4209-01 | 54 | 2 |
| GBM | TCGA-32-4210-01 | 126 | 5 |
| GBM | TCGA-32-4211-01 | 116 | 4 |
| GBM | TCGA-32-4213-01 | 92 | 3 |
| GBM | TCGA-32-4719-01 | 61 | 5 |
| GBM | TCGA-32-5222-01 | 104 | 5 |
| GBM | TCGA-41-2571-01 | 68 | 4 |
| GBM | TCGA-41-2572-01 | 90 | 6 |
| GBM | TCGA-41-2573-01 | 58 | 3 |
| GBM | TCGA-41-2575-01 | 84 | 4 |
| GBM | TCGA-41-3392-01 | 91 | 5 |
| GBM | TCGA-41-3393-01 | 103 | 4 |
| GBM | TCGA-41-3915-01 | 75 | 5 |
| GBM | TCGA-41-4097-01 | 89 | 5 |
| GBM | TCGA-41-5651-01 | 113 | 5 |
| GBM | TCGA-41-6646-01 | 62 | 3 |
| GBM | TCGA-74-6573-01 | 60 | 6 |
| GBM | TCGA-74-6575-01 | 128 | 7 |
| GBM | TCGA-74-6577-01 | 51 | 4 |
| GBM | TCGA-74-6578-01 | 94 | 4 |
| GBM | TCGA-74-6584-01 | 48 | 1 |
| GBM | TCGA-76-4925-01 | 82 | 7 |
| GBM | TCGA-76-4926-01 | 74 | 1 |
| GBM | TCGA-76-4927-01 | 73 | 4 |
| GBM | TCGA-76-4928-01 | 108 | 7 |
| GBM | TCGA-76-4929-01 | 77 | 6 |
| GBM | TCGA-76-4931-01 | 63 | 5 |
| GBM | TCGA-76-4932-01 | 84 | 0 |
| GBM | TCGA-76-4934-01 | 74 | 4 |
| GBM | TCGA-76-4935-01 | 78 | 3 |
| GBM | TCGA-76-6191-01 | 69 | 6 |
| GBM | TCGA-76-6192-01 | 72 | 2 |
| GBM | TCGA-76-6193-01 | 58 | 5 |
| GBM | TCGA-76-6280-01 | 75 | 2 |
| GBM | TCGA-76-6282-01 | 65 | 3 |
| GBM | TCGA-76-6283-01 | 119 | 5 |
| GBM | TCGA-76-6285-01 | 74 | 1 |
| GBM | TCGA-76-6286-01 | 90 | 7 |
| GBM | TCGA-76-6656-01 | 121 | 6 |
| GBM | TCGA-76-6657-01 | 74 | 10 |
| GBM | TCGA-76-6660-01 | 107 | 7 |
| GBM | TCGA-76-6661-01 | 69 | 6 |
| GBM | TCGA-76-6662-01 | 54 | 6 |
| GBM | TCGA-76-6663-01 | 76 | 9 |
| GBM | TCGA-76-6664-01 | 67 | 2 |
| GBM | TCGA-81-5910-01 | 63 | 4 |
| GBM | TCGA-81-5911-01 | 43 | 4 |
| GBM | TCGA-87-5896-01 | 70 | 2 |
| GBMLGG | TCGA-02-0003-01 | 62 | 6 |
| GBMLGG | TCGA-02-0033-01 | 51 | 6 |
| GBMLGG | TCGA-02-0047-01 | 89 | 10 |
| GBMLGG | TCGA-02-0055-01 | 82 | 3 |
| GBMLGG | TCGA-02-2470-01 | 86 | 3 |
| GBMLGG | TCGA-02-2483-01 | 72 | 4 |
| GBMLGG | TCGA-02-2485-01 | 85 | 4 |
| GBMLGG | TCGA-02-2486-01 | 72 | 3 |
| GBMLGG | TCGA-06-0119-01 | 80 | 4 |
| GBMLGG | TCGA-06-0122-01 | 100 | 2 |
| GBMLGG | TCGA-06-0124-01 | 79 | 3 |
| GBMLGG | TCGA-06-0125-02 | 89 | 6 |
| GBMLGG | TCGA-06-0126-01 | 71 | 3 |
| GBMLGG | TCGA-06-0128-01 | 89 | 7 |
| GBMLGG | TCGA-06-0129-01 | 55 | 7 |
| GBMLGG | TCGA-06-0130-01 | 38 | 5 |
| GBMLGG | TCGA-06-0132-01 | 39 | 0 |
| GBMLGG | TCGA-06-0137-01 | 107 | 6 |
| GBMLGG | TCGA-06-0139-01 | 11 | 0 |
| GBMLGG | TCGA-06-0140-01 | 56 | 4 |
| GBMLGG | TCGA-06-0141-01 | 37 | 0 |
| GBMLGG | TCGA-06-0142-01 | 59 | 3 |
| GBMLGG | TCGA-06-0145-01 | 111 | 6 |
| GBMLGG | TCGA-06-0151-01 | 30 | 1 |
| GBMLGG | TCGA-06-0152-01 | 81 | 5 |
| GBMLGG | TCGA-06-0154-01 | 81 | 4 |
| GBMLGG | TCGA-06-0155-01 | 92 | 8 |
| GBMLGG | TCGA-06-0157-01 | 66 | 5 |
| GBMLGG | TCGA-06-0158-01 | 74 | 4 |
| GBMLGG | TCGA-06-0165-01 | 9 | 1 |

## TABLE 5 (APPENDIX A)

| Group | Sample | Value 1 | Value 2 |
|---|---|---|---|
| GBMLGG | TCGA-06-0166-01 | 59 | 4 |
| GBMLGG | TCGA-06-0167-01 | 7 | 0 |
| GBMLGG | TCGA-06-0168-01 | 60 | 5 |
| GBMLGG | TCGA-06-0169-01 | 73 | 1 |
| GBMLGG | TCGA-06-0171-02 | 70 | 3 |
| GBMLGG | TCGA-06-0173-01 | 106 | 4 |
| GBMLGG | TCGA-06-0174-01 | 117 | 3 |
| GBMLGG | TCGA-06-0178-01 | 5 | 0 |
| GBMLGG | TCGA-06-0184-01 | 74 | 7 |
| GBMLGG | TCGA-06-0185-01 | 89 | 6 |
| GBMLGG | TCGA-06-0188-01 | 62 | 7 |
| GBMLGG | TCGA-06-0189-01 | 31 | 1 |
| GBMLGG | TCGA-06-0190-02 | 88 | 8 |
| GBMLGG | TCGA-06-0192-01 | 87 | 5 |
| GBMLGG | TCGA-06-0195-01 | 102 | 5 |
| GBMLGG | TCGA-06-0209-01 | 91 | 2 |
| GBMLGG | TCGA-06-0210-02 | 80 | 9 |
| GBMLGG | TCGA-06-0211-02 | 85 | 3 |
| GBMLGG | TCGA-06-0213-01 | 80 | 8 |
| GBMLGG | TCGA-06-0214-01 | 95 | 8 |
| GBMLGG | TCGA-06-0216-01 | 75 | 2 |
| GBMLGG | TCGA-06-0219-01 | 55 | 1 |
| GBMLGG | TCGA-06-0221-02 | 58 | 4 |
| GBMLGG | TCGA-06-0237-01 | 63 | 5 |
| GBMLGG | TCGA-06-0238-01 | 46 | 6 |
| GBMLGG | TCGA-06-0240-01 | 15 | 0 |
| GBMLGG | TCGA-06-0241-01 | 81 | 8 |
| GBMLGG | TCGA-06-0644-01 | 80 | 7 |
| GBMLGG | TCGA-06-0645-01 | 75 | 2 |
| GBMLGG | TCGA-06-0646-01 | 58 | 4 |
| GBMLGG | TCGA-06-0648-01 | 91 | 3 |
| GBMLGG | TCGA-06-0649-01 | 131 | 9 |
| GBMLGG | TCGA-06-0650-01 | 47 | 6 |
| GBMLGG | TCGA-06-0686-01 | 81 | 3 |
| GBMLGG | TCGA-06-0743-01 | 126 | 8 |
| GBMLGG | TCGA-06-0744-01 | 100 | 4 |
| GBMLGG | TCGA-06-0745-01 | 60 | 2 |
| GBMLGG | TCGA-06-0747-01 | 96 | 3 |
| GBMLGG | TCGA-06-0749-01 | 76 | 5 |
| GBMLGG | TCGA-06-0750-01 | 52 | 3 |
| GBMLGG | TCGA-06-0875-01 | 79 | 5 |
| GBMLGG | TCGA-06-0876-01 | 89 | 6 |
| GBMLGG | TCGA-06-0877-01 | 93 | 4 |
| GBMLGG | TCGA-06-0878-01 | 63 | 6 |
| GBMLGG | TCGA-06-0879-01 | 73 | 7 |
| GBMLGG | TCGA-06-0881-01 | 39 | 2 |
| GBMLGG | TCGA-06-0882-01 | 51 | 2 |
| GBMLGG | TCGA-06-0939-01 | 111 | 8 |
| GBMLGG | TCGA-06-1804-01 | 101 | 4 |
| GBMLGG | TCGA-06-1806-01 | 40 | 3 |
| GBMLGG | TCGA-06-2557-01 | 72 | 6 |
| GBMLGG | TCGA-06-2558-01 | 104 | 6 |
| GBMLGG | TCGA-06-2559-01 | 92 | 10 |
| GBMLGG | TCGA-06-2561-01 | 51 | 7 |
| GBMLGG | TCGA-06-2562-01 | 89 | 7 |
| GBMLGG | TCGA-06-2563-01 | 93 | 4 |
| GBMLGG | TCGA-06-2564-01 | 82 | 2 |
| GBMLGG | TCGA-06-2565-01 | 78 | 4 |
| GBMLGG | TCGA-06-2567-01 | 80 | 4 |
| GBMLGG | TCGA-06-2569-01 | 43 | 4 |
| GBMLGG | TCGA-06-2570-01 | 54 | 6 |
| GBMLGG | TCGA-06-5408-01 | 70 | 4 |
| GBMLGG | TCGA-06-5410-01 | 42 | 3 |
| GBMLGG | TCGA-06-5411-01 | 51 | 5 |
| GBMLGG | TCGA-06-5412-01 | 76 | 6 |
| GBMLGG | TCGA-06-5413-01 | 72 | 3 |
| GBMLGG | TCGA-06-5414-01 | 55 | 3 |
| GBMLGG | TCGA-06-5415-01 | 73 | 2 |
| GBMLGG | TCGA-06-5417-01 | 72 | 6 |
| GBMLGG | TCGA-06-5418-01 | 67 | 4 |
| GBMLGG | TCGA-06-5856-01 | 75 | 2 |
| GBMLGG | TCGA-06-5858-01 | 269 | 10 |
| GBMLGG | TCGA-06-5859-01 | 58 | 3 |
| GBMLGG | TCGA-06-6388-01 | 71 | 5 |
| GBMLGG | TCGA-06-6389-01 | 51 | 3 |
| GBMLGG | TCGA-06-6390-01 | 57 | 3 |
| GBMLGG | TCGA-06-6391-01 | 82 | 5 |
| GBMLGG | TCGA-06-6693-01 | 74 | 3 |
| GBMLGG | TCGA-06-6694-01 | 117 | 8 |
| GBMLGG | TCGA-06-6695-01 | 75 | 6 |
| GBMLGG | TCGA-06-6697-01 | 82 | 4 |
| GBMLGG | TCGA-06-6698-01 | 54 | 8 |
| GBMLGG | TCGA-06-6699-01 | 83 | 4 |
| GBMLGG | TCGA-06-6700-01 | 66 | 2 |
| GBMLGG | TCGA-06-6701-01 | 56 | 4 |
| GBMLGG | TCGA-08-0386-01 | 17 | 0 |
| GBMLGG | TCGA-12-0615-01 | 80 | 2 |
| GBMLGG | TCGA-12-0616-01 | 58 | 3 |
| GBMLGG | TCGA-12-0618-01 | 69 | 6 |
| GBMLGG | TCGA-12-0619-01 | 73 | 5 |
| GBMLGG | TCGA-12-0688-01 | 76 | 6 |
| GBMLGG | TCGA-12-0692-01 | 99 | 4 |
| GBMLGG | TCGA-12-0821-01 | 120 | 7 |
| GBMLGG | TCGA-12-1597-01 | 97 | 6 |
| GBMLGG | TCGA-12-3649-01 | 107 | 5 |
| GBMLGG | TCGA-12-3650-01 | 73 | 3 |
| GBMLGG | TCGA-12-3652-01 | 77 | 3 |
| GBMLGG | TCGA-12-3653-01 | 49 | 4 |
| GBMLGG | TCGA-12-5295-01 | 95 | 2 |
| GBMLGG | TCGA-12-5299-01 | 55 | 1 |
| GBMLGG | TCGA-12-5301-01 | 91 | 6 |
| GBMLGG | TCGA-14-0740-01 | 62 | 4 |
| GBMLGG | TCGA-14-0781-01 | 44 | 4 |
| GBMLGG | TCGA-14-0786-01 | 57 | 2 |
| GBMLGG | TCGA-14-0787-01 | 53 | 1 |
| GBMLGG | TCGA-14-0789-01 | 95 | 8 |
| GBMLGG | TCGA-14-0790-01 | 84 | 7 |
| GBMLGG | TCGA-14-0813-01 | 121 | 8 |
| GBMLGG | TCGA-14-0817-01 | 91 | 2 |
| GBMLGG | TCGA-14-0862-01 | 55 | 3 |
| GBMLGG | TCGA-14-0871-01 | 72 | 5 |
| GBMLGG | TCGA-14-1034-02 | 93 | 6 |
| GBMLGG | TCGA-14-1043-01 | 32 | 5 |
| GBMLGG | TCGA-14-1395-01 | 69 | 5 |
| GBMLGG | TCGA-14-1450-01 | 77 | 5 |
| GBMLGG | TCGA-14-1456-01 | 36 | 7 |
| GBMLGG | TCGA-14-1823-01 | 70 | 3 |
| GBMLGG | TCGA-14-1825-01 | 66 | 3 |
| GBMLGG | TCGA-14-1829-01 | 69 | 3 |
| GBMLGG | TCGA-14-2554-01 | 85 | 4 |
| GBMLGG | TCGA-14-3476-01 | 109 | 8 |
| GBMLGG | TCGA-14-4157-01 | 74 | 5 |
| GBMLGG | TCGA-15-0742-01 | 88 | 7 |
| GBMLGG | TCGA-15-1444-01 | 24 | 6 |
| GBMLGG | TCGA-16-0846-01 | 90 | 5 |
| GBMLGG | TCGA-16-0861-01 | 80 | 3 |
| GBMLGG | TCGA-16-1045-01 | 125 | 3 |
| GBMLGG | TCGA-16-1048-01 | 108 | 4 |
| GBMLGG | TCGA-19-1390-01 | 142 | 11 |
| GBMLGG | TCGA-19-1790-01 | 192 | 11 |
| GBMLGG | TCGA-19-2619-01 | 67 | 2 |
| GBMLGG | TCGA-19-2620-01 | 90 | 5 |
| GBMLGG | TCGA-19-2623-01 | 118 | 5 |
| GBMLGG | TCGA-19-2624-01 | 59 | 1 |
| GBMLGG | TCGA-19-2625-01 | 72 | 6 |
| GBMLGG | TCGA-19-2629-01 | 127 | 6 |
| GBMLGG | TCGA-19-2631-01 | 130 | 3 |
| GBMLGG | TCGA-19-4068-01 | 91 | 3 |
| GBMLGG | TCGA-19-5947-01 | 32 | 2 |
| GBMLGG | TCGA-19-5950-01 | 68 | 1 |
| GBMLGG | TCGA-19-5951-01 | 99 | 3 |
| GBMLGG | TCGA-19-5952-01 | 54 | 3 |
| GBMLGG | TCGA-19-5953-01 | 81 | 4 |
| GBMLGG | TCGA-19-5954-01 | 96 | 3 |
| GBMLGG | TCGA-19-5955-01 | 104 | 7 |
| GBMLGG | TCGA-19-5958-01 | 57 | 3 |
| GBMLGG | TCGA-19-5959-01 | 107 | 1 |
| GBMLGG | TCGA-19-5960-01 | 55 | 1 |
| GBMLGG | TCGA-26-1439-01 | 67 | 4 |
| GBMLGG | TCGA-26-1442-01 | 60 | 3 |
| GBMLGG | TCGA-26-5132-01 | 61 | 4 |
| GBMLGG | TCGA-26-5134-01 | 69 | 3 |
| GBMLGG | TCGA-26-5135-01 | 84 | 4 |
| GBMLGG | TCGA-26-5136-01 | 72 | 4 |
| GBMLGG | TCGA-26-5139-01 | 63 | 4 |
| GBMLGG | TCGA-26-6173-01 | 47 | 4 |
| GBMLGG | TCGA-26-6174-01 | 108 | 5 |
| GBMLGG | TCGA-27-1830-01 | 69 | 2 |
| GBMLGG | TCGA-27-1831-01 | 75 | 6 |
| GBMLGG | TCGA-27-1832-01 | 52 | 1 |
| GBMLGG | TCGA-27-1833-01 | 78 | 4 |
| GBMLGG | TCGA-27-1834-01 | 73 | 5 |
| GBMLGG | TCGA-27-1835-01 | 82 | 6 |
| GBMLGG | TCGA-27-1836-01 | 56 | 3 |
| GBMLGG | TCGA-27-1837-01 | 46 | 3 |
| GBMLGG | TCGA-27-1838-01 | 121 | 9 |
| GBMLGG | TCGA-27-2518-01 | 68 | 3 |
| GBMLGG | TCGA-27-2519-01 | 56 | 2 |
| GBMLGG | TCGA-27-2521-01 | 88 | 8 |
| GBMLGG | TCGA-27-2523-01 | 68 | 2 |
| GBMLGG | TCGA-27-2524-01 | 76 | 4 |
| GBMLGG | TCGA-27-2526-01 | 58 | 3 |
| GBMLGG | TCGA-27-2527-01 | 85 | 5 |
| GBMLGG | TCGA-27-2528-01 | 61 | 5 |
| GBMLGG | TCGA-28-1747-01 | 59 | 4 |
| GBMLGG | TCGA-28-1753-01 | 71 | 4 |
| GBMLGG | TCGA-28-2499-01 | 37 | 3 |
| GBMLGG | TCGA-28-2501-01 | 65 | 1 |
| GBMLGG | TCGA-28-2502-01 | 74 | 4 |
| GBMLGG | TCGA-28-2509-01 | 76 | 6 |
| GBMLGG | TCGA-28-2510-01 | 36 | 1 |
| GBMLGG | TCGA-28-2513-01 | 101 | 4 |
| GBMLGG | TCGA-28-2514-01 | 64 | 1 |
| GBMLGG | TCGA-28-5204-01 | 57 | 1 |
| GBMLGG | TCGA-28-5207-01 | 73 | 8 |
| GBMLGG | TCGA-28-5208-01 | 78 | 7 |
| GBMLGG | TCGA-28-5209-01 | 120 | 7 |
| GBMLGG | TCGA-28-5211-01 | 48 | 0 |
| GBMLGG | TCGA-28-5213-01 | 66 | 4 |
| GBMLGG | TCGA-28-5214-01 | 62 | 5 |
| GBMLGG | TCGA-28-5215-01 | 50 | 1 |
| GBMLGG | TCGA-28-5216-01 | 65 | 4 |
| GBMLGG | TCGA-28-5218-01 | 36 | 3 |
| GBMLGG | TCGA-28-5219-01 | 67 | 6 |
| GBMLGG | TCGA-28-5220-01 | 52 | 2 |
| GBMLGG | TCGA-28-6450-01 | 72 | 4 |
| GBMLGG | TCGA-32-1970-01 | 103 | 9 |
| GBMLGG | TCGA-32-1977-01 | 122 | 6 |
| GBMLGG | TCGA-32-1979-01 | 105 | 6 |
| GBMLGG | TCGA-32-1980-01 | 24 | 2 |
| GBMLGG | TCGA-32-1982-01 | 102 | 7 |
| GBMLGG | TCGA-32-1986-01 | 64 | 3 |
| GBMLGG | TCGA-32-1991-01 | 88 | 6 |
| GBMLGG | TCGA-32-2491-01 | 134 | 7 |
| GBMLGG | TCGA-32-2494-01 | 101 | 6 |
| GBMLGG | TCGA-32-2495-01 | 120 | 9 |
| GBMLGG | TCGA-32-2615-01 | 56 | 1 |
| GBMLGG | TCGA-32-2632-01 | 126 | 8 |
| GBMLGG | TCGA-32-2634-01 | 63 | 5 |
| GBMLGG | TCGA-32-2638-01 | 78 | 2 |
| GBMLGG | TCGA-32-4208-01 | 87 | 5 |
| GBMLGG | TCGA-32-4209-01 | 54 | 2 |
| GBMLGG | TCGA-32-4210-01 | 126 | 5 |
| GBMLGG | TCGA-32-4211-01 | 116 | 4 |
| GBMLGG | TCGA-32-4213-01 | 92 | 3 |
| GBMLGG | TCGA-32-4719-01 | 61 | 5 |
| GBMLGG | TCGA-32-5222-01 | 104 | 5 |
| GBMLGG | TCGA-41-2571-01 | 68 | 4 |
| GBMLGG | TCGA-41-2572-01 | 90 | 6 |
| GBMLGG | TCGA-41-2573-01 | 58 | 3 |
| GBMLGG | TCGA-41-2575-01 | 84 | 4 |
| GBMLGG | TCGA-41-3392-01 | 91 | 5 |
| GBMLGG | TCGA-41-3393-01 | 103 | 4 |
| GBMLGG | TCGA-41-3915-01 | 75 | 5 |
| GBMLGG | TCGA-41-4097-01 | 89 | 5 |
| GBMLGG | TCGA-41-5651-01 | 113 | 5 |
| GBMLGG | TCGA-41-6646-01 | 62 | 3 |
| GBMLGG | TCGA-74-6573-01 | 60 | 6 |
| GBMLGG | TCGA-74-6575-01 | 128 | 7 |
| GBMLGG | TCGA-74-6577-01 | 51 | 4 |
| GBMLGG | TCGA-74-6578-01 | 94 | 4 |
| GBMLGG | TCGA-74-6584-01 | 48 | 1 |
| GBMLGG | TCGA-76-4925-01 | 82 | 7 |
| GBMLGG | TCGA-76-4926-01 | 74 | 1 |
| GBMLGG | TCGA-76-4927-01 | 73 | 4 |
| GBMLGG | TCGA-76-4928-01 | 108 | 7 |
| GBMLGG | TCGA-76-4929-01 | 77 | 6 |
| GBMLGG | TCGA-76-4931-01 | 63 | 5 |
| GBMLGG | TCGA-76-4932-01 | 84 | 0 |
| GBMLGG | TCGA-76-4934-01 | 74 | 4 |
| GBMLGG | TCGA-76-4935-01 | 78 | 3 |
| GBMLGG | TCGA-76-6191-01 | 69 | 6 |
| GBMLGG | TCGA-76-6192-01 | 72 | 2 |
| GBMLGG | TCGA-76-6193-01 | 58 | 5 |
| GBMLGG | TCGA-76-6280-01 | 75 | 2 |
| GBMLGG | TCGA-76-6282-01 | 65 | 3 |
| GBMLGG | TCGA-76-6283-01 | 119 | 5 |
| GBMLGG | TCGA-76-6285-01 | 74 | 1 |
| GBMLGG | TCGA-76-6286-01 | 90 | 7 |
| GBMLGG | TCGA-76-6656-01 | 121 | 6 |
| GBMLGG | TCGA-76-6657-01 | 74 | 10 |
| GBMLGG | TCGA-76-6660-01 | 107 | 7 |
| GBMLGG | TCGA-76-6661-01 | 69 | 6 |
| GBMLGG | TCGA-76-6662-01 | 54 | 6 |
| GBMLGG | TCGA-76-6663-01 | 76 | 9 |
| GBMLGG | TCGA-76-6664-01 | 67 | 2 |
| GBMLGG | TCGA-81-5910-01 | 63 | 4 |
| GBMLGG | TCGA-81-5911-01 | 43 | 4 |
| GBMLGG | TCGA-87-5896-01 | 70 | 2 |
| GBMLGG | TCGA-CS-4938-01 | 18 | 3 |
| GBMLGG | TCGA-CS-4941-01 | 51 | 2 |
| GBMLGG | TCGA-CS-4942-01 | 25 | 6 |
| GBMLGG | TCGA-CS-4943-01 | 31 | 7 |

# TABLE 5 (APPENDIX A)

| | | | | | | | | | | | | |
|---|---|---|---|---|---|---|---|---|---|---|---|
| GBMLGG | TCGA-CS-4944-01 | 22 | 3 | GBMLGG | TCGA-DU-7294-01 | 31 | 5 | GBMLGG | TCGA-HT-7606-01 | 34 | 3 |
| GBMLGG | TCGA-CS-5390-01 | 36 | 5 | GBMLGG | TCGA-DU-7298-01 | 34 | 7 | GBMLGG | TCGA-HT-7607-01 | 30 | 3 |
| GBMLGG | TCGA-CS-5393-01 | 28 | 4 | GBMLGG | TCGA-DU-7299-01 | 29 | 4 | GBMLGG | TCGA-HT-7608-01 | 21 | 5 |
| GBMLGG | TCGA-CS-5394-01 | 25 | 6 | GBMLGG | TCGA-DU-7300-01 | 53 | 6 | GBMLGG | TCGA-HT-7609-01 | 29 | 3 |
| GBMLGG | TCGA-CS-5395-01 | 40 | 3 | GBMLGG | TCGA-DU-7301-01 | 30 | 5 | GBMLGG | TCGA-HT-7610-01 | 17 | 7 |
| GBMLGG | TCGA-CS-5396-01 | 30 | 6 | GBMLGG | TCGA-DU-7302-01 | 32 | 4 | GBMLGG | TCGA-HT-7611-01 | 33 | 7 |
| GBMLGG | TCGA-CS-5397-01 | 46 | 7 | GBMLGG | TCGA-DU-7304-01 | 30 | 6 | GBMLGG | TCGA-HT-7616-01 | 48 | 4 |
| GBMLGG | TCGA-CS-6186-01 | 59 | 0 | GBMLGG | TCGA-DU-7306-01 | 56 | 3 | GBMLGG | TCGA-HT-7620-01 | 21 | 3 |
| GBMLGG | TCGA-CS-6188-01 | 49 | 2 | GBMLGG | TCGA-DU-7309-01 | 33 | 4 | GBMLGG | TCGA-HT-7676-01 | 17 | 3 |
| GBMLGG | TCGA-CS-6290-01 | 20 | 2 | GBMLGG | TCGA-DU-8158-01 | 39 | 5 | GBMLGG | TCGA-HT-7677-01 | 37 | 4 |
| GBMLGG | TCGA-CS-6665-01 | 79 | 2 | GBMLGG | TCGA-DU-8161-01 | 46 | 4 | GBMLGG | TCGA-HT-7680-01 | 3 | 0 |
| GBMLGG | TCGA-CS-6666-01 | 28 | 5 | GBMLGG | TCGA-DU-8162-01 | 28 | 1 | GBMLGG | TCGA-HT-7681-01 | 15 | 3 |
| GBMLGG | TCGA-CS-6667-01 | 27 | 5 | GBMLGG | TCGA-DU-8163-01 | 17 | 5 | GBMLGG | TCGA-HT-7684-01 | 34 | 3 |
| GBMLGG | TCGA-CS-6668-01 | 24 | 4 | GBMLGG | TCGA-DU-8164-01 | 31 | 5 | GBMLGG | TCGA-HT-7686-01 | 19 | 3 |
| GBMLGG | TCGA-DB-5273-01 | 17 | 3 | GBMLGG | TCGA-DU-8165-01 | 72 | 4 | GBMLGG | TCGA-HT-7687-01 | 42 | 1 |
| GBMLGG | TCGA-DB-5274-01 | 44 | 3 | GBMLGG | TCGA-DU-8166-01 | 31 | 5 | GBMLGG | TCGA-HT-7688-01 | 74 | 8 |
| GBMLGG | TCGA-DB-5275-01 | 32 | 5 | GBMLGG | TCGA-DU-8167-01 | 60 | 5 | GBMLGG | TCGA-HT-7689-01 | 52 | 4 |
| GBMLGG | TCGA-DB-5276-01 | 16 | 3 | GBMLGG | TCGA-DU-8168-01 | 65 | 9 | GBMLGG | TCGA-HT-7690-01 | 20 | 6 |
| GBMLGG | TCGA-DB-5277-01 | 43 | 3 | GBMLGG | TCGA-DU-A5TP-01 | 29 | 3 | GBMLGG | TCGA-HT-7691-01 | 9 | 0 |
| GBMLGG | TCGA-DB-5278-01 | 7 | 3 | GBMLGG | TCGA-DU-A5TR-01 | 36 | 5 | GBMLGG | TCGA-HT-7692-01 | 24 | 3 |
| GBMLGG | TCGA-DB-5279-01 | 55 | 3 | GBMLGG | TCGA-DU-A5TS-01 | 41 | 4 | GBMLGG | TCGA-HT-7693-01 | 31 | 5 |
| GBMLGG | TCGA-DB-5280-01 | 22 | 5 | GBMLGG | TCGA-DU-A5TT-01 | 57 | 5 | GBMLGG | TCGA-HT-7694-01 | 35 | 4 |
| GBMLGG | TCGA-DB-5281-01 | 53 | 3 | GBMLGG | TCGA-DU-A5TU-01 | 43 | 5 | GBMLGG | TCGA-HT-7695-01 | 25 | 2 |
| GBMLGG | TCGA-DB-A4X9-01 | 27 | 6 | GBMLGG | TCGA-DU-A5TW-01 | 48 | 3 | GBMLGG | TCGA-HT-7854-01 | 29 | 2 |
| GBMLGG | TCGA-DB-A4XA-01 | 9 | 4 | GBMLGG | TCGA-DU-A5TY-01 | 55 | 2 | GBMLGG | TCGA-HT-7855-01 | 36 | 4 |
| GBMLGG | TCGA-DB-A4XB-01 | 31 | 5 | GBMLGG | TCGA-E1-5302-01 | 35 | 3 | GBMLGG | TCGA-HT-7856-01 | 11 | 2 |
| GBMLGG | TCGA-DB-A4XC-01 | 17 | 3 | GBMLGG | TCGA-E1-5303-01 | 28 | 4 | GBMLGG | TCGA-HT-7857-01 | 20 | 4 |
| GBMLGG | TCGA-DB-A4XD-01 | 34 | 7 | GBMLGG | TCGA-E1-5304-01 | 33 | 4 | GBMLGG | TCGA-HT-7858-01 | 17 | 3 |
| GBMLGG | TCGA-DB-A4XE-01 | 28 | 6 | GBMLGG | TCGA-E1-5305-01 | 25 | 3 | GBMLGG | TCGA-HT-7860-01 | 86 | 9 |
| GBMLGG | TCGA-DB-A4XF-01 | 23 | 2 | GBMLGG | TCGA-E1-5307-01 | 69 | 5 | GBMLGG | TCGA-HT-7873-01 | 29 | 3 |
| GBMLGG | TCGA-DB-A4XG-01 | 23 | 3 | GBMLGG | TCGA-E1-5311-01 | 17 | 3 | GBMLGG | TCGA-HT-7874-01 | 24 | 3 |
| GBMLGG | TCGA-DB-A4XH-01 | 44 | 3 | GBMLGG | TCGA-E1-5318-01 | 36 | 5 | GBMLGG | TCGA-HT-7875-01 | 36 | 5 |
| GBMLGG | TCGA-DB-A64L-01 | 77 | 8 | GBMLGG | TCGA-E1-5319-01 | 38 | 4 | GBMLGG | TCGA-HT-7877-01 | 15 | 2 |
| GBMLGG | TCGA-DB-A64O-01 | 21 | 3 | GBMLGG | TCGA-E1-5322-01 | 26 | 4 | GBMLGG | TCGA-HT-7879-01 | 17 | 4 |
| GBMLGG | TCGA-DB-A64P-01 | 24 | 3 | GBMLGG | TCGA-EZ-7264-01 | 28 | 3 | GBMLGG | TCGA-HT-7880-01 | 8 | 5 |
| GBMLGG | TCGA-DB-A64Q-01 | 20 | 4 | GBMLGG | TCGA-FG-5962-01 | 34 | 6 | GBMLGG | TCGA-HT-7881-01 | 15 | 1 |
| GBMLGG | TCGA-DB-A64R-01 | 14 | 2 | GBMLGG | TCGA-FG-5963-01 | 26 | 5 | GBMLGG | TCGA-HT-7882-01 | 50 | 3 |
| GBMLGG | TCGA-DB-A64S-01 | 11 | 5 | GBMLGG | TCGA-FG-5964-01 | 34 | 2 | GBMLGG | TCGA-HT-7884-01 | 34 | 5 |
| GBMLGG | TCGA-DB-A64U-01 | 12 | 2 | GBMLGG | TCGA-FG-5965-01 | 45 | 4 | GBMLGG | TCGA-HT-7902-01 | 20 | 4 |
| GBMLGG | TCGA-DB-A64V-01 | 27 | 5 | GBMLGG | TCGA-FG-6688-01 | 73 | 3 | GBMLGG | TCGA-HT-8010-01 | 14 | 2 |
| GBMLGG | TCGA-DB-A64W-01 | 49 | 5 | GBMLGG | TCGA-FG-6689-01 | 21 | 6 | GBMLGG | TCGA-HT-8011-01 | 51 | 4 |
| GBMLGG | TCGA-DB-A64X-01 | 76 | 7 | GBMLGG | TCGA-FG-6690-01 | 28 | 5 | GBMLGG | TCGA-HT-8012-01 | 23 | 6 |
| GBMLGG | TCGA-DH-5140-01 | 29 | 5 | GBMLGG | TCGA-FG-6691-01 | 12 | 3 | GBMLGG | TCGA-HT-8013-01 | 28 | 4 |
| GBMLGG | TCGA-DH-5141-01 | 26 | 4 | GBMLGG | TCGA-FG-6692-01 | 88 | 5 | GBMLGG | TCGA-HT-8015-01 | 1 | 1 |
| GBMLGG | TCGA-DH-5142-01 | 35 | 4 | GBMLGG | TCGA-FG-7634-01 | 21 | 4 | GBMLGG | TCGA-HT-8018-01 | 16 | 4 |
| GBMLGG | TCGA-DH-5143-01 | 28 | 3 | GBMLGG | TCGA-FG-7636-01 | 40 | 5 | GBMLGG | TCGA-HT-8019-01 | 1 | 0 |
| GBMLGG | TCGA-DH-5144-01 | 36 | 6 | GBMLGG | TCGA-FG-7637-01 | 33 | 3 | GBMLGG | TCGA-HT-8104-01 | 66 | 7 |
| GBMLGG | TCGA-DH-A668-01 | 45 | 5 | GBMLGG | TCGA-FG-7638-01 | 17 | 5 | GBMLGG | TCGA-HT-8105-01 | 49 | 6 |
| GBMLGG | TCGA-DH-A66F-01 | 18 | 3 | GBMLGG | TCGA-FG-7641-01 | 27 | 5 | GBMLGG | TCGA-HT-8106-01 | 33 | 2 |
| GBMLGG | TCGA-DU-5847-01 | 40 | 5 | GBMLGG | TCGA-FG-7643-01 | 55 | 3 | GBMLGG | TCGA-HT-8108-01 | 20 | 3 |
| GBMLGG | TCGA-DU-5849-01 | 35 | 5 | GBMLGG | TCGA-FG-8182-01 | 31 | 6 | GBMLGG | TCGA-HT-8109-01 | 40 | 4 |
| GBMLGG | TCGA-DU-5851-01 | 28 | 3 | GBMLGG | TCGA-FG-8185-01 | 41 | 6 | GBMLGG | TCGA-HT-8110-01 | 36 | 3 |
| GBMLGG | TCGA-DU-5852-01 | 76 | 6 | GBMLGG | TCGA-FG-8186-01 | 30 | 2 | GBMLGG | TCGA-HT-8111-01 | 11 | 2 |
| GBMLGG | TCGA-DU-5853-01 | 18 | 4 | GBMLGG | TCGA-FG-8187-01 | 14 | 2 | GBMLGG | TCGA-HT-8113-01 | 22 | 1 |
| GBMLGG | TCGA-DU-5854-01 | 51 | 1 | GBMLGG | TCGA-FG-8188-01 | 33 | 7 | GBMLGG | TCGA-HT-8114-01 | 16 | 5 |
| GBMLGG | TCGA-DU-5855-01 | 53 | 5 | GBMLGG | TCGA-FG-8189-01 | 3 | 1 | GBMLGG | TCGA-HT-8558-01 | 1 | 0 |
| GBMLGG | TCGA-DU-5870-01 | 18 | 3 | GBMLGG | TCGA-FG-8191-01 | 27 | 5 | GBMLGG | TCGA-HT-8563-01 | 31 | 5 |
| GBMLGG | TCGA-DU-5871-01 | 27 | 4 | GBMLGG | TCGA-FG-A4MT-01 | 19 | 6 | GBMLGG | TCGA-HT-8564-01 | 597 | 22 |
| GBMLGG | TCGA-DU-5872-01 | 30 | 4 | GBMLGG | TCGA-FG-A4MU-01 | 77 | 2 | GBMLGG | TCGA-HT-A4DS-01 | 40 | 3 |
| GBMLGG | TCGA-DU-5874-01 | 43 | 4 | GBMLGG | TCGA-FG-A4MW-01 | 88 | 7 | GBMLGG | TCGA-HT-A4DV-01 | 12 | 2 |
| GBMLGG | TCGA-DU-6393-01 | 32 | 3 | GBMLGG | TCGA-FG-A4MX-01 | 21 | 3 | GBMLGG | TCGA-HT-A5R5-01 | 26 | 5 |
| GBMLGG | TCGA-DU-6394-01 | 39 | 10 | GBMLGG | TCGA-FG-A4MY-01 | 28 | 5 | GBMLGG | TCGA-HT-A5R7-01 | 21 | 3 |
| GBMLGG | TCGA-DU-6395-01 | 26 | 5 | GBMLGG | TCGA-FG-A60J-01 | 38 | 3 | GBMLGG | TCGA-HT-A5R9-01 | 38 | 3 |
| GBMLGG | TCGA-DU-6396-01 | 43 | 4 | GBMLGG | TCGA-FG-A60K-01 | 19 | 4 | GBMLGG | TCGA-HT-A5RA-01 | 61 | 3 |
| GBMLGG | TCGA-DU-6397-01 | 27 | 6 | GBMLGG | TCGA-FN-7833-01 | 20 | 5 | GBMLGG | TCGA-HT-A5RB-01 | 19 | 4 |
| GBMLGG | TCGA-DU-6399-01 | 51 | 6 | GBMLGG | TCGA-HT-7467-01 | 28 | 3 | GBMLGG | TCGA-HT-A5RC-01 | 59 | 1 |
| GBMLGG | TCGA-DU-6400-01 | 51 | 4 | GBMLGG | TCGA-HT-7468-01 | 15 | 5 | GBMLGG | TCGA-HT-A614-01 | 28 | 4 |
| GBMLGG | TCGA-DU-6401-01 | 24 | 5 | GBMLGG | TCGA-HT-7469-01 | 39 | 7 | GBMLGG | TCGA-HT-A615-01 | 37 | 6 |
| GBMLGG | TCGA-DU-6402-01 | 49 | 5 | GBMLGG | TCGA-HT-7470-01 | 43 | 6 | GBMLGG | TCGA-HT-A616-01 | 23 | 2 |
| GBMLGG | TCGA-DU-6403-01 | 22 | 4 | GBMLGG | TCGA-HT-7471-01 | 21 | 4 | GBMLGG | TCGA-HT-A617-01 | 22 | 1 |
| GBMLGG | TCGA-DU-6404-01 | 14 | 0 | GBMLGG | TCGA-HT-7472-01 | 21 | 4 | GBMLGG | TCGA-HT-A618-01 | 24 | 5 |
| GBMLGG | TCGA-DU-6405-01 | 58 | 2 | GBMLGG | TCGA-HT-7473-01 | 17 | 3 | GBMLGG | TCGA-HT-A619-01 | 60 | 4 |
| GBMLGG | TCGA-DU-6407-01 | 23 | 6 | GBMLGG | TCGA-HT-7474-01 | 23 | 3 | GBMLGG | TCGA-HT-A61A-01 | 8 | 2 |
| GBMLGG | TCGA-DU-6408-01 | 21 | 6 | GBMLGG | TCGA-HT-7475-01 | 54 | 7 | GBMLGG | TCGA-HT-A61B-01 | 42 | 3 |
| GBMLGG | TCGA-DU-6410-01 | 44 | 3 | GBMLGG | TCGA-HT-7476-01 | 18 | 3 | GBMLGG | TCGA-HT-A61C-01 | 44 | 3 |
| GBMLGG | TCGA-DU-6542-01 | 25 | 3 | GBMLGG | TCGA-HT-7477-01 | 47 | 5 | GBMLGG | TCGA-HW-7486-01 | 13 | 2 |
| GBMLGG | TCGA-DU-7006-01 | 60 | 5 | GBMLGG | TCGA-HT-7478-01 | 27 | 6 | GBMLGG | TCGA-HW-7487-01 | 21 | 4 |
| GBMLGG | TCGA-DU-7007-01 | 39 | 6 | GBMLGG | TCGA-HT-7479-01 | 22 | 2 | GBMLGG | TCGA-HW-7489-01 | 19 | 3 |
| GBMLGG | TCGA-DU-7008-01 | 31 | 7 | GBMLGG | TCGA-HT-7480-01 | 27 | 2 | GBMLGG | TCGA-HW-7490-01 | 45 | 5 |
| GBMLGG | TCGA-DU-7009-01 | 16 | 2 | GBMLGG | TCGA-HT-7481-01 | 29 | 8 | GBMLGG | TCGA-HW-7491-01 | 16 | 3 |
| GBMLGG | TCGA-DU-7010-01 | 105 | 7 | GBMLGG | TCGA-HT-7482-01 | 16 | 5 | GBMLGG | TCGA-HW-7495-01 | 15 | 1 |
| GBMLGG | TCGA-DU-7012-01 | 61 | 3 | GBMLGG | TCGA-HT-7483-01 | 16 | 3 | GBMLGG | TCGA-HW-8319-01 | 36 | 4 |
| GBMLGG | TCGA-DU-7013-01 | 40 | 2 | GBMLGG | TCGA-HT-7485-01 | 14 | 4 | GBMLGG | TCGA-HW-8320-01 | 30 | 2 |
| GBMLGG | TCGA-DU-7015-01 | 31 | 3 | GBMLGG | TCGA-HT-7601-01 | 23 | 3 | GBMLGG | TCGA-HW-8321-01 | 32 | 4 |
| GBMLGG | TCGA-DU-7018-01 | 38 | 6 | GBMLGG | TCGA-HT-7602-01 | 10 | 3 | GBMLGG | TCGA-HW-8322-01 | 21 | 4 |
| GBMLGG | TCGA-DU-7019-01 | 34 | 3 | GBMLGG | TCGA-HT-7603-01 | 29 | 3 | GBMLGG | TCGA-HW-A5KJ-01 | 46 | 5 |
| GBMLGG | TCGA-DU-7290-01 | 36 | 5 | GBMLGG | TCGA-HT-7604-01 | 50 | 6 | GBMLGG | TCGA-HW-A5KK-01 | 34 | 1 |
| GBMLGG | TCGA-DU-7292-01 | 47 | 1 | GBMLGG | TCGA-HT-7605-01 | 26 | 2 | GBMLGG | TCGA-HW-A5KL-01 | 18 | 4 |

# TABLE 5 (APPENDIX A)

| | | | | | | | | | | | |
|---|---|---|---|---|---|---|---|---|---|---|---|
| GBMLGG | TCGA-HW-A5KM-01 | 16 | 2 | HNSC | TCGA-CN-6989-01 | 124 | 10 | HNSC | TCGA-CV-5442-01 | 306 | 15 |
| GBMLGG | TCGA-IK-7675-01 | 44 | 4 | HNSC | TCGA-CN-6992-01 | 392 | 14 | HNSC | TCGA-CV-5443-01 | 68 | 6 |
| GBMLGG | TCGA-IK-8125-01 | 55 | 9 | HNSC | TCGA-CN-6994-01 | 140 | 12 | HNSC | TCGA-CV-5444-01 | 155 | 13 |
| GBMLGG | TCGA-P5-A5ET-01 | 23 | 3 | HNSC | TCGA-CN-6995-01 | 258 | 14 | HNSC | TCGA-CV-5966-01 | 125 | 6 |
| GBMLGG | TCGA-P5-A5EU-01 | 27 | 4 | HNSC | TCGA-CN-6997-01 | 161 | 8 | HNSC | TCGA-CV-5970-01 | 231 | 11 |
| GBMLGG | TCGA-P5-A5EV-01 | 84 | 4 | HNSC | TCGA-CN-6998-01 | 101 | 7 | HNSC | TCGA-CV-5971-01 | 34 | 2 |
| GBMLGG | TCGA-P5-A5EW-01 | 16 | 3 | HNSC | TCGA-CQ-5323-01 | 83 | 1 | HNSC | TCGA-CV-5973-01 | 74 | 6 |
| GBMLGG | TCGA-P5-A5EX-01 | 25 | 2 | HNSC | TCGA-CQ-5324-01 | 82 | 10 | HNSC | TCGA-CV-5976-01 | 92 | 8 |
| GBMLGG | TCGA-P5-A5EY-01 | 1 | 0 | HNSC | TCGA-CQ-5325-01 | 63 | 4 | HNSC | TCGA-CV-5977-01 | 108 | 7 |
| GBMLGG | TCGA-P5-A5EZ-01 | 36 | 2 | HNSC | TCGA-CQ-5326-01 | 260 | 11 | HNSC | TCGA-CV-5978-01 | 200 | 13 |
| GBMLGG | TCGA-P5-A5F0-01 | 30 | 6 | HNSC | TCGA-CQ-5329-01 | 40 | 3 | HNSC | TCGA-CV-5979-01 | 44 | 1 |
| GBMLGG | TCGA-P5-A5F1-01 | 18 | 4 | HNSC | TCGA-CQ-5330-01 | 81 | 3 | HNSC | TCGA-CV-6003-01 | 115 | 11 |
| GBMLGG | TCGA-P5-A5F2-01 | 26 | 5 | HNSC | TCGA-CQ-5331-01 | 173 | 9 | HNSC | TCGA-CV-6433-01 | 71 | 2 |
| GBMLGG | TCGA-P5-A5F4-01 | 35 | 4 | HNSC | TCGA-CQ-5332-01 | 162 | 6 | HNSC | TCGA-CV-6436-01 | 91 | 9 |
| GBMLGG | TCGA-P5-A5F6-01 | 1 | 0 | HNSC | TCGA-CQ-5334-01 | 213 | 13 | HNSC | TCGA-CV-6441-01 | 259 | 15 |
| GBMLGG | TCGA-QH-A65S-01 | 22 | 3 | HNSC | TCGA-CQ-6218-01 | 160 | 9 | HNSC | TCGA-CV-6933-01 | 138 | 9 |
| GBMLGG | TCGA-QH-A65V-01 | 24 | 3 | HNSC | TCGA-CQ-6220-01 | 134 | 10 | HNSC | TCGA-CV-6934-01 | 108 | 4 |
| GBMLGG | TCGA-QH-A65Z-01 | 29 | 4 | HNSC | TCGA-CQ-6221-01 | 136 | 9 | HNSC | TCGA-CV-6935-01 | 253 | 14 |
| HNSC | TCGA-BA-4074-01 | 175 | 11 | HNSC | TCGA-CQ-6223-01 | 106 | 8 | HNSC | TCGA-CV-6936-01 | 319 | 16 |
| HNSC | TCGA-BA-4076-01 | 380 | 7 | HNSC | TCGA-CQ-6224-01 | 87 | 4 | HNSC | TCGA-CV-6937-01 | 156 | 11 |
| HNSC | TCGA-BA-4077-01 | 377 | 14 | HNSC | TCGA-CQ-6225-01 | 179 | 6 | HNSC | TCGA-CV-6938-01 | 177 | 18 |
| HNSC | TCGA-BA-4078-01 | 502 | 19 | HNSC | TCGA-CQ-6227-01 | 49 | 3 | HNSC | TCGA-CV-6939-01 | 55 | 3 |
| HNSC | TCGA-BA-5149-01 | 143 | 9 | HNSC | TCGA-CQ-6228-01 | 123 | 5 | HNSC | TCGA-CV-6940-01 | 157 | 5 |
| HNSC | TCGA-BA-5151-01 | 104 | 10 | HNSC | TCGA-CQ-6229-01 | 36 | 3 | HNSC | TCGA-CV-6941-01 | 169 | 11 |
| HNSC | TCGA-BA-5152-01 | 603 | 18 | HNSC | TCGA-CQ-7065-01 | 39 | 4 | HNSC | TCGA-CV-6942-01 | 265 | 13 |
| HNSC | TCGA-BA-5153-01 | 62 | 1 | HNSC | TCGA-CQ-7068-01 | 126 | 7 | HNSC | TCGA-CV-6943-01 | 72 | 8 |
| HNSC | TCGA-BA-5555-01 | 157 | 9 | HNSC | TCGA-CR-5243-01 | 85 | 4 | HNSC | TCGA-CV-6945-01 | 159 | 6 |
| HNSC | TCGA-BA-5556-01 | 163 | 12 | HNSC | TCGA-CR-5247-01 | 90 | 4 | HNSC | TCGA-CV-6948-01 | 235 | 13 |
| HNSC | TCGA-BA-5557-01 | 57 | 4 | HNSC | TCGA-CR-5248-01 | 393 | 9 | HNSC | TCGA-CV-6950-01 | 70 | 3 |
| HNSC | TCGA-BA-5558-01 | 186 | 8 | HNSC | TCGA-CR-5249-01 | 54 | 1 | HNSC | TCGA-CV-6951-01 | 154 | 12 |
| HNSC | TCGA-BA-5559-01 | 95 | 5 | HNSC | TCGA-CR-5250-01 | 41 | 2 | HNSC | TCGA-CV-6952-01 | 193 | 10 |
| HNSC | TCGA-BA-6868-01 | 185 | 15 | HNSC | TCGA-CR-6467-01 | 58 | 3 | HNSC | TCGA-CV-6953-01 | 122 | 8 |
| HNSC | TCGA-BA-6869-01 | 539 | 19 | HNSC | TCGA-CR-6470-01 | 50 | 4 | HNSC | TCGA-CV-6954-01 | 187 | 9 |
| HNSC | TCGA-BA-6870-01 | 111 | 11 | HNSC | TCGA-CR-6471-01 | 159 | 11 | HNSC | TCGA-CV-6955-01 | 155 | 12 |
| HNSC | TCGA-BA-6871-01 | 166 | 13 | HNSC | TCGA-CR-6472-01 | 545 | 15 | HNSC | TCGA-CV-6956-01 | 214 | 12 |
| HNSC | TCGA-BA-6872-01 | 195 | 11 | HNSC | TCGA-CR-6473-01 | 112 | 1 | HNSC | TCGA-CV-6959-01 | 76 | 5 |
| HNSC | TCGA-BA-6873-01 | 82 | 8 | HNSC | TCGA-CR-6474-01 | 128 | 4 | HNSC | TCGA-CV-6960-01 | 204 | 6 |
| HNSC | TCGA-BA-7269-01 | 111 | 11 | HNSC | TCGA-CR-6477-01 | 105 | 6 | HNSC | TCGA-CV-6961-01 | 654 | 23 |
| HNSC | TCGA-BB-4217-01 | 34 | 2 | HNSC | TCGA-CR-6478-01 | 151 | 7 | HNSC | TCGA-CV-6962-01 | 168 | 12 |
| HNSC | TCGA-BB-4223-01 | 347 | 8 | HNSC | TCGA-CR-6481-01 | 560 | 21 | HNSC | TCGA-CV-7089-01 | 193 | 11 |
| HNSC | TCGA-BB-4224-01 | 92 | 3 | HNSC | TCGA-CR-6482-01 | 60 | 1 | HNSC | TCGA-CV-7090-01 | 59 | 5 |
| HNSC | TCGA-BB-4225-01 | 96 | 3 | HNSC | TCGA-CR-6484-01 | 323 | 18 | HNSC | TCGA-CV-7091-01 | 155 | 4 |
| HNSC | TCGA-BB-4228-01 | 50 | 2 | HNSC | TCGA-CR-6487-01 | 150 | 9 | HNSC | TCGA-CV-7095-01 | 278 | 15 |
| HNSC | TCGA-CN-4723-01 | 1040 | 34 | HNSC | TCGA-CR-6488-01 | 44 | 0 | HNSC | TCGA-CV-7097-01 | 107 | 6 |
| HNSC | TCGA-CN-4725-01 | 73 | 4 | HNSC | TCGA-CR-6491-01 | 168 | 7 | HNSC | TCGA-CV-7099-01 | 348 | 23 |
| HNSC | TCGA-CN-4726-01 | 98 | 5 | HNSC | TCGA-CR-6492-01 | 78 | 7 | HNSC | TCGA-CV-7100-01 | 21 | 2 |
| HNSC | TCGA-CN-4727-01 | 478 | 21 | HNSC | TCGA-CR-6493-01 | 46 | 3 | HNSC | TCGA-CV-7101-01 | 227 | 8 |
| HNSC | TCGA-CN-4728-01 | 163 | 8 | HNSC | TCGA-CR-7364-01 | 518 | 22 | HNSC | TCGA-CV-7102-01 | 166 | 10 |
| HNSC | TCGA-CN-4729-01 | 173 | 13 | HNSC | TCGA-CR-7365-01 | 196 | 8 | HNSC | TCGA-CV-7103-01 | 54 | 5 |
| HNSC | TCGA-CN-4730-01 | 196 | 7 | HNSC | TCGA-CR-7367-01 | 201 | 10 | HNSC | TCGA-CV-7104-01 | 126 | 4 |
| HNSC | TCGA-CN-4731-01 | 141 | 6 | HNSC | TCGA-CR-7368-01 | 240 | 10 | HNSC | TCGA-CV-7177-01 | 134 | 8 |
| HNSC | TCGA-CN-4733-01 | 47 | 3 | HNSC | TCGA-CR-7369-01 | 87 | 2 | HNSC | TCGA-CV-7178-01 | 140 | 8 |
| HNSC | TCGA-CN-4735-01 | 177 | 3 | HNSC | TCGA-CR-7370-01 | 612 | 22 | HNSC | TCGA-CV-7180-01 | 77 | 2 |
| HNSC | TCGA-CN-4736-01 | 106 | 7 | HNSC | TCGA-CR-7371-01 | 338 | 10 | HNSC | TCGA-CV-7183-01 | 84 | 5 |
| HNSC | TCGA-CN-4737-01 | 62 | 3 | HNSC | TCGA-CR-7372-01 | 58 | 5 | HNSC | TCGA-CV-7235-01 | 159 | 7 |
| HNSC | TCGA-CN-4738-01 | 134 | 6 | HNSC | TCGA-CR-7373-01 | 138 | 8 | HNSC | TCGA-CV-7236-01 | 67 | 5 |
| HNSC | TCGA-CN-4739-01 | 241 | 17 | HNSC | TCGA-CR-7374-01 | 419 | 19 | HNSC | TCGA-CV-7238-01 | 81 | 5 |
| HNSC | TCGA-CN-4740-01 | 177 | 9 | HNSC | TCGA-CR-7376-01 | 75 | 8 | HNSC | TCGA-CV-7242-01 | 337 | 15 |
| HNSC | TCGA-CN-4741-01 | 199 | 7 | HNSC | TCGA-CR-7377-01 | 151 | 7 | HNSC | TCGA-CV-7243-01 | 86 | 2 |
| HNSC | TCGA-CN-4742-01 | 98 | 8 | HNSC | TCGA-CR-7379-01 | 177 | 10 | HNSC | TCGA-CV-7245-01 | 558 | 21 |
| HNSC | TCGA-CN-5355-01 | 124 | 8 | HNSC | TCGA-CR-7380-01 | 114 | 9 | HNSC | TCGA-CV-7247-01 | 88 | 5 |
| HNSC | TCGA-CN-5356-01 | 412 | 28 | HNSC | TCGA-CR-7382-01 | 101 | 9 | HNSC | TCGA-CV-7248-01 | 328 | 14 |
| HNSC | TCGA-CN-5358-01 | 64 | 6 | HNSC | TCGA-CR-7383-01 | 120 | 9 | HNSC | TCGA-CV-7250-01 | 244 | 20 |
| HNSC | TCGA-CN-5359-01 | 178 | 9 | HNSC | TCGA-CR-7385-01 | 59 | 2 | HNSC | TCGA-CV-7252-01 | 267 | 19 |
| HNSC | TCGA-CN-5360-01 | 569 | 20 | HNSC | TCGA-CR-7386-01 | 224 | 17 | HNSC | TCGA-CV-7253-01 | 224 | 9 |
| HNSC | TCGA-CN-5363-01 | 226 | 8 | HNSC | TCGA-CR-7388-01 | 832 | 32 | HNSC | TCGA-CV-7254-01 | 296 | 16 |
| HNSC | TCGA-CN-5364-01 | 157 | 12 | HNSC | TCGA-CR-7389-01 | 137 | 8 | HNSC | TCGA-CV-7255-01 | 127 | 5 |
| HNSC | TCGA-CN-5365-01 | 104 | 8 | HNSC | TCGA-CR-7390-01 | 159 | 9 | HNSC | TCGA-CV-7261-01 | 136 | 9 |
| HNSC | TCGA-CN-5366-01 | 124 | 7 | HNSC | TCGA-CR-7391-01 | 13 | 1 | HNSC | TCGA-CV-7263-01 | 125 | 8 |
| HNSC | TCGA-CN-5367-01 | 125 | 6 | HNSC | TCGA-CR-7392-01 | 39 | 1 | HNSC | TCGA-CV-7406-01 | 224 | 7 |
| HNSC | TCGA-CN-5369-01 | 636 | 25 | HNSC | TCGA-CR-7393-01 | 4 | 1 | HNSC | TCGA-CV-7407-01 | 151 | 9 |
| HNSC | TCGA-CN-5370-01 | 121 | 8 | HNSC | TCGA-CR-7394-01 | 213 | 21 | HNSC | TCGA-CV-7410-01 | 48 | 4 |
| HNSC | TCGA-CN-5373-01 | 179 | 9 | HNSC | TCGA-CR-7395-01 | 210 | 24 | HNSC | TCGA-CV-7411-01 | 138 | 5 |
| HNSC | TCGA-CN-5374-01 | 209 | 6 | HNSC | TCGA-CR-7397-01 | 55 | 4 | HNSC | TCGA-CV-7413-01 | 80 | 7 |
| HNSC | TCGA-CN-6010-01 | 197 | 5 | HNSC | TCGA-CR-7398-01 | 396 | 18 | HNSC | TCGA-CV-7414-01 | 313 | 10 |
| HNSC | TCGA-CN-6011-01 | 413 | 17 | HNSC | TCGA-CR-7399-01 | 322 | 16 | HNSC | TCGA-CV-7415-01 | 100 | 3 |
| HNSC | TCGA-CN-6012-01 | 140 | 5 | HNSC | TCGA-CR-7401-01 | 109 | 9 | HNSC | TCGA-CV-7416-01 | 89 | 3 |
| HNSC | TCGA-CN-6013-01 | 219 | 13 | HNSC | TCGA-CR-7402-01 | 1073 | 52 | HNSC | TCGA-CV-7418-01 | 189 | 5 |
| HNSC | TCGA-CN-6016-01 | 141 | 3 | HNSC | TCGA-CR-7404-01 | 151 | 5 | HNSC | TCGA-CV-7421-01 | 84 | 5 |
| HNSC | TCGA-CN-6017-01 | 11 | 1 | HNSC | TCGA-CV-5430-01 | 180 | 14 | HNSC | TCGA-CV-7422-01 | 165 | 10 |
| HNSC | TCGA-CN-6018-01 | 126 | 7 | HNSC | TCGA-CV-5431-01 | 106 | 5 | HNSC | TCGA-CV-7423-01 | 105 | 0 |
| HNSC | TCGA-CN-6019-01 | 152 | 7 | HNSC | TCGA-CV-5432-01 | 312 | 15 | HNSC | TCGA-CV-7424-01 | 214 | 15 |
| HNSC | TCGA-CN-6020-01 | 181 | 8 | HNSC | TCGA-CV-5434-01 | 169 | 11 | HNSC | TCGA-CV-7427-01 | 512 | 22 |
| HNSC | TCGA-CN-6021-01 | 219 | 13 | HNSC | TCGA-CV-5435-01 | 135 | 3 | HNSC | TCGA-CV-7429-01 | 65 | 6 |
| HNSC | TCGA-CN-6022-01 | 94 | 5 | HNSC | TCGA-CV-5436-01 | 129 | 7 | HNSC | TCGA-CV-7430-01 | 118 | 3 |
| HNSC | TCGA-CN-6023-01 | 121 | 7 | HNSC | TCGA-CV-5439-01 | 128 | 8 | HNSC | TCGA-CV-7432-01 | 182 | 7 |
| HNSC | TCGA-CN-6024-01 | 204 | 14 | HNSC | TCGA-CV-5440-01 | 136 | 10 | HNSC | TCGA-CV-7433-01 | 136 | 10 |
| HNSC | TCGA-CN-6988-01 | 176 | 10 | HNSC | TCGA-CV-5441-01 | 426 | 17 | HNSC | TCGA-CV-7434-01 | 109 | 9 |

## TABLE 5 (APPENDIX A)

| | | | |
|---|---|---|---|
| HNSC | TCGA-CV-7435-01 | 95 | 4 |
| HNSC | TCGA-CV-7437-01 | 160 | 10 |
| HNSC | TCGA-CV-7438-01 | 131 | 7 |
| HNSC | TCGA-CV-7440-01 | 242 | 16 |
| HNSC | TCGA-CX-7082-01 | 114 | 5 |
| HNSC | TCGA-CX-7085-01 | 177 | 12 |
| HNSC | TCGA-CX-7086-01 | 259 | 5 |
| HNSC | TCGA-CX-7219-01 | 150 | 5 |
| HNSC | TCGA-D6-6515-01 | 68 | 5 |
| HNSC | TCGA-D6-6516-01 | 2131 | 54 |
| HNSC | TCGA-D6-6517-01 | 83 | 4 |
| HNSC | TCGA-D6-6823-01 | 119 | 5 |
| HNSC | TCGA-D6-6824-01 | 74 | 4 |
| HNSC | TCGA-D6-6825-01 | 86 | 6 |
| HNSC | TCGA-D6-6826-01 | 120 | 6 |
| HNSC | TCGA-DQ-5624-01 | 123 | 9 |
| HNSC | TCGA-DQ-5625-01 | 226 | 11 |
| HNSC | TCGA-DQ-5629-01 | 384 | 17 |
| HNSC | TCGA-DQ-5630-01 | 96 | 3 |
| HNSC | TCGA-DQ-5631-01 | 110 | 11 |
| HNSC | TCGA-DQ-7588-01 | 122 | 5 |
| HNSC | TCGA-DQ-7591-01 | 45 | 3 |
| HNSC | TCGA-DQ-7592-01 | 91 | 5 |
| HNSC | TCGA-F7-7848-01 | 367 | 17 |
| HNSC | TCGA-H7-7774-01 | 176 | 14 |
| HNSC | TCGA-HD-7229-01 | 190 | 7 |
| HNSC | TCGA-HD-7753-01 | 109 | 4 |
| HNSC | TCGA-HD-7754-01 | 79 | 5 |
| HNSC | TCGA-HD-7831-01 | 46 | 1 |
| HNSC | TCGA-HD-7832-01 | 117 | 6 |
| HNSC | TCGA-IQ-7630-01 | 152 | 9 |
| HNSC | TCGA-IQ-7631-01 | 40 | 3 |
| HNSC | TCGA-IQ-7632-01 | 63 | 2 |
| KICH | TCGA-KL-8323-01 | 76 | 2 |
| KICH | TCGA-KL-8324-01 | 119 | 6 |
| KICH | TCGA-KL-8325-01 | 81 | 2 |
| KICH | TCGA-KL-8326-01 | 81 | 4 |
| KICH | TCGA-KL-8327-01 | 60 | 3 |
| KICH | TCGA-KL-8328-01 | 101 | 4 |
| KICH | TCGA-KL-8329-01 | 131 | 11 |
| KICH | TCGA-KL-8330-01 | 104 | 2 |
| KICH | TCGA-KL-8331-01 | 83 | 3 |
| KICH | TCGA-KL-8332-01 | 77 | 4 |
| KICH | TCGA-KL-8333-01 | 72 | 3 |
| KICH | TCGA-KL-8334-01 | 82 | 3 |
| KICH | TCGA-KL-8335-01 | 114 | 4 |
| KICH | TCGA-KL-8336-01 | 85 | 3 |
| KICH | TCGA-KL-8337-01 | 84 | 4 |
| KICH | TCGA-KL-8338-01 | 59 | 3 |
| KICH | TCGA-KL-8339-01 | 120 | 5 |
| KICH | TCGA-KL-8340-01 | 61 | 0 |
| KICH | TCGA-KL-8341-01 | 160 | 7 |
| KICH | TCGA-KL-8342-01 | 77 | 7 |
| KICH | TCGA-KL-8343-01 | 95 | 2 |
| KICH | TCGA-KL-8344-01 | 79 | 3 |
| KICH | TCGA-KL-8345-01 | 71 | 5 |
| KICH | TCGA-KL-8346-01 | 93 | 3 |
| KICH | TCGA-KM-8438-01 | 112 | 4 |
| KICH | TCGA-KM-8439-01 | 57 | 2 |
| KICH | TCGA-KM-8440-01 | 79 | 3 |
| KICH | TCGA-KM-8441-01 | 69 | 2 |
| KICH | TCGA-KM-8442-01 | 94 | 1 |
| KICH | TCGA-KM-8443-01 | 77 | 1 |
| KICH | TCGA-KM-8476-01 | 117 | 2 |
| KICH | TCGA-KM-8477-01 | 67 | 2 |
| KICH | TCGA-KM-8639-01 | 72 | 2 |
| KICH | TCGA-KN-8418-01 | 71 | 4 |
| KICH | TCGA-KN-8419-01 | 78 | 3 |
| KICH | TCGA-KN-8421-01 | 72 | 2 |
| KICH | TCGA-KN-8422-01 | 81 | 0 |
| KICH | TCGA-KN-8423-01 | 81 | 3 |
| KICH | TCGA-KN-8424-01 | 107 | 7 |
| KICH | TCGA-KN-8425-01 | 94 | 9 |
| KICH | TCGA-KN-8426-01 | 68 | 3 |
| KICH | TCGA-KN-8427-01 | 143 | 3 |
| KICH | TCGA-KN-8428-01 | 1174 | 43 |
| KICH | TCGA-KN-8429-01 | 90 | 2 |
| KICH | TCGA-KN-8430-01 | 138 | 11 |
| KICH | TCGA-KN-8431-01 | 80 | 3 |
| KICH | TCGA-KN-8432-01 | 147 | 6 |
| KICH | TCGA-KN-8433-01 | 300 | 11 |
| KICH | TCGA-KN-8434-01 | 87 | 4 |
| KICH | TCGA-KN-8435-01 | 105 | 2 |
| KICH | TCGA-KN-8436-01 | 173 | 9 |
| KICH | TCGA-KN-8437-01 | 56 | 5 |
| KICH | TCGA-KO-8403-01 | 63 | 4 |
| KICH | TCGA-KO-8404-01 | 178 | 6 |
| KICH | TCGA-KO-8405-01 | 67 | 5 |
| KICH | TCGA-KO-8406-01 | 105 | 3 |
| KICH | TCGA-KO-8407-01 | 105 | 1 |
| KICH | TCGA-KO-8408-01 | 182 | 10 |
| KICH | TCGA-KO-8409-01 | 113 | 2 |
| KICH | TCGA-KO-8410-01 | 104 | 1 |
| KICH | TCGA-KO-8411-01 | 88 | 4 |
| KICH | TCGA-KO-8413-01 | 74 | 1 |
| KICH | TCGA-KO-8414-01 | 74 | 2 |
| KICH | TCGA-KO-8415-01 | 102 | 4 |
| KICH | TCGA-KO-8416-01 | 91 | 2 |
| KICH | TCGA-KO-8417-01 | 118 | 4 |
| KIPAN | TCGA-A3-3308-01 | 105 | 5 |
| KIPAN | TCGA-A3-3311-01 | 73 | 5 |
| KIPAN | TCGA-A3-3316-01 | 46 | 4 |
| KIPAN | TCGA-A3-3317-01 | 88 | 4 |
| KIPAN | TCGA-A3-3319-01 | 90 | 1 |
| KIPAN | TCGA-A3-3320-01 | 101 | 5 |
| KIPAN | TCGA-A3-3322-01 | 64 | 6 |
| KIPAN | TCGA-A3-3323-01 | 59 | 2 |
| KIPAN | TCGA-A3-3326-01 | 65 | 3 |
| KIPAN | TCGA-A3-3346-01 | 105 | 6 |
| KIPAN | TCGA-A3-3347-01 | 39 | 1 |
| KIPAN | TCGA-A3-3349-01 | 54 | 3 |
| KIPAN | TCGA-A3-3357-01 | 107 | 2 |
| KIPAN | TCGA-A3-3358-01 | 82 | 5 |
| KIPAN | TCGA-A3-3362-01 | 59 | 3 |
| KIPAN | TCGA-A3-3363-01 | 57 | 2 |
| KIPAN | TCGA-A3-3365-01 | 41 | 1 |
| KIPAN | TCGA-A3-3367-01 | 80 | 2 |
| KIPAN | TCGA-A3-3370-01 | 46 | 5 |
| KIPAN | TCGA-A3-3372-01 | 90 | 3 |
| KIPAN | TCGA-A3-3373-01 | 69 | 3 |
| KIPAN | TCGA-A3-3376-01 | 57 | 7 |
| KIPAN | TCGA-A3-3378-01 | 84 | 3 |
| KIPAN | TCGA-A3-3380-01 | 53 | 2 |
| KIPAN | TCGA-A3-3382-01 | 115 | 5 |
| KIPAN | TCGA-A3-3383-01 | 59 | 3 |
| KIPAN | TCGA-A3-3385-01 | 72 | 7 |
| KIPAN | TCGA-A3-3387-01 | 92 | 4 |
| KIPAN | TCGA-A4-7286-01 | 60 | 0 |
| KIPAN | TCGA-A4-7287-01 | 66 | 4 |
| KIPAN | TCGA-A4-7288-01 | 100 | 7 |
| KIPAN | TCGA-A4-7583-01 | 87 | 4 |
| KIPAN | TCGA-A4-7584-01 | 90 | 2 |
| KIPAN | TCGA-A4-7585-01 | 121 | 2 |
| KIPAN | TCGA-A4-7732-01 | 83 | 2 |
| KIPAN | TCGA-A4-7734-01 | 74 | 2 |
| KIPAN | TCGA-A4-7828-01 | 72 | 2 |
| KIPAN | TCGA-A4-7915-01 | 54 | 1 |
| KIPAN | TCGA-A4-7996-01 | 108 | 4 |
| KIPAN | TCGA-A4-7997-01 | 105 | 9 |
| KIPAN | TCGA-A4-8098-01 | 148 | 5 |
| KIPAN | TCGA-A4-8310-01 | 118 | 4 |
| KIPAN | TCGA-A4-8311-01 | 85 | 4 |
| KIPAN | TCGA-A4-8312-01 | 59 | 1 |
| KIPAN | TCGA-A4-8515-01 | 97 | 5 |
| KIPAN | TCGA-A4-8517-01 | 108 | 6 |
| KIPAN | TCGA-A4-8518-01 | 106 | 2 |
| KIPAN | TCGA-A4-8630-01 | 99 | 3 |
| KIPAN | TCGA-A4-A48D-01 | 154 | 4 |
| KIPAN | TCGA-A4-A4ZT-01 | 77 | 1 |
| KIPAN | TCGA-A4-A57E-01 | 120 | 7 |
| KIPAN | TCGA-A4-A5DU-01 | 60 | 2 |
| KIPAN | TCGA-A4-A5XZ-01 | 92 | 4 |
| KIPAN | TCGA-A4-A5Y0-01 | 90 | 7 |
| KIPAN | TCGA-A4-A5Y1-01 | 119 | 5 |
| KIPAN | TCGA-A4-A6HP-01 | 147 | 5 |
| KIPAN | TCGA-AK-3425-01 | 80 | 6 |
| KIPAN | TCGA-AK-3428-01 | 79 | 2 |
| KIPAN | TCGA-AK-3429-01 | 46 | 4 |
| KIPAN | TCGA-AK-3430-01 | 86 | 3 |
| KIPAN | TCGA-AK-3431-01 | 80 | 5 |
| KIPAN | TCGA-AK-3434-01 | 80 | 1 |
| KIPAN | TCGA-AK-3436-01 | 68 | 3 |
| KIPAN | TCGA-AK-3444-01 | 109 | 9 |
| KIPAN | TCGA-AK-3445-01 | 1 | 1 |
| KIPAN | TCGA-AK-3450-01 | 1 | 1 |
| KIPAN | TCGA-AK-3451-01 | 98 | 5 |
| KIPAN | TCGA-AK-3454-01 | 70 | 2 |
| KIPAN | TCGA-AK-3455-01 | 71 | 4 |
| KIPAN | TCGA-AK-3456-01 | 60 | 1 |
| KIPAN | TCGA-AK-3458-01 | 41 | 3 |
| KIPAN | TCGA-AK-3460-01 | 80 | 5 |
| KIPAN | TCGA-AK-3461-01 | 59 | 2 |
| KIPAN | TCGA-AL-3466-01 | 91 | 3 |
| KIPAN | TCGA-AL-3467-01 | 44 | 1 |
| KIPAN | TCGA-AL-3472-01 | 91 | 4 |
| KIPAN | TCGA-AL-3473-01 | 104 | 2 |
| KIPAN | TCGA-AL-7173-01 | 85 | 11 |
| KIPAN | TCGA-A5-3778-01 | 81 | 3 |
| KIPAN | TCGA-AT-A5NU-01 | 115 | 4 |
| KIPAN | TCGA-B0-4690-01 | 1 | 1 |
| KIPAN | TCGA-B0-4691-01 | 109 | 8 |
| KIPAN | TCGA-B0-4693-01 | 106 | 4 |
| KIPAN | TCGA-B0-4694-01 | 83 | 3 |
| KIPAN | TCGA-B0-4697-01 | 52 | 2 |
| KIPAN | TCGA-B0-4700-01 | 34 | 2 |
| KIPAN | TCGA-B0-4703-01 | 1 | 1 |
| KIPAN | TCGA-B0-4706-01 | 61 | 4 |
| KIPAN | TCGA-B0-4707-01 | 1 | 1 |
| KIPAN | TCGA-B0-4710-01 | 99 | 5 |
| KIPAN | TCGA-B0-4712-01 | 125 | 6 |
| KIPAN | TCGA-B0-4713-01 | 1 | 1 |
| KIPAN | TCGA-B0-4714-01 | 1 | 1 |
| KIPAN | TCGA-B0-4718-01 | 79 | 7 |
| KIPAN | TCGA-B0-4810-01 | 74 | 6 |
| KIPAN | TCGA-B0-4811-01 | 132 | 3 |
| KIPAN | TCGA-B0-4813-01 | 67 | 3 |
| KIPAN | TCGA-B0-4814-01 | 73 | 4 |
| KIPAN | TCGA-B0-4815-01 | 81 | 5 |
| KIPAN | TCGA-B0-4816-01 | 65 | 7 |
| KIPAN | TCGA-B0-4817-01 | 67 | 2 |
| KIPAN | TCGA-B0-4818-01 | 81 | 5 |
| KIPAN | TCGA-B0-4819-01 | 45 | 4 |
| KIPAN | TCGA-B0-4822-01 | 1 | 1 |
| KIPAN | TCGA-B0-4823-01 | 125 | 6 |
| KIPAN | TCGA-B0-4824-01 | 1 | 1 |
| KIPAN | TCGA-B0-4827-01 | 107 | 11 |
| KIPAN | TCGA-B0-4828-01 | 106 | 4 |
| KIPAN | TCGA-B0-4833-01 | 1 | 1 |
| KIPAN | TCGA-B0-4836-01 | 29 | 0 |
| KIPAN | TCGA-B0-4837-01 | 48 | 3 |
| KIPAN | TCGA-B0-4838-01 | 53 | 0 |
| KIPAN | TCGA-B0-4839-01 | 73 | 3 |
| KIPAN | TCGA-B0-4841-01 | 111 | 1 |
| KIPAN | TCGA-B0-4842-01 | 62 | 7 |
| KIPAN | TCGA-B0-4843-01 | 81 | 5 |
| KIPAN | TCGA-B0-4844-01 | 53 | 3 |
| KIPAN | TCGA-B0-4845-01 | 1 | 1 |
| KIPAN | TCGA-B0-4846-01 | 47 | 2 |
| KIPAN | TCGA-B0-4847-01 | 49 | 6 |
| KIPAN | TCGA-B0-4848-01 | 1 | 0 |
| KIPAN | TCGA-B0-4849-01 | 26 | 2 |
| KIPAN | TCGA-B0-4852-01 | 89 | 5 |
| KIPAN | TCGA-B0-4945-01 | 44 | 3 |
| KIPAN | TCGA-B0-5075-01 | 106 | 4 |
| KIPAN | TCGA-B0-5077-01 | 64 | 5 |
| KIPAN | TCGA-B0-5080-01 | 69 | 2 |
| KIPAN | TCGA-B0-5081-01 | 49 | 3 |
| KIPAN | TCGA-B0-5085-01 | 79 | 4 |
| KIPAN | TCGA-B0-5088-01 | 75 | 3 |
| KIPAN | TCGA-B0-5092-01 | 78 | 5 |
| KIPAN | TCGA-B0-5094-01 | 94 | 6 |
| KIPAN | TCGA-B0-5095-01 | 87 | 3 |
| KIPAN | TCGA-B0-5096-01 | 108 | 4 |
| KIPAN | TCGA-B0-5097-01 | 68 | 7 |
| KIPAN | TCGA-B0-5099-01 | 84 | 6 |
| KIPAN | TCGA-B0-5100-01 | 41 | 1 |
| KIPAN | TCGA-B0-5102-01 | 58 | 4 |
| KIPAN | TCGA-B0-5104-01 | 73 | 2 |
| KIPAN | TCGA-B0-5106-01 | 96 | 4 |
| KIPAN | TCGA-B0-5108-01 | 65 | 5 |
| KIPAN | TCGA-B0-5108-01 | 63 | 4 |
| KIPAN | TCGA-B0-5109-01 | 61 | 3 |
| KIPAN | TCGA-B0-5110-01 | 79 | 7 |
| KIPAN | TCGA-B0-5113-01 | 43 | 2 |
| KIPAN | TCGA-B0-5115-01 | 64 | 7 |
| KIPAN | TCGA-B0-5116-01 | 67 | 4 |
| KIPAN | TCGA-B0-5119-01 | 102 | 6 |
| KIPAN | TCGA-B0-5120-01 | 67 | 4 |
| KIPAN | TCGA-B0-5121-01 | 59 | 3 |
| KIPAN | TCGA-B0-5399-01 | 56 | 6 |
| KIPAN | TCGA-B0-5400-01 | 49 | 3 |
| KIPAN | TCGA-B0-5402-01 | 62 | 3 |
| KIPAN | TCGA-B0-5691-01 | 75 | 2 |
| KIPAN | TCGA-B0-5692-01 | 87 | 5 |
| KIPAN | TCGA-B0-5693-01 | 49 | 4 |
| KIPAN | TCGA-B0-5694-01 | 72 | 4 |
| KIPAN | TCGA-B0-5695-01 | 77 | 6 |
| KIPAN | TCGA-B0-5696-01 | 77 | 7 |
| KIPAN | TCGA-B0-5697-01 | 79 | 3 |
| KIPAN | TCGA-B0-5698-01 | 83 | 3 |
| KIPAN | TCGA-B0-5699-01 | 55 | 5 |
| KIPAN | TCGA-B0-5701-01 | 121 | 9 |
| KIPAN | TCGA-B0-5702-01 | 75 | 3 |
| KIPAN | TCGA-B0-5703-01 | 99 | 7 |

# TABLE 5 (APPENDIX A)

| | | | | | | | | | | | |
|---|---|---|---|---|---|---|---|---|---|---|---|
| KIPAN | TCGA-B0-5705-01 | 91 | 5 | KIPAN | TCGA-BP-4347-01 | 42 | 0 | KIPAN | TCGA-BP-5192-01 | 61 | 4 |
| KIPAN | TCGA-B0-5706-01 | 68 | 5 | KIPAN | TCGA-BP-4349-01 | 1 | 1 | KIPAN | TCGA-BP-5194-01 | 30 | 2 |
| KIPAN | TCGA-B0-5707-01 | 51 | 3 | KIPAN | TCGA-BP-4351-01 | 73 | 4 | KIPAN | TCGA-BP-5195-01 | 66 | 3 |
| KIPAN | TCGA-B0-5709-01 | 83 | 5 | KIPAN | TCGA-BP-4352-01 | 99 | 3 | KIPAN | TCGA-BP-5196-01 | 64 | 1 |
| KIPAN | TCGA-B0-5710-01 | 43 | 4 | KIPAN | TCGA-BP-4354-01 | 32 | 3 | KIPAN | TCGA-BP-5198-01 | 83 | 6 |
| KIPAN | TCGA-B0-5711-01 | 40 | 3 | KIPAN | TCGA-BP-4355-01 | 1 | 1 | KIPAN | TCGA-BP-5199-01 | 87 | 5 |
| KIPAN | TCGA-B0-5713-01 | 119 | 11 | KIPAN | TCGA-BP-4756-01 | 49 | 1 | KIPAN | TCGA-BP-5200-01 | 51 | 1 |
| KIPAN | TCGA-B0-5812-01 | 51 | 4 | KIPAN | TCGA-BP-4758-01 | 1 | 1 | KIPAN | TCGA-BP-5201-01 | 45 | 2 |
| KIPAN | TCGA-B1-5398-01 | 142 | 6 | KIPAN | TCGA-BP-4759-01 | 1 | 1 | KIPAN | TCGA-BP-5202-01 | 46 | 3 |
| KIPAN | TCGA-B1-A47M-01 | 123 | 4 | KIPAN | TCGA-BP-4761-01 | 89 | 2 | KIPAN | TCGA-BQ-5875-01 | 101 | 7 |
| KIPAN | TCGA-B1-A47N-01 | 63 | 1 | KIPAN | TCGA-BP-4762-01 | 1 | 1 | KIPAN | TCGA-BQ-5876-01 | 118 | 7 |
| KIPAN | TCGA-B1-A654-01 | 95 | 4 | KIPAN | TCGA-BP-4763-01 | 1 | 1 | KIPAN | TCGA-BQ-5877-01 | 114 | 8 |
| KIPAN | TCGA-B1-A655-01 | 128 | 5 | KIPAN | TCGA-BP-4765-01 | 1 | 1 | KIPAN | TCGA-BQ-5878-01 | 136 | 4 |
| KIPAN | TCGA-B1-A656-01 | 172 | 10 | KIPAN | TCGA-BP-4766-01 | 48 | 2 | KIPAN | TCGA-BQ-5879-01 | 44 | 1 |
| KIPAN | TCGA-B1-A657-01 | 116 | 7 | KIPAN | TCGA-BP-4768-01 | 65 | 7 | KIPAN | TCGA-BQ-5880-01 | 83 | 3 |
| KIPAN | TCGA-B2-3924-01 | 26 | 1 | KIPAN | TCGA-BP-4770-01 | 84 | 4 | KIPAN | TCGA-BQ-5881-01 | 62 | 3 |
| KIPAN | TCGA-B2-4098-01 | 41 | 4 | KIPAN | TCGA-BP-4771-01 | 30 | 2 | KIPAN | TCGA-BQ-5882-01 | 99 | 3 |
| KIPAN | TCGA-B2-4099-01 | 24 | 0 | KIPAN | TCGA-BP-4774-01 | 56 | 4 | KIPAN | TCGA-BQ-5883-01 | 57 | 3 |
| KIPAN | TCGA-B2-4101-01 | 40 | 3 | KIPAN | TCGA-BP-4775-01 | 50 | 1 | KIPAN | TCGA-BQ-5884-01 | 82 | 4 |
| KIPAN | TCGA-B2-5633-01 | 59 | 3 | KIPAN | TCGA-BP-4777-01 | 14 | 1 | KIPAN | TCGA-BQ-5885-01 | 148 | 6 |
| KIPAN | TCGA-B2-5635-01 | 69 | 3 | KIPAN | TCGA-BP-4781-01 | 49 | 5 | KIPAN | TCGA-BQ-5886-01 | 94 | 3 |
| KIPAN | TCGA-B2-5641-01 | 81 | 7 | KIPAN | TCGA-BP-4782-01 | 93 | 5 | KIPAN | TCGA-BQ-5887-01 | 34 | 2 |
| KIPAN | TCGA-B3-3925-01 | 94 | 4 | KIPAN | TCGA-BP-4787-01 | 61 | 2 | KIPAN | TCGA-BQ-5888-01 | 45 | 0 |
| KIPAN | TCGA-B3-3926-01 | 41 | 2 | KIPAN | TCGA-BP-4789-01 | 1 | 1 | KIPAN | TCGA-BQ-5889-01 | 95 | 3 |
| KIPAN | TCGA-B3-4103-01 | 83 | 2 | KIPAN | TCGA-BP-4790-01 | 29 | 2 | KIPAN | TCGA-BQ-5890-01 | 108 | 8 |
| KIPAN | TCGA-B3-4104-01 | 124 | 9 | KIPAN | TCGA-BP-4797-01 | 22 | 2 | KIPAN | TCGA-BQ-5891-01 | 62 | 4 |
| KIPAN | TCGA-B3-8121-01 | 71 | 1 | KIPAN | TCGA-BP-4798-01 | 1 | 1 | KIPAN | TCGA-BQ-5892-01 | 91 | 3 |
| KIPAN | TCGA-B4-5377-01 | 57 | 3 | KIPAN | TCGA-BP-4799-01 | 26 | 0 | KIPAN | TCGA-BQ-5893-01 | 93 | 6 |
| KIPAN | TCGA-B8-4143-01 | 100 | 5 | KIPAN | TCGA-BP-4801-01 | 73 | 5 | KIPAN | TCGA-BQ-5894-01 | 59 | 2 |
| KIPAN | TCGA-B8-4146-01 | 52 | 0 | KIPAN | TCGA-BP-4803-01 | 1 | 1 | KIPAN | TCGA-BQ-7044-01 | 98 | 6 |
| KIPAN | TCGA-B8-4148-01 | 53 | 3 | KIPAN | TCGA-BP-4804-01 | 26 | 0 | KIPAN | TCGA-BQ-7045-01 | 105 | 4 |
| KIPAN | TCGA-B8-4151-01 | 81 | 4 | KIPAN | TCGA-BP-4807-01 | 63 | 2 | KIPAN | TCGA-BQ-7046-01 | 57 | 3 |
| KIPAN | TCGA-B8-4153-01 | 76 | 9 | KIPAN | TCGA-BP-4960-01 | 80 | 3 | KIPAN | TCGA-BQ-7048-01 | 93 | 4 |
| KIPAN | TCGA-B8-4154-01 | 68 | 2 | KIPAN | TCGA-BP-4961-01 | 45 | 4 | KIPAN | TCGA-BQ-7050-01 | 34 | 2 |
| KIPAN | TCGA-B8-4620-01 | 24 | 0 | KIPAN | TCGA-BP-4962-01 | 37 | 0 | KIPAN | TCGA-BQ-7051-01 | 111 | 5 |
| KIPAN | TCGA-B8-4621-01 | 109 | 8 | KIPAN | TCGA-BP-4963-01 | 90 | 6 | KIPAN | TCGA-BQ-7053-01 | 81 | 4 |
| KIPAN | TCGA-B8-4622-01 | 45 | 4 | KIPAN | TCGA-BP-4964-01 | 98 | 4 | KIPAN | TCGA-BQ-7055-01 | 19 | 0 |
| KIPAN | TCGA-B8-5158-01 | 68 | 7 | KIPAN | TCGA-BP-4967-01 | 85 | 1 | KIPAN | TCGA-BQ-7058-01 | 89 | 5 |
| KIPAN | TCGA-B8-5159-01 | 62 | 3 | KIPAN | TCGA-BP-4968-01 | 58 | 1 | KIPAN | TCGA-BQ-7059-01 | 104 | 4 |
| KIPAN | TCGA-B8-5163-01 | 83 | 6 | KIPAN | TCGA-BP-4970-01 | 40 | 1 | KIPAN | TCGA-BQ-7060-01 | 48 | 4 |
| KIPAN | TCGA-B8-5164-01 | 96 | 1 | KIPAN | TCGA-BP-4971-01 | 53 | 4 | KIPAN | TCGA-BQ-7061-01 | 103 | 8 |
| KIPAN | TCGA-B8-5165-01 | 23 | 2 | KIPAN | TCGA-BP-4972-01 | 50 | 3 | KIPAN | TCGA-BQ-7062-01 | 63 | 1 |
| KIPAN | TCGA-B8-5545-01 | 31 | 3 | KIPAN | TCGA-BP-4973-01 | 42 | 6 | KIPAN | TCGA-CJ-4634-01 | 103 | 4 |
| KIPAN | TCGA-B8-5546-01 | 25 | 0 | KIPAN | TCGA-BP-4974-01 | 29 | 1 | KIPAN | TCGA-CJ-4635-01 | 62 | 3 |
| KIPAN | TCGA-B8-5549-01 | 81 | 5 | KIPAN | TCGA-BP-4975-01 | 29 | 3 | KIPAN | TCGA-CJ-4636-01 | 72 | 4 |
| KIPAN | TCGA-B8-5550-01 | 99 | 10 | KIPAN | TCGA-BP-4976-01 | 112 | 8 | KIPAN | TCGA-CJ-4637-01 | 46 | 2 |
| KIPAN | TCGA-B8-5551-01 | 48 | 2 | KIPAN | TCGA-BP-4977-01 | 48 | 3 | KIPAN | TCGA-CJ-4638-01 | 86 | 5 |
| KIPAN | TCGA-B8-5552-01 | 43 | 2 | KIPAN | TCGA-BP-4981-01 | 61 | 5 | KIPAN | TCGA-CJ-4639-01 | 74 | 3 |
| KIPAN | TCGA-B8-5553-01 | 48 | 2 | KIPAN | TCGA-BP-4982-01 | 41 | 3 | KIPAN | TCGA-CJ-4640-01 | 67 | 2 |
| KIPAN | TCGA-B9-4113-01 | 104 | 9 | KIPAN | TCGA-BP-4983-01 | 84 | 5 | KIPAN | TCGA-CJ-4641-01 | 61 | 4 |
| KIPAN | TCGA-B9-4114-01 | 135 | 9 | KIPAN | TCGA-BP-4985-01 | 105 | 5 | KIPAN | TCGA-CJ-4643-01 | 146 | 8 |
| KIPAN | TCGA-B9-4115-01 | 99 | 4 | KIPAN | TCGA-BP-4986-01 | 47 | 5 | KIPAN | TCGA-CJ-4644-01 | 130 | 7 |
| KIPAN | TCGA-B9-4116-01 | 137 | 8 | KIPAN | TCGA-BP-4987-01 | 26 | 3 | KIPAN | TCGA-CJ-4868-01 | 25 | 1 |
| KIPAN | TCGA-B9-4117-01 | 363 | 18 | KIPAN | TCGA-BP-4988-01 | 48 | 3 | KIPAN | TCGA-CJ-4870-01 | 50 | 0 |
| KIPAN | TCGA-B9-5156-01 | 112 | 9 | KIPAN | TCGA-BP-4989-01 | 88 | 7 | KIPAN | TCGA-CJ-4871-01 | 49 | 6 |
| KIPAN | TCGA-B9-7268-01 | 78 | 3 | KIPAN | TCGA-BP-4991-01 | 51 | 3 | KIPAN | TCGA-CJ-4872-01 | 1 | 1 |
| KIPAN | TCGA-B9-A44B-01 | 185 | 12 | KIPAN | TCGA-BP-4992-01 | 43 | 3 | KIPAN | TCGA-CJ-4873-01 | 79 | 5 |
| KIPAN | TCGA-B9-A5W7-01 | 46 | 1 | KIPAN | TCGA-BP-4993-01 | 87 | 5 | KIPAN | TCGA-CJ-4874-01 | 68 | 4 |
| KIPAN | TCGA-B9-A5W8-01 | 99 | 5 | KIPAN | TCGA-BP-4995-01 | 54 | 6 | KIPAN | TCGA-CJ-4875-01 | 116 | 6 |
| KIPAN | TCGA-B9-A5W9-01 | 102 | 2 | KIPAN | TCGA-BP-4998-01 | 43 | 3 | KIPAN | TCGA-CJ-4876-01 | 46 | 1 |
| KIPAN | TCGA-B9-A69E-01 | 111 | 5 | KIPAN | TCGA-BP-4999-01 | 59 | 3 | KIPAN | TCGA-CJ-4878-01 | 35 | 0 |
| KIPAN | TCGA-BP-4158-01 | 63 | 3 | KIPAN | TCGA-BP-5000-01 | 48 | 2 | KIPAN | TCGA-CJ-4881-01 | 52 | 3 |
| KIPAN | TCGA-BP-4159-01 | 73 | 7 | KIPAN | TCGA-BP-5001-01 | 41 | 4 | KIPAN | TCGA-CJ-4882-01 | 71 | 1 |
| KIPAN | TCGA-BP-4160-01 | 69 | 9 | KIPAN | TCGA-BP-5004-01 | 55 | 2 | KIPAN | TCGA-CJ-4884-01 | 1 | 1 |
| KIPAN | TCGA-BP-4161-01 | 84 | 6 | KIPAN | TCGA-BP-5006-01 | 47 | 2 | KIPAN | TCGA-CJ-4885-01 | 1 | 1 |
| KIPAN | TCGA-BP-4162-01 | 58 | 2 | KIPAN | TCGA-BP-5007-01 | 39 | 3 | KIPAN | TCGA-CJ-4886-01 | 26 | 1 |
| KIPAN | TCGA-BP-4163-01 | 64 | 4 | KIPAN | TCGA-BP-5008-01 | 42 | 0 | KIPAN | TCGA-CJ-4887-01 | 45 | 2 |
| KIPAN | TCGA-BP-4164-01 | 96 | 9 | KIPAN | TCGA-BP-5009-01 | 55 | 6 | KIPAN | TCGA-CJ-4888-01 | 17 | 3 |
| KIPAN | TCGA-BP-4165-01 | 2 | 0 | KIPAN | TCGA-BP-5010-01 | 73 | 5 | KIPAN | TCGA-CJ-4889-01 | 23 | 2 |
| KIPAN | TCGA-BP-4166-01 | 1 | 1 | KIPAN | TCGA-BP-5168-01 | 140 | 2 | KIPAN | TCGA-CJ-4890-01 | 22 | 0 |
| KIPAN | TCGA-BP-4167-01 | 87 | 3 | KIPAN | TCGA-BP-5169-01 | 71 | 4 | KIPAN | TCGA-CJ-4891-01 | 15 | 3 |
| KIPAN | TCGA-BP-4169-01 | 51 | 4 | KIPAN | TCGA-BP-5170-01 | 47 | 5 | KIPAN | TCGA-CJ-4892-01 | 1 | 1 |
| KIPAN | TCGA-BP-4170-01 | 57 | 5 | KIPAN | TCGA-BP-5173-01 | 101 | 4 | KIPAN | TCGA-CJ-4893-01 | 37 | 2 |
| KIPAN | TCGA-BP-4173-01 | 44 | 3 | KIPAN | TCGA-BP-5174-01 | 44 | 3 | KIPAN | TCGA-CJ-4894-01 | 74 | 3 |
| KIPAN | TCGA-BP-4174-01 | 43 | 1 | KIPAN | TCGA-BP-5175-01 | 56 | 5 | KIPAN | TCGA-CJ-4895-01 | 50 | 1 |
| KIPAN | TCGA-BP-4176-01 | 109 | 8 | KIPAN | TCGA-BP-5176-01 | 137 | 8 | KIPAN | TCGA-CJ-4897-01 | 79 | 1 |
| KIPAN | TCGA-BP-4326-01 | 64 | 4 | KIPAN | TCGA-BP-5177-01 | 49 | 1 | KIPAN | TCGA-CJ-4899-01 | 44 | 2 |
| KIPAN | TCGA-BP-4329-01 | 52 | 4 | KIPAN | TCGA-BP-5178-01 | 68 | 5 | KIPAN | TCGA-CJ-4901-01 | 60 | 2 |
| KIPAN | TCGA-BP-4330-01 | 53 | 1 | KIPAN | TCGA-BP-5180-01 | 47 | 4 | KIPAN | TCGA-CJ-4902-01 | 75 | 0 |
| KIPAN | TCGA-BP-4331-01 | 51 | 2 | KIPAN | TCGA-BP-5182-01 | 70 | 2 | KIPAN | TCGA-CJ-4903-01 | 59 | 6 |
| KIPAN | TCGA-BP-4337-01 | 42 | 2 | KIPAN | TCGA-BP-5183-01 | 59 | 4 | KIPAN | TCGA-CJ-4904-01 | 48 | 2 |
| KIPAN | TCGA-BP-4338-01 | 70 | 1 | KIPAN | TCGA-BP-5184-01 | 37 | 2 | KIPAN | TCGA-CJ-4905-01 | 72 | 3 |
| KIPAN | TCGA-BP-4340-01 | 1 | 1 | KIPAN | TCGA-BP-5185-01 | 88 | 5 | KIPAN | TCGA-CJ-4907-01 | 71 | 2 |
| KIPAN | TCGA-BP-4341-01 | 54 | 5 | KIPAN | TCGA-BP-5186-01 | 41 | 2 | KIPAN | TCGA-CJ-4908-01 | 35 | 3 |
| KIPAN | TCGA-BP-4342-01 | 1 | 1 | KIPAN | TCGA-BP-5187-01 | 72 | 2 | KIPAN | TCGA-CJ-4912-01 | 96 | 7 |
| KIPAN | TCGA-BP-4343-01 | 36 | 4 | KIPAN | TCGA-BP-5189-01 | 52 | 5 | KIPAN | TCGA-CJ-4913-01 | 60 | 3 |
| KIPAN | TCGA-BP-4345-01 | 21 | 2 | KIPAN | TCGA-BP-5190-01 | 48 | 5 | KIPAN | TCGA-CJ-4916-01 | 54 | 6 |
| KIPAN | TCGA-BP-4346-01 | 47 | 1 | KIPAN | TCGA-BP-5191-01 | 69 | 1 | KIPAN | TCGA-CJ-4918-01 | 91 | 4 |

## TABLE 5 (APPENDIX A)

| | | | | | | | | | | | |
|---|---|---|---|---|---|---|---|---|---|---|---|
| KIPAN | TCGA-CJ-4920-01 | 157 | 9 | KIPAN | TCGA-DZ-6134-01 | 63 | 1 | KIPAN | TCGA-KN-8429-01 | 90 | 2 |
| KIPAN | TCGA-CJ-4923-01 | 59 | 3 | KIPAN | TCGA-DZ-6135-01 | 47 | 3 | KIPAN | TCGA-KN-8430-01 | 138 | 11 |
| KIPAN | TCGA-CJ-5671-01 | 76 | 3 | KIPAN | TCGA-EU-5904-01 | 54 | 1 | KIPAN | TCGA-KN-8431-01 | 80 | 3 |
| KIPAN | TCGA-CJ-5672-01 | 100 | 6 | KIPAN | TCGA-EU-5905-01 | 66 | 3 | KIPAN | TCGA-KN-8432-01 | 147 | 6 |
| KIPAN | TCGA-CJ-5675-01 | 79 | 2 | KIPAN | TCGA-EU-5906-01 | 70 | 3 | KIPAN | TCGA-KN-8433-01 | 300 | 11 |
| KIPAN | TCGA-CJ-5676-01 | 78 | 6 | KIPAN | TCGA-EU-5907-01 | 53 | 6 | KIPAN | TCGA-KN-8434-01 | 87 | 4 |
| KIPAN | TCGA-CJ-5677-01 | 92 | 4 | KIPAN | TCGA-EV-5901-01 | 57 | 4 | KIPAN | TCGA-KN-8435-01 | 105 | 2 |
| KIPAN | TCGA-CJ-5678-01 | 60 | 4 | KIPAN | TCGA-EV-5902-01 | 116 | 5 | KIPAN | TCGA-KN-8436-01 | 173 | 9 |
| KIPAN | TCGA-CJ-5679-01 | 118 | 4 | KIPAN | TCGA-EV-5903-01 | 142 | 6 | KIPAN | TCGA-KN-8437-01 | 56 | 5 |
| KIPAN | TCGA-CJ-5680-01 | 62 | 2 | KIPAN | TCGA-F9-A4JJ-01 | 58 | 2 | KIPAN | TCGA-KO-8403-01 | 63 | 4 |
| KIPAN | TCGA-CJ-5681-01 | 48 | 0 | KIPAN | TCGA-G7-6789-01 | 38 | 1 | KIPAN | TCGA-KO-8404-01 | 178 | 6 |
| KIPAN | TCGA-CJ-5682-01 | 93 | 5 | KIPAN | TCGA-G7-6790-01 | 90 | 5 | KIPAN | TCGA-KO-8405-01 | 67 | 5 |
| KIPAN | TCGA-CJ-5683-01 | 88 | 2 | KIPAN | TCGA-G7-6792-01 | 57 | 1 | KIPAN | TCGA-KO-8406-01 | 105 | 3 |
| KIPAN | TCGA-CJ-5684-01 | 71 | 5 | KIPAN | TCGA-G7-6793-01 | 50 | 1 | KIPAN | TCGA-KO-8407-01 | 105 | 1 |
| KIPAN | TCGA-CJ-5686-01 | 78 | 5 | KIPAN | TCGA-G7-6795-01 | 85 | 1 | KIPAN | TCGA-KO-8408-01 | 182 | 10 |
| KIPAN | TCGA-CJ-6027-01 | 87 | 4 | KIPAN | TCGA-G7-6796-01 | 76 | 4 | KIPAN | TCGA-KO-8409-01 | 113 | 2 |
| KIPAN | TCGA-CJ-6028-01 | 63 | 0 | KIPAN | TCGA-G7-6797-01 | 83 | 3 | KIPAN | TCGA-KO-8410-01 | 104 | 1 |
| KIPAN | TCGA-CJ-6030-01 | 125 | 8 | KIPAN | TCGA-G7-7501-01 | 56 | 0 | KIPAN | TCGA-KO-8411-01 | 88 | 4 |
| KIPAN | TCGA-CJ-6031-01 | 67 | 6 | KIPAN | TCGA-G7-7502-01 | 100 | 2 | KIPAN | TCGA-KO-8413-01 | 74 | 1 |
| KIPAN | TCGA-CJ-6032-01 | 57 | 3 | KIPAN | TCGA-G7-A4TM-01 | 58 | 3 | KIPAN | TCGA-KO-8414-01 | 74 | 2 |
| KIPAN | TCGA-CJ-6033-01 | 92 | 5 | KIPAN | TCGA-GL-6846-01 | 96 | 4 | KIPAN | TCGA-KO-8415-01 | 102 | 4 |
| KIPAN | TCGA-CW-5580-01 | 109 | 5 | KIPAN | TCGA-GL-7773-01 | 155 | 4 | KIPAN | TCGA-KO-8416-01 | 91 | 2 |
| KIPAN | TCGA-CW-5581-01 | 77 | 7 | KIPAN | TCGA-GL-7966-01 | 65 | 3 | KIPAN | TCGA-KO-8417-01 | 118 | 4 |
| KIPAN | TCGA-CW-5583-01 | 50 | 2 | KIPAN | TCGA-GL-8500-01 | 126 | 4 | KIPAN | TCGA-KV-A6GD-01 | 102 | 5 |
| KIPAN | TCGA-CW-5585-01 | 54 | 1 | KIPAN | TCGA-GL-A4EM-01 | 102 | 7 | KIPAN | TCGA-KV-A6GE-01 | 88 | 3 |
| KIPAN | TCGA-CW-5588-01 | 97 | 2 | KIPAN | TCGA-GL-A59R-01 | 117 | 8 | KIPAN | TCGA-MH-A55W-01 | 124 | 6 |
| KIPAN | TCGA-CW-5589-01 | 100 | 6 | KIPAN | TCGA-GL-A59T-01 | 125 | 8 | KIPAN | TCGA-MH-A55Z-01 | 130 | 5 |
| KIPAN | TCGA-CW-5591-01 | 62 | 2 | KIPAN | TCGA-HE-7128-01 | 60 | 1 | KIPAN | TCGA-MH-A560-01 | 85 | 2 |
| KIPAN | TCGA-CW-6087-01 | 68 | 4 | KIPAN | TCGA-HE-7129-01 | 67 | 1 | KIPAN | TCGA-MH-A561-01 | 150 | 2 |
| KIPAN | TCGA-CW-6090-01 | 124 | 4 | KIPAN | TCGA-HE-7130-01 | 234 | 11 | KIPAN | TCGA-MH-A562-01 | 93 | 4 |
| KIPAN | TCGA-CW-6093-01 | 130 | 6 | KIPAN | TCGA-HE-A5NF-01 | 93 | 4 | KIPAN | TCGA-P4-A5E6-01 | 133 | 3 |
| KIPAN | TCGA-CZ-4853-01 | 121 | 9 | KIPAN | TCGA-HE-A5NH-01 | 104 | 3 | KIPAN | TCGA-P4-A5E7-01 | 139 | 6 |
| KIPAN | TCGA-CZ-4854-01 | 94 | 2 | KIPAN | TCGA-HE-A5NI-01 | 138 | 8 | KIPAN | TCGA-P4-A5E8-01 | 75 | 4 |
| KIPAN | TCGA-CZ-4856-01 | 60 | 3 | KIPAN | TCGA-HE-A5NJ-01 | 89 | 5 | KIPAN | TCGA-P4-A5EA-01 | 123 | 8 |
| KIPAN | TCGA-CZ-4857-01 | 104 | 9 | KIPAN | TCGA-HE-A5NK-01 | 131 | 5 | KIPAN | TCGA-P4-A5EB-01 | 207 | 10 |
| KIPAN | TCGA-CZ-4858-01 | 2 | 2 | KIPAN | TCGA-HE-A5NL-01 | 106 | 2 | KIPAN | TCGA-P4-A5ED-01 | 54 | 2 |
| KIPAN | TCGA-CZ-4859-01 | 120 | 8 | KIPAN | TCGA-IA-A40U-01 | 66 | 4 | KIPAN | TCGA-PJ-A5Z8-01 | 107 | 4 |
| KIPAN | TCGA-CZ-4861-01 | 102 | 12 | KIPAN | TCGA-IA-A40X-01 | 44 | 2 | KIPAN | TCGA-PJ-A5Z9-01 | 94 | 4 |
| KIPAN | TCGA-CZ-4862-01 | 86 | 1 | KIPAN | TCGA-IA-A40Y-01 | 106 | 6 | KIPAN | TCGA-Q2-A5QZ-01 | 170 | 3 |
| KIPAN | TCGA-CZ-4863-01 | 48 | 2 | KIPAN | TCGA-IZ-8195-01 | 107 | 1 | KIRC | TCGA-A3-3308-01 | 105 | 5 |
| KIPAN | TCGA-CZ-4865-01 | 76 | 3 | KIPAN | TCGA-IZ-8196-01 | 139 | 7 | KIRC | TCGA-A3-3311-01 | 73 | 5 |
| KIPAN | TCGA-CZ-4866-01 | 97 | 4 | KIPAN | TCGA-IZ-A6M8-01 | 115 | 6 | KIRC | TCGA-A3-3316-01 | 46 | 4 |
| KIPAN | TCGA-CZ-5451-01 | 93 | 5 | KIPAN | TCGA-IZ-A6M9-01 | 121 | 1 | KIRC | TCGA-A3-3317-01 | 88 | 4 |
| KIPAN | TCGA-CZ-5452-01 | 40 | 2 | KIPAN | TCGA-J7-6720-01 | 65 | 0 | KIRC | TCGA-A3-3319-01 | 90 | 1 |
| KIPAN | TCGA-CZ-5453-01 | 104 | 4 | KIPAN | TCGA-J7-8537-01 | 77 | 5 | KIRC | TCGA-A3-3320-01 | 101 | 5 |
| KIPAN | TCGA-CZ-5454-01 | 31 | 3 | KIPAN | TCGA-KL-8323-01 | 76 | 2 | KIRC | TCGA-A3-3322-01 | 64 | 6 |
| KIPAN | TCGA-CZ-5455-01 | 35 | 4 | KIPAN | TCGA-KL-8324-01 | 119 | 6 | KIRC | TCGA-A3-3323-01 | 59 | 2 |
| KIPAN | TCGA-CZ-5456-01 | 82 | 5 | KIPAN | TCGA-KL-8325-01 | 81 | 2 | KIRC | TCGA-A3-3326-01 | 65 | 3 |
| KIPAN | TCGA-CZ-5457-01 | 92 | 9 | KIPAN | TCGA-KL-8326-01 | 81 | 4 | KIRC | TCGA-A3-3346-01 | 105 | 6 |
| KIPAN | TCGA-CZ-5458-01 | 40 | 2 | KIPAN | TCGA-KL-8327-01 | 60 | 3 | KIRC | TCGA-A3-3347-01 | 39 | 1 |
| KIPAN | TCGA-CZ-5459-01 | 112 | 5 | KIPAN | TCGA-KL-8328-01 | 101 | 4 | KIRC | TCGA-A3-3349-01 | 54 | 3 |
| KIPAN | TCGA-CZ-5460-01 | 69 | 4 | KIPAN | TCGA-KL-8329-01 | 131 | 11 | KIRC | TCGA-A3-3357-01 | 107 | 2 |
| KIPAN | TCGA-CZ-5461-01 | 79 | 4 | KIPAN | TCGA-KL-8330-01 | 104 | 2 | KIRC | TCGA-A3-3358-01 | 82 | 5 |
| KIPAN | TCGA-CZ-5462-01 | 77 | 5 | KIPAN | TCGA-KL-8331-01 | 83 | 3 | KIRC | TCGA-A3-3362-01 | 59 | 3 |
| KIPAN | TCGA-CZ-5463-01 | 60 | 1 | KIPAN | TCGA-KL-8332-01 | 77 | 4 | KIRC | TCGA-A3-3363-01 | 57 | 2 |
| KIPAN | TCGA-CZ-5465-01 | 147 | 7 | KIPAN | TCGA-KL-8333-01 | 72 | 3 | KIRC | TCGA-A3-3365-01 | 41 | 1 |
| KIPAN | TCGA-CZ-5466-01 | 77 | 10 | KIPAN | TCGA-KL-8334-01 | 82 | 3 | KIRC | TCGA-A3-3367-01 | 80 | 2 |
| KIPAN | TCGA-CZ-5467-01 | 68 | 3 | KIPAN | TCGA-KL-8335-01 | 114 | 4 | KIRC | TCGA-A3-3370-01 | 46 | 5 |
| KIPAN | TCGA-CZ-5468-01 | 150 | 4 | KIPAN | TCGA-KL-8336-01 | 85 | 3 | KIRC | TCGA-A3-3372-01 | 90 | 3 |
| KIPAN | TCGA-CZ-5469-01 | 60 | 4 | KIPAN | TCGA-KL-8337-01 | 84 | 4 | KIRC | TCGA-A3-3373-01 | 69 | 3 |
| KIPAN | TCGA-CZ-5470-01 | 58 | 6 | KIPAN | TCGA-KL-8338-01 | 59 | 3 | KIRC | TCGA-A3-3376-01 | 57 | 7 |
| KIPAN | TCGA-CZ-5982-01 | 53 | 5 | KIPAN | TCGA-KL-8339-01 | 120 | 5 | KIRC | TCGA-A3-3378-01 | 84 | 3 |
| KIPAN | TCGA-CZ-5984-01 | 51 | 2 | KIPAN | TCGA-KL-8340-01 | 61 | 0 | KIRC | TCGA-A3-3380-01 | 53 | 2 |
| KIPAN | TCGA-CZ-5985-01 | 71 | 2 | KIPAN | TCGA-KL-8341-01 | 160 | 7 | KIRC | TCGA-A3-3382-01 | 115 | 5 |
| KIPAN | TCGA-CZ-5986-01 | 67 | 6 | KIPAN | TCGA-KL-8342-01 | 77 | 7 | KIRC | TCGA-A3-3383-01 | 59 | 3 |
| KIPAN | TCGA-CZ-5987-01 | 62 | 4 | KIPAN | TCGA-KL-8343-01 | 95 | 2 | KIRC | TCGA-A3-3385-01 | 72 | 7 |
| KIPAN | TCGA-CZ-5988-01 | 52 | 0 | KIPAN | TCGA-KL-8344-01 | 79 | 3 | KIRC | TCGA-A3-3387-01 | 92 | 4 |
| KIPAN | TCGA-CZ-5989-01 | 44 | 3 | KIPAN | TCGA-KL-8345-01 | 71 | 5 | KIRC | TCGA-AK-3425-01 | 80 | 6 |
| KIPAN | TCGA-DV-5565-01 | 55 | 0 | KIPAN | TCGA-KL-8346-01 | 93 | 3 | KIRC | TCGA-AK-3428-01 | 79 | 2 |
| KIPAN | TCGA-DV-5566-01 | 42 | 1 | KIPAN | TCGA-KM-8438-01 | 112 | 4 | KIRC | TCGA-AK-3429-01 | 46 | 4 |
| KIPAN | TCGA-DV-5568-01 | 13 | 1 | KIPAN | TCGA-KM-8439-01 | 57 | 2 | KIRC | TCGA-AK-3430-01 | 86 | 3 |
| KIPAN | TCGA-DV-5569-01 | 31 | 2 | KIPAN | TCGA-KM-8440-01 | 79 | 3 | KIRC | TCGA-AK-3431-01 | 80 | 5 |
| KIPAN | TCGA-DV-5574-01 | 47 | 4 | KIPAN | TCGA-KM-8441-01 | 69 | 2 | KIRC | TCGA-AK-3434-01 | 80 | 1 |
| KIPAN | TCGA-DV-5575-01 | 51 | 0 | KIPAN | TCGA-KM-8442-01 | 94 | 1 | KIRC | TCGA-AK-3442-01 | 68 | 3 |
| KIPAN | TCGA-DV-5576-01 | 46 | 4 | KIPAN | TCGA-KM-8443-01 | 77 | 1 | KIRC | TCGA-AK-3444-01 | 109 | 9 |
| KIPAN | TCGA-DW-5560-01 | 81 | 2 | KIPAN | TCGA-KM-8476-01 | 117 | 2 | KIRC | TCGA-AK-3445-01 | 1 | 1 |
| KIPAN | TCGA-DW-5561-01 | 63 | 1 | KIPAN | TCGA-KM-8477-01 | 67 | 2 | KIRC | TCGA-AK-3450-01 | 1 | 1 |
| KIPAN | TCGA-DW-7834-01 | 94 | 2 | KIPAN | TCGA-KM-8639-01 | 72 | 2 | KIRC | TCGA-AK-3451-01 | 98 | 5 |
| KIPAN | TCGA-DW-7837-01 | 47 | 4 | KIPAN | TCGA-KN-8418-01 | 71 | 4 | KIRC | TCGA-AK-3454-01 | 70 | 2 |
| KIPAN | TCGA-DW-7838-01 | 129 | 4 | KIPAN | TCGA-KN-8419-01 | 78 | 3 | KIRC | TCGA-AK-3455-01 | 71 | 4 |
| KIPAN | TCGA-DW-7839-01 | 61 | 2 | KIPAN | TCGA-KN-8421-01 | 72 | 2 | KIRC | TCGA-AK-3456-01 | 60 | 1 |
| KIPAN | TCGA-DW-7840-01 | 123 | 11 | KIPAN | TCGA-KN-8422-01 | 81 | 0 | KIRC | TCGA-AK-3458-01 | 41 | 3 |
| KIPAN | TCGA-DW-7841-01 | 71 | 3 | KIPAN | TCGA-KN-8423-01 | 81 | 3 | KIRC | TCGA-AK-3460-01 | 80 | 5 |
| KIPAN | TCGA-DW-7842-01 | 46 | 2 | KIPAN | TCGA-KN-8424-01 | 107 | 7 | KIRC | TCGA-AK-3461-01 | 59 | 2 |
| KIPAN | TCGA-DW-7963-01 | 66 | 1 | KIPAN | TCGA-KN-8425-01 | 94 | 9 | KIRC | TCGA-AS-3778-01 | 81 | 3 |
| KIPAN | TCGA-DZ-6131-01 | 44 | 0 | KIPAN | TCGA-KN-8426-01 | 106 | 3 | KIRC | TCGA-B0-4690-01 | 1 | 1 |
| KIPAN | TCGA-DZ-6132-01 | 144 | 5 | KIPAN | TCGA-KN-8427-01 | 143 | 3 | KIRC | TCGA-B0-4691-01 | 109 | 8 |
| KIPAN | TCGA-DZ-6133-01 | 126 | 6 | KIPAN | TCGA-KN-8428-01 | 1174 | 43 | KIRC | TCGA-B0-4693-01 | 106 | 4 |

# TABLE 5 (APPENDIX A)

| | | | |
|---|---|---|---|
| KIRC | TCGA-B0-4694-01 | 83 | 3 |
| KIRC | TCGA-B0-4697-01 | 52 | 2 |
| KIRC | TCGA-B0-4700-01 | 34 | 2 |
| KIRC | TCGA-B0-4703-01 | 1 | 1 |
| KIRC | TCGA-B0-4706-01 | 61 | 4 |
| KIRC | TCGA-B0-4707-01 | 1 | 1 |
| KIRC | TCGA-B0-4710-01 | 99 | 5 |
| KIRC | TCGA-B0-4712-01 | 125 | 6 |
| KIRC | TCGA-B0-4713-01 | 1 | 1 |
| KIRC | TCGA-B0-4714-01 | 1 | 1 |
| KIRC | TCGA-B0-4718-01 | 79 | 7 |
| KIRC | TCGA-B0-4810-01 | 74 | 6 |
| KIRC | TCGA-B0-4811-01 | 132 | 3 |
| KIRC | TCGA-B0-4813-01 | 67 | 3 |
| KIRC | TCGA-B0-4814-01 | 73 | 4 |
| KIRC | TCGA-B0-4815-01 | 81 | 5 |
| KIRC | TCGA-B0-4816-01 | 65 | 7 |
| KIRC | TCGA-B0-4817-01 | 67 | 2 |
| KIRC | TCGA-B0-4818-01 | 81 | 5 |
| KIRC | TCGA-B0-4819-01 | 45 | 4 |
| KIRC | TCGA-B0-4822-01 | 1 | 1 |
| KIRC | TCGA-B0-4823-01 | 125 | 6 |
| KIRC | TCGA-B0-4824-01 | 1 | 1 |
| KIRC | TCGA-B0-4827-01 | 107 | 11 |
| KIRC | TCGA-B0-4828-01 | 106 | 4 |
| KIRC | TCGA-B0-4833-01 | 1 | 1 |
| KIRC | TCGA-B0-4836-01 | 29 | 0 |
| KIRC | TCGA-B0-4837-01 | 48 | 3 |
| KIRC | TCGA-B0-4838-01 | 53 | 0 |
| KIRC | TCGA-B0-4839-01 | 73 | 3 |
| KIRC | TCGA-B0-4841-01 | 111 | 1 |
| KIRC | TCGA-B0-4842-01 | 62 | 7 |
| KIRC | TCGA-B0-4843-01 | 81 | 5 |
| KIRC | TCGA-B0-4844-01 | 53 | 3 |
| KIRC | TCGA-B0-4845-01 | 1 | 1 |
| KIRC | TCGA-B0-4846-01 | 47 | 2 |
| KIRC | TCGA-B0-4847-01 | 49 | 6 |
| KIRC | TCGA-B0-4848-01 | 1 | 0 |
| KIRC | TCGA-B0-4849-01 | 26 | 2 |
| KIRC | TCGA-B0-4852-01 | 89 | 5 |
| KIRC | TCGA-B0-4945-01 | 44 | 3 |
| KIRC | TCGA-B0-5075-01 | 106 | 4 |
| KIRC | TCGA-B0-5077-01 | 64 | 5 |
| KIRC | TCGA-B0-5080-01 | 69 | 2 |
| KIRC | TCGA-B0-5081-01 | 49 | 3 |
| KIRC | TCGA-B0-5085-01 | 79 | 4 |
| KIRC | TCGA-B0-5088-01 | 75 | 3 |
| KIRC | TCGA-B0-5092-01 | 78 | 5 |
| KIRC | TCGA-B0-5094-01 | 94 | 6 |
| KIRC | TCGA-B0-5095-01 | 87 | 3 |
| KIRC | TCGA-B0-5096-01 | 108 | 4 |
| KIRC | TCGA-B0-5097-01 | 68 | 7 |
| KIRC | TCGA-B0-5099-01 | 84 | 6 |
| KIRC | TCGA-B0-5100-01 | 41 | 1 |
| KIRC | TCGA-B0-5102-01 | 58 | 4 |
| KIRC | TCGA-B0-5104-01 | 73 | 2 |
| KIRC | TCGA-B0-5106-01 | 96 | 4 |
| KIRC | TCGA-B0-5107-01 | 65 | 5 |
| KIRC | TCGA-B0-5108-01 | 63 | 4 |
| KIRC | TCGA-B0-5109-01 | 61 | 3 |
| KIRC | TCGA-B0-5110-01 | 79 | 7 |
| KIRC | TCGA-B0-5113-01 | 43 | 2 |
| KIRC | TCGA-B0-5115-01 | 64 | 7 |
| KIRC | TCGA-B0-5116-01 | 67 | 4 |
| KIRC | TCGA-B0-5119-01 | 102 | 6 |
| KIRC | TCGA-B0-5120-01 | 67 | 4 |
| KIRC | TCGA-B0-5121-01 | 59 | 3 |
| KIRC | TCGA-B0-5399-01 | 56 | 6 |
| KIRC | TCGA-B0-5400-01 | 49 | 3 |
| KIRC | TCGA-B0-5402-01 | 62 | 3 |
| KIRC | TCGA-B0-5691-01 | 75 | 2 |
| KIRC | TCGA-B0-5692-01 | 87 | 5 |
| KIRC | TCGA-B0-5693-01 | 49 | 4 |
| KIRC | TCGA-B0-5694-01 | 72 | 4 |
| KIRC | TCGA-B0-5695-01 | 77 | 6 |
| KIRC | TCGA-B0-5696-01 | 77 | 7 |
| KIRC | TCGA-B0-5697-01 | 79 | 3 |
| KIRC | TCGA-B0-5698-01 | 83 | 3 |
| KIRC | TCGA-B0-5699-01 | 55 | 5 |
| KIRC | TCGA-B0-5701-01 | 121 | 9 |
| KIRC | TCGA-B0-5702-01 | 75 | 3 |
| KIRC | TCGA-B0-5703-01 | 99 | 7 |
| KIRC | TCGA-B0-5705-01 | 91 | 5 |
| KIRC | TCGA-B0-5706-01 | 68 | 5 |
| KIRC | TCGA-B0-5707-01 | 51 | 3 |
| KIRC | TCGA-B0-5709-01 | 83 | 5 |
| KIRC | TCGA-B0-5710-01 | 43 | 4 |
| KIRC | TCGA-B0-5711-01 | 40 | 3 |
| KIRC | TCGA-B0-5713-01 | 119 | 11 |
| KIRC | TCGA-B0-5812-01 | 51 | 4 |
| KIRC | TCGA-B2-3924-01 | 26 | 1 |
| KIRC | TCGA-B2-4098-01 | 41 | 4 |
| KIRC | TCGA-B2-4099-01 | 24 | 0 |
| KIRC | TCGA-B2-4101-01 | 40 | 3 |
| KIRC | TCGA-B2-5633-01 | 59 | 3 |
| KIRC | TCGA-B2-5635-01 | 69 | 3 |
| KIRC | TCGA-B2-5641-01 | 81 | 7 |
| KIRC | TCGA-B4-5377-01 | 57 | 3 |
| KIRC | TCGA-B8-4143-01 | 100 | 5 |
| KIRC | TCGA-B8-4146-01 | 52 | 0 |
| KIRC | TCGA-B8-4148-01 | 53 | 3 |
| KIRC | TCGA-B8-4151-01 | 81 | 4 |
| KIRC | TCGA-B8-4153-01 | 76 | 9 |
| KIRC | TCGA-B8-4154-01 | 68 | 2 |
| KIRC | TCGA-B8-4620-01 | 24 | 0 |
| KIRC | TCGA-B8-4621-01 | 109 | 8 |
| KIRC | TCGA-B8-4622-01 | 45 | 4 |
| KIRC | TCGA-B8-5158-01 | 68 | 7 |
| KIRC | TCGA-B8-5159-01 | 62 | 3 |
| KIRC | TCGA-B8-5163-01 | 83 | 6 |
| KIRC | TCGA-B8-5164-01 | 96 | 1 |
| KIRC | TCGA-B8-5165-01 | 23 | 2 |
| KIRC | TCGA-B8-5545-01 | 31 | 3 |
| KIRC | TCGA-B8-5546-01 | 25 | 0 |
| KIRC | TCGA-B8-5549-01 | 81 | 5 |
| KIRC | TCGA-B8-5550-01 | 99 | 10 |
| KIRC | TCGA-B8-5551-01 | 63 | 2 |
| KIRC | TCGA-B8-5552-01 | 43 | 2 |
| KIRC | TCGA-B8-5553-01 | 48 | 2 |
| KIRC | TCGA-BP-4158-01 | 63 | 3 |
| KIRC | TCGA-BP-4159-01 | 73 | 7 |
| KIRC | TCGA-BP-4160-01 | 69 | 9 |
| KIRC | TCGA-BP-4161-01 | 84 | 6 |
| KIRC | TCGA-BP-4162-01 | 58 | 2 |
| KIRC | TCGA-BP-4163-01 | 64 | 4 |
| KIRC | TCGA-BP-4164-01 | 96 | 9 |
| KIRC | TCGA-BP-4165-01 | 2 | 0 |
| KIRC | TCGA-BP-4166-01 | 1 | 1 |
| KIRC | TCGA-BP-4167-01 | 87 | 3 |
| KIRC | TCGA-BP-4169-01 | 51 | 4 |
| KIRC | TCGA-BP-4170-01 | 57 | 5 |
| KIRC | TCGA-BP-4173-01 | 21 | 3 |
| KIRC | TCGA-BP-4174-01 | 43 | 1 |
| KIRC | TCGA-BP-4176-01 | 109 | 8 |
| KIRC | TCGA-BP-4326-01 | 64 | 4 |
| KIRC | TCGA-BP-4329-01 | 52 | 4 |
| KIRC | TCGA-BP-4330-01 | 53 | 1 |
| KIRC | TCGA-BP-4331-01 | 51 | 2 |
| KIRC | TCGA-BP-4337-01 | 42 | 2 |
| KIRC | TCGA-BP-4338-01 | 70 | 1 |
| KIRC | TCGA-BP-4340-01 | 1 | 1 |
| KIRC | TCGA-BP-4341-01 | 54 | 5 |
| KIRC | TCGA-BP-4342-01 | 1 | 1 |
| KIRC | TCGA-BP-4343-01 | 36 | 4 |
| KIRC | TCGA-BP-4345-01 | 21 | 2 |
| KIRC | TCGA-BP-4346-01 | 47 | 1 |
| KIRC | TCGA-BP-4347-01 | 42 | 0 |
| KIRC | TCGA-BP-4349-01 | 1 | 1 |
| KIRC | TCGA-BP-4351-01 | 73 | 4 |
| KIRC | TCGA-BP-4352-01 | 99 | 3 |
| KIRC | TCGA-BP-4354-01 | 32 | 3 |
| KIRC | TCGA-BP-4355-01 | 1 | 1 |
| KIRC | TCGA-BP-4756-01 | 49 | 1 |
| KIRC | TCGA-BP-4758-01 | 1 | 1 |
| KIRC | TCGA-BP-4759-01 | 1 | 1 |
| KIRC | TCGA-BP-4761-01 | 89 | 2 |
| KIRC | TCGA-BP-4762-01 | 1 | 1 |
| KIRC | TCGA-BP-4763-01 | 1 | 1 |
| KIRC | TCGA-BP-4765-01 | 1 | 1 |
| KIRC | TCGA-BP-4766-01 | 48 | 2 |
| KIRC | TCGA-BP-4768-01 | 65 | 7 |
| KIRC | TCGA-BP-4770-01 | 84 | 4 |
| KIRC | TCGA-BP-4771-01 | 30 | 2 |
| KIRC | TCGA-BP-4774-01 | 56 | 4 |
| KIRC | TCGA-BP-4775-01 | 50 | 1 |
| KIRC | TCGA-BP-4777-01 | 14 | 1 |
| KIRC | TCGA-BP-4781-01 | 49 | 5 |
| KIRC | TCGA-BP-4782-01 | 93 | 5 |
| KIRC | TCGA-BP-4787-01 | 61 | 2 |
| KIRC | TCGA-BP-4789-01 | 1 | 1 |
| KIRC | TCGA-BP-4790-01 | 29 | 2 |
| KIRC | TCGA-BP-4797-01 | 22 | 2 |
| KIRC | TCGA-BP-4798-01 | 1 | 1 |
| KIRC | TCGA-BP-4799-01 | 26 | 0 |
| KIRC | TCGA-BP-4801-01 | 73 | 5 |
| KIRC | TCGA-BP-4803-01 | 1 | 1 |
| KIRC | TCGA-BP-4804-01 | 26 | 0 |
| KIRC | TCGA-BP-4807-01 | 63 | 2 |
| KIRC | TCGA-BP-4960-01 | 80 | 3 |
| KIRC | TCGA-BP-4961-01 | 45 | 4 |
| KIRC | TCGA-BP-4962-01 | 37 | 0 |
| KIRC | TCGA-BP-4963-01 | 90 | 6 |
| KIRC | TCGA-BP-4964-01 | 98 | 4 |
| KIRC | TCGA-BP-4967-01 | 85 | 1 |
| KIRC | TCGA-BP-4968-01 | 58 | 1 |
| KIRC | TCGA-BP-4970-01 | 40 | 1 |
| KIRC | TCGA-BP-4971-01 | 53 | 4 |
| KIRC | TCGA-BP-4972-01 | 50 | 3 |
| KIRC | TCGA-BP-4973-01 | 42 | 6 |
| KIRC | TCGA-BP-4974-01 | 29 | 1 |
| KIRC | TCGA-BP-4975-01 | 29 | 3 |
| KIRC | TCGA-BP-4976-01 | 112 | 8 |
| KIRC | TCGA-BP-4977-01 | 48 | 3 |
| KIRC | TCGA-BP-4981-01 | 61 | 5 |
| KIRC | TCGA-BP-4982-01 | 41 | 3 |
| KIRC | TCGA-BP-4983-01 | 84 | 5 |
| KIRC | TCGA-BP-4985-01 | 105 | 5 |
| KIRC | TCGA-BP-4986-01 | 47 | 5 |
| KIRC | TCGA-BP-4987-01 | 26 | 3 |
| KIRC | TCGA-BP-4988-01 | 48 | 3 |
| KIRC | TCGA-BP-4989-01 | 88 | 7 |
| KIRC | TCGA-BP-4991-01 | 51 | 3 |
| KIRC | TCGA-BP-4992-01 | 43 | 3 |
| KIRC | TCGA-BP-4993-01 | 87 | 5 |
| KIRC | TCGA-BP-4995-01 | 54 | 6 |
| KIRC | TCGA-BP-4998-01 | 43 | 3 |
| KIRC | TCGA-BP-4999-01 | 59 | 3 |
| KIRC | TCGA-BP-5000-01 | 48 | 2 |
| KIRC | TCGA-BP-5001-01 | 41 | 4 |
| KIRC | TCGA-BP-5004-01 | 55 | 2 |
| KIRC | TCGA-BP-5006-01 | 47 | 2 |
| KIRC | TCGA-BP-5007-01 | 39 | 3 |
| KIRC | TCGA-BP-5008-01 | 42 | 0 |
| KIRC | TCGA-BP-5009-01 | 55 | 6 |
| KIRC | TCGA-BP-5010-01 | 73 | 5 |
| KIRC | TCGA-BP-5168-01 | 140 | 2 |
| KIRC | TCGA-BP-5169-01 | 71 | 4 |
| KIRC | TCGA-BP-5170-01 | 47 | 5 |
| KIRC | TCGA-BP-5173-01 | 101 | 4 |
| KIRC | TCGA-BP-5174-01 | 44 | 3 |
| KIRC | TCGA-BP-5175-01 | 56 | 5 |
| KIRC | TCGA-BP-5176-01 | 137 | 8 |
| KIRC | TCGA-BP-5177-01 | 49 | 1 |
| KIRC | TCGA-BP-5178-01 | 68 | 5 |
| KIRC | TCGA-BP-5180-01 | 47 | 4 |
| KIRC | TCGA-BP-5182-01 | 70 | 2 |
| KIRC | TCGA-BP-5183-01 | 59 | 4 |
| KIRC | TCGA-BP-5184-01 | 37 | 2 |
| KIRC | TCGA-BP-5185-01 | 88 | 5 |
| KIRC | TCGA-BP-5186-01 | 41 | 2 |
| KIRC | TCGA-BP-5187-01 | 72 | 2 |
| KIRC | TCGA-BP-5189-01 | 52 | 5 |
| KIRC | TCGA-BP-5190-01 | 48 | 5 |
| KIRC | TCGA-BP-5191-01 | 69 | 1 |
| KIRC | TCGA-BP-5192-01 | 61 | 4 |
| KIRC | TCGA-BP-5194-01 | 30 | 2 |
| KIRC | TCGA-BP-5195-01 | 66 | 3 |
| KIRC | TCGA-BP-5196-01 | 64 | 1 |
| KIRC | TCGA-BP-5198-01 | 83 | 6 |
| KIRC | TCGA-BP-5199-01 | 87 | 5 |
| KIRC | TCGA-BP-5200-01 | 51 | 1 |
| KIRC | TCGA-BP-5201-01 | 45 | 2 |
| KIRC | TCGA-BP-5202-01 | 46 | 3 |
| KIRC | TCGA-CJ-4634-01 | 103 | 4 |
| KIRC | TCGA-CJ-4635-01 | 62 | 3 |
| KIRC | TCGA-CJ-4636-01 | 72 | 4 |
| KIRC | TCGA-CJ-4637-01 | 46 | 2 |
| KIRC | TCGA-CJ-4638-01 | 86 | 5 |
| KIRC | TCGA-CJ-4639-01 | 74 | 3 |
| KIRC | TCGA-CJ-4640-01 | 67 | 2 |
| KIRC | TCGA-CJ-4641-01 | 61 | 4 |
| KIRC | TCGA-CJ-4643-01 | 146 | 8 |
| KIRC | TCGA-CJ-4644-01 | 130 | 7 |
| KIRC | TCGA-CJ-4868-01 | 25 | 1 |
| KIRC | TCGA-CJ-4870-01 | 50 | 0 |
| KIRC | TCGA-CJ-4871-01 | 49 | 6 |
| KIRC | TCGA-CJ-4872-01 | 1 | 1 |
| KIRC | TCGA-CJ-4873-01 | 79 | 5 |
| KIRC | TCGA-CJ-4874-01 | 68 | 4 |
| KIRC | TCGA-CJ-4875-01 | 116 | 6 |
| KIRC | TCGA-CJ-4876-01 | 46 | 1 |
| KIRC | TCGA-CJ-4878-01 | 35 | 0 |
| KIRC | TCGA-CJ-4881-01 | 52 | 3 |
| KIRC | TCGA-CJ-4882-01 | 71 | 1 |

# TABLE 5 (APPENDIX A)

| Type | Sample | Value 1 | Value 2 |
|---|---|---|---|
| KIRC | TCGA-CJ-4884-01 | 1 | 1 |
| KIRC | TCGA-CJ-4885-01 | 1 | 1 |
| KIRC | TCGA-CJ-4886-01 | 26 | 1 |
| KIRC | TCGA-CJ-4887-01 | 45 | 2 |
| KIRC | TCGA-CJ-4888-01 | 17 | 3 |
| KIRC | TCGA-CJ-4889-01 | 23 | 2 |
| KIRC | TCGA-CJ-4890-01 | 22 | 0 |
| KIRC | TCGA-CJ-4891-01 | 15 | 3 |
| KIRC | TCGA-CJ-4892-01 | 1 | 1 |
| KIRC | TCGA-CJ-4893-01 | 37 | 2 |
| KIRC | TCGA-CJ-4894-01 | 74 | 3 |
| KIRC | TCGA-CJ-4895-01 | 50 | 1 |
| KIRC | TCGA-CJ-4897-01 | 79 | 1 |
| KIRC | TCGA-CJ-4899-01 | 44 | 2 |
| KIRC | TCGA-CJ-4901-01 | 60 | 2 |
| KIRC | TCGA-CJ-4902-01 | 75 | 0 |
| KIRC | TCGA-CJ-4903-01 | 59 | 6 |
| KIRC | TCGA-CJ-4904-01 | 48 | 2 |
| KIRC | TCGA-CJ-4905-01 | 72 | 3 |
| KIRC | TCGA-CJ-4907-01 | 71 | 2 |
| KIRC | TCGA-CJ-4908-01 | 35 | 3 |
| KIRC | TCGA-CJ-4912-01 | 96 | 7 |
| KIRC | TCGA-CJ-4913-01 | 60 | 3 |
| KIRC | TCGA-CJ-4916-01 | 54 | 6 |
| KIRC | TCGA-CJ-4918-01 | 91 | 4 |
| KIRC | TCGA-CJ-4920-01 | 157 | 9 |
| KIRC | TCGA-CJ-4923-01 | 59 | 3 |
| KIRC | TCGA-CJ-5671-01 | 76 | 3 |
| KIRC | TCGA-CJ-5672-01 | 100 | 6 |
| KIRC | TCGA-CJ-5675-01 | 79 | 2 |
| KIRC | TCGA-CJ-5676-01 | 78 | 6 |
| KIRC | TCGA-CJ-5677-01 | 92 | 4 |
| KIRC | TCGA-CJ-5678-01 | 60 | 4 |
| KIRC | TCGA-CJ-5679-01 | 118 | 4 |
| KIRC | TCGA-CJ-5680-01 | 62 | 2 |
| KIRC | TCGA-CJ-5681-01 | 48 | 0 |
| KIRC | TCGA-CJ-5682-01 | 93 | 5 |
| KIRC | TCGA-CJ-5683-01 | 88 | 2 |
| KIRC | TCGA-CJ-5684-01 | 71 | 5 |
| KIRC | TCGA-CJ-5686-01 | 78 | 5 |
| KIRC | TCGA-CJ-6027-01 | 87 | 4 |
| KIRC | TCGA-CJ-6028-01 | 63 | 0 |
| KIRC | TCGA-CJ-6030-01 | 125 | 8 |
| KIRC | TCGA-CJ-6031-01 | 67 | 6 |
| KIRC | TCGA-CJ-6032-01 | 57 | 3 |
| KIRC | TCGA-CJ-6033-01 | 92 | 5 |
| KIRC | TCGA-CW-5580-01 | 109 | 5 |
| KIRC | TCGA-CW-5581-01 | 77 | 7 |
| KIRC | TCGA-CW-5583-01 | 50 | 2 |
| KIRC | TCGA-CW-5585-01 | 54 | 1 |
| KIRC | TCGA-CW-5588-01 | 97 | 2 |
| KIRC | TCGA-CW-5589-01 | 100 | 6 |
| KIRC | TCGA-CW-5591-01 | 62 | 2 |
| KIRC | TCGA-CW-6087-01 | 68 | 4 |
| KIRC | TCGA-CW-6090-01 | 124 | 4 |
| KIRC | TCGA-CW-6093-01 | 130 | 6 |
| KIRC | TCGA-CZ-4853-01 | 121 | 9 |
| KIRC | TCGA-CZ-4854-01 | 94 | 2 |
| KIRC | TCGA-CZ-4856-01 | 60 | 3 |
| KIRC | TCGA-CZ-4857-01 | 104 | 9 |
| KIRC | TCGA-CZ-4858-01 | 2 | 2 |
| KIRC | TCGA-CZ-4859-01 | 120 | 8 |
| KIRC | TCGA-CZ-4861-01 | 102 | 12 |
| KIRC | TCGA-CZ-4862-01 | 86 | 1 |
| KIRC | TCGA-CZ-4863-01 | 48 | 2 |
| KIRC | TCGA-CZ-4865-01 | 76 | 3 |
| KIRC | TCGA-CZ-4866-01 | 97 | 4 |
| KIRC | TCGA-CZ-5451-01 | 93 | 5 |
| KIRC | TCGA-CZ-5452-01 | 40 | 2 |
| KIRC | TCGA-CZ-5453-01 | 104 | 4 |
| KIRC | TCGA-CZ-5454-01 | 31 | 3 |
| KIRC | TCGA-CZ-5455-01 | 35 | 4 |
| KIRC | TCGA-CZ-5456-01 | 82 | 5 |
| KIRC | TCGA-CZ-5457-01 | 92 | 9 |
| KIRC | TCGA-CZ-5458-01 | 40 | 2 |
| KIRC | TCGA-CZ-5459-01 | 112 | 5 |
| KIRC | TCGA-CZ-5460-01 | 69 | 4 |
| KIRC | TCGA-CZ-5461-01 | 79 | 4 |
| KIRC | TCGA-CZ-5462-01 | 77 | 5 |
| KIRC | TCGA-CZ-5463-01 | 60 | 1 |
| KIRC | TCGA-CZ-5465-01 | 147 | 7 |
| KIRC | TCGA-CZ-5466-01 | 77 | 10 |
| KIRC | TCGA-CZ-5467-01 | 68 | 3 |
| KIRC | TCGA-CZ-5468-01 | 150 | 4 |
| KIRC | TCGA-CZ-5469-01 | 60 | 4 |
| KIRC | TCGA-CZ-5470-01 | 58 | 6 |
| KIRC | TCGA-CZ-5982-01 | 53 | 5 |
| KIRC | TCGA-CZ-5984-01 | 51 | 2 |
| KIRC | TCGA-CZ-5985-01 | 71 | 2 |
| KIRC | TCGA-CZ-5986-01 | 67 | 6 |
| KIRC | TCGA-CZ-5987-01 | 62 | 4 |
| KIRC | TCGA-CZ-5988-01 | 52 | 0 |
| KIRC | TCGA-CZ-5989-01 | 44 | 3 |
| KIRC | TCGA-DV-5565-01 | 55 | 0 |
| KIRC | TCGA-DV-5566-01 | 42 | 1 |
| KIRC | TCGA-DV-5568-01 | 13 | 1 |
| KIRC | TCGA-DV-5569-01 | 31 | 2 |
| KIRC | TCGA-DV-5574-01 | 47 | 4 |
| KIRC | TCGA-DV-5575-01 | 51 | 0 |
| KIRC | TCGA-DV-5576-01 | 46 | 4 |
| KIRC | TCGA-EU-5904-01 | 54 | 1 |
| KIRC | TCGA-EU-5905-01 | 66 | 3 |
| KIRC | TCGA-EU-5906-01 | 70 | 3 |
| KIRC | TCGA-EU-5907-01 | 53 | 6 |
| KIRP | TCGA-A4-7286-01 | 60 | 0 |
| KIRP | TCGA-A4-7287-01 | 66 | 4 |
| KIRP | TCGA-A4-7288-01 | 100 | 7 |
| KIRP | TCGA-A4-7583-01 | 87 | 4 |
| KIRP | TCGA-A4-7584-01 | 90 | 2 |
| KIRP | TCGA-A4-7585-01 | 121 | 2 |
| KIRP | TCGA-A4-7732-01 | 83 | 2 |
| KIRP | TCGA-A4-7734-01 | 74 | 2 |
| KIRP | TCGA-A4-7828-01 | 72 | 2 |
| KIRP | TCGA-A4-7915-01 | 54 | 1 |
| KIRP | TCGA-A4-7996-01 | 108 | 4 |
| KIRP | TCGA-A4-7997-01 | 105 | 9 |
| KIRP | TCGA-A4-8098-01 | 148 | 5 |
| KIRP | TCGA-A4-8310-01 | 118 | 4 |
| KIRP | TCGA-A4-8311-01 | 95 | 4 |
| KIRP | TCGA-A4-8312-01 | 59 | 1 |
| KIRP | TCGA-A4-8515-01 | 97 | 5 |
| KIRP | TCGA-A4-8517-01 | 108 | 6 |
| KIRP | TCGA-A4-8518-01 | 106 | 2 |
| KIRP | TCGA-A4-8630-01 | 99 | 3 |
| KIRP | TCGA-A4-A48D-01 | 154 | 4 |
| KIRP | TCGA-A4-A4ZT-01 | 77 | 1 |
| KIRP | TCGA-A4-A57E-01 | 120 | 7 |
| KIRP | TCGA-A4-A5DU-01 | 60 | 2 |
| KIRP | TCGA-A4-A5XZ-01 | 92 | 4 |
| KIRP | TCGA-A4-A5Y0-01 | 90 | 7 |
| KIRP | TCGA-A4-A5Y1-01 | 119 | 5 |
| KIRP | TCGA-A4-A6HP-01 | 147 | 5 |
| KIRP | TCGA-AL-3466-01 | 91 | 3 |
| KIRP | TCGA-AL-3467-01 | 44 | 1 |
| KIRP | TCGA-AL-3472-01 | 91 | 4 |
| KIRP | TCGA-AL-3473-01 | 104 | 2 |
| KIRP | TCGA-AL-7173-01 | 85 | 11 |
| KIRP | TCGA-AT-A5NU-01 | 115 | 4 |
| KIRP | TCGA-B1-5398-01 | 142 | 6 |
| KIRP | TCGA-B1-A47M-01 | 123 | 4 |
| KIRP | TCGA-B1-A47N-01 | 63 | 1 |
| KIRP | TCGA-B1-A654-01 | 95 | 4 |
| KIRP | TCGA-B1-A655-01 | 128 | 5 |
| KIRP | TCGA-B1-A656-01 | 172 | 10 |
| KIRP | TCGA-B1-A657-01 | 116 | 7 |
| KIRP | TCGA-B3-3925-01 | 94 | 4 |
| KIRP | TCGA-B3-3926-01 | 41 | 2 |
| KIRP | TCGA-B3-4103-01 | 83 | 2 |
| KIRP | TCGA-B3-4104-01 | 124 | 9 |
| KIRP | TCGA-B3-8121-01 | 71 | 1 |
| KIRP | TCGA-B9-4113-01 | 104 | 9 |
| KIRP | TCGA-B9-4114-01 | 135 | 9 |
| KIRP | TCGA-B9-4115-01 | 99 | 4 |
| KIRP | TCGA-B9-4116-01 | 137 | 8 |
| KIRP | TCGA-B9-4117-01 | 363 | 18 |
| KIRP | TCGA-B9-5156-01 | 112 | 9 |
| KIRP | TCGA-B9-7268-01 | 78 | 3 |
| KIRP | TCGA-B9-A44B-01 | 185 | 12 |
| KIRP | TCGA-B9-A5W7-01 | 46 | 1 |
| KIRP | TCGA-B9-A5W8-01 | 99 | 5 |
| KIRP | TCGA-B9-A5W9-01 | 102 | 2 |
| KIRP | TCGA-B9-A69E-01 | 111 | 5 |
| KIRP | TCGA-BQ-5875-01 | 101 | 7 |
| KIRP | TCGA-BQ-5876-01 | 118 | 7 |
| KIRP | TCGA-BQ-5877-01 | 114 | 8 |
| KIRP | TCGA-BQ-5878-01 | 136 | 4 |
| KIRP | TCGA-BQ-5879-01 | 44 | 1 |
| KIRP | TCGA-BQ-5880-01 | 83 | 3 |
| KIRP | TCGA-BQ-5881-01 | 62 | 3 |
| KIRP | TCGA-BQ-5882-01 | 99 | 3 |
| KIRP | TCGA-BQ-5883-01 | 57 | 3 |
| KIRP | TCGA-BQ-5884-01 | 82 | 4 |
| KIRP | TCGA-BQ-5885-01 | 148 | 6 |
| KIRP | TCGA-BQ-5886-01 | 94 | 3 |
| KIRP | TCGA-BQ-5887-01 | 34 | 2 |
| KIRP | TCGA-BQ-5888-01 | 45 | 0 |
| KIRP | TCGA-BQ-5889-01 | 95 | 3 |
| KIRP | TCGA-BQ-5890-01 | 108 | 8 |
| KIRP | TCGA-BQ-5891-01 | 62 | 4 |
| KIRP | TCGA-BQ-5892-01 | 91 | 3 |
| KIRP | TCGA-BQ-5893-01 | 93 | 6 |
| KIRP | TCGA-BQ-5894-01 | 59 | 2 |
| KIRP | TCGA-BQ-7044-01 | 98 | 6 |
| KIRP | TCGA-BQ-7045-01 | 105 | 4 |
| KIRP | TCGA-BQ-7046-01 | 57 | 3 |
| KIRP | TCGA-BQ-7048-01 | 93 | 4 |
| KIRP | TCGA-BQ-7050-01 | 34 | 2 |
| KIRP | TCGA-BQ-7051-01 | 111 | 5 |
| KIRP | TCGA-BQ-7053-01 | 81 | 4 |
| KIRP | TCGA-BQ-7055-01 | 19 | 0 |
| KIRP | TCGA-BQ-7058-01 | 89 | 5 |
| KIRP | TCGA-BQ-7059-01 | 104 | 4 |
| KIRP | TCGA-BQ-7060-01 | 48 | 4 |
| KIRP | TCGA-BQ-7061-01 | 103 | 8 |
| KIRP | TCGA-BQ-7062-01 | 63 | 1 |
| KIRP | TCGA-DW-5560-01 | 81 | 2 |
| KIRP | TCGA-DW-5561-01 | 63 | 1 |
| KIRP | TCGA-DW-7834-01 | 94 | 2 |
| KIRP | TCGA-DW-7837-01 | 47 | 4 |
| KIRP | TCGA-DW-7838-01 | 129 | 4 |
| KIRP | TCGA-DW-7839-01 | 61 | 2 |
| KIRP | TCGA-DW-7840-01 | 123 | 11 |
| KIRP | TCGA-DW-7841-01 | 71 | 3 |
| KIRP | TCGA-DW-7842-01 | 46 | 2 |
| KIRP | TCGA-DW-7963-01 | 66 | 1 |
| KIRP | TCGA-DZ-6131-01 | 44 | 0 |
| KIRP | TCGA-DZ-6132-01 | 144 | 5 |
| KIRP | TCGA-DZ-6133-01 | 126 | 6 |
| KIRP | TCGA-DZ-6134-01 | 63 | 1 |
| KIRP | TCGA-DZ-6135-01 | 47 | 3 |
| KIRP | TCGA-EV-5901-01 | 57 | 4 |
| KIRP | TCGA-EV-5902-01 | 116 | 5 |
| KIRP | TCGA-EV-5903-01 | 142 | 6 |
| KIRP | TCGA-F9-A4JJ-01 | 58 | 2 |
| KIRP | TCGA-G7-6789-01 | 38 | 1 |
| KIRP | TCGA-G7-6790-01 | 90 | 5 |
| KIRP | TCGA-G7-6792-01 | 57 | 1 |
| KIRP | TCGA-G7-6793-01 | 50 | 1 |
| KIRP | TCGA-G7-6795-01 | 85 | 1 |
| KIRP | TCGA-G7-6796-01 | 76 | 4 |
| KIRP | TCGA-G7-6797-01 | 83 | 3 |
| KIRP | TCGA-G7-7501-01 | 56 | 0 |
| KIRP | TCGA-G7-7502-01 | 100 | 2 |
| KIRP | TCGA-G7-A4TM-01 | 58 | 3 |
| KIRP | TCGA-GL-6846-01 | 96 | 4 |
| KIRP | TCGA-GL-7773-01 | 155 | 4 |
| KIRP | TCGA-GL-7966-01 | 65 | 3 |
| KIRP | TCGA-GL-8500-01 | 126 | 4 |
| KIRP | TCGA-GL-A4EM-01 | 102 | 7 |
| KIRP | TCGA-GL-A59R-01 | 117 | 8 |
| KIRP | TCGA-GL-A59T-01 | 125 | 8 |
| KIRP | TCGA-HE-7128-01 | 60 | 1 |
| KIRP | TCGA-HE-7129-01 | 67 | 1 |
| KIRP | TCGA-HE-7130-01 | 234 | 11 |
| KIRP | TCGA-HE-A5NF-01 | 93 | 4 |
| KIRP | TCGA-HE-A5NH-01 | 104 | 3 |
| KIRP | TCGA-HE-A5NI-01 | 138 | 8 |
| KIRP | TCGA-HE-A5NJ-01 | 89 | 5 |
| KIRP | TCGA-HE-A5NK-01 | 131 | 5 |
| KIRP | TCGA-HE-A5NL-01 | 106 | 2 |
| KIRP | TCGA-IA-A40U-01 | 66 | 4 |
| KIRP | TCGA-IA-A40X-01 | 44 | 2 |
| KIRP | TCGA-IA-A40Y-01 | 106 | 6 |
| KIRP | TCGA-IZ-8195-01 | 107 | 1 |
| KIRP | TCGA-IZ-8196-01 | 139 | 7 |
| KIRP | TCGA-IZ-A6M8-01 | 115 | 6 |
| KIRP | TCGA-IZ-A6M9-01 | 121 | 1 |
| KIRP | TCGA-J7-6720-01 | 65 | 0 |
| KIRP | TCGA-J7-8537-01 | 77 | 5 |
| KIRP | TCGA-KV-A6GD-01 | 102 | 5 |
| KIRP | TCGA-KV-A6GE-01 | 88 | 3 |
| KIRP | TCGA-MH-A55W-01 | 124 | 6 |
| KIRP | TCGA-MH-A55Z-01 | 130 | 5 |
| KIRP | TCGA-MH-A560-01 | 85 | 2 |
| KIRP | TCGA-MH-A561-01 | 150 | 2 |
| KIRP | TCGA-MH-A562-01 | 93 | 4 |
| KIRP | TCGA-P4-A5E6-01 | 133 | 3 |
| KIRP | TCGA-P4-A5E7-01 | 139 | 6 |
| KIRP | TCGA-P4-A5E8-01 | 75 | 4 |
| KIRP | TCGA-P4-A5EA-01 | 123 | 8 |
| KIRP | TCGA-P4-A5EB-01 | 207 | 10 |
| KIRP | TCGA-P4-A5ED-01 | 54 | 2 |
| KIRP | TCGA-PJ-A5Z8-01 | 107 | 4 |
| KIRP | TCGA-PJ-A5Z9-01 | 94 | 4 |

## TABLE 5 (APPENDIX A)

| Type | Sample | Value 1 | Value 2 |
|---|---|---|---|
| KIRP | TCGA-Q2-A5QZ-01 | 170 | 3 |
| LAML | TCGA-AB-2802-03 | 15 | 5 |
| LAML | TCGA-AB-2803-03 | 18 | 1 |
| LAML | TCGA-AB-2804-03 | 10 | 0 |
| LAML | TCGA-AB-2805-03 | 18 | 3 |
| LAML | TCGA-AB-2806-03 | 19 | 1 |
| LAML | TCGA-AB-2807-03 | 35 | 5 |
| LAML | TCGA-AB-2808-03 | 11 | 2 |
| LAML | TCGA-AB-2809-03 | 5 | 2 |
| LAML | TCGA-AB-2810-03 | 16 | 2 |
| LAML | TCGA-AB-2811-03 | 10 | 4 |
| LAML | TCGA-AB-2812-03 | 11 | 3 |
| LAML | TCGA-AB-2813-03 | 18 | 2 |
| LAML | TCGA-AB-2814-03 | 14 | 3 |
| LAML | TCGA-AB-2816-03 | 9 | 4 |
| LAML | TCGA-AB-2817-03 | 17 | 3 |
| LAML | TCGA-AB-2818-03 | 16 | 3 |
| LAML | TCGA-AB-2819-03 | 20 | 3 |
| LAML | TCGA-AB-2820-03 | 20 | 1 |
| LAML | TCGA-AB-2821-03 | 15 | 7 |
| LAML | TCGA-AB-2822-03 | 24 | 4 |
| LAML | TCGA-AB-2823-03 | 1 | 0 |
| LAML | TCGA-AB-2824-03 | 5 | 2 |
| LAML | TCGA-AB-2825-03 | 3 | 3 |
| LAML | TCGA-AB-2826-03 | 6 | 4 |
| LAML | TCGA-AB-2827-03 | 14 | 1 |
| LAML | TCGA-AB-2828-03 | 13 | 0 |
| LAML | TCGA-AB-2829-03 | 10 | 3 |
| LAML | TCGA-AB-2830-03 | 18 | 4 |
| LAML | TCGA-AB-2831-03 | 4 | 1 |
| LAML | TCGA-AB-2832-03 | 12 | 0 |
| LAML | TCGA-AB-2833-03 | 10 | 2 |
| LAML | TCGA-AB-2834-03 | 1 | 1 |
| LAML | TCGA-AB-2835-03 | 3 | 1 |
| LAML | TCGA-AB-2836-03 | 4 | 2 |
| LAML | TCGA-AB-2837-03 | 2 | 1 |
| LAML | TCGA-AB-2838-03 | 24 | 2 |
| LAML | TCGA-AB-2839-03 | 21 | 7 |
| LAML | TCGA-AB-2840-03 | 1 | 1 |
| LAML | TCGA-AB-2841-03 | 7 | 0 |
| LAML | TCGA-AB-2842-03 | 2 | 0 |
| LAML | TCGA-AB-2843-03 | 12 | 3 |
| LAML | TCGA-AB-2844-03 | 17 | 3 |
| LAML | TCGA-AB-2845-03 | 7 | 4 |
| LAML | TCGA-AB-2846-03 | 18 | 3 |
| LAML | TCGA-AB-2847-03 | 11 | 2 |
| LAML | TCGA-AB-2848-03 | 1 | 1 |
| LAML | TCGA-AB-2849-03 | 28 | 1 |
| LAML | TCGA-AB-2850-03 | 6 | 4 |
| LAML | TCGA-AB-2851-03 | 8 | 2 |
| LAML | TCGA-AB-2853-03 | 13 | 3 |
| LAML | TCGA-AB-2854-03 | 12 | 0 |
| LAML | TCGA-AB-2855-03 | 6 | 1 |
| LAML | TCGA-AB-2857-03 | 16 | 2 |
| LAML | TCGA-AB-2858-03 | 15 | 0 |
| LAML | TCGA-AB-2859-03 | 17 | 2 |
| LAML | TCGA-AB-2860-03 | 15 | 1 |
| LAML | TCGA-AB-2861-03 | 22 | 4 |
| LAML | TCGA-AB-2862-03 | 14 | 0 |
| LAML | TCGA-AB-2863-03 | 18 | 4 |
| LAML | TCGA-AB-2864-03 | 18 | 4 |
| LAML | TCGA-AB-2865-03 | 22 | 9 |
| LAML | TCGA-AB-2866-03 | 1 | 1 |
| LAML | TCGA-AB-2867-03 | 13 | 4 |
| LAML | TCGA-AB-2868-03 | 18 | 2 |
| LAML | TCGA-AB-2869-03 | 15 | 3 |
| LAML | TCGA-AB-2870-03 | 12 | 2 |
| LAML | TCGA-AB-2871-03 | 16 | 4 |
| LAML | TCGA-AB-2872-03 | 14 | 0 |
| LAML | TCGA-AB-2873-03 | 3 | 2 |
| LAML | TCGA-AB-2874-03 | 16 | 3 |
| LAML | TCGA-AB-2875-03 | 11 | 1 |
| LAML | TCGA-AB-2876-03 | 13 | 1 |
| LAML | TCGA-AB-2877-03 | 24 | 3 |
| LAML | TCGA-AB-2878-03 | 16 | 4 |
| LAML | TCGA-AB-2879-03 | 5 | 4 |
| LAML | TCGA-AB-2880-03 | 3 | 2 |
| LAML | TCGA-AB-2881-03 | 9 | 2 |
| LAML | TCGA-AB-2882-03 | 18 | 1 |
| LAML | TCGA-AB-2883-03 | 1 | 1 |
| LAML | TCGA-AB-2884-03 | 6 | 4 |
| LAML | TCGA-AB-2885-03 | 14 | 4 |
| LAML | TCGA-AB-2886-03 | 17 | 1 |
| LAML | TCGA-AB-2887-03 | 16 | 4 |
| LAML | TCGA-AB-2888-03 | 10 | 2 |
| LAML | TCGA-AB-2889-03 | 6 | 0 |
| LAML | TCGA-AB-2890-03 | 9 | 2 |
| LAML | TCGA-AB-2891-03 | 16 | 4 |
| LAML | TCGA-AB-2892-03 | 3 | 1 |
| LAML | TCGA-AB-2893-03 | 2 | 0 |
| LAML | TCGA-AB-2894-03 | 5 | 0 |
| LAML | TCGA-AB-2895-03 | 17 | 3 |
| LAML | TCGA-AB-2896-03 | 3 | 2 |
| LAML | TCGA-AB-2897-03 | 8 | 0 |
| LAML | TCGA-AB-2898-03 | 19 | 3 |
| LAML | TCGA-AB-2899-03 | 17 | 1 |
| LAML | TCGA-AB-2900-03 | 22 | 5 |
| LAML | TCGA-AB-2901-03 | 9 | 1 |
| LAML | TCGA-AB-2903-03 | 3 | 1 |
| LAML | TCGA-AB-2904-03 | 26 | 1 |
| LAML | TCGA-AB-2905-03 | 23 | 2 |
| LAML | TCGA-AB-2906-03 | 16 | 4 |
| LAML | TCGA-AB-2907-03 | 16 | 3 |
| LAML | TCGA-AB-2908-03 | 23 | 6 |
| LAML | TCGA-AB-2909-03 | 1 | 1 |
| LAML | TCGA-AB-2910-03 | 15 | 2 |
| LAML | TCGA-AB-2911-03 | 4 | 0 |
| LAML | TCGA-AB-2912-03 | 24 | 4 |
| LAML | TCGA-AB-2913-03 | 18 | 4 |
| LAML | TCGA-AB-2914-03 | 20 | 2 |
| LAML | TCGA-AB-2915-03 | 28 | 4 |
| LAML | TCGA-AB-2916-03 | 15 | 1 |
| LAML | TCGA-AB-2917-03 | 17 | 2 |
| LAML | TCGA-AB-2918-03 | 2 | 1 |
| LAML | TCGA-AB-2919-03 | 14 | 4 |
| LAML | TCGA-AB-2920-03 | 13 | 1 |
| LAML | TCGA-AB-2921-03 | 11 | 1 |
| LAML | TCGA-AB-2922-03 | 16 | 1 |
| LAML | TCGA-AB-2923-03 | 24 | 3 |
| LAML | TCGA-AB-2924-03 | 12 | 2 |
| LAML | TCGA-AB-2925-03 | 16 | 5 |
| LAML | TCGA-AB-2926-03 | 16 | 2 |
| LAML | TCGA-AB-2927-03 | 30 | 6 |
| LAML | TCGA-AB-2928-03 | 11 | 6 |
| LAML | TCGA-AB-2929-03 | 18 | 2 |
| LAML | TCGA-AB-2930-03 | 13 | 3 |
| LAML | TCGA-AB-2931-03 | 13 | 3 |
| LAML | TCGA-AB-2932-03 | 8 | 3 |
| LAML | TCGA-AB-2933-03 | 2 | 1 |
| LAML | TCGA-AB-2934-03 | 13 | 4 |
| LAML | TCGA-AB-2935-03 | 13 | 1 |
| LAML | TCGA-AB-2936-03 | 11 | 2 |
| LAML | TCGA-AB-2937-03 | 14 | 2 |
| LAML | TCGA-AB-2938-03 | 24 | 4 |
| LAML | TCGA-AB-2939-03 | 22 | 1 |
| LAML | TCGA-AB-2940-03 | 5 | 3 |
| LAML | TCGA-AB-2941-03 | 10 | 1 |
| LAML | TCGA-AB-2942-03 | 2 | 1 |
| LAML | TCGA-AB-2943-03 | 19 | 3 |
| LAML | TCGA-AB-2945-03 | 13 | 5 |
| LAML | TCGA-AB-2946-03 | 3 | 0 |
| LAML | TCGA-AB-2947-03 | 5 | 3 |
| LAML | TCGA-AB-2948-03 | 3 | 1 |
| LAML | TCGA-AB-2949-03 | 17 | 3 |
| LAML | TCGA-AB-2950-03 | 13 | 0 |
| LAML | TCGA-AB-2952-03 | 16 | 5 |
| LAML | TCGA-AB-2954-03 | 1 | 0 |
| LAML | TCGA-AB-2955-03 | 22 | 4 |
| LAML | TCGA-AB-2956-03 | 4 | 1 |
| LAML | TCGA-AB-2957-03 | 3 | 1 |
| LAML | TCGA-AB-2959-03 | 29 | 4 |
| LAML | TCGA-AB-2963-03 | 20 | 3 |
| LAML | TCGA-AB-2964-03 | 20 | 3 |
| LAML | TCGA-AB-2965-03 | 11 | 4 |
| LAML | TCGA-AB-2966-03 | 23 | 4 |
| LAML | TCGA-AB-2967-03 | 12 | 3 |
| LAML | TCGA-AB-2968-03 | 20 | 6 |
| LAML | TCGA-AB-2969-03 | 11 | 6 |
| LAML | TCGA-AB-2970-03 | 12 | 4 |
| LAML | TCGA-AB-2971-03 | 14 | 2 |
| LAML | TCGA-AB-2972-03 | 27 | 4 |
| LAML | TCGA-AB-2973-03 | 7 | 3 |
| LAML | TCGA-AB-2974-03 | 9 | 3 |
| LAML | TCGA-AB-2975-03 | 2 | 1 |
| LAML | TCGA-AB-2976-03 | 25 | 4 |
| LAML | TCGA-AB-2977-03 | 12 | 1 |
| LAML | TCGA-AB-2978-03 | 19 | 6 |
| LAML | TCGA-AB-2979-03 | 11 | 2 |
| LAML | TCGA-AB-2980-03 | 7 | 1 |
| LAML | TCGA-AB-2981-03 | 8 | 3 |
| LAML | TCGA-AB-2982-03 | 5 | 0 |
| LAML | TCGA-AB-2983-03 | 15 | 1 |
| LAML | TCGA-AB-2984-03 | 12 | 3 |
| LAML | TCGA-AB-2985-03 | 6 | 1 |
| LAML | TCGA-AB-2986-03 | 16 | 2 |
| LAML | TCGA-AB-2987-03 | 7 | 4 |
| LAML | TCGA-AB-2988-03 | 19 | 3 |
| LAML | TCGA-AB-2989-03 | 13 | 3 |
| LAML | TCGA-AB-2990-03 | 9 | 2 |
| LAML | TCGA-AB-2991-03 | 12 | 1 |
| LAML | TCGA-AB-2992-03 | 9 | 3 |
| LAML | TCGA-AB-2993-03 | 17 | 3 |
| LAML | TCGA-AB-2994-03 | 13 | 1 |
| LAML | TCGA-AB-2995-03 | 7 | 0 |
| LAML | TCGA-AB-2996-03 | 21 | 4 |
| LAML | TCGA-AB-2997-03 | 16 | 2 |
| LAML | TCGA-AB-2998-03 | 10 | 1 |
| LAML | TCGA-AB-2999-03 | 12 | 2 |
| LAML | TCGA-AB-3000-03 | 6 | 2 |
| LAML | TCGA-AB-3001-03 | 13 | 0 |
| LAML | TCGA-AB-3002-03 | 36 | 1 |
| LAML | TCGA-AB-3005-03 | 23 | 1 |
| LAML | TCGA-AB-3006-03 | 20 | 3 |
| LAML | TCGA-AB-3007-03 | 10 | 1 |
| LAML | TCGA-AB-3008-03 | 6 | 1 |
| LAML | TCGA-AB-3009-03 | 51 | 5 |
| LAML | TCGA-AB-3011-03 | 7 | 2 |
| LAML | TCGA-AB-3012-03 | 9 | 0 |
| LGG | TCGA-CS-4938-01 | 18 | 3 |
| LGG | TCGA-CS-4941-01 | 51 | 2 |
| LGG | TCGA-CS-4942-01 | 25 | 6 |
| LGG | TCGA-CS-4943-01 | 31 | 7 |
| LGG | TCGA-CS-4944-01 | 22 | 3 |
| LGG | TCGA-CS-5390-01 | 36 | 5 |
| LGG | TCGA-CS-5393-01 | 28 | 4 |
| LGG | TCGA-CS-5394-01 | 25 | 6 |
| LGG | TCGA-CS-5395-01 | 40 | 3 |
| LGG | TCGA-CS-5396-01 | 30 | 6 |
| LGG | TCGA-CS-5397-01 | 46 | 7 |
| LGG | TCGA-CS-6186-01 | 59 | 0 |
| LGG | TCGA-CS-6188-01 | 49 | 2 |
| LGG | TCGA-CS-6290-01 | 20 | 2 |
| LGG | TCGA-CS-6665-01 | 79 | 2 |
| LGG | TCGA-CS-6666-01 | 28 | 5 |
| LGG | TCGA-CS-6667-01 | 27 | 5 |
| LGG | TCGA-CS-6668-01 | 24 | 4 |
| LGG | TCGA-DB-5273-01 | 17 | 3 |
| LGG | TCGA-DB-5274-01 | 44 | 3 |
| LGG | TCGA-DB-5275-01 | 32 | 5 |
| LGG | TCGA-DB-5276-01 | 16 | 3 |
| LGG | TCGA-DB-5277-01 | 43 | 3 |
| LGG | TCGA-DB-5278-01 | 7 | 3 |
| LGG | TCGA-DB-5279-01 | 55 | 3 |
| LGG | TCGA-DB-5280-01 | 22 | 5 |
| LGG | TCGA-DB-5281-01 | 53 | 3 |
| LGG | TCGA-DB-A4X9-01 | 27 | 6 |
| LGG | TCGA-DB-A4XA-01 | 9 | 4 |
| LGG | TCGA-DB-A4XB-01 | 31 | 5 |
| LGG | TCGA-DB-A4XC-01 | 17 | 3 |
| LGG | TCGA-DB-A4XD-01 | 34 | 7 |
| LGG | TCGA-DB-A4XE-01 | 28 | 6 |
| LGG | TCGA-DB-A4XF-01 | 23 | 2 |
| LGG | TCGA-DB-A4XG-01 | 23 | 3 |
| LGG | TCGA-DB-A4XH-01 | 44 | 3 |
| LGG | TCGA-DB-A64L-01 | 77 | 8 |
| LGG | TCGA-DB-A64O-01 | 21 | 3 |
| LGG | TCGA-DB-A64P-01 | 24 | 3 |
| LGG | TCGA-DB-A64Q-01 | 20 | 4 |
| LGG | TCGA-DB-A64R-01 | 14 | 2 |
| LGG | TCGA-DB-A64S-01 | 11 | 5 |
| LGG | TCGA-DB-A64U-01 | 12 | 2 |
| LGG | TCGA-DB-A64V-01 | 27 | 5 |
| LGG | TCGA-DB-A64W-01 | 49 | 5 |
| LGG | TCGA-DB-A64X-01 | 76 | 7 |
| LGG | TCGA-DH-5140-01 | 29 | 5 |
| LGG | TCGA-DH-5141-01 | 26 | 4 |
| LGG | TCGA-DH-5142-01 | 35 | 4 |
| LGG | TCGA-DH-5143-01 | 28 | 3 |
| LGG | TCGA-DH-5144-01 | 36 | 6 |
| LGG | TCGA-DH-A66B-01 | 45 | 5 |
| LGG | TCGA-DH-A66F-01 | 18 | 3 |
| LGG | TCGA-DU-5847-01 | 40 | 5 |
| LGG | TCGA-DU-5849-01 | 35 | 5 |
| LGG | TCGA-DU-5851-01 | 28 | 3 |
| LGG | TCGA-DU-5852-01 | 76 | 6 |
| LGG | TCGA-DU-5853-01 | 18 | 4 |
| LGG | TCGA-DU-5854-01 | 51 | 1 |
| LGG | TCGA-DU-5855-01 | 53 | 5 |
| LGG | TCGA-DU-5870-01 | 18 | 3 |
| LGG | TCGA-DU-5871-01 | 27 | 4 |
| LGG | TCGA-DU-5872-01 | 30 | 4 |
| LGG | TCGA-DU-5874-01 | 43 | 4 |
| LGG | TCGA-DU-6393-01 | 32 | 3 |
| LGG | TCGA-DU-6394-01 | 39 | 10 |

## TABLE 5 (APPENDIX A)

| | | | | | | | | | | | |
|---|---|---|---|---|---|---|---|---|---|---|---|
| LGG | TCGA-DU-6395-01 | 26 | 5 | LGG | TCGA-FG-A60J-01 | 38 | 3 | LGG | TCGA-HT-A5R9-01 | 38 | 3 |
| LGG | TCGA-DU-6396-01 | 43 | 4 | LGG | TCGA-FG-A60K-01 | 19 | 4 | LGG | TCGA-HT-A5RA-01 | 61 | 3 |
| LGG | TCGA-DU-6397-01 | 27 | 6 | LGG | TCGA-FN-7833-01 | 20 | 5 | LGG | TCGA-HT-A5RB-01 | 19 | 4 |
| LGG | TCGA-DU-6399-01 | 51 | 6 | LGG | TCGA-HT-7467-01 | 28 | 3 | LGG | TCGA-HT-A5RC-01 | 59 | 1 |
| LGG | TCGA-DU-6400-01 | 51 | 4 | LGG | TCGA-HT-7468-01 | 15 | 5 | LGG | TCGA-HT-A614-01 | 28 | 4 |
| LGG | TCGA-DU-6401-01 | 24 | 5 | LGG | TCGA-HT-7469-01 | 39 | 7 | LGG | TCGA-HT-A615-01 | 37 | 6 |
| LGG | TCGA-DU-6402-01 | 49 | 5 | LGG | TCGA-HT-7470-01 | 43 | 6 | LGG | TCGA-HT-A616-01 | 23 | 2 |
| LGG | TCGA-DU-6403-01 | 60 | 4 | LGG | TCGA-HT-7471-01 | 22 | 4 | LGG | TCGA-HT-A617-01 | 22 | 1 |
| LGG | TCGA-DU-6404-01 | 14 | 0 | LGG | TCGA-HT-7472-01 | 21 | 4 | LGG | TCGA-HT-A618-01 | 24 | 5 |
| LGG | TCGA-DU-6405-01 | 58 | 2 | LGG | TCGA-HT-7473-01 | 17 | 3 | LGG | TCGA-HT-A619-01 | 60 | 4 |
| LGG | TCGA-DU-6407-01 | 23 | 6 | LGG | TCGA-HT-7474-01 | 23 | 3 | LGG | TCGA-HT-A61A-01 | 8 | 2 |
| LGG | TCGA-DU-6408-01 | 21 | 6 | LGG | TCGA-HT-7475-01 | 54 | 7 | LGG | TCGA-HT-A61B-01 | 42 | 3 |
| LGG | TCGA-DU-6410-01 | 44 | 3 | LGG | TCGA-HT-7476-01 | 18 | 3 | LGG | TCGA-HT-A61C-01 | 44 | 3 |
| LGG | TCGA-DU-6542-01 | 25 | 3 | LGG | TCGA-HT-7477-01 | 47 | 5 | LGG | TCGA-HW-7486-01 | 13 | 2 |
| LGG | TCGA-DU-7006-01 | 60 | 5 | LGG | TCGA-HT-7478-01 | 27 | 6 | LGG | TCGA-HW-7487-01 | 21 | 4 |
| LGG | TCGA-DU-7007-01 | 39 | 6 | LGG | TCGA-HT-7479-01 | 22 | 2 | LGG | TCGA-HW-7489-01 | 19 | 3 |
| LGG | TCGA-DU-7008-01 | 31 | 7 | LGG | TCGA-HT-7480-01 | 27 | 2 | LGG | TCGA-HW-7490-01 | 45 | 5 |
| LGG | TCGA-DU-7009-01 | 16 | 2 | LGG | TCGA-HT-7481-01 | 29 | 8 | LGG | TCGA-HW-7491-01 | 16 | 3 |
| LGG | TCGA-DU-7010-01 | 105 | 7 | LGG | TCGA-HT-7482-01 | 16 | 5 | LGG | TCGA-HW-7495-01 | 15 | 1 |
| LGG | TCGA-DU-7012-01 | 61 | 3 | LGG | TCGA-HT-7483-01 | 16 | 3 | LGG | TCGA-HW-8319-01 | 36 | 4 |
| LGG | TCGA-DU-7013-01 | 40 | 2 | LGG | TCGA-HT-7485-01 | 14 | 4 | LGG | TCGA-HW-8320-01 | 30 | 2 |
| LGG | TCGA-DU-7015-01 | 31 | 3 | LGG | TCGA-HT-7601-01 | 23 | 3 | LGG | TCGA-HW-8321-01 | 32 | 4 |
| LGG | TCGA-DU-7018-01 | 38 | 6 | LGG | TCGA-HT-7602-01 | 10 | 3 | LGG | TCGA-HW-8322-01 | 21 | 4 |
| LGG | TCGA-DU-7019-01 | 34 | 3 | LGG | TCGA-HT-7603-01 | 29 | 3 | LGG | TCGA-HW-A5KJ-01 | 46 | 5 |
| LGG | TCGA-DU-7290-01 | 36 | 5 | LGG | TCGA-HT-7604-01 | 50 | 6 | LGG | TCGA-HW-A5KK-01 | 34 | 1 |
| LGG | TCGA-DU-7292-01 | 47 | 1 | LGG | TCGA-HT-7605-01 | 26 | 2 | LGG | TCGA-HW-A5KL-01 | 18 | 4 |
| LGG | TCGA-DU-7294-01 | 31 | 5 | LGG | TCGA-HT-7606-01 | 34 | 3 | LGG | TCGA-HW-A5KM-01 | 16 | 2 |
| LGG | TCGA-DU-7298-01 | 34 | 7 | LGG | TCGA-HT-7607-01 | 30 | 3 | LGG | TCGA-IK-7675-01 | 44 | 4 |
| LGG | TCGA-DU-7299-01 | 29 | 4 | LGG | TCGA-HT-7608-01 | 21 | 5 | LGG | TCGA-IK-8125-01 | 55 | 9 |
| LGG | TCGA-DU-7300-01 | 53 | 6 | LGG | TCGA-HT-7609-01 | 29 | 3 | LGG | TCGA-P5-A5ET-01 | 23 | 3 |
| LGG | TCGA-DU-7301-01 | 30 | 5 | LGG | TCGA-HT-7610-01 | 17 | 7 | LGG | TCGA-P5-A5EU-01 | 27 | 4 |
| LGG | TCGA-DU-7302-01 | 32 | 4 | LGG | TCGA-HT-7611-01 | 33 | 7 | LGG | TCGA-P5-A5EV-01 | 84 | 4 |
| LGG | TCGA-DU-7304-01 | 30 | 6 | LGG | TCGA-HT-7616-01 | 48 | 4 | LGG | TCGA-P5-A5EW-01 | 16 | 3 |
| LGG | TCGA-DU-7306-01 | 56 | 3 | LGG | TCGA-HT-7620-01 | 21 | 3 | LGG | TCGA-P5-A5EX-01 | 25 | 2 |
| LGG | TCGA-DU-7309-01 | 33 | 4 | LGG | TCGA-HT-7676-01 | 17 | 3 | LGG | TCGA-P5-A5EY-01 | 1 | 0 |
| LGG | TCGA-DU-8158-01 | 39 | 5 | LGG | TCGA-HT-7677-01 | 37 | 4 | LGG | TCGA-P5-A5EZ-01 | 36 | 2 |
| LGG | TCGA-DU-8161-01 | 46 | 4 | LGG | TCGA-HT-7680-01 | 3 | 0 | LGG | TCGA-P5-A5F0-01 | 30 | 6 |
| LGG | TCGA-DU-8162-01 | 28 | 1 | LGG | TCGA-HT-7681-01 | 15 | 3 | LGG | TCGA-P5-A5F1-01 | 18 | 4 |
| LGG | TCGA-DU-8163-01 | 17 | 5 | LGG | TCGA-HT-7684-01 | 34 | 3 | LGG | TCGA-P5-A5F2-01 | 26 | 5 |
| LGG | TCGA-DU-8164-01 | 31 | 5 | LGG | TCGA-HT-7686-01 | 19 | 3 | LGG | TCGA-P5-A5F4-01 | 35 | 4 |
| LGG | TCGA-DU-8165-01 | 72 | 4 | LGG | TCGA-HT-7687-01 | 42 | 1 | LGG | TCGA-P5-A5F6-01 | 1 | 0 |
| LGG | TCGA-DU-8166-01 | 31 | 5 | LGG | TCGA-HT-7688-01 | 74 | 8 | LGG | TCGA-QH-A65S-01 | 22 | 3 |
| LGG | TCGA-DU-8167-01 | 60 | 5 | LGG | TCGA-HT-7689-01 | 52 | 4 | LGG | TCGA-QH-A65V-01 | 24 | 3 |
| LGG | TCGA-DU-8168-01 | 65 | 9 | LGG | TCGA-HT-7690-01 | 20 | 6 | LGG | TCGA-QH-A65Z-01 | 29 | 4 |
| LGG | TCGA-DU-A5TP-01 | 29 | 3 | LGG | TCGA-HT-7691-01 | 9 | 0 | LIHC | TCGA-BC-4073-01 | 175 | 5 |
| LGG | TCGA-DU-A5TR-01 | 36 | 5 | LGG | TCGA-HT-7692-01 | 24 | 3 | LIHC | TCGA-BC-A10Q-01 | 61 | 6 |
| LGG | TCGA-DU-A5TS-01 | 41 | 4 | LGG | TCGA-HT-7693-01 | 31 | 5 | LIHC | TCGA-BC-A10R-01 | 148 | 4 |
| LGG | TCGA-DU-A5TT-01 | 57 | 5 | LGG | TCGA-HT-7694-01 | 35 | 4 | LIHC | TCGA-BC-A10T-01 | 95 | 4 |
| LGG | TCGA-DU-A5TU-01 | 43 | 5 | LGG | TCGA-HT-7695-01 | 25 | 2 | LIHC | TCGA-BC-A10U-01 | 163 | 10 |
| LGG | TCGA-DU-A5TW-01 | 48 | 3 | LGG | TCGA-HT-7854-01 | 29 | 2 | LIHC | TCGA-BC-A10W-01 | 191 | 12 |
| LGG | TCGA-DU-A5TY-01 | 55 | 2 | LGG | TCGA-HT-7855-01 | 36 | 4 | LIHC | TCGA-BC-A10X-01 | 12 | 1 |
| LGG | TCGA-E1-5302-01 | 35 | 3 | LGG | TCGA-HT-7856-01 | 11 | 2 | LIHC | TCGA-BC-A10Y-01 | 90 | 4 |
| LGG | TCGA-E1-5303-01 | 28 | 4 | LGG | TCGA-HT-7857-01 | 20 | 4 | LIHC | TCGA-BC-A10Z-01 | 204 | 11 |
| LGG | TCGA-E1-5304-01 | 33 | 4 | LGG | TCGA-HT-7858-01 | 17 | 3 | LIHC | TCGA-BC-A110-01 | 30 | 1 |
| LGG | TCGA-E1-5305-01 | 25 | 3 | LGG | TCGA-HT-7860-01 | 86 | 9 | LIHC | TCGA-BC-A112-01 | 318 | 18 |
| LGG | TCGA-E1-5307-01 | 69 | 5 | LGG | TCGA-HT-7873-01 | 29 | 3 | LIHC | TCGA-BC-A216-01 | 93 | 4 |
| LGG | TCGA-E1-5311-01 | 17 | 3 | LGG | TCGA-HT-7874-01 | 24 | 3 | LIHC | TCGA-BC-A217-01 | 136 | 5 |
| LGG | TCGA-E1-5318-01 | 36 | 5 | LGG | TCGA-HT-7875-01 | 36 | 5 | LIHC | TCGA-BC-A3KF-01 | 185 | 9 |
| LGG | TCGA-E1-5319-01 | 38 | 4 | LGG | TCGA-HT-7877-01 | 15 | 2 | LIHC | TCGA-BC-A3KG-01 | 72 | 2 |
| LGG | TCGA-E1-5322-01 | 26 | 4 | LGG | TCGA-HT-7879-01 | 17 | 4 | LIHC | TCGA-BC-A5W4-01 | 81 | 5 |
| LGG | TCGA-EZ-7264-01 | 28 | 3 | LGG | TCGA-HT-7880-01 | 8 | 5 | LIHC | TCGA-BC-A69H-01 | 112 | 6 |
| LGG | TCGA-FG-5962-01 | 34 | 6 | LGG | TCGA-HT-7881-01 | 15 | 1 | LIHC | TCGA-BD-A2L6-01 | 87 | 3 |
| LGG | TCGA-FG-5963-01 | 26 | 5 | LGG | TCGA-HT-7882-01 | 50 | 3 | LIHC | TCGA-BD-A3EP-01 | 145 | 10 |
| LGG | TCGA-FG-5964-01 | 34 | 2 | LGG | TCGA-HT-7884-01 | 34 | 5 | LIHC | TCGA-BW-A5NO-01 | 141 | 4 |
| LGG | TCGA-FG-5965-01 | 45 | 4 | LGG | TCGA-HT-7902-01 | 20 | 4 | LIHC | TCGA-BW-A5NP-01 | 88 | 7 |
| LGG | TCGA-FG-6688-01 | 73 | 3 | LGG | TCGA-HT-8010-01 | 14 | 2 | LIHC | TCGA-BW-A5NQ-01 | 112 | 6 |
| LGG | TCGA-FG-6689-01 | 21 | 6 | LGG | TCGA-HT-8011-01 | 51 | 4 | LIHC | TCGA-CC-5258-01 | 158 | 7 |
| LGG | TCGA-FG-6690-01 | 28 | 5 | LGG | TCGA-HT-8012-01 | 23 | 6 | LIHC | TCGA-CC-5259-01 | 182 | 11 |
| LGG | TCGA-FG-6691-01 | 12 | 3 | LGG | TCGA-HT-8013-01 | 28 | 4 | LIHC | TCGA-CC-5260-01 | 73 | 4 |
| LGG | TCGA-FG-6692-01 | 88 | 5 | LGG | TCGA-HT-8015-01 | 1 | 1 | LIHC | TCGA-CC-5261-01 | 82 | 2 |
| LGG | TCGA-FG-7634-01 | 21 | 4 | LGG | TCGA-HT-8018-01 | 16 | 4 | LIHC | TCGA-CC-5262-01 | 154 | 9 |
| LGG | TCGA-FG-7636-01 | 40 | 5 | LGG | TCGA-HT-8019-01 | 1 | 0 | LIHC | TCGA-CC-5263-01 | 136 | 4 |
| LGG | TCGA-FG-7637-01 | 33 | 3 | LGG | TCGA-HT-8104-01 | 66 | 7 | LIHC | TCGA-CC-5264-01 | 129 | 4 |
| LGG | TCGA-FG-7638-01 | 17 | 5 | LGG | TCGA-HT-8105-01 | 49 | 6 | LIHC | TCGA-CC-A123-01 | 117 | 7 |
| LGG | TCGA-FG-7641-01 | 27 | 5 | LGG | TCGA-HT-8106-01 | 33 | 2 | LIHC | TCGA-CC-A1HT-01 | 122 | 10 |
| LGG | TCGA-FG-7643-01 | 55 | 3 | LGG | TCGA-HT-8108-01 | 20 | 3 | LIHC | TCGA-CC-A3M9-01 | 175 | 9 |
| LGG | TCGA-FG-8182-01 | 31 | 6 | LGG | TCGA-HT-8109-01 | 40 | 4 | LIHC | TCGA-CC-A3MA-01 | 154 | 7 |
| LGG | TCGA-FG-8185-01 | 41 | 6 | LGG | TCGA-HT-8110-01 | 36 | 3 | LIHC | TCGA-CC-A3M8-01 | 218 | 8 |
| LGG | TCGA-FG-8186-01 | 30 | 2 | LGG | TCGA-HT-8111-01 | 11 | 2 | LIHC | TCGA-CC-A3MC-01 | 118 | 3 |
| LGG | TCGA-FG-8187-01 | 14 | 2 | LGG | TCGA-HT-8113-01 | 22 | 1 | LIHC | TCGA-CC-A5UC-01 | 64 | 4 |
| LGG | TCGA-FG-8188-01 | 33 | 7 | LGG | TCGA-HT-8114-01 | 16 | 5 | LIHC | TCGA-CC-A5UD-01 | 234 | 13 |
| LGG | TCGA-FG-8189-01 | 3 | 1 | LGG | TCGA-HT-8558-01 | 1 | 0 | LIHC | TCGA-CC-A5UE-01 | 152 | 8 |
| LGG | TCGA-FG-8191-01 | 27 | 5 | LGG | TCGA-HT-8563-01 | 31 | 5 | LIHC | TCGA-CC-A7IF-01 | 142 | 6 |
| LGG | TCGA-FG-A4MT-01 | 19 | 6 | LGG | TCGA-HT-8564-01 | 597 | 22 | LIHC | TCGA-CC-A7IG-01 | 162 | 7 |
| LGG | TCGA-FG-A4MU-01 | 77 | 2 | LGG | TCGA-HT-A4DS-01 | 40 | 3 | LIHC | TCGA-CC-A7IH-01 | 874 | 33 |
| LGG | TCGA-FG-A4MW-01 | 88 | 7 | LGG | TCGA-HT-A4DV-01 | 12 | 2 | LIHC | TCGA-CC-A7II-01 | 186 | 5 |
| LGG | TCGA-FG-A4MX-01 | 21 | 3 | LGG | TCGA-HT-A5R5-01 | 26 | 5 | LIHC | TCGA-CC-A7IJ-01 | 170 | 4 |
| LGG | TCGA-FG-A4MY-01 | 28 | 5 | LGG | TCGA-HT-A5R7-01 | 21 | 3 | LIHC | TCGA-CC-A7IK-01 | 369 | 10 |

# EP 3 423 828 B1

## TABLE 5 (APPENDIX A)

| | | | |
|---|---|---|---|
| LIHC | TCGA-CC-A7IL-01 | 124 | 7 |
| LIHC | TCGA-DD-A113-01 | 205 | 8 |
| LIHC | TCGA-DD-A114-01 | 127 | 5 |
| LIHC | TCGA-DD-A115-01 | 116 | 5 |
| LIHC | TCGA-DD-A116-01 | 146 | 5 |
| LIHC | TCGA-DD-A118-01 | 150 | 7 |
| LIHC | TCGA-DD-A119-01 | 241 | 10 |
| LIHC | TCGA-DD-A11A-01 | 185 | 8 |
| LIHC | TCGA-DD-A11B-01 | 78 | 5 |
| LIHC | TCGA-DD-A11C-01 | 203 | 11 |
| LIHC | TCGA-DD-A11D-01 | 178 | 7 |
| LIHC | TCGA-DD-A1E9-01 | 91 | 5 |
| LIHC | TCGA-DD-A1EA-01 | 116 | 8 |
| LIHC | TCGA-DD-A1EB-01 | 211 | 6 |
| LIHC | TCGA-DD-A1EC-01 | 36 | 2 |
| LIHC | TCGA-DD-A1ED-01 | 91 | 6 |
| LIHC | TCGA-DD-A1EF-01 | 177 | 6 |
| LIHC | TCGA-DD-A1EG-01 | 79 | 5 |
| LIHC | TCGA-DD-A1EH-01 | 59 | 5 |
| LIHC | TCGA-DD-A1EI-01 | 86 | 3 |
| LIHC | TCGA-DD-A1EJ-01 | 83 | 3 |
| LIHC | TCGA-DD-A1EK-01 | 84 | 3 |
| LIHC | TCGA-DD-A1EL-01 | 171 | 6 |
| LIHC | TCGA-DD-A39W-01 | 95 | 7 |
| LIHC | TCGA-DD-A39X-01 | 60 | 4 |
| LIHC | TCGA-DD-A39X-01 | 143 | 0 |
| LIHC | TCGA-DD-A39Y-01 | 151 | 7 |
| LIHC | TCGA-DD-A39Z-01 | 180 | 6 |
| LIHC | TCGA-DD-A3A0-01 | 159 | 8 |
| LIHC | TCGA-DD-A3A1-01 | 116 | 6 |
| LIHC | TCGA-DD-A3A2-01 | 92 | 4 |
| LIHC | TCGA-DD-A3A3-01 | 76 | 5 |
| LIHC | TCGA-DD-A3A4-01 | 49 | 4 |
| LIHC | TCGA-DD-A3A5-01 | 85 | 4 |
| LIHC | TCGA-DD-A3A6-01 | 37 | 3 |
| LIHC | TCGA-DD-A3A7-01 | 148 | 4 |
| LIHC | TCGA-DD-A3A8-01 | 155 | 8 |
| LIHC | TCGA-DD-A3A9-01 | 459 | 11 |
| LIHC | TCGA-DD-A4NA-01 | 43 | 2 |
| LIHC | TCGA-DD-A4NB-01 | 32 | 3 |
| LIHC | TCGA-DD-A4ND-01 | 68 | 3 |
| LIHC | TCGA-DD-A4NE-01 | 67 | 6 |
| LIHC | TCGA-DD-A4NF-01 | 141 | 8 |
| LIHC | TCGA-DD-A4NG-01 | 82 | 2 |
| LIHC | TCGA-DD-A4NH-01 | 56 | 3 |
| LIHC | TCGA-DD-A4NI-01 | 190 | 4 |
| LIHC | TCGA-DD-A4NJ-01 | 94 | 7 |
| LIHC | TCGA-DD-A4NK-01 | 98 | 6 |
| LIHC | TCGA-DD-A4NL-01 | 37 | 3 |
| LIHC | TCGA-DD-A4NN-01 | 66 | 6 |
| LIHC | TCGA-DD-A4NO-01 | 65 | 4 |
| LIHC | TCGA-DD-A4NP-01 | 30 | 1 |
| LIHC | TCGA-DD-A4NQ-01 | 107 | 5 |
| LIHC | TCGA-DD-A4NR-01 | 223 | 4 |
| LIHC | TCGA-DD-A4NS-01 | 62 | 2 |
| LIHC | TCGA-DD-A4NV-01 | 161 | 5 |
| LIHC | TCGA-DD-A73A-01 | 112 | 5 |
| LIHC | TCGA-DD-A73B-01 | 87 | 5 |
| LIHC | TCGA-DD-A73C-01 | 101 | 5 |
| LIHC | TCGA-DD-A73D-01 | 80 | 6 |
| LIHC | TCGA-DD-A73E-01 | 146 | 4 |
| LIHC | TCGA-DD-A73F-01 | 92 | 1 |
| LIHC | TCGA-DD-A73G-01 | 136 | 5 |
| LIHC | TCGA-ED-A459-01 | 270 | 7 |
| LIHC | TCGA-ED-A4XI-01 | 168 | 6 |
| LIHC | TCGA-ED-A5KG-01 | 33 | 2 |
| LIHC | TCGA-ED-A627-01 | 2 | 1 |
| LIHC | TCGA-ED-A66X-01 | 58 | 3 |
| LIHC | TCGA-ED-A66Y-01 | 94 | 3 |
| LIHC | TCGA-ED-A7PX-01 | 34 | 3 |
| LIHC | TCGA-ED-A7PY-01 | 33 | 1 |
| LIHC | TCGA-ED-A7PZ-01 | 298 | 13 |
| LIHC | TCGA-ED-A7XO-01 | 66 | 2 |
| LIHC | TCGA-ED-A7XP-01 | 79 | 12 |
| LIHC | TCGA-ED-A82E-01 | 35 | 2 |
| LIHC | TCGA-EP-A12J-01 | 82 | 3 |
| LIHC | TCGA-EP-A26S-01 | 183 | 9 |
| LIHC | TCGA-EP-A2KA-01 | 402 | 18 |
| LIHC | TCGA-EP-A2KB-01 | 340 | 14 |
| LIHC | TCGA-EP-A2KC-01 | 103 | 6 |
| LIHC | TCGA-EP-A3JL-01 | 102 | 6 |
| LIHC | TCGA-EP-A3RK-01 | 73 | 2 |
| LIHC | TCGA-ES-A2HS-01 | 319 | 16 |
| LIHC | TCGA-ES-A2HT-01 | 290 | 11 |
| LIHC | TCGA-FV-A23B-01 | 266 | 13 |
| LIHC | TCGA-FV-A2QQ-01 | 159 | 7 |
| LIHC | TCGA-FV-A2QR-01 | 186 | 11 |
| LIHC | TCGA-FV-A3IQ-01 | 100 | 3 |
| LIHC | TCGA-FV-A3I1-01 | 84 | 3 |
| LIHC | TCGA-FV-A3R2-01 | 96 | 6 |
| LIHC | TCGA-FV-A3R3-01 | 77 | 4 |
| LIHC | TCGA-FV-A495-01 | 124 | 1 |
| LIHC | TCGA-FV-A496-01 | 145 | 5 |
| LIHC | TCGA-FV-A4ZP-01 | 106 | 6 |
| LIHC | TCGA-FV-A4ZQ-01 | 95 | 8 |
| LIHC | TCGA-G3-A25S-01 | 341 | 12 |
| LIHC | TCGA-G3-A25T-01 | 247 | 12 |
| LIHC | TCGA-G3-A25U-01 | 252 | 13 |
| LIHC | TCGA-G3-A25V-01 | 182 | 9 |
| LIHC | TCGA-G3-A25W-01 | 211 | 3 |
| LIHC | TCGA-G3-A25Y-01 | 178 | 12 |
| LIHC | TCGA-G3-A25Z-01 | 148 | 8 |
| LIHC | TCGA-G3-A3CH-01 | 56 | 2 |
| LIHC | TCGA-G3-A3CI-01 | 25 | 1 |
| LIHC | TCGA-G3-A3CJ-01 | 106 | 2 |
| LIHC | TCGA-G3-A3CK-01 | 265 | 12 |
| LIHC | TCGA-G3-A5SI-01 | 43 | 2 |
| LIHC | TCGA-G3-A5SJ-01 | 125 | 10 |
| LIHC | TCGA-G3-A5SK-01 | 104 | 3 |
| LIHC | TCGA-G3-A5SL-01 | 143 | 6 |
| LIHC | TCGA-G3-A5SM-01 | 158 | 5 |
| LIHC | TCGA-G3-A6UC-01 | 146 | 8 |
| LIHC | TCGA-G3-A7M5-01 | 210 | 3 |
| LIHC | TCGA-G3-A7M6-01 | 106 | 4 |
| LIHC | TCGA-G3-A7M7-01 | 74 | 7 |
| LIHC | TCGA-G3-A7M8-01 | 31 | 3 |
| LIHC | TCGA-G3-A7M9-01 | 146 | 12 |
| LIHC | TCGA-GJ-A6C0-01 | 65 | 4 |
| LIHC | TCGA-HP-A5MZ-01 | 61 | 0 |
| LIHC | TCGA-HP-A5N0-01 | 151 | 5 |
| LIHC | TCGA-K7-A5RF-01 | 67 | 3 |
| LIHC | TCGA-K7-A5RG-01 | 143 | 5 |
| LIHC | TCGA-K7-A6G5-01 | 102 | 4 |
| LIHC | TCGA-KR-A7K0-01 | 111 | 2 |
| LIHC | TCGA-KR-A7K2-01 | 67 | 2 |
| LIHC | TCGA-KR-A7K7-01 | 75 | 2 |
| LIHC | TCGA-KR-A7K8-01 | 56 | 6 |
| LIHC | TCGA-LG-A6GG-01 | 233 | 8 |
| LIHC | TCGA-MI-A75C-01 | 134 | 9 |
| LIHC | TCGA-MI-A75E-01 | 144 | 5 |
| LIHC | TCGA-MI-A75G-01 | 272 | 14 |
| LIHC | TCGA-MI-A75H-01 | 156 | 7 |
| LIHC | TCGA-MI-A75I-01 | 202 | 12 |
| LIHC | TCGA-MR-A520-01 | 26 | 2 |
| LIHC | TCGA-NI-A4U2-01 | 139 | 5 |
| LIHC | TCGA-O8-A75V-01 | 99 | 6 |
| LIHC | TCGA-PD-A5DF-01 | 53 | 3 |
| LIHC | TCGA-QA-A7B7-01 | 110 | 3 |
| LIHC | TCGA-RC-A6M3-01 | 92 | 2 |
| LIHC | TCGA-RC-A6M4-01 | 210 | 7 |
| LIHC | TCGA-RC-A6M5-01 | 26 | 0 |
| LIHC | TCGA-RC-A6M6-01 | 223 | 6 |
| LIHC | TCGA-RC-A759-01 | 65 | 7 |
| LIHC | TCGA-RC-A7SB-01 | 73 | 3 |
| LIHC | TCGA-RC-A7SF-01 | 88 | 3 |
| LIHC | TCGA-RC-A7SK-01 | 173 | 6 |
| LIHC | TCGA-RG-A7D4-01 | 114 | 5 |
| LIHC | TCGA-T1-A6J8-01 | 102 | 5 |
| LIHC | TCGA-UB-A7MA-01 | 106 | 5 |
| LIHC | TCGA-UB-A7M8-01 | 1711 | 61 |
| LIHC | TCGA-UB-A7MC-01 | 71 | 5 |
| LIHC | TCGA-UB-A7MD-01 | 127 | 9 |
| LIHC | TCGA-UB-A7ME-01 | 106 | 2 |
| LIHC | TCGA-UB-A7MF-01 | 122 | 7 |
| LUAD | TCGA-05-4249-01 | 373 | 13 |
| LUAD | TCGA-05-4382-01 | 1690 | 72 |
| LUAD | TCGA-05-4384-01 | 161 | 13 |
| LUAD | TCGA-05-4389-01 | 308 | 13 |
| LUAD | TCGA-05-4390-01 | 646 | 24 |
| LUAD | TCGA-05-4395-01 | 306 | 19 |
| LUAD | TCGA-05-4396-01 | 622 | 28 |
| LUAD | TCGA-05-4398-01 | 923 | 32 |
| LUAD | TCGA-05-4402-01 | 190 | 8 |
| LUAD | TCGA-05-4403-01 | 168 | 10 |
| LUAD | TCGA-05-4405-01 | 481 | 19 |
| LUAD | TCGA-05-4410-01 | 1309 | 51 |
| LUAD | TCGA-05-4415-01 | 236 | 8 |
| LUAD | TCGA-05-4417-01 | 344 | 18 |
| LUAD | TCGA-05-4418-01 | 314 | 8 |
| LUAD | TCGA-05-4420-01 | 353 | 19 |
| LUAD | TCGA-05-4422-01 | 64 | 8 |
| LUAD | TCGA-05-4424-01 | 893 | 44 |
| LUAD | TCGA-05-4425-01 | 48 | 6 |
| LUAD | TCGA-05-4426-01 | 63 | 5 |
| LUAD | TCGA-05-4430-01 | 352 | 12 |
| LUAD | TCGA-05-4432-01 | 808 | 19 |
| LUAD | TCGA-05-4433-01 | 53 | 2 |
| LUAD | TCGA-05-5420-01 | 120 | 4 |
| LUAD | TCGA-05-5423-01 | 176 | 12 |
| LUAD | TCGA-05-5428-01 | 437 | 17 |
| LUAD | TCGA-05-5429-01 | 48 | 2 |
| LUAD | TCGA-05-5715-01 | 144 | 6 |
| LUAD | TCGA-35-3615-01 | 135 | 11 |
| LUAD | TCGA-38-4625-01 | 352 | 10 |
| LUAD | TCGA-38-4627-01 | 41 | 2 |
| LUAD | TCGA-38-4628-01 | 166 | 5 |
| LUAD | TCGA-38-4631-01 | 861 | 34 |
| LUAD | TCGA-38-4632-01 | 594 | 24 |
| LUAD | TCGA-38-6178-01 | 93 | 6 |
| LUAD | TCGA-44-2655-01 | 227 | 10 |
| LUAD | TCGA-44-2656-01 | 1225 | 44 |
| LUAD | TCGA-44-2657-01 | 473 | 22 |
| LUAD | TCGA-44-2659-01 | 480 | 18 |
| LUAD | TCGA-44-2661-01 | 70 | 5 |
| LUAD | TCGA-44-2662-01 | 262 | 15 |
| LUAD | TCGA-44-2665-01 | 58 | 4 |
| LUAD | TCGA-44-2666-01 | 59 | 7 |
| LUAD | TCGA-44-3396-01 | 204 | 10 |
| LUAD | TCGA-44-3919-01 | 129 | 3 |
| LUAD | TCGA-44-4112-01 | 506 | 16 |
| LUAD | TCGA-44-5645-01 | 51 | 5 |
| LUAD | TCGA-44-6145-01 | 331 | 17 |
| LUAD | TCGA-44-6146-01 | 53 | 1 |
| LUAD | TCGA-44-6147-01 | 247 | 16 |
| LUAD | TCGA-44-6774-01 | 357 | 14 |
| LUAD | TCGA-44-6775-01 | 88 | 5 |
| LUAD | TCGA-44-6776-01 | 233 | 13 |
| LUAD | TCGA-44-6777-01 | 443 | 23 |
| LUAD | TCGA-44-6779-01 | 127 | 9 |
| LUAD | TCGA-44-7659-01 | 173 | 12 |
| LUAD | TCGA-44-7662-01 | 644 | 28 |
| LUAD | TCGA-44-7670-01 | 1221 | 40 |
| LUAD | TCGA-44-7671-01 | 265 | 14 |
| LUAD | TCGA-44-7672-01 | 190 | 5 |
| LUAD | TCGA-49-4486-01 | 308 | 13 |
| LUAD | TCGA-49-4487-01 | 458 | 19 |
| LUAD | TCGA-49-4488-01 | 350 | 14 |
| LUAD | TCGA-49-4490-01 | 89 | 6 |
| LUAD | TCGA-49-4494-01 | 229 | 8 |
| LUAD | TCGA-49-4501-01 | 103 | 6 |
| LUAD | TCGA-49-4505-01 | 222 | 14 |
| LUAD | TCGA-49-4506-01 | 155 | 10 |
| LUAD | TCGA-49-4507-01 | 240 | 10 |
| LUAD | TCGA-49-4510-01 | 64 | 8 |
| LUAD | TCGA-49-4512-01 | 61 | 1 |
| LUAD | TCGA-49-4514-01 | 378 | 15 |
| LUAD | TCGA-49-6742-01 | 344 | 15 |
| LUAD | TCGA-49-6744-01 | 306 | 12 |
| LUAD | TCGA-49-6745-01 | 165 | 5 |
| LUAD | TCGA-49-6761-01 | 219 | 7 |
| LUAD | TCGA-49-6767-01 | 568 | 28 |
| LUAD | TCGA-50-5044-01 | 173 | 8 |
| LUAD | TCGA-50-5049-01 | 904 | 39 |
| LUAD | TCGA-50-5051-01 | 156 | 9 |
| LUAD | TCGA-50-5055-01 | 22 | 0 |
| LUAD | TCGA-50-5068-01 | 118 | 4 |
| LUAD | TCGA-50-5072-01 | 203 | 9 |
| LUAD | TCGA-50-5931-01 | 357 | 12 |
| LUAD | TCGA-50-5932-01 | 89 | 4 |
| LUAD | TCGA-50-5933-01 | 615 | 32 |
| LUAD | TCGA-50-5935-01 | 139 | 6 |
| LUAD | TCGA-50-5936-01 | 144 | 7 |
| LUAD | TCGA-50-5939-01 | 97 | 6 |
| LUAD | TCGA-50-5941-01 | 468 | 27 |
| LUAD | TCGA-50-5942-01 | 85 | 7 |
| LUAD | TCGA-50-5944-01 | 86 | 3 |
| LUAD | TCGA-50-6593-01 | 354 | 14 |
| LUAD | TCGA-50-6595-01 | 132 | 9 |
| LUAD | TCGA-50-6597-01 | 78 | 5 |
| LUAD | TCGA-50-6673-01 | 65 | 5 |
| LUAD | TCGA-50-7109-01 | 295 | 6 |
| LUAD | TCGA-53-7626-01 | 420 | 20 |
| LUAD | TCGA-53-7813-01 | 180 | 13 |
| LUAD | TCGA-55-1592-01 | 662 | 17 |
| LUAD | TCGA-55-1594-01 | 259 | 8 |
| LUAD | TCGA-55-1596-01 | 206 | 15 |
| LUAD | TCGA-55-6543-01 | 34 | 2 |
| LUAD | TCGA-55-6642-01 | 167 | 4 |
| LUAD | TCGA-55-6712-01 | 205 | 7 |
| LUAD | TCGA-55-6970-01 | 207 | 13 |
| LUAD | TCGA-55-6971-01 | 103 | 6 |
| LUAD | TCGA-55-6972-01 | 335 | 10 |
| LUAD | TCGA-55-6978-01 | 45 | 1 |
| LUAD | TCGA-55-6979-01 | 175 | 4 |

# TABLE 5 (APPENDIX A)

| Type | Sample | Value 1 | Value 2 |
|---|---|---|---|
| LUAD | TCGA-55-6980-01 | 21 | 1 |
| LUAD | TCGA-55-6981-01 | 74 | 4 |
| LUAD | TCGA-55-6982-01 | 284 | 13 |
| LUAD | TCGA-55-6983-01 | 134 | 8 |
| LUAD | TCGA-55-6985-01 | 549 | 18 |
| LUAD | TCGA-55-6986-01 | 29 | 2 |
| LUAD | TCGA-55-7281-01 | 529 | 20 |
| LUAD | TCGA-55-7283-01 | 278 | 15 |
| LUAD | TCGA-55-7573-01 | 65 | 5 |
| LUAD | TCGA-55-7574-01 | 318 | 15 |
| LUAD | TCGA-55-7576-01 | 432 | 21 |
| LUAD | TCGA-55-7724-01 | 310 | 13 |
| LUAD | TCGA-55-7725-01 | 163 | 8 |
| LUAD | TCGA-55-7726-01 | 144 | 11 |
| LUAD | TCGA-55-7727-01 | 311 | 15 |
| LUAD | TCGA-55-7728-01 | 124 | 11 |
| LUAD | TCGA-55-7815-01 | 174 | 14 |
| LUAD | TCGA-55-7903-01 | 185 | 10 |
| LUAD | TCGA-55-7907-01 | 1263 | 60 |
| LUAD | TCGA-55-7911-01 | 269 | 14 |
| LUAD | TCGA-55-7914-01 | 200 | 6 |
| LUAD | TCGA-64-1676-01 | 672 | 24 |
| LUAD | TCGA-64-1677-01 | 287 | 18 |
| LUAD | TCGA-64-1678-01 | 618 | 29 |
| LUAD | TCGA-64-1679-01 | 571 | 20 |
| LUAD | TCGA-64-1680-01 | 104 | 8 |
| LUAD | TCGA-64-1681-01 | 102 | 7 |
| LUAD | TCGA-64-5774-01 | 161 | 8 |
| LUAD | TCGA-64-5775-01 | 544 | 26 |
| LUAD | TCGA-64-5778-01 | 530 | 17 |
| LUAD | TCGA-64-5781-01 | 1246 | 47 |
| LUAD | TCGA-67-3771-01 | 1256 | 43 |
| LUAD | TCGA-67-3772-01 | 106 | 3 |
| LUAD | TCGA-67-3773-01 | 121 | 9 |
| LUAD | TCGA-67-3774-01 | 134 | 5 |
| LUAD | TCGA-67-6215-01 | 143 | 9 |
| LUAD | TCGA-67-6216-01 | 58 | 3 |
| LUAD | TCGA-67-6217-01 | 224 | 11 |
| LUAD | TCGA-69-7760-01 | 108 | 6 |
| LUAD | TCGA-69-7761-01 | 45 | 1 |
| LUAD | TCGA-69-7763-01 | 97 | 4 |
| LUAD | TCGA-69-7764-01 | 114 | 2 |
| LUAD | TCGA-69-7765-01 | 725 | 30 |
| LUAD | TCGA-69-7980-01 | 906 | 31 |
| LUAD | TCGA-71-6725-01 | 80 | 5 |
| LUAD | TCGA-73-4658-01 | 363 | 24 |
| LUAD | TCGA-73-4659-01 | 254 | 10 |
| LUAD | TCGA-73-4662-01 | 267 | 15 |
| LUAD | TCGA-73-4675-01 | 86 | 1 |
| LUAD | TCGA-73-4676-01 | 111 | 6 |
| LUAD | TCGA-73-7498-01 | 146 | 9 |
| LUAD | TCGA-73-7499-01 | 101 | 4 |
| LUAD | TCGA-75-5122-01 | 178 | 5 |
| LUAD | TCGA-75-5126-01 | 752 | 29 |
| LUAD | TCGA-75-5146-01 | 216 | 13 |
| LUAD | TCGA-75-5147-01 | 59 | 3 |
| LUAD | TCGA-75-6203-01 | 22 | 3 |
| LUAD | TCGA-75-6205-01 | 56 | 2 |
| LUAD | TCGA-75-6207-01 | 84 | 6 |
| LUAD | TCGA-75-6211-01 | 404 | 16 |
| LUAD | TCGA-75-6212-01 | 32 | 2 |
| LUAD | TCGA-75-6214-01 | 847 | 30 |
| LUAD | TCGA-75-7025-01 | 48 | 4 |
| LUAD | TCGA-75-7027-01 | 286 | 8 |
| LUAD | TCGA-75-7030-01 | 31 | 3 |
| LUAD | TCGA-75-7031-01 | 265 | 18 |
| LUAD | TCGA-78-7143-01 | 50 | 6 |
| LUAD | TCGA-78-7145-01 | 221 | 14 |
| LUAD | TCGA-78-7147-01 | 399 | 17 |
| LUAD | TCGA-78-7148-01 | 272 | 19 |
| LUAD | TCGA-78-7149-01 | 191 | 11 |
| LUAD | TCGA-78-7150-01 | 482 | 18 |
| LUAD | TCGA-78-7152-01 | 252 | 11 |
| LUAD | TCGA-78-7153-01 | 150 | 9 |
| LUAD | TCGA-78-7154-01 | 303 | 15 |
| LUAD | TCGA-78-7155-01 | 1700 | 65 |
| LUAD | TCGA-78-7156-01 | 318 | 17 |
| LUAD | TCGA-78-7158-01 | 268 | 15 |
| LUAD | TCGA-78-7159-01 | 348 | 14 |
| LUAD | TCGA-78-7160-01 | 57 | 3 |
| LUAD | TCGA-78-7161-01 | 219 | 9 |
| LUAD | TCGA-78-7162-01 | 112 | 4 |
| LUAD | TCGA-78-7163-01 | 33 | 1 |
| LUAD | TCGA-78-7166-01 | 260 | 22 |
| LUAD | TCGA-78-7167-01 | 227 | 15 |
| LUAD | TCGA-78-7535-01 | 206 | 7 |
| LUAD | TCGA-78-7536-01 | 676 | 32 |
| LUAD | TCGA-78-7537-01 | 163 | 7 |
| LUAD | TCGA-78-7539-01 | 571 | 22 |
| LUAD | TCGA-78-7540-01 | 38 | 5 |
| LUAD | TCGA-78-7542-01 | 424 | 17 |
| LUAD | TCGA-78-7633-01 | 126 | 5 |
| LUAD | TCGA-80-5607-01 | 158 | 5 |
| LUAD | TCGA-80-5608-01 | 201 | 16 |
| LUAD | TCGA-86-6562-01 | 74 | 4 |
| LUAD | TCGA-86-6851-01 | 1074 | 36 |
| LUAD | TCGA-86-7713-01 | 184 | 10 |
| LUAD | TCGA-86-7714-01 | 95 | 8 |
| LUAD | TCGA-91-6828-01 | 356 | 10 |
| LUAD | TCGA-91-6829-01 | 736 | 31 |
| LUAD | TCGA-91-6835-01 | 116 | 8 |
| LUAD | TCGA-91-6840-01 | 138 | 8 |
| LUAD | TCGA-91-6847-01 | 117 | 7 |
| LUAD | TCGA-91-6849-01 | 129 | 10 |
| LUAD | TCGA-91-7771-01 | 259 | 8 |
| LUAD | TCGA-93-7347-01 | 151 | 4 |
| LUAD | TCGA-93-7348-01 | 127 | 5 |
| LUAD | TCGA-95-7039-01 | 1471 | 59 |
| LUAD | TCGA-95-7043-01 | 1272 | 51 |
| LUAD | TCGA-95-7567-01 | 1139 | 42 |
| LUAD | TCGA-95-7947-01 | 614 | 26 |
| LUAD | TCGA-95-7948-01 | 183 | 4 |
| LUAD | TCGA-97-7546-01 | 310 | 16 |
| LUAD | TCGA-97-7552-01 | 44 | 2 |
| LUAD | TCGA-97-7553-01 | 95 | 5 |
| LUAD | TCGA-97-7554-01 | 530 | 23 |
| LUAD | TCGA-97-7937-01 | 525 | 25 |
| LUAD | TCGA-97-7938-01 | 452 | 16 |
| LUAD | TCGA-97-7941-01 | 122 | 7 |
| LUAD | TCGA-99-7458-01 | 562 | 25 |
| LUSC | TCGA-18-3406-01 | 319 | 12 |
| LUSC | TCGA-18-3407-01 | 176 | 7 |
| LUSC | TCGA-18-3408-01 | 115 | 7 |
| LUSC | TCGA-18-3409-01 | 3740 | 119 |
| LUSC | TCGA-18-3410-01 | 316 | 9 |
| LUSC | TCGA-18-3411-01 | 443 | 15 |
| LUSC | TCGA-18-3412-01 | 215 | 12 |
| LUSC | TCGA-18-3414-01 | 414 | 13 |
| LUSC | TCGA-18-3415-01 | 213 | 5 |
| LUSC | TCGA-18-3416-01 | 518 | 21 |
| LUSC | TCGA-18-3417-01 | 339 | 23 |
| LUSC | TCGA-18-3419-01 | 461 | 20 |
| LUSC | TCGA-18-3421-01 | 463 | 14 |
| LUSC | TCGA-18-4083-01 | 358 | 9 |
| LUSC | TCGA-18-4086-01 | 197 | 11 |
| LUSC | TCGA-18-4721-01 | 204 | 13 |
| LUSC | TCGA-18-5592-01 | 334 | 15 |
| LUSC | TCGA-18-5595-01 | 280 | 9 |
| LUSC | TCGA-21-1070-01 | 553 | 19 |
| LUSC | TCGA-21-1071-01 | 211 | 7 |
| LUSC | TCGA-21-1076-01 | 349 | 13 |
| LUSC | TCGA-21-1077-01 | 246 | 6 |
| LUSC | TCGA-21-1078-01 | 94 | 15 |
| LUSC | TCGA-21-1081-01 | 219 | 8 |
| LUSC | TCGA-21-5782-01 | 444 | 25 |
| LUSC | TCGA-21-5784-01 | 252 | 11 |
| LUSC | TCGA-21-5786-01 | 438 | 20 |
| LUSC | TCGA-21-5787-01 | 473 | 15 |
| LUSC | TCGA-22-0944-01 | 147 | 8 |
| LUSC | TCGA-22-1002-01 | 262 | 6 |
| LUSC | TCGA-22-1011-01 | 124 | 6 |
| LUSC | TCGA-22-1012-01 | 318 | 8 |
| LUSC | TCGA-22-1016-01 | 388 | 10 |
| LUSC | TCGA-22-4591-01 | 291 | 9 |
| LUSC | TCGA-22-4593-01 | 223 | 13 |
| LUSC | TCGA-22-4595-01 | 390 | 12 |
| LUSC | TCGA-22-4599-01 | 431 | 24 |
| LUSC | TCGA-22-4601-01 | 238 | 8 |
| LUSC | TCGA-22-4604-01 | 306 | 19 |
| LUSC | TCGA-22-4607-01 | 105 | 6 |
| LUSC | TCGA-22-4613-01 | 726 | 25 |
| LUSC | TCGA-22-5471-01 | 296 | 13 |
| LUSC | TCGA-22-5472-01 | 474 | 18 |
| LUSC | TCGA-22-5473-01 | 950 | 41 |
| LUSC | TCGA-22-5474-01 | 113 | 7 |
| LUSC | TCGA-22-5477-01 | 212 | 10 |
| LUSC | TCGA-22-5478-01 | 217 | 4 |
| LUSC | TCGA-22-5480-01 | 184 | 7 |
| LUSC | TCGA-22-5482-01 | 197 | 8 |
| LUSC | TCGA-22-5485-01 | 247 | 8 |
| LUSC | TCGA-22-5489-01 | 284 | 5 |
| LUSC | TCGA-22-5491-01 | 408 | 17 |
| LUSC | TCGA-22-5492-01 | 259 | 15 |
| LUSC | TCGA-33-4532-01 | 456 | 23 |
| LUSC | TCGA-33-4533-01 | 341 | 15 |
| LUSC | TCGA-33-4538-01 | 231 | 11 |
| LUSC | TCGA-33-4547-01 | 256 | 14 |
| LUSC | TCGA-33-4566-01 | 1456 | 52 |
| LUSC | TCGA-33-4582-01 | 246 | 11 |
| LUSC | TCGA-33-4583-01 | 629 | 21 |
| LUSC | TCGA-33-4586-01 | 425 | 18 |
| LUSC | TCGA-33-6737-01 | 520 | 25 |
| LUSC | TCGA-34-2596-01 | 314 | 12 |
| LUSC | TCGA-34-2600-01 | 583 | 32 |
| LUSC | TCGA-34-2608-01 | 187 | 10 |
| LUSC | TCGA-34-5231-01 | 751 | 23 |
| LUSC | TCGA-34-5232-01 | 230 | 13 |
| LUSC | TCGA-34-5234-01 | 216 | 7 |
| LUSC | TCGA-34-5236-01 | 259 | 11 |
| LUSC | TCGA-34-5239-01 | 282 | 11 |
| LUSC | TCGA-34-5240-01 | 205 | 16 |
| LUSC | TCGA-34-5241-01 | 5 | 0 |
| LUSC | TCGA-34-5927-01 | 327 | 17 |
| LUSC | TCGA-34-5928-01 | 456 | 21 |
| LUSC | TCGA-34-5929-01 | 243 | 9 |
| LUSC | TCGA-37-3783-01 | 461 | 16 |
| LUSC | TCGA-37-3789-01 | 505 | 19 |
| LUSC | TCGA-37-4133-01 | 341 | 6 |
| LUSC | TCGA-37-4135-01 | 340 | 11 |
| LUSC | TCGA-37-4141-01 | 382 | 12 |
| LUSC | TCGA-37-5819-01 | 847 | 29 |
| LUSC | TCGA-39-5016-01 | 419 | 9 |
| LUSC | TCGA-39-5019-01 | 222 | 5 |
| LUSC | TCGA-39-5021-01 | 170 | 7 |
| LUSC | TCGA-39-5022-01 | 334 | 11 |
| LUSC | TCGA-39-5024-01 | 259 | 11 |
| LUSC | TCGA-39-5027-01 | 381 | 15 |
| LUSC | TCGA-39-5028-01 | 229 | 13 |
| LUSC | TCGA-39-5029-01 | 175 | 9 |
| LUSC | TCGA-39-5030-01 | 329 | 13 |
| LUSC | TCGA-39-5031-01 | 753 | 39 |
| LUSC | TCGA-39-5035-01 | 185 | 6 |
| LUSC | TCGA-39-5036-01 | 339 | 12 |
| LUSC | TCGA-39-5037-01 | 288 | 9 |
| LUSC | TCGA-39-5039-01 | 199 | 12 |
| LUSC | TCGA-43-2578-01 | 360 | 15 |
| LUSC | TCGA-43-3394-01 | 186 | 11 |
| LUSC | TCGA-43-3920-01 | 353 | 10 |
| LUSC | TCGA-43-5668-01 | 723 | 32 |
| LUSC | TCGA-43-6143-01 | 374 | 12 |
| LUSC | TCGA-43-6647-01 | 200 | 14 |
| LUSC | TCGA-43-6770-01 | 263 | 8 |
| LUSC | TCGA-43-6771-01 | 224 | 8 |
| LUSC | TCGA-46-3765-01 | 385 | 15 |
| LUSC | TCGA-46-3766-01 | 6 | 0 |
| LUSC | TCGA-46-3767-01 | 252 | 11 |
| LUSC | TCGA-46-3768-01 | 408 | 14 |
| LUSC | TCGA-46-3769-01 | 1033 | 38 |
| LUSC | TCGA-46-6025-01 | 222 | 9 |
| LUSC | TCGA-46-6026-01 | 243 | 11 |
| LUSC | TCGA-51-4079-01 | 433 | 14 |
| LUSC | TCGA-51-4080-01 | 327 | 12 |
| LUSC | TCGA-51-4081-01 | 233 | 12 |
| LUSC | TCGA-56-1622-01 | 250 | 13 |
| LUSC | TCGA-56-5897-01 | 131 | 8 |
| LUSC | TCGA-56-5898-01 | 174 | 4 |
| LUSC | TCGA-56-6545-01 | 327 | 13 |
| LUSC | TCGA-56-6546-01 | 373 | 13 |
| LUSC | TCGA-60-2698-01 | 1392 | 55 |
| LUSC | TCGA-60-2707-01 | 147 | 10 |
| LUSC | TCGA-60-2708-01 | 237 | 6 |
| LUSC | TCGA-60-2709-01 | 222 | 5 |
| LUSC | TCGA-60-2710-01 | 231 | 13 |
| LUSC | TCGA-60-2711-01 | 151 | 4 |
| LUSC | TCGA-60-2712-01 | 105 | 7 |
| LUSC | TCGA-60-2713-01 | 289 | 12 |
| LUSC | TCGA-60-2715-01 | 195 | 5 |
| LUSC | TCGA-60-2719-01 | 227 | 10 |
| LUSC | TCGA-60-2720-01 | 460 | 17 |
| LUSC | TCGA-60-2721-01 | 97 | 6 |
| LUSC | TCGA-60-2722-01 | 360 | 12 |
| LUSC | TCGA-60-2723-01 | 318 | 14 |
| LUSC | TCGA-60-2724-01 | 360 | 13 |
| LUSC | TCGA-60-2725-01 | 192 | 6 |
| LUSC | TCGA-60-2726-01 | 334 | 14 |
| LUSC | TCGA-63-5128-01 | 353 | 15 |
| LUSC | TCGA-63-5131-01 | 316 | 20 |
| LUSC | TCGA-63-6202-01 | 395 | 14 |
| LUSC | TCGA-66-2727-01 | 276 | 7 |
| LUSC | TCGA-66-2734-01 | 342 | 9 |
| LUSC | TCGA-66-2742-01 | 260 | 10 |
| LUSC | TCGA-66-2744-01 | 296 | 13 |
| LUSC | TCGA-66-2754-01 | 353 | 23 |
| LUSC | TCGA-66-2755-01 | 235 | 11 |

## TABLE 5 (APPENDIX A)

| Type | Sample | Value 1 | Value 2 |
|---|---|---|---|
| LUSC | TCGA-66-2756-01 | 554 | 24 |
| LUSC | TCGA-66-2757-01 | 295 | 15 |
| LUSC | TCGA-66-2758-01 | 391 | 10 |
| LUSC | TCGA-66-2759-01 | 505 | 18 |
| LUSC | TCGA-66-2763-01 | 457 | 12 |
| LUSC | TCGA-66-2765-01 | 115 | 4 |
| LUSC | TCGA-66-2766-01 | 446 | 21 |
| LUSC | TCGA-66-2767-01 | 388 | 14 |
| LUSC | TCGA-66-2768-01 | 297 | 6 |
| LUSC | TCGA-66-2770-01 | 278 | 9 |
| LUSC | TCGA-66-2771-01 | 305 | 16 |
| LUSC | TCGA-66-2773-01 | 687 | 31 |
| LUSC | TCGA-66-2777-01 | 230 | 16 |
| LUSC | TCGA-66-2778-01 | 176 | 9 |
| LUSC | TCGA-66-2780-01 | 148 | 13 |
| LUSC | TCGA-66-2781-01 | 168 | 7 |
| LUSC | TCGA-66-2782-01 | 396 | 14 |
| LUSC | TCGA-66-2783-01 | 278 | 15 |
| LUSC | TCGA-66-2785-01 | 1336 | 42 |
| LUSC | TCGA-66-2786-01 | 270 | 13 |
| LUSC | TCGA-66-2787-01 | 595 | 26 |
| LUSC | TCGA-66-2788-01 | 229 | 8 |
| LUSC | TCGA-66-2789-01 | 404 | 13 |
| LUSC | TCGA-66-2791-01 | 392 | 13 |
| LUSC | TCGA-66-2792-01 | 201 | 7 |
| LUSC | TCGA-66-2793-01 | 358 | 12 |
| LUSC | TCGA-66-2794-01 | 184 | 7 |
| LUSC | TCGA-66-2795-01 | 423 | 13 |
| LUSC | TCGA-66-2800-01 | 273 | 12 |
| LUSC | TCGA-70-6722-01 | 323 | 12 |
| LUSC | TCGA-70-6723-01 | 138 | 3 |
| LUSC | TCGA-85-6175-01 | 68 | 2 |
| LUSC | TCGA-85-6560-01 | 498 | 16 |
| LUSC | TCGA-85-6561-01 | 982 | 46 |
| OV | TCGA-04-1331-01 | 95 | 5 |
| OV | TCGA-04-1332-01 | 34 | 2 |
| OV | TCGA-04-1336-01 | 66 | 3 |
| OV | TCGA-04-1337-01 | 99 | 4 |
| OV | TCGA-04-1338-01 | 149 | 7 |
| OV | TCGA-04-1342-01 | 88 | 3 |
| OV | TCGA-04-1343-01 | 67 | 4 |
| OV | TCGA-04-1346-01 | 51 | 2 |
| OV | TCGA-04-1347-01 | 128 | 7 |
| OV | TCGA-04-1348-01 | 45 | 1 |
| OV | TCGA-04-1349-01 | 40 | 1 |
| OV | TCGA-04-1350-01 | 48 | 5 |
| OV | TCGA-04-1356-01 | 67 | 1 |
| OV | TCGA-04-1357-01 | 68 | 6 |
| OV | TCGA-04-1361-01 | 66 | 3 |
| OV | TCGA-04-1362-01 | 73 | 2 |
| OV | TCGA-04-1364-01 | 44 | 6 |
| OV | TCGA-04-1365-01 | 44 | 2 |
| OV | TCGA-04-1367-01 | 94 | 4 |
| OV | TCGA-04-1514-01 | 31 | 3 |
| OV | TCGA-04-1517-01 | 20 | 1 |
| OV | TCGA-04-1525-01 | 55 | 6 |
| OV | TCGA-04-1530-01 | 70 | 1 |
| OV | TCGA-04-1542-01 | 68 | 2 |
| OV | TCGA-09-0366-01 | 47 | 2 |
| OV | TCGA-09-0369-01 | 65 | 3 |
| OV | TCGA-09-1659-01 | 20 | 1 |
| OV | TCGA-09-1661-01 | 45 | 4 |
| OV | TCGA-09-1662-01 | 35 | 3 |
| OV | TCGA-09-1665-01 | 101 | 10 |
| OV | TCGA-09-1666-01 | 23 | 2 |
| OV | TCGA-09-1669-01 | 51 | 4 |
| OV | TCGA-09-2044-01 | 99 | 7 |
| OV | TCGA-09-2045-01 | 37 | 3 |
| OV | TCGA-09-2049-01 | 127 | 8 |
| OV | TCGA-09-2050-01 | 120 | 6 |
| OV | TCGA-09-2051-01 | 108 | 3 |
| OV | TCGA-09-2053-01 | 56 | 5 |
| OV | TCGA-09-2056-01 | 78 | 6 |
| OV | TCGA-10-0926-01 | 44 | 1 |
| OV | TCGA-10-0927-01 | 28 | 2 |
| OV | TCGA-10-0928-01 | 43 | 0 |
| OV | TCGA-10-0930-01 | 170 | 9 |
| OV | TCGA-10-0931-01 | 34 | 2 |
| OV | TCGA-10-0933-01 | 46 | 2 |
| OV | TCGA-10-0934-01 | 30 | 3 |
| OV | TCGA-10-0935-01 | 48 | 4 |
| OV | TCGA-10-0937-01 | 48 | 1 |
| OV | TCGA-10-0938-01 | 66 | 1 |
| OV | TCGA-13-0714-01 | 71 | 3 |
| OV | TCGA-13-0717-01 | 50 | 5 |
| OV | TCGA-13-0720-01 | 58 | 5 |
| OV | TCGA-13-0723-01 | 48 | 2 |
| OV | TCGA-13-0724-01 | 48 | 6 |
| OV | TCGA-13-0726-01 | 74 | 5 |
| OV | TCGA-13-0727-01 | 35 | 2 |
| OV | TCGA-13-0730-01 | 43 | 5 |
| OV | TCGA-13-0751-01 | 46 | 2 |
| OV | TCGA-13-0755-01 | 102 | 5 |
| OV | TCGA-13-0760-01 | 198 | 5 |
| OV | TCGA-13-0761-01 | 69 | 8 |
| OV | TCGA-13-0762-01 | 79 | 6 |
| OV | TCGA-13-0765-01 | 28 | 1 |
| OV | TCGA-13-0791-01 | 84 | 5 |
| OV | TCGA-13-0792-01 | 78 | 3 |
| OV | TCGA-13-0793-01 | 86 | 6 |
| OV | TCGA-13-0795-01 | 71 | 3 |
| OV | TCGA-13-0800-01 | 67 | 4 |
| OV | TCGA-13-0804-01 | 40 | 2 |
| OV | TCGA-13-0807-01 | 65 | 3 |
| OV | TCGA-13-0883-01 | 65 | 6 |
| OV | TCGA-13-0884-01 | 97 | 3 |
| OV | TCGA-13-0885-01 | 178 | 3 |
| OV | TCGA-13-0886-01 | 69 | 4 |
| OV | TCGA-13-0887-01 | 115 | 1 |
| OV | TCGA-13-0889-01 | 33 | 3 |
| OV | TCGA-13-0890-01 | 65 | 8 |
| OV | TCGA-13-0891-01 | 32 | 3 |
| OV | TCGA-13-0893-01 | 83 | 3 |
| OV | TCGA-13-0894-01 | 59 | 2 |
| OV | TCGA-13-0897-01 | 58 | 2 |
| OV | TCGA-13-0899-01 | 44 | 2 |
| OV | TCGA-13-0900-01 | 116 | 6 |
| OV | TCGA-13-0903-01 | 66 | 3 |
| OV | TCGA-13-0904-01 | 118 | 2 |
| OV | TCGA-13-0905-01 | 66 | 3 |
| OV | TCGA-13-0906-01 | 122 | 5 |
| OV | TCGA-13-0910-01 | 28 | 1 |
| OV | TCGA-13-0911-01 | 27 | 1 |
| OV | TCGA-13-0912-01 | 37 | 2 |
| OV | TCGA-13-0913-01 | 93 | 5 |
| OV | TCGA-13-0916-01 | 75 | 2 |
| OV | TCGA-13-0919-01 | 92 | 4 |
| OV | TCGA-13-0920-01 | 137 | 5 |
| OV | TCGA-13-0923-01 | 139 | 4 |
| OV | TCGA-13-0924-01 | 69 | 4 |
| OV | TCGA-13-1403-01 | 54 | 3 |
| OV | TCGA-13-1404-01 | 58 | 5 |
| OV | TCGA-13-1405-01 | 34 | 2 |
| OV | TCGA-13-1407-01 | 34 | 3 |
| OV | TCGA-13-1408-01 | 142 | 4 |
| OV | TCGA-13-1409-01 | 58 | 2 |
| OV | TCGA-13-1410-01 | 66 | 2 |
| OV | TCGA-13-1411-01 | 54 | 4 |
| OV | TCGA-13-1412-01 | 39 | 3 |
| OV | TCGA-13-1477-01 | 58 | 4 |
| OV | TCGA-13-1481-01 | 118 | 6 |
| OV | TCGA-13-1482-01 | 67 | 3 |
| OV | TCGA-13-1483-01 | 61 | 10 |
| OV | TCGA-13-1484-01 | 45 | 3 |
| OV | TCGA-13-1487-01 | 47 | 2 |
| OV | TCGA-13-1488-01 | 127 | 4 |
| OV | TCGA-13-1489-01 | 62 | 6 |
| OV | TCGA-13-1491-01 | 48 | 2 |
| OV | TCGA-13-1492-01 | 45 | 5 |
| OV | TCGA-13-1494-01 | 60 | 4 |
| OV | TCGA-13-1495-01 | 44 | 2 |
| OV | TCGA-13-1496-01 | 64 | 4 |
| OV | TCGA-13-1497-01 | 144 | 7 |
| OV | TCGA-13-1498-01 | 126 | 6 |
| OV | TCGA-13-1499-01 | 70 | 6 |
| OV | TCGA-13-1501-01 | 74 | 5 |
| OV | TCGA-13-1504-01 | 38 | 1 |
| OV | TCGA-13-1505-01 | 65 | 1 |
| OV | TCGA-13-1506-01 | 28 | 1 |
| OV | TCGA-13-1507-01 | 95 | 2 |
| OV | TCGA-13-1509-01 | 96 | 6 |
| OV | TCGA-13-1510-01 | 116 | 5 |
| OV | TCGA-13-1512-01 | 60 | 3 |
| OV | TCGA-13-2060-01 | 51 | 2 |
| OV | TCGA-20-0987-01 | 27 | 3 |
| OV | TCGA-20-0990-01 | 83 | 4 |
| OV | TCGA-20-0991-01 | 85 | 2 |
| OV | TCGA-23-1021-01 | 89 | 8 |
| OV | TCGA-23-1022-01 | 199 | 9 |
| OV | TCGA-23-1023-01 | 42 | 3 |
| OV | TCGA-23-1024-01 | 46 | 2 |
| OV | TCGA-23-1026-01 | 28 | 1 |
| OV | TCGA-23-1027-01 | 43 | 2 |
| OV | TCGA-23-1028-01 | 58 | 4 |
| OV | TCGA-23-1030-01 | 47 | 4 |
| OV | TCGA-23-1031-01 | 113 | 2 |
| OV | TCGA-23-1032-01 | 105 | 3 |
| OV | TCGA-23-1110-01 | 118 | 3 |
| OV | TCGA-23-1116-01 | 73 | 1 |
| OV | TCGA-23-1117-01 | 110 | 2 |
| OV | TCGA-23-1118-01 | 71 | 4 |
| OV | TCGA-23-1120-01 | 157 | 4 |
| OV | TCGA-23-1122-01 | 120 | 2 |
| OV | TCGA-23-1123-01 | 90 | 6 |
| OV | TCGA-23-1124-01 | 155 | 1 |
| OV | TCGA-23-2072-01 | 57 | 1 |
| OV | TCGA-23-2077-01 | 77 | 2 |
| OV | TCGA-23-2078-01 | 114 | 7 |
| OV | TCGA-23-2079-01 | 51 | 2 |
| OV | TCGA-23-2081-01 | 54 | 2 |
| OV | TCGA-24-0966-01 | 38 | 4 |
| OV | TCGA-24-0968-01 | 19 | 1 |
| OV | TCGA-24-0970-01 | 30 | 2 |
| OV | TCGA-24-0975-01 | 69 | 3 |
| OV | TCGA-24-0979-01 | 100 | 5 |
| OV | TCGA-24-0980-01 | 41 | 2 |
| OV | TCGA-24-0982-01 | 59 | 2 |
| OV | TCGA-24-1103-01 | 84 | 6 |
| OV | TCGA-24-1104-01 | 67 | 3 |
| OV | TCGA-24-1105-01 | 21 | 2 |
| OV | TCGA-24-1413-01 | 45 | 2 |
| OV | TCGA-24-1416-01 | 17 | 3 |
| OV | TCGA-24-1417-01 | 74 | 1 |
| OV | TCGA-24-1418-01 | 49 | 2 |
| OV | TCGA-24-1419-01 | 39 | 2 |
| OV | TCGA-24-1422-01 | 130 | 3 |
| OV | TCGA-24-1423-01 | 73 | 1 |
| OV | TCGA-24-1424-01 | 60 | 2 |
| OV | TCGA-24-1425-01 | 38 | 1 |
| OV | TCGA-24-1426-01 | 39 | 2 |
| OV | TCGA-24-1427-01 | 60 | 4 |
| OV | TCGA-24-1428-01 | 17 | 2 |
| OV | TCGA-24-1431-01 | 55 | 1 |
| OV | TCGA-24-1434-01 | 52 | 3 |
| OV | TCGA-24-1435-01 | 84 | 3 |
| OV | TCGA-24-1436-01 | 49 | 3 |
| OV | TCGA-24-1463-01 | 77 | 4 |
| OV | TCGA-24-1464-01 | 62 | 2 |
| OV | TCGA-24-1466-01 | 52 | 4 |
| OV | TCGA-24-1469-01 | 166 | 9 |
| OV | TCGA-24-1470-01 | 83 | 6 |
| OV | TCGA-24-1471-01 | 28 | 2 |
| OV | TCGA-24-1474-01 | 61 | 5 |
| OV | TCGA-24-1544-01 | 29 | 1 |
| OV | TCGA-24-1545-01 | 21 | 2 |
| OV | TCGA-24-1548-01 | 37 | 5 |
| OV | TCGA-24-1549-01 | 41 | 2 |
| OV | TCGA-24-1551-01 | 42 | 3 |
| OV | TCGA-24-1552-01 | 41 | 2 |
| OV | TCGA-24-1553-01 | 45 | 1 |
| OV | TCGA-24-1555-01 | 48 | 4 |
| OV | TCGA-24-1556-01 | 68 | 7 |
| OV | TCGA-24-1557-01 | 48 | 1 |
| OV | TCGA-24-1558-01 | 27 | 4 |
| OV | TCGA-24-1560-01 | 31 | 2 |
| OV | TCGA-24-1562-01 | 36 | 4 |
| OV | TCGA-24-1563-01 | 78 | 3 |
| OV | TCGA-24-1564-01 | 46 | 2 |
| OV | TCGA-24-1565-01 | 35 | 3 |
| OV | TCGA-24-1567-01 | 52 | 2 |
| OV | TCGA-24-1603-01 | 28 | 3 |
| OV | TCGA-24-1604-01 | 69 | 4 |
| OV | TCGA-24-1614-01 | 39 | 3 |
| OV | TCGA-24-1616-01 | 63 | 3 |
| OV | TCGA-24-2019-01 | 42 | 3 |
| OV | TCGA-24-2024-01 | 89 | 7 |
| OV | TCGA-24-2030-01 | 64 | 2 |
| OV | TCGA-24-2035-01 | 109 | 7 |
| OV | TCGA-24-2038-01 | 12 | 1 |
| OV | TCGA-24-2254-01 | 56 | 2 |
| OV | TCGA-24-2260-01 | 53 | 3 |
| OV | TCGA-24-2261-01 | 44 | 2 |
| OV | TCGA-24-2262-01 | 84 | 3 |
| OV | TCGA-24-2267-01 | 116 | 7 |
| OV | TCGA-24-2271-01 | 42 | 1 |
| OV | TCGA-24-2280-01 | 157 | 9 |
| OV | TCGA-24-2281-01 | 60 | 1 |
| OV | TCGA-24-2288-01 | 114 | 7 |
| OV | TCGA-24-2289-01 | 151 | 5 |
| OV | TCGA-24-2290-01 | 61 | 4 |
| OV | TCGA-24-2293-01 | 78 | 6 |
| OV | TCGA-24-2298-01 | 87 | 1 |
| OV | TCGA-25-1313-01 | 152 | 7 |
| OV | TCGA-25-1315-01 | 56 | 7 |

# EP 3 423 828 B1

## TABLE 5 (APPENDIX A)

| Type | Sample ID | Count 1 | Count 2 |
|---|---|---|---|
| OV | TCGA-25-1316-01 | 23 | 2 |
| OV | TCGA-25-1317-01 | 55 | 2 |
| OV | TCGA-25-1318-01 | 77 | 3 |
| OV | TCGA-25-1319-01 | 58 | 4 |
| OV | TCGA-25-1320-01 | 59 | 4 |
| OV | TCGA-25-1321-01 | 42 | 1 |
| OV | TCGA-25-1322-01 | 44 | 2 |
| OV | TCGA-25-1324-01 | 49 | 4 |
| OV | TCGA-25-1326-01 | 145 | 5 |
| OV | TCGA-25-1328-01 | 13 | 0 |
| OV | TCGA-25-1329-01 | 40 | 3 |
| OV | TCGA-25-1623-01 | 19 | 2 |
| OV | TCGA-25-1625-01 | 34 | 4 |
| OV | TCGA-25-1626-01 | 12 | 2 |
| OV | TCGA-25-1627-01 | 32 | 3 |
| OV | TCGA-25-1628-01 | 24 | 2 |
| OV | TCGA-25-1630-01 | 47 | 3 |
| OV | TCGA-25-1631-01 | 31 | 3 |
| OV | TCGA-25-1632-01 | 61 | 4 |
| OV | TCGA-25-1633-01 | 20 | 2 |
| OV | TCGA-25-1634-01 | 32 | 3 |
| OV | TCGA-25-1635-01 | 20 | 3 |
| OV | TCGA-25-2042-01 | 100 | 4 |
| OV | TCGA-25-2391-01 | 65 | 6 |
| OV | TCGA-25-2392-01 | 109 | 6 |
| OV | TCGA-25-2393-01 | 56 | 4 |
| OV | TCGA-25-2396-01 | 35 | 3 |
| OV | TCGA-25-2398-01 | 54 | 2 |
| OV | TCGA-25-2399-01 | 49 | 5 |
| OV | TCGA-25-2400-01 | 81 | 3 |
| OV | TCGA-25-2401-01 | 69 | 2 |
| OV | TCGA-25-2404-01 | 45 | 1 |
| OV | TCGA-25-2408-01 | 17 | 2 |
| OV | TCGA-25-2409-01 | 28 | 1 |
| OV | TCGA-29-2427-01 | 69 | 5 |
| OV | TCGA-30-1853-01 | 46 | 2 |
| OV | TCGA-30-1862-01 | 38 | 4 |
| OV | TCGA-30-1891-01 | 69 | 3 |
| OV | TCGA-31-1950-01 | 49 | 2 |
| OV | TCGA-31-1953-01 | 38 | 4 |
| OV | TCGA-31-1959-01 | 54 | 3 |
| OV | TCGA-36-1568-01 | 43 | 4 |
| OV | TCGA-36-1569-01 | 10 | 3 |
| OV | TCGA-36-1570-01 | 32 | 1 |
| OV | TCGA-36-1571-01 | 16 | 2 |
| OV | TCGA-36-1574-01 | 19 | 0 |
| OV | TCGA-36-1575-01 | 40 | 2 |
| OV | TCGA-36-1576-01 | 17 | 5 |
| OV | TCGA-36-1577-01 | 93 | 5 |
| OV | TCGA-36-1578-01 | 62 | 5 |
| OV | TCGA-36-1580-01 | 19 | 2 |
| OV | TCGA-57-1582-01 | 71 | 9 |
| OV | TCGA-57-1583-01 | 12 | 1 |
| OV | TCGA-57-1584-01 | 32 | 2 |
| OV | TCGA-57-1993-01 | 57 | 5 |
| OV | TCGA-59-2348-01 | 93 | 3 |
| OV | TCGA-59-2350-01 | 33 | 2 |
| OV | TCGA-59-2351-01 | 97 | 6 |
| OV | TCGA-59-2352-01 | 86 | 3 |
| OV | TCGA-59-2354-01 | 63 | 4 |
| OV | TCGA-59-2355-01 | 27 | 1 |
| OV | TCGA-59-2363-01 | 86 | 6 |
| OV | TCGA-61-1728-01 | 36 | 1 |
| OV | TCGA-61-1736-01 | 43 | 2 |
| OV | TCGA-61-1919-01 | 39 | 2 |
| OV | TCGA-61-1995-01 | 27 | 3 |
| OV | TCGA-61-1998-01 | 141 | 6 |
| OV | TCGA-61-2000-01 | 47 | 3 |
| OV | TCGA-61-2002-01 | 44 | 1 |
| OV | TCGA-61-2003-01 | 52 | 3 |
| OV | TCGA-61-2008-01 | 61 | 5 |
| OV | TCGA-61-2009-01 | 64 | 3 |
| OV | TCGA-61-2012-01 | 120 | 3 |
| OV | TCGA-61-2016-01 | 22 | 3 |
| OV | TCGA-61-2088-01 | 19 | 3 |
| OV | TCGA-61-2092-01 | 34 | 2 |
| OV | TCGA-61-2094-01 | 59 | 4 |
| OV | TCGA-61-2095-01 | 160 | 6 |
| OV | TCGA-61-2097-01 | 52 | 5 |
| OV | TCGA-61-2101-01 | 42 | 2 |
| OV | TCGA-61-2102-01 | 63 | 3 |
| OV | TCGA-61-2104-01 | 56 | 2 |
| OV | TCGA-61-2109-01 | 65 | 4 |
| OV | TCGA-61-2110-01 | 53 | 1 |
| OV | TCGA-61-2111-01 | 53 | 3 |
| OV | TCGA-61-2113-01 | 100 | 5 |
| PAAD | TCGA-2J-AAB1-01 | 130 | 10 |
| PAAD | TCGA-2J-AA84-01 | 74 | 3 |
| PAAD | TCGA-2J-AAB6-01 | 121 | 5 |
| PAAD | TCGA-2J-AAB8-01 | 109 | 8 |
| PAAD | TCGA-2J-AAB9-01 | 55 | 5 |
| PAAD | TCGA-2J-AA8A-01 | 68 | 7 |
| PAAD | TCGA-2J-AA8E-01 | 73 | 6 |
| PAAD | TCGA-2J-AA8F-01 | 97 | 5 |
| PAAD | TCGA-2J-AABH-01 | 75 | 3 |
| PAAD | TCGA-2J-AA8K-01 | 87 | 6 |
| PAAD | TCGA-2J-AA8O-01 | 143 | 8 |
| PAAD | TCGA-2J-AA8R-01 | 69 | 2 |
| PAAD | TCGA-2J-AA8U-01 | 62 | 5 |
| PAAD | TCGA-2J-AA8V-01 | 72 | 4 |
| PAAD | TCGA-2L-AAQA-01 | 97 | 5 |
| PAAD | TCGA-2L-AAQE-01 | 79 | 6 |
| PAAD | TCGA-2L-AAQI-01 | 87 | 4 |
| PAAD | TCGA-2L-AAQJ-01 | 158 | 6 |
| PAAD | TCGA-2L-AAQL-01 | 115 | 7 |
| PAAD | TCGA-3A-A9I5-01 | 78 | 5 |
| PAAD | TCGA-3A-A9I7-01 | 59 | 4 |
| PAAD | TCGA-3A-A9I9-01 | 151 | 8 |
| PAAD | TCGA-3A-A9IB-01 | 104 | 7 |
| PAAD | TCGA-3A-A9IC-01 | 92 | 5 |
| PAAD | TCGA-3A-A9IH-01 | 109 | 8 |
| PAAD | TCGA-3A-A9IU-01 | 87 | 6 |
| PAAD | TCGA-3A-A9IX-01 | 65 | 5 |
| PAAD | TCGA-3A-A9IZ-01 | 113 | 5 |
| PAAD | TCGA-3A-A9J0-01 | 129 | 9 |
| PAAD | TCGA-3E-AAAY-01 | 65 | 4 |
| PAAD | TCGA-3E-AAAZ-01 | 90 | 8 |
| PAAD | TCGA-F2-6879-01 | 103 | 7 |
| PAAD | TCGA-F2-A44G-01 | 63 | 5 |
| PAAD | TCGA-F2-A44H-01 | 31 | 2 |
| PAAD | TCGA-F2-A7TX-01 | 94 | 4 |
| PAAD | TCGA-F2-A8YN-01 | 145 | 10 |
| PAAD | TCGA-FB-A4P5-01 | 60 | 0 |
| PAAD | TCGA-FB-A4P6-01 | 27 | 1 |
| PAAD | TCGA-FB-A545-01 | 61 | 5 |
| PAAD | TCGA-FB-A5VM-01 | 113 | 5 |
| PAAD | TCGA-FB-A78T-01 | 151 | 11 |
| PAAD | TCGA-FB-AAPS-01 | 79 | 6 |
| PAAD | TCGA-FB-AAPU-01 | 151 | 3 |
| PAAD | TCGA-FB-AAPY-01 | 85 | 7 |
| PAAD | TCGA-FB-AAPZ-01 | 80 | 2 |
| PAAD | TCGA-FB-AAQ0-01 | 99 | 8 |
| PAAD | TCGA-FB-AAQ1-01 | 80 | 2 |
| PAAD | TCGA-FB-AAQ2-01 | 79 | 7 |
| PAAD | TCGA-FB-AAQ3-01 | 89 | 6 |
| PAAD | TCGA-H6-8124-01 | 62 | 5 |
| PAAD | TCGA-H6-A45N-01 | 65 | 2 |
| PAAD | TCGA-HV-A5A3-01 | 62 | 3 |
| PAAD | TCGA-HV-A5A4-01 | 65 | 4 |
| PAAD | TCGA-HV-A5A5-01 | 55 | 6 |
| PAAD | TCGA-HV-A5A6-01 | 77 | 3 |
| PAAD | TCGA-HV-A7OL-01 | 151 | 9 |
| PAAD | TCGA-HV-AA8V-01 | 62 | 6 |
| PAAD | TCGA-HV-AA8X-01 | 101 | 6 |
| PAAD | TCGA-HZ-7919-01 | 100 | 3 |
| PAAD | TCGA-HZ-7922-01 | 75 | 4 |
| PAAD | TCGA-HZ-7925-01 | 336 | 15 |
| PAAD | TCGA-HZ-7926-01 | 157 | 8 |
| PAAD | TCGA-HZ-8001-01 | 97 | 6 |
| PAAD | TCGA-HZ-8002-01 | 119 | 8 |
| PAAD | TCGA-HZ-8003-01 | 80 | 5 |
| PAAD | TCGA-HZ-8005-01 | 138 | 7 |
| PAAD | TCGA-HZ-8315-01 | 95 | 4 |
| PAAD | TCGA-HZ-8317-01 | 87 | 2 |
| PAAD | TCGA-HZ-8519-01 | 47 | 2 |
| PAAD | TCGA-HZ-8636-01 | 108 | 4 |
| PAAD | TCGA-HZ-8637-01 | 116 | 9 |
| PAAD | TCGA-HZ-A49G-01 | 65 | 4 |
| PAAD | TCGA-HZ-A49H-01 | 44 | 4 |
| PAAD | TCGA-HZ-A49I-01 | 70 | 5 |
| PAAD | TCGA-HZ-A48H-01 | 101 | 4 |
| PAAD | TCGA-HZ-A48K-01 | 83 | 4 |
| PAAD | TCGA-HZ-A77O-01 | 60 | 5 |
| PAAD | TCGA-HZ-A77P-01 | 83 | 4 |
| PAAD | TCGA-HZ-A77Q-01 | 438 | 22 |
| PAAD | TCGA-HZ-A8P0-01 | 106 | 6 |
| PAAD | TCGA-HZ-A8P1-01 | 116 | 7 |
| PAAD | TCGA-HZ-A9TJ-01 | 120 | 5 |
| PAAD | TCGA-IB-7644-01 | 98 | 8 |
| PAAD | TCGA-IB-7645-01 | 88 | 4 |
| PAAD | TCGA-IB-7646-01 | 87 | 5 |
| PAAD | TCGA-IB-7647-01 | 103 | 5 |
| PAAD | TCGA-IB-7649-01 | 190 | 7 |
| PAAD | TCGA-IB-7651-01 | 22205 | 777 |
| PAAD | TCGA-IB-7652-01 | 136 | 8 |
| PAAD | TCGA-IB-7885-01 | 298 | 13 |
| PAAD | TCGA-IB-7886-01 | 79 | 8 |
| PAAD | TCGA-IB-7887-01 | 313 | 8 |
| PAAD | TCGA-IB-7888-01 | 129 | 5 |
| PAAD | TCGA-IB-7889-01 | 89 | 9 |
| PAAD | TCGA-IB-7890-01 | 101 | 7 |
| PAAD | TCGA-IB-7891-01 | 70 | 3 |
| PAAD | TCGA-IB-7893-01 | 79 | 3 |
| PAAD | TCGA-IB-7897-01 | 82 | 3 |
| PAAD | TCGA-IB-8126-01 | 80 | 5 |
| PAAD | TCGA-IB-8127-01 | 106 | 10 |
| PAAD | TCGA-IB-A5SO-01 | 109 | 9 |
| PAAD | TCGA-IB-A5SP-01 | 152 | 8 |
| PAAD | TCGA-IB-A5SQ-01 | 117 | 5 |
| PAAD | TCGA-IB-A5SS-01 | 111 | 6 |
| PAAD | TCGA-IB-A5ST-01 | 105 | 4 |
| PAAD | TCGA-IB-A6UF-01 | 148 | 3 |
| PAAD | TCGA-IB-A6UG-01 | 175 | 13 |
| PAAD | TCGA-IB-A7LX-01 | 83 | 5 |
| PAAD | TCGA-IB-A7M4-01 | 115 | 6 |
| PAAD | TCGA-IB-AAUM-01 | 60 | 4 |
| PAAD | TCGA-IB-AAUN-01 | 81 | 5 |
| PAAD | TCGA-IB-AAUO-01 | 105 | 4 |
| PAAD | TCGA-IB-AAUP-01 | 107 | 4 |
| PAAD | TCGA-IB-AAUQ-01 | 97 | 3 |
| PAAD | TCGA-IB-AAUR-01 | 86 | 6 |
| PAAD | TCGA-IB-AAUS-01 | 73 | 11 |
| PAAD | TCGA-IB-AAUT-01 | 60 | 3 |
| PAAD | TCGA-IB-AAUU-01 | 111 | 6 |
| PAAD | TCGA-L8-A7SX-01 | 206 | 10 |
| PAAD | TCGA-LB-A8F3-01 | 123 | 4 |
| PAAD | TCGA-LB-A9Q5-01 | 90 | 6 |
| PAAD | TCGA-M8-A5N4-01 | 64 | 3 |
| PAAD | TCGA-OE-A75W-01 | 230 | 11 |
| PAAD | TCGA-PZ-A5RE-01 | 43 | 6 |
| PAAD | TCGA-Q3-A5QY-01 | 97 | 2 |
| PAAD | TCGA-Q3-AA2A-01 | 78 | 2 |
| PAAD | TCGA-RB-A7B8-01 | 176 | 9 |
| PAAD | TCGA-RB-AA9M-01 | 87 | 8 |
| PAAD | TCGA-S4-A8RM-01 | 101 | 8 |
| PAAD | TCGA-S4-A8RO-01 | 109 | 7 |
| PAAD | TCGA-S4-A8RP-01 | 78 | 5 |
| PAAD | TCGA-US-A774-01 | 124 | 4 |
| PAAD | TCGA-US-A776-01 | 201 | 6 |
| PAAD | TCGA-US-A779-01 | 120 | 10 |
| PAAD | TCGA-US-A77E-01 | 161 | 7 |
| PAAD | TCGA-US-A77G-01 | 118 | 7 |
| PAAD | TCGA-XD-AAUG-01 | 66 | 6 |
| PAAD | TCGA-XD-AAUH-01 | 50 | 5 |
| PAAD | TCGA-XD-AAUI-01 | 78 | 6 |
| PAAD | TCGA-XD-AAUL-01 | 94 | 7 |
| PAAD | TCGA-XN-A8T3-01 | 161 | 6 |
| PAAD | TCGA-XN-A8T5-01 | 92 | 4 |
| PAAD | TCGA-YB-A89D-01 | 294 | 14 |
| PAAD | TCGA-YH-A8SY-01 | 122 | 10 |
| PAAD | TCGA-YY-A8LH-01 | 182 | 7 |
| PAAD | TCGA-Z5-AAPL-01 | 67 | 5 |
| PCPG | TCGA-P7-A5NX-01 | 38 | 2 |
| PCPG | TCGA-P7-A5NY-01 | 18 | 0 |
| PCPG | TCGA-P7-A5NY-05 | 18 | 0 |
| PCPG | TCGA-P8-A5KC-01 | 49 | 2 |
| PCPG | TCGA-P8-A5KD-01 | 30 | 2 |
| PCPG | TCGA-P8-A6RX-01 | 31 | 0 |
| PCPG | TCGA-P8-A6RY-01 | 12 | 2 |
| PCPG | TCGA-PR-A5PF-01 | 34 | 0 |
| PCPG | TCGA-PR-A5PG-01 | 38 | 1 |
| PCPG | TCGA-PR-A5PH-01 | 24 | 2 |
| PCPG | TCGA-QR-A6GO-01 | 20 | 1 |
| PCPG | TCGA-QR-A6GR-01 | 27 | 1 |
| PCPG | TCGA-QR-A6GS-01 | 25 | 1 |
| PCPG | TCGA-QR-A6GT-01 | 53 | 2 |
| PCPG | TCGA-QR-A6GU-01 | 35 | 2 |
| PCPG | TCGA-QR-A6GW-01 | 30 | 3 |
| PCPG | TCGA-QR-A6GX-01 | 23 | 2 |
| PCPG | TCGA-QR-A6GY-01 | 34 | 3 |
| PCPG | TCGA-QR-A6GZ-01 | 21 | 1 |
| PCPG | TCGA-QR-A6GZ-05 | 13 | 0 |
| PCPG | TCGA-QR-A6H0-01 | 19 | 0 |
| PCPG | TCGA-QR-A6H1-01 | 32 | 1 |
| PCPG | TCGA-QR-A6H2-01 | 16 | 1 |
| PCPG | TCGA-QR-A6H3-01 | 13 | 0 |
| PCPG | TCGA-QR-A6H4-01 | 32 | 4 |
| PCPG | TCGA-QR-A6H5-01 | 20 | 1 |
| PCPG | TCGA-QR-A6H6-01 | 25 | 1 |
| PCPG | TCGA-QR-A6ZZ-01 | 24 | 2 |
| PCPG | TCGA-QR-A700-01 | 55 | 1 |
| PCPG | TCGA-QR-A702-01 | 16 | 1 |
| PCPG | TCGA-QR-A703-01 | 17 | 1 |
| PCPG | TCGA-QR-A705-01 | 18 | 0 |

## TABLE 5 (APPENDIX A)

| | | | | | | | | | | | |
|---|---|---|---|---|---|---|---|---|---|---|---|
| PCPG | TCGA-QR-A706-01 | 29 | 0 | PCPG | TCGA-SR-A6MQ-01 | 25 | 2 | PRAD | TCGA-CH-5766-01 | 32 | 1 |
| PCPG | TCGA-QR-A707-01 | 23 | 0 | PCPG | TCGA-SR-A6MR-01 | 18 | 1 | PRAD | TCGA-CH-5767-01 | 33 | 1 |
| PCPG | TCGA-QR-A708-01 | 31 | 2 | PCPG | TCGA-SR-A6MS-01 | 20 | 0 | PRAD | TCGA-CH-5768-01 | 39 | 4 |
| PCPG | TCGA-QR-A70A-01 | 30 | 3 | PCPG | TCGA-SR-A6MT-01 | 22 | 1 | PRAD | TCGA-CH-5769-01 | 66 | 0 |
| PCPG | TCGA-QR-A70C-01 | 23 | 1 | PCPG | TCGA-SR-A6MU-01 | 23 | 1 | PRAD | TCGA-CH-5771-01 | 22 | 1 |
| PCPG | TCGA-QR-A70D-01 | 14 | 2 | PCPG | TCGA-SR-A6MV-01 | 20 | 0 | PRAD | TCGA-CH-5772-01 | 67 | 2 |
| PCPG | TCGA-QR-A70E-01 | 17 | 0 | PCPG | TCGA-SR-A6MX-01 | 36 | 3 | PRAD | TCGA-CH-5788-01 | 44 | 2 |
| PCPG | TCGA-QR-A70G-01 | 23 | 0 | PCPG | TCGA-SR-A6MX-05 | 36 | 2 | PRAD | TCGA-CH-5789-01 | 15 | 0 |
| PCPG | TCGA-QR-A70H-01 | 22 | 2 | PCPG | TCGA-SR-A6MX-06 | 33 | 2 | PRAD | TCGA-CH-5790-01 | 23 | 0 |
| PCPG | TCGA-QR-A70I-01 | 12 | 1 | PCPG | TCGA-SR-A6MY-01 | 22 | 1 | PRAD | TCGA-CH-5791-01 | 38 | 4 |
| PCPG | TCGA-QR-A70J-01 | 33 | 0 | PCPG | TCGA-SR-A6MZ-01 | 19 | 1 | PRAD | TCGA-CH-5792-01 | 29 | 2 |
| PCPG | TCGA-QR-A70K-01 | 40 | 0 | PCPG | TCGA-SR-A6N0-01 | 22 | 2 | PRAD | TCGA-CH-5794-01 | 29 | 2 |
| PCPG | TCGA-QR-A70M-01 | 24 | 1 | PCPG | TCGA-TT-A6YJ-01 | 15 | 0 | PRAD | TCGA-EJ-5494-01 | 23 | 2 |
| PCPG | TCGA-QR-A70N-01 | 22 | 1 | PCPG | TCGA-TT-A6YK-01 | 27 | 0 | PRAD | TCGA-EJ-5495-01 | 77 | 1 |
| PCPG | TCGA-QR-A70O-01 | 34 | 0 | PCPG | TCGA-TT-A6YN-01 | 17 | 0 | PRAD | TCGA-EJ-5496-01 | 42 | 7 |
| PCPG | TCGA-QR-A70P-01 | 46 | 1 | PCPG | TCGA-TT-A6YO-01 | 17 | 0 | PRAD | TCGA-EJ-5497-01 | 27 | 2 |
| PCPG | TCGA-QR-A70Q-01 | 24 | 0 | PCPG | TCGA-TT-A6YP-01 | 14 | 0 | PRAD | TCGA-EJ-5498-01 | 38 | 2 |
| PCPG | TCGA-QR-A70R-01 | 28 | 1 | PCPG | TCGA-W2-A7H5-01 | 11 | 1 | PRAD | TCGA-EJ-5499-01 | 27 | 2 |
| PCPG | TCGA-QR-A70T-01 | 28 | 2 | PCPG | TCGA-W2-A7H7-01 | 26 | 0 | PRAD | TCGA-EJ-5501-01 | 18 | 2 |
| PCPG | TCGA-QR-A70U-01 | 51 | 1 | PCPG | TCGA-W2-A7HA-01 | 41 | 3 | PRAD | TCGA-EJ-5502-01 | 9 | 1 |
| PCPG | TCGA-QR-A70V-01 | 24 | 1 | PCPG | TCGA-W2-A7HB-01 | 14 | 1 | PRAD | TCGA-EJ-5503-01 | 9 | 0 |
| PCPG | TCGA-QR-A70W-01 | 14 | 1 | PCPG | TCGA-W2-A7HC-01 | 36 | 2 | PRAD | TCGA-EJ-5504-01 | 40 | 0 |
| PCPG | TCGA-QR-A70X-01 | 18 | 0 | PCPG | TCGA-W2-A7HD-01 | 25 | 1 | PRAD | TCGA-EJ-5505-01 | 30 | 3 |
| PCPG | TCGA-QR-A7IN-01 | 23 | 0 | PCPG | TCGA-W2-A7HE-01 | 18 | 0 | PRAD | TCGA-EJ-5506-01 | 9 | 0 |
| PCPG | TCGA-QR-A7IP-01 | 31 | 0 | PCPG | TCGA-W2-A7HF-01 | 19 | 1 | PRAD | TCGA-EJ-5507-01 | 26 | 3 |
| PCPG | TCGA-QT-A5XJ-01 | 39 | 0 | PCPG | TCGA-W2-A7HH-01 | 31 | 3 | PRAD | TCGA-EJ-5508-01 | 38 | 1 |
| PCPG | TCGA-QT-A5XK-01 | 31 | 2 | PCPG | TCGA-W2-A7UY-01 | 45 | 3 | PRAD | TCGA-EJ-5509-01 | 30 | 3 |
| PCPG | TCGA-QT-A5XL-01 | 20 | 1 | PCPG | TCGA-W8-A80K-01 | 32 | 1 | PRAD | TCGA-EJ-5510-01 | 19 | 1 |
| PCPG | TCGA-QT-A5XM-01 | 18 | 1 | PCPG | TCGA-W8-A80L-01 | 15 | 1 | PRAD | TCGA-EJ-5511-01 | 43 | 1 |
| PCPG | TCGA-QT-A5XN-01 | 27 | 2 | PCPG | TCGA-W8-A80M-01 | 24 | 0 | PRAD | TCGA-EJ-5512-01 | 18 | 1 |
| PCPG | TCGA-QT-A5XO-01 | 32 | 2 | PCPG | TCGA-W8-A80N-01 | 35 | 2 | PRAD | TCGA-EJ-5514-01 | 33 | 0 |
| PCPG | TCGA-QT-A5XP-01 | 33 | 3 | PCPG | TCGA-W8-A80O-01 | 25 | 3 | PRAD | TCGA-EJ-5515-01 | 19 | 1 |
| PCPG | TCGA-QT-A69Q-01 | 18 | 1 | PCPG | TCGA-W8-A80P-01 | 16 | 0 | PRAD | TCGA-EJ-5516-01 | 33 | 1 |
| PCPG | TCGA-QT-A7U0-01 | 24 | 2 | PCPG | TCGA-W8-A80Q-01 | 27 | 4 | PRAD | TCGA-EJ-5517-01 | 33 | 0 |
| PCPG | TCGA-RM-A68T-01 | 17 | 0 | PCPG | TCGA-W8-A80V-01 | 31 | 1 | PRAD | TCGA-EJ-5518-01 | 38 | 6 |
| PCPG | TCGA-RM-A68W-01 | 22 | 1 | PCPG | TCGA-W8-A80Y-01 | 18 | 0 | PRAD | TCGA-EJ-5519-01 | 89 | 3 |
| PCPG | TCGA-RT-A6Y9-01 | 15 | 1 | PCPG | TCGA-W8-A814-01 | 18 | 0 | PRAD | TCGA-EJ-5521-01 | 47 | 0 |
| PCPG | TCGA-RT-A6YA-01 | 32 | 2 | PCPG | TCGA-W8-A815-01 | 32 | 1 | PRAD | TCGA-EJ-5522-01 | 22 | 0 |
| PCPG | TCGA-RT-A6YC-01 | 13 | 0 | PCPG | TCGA-W8-A816-01 | 24 | 1 | PRAD | TCGA-EJ-5524-01 | 29 | 4 |
| PCPG | TCGA-RW-A67V-01 | 26 | 0 | PCPG | TCGA-W8-A817-01 | 20 | 0 | PRAD | TCGA-EJ-5525-01 | 55 | 5 |
| PCPG | TCGA-RW-A67W-01 | 26 | 1 | PCPG | TCGA-W8-A818-01 | 20 | 1 | PRAD | TCGA-EJ-5526-01 | 25 | 1 |
| PCPG | TCGA-RW-A67X-01 | 32 | 1 | PCPG | TCGA-W8-A819-01 | 22 | 1 | PRAD | TCGA-EJ-5527-01 | 137 | 4 |
| PCPG | TCGA-RW-A67Y-01 | 22 | 1 | PCPG | TCGA-W8-A81A-01 | 21 | 1 | PRAD | TCGA-EJ-5530-01 | 41 | 1 |
| PCPG | TCGA-RW-A680-01 | 52 | 2 | PCPG | TCGA-W8-A81D-01 | 28 | 1 | PRAD | TCGA-EJ-5531-01 | 29 | 2 |
| PCPG | TCGA-RW-A681-01 | 38 | 0 | PCPG | TCGA-W8-A81E-01 | 38 | 0 | PRAD | TCGA-EJ-5532-01 | 20 | 1 |
| PCPG | TCGA-RW-A684-01 | 25 | 5 | PCPG | TCGA-W8-A81F-01 | 13 | 0 | PRAD | TCGA-EJ-5542-01 | 37 | 1 |
| PCPG | TCGA-RW-A685-01 | 21 | 1 | PCPG | TCGA-W8-A81G-01 | 18 | 1 | PRAD | TCGA-EJ-7115-01 | 38 | 1 |
| PCPG | TCGA-RW-A686-01 | 19 | 0 | PCPG | TCGA-W8-A81H-01 | 34 | 2 | PRAD | TCGA-EJ-7123-01 | 179 | 5 |
| PCPG | TCGA-RW-A686-06 | 30 | 2 | PCPG | TCGA-W8-A81I-01 | 23 | 1 | PRAD | TCGA-EJ-7125-01 | 483 | 19 |
| PCPG | TCGA-RW-A688-01 | 27 | 2 | PCPG | TCGA-W8-A81J-01 | 22 | 0 | PRAD | TCGA-EJ-7218-01 | 30 | 1 |
| PCPG | TCGA-RW-A689-01 | 22 | 1 | PCPG | TCGA-W8-A81K-01 | 23 | 0 | PRAD | TCGA-EJ-7314-01 | 32 | 0 |
| PCPG | TCGA-RW-A68A-01 | 21 | 1 | PCPG | TCGA-W8-A81M-01 | 27 | 1 | PRAD | TCGA-EJ-7315-01 | 19 | 2 |
| PCPG | TCGA-RW-A68B-01 | 14 | 0 | PCPG | TCGA-W8-A81N-01 | 18 | 1 | PRAD | TCGA-EJ-7317-01 | 39 | 2 |
| PCPG | TCGA-RW-A68C-01 | 18 | 1 | PCPG | TCGA-W8-A81P-01 | 29 | 4 | PRAD | TCGA-EJ-7318-01 | 27 | 1 |
| PCPG | TCGA-RW-A68D-01 | 19 | 1 | PCPG | TCGA-W8-A81Q-01 | 38 | 2 | PRAD | TCGA-EJ-7321-01 | 31 | 0 |
| PCPG | TCGA-RW-A68F-01 | 38 | 1 | PCPG | TCGA-W8-A81R-01 | 25 | 1 | PRAD | TCGA-EJ-7327-01 | 29 | 2 |
| PCPG | TCGA-RW-A68G-01 | 12 | 0 | PCPG | TCGA-W8-A81S-01 | 23 | 2 | PRAD | TCGA-EJ-7328-01 | 26 | 0 |
| PCPG | TCGA-RW-A7CZ-01 | 28 | 3 | PCPG | TCGA-W8-A81T-01 | 31 | 2 | PRAD | TCGA-EJ-7330-01 | 38 | 2 |
| PCPG | TCGA-RW-A7D0-01 | 21 | 4 | PCPG | TCGA-W8-A81V-01 | 29 | 3 | PRAD | TCGA-EJ-7331-01 | 30 | 3 |
| PCPG | TCGA-RW-A8AZ-01 | 39 | 5 | PCPG | TCGA-W8-A81W-01 | 29 | 2 | PRAD | TCGA-EJ-7781-01 | 58 | 1 |
| PCPG | TCGA-RX-A8JQ-01 | 18 | 2 | PCPG | TCGA-W8-A820-01 | 24 | 1 | PRAD | TCGA-EJ-7782-01 | 242 | 10 |
| PCPG | TCGA-S7-A7WL-01 | 30 | 1 | PCPG | TCGA-W8-A821-01 | 48 | 0 | PRAD | TCGA-EJ-7783-01 | 31 | 4 |
| PCPG | TCGA-S7-A7WM-01 | 18 | 2 | PCPG | TCGA-W8-A822-01 | 15 | 0 | PRAD | TCGA-EJ-7784-01 | 36 | 2 |
| PCPG | TCGA-S7-A7WN-01 | 19 | 2 | PCPG | TCGA-XG-A823-01 | 19 | 2 | PRAD | TCGA-EJ-7785-01 | 16 | 1 |
| PCPG | TCGA-S7-A7WO-01 | 10 | 0 | PRAD | TCGA-2A-A8VL-01 | 19 | 0 | PRAD | TCGA-EJ-7786-01 | 34 | 3 |
| PCPG | TCGA-S7-A7WP-01 | 14 | 0 | PRAD | TCGA-2A-A8VO-01 | 19 | 1 | PRAD | TCGA-EJ-7788-01 | 28 | 1 |
| PCPG | TCGA-S7-A7WQ-01 | 22 | 1 | PRAD | TCGA-2A-A8VT-01 | 37 | 1 | PRAD | TCGA-EJ-7789-01 | 29 | 0 |
| PCPG | TCGA-S7-A7WR-01 | 16 | 1 | PRAD | TCGA-2A-A8VV-01 | 11 | 0 | PRAD | TCGA-EJ-7791-01 | 24 | 2 |
| PCPG | TCGA-S7-A7WT-01 | 33 | 2 | PRAD | TCGA-2A-A8W1-01 | 14 | 2 | PRAD | TCGA-EJ-7792-01 | 15 | 1 |
| PCPG | TCGA-S7-A7WU-01 | 10 | 0 | PRAD | TCGA-2A-A8W3-01 | 25 | 0 | PRAD | TCGA-EJ-7793-01 | 13 | 0 |
| PCPG | TCGA-S7-A7WV-01 | 19 | 1 | PRAD | TCGA-CH-5737-01 | 92 | 5 | PRAD | TCGA-EJ-7794-01 | 15 | 0 |
| PCPG | TCGA-S7-A7WW-01 | 18 | 0 | PRAD | TCGA-CH-5738-01 | 30 | 0 | PRAD | TCGA-EJ-7797-01 | 24 | 2 |
| PCPG | TCGA-S7-A7WX-01 | 19 | 1 | PRAD | TCGA-CH-5739-01 | 943 | 34 | PRAD | TCGA-EJ-8468-01 | 23 | 1 |
| PCPG | TCGA-S7-A7X0-01 | 29 | 2 | PRAD | TCGA-CH-5740-01 | 35 | 2 | PRAD | TCGA-EJ-8469-01 | 30 | 3 |
| PCPG | TCGA-S7-A7X1-01 | 20 | 0 | PRAD | TCGA-CH-5741-01 | 39 | 1 | PRAD | TCGA-EJ-8470-01 | 25 | 2 |
| PCPG | TCGA-S7-A7X2-01 | 28 | 1 | PRAD | TCGA-CH-5744-01 | 32 | 3 | PRAD | TCGA-EJ-8472-01 | 36 | 2 |
| PCPG | TCGA-SA-A6C2-01 | 38 | 0 | PRAD | TCGA-CH-5746-01 | 14 | 1 | PRAD | TCGA-EJ-8474-01 | 37 | 1 |
| PCPG | TCGA-SP-A6QC-01 | 31 | 0 | PRAD | TCGA-CH-5748-01 | 34 | 2 | PRAD | TCGA-EJ-A46D-01 | 11 | 0 |
| PCPG | TCGA-SP-A6QD-01 | 15 | 1 | PRAD | TCGA-CH-5750-01 | 42 | 1 | PRAD | TCGA-EJ-A46G-01 | 41 | 4 |
| PCPG | TCGA-SP-A6QF-01 | 25 | 1 | PRAD | TCGA-CH-5751-01 | 20 | 0 | PRAD | TCGA-EJ-A46I-01 | 5 | 0 |
| PCPG | TCGA-SP-A6QG-01 | 21 | 0 | PRAD | TCGA-CH-5752-01 | 52 | 8 | PRAD | TCGA-EJ-A65E-01 | 30 | 2 |
| PCPG | TCGA-SP-A6QH-01 | 16 | 0 | PRAD | TCGA-CH-5753-01 | 37 | 1 | PRAD | TCGA-EJ-A65F-01 | 73 | 1 |
| PCPG | TCGA-SP-A6QI-01 | 17 | 0 | PRAD | TCGA-CH-5754-01 | 88 | 5 | PRAD | TCGA-EJ-A65G-01 | 20 | 0 |
| PCPG | TCGA-SP-A6QJ-01 | 24 | 1 | PRAD | TCGA-CH-5761-01 | 58 | 1 | PRAD | TCGA-EJ-A65J-01 | 35 | 0 |
| PCPG | TCGA-SP-A6QK-01 | 25 | 4 | PRAD | TCGA-CH-5762-01 | 87 | 5 | PRAD | TCGA-EJ-A6RA-01 | 14 | 1 |
| PCPG | TCGA-SQ-A614-01 | 25 | 2 | PRAD | TCGA-CH-5763-01 | 23 | 2 | PRAD | TCGA-EJ-A6RC-01 | 25 | 0 |
| PCPG | TCGA-SQ-A616-01 | 56 | 3 | PRAD | TCGA-CH-5764-01 | 34 | 3 | PRAD | TCGA-EJ-A7NF-01 | 20 | 0 |
| PCPG | TCGA-SR-A6MP-01 | 43 | 1 | PRAD | TCGA-CH-5765-01 | 32 | 2 | PRAD | TCGA-EJ-A7NG-01 | 2 | 1 |

## TABLE 5 (APPENDIX A)

| | | | |
|---|---|---|---|
| PRAD | TCGA-EJ-A7NH-01 | 16 | 3 |
| PRAD | TCGA-EJ-A7NJ-01 | 12 | 2 |
| PRAD | TCGA-EJ-A7NK-01 | 3 | 1 |
| PRAD | TCGA-EJ-A7NM-01 | 39 | 1 |
| PRAD | TCGA-EJ-A8FN-01 | 29 | 0 |
| PRAD | TCGA-EJ-A8FS-01 | 31 | 2 |
| PRAD | TCGA-EJ-A8FU-01 | 12 | 0 |
| PRAD | TCGA-FC-7708-01 | 23 | 0 |
| PRAD | TCGA-FC-7961-01 | 47 | 4 |
| PRAD | TCGA-FC-A4JI-01 | 24 | 1 |
| PRAD | TCGA-FC-A5OB-01 | 24 | 1 |
| PRAD | TCGA-FC-A8OO-01 | 14 | 0 |
| PRAD | TCGA-G9-6329-01 | 26 | 0 |
| PRAD | TCGA-G9-6333-01 | 59 | 2 |
| PRAD | TCGA-G9-6336-01 | 15 | 1 |
| PRAD | TCGA-G9-6342-01 | 242 | 9 |
| PRAD | TCGA-G9-6348-01 | 31 | 1 |
| PRAD | TCGA-G9-6351-01 | 280 | 12 |
| PRAD | TCGA-G9-6353-01 | 11 | 0 |
| PRAD | TCGA-G9-6356-01 | 29 | 1 |
| PRAD | TCGA-G9-6361-01 | 40 | 1 |
| PRAD | TCGA-G9-6363-01 | 63 | 3 |
| PRAD | TCGA-G9-6364-01 | 16 | 0 |
| PRAD | TCGA-G9-6365-01 | 42 | 2 |
| PRAD | TCGA-G9-6366-01 | 31 | 2 |
| PRAD | TCGA-G9-6367-01 | 3 | 0 |
| PRAD | TCGA-G9-6370-01 | 1 | 0 |
| PRAD | TCGA-G9-6371-01 | 32 | 2 |
| PRAD | TCGA-G9-6377-01 | 119 | 7 |
| PRAD | TCGA-G9-6378-01 | 29 | 1 |
| PRAD | TCGA-G9-6384-01 | 18 | 0 |
| PRAD | TCGA-G9-6385-01 | 19 | 1 |
| PRAD | TCGA-G9-6494-01 | 33 | 3 |
| PRAD | TCGA-G9-6499-01 | 53 | 1 |
| PRAD | TCGA-G9-7510-01 | 30 | 3 |
| PRAD | TCGA-G9-7519-01 | 28 | 1 |
| PRAD | TCGA-G9-7521-01 | 25 | 3 |
| PRAD | TCGA-G9-7522-01 | 11 | 0 |
| PRAD | TCGA-G9-7523-01 | 30 | 1 |
| PRAD | TCGA-H9-7775-01 | 22 | 2 |
| PRAD | TCGA-HC-7075-01 | 45 | 1 |
| PRAD | TCGA-HC-7077-01 | 53 | 3 |
| PRAD | TCGA-HC-7078-01 | 27 | 2 |
| PRAD | TCGA-HC-7079-01 | 20 | 2 |
| PRAD | TCGA-HC-7080-01 | 90 | 3 |
| PRAD | TCGA-HC-7081-01 | 353 | 13 |
| PRAD | TCGA-HC-7209-01 | 17 | 2 |
| PRAD | TCGA-HC-7210-01 | 30 | 1 |
| PRAD | TCGA-HC-7211-01 | 14 | 0 |
| PRAD | TCGA-HC-7212-01 | 26 | 4 |
| PRAD | TCGA-HC-7213-01 | 31 | 1 |
| PRAD | TCGA-HC-7230-01 | 22 | 1 |
| PRAD | TCGA-HC-7231-01 | 32 | 1 |
| PRAD | TCGA-HC-7232-01 | 28 | 2 |
| PRAD | TCGA-HC-7233-01 | 38 | 2 |
| PRAD | TCGA-HC-7736-01 | 22 | 0 |
| PRAD | TCGA-HC-7737-01 | 21 | 0 |
| PRAD | TCGA-HC-7740-01 | 16 | 1 |
| PRAD | TCGA-HC-7742-01 | 19 | 2 |
| PRAD | TCGA-HC-7744-01 | 29 | 3 |
| PRAD | TCGA-HC-7747-01 | 41 | 2 |
| PRAD | TCGA-HC-7748-01 | 24 | 0 |
| PRAD | TCGA-HC-7749-01 | 36 | 1 |
| PRAD | TCGA-HC-7750-01 | 27 | 1 |
| PRAD | TCGA-HC-7818-01 | 27 | 2 |
| PRAD | TCGA-HC-7820-01 | 28 | 1 |
| PRAD | TCGA-HC-7821-01 | 21 | 0 |
| PRAD | TCGA-HC-8213-01 | 15 | 1 |
| PRAD | TCGA-HC-8216-01 | 111 | 6 |
| PRAD | TCGA-HC-8256-01 | 29 | 1 |
| PRAD | TCGA-HC-8257-01 | 36 | 0 |
| PRAD | TCGA-HC-8258-01 | 11 | 1 |
| PRAD | TCGA-HC-8260-01 | 9 | 1 |
| PRAD | TCGA-HC-8261-01 | 29 | 3 |
| PRAD | TCGA-HC-8262-01 | 28 | 2 |
| PRAD | TCGA-HC-8264-01 | 31 | 1 |
| PRAD | TCGA-HC-8265-01 | 61 | 2 |
| PRAD | TCGA-HC-8266-01 | 19 | 0 |
| PRAD | TCGA-HC-A48F-01 | 13 | 3 |
| PRAD | TCGA-HC-A4ZV-01 | 120 | 8 |
| PRAD | TCGA-HC-A631-01 | 33 | 3 |
| PRAD | TCGA-HC-A632-01 | 20 | 2 |
| PRAD | TCGA-HC-A76W-01 | 32 | 0 |
| PRAD | TCGA-HC-A76X-01 | 20 | 0 |
| PRAD | TCGA-HC-A8CY-01 | 29 | 1 |
| PRAD | TCGA-HC-A8D0-01 | 16 | 0 |
| PRAD | TCGA-HC-A8D1-01 | 19 | 2 |
| PRAD | TCGA-HI-7168-01 | 43 | 5 |
| PRAD | TCGA-HI-7170-01 | 26 | 1 |
| PRAD | TCGA-HI-7171-01 | 34 | 4 |
| PRAD | TCGA-J4-8198-01 | 19 | 0 |
| PRAD | TCGA-J4-8200-01 | 18 | 1 |
| PRAD | TCGA-J4-A67T-01 | 14 | 0 |
| PRAD | TCGA-J4-A6G1-01 | 14 | 0 |
| PRAD | TCGA-J4-A6G3-01 | 22 | 4 |
| PRAD | TCGA-J4-A6M7-01 | 14 | 0 |
| PRAD | TCGA-J4-A83I-01 | 28 | 0 |
| PRAD | TCGA-J4-A83K-01 | 19 | 2 |
| PRAD | TCGA-J4-A83M-01 | 31 | 2 |
| PRAD | TCGA-J4-A83N-01 | 20 | 1 |
| PRAD | TCGA-J9-A52B-01 | 25 | 2 |
| PRAD | TCGA-J9-A8CK-01 | 19 | 1 |
| PRAD | TCGA-J9-A8CL-01 | 45 | 2 |
| PRAD | TCGA-J9-A8CM-01 | 65 | 4 |
| PRAD | TCGA-J9-A8CN-01 | 17 | 1 |
| PRAD | TCGA-J9-A8CP-01 | 30 | 1 |
| PRAD | TCGA-KC-A4BL-01 | 23 | 1 |
| PRAD | TCGA-KC-A4BN-01 | 14 | 1 |
| PRAD | TCGA-KC-A4BR-01 | 4 | 1 |
| PRAD | TCGA-KC-A4BV-01 | 30 | 1 |
| PRAD | TCGA-KC-A7F3-01 | 33 | 2 |
| PRAD | TCGA-KC-A7F6-01 | 17 | 1 |
| PRAD | TCGA-KC-A7FA-01 | 36 | 3 |
| PRAD | TCGA-KC-A7FD-01 | 29 | 2 |
| PRAD | TCGA-KC-A7FE-01 | 4 | 0 |
| PRAD | TCGA-KK-A59X-01 | 40 | 6 |
| PRAD | TCGA-KK-A59Y-01 | 9 | 1 |
| PRAD | TCGA-KK-A59Z-01 | 42 | 1 |
| PRAD | TCGA-KK-A6DY-01 | 24 | 2 |
| PRAD | TCGA-KK-A6E0-01 | 21 | 1 |
| PRAD | TCGA-KK-A6E1-01 | 17 | 1 |
| PRAD | TCGA-KK-A6E2-01 | 27 | 0 |
| PRAD | TCGA-KK-A6E5-01 | 19 | 1 |
| PRAD | TCGA-KK-A6E6-01 | 34 | 0 |
| PRAD | TCGA-KK-A7AP-01 | 29 | 2 |
| PRAD | TCGA-KK-A7AU-01 | 16 | 0 |
| PRAD | TCGA-KK-A7AV-01 | 16 | 0 |
| PRAD | TCGA-KK-A7B1-01 | 15 | 1 |
| PRAD | TCGA-KK-A7B3-01 | 25 | 1 |
| PRAD | TCGA-KK-A7B4-01 | 21 | 2 |
| PRAD | TCGA-KK-A8I4-01 | 21 | 1 |
| PRAD | TCGA-KK-A8I5-01 | 26 | 0 |
| PRAD | TCGA-KK-A8I6-01 | 31 | 3 |
| PRAD | TCGA-KK-A8I8-01 | 9 | 1 |
| PRAD | TCGA-KK-A8I9-01 | 42 | 2 |
| PRAD | TCGA-KK-A8IA-01 | 25 | 0 |
| PRAD | TCGA-KK-A8IB-01 | 38 | 1 |
| PRAD | TCGA-KK-A8IC-01 | 16 | 0 |
| PRAD | TCGA-KK-A8ID-01 | 51 | 2 |
| PRAD | TCGA-KK-A8IF-01 | 28 | 3 |
| PRAD | TCGA-KK-A8IG-01 | 29 | 1 |
| PRAD | TCGA-KK-A8IH-01 | 25 | 1 |
| PRAD | TCGA-KK-A8II-01 | 22 | 2 |
| PRAD | TCGA-KK-A8IJ-01 | 28 | 1 |
| PRAD | TCGA-KK-A8IK-01 | 23 | 2 |
| PRAD | TCGA-KK-A8IL-01 | 29 | 2 |
| PRAD | TCGA-M7-A71Y-01 | 16 | 0 |
| PRAD | TCGA-M7-A720-01 | 13 | 1 |
| PRAD | TCGA-M7-A721-01 | 37 | 0 |
| PRAD | TCGA-M7-A725-01 | 25 | 2 |
| PRAD | TCGA-QU-A6IP-01 | 18 | 0 |
| PRAD | TCGA-SU-A7E7-01 | 17 | 0 |
| PRAD | TCGA-TK-A8OK-01 | 31 | 1 |
| PRAD | TCGA-V1-A8MF-01 | 23 | 1 |
| PRAD | TCGA-V1-A8MG-01 | 23 | 1 |
| PRAD | TCGA-V1-A8MJ-01 | 17 | 0 |
| PRAD | TCGA-V1-A8ML-01 | 15 | 1 |
| PRAD | TCGA-V1-A8MU-01 | 20 | 1 |
| PRAD | TCGA-V1-A8WL-01 | 17 | 3 |
| PRAD | TCGA-V1-A8WN-01 | 12 | 0 |
| PRAD | TCGA-V1-A8WS-01 | 19 | 1 |
| PRAD | TCGA-V1-A8WV-01 | 39 | 1 |
| PRAD | TCGA-V1-A8WW-01 | 28 | 2 |
| PRAD | TCGA-V1-A8X3-01 | 20 | 0 |
| PRAD | TCGA-VN-A88I-01 | 12 | 1 |
| PRAD | TCGA-VN-A88K-01 | 20 | 0 |
| PRAD | TCGA-VN-A88L-01 | 22 | 1 |
| PRAD | TCGA-VN-A88N-01 | 19 | 2 |
| PRAD | TCGA-VN-A88O-01 | 28 | 1 |
| PRAD | TCGA-VN-A88P-01 | 36 | 0 |
| PRAD | TCGA-VN-A88Q-01 | 27 | 2 |
| PRAD | TCGA-VN-A88R-01 | 25 | 3 |
| PRAD | TCGA-VP-A872-01 | 15 | 0 |
| PRAD | TCGA-VP-A875-01 | 29 | 1 |
| PRAD | TCGA-VP-A876-01 | 20 | 1 |
| PRAD | TCGA-VP-A878-01 | 28 | 4 |
| PRAD | TCGA-VP-A879-01 | 40 | 2 |
| PRAD | TCGA-VP-A87B-01 | 27 | 1 |
| PRAD | TCGA-VP-A87C-01 | 19 | 1 |
| PRAD | TCGA-VP-A87D-01 | 39 | 1 |
| PRAD | TCGA-VP-A87E-01 | 26 | 1 |
| PRAD | TCGA-VP-A87H-01 | 24 | 4 |
| PRAD | TCGA-VP-A87J-01 | 26 | 4 |
| PRAD | TCGA-VP-A87K-01 | 27 | 1 |
| PRAD | TCGA-WW-A8ZI-01 | 28 | 1 |
| PRAD | TCGA-XA-A8JR-01 | 23 | 1 |
| PRAD | TCGA-XJ-A83G-01 | 23 | 1 |
| PRAD | TCGA-XJ-A83H-01 | 32 | 1 |
| PRAD | TCGA-XJ-A9DI-01 | 14 | 1 |
| PRAD | TCGA-XJ-A9DK-01 | 6 | 1 |
| PRAD | TCGA-XQ-A8TA-01 | 104 | 6 |
| PRAD | TCGA-XQ-A8TB-01 | 23 | 0 |
| PRAD | TCGA-Y6-A8TL-01 | 23 | 4 |
| PRAD | TCGA-YJ-A8SW-01 | 46 | 3 |
| PRAD | TCGA-YL-A8HJ-01 | 20 | 0 |
| PRAD | TCGA-YL-A8HK-01 | 13 | 1 |
| PRAD | TCGA-YL-A8HL-01 | 15 | 2 |
| PRAD | TCGA-YL-A8HM-01 | 30 | 1 |
| PRAD | TCGA-YL-A8HO-01 | 31 | 1 |
| PRAD | TCGA-YL-A8S8-01 | 29 | 2 |
| PRAD | TCGA-YL-A8S9-01 | 25 | 1 |
| PRAD | TCGA-YL-A8SA-01 | 23 | 0 |
| PRAD | TCGA-YL-A8SB-01 | 24 | 0 |
| PRAD | TCGA-YL-A8SC-01 | 26 | 3 |
| PRAD | TCGA-YL-A8SF-01 | 8 | 0 |
| PRAD | TCGA-YL-A8SH-01 | 28 | 4 |
| PRAD | TCGA-YL-A8SJ-01 | 21 | 2 |
| PRAD | TCGA-YL-A8SK-01 | 29 | 2 |
| PRAD | TCGA-YL-A8SL-01 | 38 | 1 |
| PRAD | TCGA-YL-A8SO-01 | 41 | 3 |
| PRAD | TCGA-YL-A8SP-01 | 20 | 2 |
| PRAD | TCGA-YL-A8SQ-01 | 32 | 1 |
| PRAD | TCGA-YL-A8SR-01 | 24 | 1 |
| PRAD | TCGA-YL-A9WH-01 | 42 | 7 |
| PRAD | TCGA-YL-A9WI-01 | 36 | 3 |
| PRAD | TCGA-YL-A9WJ-01 | 25 | 1 |
| PRAD | TCGA-ZG-A8QW-01 | 29 | 2 |
| PRAD | TCGA-ZG-A8QX-01 | 24 | 2 |
| PRAD | TCGA-ZG-A8QY-01 | 49 | 3 |
| PRAD | TCGA-ZG-A8QZ-01 | 21 | 0 |
| READ | TCGA-AF-2689-01 | 64 | 3 |
| READ | TCGA-AF-2691-01 | 73 | 9 |
| READ | TCGA-AF-2692-01 | 45 | 3 |
| READ | TCGA-AF-3400-01 | 33 | 2 |
| READ | TCGA-AF-3913-01 | 118 | 6 |
| READ | TCGA-AG-3574-01 | 44 | 2 |
| READ | TCGA-AG-3575-01 | 51 | 5 |
| READ | TCGA-AG-3578-01 | 29 | 3 |
| READ | TCGA-AG-3580-01 | 44 | 2 |
| READ | TCGA-AG-3581-01 | 54 | 4 |
| READ | TCGA-AG-3582-01 | 35 | 2 |
| READ | TCGA-AG-3583-01 | 67 | 5 |
| READ | TCGA-AG-3584-01 | 35 | 1 |
| READ | TCGA-AG-3586-01 | 83 | 5 |
| READ | TCGA-AG-3587-01 | 69 | 3 |
| READ | TCGA-AG-3593-01 | 78 | 3 |
| READ | TCGA-AG-3594-01 | 46 | 6 |
| READ | TCGA-AG-3598-01 | 75 | 7 |
| READ | TCGA-AG-3599-01 | 67 | 7 |
| READ | TCGA-AG-3600-01 | 97 | 6 |
| READ | TCGA-AG-3601-01 | 107 | 13 |
| READ | TCGA-AG-3602-01 | 49 | 5 |
| READ | TCGA-AG-3605-01 | 68 | 5 |
| READ | TCGA-AG-3608-01 | 61 | 6 |
| READ | TCGA-AG-3609-01 | 97 | 8 |
| READ | TCGA-AG-3611-01 | 54 | 5 |
| READ | TCGA-AG-3612-01 | 73 | 4 |
| READ | TCGA-AG-3726-01 | 158 | 10 |
| READ | TCGA-AG-3727-01 | 85 | 6 |
| READ | TCGA-AG-3878-01 | 91 | 9 |
| READ | TCGA-AG-3881-01 | 102 | 8 |
| READ | TCGA-AG-3882-01 | 68 | 7 |
| READ | TCGA-AG-3883-01 | 133 | 4 |
| READ | TCGA-AG-3887-01 | 79 | 6 |
| READ | TCGA-AG-3890-01 | 82 | 7 |
| READ | TCGA-AG-3892-01 | 2267 | 97 |
| READ | TCGA-AG-3893-01 | 118 | 5 |
| READ | TCGA-AG-3894-01 | 106 | 2 |
| READ | TCGA-AG-3896-01 | 107 | 8 |
| READ | TCGA-AG-3898-01 | 111 | 6 |
| READ | TCGA-AG-3901-01 | 68 | 6 |
| READ | TCGA-AG-3902-01 | 136 | 7 |
| READ | TCGA-AG-3909-01 | 92 | 8 |
| READ | TCGA-AG-3999-01 | 110 | 4 |

# EP 3 423 828 B1

## TABLE 5 (APPENDIX A)

| Type | Sample | Value 1 | Value 2 |
|---|---|---|---|
| READ | TCGA-AG-4001-01 | 127 | 12 |
| READ | TCGA-AG-4005-01 | 127 | 8 |
| READ | TCGA-AG-4007-01 | 171 | 9 |
| READ | TCGA-AG-4008-01 | 81 | 4 |
| READ | TCGA-AG-4015-01 | 104 | 7 |
| READ | TCGA-AG-A002-01 | 12543 | 423 |
| READ | TCGA-AG-A008-01 | 49 | 5 |
| READ | TCGA-AG-A00C-01 | 69 | 6 |
| READ | TCGA-AG-A00H-01 | 79 | 5 |
| READ | TCGA-AG-A00Y-01 | 284 | 16 |
| READ | TCGA-AG-A011-01 | 115 | 9 |
| READ | TCGA-AG-A014-01 | 159 | 8 |
| READ | TCGA-AG-A015-01 | 64 | 6 |
| READ | TCGA-AG-A016-01 | 58 | 4 |
| READ | TCGA-AG-A01L-01 | 77 | 9 |
| READ | TCGA-AG-A01W-01 | 127 | 6 |
| READ | TCGA-AG-A01Y-01 | 95 | 6 |
| READ | TCGA-AG-A020-01 | 81 | 9 |
| READ | TCGA-AG-A025-01 | 100 | 7 |
| READ | TCGA-AG-A026-01 | 225 | 8 |
| READ | TCGA-AG-A02G-01 | 61 | 3 |
| READ | TCGA-AG-A02N-01 | 1251 | 52 |
| READ | TCGA-AG-A02X-01 | 154 | 10 |
| READ | TCGA-AG-A032-01 | 91 | 6 |
| READ | TCGA-AG-A036-01 | 154 | 13 |
| SARC | TCGA-3B-A9HI-01 | 75 | 3 |
| SARC | TCGA-3B-A9HJ-01 | 27 | 1 |
| SARC | TCGA-3B-A9HL-01 | 80 | 3 |
| SARC | TCGA-3B-A9HO-01 | 83 | 3 |
| SARC | TCGA-3B-A9HP-01 | 84 | 2 |
| SARC | TCGA-3B-A9HQ-01 | 105 | 6 |
| SARC | TCGA-3B-A9HR-01 | 71 | 1 |
| SARC | TCGA-3B-A9HS-01 | 94 | 5 |
| SARC | TCGA-3B-A9HT-01 | 429 | 19 |
| SARC | TCGA-3B-A9HU-01 | 64 | 2 |
| SARC | TCGA-3B-A9HV-01 | 77 | 3 |
| SARC | TCGA-3B-A9HX-01 | 65 | 5 |
| SARC | TCGA-3B-A9HY-01 | 86 | 4 |
| SARC | TCGA-3B-A9HZ-01 | 58 | 4 |
| SARC | TCGA-3B-A9I0-01 | 67 | 4 |
| SARC | TCGA-3B-A9I1-01 | 67 | 7 |
| SARC | TCGA-3B-A9I3-01 | 73 | 5 |
| SARC | TCGA-3R-A8YX-01 | 128 | 5 |
| SARC | TCGA-DX-A1KU-01 | 87 | 0 |
| SARC | TCGA-DX-A1KW-01 | 72 | 2 |
| SARC | TCGA-DX-A1KX-01 | 103 | 3 |
| SARC | TCGA-DX-A1KY-01 | 135 | 6 |
| SARC | TCGA-DX-A1KZ-01 | 56 | 0 |
| SARC | TCGA-DX-A1L0-01 | 116 | 6 |
| SARC | TCGA-DX-A1L1-01 | 113 | 2 |
| SARC | TCGA-DX-A1L2-01 | 160 | 3 |
| SARC | TCGA-DX-A1L3-01 | 87 | 3 |
| SARC | TCGA-DX-A1L4-01 | 34 | 1 |
| SARC | TCGA-DX-A23R-01 | 59 | 1 |
| SARC | TCGA-DX-A23T-01 | 28 | 1 |
| SARC | TCGA-DX-A23U-01 | 57 | 3 |
| SARC | TCGA-DX-A23V-01 | 36 | 1 |
| SARC | TCGA-DX-A23Y-01 | 79 | 4 |
| SARC | TCGA-DX-A240-01 | 34 | 0 |
| SARC | TCGA-DX-A2IZ-01 | 62 | 3 |
| SARC | TCGA-DX-A2J0-01 | 67 | 3 |
| SARC | TCGA-DX-A2J1-01 | 49 | 3 |
| SARC | TCGA-DX-A2J4-01 | 45 | 1 |
| SARC | TCGA-DX-A3LS-01 | 77 | 3 |
| SARC | TCGA-DX-A3LT-01 | 52 | 3 |
| SARC | TCGA-DX-A3LU-01 | 51 | 4 |
| SARC | TCGA-DX-A3LW-01 | 43 | 2 |
| SARC | TCGA-DX-A3LY-01 | 77 | 2 |
| SARC | TCGA-DX-A3M1-01 | 84 | 0 |
| SARC | TCGA-DX-A3M2-01 | 118 | 6 |
| SARC | TCGA-DX-A3U5-01 | 39 | 1 |
| SARC | TCGA-DX-A3U6-01 | 16 | 0 |
| SARC | TCGA-DX-A3U7-01 | 54 | 1 |
| SARC | TCGA-DX-A3U8-01 | 58 | 2 |
| SARC | TCGA-DX-A3U9-01 | 99 | 2 |
| SARC | TCGA-DX-A3UA-01 | 67 | 5 |
| SARC | TCGA-DX-A3UB-01 | 37 | 0 |
| SARC | TCGA-DX-A3UC-01 | 86 | 3 |
| SARC | TCGA-DX-A3UD-01 | 57 | 3 |
| SARC | TCGA-DX-A3UE-01 | 86 | 4 |
| SARC | TCGA-DX-A3UF-01 | 74 | 3 |
| SARC | TCGA-DX-A4BJ-01 | 47 | 2 |
| SARC | TCGA-DX-A4BK-01 | 67 | 1 |
| SARC | TCGA-DX-A4BL-01 | 101 | 4 |
| SARC | TCGA-DX-A4BN-01 | 59 | 1 |
| SARC | TCGA-DX-A4BO-01 | 88 | 4 |
| SARC | TCGA-DX-A4BP-01 | 84 | 1 |
| SARC | TCGA-DX-A4BR-01 | 75 | 2 |
| SARC | TCGA-DX-A48U-01 | 51 | 3 |
| SARC | TCGA-DX-A48V-01 | 57 | 1 |
| SARC | TCGA-DX-A6B7-01 | 55 | 5 |
| SARC | TCGA-DX-A6B8-01 | 95 | 4 |
| SARC | TCGA-DX-A6B9-01 | 62 | 3 |
| SARC | TCGA-DX-A6BA-01 | 75 | 3 |
| SARC | TCGA-DX-A6BB-01 | 72 | 1 |
| SARC | TCGA-DX-A6BE-01 | 55 | 2 |
| SARC | TCGA-DX-A6BF-01 | 107 | 3 |
| SARC | TCGA-DX-A6BG-01 | 27 | 1 |
| SARC | TCGA-DX-A6BK-01 | 61 | 2 |
| SARC | TCGA-DX-A6YQ-01 | 212 | 8 |
| SARC | TCGA-DX-A6YR-01 | 115 | 1 |
| SARC | TCGA-DX-A6YU-01 | 124 | 4 |
| SARC | TCGA-DX-A6YX-01 | 79 | 2 |
| SARC | TCGA-DX-A6YZ-01 | 99 | 5 |
| SARC | TCGA-DX-A6Z0-01 | 75 | 2 |
| SARC | TCGA-DX-A6Z2-01 | 93 | 5 |
| SARC | TCGA-DX-A7EF-01 | 89 | 4 |
| SARC | TCGA-DX-A7EI-01 | 77 | 3 |
| SARC | TCGA-DX-A7EL-01 | 110 | 4 |
| SARC | TCGA-DX-A7EM-01 | 120 | 2 |
| SARC | TCGA-DX-A7EN-01 | 53 | 3 |
| SARC | TCGA-DX-A7EO-01 | 48 | 0 |
| SARC | TCGA-DX-A7EQ-01 | 69 | 3 |
| SARC | TCGA-DX-A7ER-01 | 59 | 2 |
| SARC | TCGA-DX-A7ES-01 | 65 | 2 |
| SARC | TCGA-DX-A7ET-01 | 77 | 1 |
| SARC | TCGA-DX-A7EU-01 | 114 | 8 |
| SARC | TCGA-DX-A88G-01 | 103 | 5 |
| SARC | TCGA-DX-A88H-01 | 115 | 8 |
| SARC | TCGA-DX-A88J-01 | 113 | 2 |
| SARC | TCGA-DX-A88K-01 | 85 | 2 |
| SARC | TCGA-DX-A88L-01 | 144 | 6 |
| SARC | TCGA-DX-A88M-01 | 114 | 3 |
| SARC | TCGA-DX-A88N-01 | 92 | 2 |
| SARC | TCGA-DX-A88O-01 | 63 | 1 |
| SARC | TCGA-DX-A88P-01 | 951 | 30 |
| SARC | TCGA-DX-A88Q-01 | 90 | 3 |
| SARC | TCGA-DX-A88R-01 | 97 | 3 |
| SARC | TCGA-DX-A88S-01 | 93 | 5 |
| SARC | TCGA-DX-A88T-01 | 178 | 5 |
| SARC | TCGA-DX-A88U-01 | 115 | 4 |
| SARC | TCGA-DX-A88V-01 | 107 | 5 |
| SARC | TCGA-DX-A88X-01 | 134 | 7 |
| SARC | TCGA-DX-A88Z-01 | 107 | 3 |
| SARC | TCGA-DX-AATS-01 | 164 | 6 |
| SARC | TCGA-DX-AB2E-01 | 1310 | 34 |
| SARC | TCGA-DX-AB2F-01 | 70 | 1 |
| SARC | TCGA-DX-AB2G-01 | 57 | 2 |
| SARC | TCGA-DX-AB2H-01 | 91 | 1 |
| SARC | TCGA-DX-AB2J-01 | 42 | 4 |
| SARC | TCGA-DX-AB2O-01 | 101 | 7 |
| SARC | TCGA-DX-AB2P-01 | 66 | 2 |
| SARC | TCGA-DX-AB2Q-01 | 140 | 6 |
| SARC | TCGA-DX-AB2S-01 | 32 | 1 |
| SARC | TCGA-DX-AB2T-01 | 94 | 1 |
| SARC | TCGA-DX-AB2V-01 | 132 | 3 |
| SARC | TCGA-DX-AB2W-01 | 190 | 6 |
| SARC | TCGA-DX-AB2X-01 | 89 | 6 |
| SARC | TCGA-DX-AB2Z-01 | 107 | 5 |
| SARC | TCGA-DX-AB30-01 | 38 | 4 |
| SARC | TCGA-DX-AB32-01 | 419 | 15 |
| SARC | TCGA-DX-AB35-01 | 103 | 2 |
| SARC | TCGA-DX-AB36-01 | 63 | 0 |
| SARC | TCGA-DX-AB37-01 | 85 | 3 |
| SARC | TCGA-DX-AB3A-01 | 86 | 4 |
| SARC | TCGA-DX-AB3B-01 | 64 | 2 |
| SARC | TCGA-DX-AB3C-01 | 40 | 3 |
| SARC | TCGA-FX-A2QS-01 | 88 | 1 |
| SARC | TCGA-FX-A3NJ-01 | 48 | 2 |
| SARC | TCGA-FX-A3NK-01 | 40 | 2 |
| SARC | TCGA-FX-A3RE-01 | 142 | 1 |
| SARC | TCGA-FX-A3TO-01 | 100 | 8 |
| SARC | TCGA-FX-A48G-01 | 91 | 2 |
| SARC | TCGA-FX-A76Y-01 | 54 | 2 |
| SARC | TCGA-FX-A8OO-01 | 73 | 2 |
| SARC | TCGA-HB-A2OT-01 | 98 | 5 |
| SARC | TCGA-HB-A3L4-01 | 64 | 4 |
| SARC | TCGA-HB-A3YV-01 | 82 | 4 |
| SARC | TCGA-HB-A43Z-01 | 81 | 4 |
| SARC | TCGA-HB-A5W3-01 | 55 | 3 |
| SARC | TCGA-HS-A5N7-01 | 70 | 6 |
| SARC | TCGA-HS-A5N8-01 | 95 | 4 |
| SARC | TCGA-HS-A5N9-01 | 558 | 25 |
| SARC | TCGA-IE-A3OV-01 | 53 | 0 |
| SARC | TCGA-IE-A4EH-01 | 54 | 2 |
| SARC | TCGA-IE-A4EI-01 | 44 | 3 |
| SARC | TCGA-IE-A4EJ-01 | 71 | 6 |
| SARC | TCGA-IE-A4EK-01 | 47 | 3 |
| SARC | TCGA-IE-A6BZ-01 | 66 | 1 |
| SARC | TCGA-IF-A4AJ-01 | 80 | 1 |
| SARC | TCGA-IF-A4AK-01 | 53 | 0 |
| SARC | TCGA-IS-A3K6-01 | 64 | 2 |
| SARC | TCGA-IS-A3K7-01 | 135 | 5 |
| SARC | TCGA-IS-A3K8-01 | 23 | 0 |
| SARC | TCGA-IS-A3KA-01 | 96 | 3 |
| SARC | TCGA-IW-A3M4-01 | 48 | 0 |
| SARC | TCGA-IW-A3M5-01 | 76 | 5 |
| SARC | TCGA-IW-A3M6-01 | 89 | 3 |
| SARC | TCGA-JV-A5VE-01 | 66 | 4 |
| SARC | TCGA-JV-A5VF-01 | 52 | 2 |
| SARC | TCGA-JV-A75J-01 | 148 | 6 |
| SARC | TCGA-K1-A3PN-01 | 54 | 1 |
| SARC | TCGA-K1-A3PN-02 | 69 | 2 |
| SARC | TCGA-K1-A3PO-01 | 38 | 0 |
| SARC | TCGA-K1-A42W-01 | 69 | 4 |
| SARC | TCGA-K1-A42X-01 | 95 | 7 |
| SARC | TCGA-K1-A6RT-01 | 75 | 2 |
| SARC | TCGA-K1-A6RU-01 | 58 | 5 |
| SARC | TCGA-K1-A6RV-01 | 109 | 2 |
| SARC | TCGA-KD-A5QS-01 | 105 | 3 |
| SARC | TCGA-KD-A5QT-01 | 60 | 4 |
| SARC | TCGA-KD-A5QU-01 | 91 | 4 |
| SARC | TCGA-KF-A41W-01 | 94 | 7 |
| SARC | TCGA-LI-A67I-01 | 66 | 4 |
| SARC | TCGA-LI-A9QH-01 | 168 | 4 |
| SARC | TCGA-MB-A5Y8-01 | 92 | 2 |
| SARC | TCGA-MB-A5Y9-01 | 38 | 2 |
| SARC | TCGA-MB-A5YA-01 | 43 | 0 |
| SARC | TCGA-MB-A8JK-01 | 83 | 5 |
| SARC | TCGA-MB-A8JL-01 | 64 | 3 |
| SARC | TCGA-MJ-A68H-01 | 71 | 2 |
| SARC | TCGA-MJ-A68J-01 | 24 | 2 |
| SARC | TCGA-MJ-A850-01 | 40 | 0 |
| SARC | TCGA-MO-A47P-01 | 32 | 1 |
| SARC | TCGA-MO-A47R-01 | 69 | 6 |
| SARC | TCGA-N1-A6IA-01 | 60 | 1 |
| SARC | TCGA-PC-A5DK-01 | 39 | 1 |
| SARC | TCGA-PC-A5DL-01 | 52 | 0 |
| SARC | TCGA-PC-A5DM-01 | 65 | 3 |
| SARC | TCGA-PC-A5DN-01 | 74 | 1 |
| SARC | TCGA-PC-A5DO-01 | 63 | 3 |
| SARC | TCGA-PC-A5DP-01 | 63 | 2 |
| SARC | TCGA-PT-A8TR-01 | 74 | 2 |
| SARC | TCGA-QC-A6FX-01 | 71 | 5 |
| SARC | TCGA-QC-A7B5-01 | 2821 | 67 |
| SARC | TCGA-QC-AA9N-01 | 78 | 2 |
| SARC | TCGA-QQ-A5V2-01 | 52 | 0 |
| SARC | TCGA-QQ-A5V9-01 | 97 | 2 |
| SARC | TCGA-QQ-A5VA-01 | 88 | 6 |
| SARC | TCGA-QQ-A5VB-01 | 109 | 8 |
| SARC | TCGA-QQ-A5VC-01 | 118 | 9 |
| SARC | TCGA-QQ-A5VD-01 | 580 | 22 |
| SARC | TCGA-QQ-A8VB-01 | 142 | 5 |
| SARC | TCGA-QQ-A8VD-01 | 29 | 1 |
| SARC | TCGA-QQ-A8VG-01 | 552 | 18 |
| SARC | TCGA-QQ-A8VH-01 | 74 | 5 |
| SARC | TCGA-RN-A68Q-01 | 26 | 2 |
| SARC | TCGA-RN-AAAQ-01 | 78 | 4 |
| SARC | TCGA-SG-A6Z4-01 | 150 | 4 |
| SARC | TCGA-SG-A6Z7-01 | 94 | 6 |
| SARC | TCGA-SG-A849-01 | 86 | 3 |
| SARC | TCGA-SI-A71O-01 | 91 | 3 |
| SARC | TCGA-SI-A71P-01 | 83 | 5 |
| SARC | TCGA-SI-A71Q-01 | 104 | 6 |
| SARC | TCGA-SI-AA8B-01 | 88 | 5 |
| SARC | TCGA-SI-AA8C-01 | 57 | 2 |
| SARC | TCGA-UE-A6QT-01 | 37 | 3 |
| SARC | TCGA-UE-A6QU-01 | 92 | 2 |
| SARC | TCGA-VT-A80G-01 | 95 | 1 |
| SARC | TCGA-VT-A80J-01 | 54 | 3 |
| SARC | TCGA-VT-A80J-02 | 76 | 1 |
| SARC | TCGA-VT-AB3D-01 | 78 | 4 |
| SARC | TCGA-WK-A8XO-01 | 143 | 3 |
| SARC | TCGA-WK-A8XQ-01 | 51 | 1 |
| SARC | TCGA-WK-A8XS-01 | 85 | 6 |
| SARC | TCGA-WK-A8XT-01 | 85 | 2 |
| SARC | TCGA-WK-A8XX-01 | 117 | 8 |
| SARC | TCGA-WK-A8XY-01 | 88 | 3 |
| SARC | TCGA-WK-A8XZ-01 | 78 | 6 |
| SARC | TCGA-WK-A8Y0-01 | 91 | 8 |
| SARC | TCGA-WP-A9GB-01 | 212 | 8 |
| SARC | TCGA-X2-A95T-01 | 129 | 3 |

## TABLE 5 (APPENDIX A)

| Type | Sample | | |
|---|---|---|---|
| SARC | TCGA-X6-A7W8-01 | 124 | 2 |
| SARC | TCGA-X6-A7WC-01 | 136 | 7 |
| SARC | TCGA-X6-A7WD-01 | 73 | 3 |
| SARC | TCGA-X6-A8C2-01 | 148 | 7 |
| SARC | TCGA-X6-A8C3-01 | 102 | 1 |
| SARC | TCGA-X6-A8C4-01 | 131 | 1 |
| SARC | TCGA-X6-A8C5-01 | 123 | 2 |
| SARC | TCGA-X6-A8C6-01 | 110 | 2 |
| SARC | TCGA-X6-A8C7-01 | 60 | 1 |
| SARC | TCGA-X9-A971-01 | 90 | 6 |
| SARC | TCGA-X9-A973-01 | 73 | 4 |
| SARC | TCGA-Z4-A8JB-01 | 59 | 2 |
| SARC | TCGA-Z4-A9VC-01 | 64 | 4 |
| SARC | TCGA-Z4-AAPF-01 | 55 | 2 |
| SARC | TCGA-Z4-AAPG-01 | 88 | 2 |
| SKCM | TCGA-BF-A1PU-01 | 110 | 6 |
| SKCM | TCGA-BF-A1PV-01 | 372 | 13 |
| SKCM | TCGA-BF-A1PX-01 | 470 | 11 |
| SKCM | TCGA-BF-A1PZ-01 | 321 | 11 |
| SKCM | TCGA-BF-A1Q0-01 | 1063 | 40 |
| SKCM | TCGA-BF-A3DJ-01 | 184 | 7 |
| SKCM | TCGA-BF-A3DL-01 | 585 | 23 |
| SKCM | TCGA-BF-A3DM-01 | 610 | 23 |
| SKCM | TCGA-BF-A3DN-01 | 106 | 4 |
| SKCM | TCGA-BF-A5EO-01 | 773 | 24 |
| SKCM | TCGA-BF-A5EQ-01 | 776 | 22 |
| SKCM | TCGA-D3-A1Q1-06 | 112 | 4 |
| SKCM | TCGA-D3-A1Q3-06 | 165 | 10 |
| SKCM | TCGA-D3-A1Q4-06 | 330 | 12 |
| SKCM | TCGA-D3-A1Q5-06 | 452 | 10 |
| SKCM | TCGA-D3-A1Q6-06 | 1086 | 31 |
| SKCM | TCGA-D3-A1Q7-06 | 131 | 11 |
| SKCM | TCGA-D3-A1Q8-06 | 299 | 11 |
| SKCM | TCGA-D3-A1Q9-06 | 54 | 4 |
| SKCM | TCGA-D3-A1QA-06 | 660 | 25 |
| SKCM | TCGA-D3-A1QB-06 | 453 | 16 |
| SKCM | TCGA-D3-A2J6-06 | 112 | 15 |
| SKCM | TCGA-D3-A2J7-06 | 428 | 16 |
| SKCM | TCGA-D3-A2J8-06 | 781 | 19 |
| SKCM | TCGA-D3-A2J9-06 | 103 | 3 |
| SKCM | TCGA-D3-A2JA-06 | 441 | 19 |
| SKCM | TCGA-D3-A2JB-06 | 56 | 2 |
| SKCM | TCGA-D3-A2JC-06 | 298 | 11 |
| SKCM | TCGA-D3-A2JD-06 | 502 | 15 |
| SKCM | TCGA-D3-A2JF-06 | 1770 | 57 |
| SKCM | TCGA-D3-A2JG-06 | 150 | 8 |
| SKCM | TCGA-D3-A2JH-06 | 786 | 31 |
| SKCM | TCGA-D3-A2JK-06 | 369 | 11 |
| SKCM | TCGA-D3-A2JL-06 | 403 | 13 |
| SKCM | TCGA-D3-A2JN-06 | 399 | 13 |
| SKCM | TCGA-D3-A2JO-06 | 798 | 30 |
| SKCM | TCGA-D3-A2JP-06 | 326 | 13 |
| SKCM | TCGA-D3-A38Z-06 | 88 | 0 |
| SKCM | TCGA-D3-A3C1-06 | 189 | 5 |
| SKCM | TCGA-D3-A3C3-06 | 326 | 15 |
| SKCM | TCGA-D3-A3C6-06 | 173 | 11 |
| SKCM | TCGA-D3-A3C7-06 | 1160 | 36 |
| SKCM | TCGA-D3-A3C8-06 | 849 | 34 |
| SKCM | TCGA-D3-A3CB-06 | 336 | 11 |
| SKCM | TCGA-D3-A3CC-06 | 48 | 2 |
| SKCM | TCGA-D3-A3CE-06 | 110 | 8 |
| SKCM | TCGA-D3-A3CF-06 | 44 | 1 |
| SKCM | TCGA-D3-A3ML-06 | 1141 | 33 |
| SKCM | TCGA-D3-A3MO-06 | 93 | 7 |
| SKCM | TCGA-D3-A3MR-06 | 1084 | 36 |
| SKCM | TCGA-D3-A3MU-06 | 595 | 25 |
| SKCM | TCGA-D3-A3MV-06 | 671 | 28 |
| SKCM | TCGA-D3-A51E-06 | 347 | 13 |
| SKCM | TCGA-D3-A51F-06 | 150 | 5 |
| SKCM | TCGA-D3-A51G-06 | 1530 | 37 |
| SKCM | TCGA-D3-A51H-06 | 78 | 4 |
| SKCM | TCGA-D3-A51J-06 | 558 | 15 |
| SKCM | TCGA-D3-A51K-06 | 37 | 3 |
| SKCM | TCGA-D3-A51N-06 | 136 | 9 |
| SKCM | TCGA-D3-A51R-06 | 893 | 33 |
| SKCM | TCGA-D3-A51T-06 | 1457 | 50 |
| SKCM | TCGA-D3-A5GN-06 | 610 | 23 |
| SKCM | TCGA-D3-A5GO-06 | 1974 | 62 |
| SKCM | TCGA-D3-A5GR-06 | 343 | 17 |
| SKCM | TCGA-D3-A5GS-06 | 495 | 19 |
| SKCM | TCGA-D3-A5GT-01 | 41 | 1 |
| SKCM | TCGA-D3-A5GU-06 | 884 | 39 |
| SKCM | TCGA-D9-A148-06 | 362 | 14 |
| SKCM | TCGA-D9-A149-06 | 304 | 15 |
| SKCM | TCGA-D9-A1JW-06 | 1119 | 32 |
| SKCM | TCGA-D9-A1JX-06 | 234 | 5 |
| SKCM | TCGA-D9-A1X3-06 | 84 | 8 |
| SKCM | TCGA-D9-A3Z1-06 | 631 | 19 |
| SKCM | TCGA-D9-A3Z3-06 | 174 | 6 |
| SKCM | TCGA-D9-A3Z4-01 | 198 | 10 |
| SKCM | TCGA-D9-A4Z2-01 | 31 | 3 |
| SKCM | TCGA-D9-A4Z3-01 | 956 | 30 |
| SKCM | TCGA-D9-A4Z5-01 | 99 | 6 |
| SKCM | TCGA-D9-A4Z6-01 | 342 | 11 |
| SKCM | TCGA-D9-A6E9-06 | 237 | 6 |
| SKCM | TCGA-D9-A6EA-06 | 826 | 34 |
| SKCM | TCGA-D9-A6EC-06 | 5489 | 169 |
| SKCM | TCGA-DA-A1HV-06 | 1702 | 67 |
| SKCM | TCGA-DA-A1HW-06 | 416 | 20 |
| SKCM | TCGA-DA-A1HY-06 | 908 | 36 |
| SKCM | TCGA-DA-A1IO-06 | 630 | 21 |
| SKCM | TCGA-DA-A1I1-06 | 480 | 17 |
| SKCM | TCGA-DA-A1I2-06 | 172 | 4 |
| SKCM | TCGA-DA-A1I4-06 | 315 | 10 |
| SKCM | TCGA-DA-A1I5-06 | 542 | 23 |
| SKCM | TCGA-DA-A1I7-06 | 579 | 16 |
| SKCM | TCGA-DA-A1I8-06 | 236 | 7 |
| SKCM | TCGA-DA-A1IA-06 | 416 | 8 |
| SKCM | TCGA-DA-A1IB-06 | 24 | 2 |
| SKCM | TCGA-DA-A1IC-06 | 1191 | 45 |
| SKCM | TCGA-DA-A3F2-06 | 15 | 0 |
| SKCM | TCGA-DA-A3F3-06 | 379 | 15 |
| SKCM | TCGA-DA-A3F5-06 | 430 | 13 |
| SKCM | TCGA-DA-A3F8-06 | 1472 | 61 |
| SKCM | TCGA-EB-A1NK-01 | 185 | 10 |
| SKCM | TCGA-EB-A24C-01 | 49 | 5 |
| SKCM | TCGA-EB-A24D-01 | 719 | 18 |
| SKCM | TCGA-EB-A299-01 | 201 | 6 |
| SKCM | TCGA-EB-A3HV-01 | 64 | 5 |
| SKCM | TCGA-EB-A3XB-01 | 620 | 16 |
| SKCM | TCGA-EB-A3XC-01 | 506 | 18 |
| SKCM | TCGA-EB-A3XD-01 | 486 | 10 |
| SKCM | TCGA-EB-A3XE-01 | 25 | 1 |
| SKCM | TCGA-EB-A3Y6-01 | 733 | 19 |
| SKCM | TCGA-EB-A3Y7-01 | 1374 | 35 |
| SKCM | TCGA-EB-A41A-01 | 2305 | 78 |
| SKCM | TCGA-EB-A41B-01 | 494 | 17 |
| SKCM | TCGA-EB-A42Y-01 | 89 | 6 |
| SKCM | TCGA-EB-A42Z-01 | 149 | 7 |
| SKCM | TCGA-EB-A430-01 | 491 | 21 |
| SKCM | TCGA-EB-A431-01 | 2113 | 64 |
| SKCM | TCGA-EB-A44N-01 | 204 | 14 |
| SKCM | TCGA-EB-A44O-01 | 613 | 20 |
| SKCM | TCGA-EB-A44P-01 | 419 | 18 |
| SKCM | TCGA-EB-A44Q-06 | 67 | 3 |
| SKCM | TCGA-EB-A44R-06 | 78 | 4 |
| SKCM | TCGA-EB-A4IQ-01 | 26 | 1 |
| SKCM | TCGA-EB-A4IS-01 | 693 | 27 |
| SKCM | TCGA-EB-A4OY-01 | 81 | 1 |
| SKCM | TCGA-EB-A4OZ-01 | 19 | 1 |
| SKCM | TCGA-EB-A4P0-01 | 245 | 7 |
| SKCM | TCGA-EB-A551-01 | 442 | 15 |
| SKCM | TCGA-EB-A553-01 | 400 | 20 |
| SKCM | TCGA-EB-A57M-01 | 76 | 4 |
| SKCM | TCGA-EB-A5SE-01 | 360 | 13 |
| SKCM | TCGA-EB-A5SF-01 | 54 | 5 |
| SKCM | TCGA-EB-A5SG-06 | 372 | 12 |
| SKCM | TCGA-EB-A5SH-06 | 121 | 5 |
| SKCM | TCGA-EB-A5UL-06 | 589 | 24 |
| SKCM | TCGA-EB-A5UM-01 | 690 | 20 |
| SKCM | TCGA-EB-A5UN-06 | 895 | 41 |
| SKCM | TCGA-EE-A17X-06 | 993 | 40 |
| SKCM | TCGA-EE-A17Y-06 | 214 | 8 |
| SKCM | TCGA-EE-A17Z-06 | 46 | 3 |
| SKCM | TCGA-EE-A180-06 | 651 | 22 |
| SKCM | TCGA-EE-A181-06 | 3703 | 151 |
| SKCM | TCGA-EE-A182-06 | 1859 | 75 |
| SKCM | TCGA-EE-A183-06 | 948 | 22 |
| SKCM | TCGA-EE-A184-06 | 400 | 15 |
| SKCM | TCGA-EE-A185-06 | 247 | 6 |
| SKCM | TCGA-EE-A20B-06 | 232 | 10 |
| SKCM | TCGA-EE-A20C-06 | 2505 | 67 |
| SKCM | TCGA-EE-A20F-06 | 621 | 30 |
| SKCM | TCGA-EE-A20H-06 | 755 | 22 |
| SKCM | TCGA-EE-A20O-06 | 39 | 1 |
| SKCM | TCGA-EE-A29A-06 | 318 | 13 |
| SKCM | TCGA-EE-A29B-06 | 682 | 27 |
| SKCM | TCGA-EE-A29C-06 | 507 | 25 |
| SKCM | TCGA-EE-A29D-06 | 4728 | 151 |
| SKCM | TCGA-EE-A29E-06 | 4417 | 151 |
| SKCM | TCGA-EE-A29G-06 | 500 | 22 |
| SKCM | TCGA-EE-A29H-06 | 401 | 14 |
| SKCM | TCGA-EE-A29L-06 | 2510 | 68 |
| SKCM | TCGA-EE-A29M-06 | 2886 | 84 |
| SKCM | TCGA-EE-A29N-06 | 767 | 21 |
| SKCM | TCGA-EE-A29P-06 | 387 | 7 |
| SKCM | TCGA-EE-A29Q-06 | 385 | 13 |
| SKCM | TCGA-EE-A29R-06 | 861 | 26 |
| SKCM | TCGA-EE-A29S-06 | 1319 | 45 |
| SKCM | TCGA-EE-A29V-06 | 1250 | 55 |
| SKCM | TCGA-EE-A29W-06 | 99 | 5 |
| SKCM | TCGA-EE-A29X-06 | 192 | 11 |
| SKCM | TCGA-EE-A2A0-06 | 478 | 14 |
| SKCM | TCGA-EE-A2A1-06 | 493 | 10 |
| SKCM | TCGA-EE-A2A2-06 | 1971 | 62 |
| SKCM | TCGA-EE-A2A5-06 | 396 | 8 |
| SKCM | TCGA-EE-A2A6-06 | 378 | 12 |
| SKCM | TCGA-EE-A2GB-06 | 819 | 25 |
| SKCM | TCGA-EE-A2GC-06 | 2827 | 108 |
| SKCM | TCGA-EE-A2GD-06 | 687 | 24 |
| SKCM | TCGA-EE-A2GE-06 | 293 | 10 |
| SKCM | TCGA-EE-A2GH-06 | 384 | 13 |
| SKCM | TCGA-EE-A2GI-06 | 1976 | 67 |
| SKCM | TCGA-EE-A2GJ-06 | 1436 | 52 |
| SKCM | TCGA-EE-A2GK-06 | 26 | 1 |
| SKCM | TCGA-EE-A2GL-06 | 450 | 10 |
| SKCM | TCGA-EE-A2GM-06 | 880 | 37 |
| SKCM | TCGA-EE-A2GN-06 | 512 | 20 |
| SKCM | TCGA-EE-A2GO-06 | 2483 | 75 |
| SKCM | TCGA-EE-A2GP-06 | 789 | 21 |
| SKCM | TCGA-EE-A2GR-06 | 1971 | 73 |
| SKCM | TCGA-EE-A2GS-06 | 427 | 14 |
| SKCM | TCGA-EE-A2GT-06 | 434 | 15 |
| SKCM | TCGA-EE-A2GU-06 | 748 | 22 |
| SKCM | TCGA-EE-A2M5-06 | 2437 | 83 |
| SKCM | TCGA-EE-A2M6-06 | 669 | 15 |
| SKCM | TCGA-EE-A2M7-06 | 91 | 6 |
| SKCM | TCGA-EE-A2M8-06 | 155 | 4 |
| SKCM | TCGA-EE-A2MC-06 | 958 | 32 |
| SKCM | TCGA-EE-A2MD-06 | 2099 | 69 |
| SKCM | TCGA-EE-A2ME-06 | 60 | 1 |
| SKCM | TCGA-EE-A2MF-06 | 957 | 34 |
| SKCM | TCGA-EE-A2MG-06 | 273 | 8 |
| SKCM | TCGA-EE-A2MH-06 | 194 | 8 |
| SKCM | TCGA-EE-A2MI-06 | 1326 | 40 |
| SKCM | TCGA-EE-A2MJ-06 | 2692 | 90 |
| SKCM | TCGA-EE-A2MK-06 | 379 | 8 |
| SKCM | TCGA-EE-A2ML-06 | 698 | 17 |
| SKCM | TCGA-EE-A2MM-06 | 705 | 22 |
| SKCM | TCGA-EE-A2MN-06 | 198 | 9 |
| SKCM | TCGA-EE-A2MP-06 | 340 | 14 |
| SKCM | TCGA-EE-A2MQ-06 | 338 | 18 |
| SKCM | TCGA-EE-A2MR-06 | 6306 | 195 |
| SKCM | TCGA-EE-A2MS-06 | 3541 | 94 |
| SKCM | TCGA-EE-A2MT-06 | 1104 | 35 |
| SKCM | TCGA-EE-A2MU-06 | 838 | 25 |
| SKCM | TCGA-EE-A3AA-06 | 2215 | 65 |
| SKCM | TCGA-EE-A3AB-06 | 816 | 37 |
| SKCM | TCGA-EE-A3AC-06 | 1236 | 35 |
| SKCM | TCGA-EE-A3AD-06 | 569 | 19 |
| SKCM | TCGA-EE-A3AE-06 | 1804 | 53 |
| SKCM | TCGA-EE-A3AF-06 | 1163 | 42 |
| SKCM | TCGA-EE-A3AG-06 | 2103 | 67 |
| SKCM | TCGA-EE-A3AH-06 | 482 | 21 |
| SKCM | TCGA-EE-A3J3-06 | 228 | 8 |
| SKCM | TCGA-EE-A3J4-06 | 407 | 17 |
| SKCM | TCGA-EE-A3J5-06 | 2612 | 74 |
| SKCM | TCGA-EE-A3J7-06 | 1256 | 39 |
| SKCM | TCGA-EE-A3J8-06 | 344 | 12 |
| SKCM | TCGA-EE-A3JA-06 | 1269 | 37 |
| SKCM | TCGA-EE-A3JB-06 | 768 | 29 |
| SKCM | TCGA-EE-A3JD-06 | 3639 | 120 |
| SKCM | TCGA-EE-A3JE-06 | 342 | 11 |
| SKCM | TCGA-EE-A3JH-06 | 239 | 3 |
| SKCM | TCGA-EE-A3JI-06 | 1437 | 42 |
| SKCM | TCGA-ER-A193-06 | 2370 | 55 |
| SKCM | TCGA-ER-A194-01 | 1665 | 59 |
| SKCM | TCGA-ER-A195-06 | 321 | 7 |
| SKCM | TCGA-ER-A196-01 | 37 | 0 |
| SKCM | TCGA-ER-A197-06 | 58 | 1 |
| SKCM | TCGA-ER-A198-06 | 573 | 27 |
| SKCM | TCGA-ER-A199-06 | 356 | 17 |
| SKCM | TCGA-ER-A19A-06 | 447 | 19 |
| SKCM | TCGA-ER-A19B-06 | 146 | 5 |
| SKCM | TCGA-ER-A19C-06 | 138 | 9 |
| SKCM | TCGA-ER-A19D-06 | 583 | 18 |
| SKCM | TCGA-ER-A19E-06 | 558 | 24 |
| SKCM | TCGA-ER-A19F-06 | 1224 | 45 |
| SKCM | TCGA-ER-A19G-06 | 648 | 30 |
| SKCM | TCGA-ER-A19H-06 | 328 | 21 |
| SKCM | TCGA-ER-A19J-06 | 282 | 7 |
| SKCM | TCGA-ER-A19K-01 | 451 | 15 |
| SKCM | TCGA-ER-A19L-06 | 83 | 4 |
| SKCM | TCGA-ER-A19M-06 | 610 | 17 |

## TABLE 5 (APPENDIX A)

| | | | | | | | | | | | |
|---|---|---|---|---|---|---|---|---|---|---|---|
| SKCM | TCGA-ER-A19N-06 | 593 | 12 | SKCM | TCGA-HR-A2OG-01 | 244 | 10 | STAD | TCGA-BR-8369-01 | 231 | 12 |
| SKCM | TCGA-ER-A19O-06 | 220 | 8 | SKCM | TCGA-HR-A2OH-01 | 147 | 7 | STAD | TCGA-BR-8370-01 | 121 | 10 |
| SKCM | TCGA-ER-A19P-06 | 1009 | 33 | SKCM | TCGA-IH-A3EA-01 | 1846 | 56 | STAD | TCGA-BR-8371-01 | 23 | 2 |
| SKCM | TCGA-ER-A19Q-06 | 153 | 6 | SKCM | TCGA-QB-A6FS-06 | 1445 | 50 | STAD | TCGA-BR-8372-01 | 1564 | 60 |
| SKCM | TCGA-ER-A19S-06 | 274 | 19 | SKCM | TCGA-RP-A690-06 | 23 | 2 | STAD | TCGA-BR-8373-01 | 111 | 2 |
| SKCM | TCGA-ER-A19T-01 | 91 | 3 | SKCM | TCGA-RP-A693-06 | 943 | 29 | STAD | TCGA-BR-8380-01 | 81 | 7 |
| SKCM | TCGA-ER-A19T-06 | 70 | 3 | SKCM | TCGA-RP-A694-06 | 858 | 30 | STAD | TCGA-BR-8381-01 | 138 | 10 |
| SKCM | TCGA-ER-A19W-06 | 442 | 16 | SKCM | TCGA-RP-A695-06 | 1063 | 41 | STAD | TCGA-BR-8382-01 | 1030 | 39 |
| SKCM | TCGA-ER-A1A1-06 | 299 | 16 | STAD | TCGA-B7-5816-01 | 1538 | 67 | STAD | TCGA-BR-8384-01 | 46 | 2 |
| SKCM | TCGA-ER-A2NB-01 | 102 | 3 | STAD | TCGA-B7-5818-01 | 465 | 7 | STAD | TCGA-BR-8483-01 | 141 | 4 |
| SKCM | TCGA-ER-A2NC-06 | 477 | 12 | STAD | TCGA-BR-4183-01 | 71 | 2 | STAD | TCGA-BR-8484-01 | 95 | 3 |
| SKCM | TCGA-ER-A2ND-06 | 145 | 4 | STAD | TCGA-BR-4184-01 | 4630 | 194 | STAD | TCGA-BR-8485-01 | 346 | 15 |
| SKCM | TCGA-ER-A2NE-06 | 182 | 5 | STAD | TCGA-BR-4187-01 | 38 | 3 | STAD | TCGA-BR-8486-01 | 63 | 4 |
| SKCM | TCGA-ER-A2NF-01 | 46 | 4 | STAD | TCGA-BR-4188-01 | 85 | 4 | STAD | TCGA-BR-8487-01 | 3708 | 135 |
| SKCM | TCGA-ER-A2NF-06 | 41 | 4 | STAD | TCGA-BR-4191-01 | 226 | 6 | STAD | TCGA-BR-8588-01 | 139 | 13 |
| SKCM | TCGA-ER-A2NG-06 | 318 | 11 | STAD | TCGA-BR-4201-01 | 1664 | 61 | STAD | TCGA-BR-8589-01 | 1231 | 41 |
| SKCM | TCGA-ER-A2NH-06 | 397 | 13 | STAD | TCGA-BR-4253-01 | 99 | 6 | STAD | TCGA-BR-8590-01 | 138 | 6 |
| SKCM | TCGA-ER-A3E5-06 | 50 | 3 | STAD | TCGA-BR-4255-01 | 70 | 4 | STAD | TCGA-BR-8591-01 | 1787 | 61 |
| SKCM | TCGA-ER-A3ET-06 | 132 | 14 | STAD | TCGA-BR-4256-01 | 1177 | 57 | STAD | TCGA-BR-8592-01 | 42 | 3 |
| SKCM | TCGA-ER-A3EV-06 | 124 | 2 | STAD | TCGA-BR-4257-01 | 1281 | 49 | STAD | TCGA-BR-8676-01 | 94 | 10 |
| SKCM | TCGA-ER-A3PL-06 | 472 | 14 | STAD | TCGA-BR-4259-01 | 104 | 6 | STAD | TCGA-BR-8677-01 | 80 | 2 |
| SKCM | TCGA-ER-A42H-01 | 80 | 3 | STAD | TCGA-BR-4279-01 | 57 | 4 | STAD | TCGA-BR-8678-01 | 126 | 8 |
| SKCM | TCGA-ER-A42K-06 | 242 | 12 | STAD | TCGA-BR-4280-01 | 886 | 37 | STAD | TCGA-BR-8679-01 | 175 | 8 |
| SKCM | TCGA-ER-A42L-06 | 1263 | 32 | STAD | TCGA-BR-4282-01 | 1678 | 58 | STAD | TCGA-BR-8680-01 | 6710 | 250 |
| SKCM | TCGA-FR-A2OS-01 | 44 | 0 | STAD | TCGA-BR-4294-01 | 34 | 1 | STAD | TCGA-BR-8682-01 | 82 | 4 |
| SKCM | TCGA-FR-A3R1-01 | 623 | 27 | STAD | TCGA-BR-4357-01 | 283 | 12 | STAD | TCGA-BR-8683-01 | 167 | 5 |
| SKCM | TCGA-FR-A3YN-06 | 743 | 29 | STAD | TCGA-BR-4361-01 | 3392 | 137 | STAD | TCGA-BR-8686-01 | 125 | 5 |
| SKCM | TCGA-FR-A3YO-06 | 1077 | 33 | STAD | TCGA-BR-4362-01 | 2090 | 92 | STAD | TCGA-BR-8687-01 | 201 | 11 |
| SKCM | TCGA-FR-A44A-06 | 382 | 20 | STAD | TCGA-BR-4363-01 | 702 | 33 | STAD | TCGA-BR-8690-01 | 179 | 11 |
| SKCM | TCGA-FR-A69P-06 | 279 | 12 | STAD | TCGA-BR-4366-01 | 165 | 11 | STAD | TCGA-BR-A44T-01 | 29 | 2 |
| SKCM | TCGA-FS-A1YW-06 | 210 | 7 | STAD | TCGA-BR-4368-01 | 1657 | 65 | STAD | TCGA-BR-A44U-01 | 102 | 4 |
| SKCM | TCGA-FS-A1YX-06 | 33 | 3 | STAD | TCGA-BR-4369-01 | 200 | 9 | STAD | TCGA-BR-A452-01 | 128 | 6 |
| SKCM | TCGA-FS-A1YY-06 | 268 | 14 | STAD | TCGA-BR-4370-01 | 1346 | 43 | STAD | TCGA-BR-A453-01 | 76 | 4 |
| SKCM | TCGA-FS-A1Z0-06 | 323 | 7 | STAD | TCGA-BR-4371-01 | 182 | 11 | STAD | TCGA-BR-A4CQ-01 | 193 | 10 |
| SKCM | TCGA-FS-A1Z3-06 | 1277 | 42 | STAD | TCGA-BR-6452-01 | 8796 | 371 | STAD | TCGA-BR-A4CR-01 | 77 | 4 |
| SKCM | TCGA-FS-A1Z4-06 | 60 | 3 | STAD | TCGA-BR-6453-01 | 221 | 11 | STAD | TCGA-BR-A4CS-01 | 182 | 4 |
| SKCM | TCGA-FS-A1Z7-06 | 234 | 11 | STAD | TCGA-BR-6454-01 | 178 | 20 | STAD | TCGA-BR-A4IU-01 | 26 | 4 |
| SKCM | TCGA-FS-A1ZA-06 | 1472 | 40 | STAD | TCGA-BR-6455-01 | 114 | 6 | STAD | TCGA-BR-A4IV-01 | 28 | 4 |
| SKCM | TCGA-FS-A1ZB-06 | 298 | 11 | STAD | TCGA-BR-6456-01 | 51 | 2 | STAD | TCGA-BR-A4IY-01 | 158 | 3 |
| SKCM | TCGA-FS-A1ZC-06 | 894 | 34 | STAD | TCGA-BR-6457-01 | 143 | 5 | STAD | TCGA-BR-A4IZ-01 | 10 | 0 |
| SKCM | TCGA-FS-A1ZD-06 | 316 | 11 | STAD | TCGA-BR-6458-01 | 344 | 6 | STAD | TCGA-BR-A4J1-01 | 79 | 1 |
| SKCM | TCGA-FS-A1ZE-06 | 199 | 5 | STAD | TCGA-BR-6563-01 | 86 | 5 | STAD | TCGA-BR-A4J2-01 | 49 | 3 |
| SKCM | TCGA-FS-A1ZF-06 | 254 | 10 | STAD | TCGA-BR-6564-01 | 42 | 2 | STAD | TCGA-BR-A4J4-01 | 90 | 5 |
| SKCM | TCGA-FS-A1ZG-06 | 46 | 2 | STAD | TCGA-BR-6565-01 | 169 | 9 | STAD | TCGA-BR-A4J6-01 | 40 | 6 |
| SKCM | TCGA-FS-A1ZH-06 | 51 | 2 | STAD | TCGA-BR-6566-01 | 1184 | 48 | STAD | TCGA-BR-A4J7-01 | 57 | 4 |
| SKCM | TCGA-FS-A1Zi-06 | 112 | 7 | STAD | TCGA-BR-6705-01 | 120 | 7 | STAD | TCGA-BR-A4J8-01 | 111 | 9 |
| SKCM | TCGA-FS-A1ZK-06 | 1941 | 66 | STAD | TCGA-BR-6706-01 | 256 | 12 | STAD | TCGA-BR-A4PD-01 | 173 | 9 |
| SKCM | TCGA-FS-A1ZM-06 | 312 | 9 | STAD | TCGA-BR-6707-01 | 80 | 11 | STAD | TCGA-BR-A4PE-01 | 239 | 10 |
| SKCM | TCGA-FS-A1ZN-01 | 147 | 11 | STAD | TCGA-BR-6710-01 | 1 | 0 | STAD | TCGA-BR-A4PF-01 | 110 | 6 |
| SKCM | TCGA-FS-A1ZP-06 | 544 | 24 | STAD | TCGA-BR-6801-01 | 58 | 4 | STAD | TCGA-BR-A4QI-01 | 151 | 10 |
| SKCM | TCGA-FS-A1ZQ-06 | 628 | 23 | STAD | TCGA-BR-6802-01 | 585 | 27 | STAD | TCGA-BR-A4QL-01 | 2225 | 94 |
| SKCM | TCGA-FS-A1ZR-06 | 185 | 8 | STAD | TCGA-BR-6803-01 | 20 | 2 | STAD | TCGA-BR-A4QM-01 | 116 | 5 |
| SKCM | TCGA-FS-A1ZS-06 | 355 | 13 | STAD | TCGA-BR-6852-01 | 1387 | 51 | STAD | TCGA-CD-5798-01 | 100 | 5 |
| SKCM | TCGA-FS-A1ZT-06 | 233 | 12 | STAD | TCGA-BR-7196-01 | 84 | 9 | STAD | TCGA-CD-5799-01 | 56 | 5 |
| SKCM | TCGA-FS-A1ZU-06 | 62 | 1 | STAD | TCGA-BR-7197-01 | 201 | 8 | STAD | TCGA-CD-5800-01 | 177 | 11 |
| SKCM | TCGA-FS-A1ZW-06 | 812 | 30 | STAD | TCGA-BR-7703-01 | 866 | 32 | STAD | TCGA-CD-5801-01 | 594 | 28 |
| SKCM | TCGA-FS-A1ZY-06 | 47 | 3 | STAD | TCGA-BR-7707-01 | 1739 | 54 | STAD | TCGA-CD-5802-01 | 62 | 1 |
| SKCM | TCGA-FS-A1ZZ-06 | 1940 | 52 | STAD | TCGA-BR-7715-01 | 165 | 4 | STAD | TCGA-CD-5803-01 | 45 | 4 |
| SKCM | TCGA-FS-A4F0-06 | 658 | 17 | STAD | TCGA-BR-7716-01 | 177 | 9 | STAD | TCGA-CD-5804-01 | 105 | 4 |
| SKCM | TCGA-FS-A4F4-06 | 64 | 2 | STAD | TCGA-BR-7717-01 | 167 | 5 | STAD | TCGA-CD-5813-01 | 200 | 11 |
| SKCM | TCGA-FS-A4F5-06 | 944 | 39 | STAD | TCGA-BR-7722-01 | 61 | 4 | STAD | TCGA-CD-8524-01 | 170 | 5 |
| SKCM | TCGA-FS-A4F8-06 | 227 | 9 | STAD | TCGA-BR-7723-01 | 179 | 3 | STAD | TCGA-CD-8525-01 | 93 | 2 |
| SKCM | TCGA-FS-A4F9-06 | 571 | 20 | STAD | TCGA-BR-7851-01 | 1812 | 89 | STAD | TCGA-CD-8526-01 | 49 | 4 |
| SKCM | TCGA-FS-A4FB-06 | 237 | 7 | STAD | TCGA-BR-7901-01 | 235 | 9 | STAD | TCGA-CD-8527-01 | 305 | 11 |
| SKCM | TCGA-FS-A4FC-06 | 888 | 39 | STAD | TCGA-BR-7957-01 | 57 | 2 | STAD | TCGA-CD-8528-01 | 116 | 3 |
| SKCM | TCGA-FS-A4FD-06 | 560 | 15 | STAD | TCGA-BR-7958-01 | 184 | 10 | STAD | TCGA-CD-8529-01 | 458 | 17 |
| SKCM | TCGA-FW-A3I3-06 | 94 | 4 | STAD | TCGA-BR-7959-01 | 111 | 4 | STAD | TCGA-CD-8530-01 | 101 | 6 |
| SKCM | TCGA-FW-A3R5-06 | 49557 | 1400 | STAD | TCGA-BR-8058-01 | 57 | 4 | STAD | TCGA-CD-8531-01 | 267 | 25 |
| SKCM | TCGA-FW-A3TU-06 | 933 | 27 | STAD | TCGA-BR-8059-01 | 815 | 32 | STAD | TCGA-CD-8532-01 | 57 | 3 |
| SKCM | TCGA-FW-A3TV-06 | 426 | 14 | STAD | TCGA-BR-8077-01 | 135 | 2 | STAD | TCGA-CD-8534-01 | 84 | 6 |
| SKCM | TCGA-FW-A5DX-01 | 235 | 14 | STAD | TCGA-BR-8078-01 | 1953 | 72 | STAD | TCGA-CD-8535-01 | 302 | 17 |
| SKCM | TCGA-FW-A5DY-06 | 286 | 13 | STAD | TCGA-BR-8080-01 | 168 | 7 | STAD | TCGA-CD-8536-01 | 1080 | 53 |
| SKCM | TCGA-GF-A2C7-01 | 96 | 5 | STAD | TCGA-BR-8081-01 | 1114 | 57 | STAD | TCGA-CD-A486-01 | 106 | 7 |
| SKCM | TCGA-GF-A3OT-06 | 676 | 32 | STAD | TCGA-BR-8284-01 | 379 | 20 | STAD | TCGA-CD-A487-01 | 203 | 5 |
| SKCM | TCGA-GF-A4EO-06 | 319 | 18 | STAD | TCGA-BR-8286-01 | 164 | 6 | STAD | TCGA-CD-A489-01 | 87 | 5 |
| SKCM | TCGA-GF-A6C8-06 | 466 | 27 | STAD | TCGA-BR-8289-01 | 229 | 8 | STAD | TCGA-CD-A48A-01 | 264 | 12 |
| SKCM | TCGA-GF-A6C9-06 | 4232 | 147 | STAD | TCGA-BR-8291-01 | 31 | 0 | STAD | TCGA-CD-A48C-01 | 243 | 16 |
| SKCM | TCGA-GN-A262-06 | 483 | 23 | STAD | TCGA-BR-8295-01 | 60 | 2 | STAD | TCGA-CD-A4MG-01 | 3024 | 134 |
| SKCM | TCGA-GN-A263-01 | 548 | 19 | STAD | TCGA-BR-8296-01 | 88 | 6 | STAD | TCGA-CD-A4MH-01 | 98 | 3 |
| SKCM | TCGA-GN-A264-06 | 74 | 1 | STAD | TCGA-BR-8297-01 | 302 | 17 | STAD | TCGA-CD-A4MI-01 | 1170 | 40 |
| SKCM | TCGA-GN-A265-06 | 370 | 18 | STAD | TCGA-BR-8360-01 | 1103 | 41 | STAD | TCGA-CD-A4MJ-01 | 807 | 26 |
| SKCM | TCGA-GN-A266-06 | 4770 | 139 | STAD | TCGA-BR-8361-01 | 1961 | 86 | STAD | TCGA-CG-4300-01 | 159 | 4 |
| SKCM | TCGA-GN-A267-06 | 505 | 17 | STAD | TCGA-BR-8363-01 | 1433 | 64 | STAD | TCGA-CG-4301-01 | 58 | 3 |
| SKCM | TCGA-GN-A268-06 | 432 | 12 | STAD | TCGA-BR-8364-01 | 24 | 2 | STAD | TCGA-CG-4304-01 | 84 | 3 |
| SKCM | TCGA-GN-A269-01 | 925 | 25 | STAD | TCGA-BR-8365-01 | 26 | 2 | STAD | TCGA-CG-4305-01 | 1402 | 65 |
| SKCM | TCGA-GN-A26A-06 | 410 | 11 | STAD | TCGA-BR-8366-01 | 190 | 11 | STAD | TCGA-CG-4306-01 | 1333 | 57 |
| SKCM | TCGA-GN-A26C-01 | 2315 | 71 | STAD | TCGA-BR-8367-01 | 75 | 2 | STAD | TCGA-CG-4436-01 | 207 | 11 |
| SKCM | TCGA-GN-A26D-06 | 70 | 5 | STAD | TCGA-BR-8368-01 | 1237 | 32 | STAD | TCGA-CG-4437-01 | 607 | 17 |

# TABLE 5 (APPENDIX A)

| | | | | | | | | | | | |
|---|---|---|---|---|---|---|---|---|---|---|---|
| STAD | TCGA-CG-4438-01 | 156 | 10 | STAD | TCGA-HU-8602-01 | 1908 | 75 | STES | TCGA-BR-6803-01 | 20 | 2 |
| STAD | TCGA-CG-4440-01 | 138 | 7 | STAD | TCGA-HU-8604-01 | 235 | 12 | STES | TCGA-BR-6852-01 | 1387 | 51 |
| STAD | TCGA-CG-4441-01 | 137 | 8 | STAD | TCGA-HU-8608-01 | 111 | 9 | STES | TCGA-BR-7196-01 | 84 | 9 |
| STAD | TCGA-CG-4442-01 | 1888 | 78 | STAD | TCGA-HU-8610-01 | 74 | 2 | STES | TCGA-BR-7197-01 | 201 | 8 |
| STAD | TCGA-CG-4443-01 | 116 | 7 | STAD | TCGA-HU-A4G2-01 | 57 | 3 | STES | TCGA-BR-7703-01 | 866 | 32 |
| STAD | TCGA-CG-4444-01 | 203 | 10 | STAD | TCGA-HU-A4G3-01 | 118 | 5 | STES | TCGA-BR-7707-01 | 1739 | 54 |
| STAD | TCGA-CG-4449-01 | 115 | 4 | STAD | TCGA-HU-A4G6-01 | 98 | 6 | STES | TCGA-BR-7715-01 | 165 | 4 |
| STAD | TCGA-CG-4455-01 | 57 | 5 | STAD | TCGA-HU-A4G8-01 | 1783 | 65 | STES | TCGA-BR-7716-01 | 177 | 9 |
| STAD | TCGA-CG-4462-01 | 85 | 6 | STAD | TCGA-HU-A4G9-01 | 951 | 31 | STES | TCGA-BR-7717-01 | 167 | 5 |
| STAD | TCGA-CG-4465-01 | 766 | 33 | STAD | TCGA-HU-A4GC-01 | 168 | 10 | STES | TCGA-BR-7722-01 | 61 | 4 |
| STAD | TCGA-CG-4466-01 | 224 | 8 | STAD | TCGA-HU-A4GD-01 | 96 | 1 | STES | TCGA-BR-7723-01 | 179 | 3 |
| STAD | TCGA-CG-4469-01 | 346 | 19 | STAD | TCGA-HU-A4GF-01 | 152 | 8 | STES | TCGA-BR-7851-01 | 1812 | 89 |
| STAD | TCGA-CG-4474-01 | 115 | 14 | STAD | TCGA-HU-A4GH-01 | 854 | 20 | STES | TCGA-BR-7901-01 | 235 | 9 |
| STAD | TCGA-CG-4475-01 | 61 | 1 | STAD | TCGA-HU-A4GN-01 | 1405 | 56 | STES | TCGA-BR-7957-01 | 57 | 2 |
| STAD | TCGA-CG-4476-01 | 140 | 9 | STAD | TCGA-HU-A4GP-01 | 214 | 8 | STES | TCGA-BR-7958-01 | 184 | 10 |
| STAD | TCGA-CG-4477-01 | 153 | 8 | STAD | TCGA-HU-A4GQ-01 | 2206 | 81 | STES | TCGA-BR-7959-01 | 111 | 4 |
| STAD | TCGA-CG-5717-01 | 201 | 8 | STAD | TCGA-HU-A4GT-01 | 2026 | 79 | STES | TCGA-BR-8058-01 | 57 | 4 |
| STAD | TCGA-CG-5718-01 | 100 | 4 | STAD | TCGA-HU-A4GU-01 | 1764 | 77 | STES | TCGA-BR-8059-01 | 815 | 32 |
| STAD | TCGA-CG-5719-01 | 109 | 7 | STAD | TCGA-HU-A4GX-01 | 1490 | 55 | STES | TCGA-BR-8077-01 | 135 | 2 |
| STAD | TCGA-CG-5720-01 | 110 | 9 | STAD | TCGA-HU-A4GY-01 | 75 | 2 | STES | TCGA-BR-8078-01 | 1953 | 72 |
| STAD | TCGA-CG-5721-01 | 5091 | 210 | STAD | TCGA-HU-A4H0-01 | 196 | 11 | STES | TCGA-BR-8080-01 | 168 | 7 |
| STAD | TCGA-CG-5722-01 | 105 | 4 | STAD | TCGA-HU-A4H2-01 | 189 | 7 | STES | TCGA-BR-8081-01 | 1114 | 57 |
| STAD | TCGA-CG-5723-01 | 2107 | 98 | STAD | TCGA-HU-A4H3-01 | 1094 | 51 | STES | TCGA-BR-8284-01 | 379 | 20 |
| STAD | TCGA-CG-5724-01 | 132 | 6 | STAD | TCGA-HU-A4H4-01 | 802 | 41 | STES | TCGA-BR-8286-01 | 164 | 6 |
| STAD | TCGA-CG-5726-01 | 1705 | 56 | STAD | TCGA-HU-A4H5-01 | 301 | 14 | STES | TCGA-BR-8288-01 | 229 | 8 |
| STAD | TCGA-CG-5727-01 | 182 | 12 | STAD | TCGA-HU-A4H6-01 | 102 | 1 | STES | TCGA-BR-8291-01 | 31 | 0 |
| STAD | TCGA-CG-5728-01 | 1727 | 67 | STAD | TCGA-HU-A4H8-01 | 1453 | 62 | STES | TCGA-BR-8295-01 | 60 | 2 |
| STAD | TCGA-CG-5730-01 | 195 | 8 | STAD | TCGA-HU-A4HD-01 | 200 | 8 | STES | TCGA-BR-8296-01 | 88 | 6 |
| STAD | TCGA-CG-5732-01 | 81 | 4 | STAD | TCGA-IN-7806-01 | 87 | 7 | STES | TCGA-BR-8297-01 | 302 | 17 |
| STAD | TCGA-CG-5733-01 | 1422 | 56 | STAD | TCGA-IN-7808-01 | 122 | 7 | STES | TCGA-BR-8360-01 | 1103 | 41 |
| STAD | TCGA-CG-5734-01 | 121 | 3 | STAD | TCGA-IN-8462-01 | 136 | 6 | STES | TCGA-BR-8361-01 | 1961 | 86 |
| STAD | TCGA-D7-5577-01 | 134 | 8 | STAD | TCGA-IN-8663-01 | 188 | 10 | STES | TCGA-BR-8363-01 | 1433 | 64 |
| STAD | TCGA-D7-5578-01 | 290 | 18 | STAD | TCGA-IP-7968-01 | 130 | 4 | STES | TCGA-BR-8364-01 | 24 | 2 |
| STAD | TCGA-D7-5579-01 | 173 | 4 | STES | TCGA-2H-A9GF-01 | 508 | 18 | STES | TCGA-BR-8365-01 | 26 | 2 |
| STAD | TCGA-D7-6518-01 | 113 | 6 | STES | TCGA-2H-A9GG-01 | 295 | 9 | STES | TCGA-BR-8366-01 | 190 | 11 |
| STAD | TCGA-D7-6519-01 | 65 | 5 | STES | TCGA-2H-A9GH-01 | 320 | 8 | STES | TCGA-BR-8367-01 | 75 | 2 |
| STAD | TCGA-D7-6520-01 | 76 | 2 | STES | TCGA-2H-A9GI-01 | 433 | 19 | STES | TCGA-BR-8368-01 | 1237 | 32 |
| STAD | TCGA-D7-6521-01 | 93 | 9 | STES | TCGA-2H-A9GJ-01 | 265 | 9 | STES | TCGA-BR-8369-01 | 231 | 12 |
| STAD | TCGA-D7-6522-01 | 84 | 6 | STES | TCGA-2H-A9GK-01 | 471 | 25 | STES | TCGA-BR-8370-01 | 121 | 10 |
| STAD | TCGA-D7-6524-01 | 120 | 11 | STES | TCGA-2H-A9GL-01 | 383 | 18 | STES | TCGA-BR-8371-01 | 23 | 2 |
| STAD | TCGA-D7-6525-01 | 186 | 8 | STES | TCGA-2H-A9GM-01 | 271 | 14 | STES | TCGA-BR-8372-01 | 1564 | 60 |
| STAD | TCGA-D7-6526-01 | 247 | 7 | STES | TCGA-2H-A9GN-01 | 267 | 11 | STES | TCGA-BR-8373-01 | 111 | 2 |
| STAD | TCGA-D7-6527-01 | 251 | 16 | STES | TCGA-2H-A9GO-01 | 309 | 13 | STES | TCGA-BR-8380-01 | 81 | 7 |
| STAD | TCGA-D7-6528-01 | 394 | 23 | STES | TCGA-2H-A9GQ-01 | 296 | 12 | STES | TCGA-BR-8381-01 | 138 | 10 |
| STAD | TCGA-D7-6815-01 | 171 | 10 | STES | TCGA-2H-A9GR-01 | 543 | 23 | STES | TCGA-BR-8382-01 | 1030 | 39 |
| STAD | TCGA-D7-6817-01 | 102 | 5 | STES | TCGA-B7-5816-01 | 1538 | 67 | STES | TCGA-BR-8384-01 | 46 | 2 |
| STAD | TCGA-D7-6818-01 | 175 | 5 | STES | TCGA-B7-5818-01 | 465 | 7 | STES | TCGA-BR-8483-01 | 141 | 4 |
| STAD | TCGA-D7-6820-01 | 111 | 7 | STES | TCGA-BR-4183-01 | 71 | 2 | STES | TCGA-BR-8484-01 | 95 | 3 |
| STAD | TCGA-D7-6822-01 | 279 | 15 | STES | TCGA-BR-4184-01 | 4630 | 194 | STES | TCGA-BR-8485-01 | 346 | 15 |
| STAD | TCGA-D7-8570-01 | 146 | 4 | STES | TCGA-BR-4187-01 | 38 | 3 | STES | TCGA-BR-8486-01 | 63 | 4 |
| STAD | TCGA-D7-8572-01 | 196 | 11 | STES | TCGA-BR-4188-01 | 85 | 4 | STES | TCGA-BR-8487-01 | 3708 | 135 |
| STAD | TCGA-D7-8573-01 | 97 | 6 | STES | TCGA-BR-4191-01 | 226 | 6 | STES | TCGA-BR-8588-01 | 139 | 13 |
| STAD | TCGA-D7-8574-01 | 35 | 3 | STES | TCGA-BR-4201-01 | 1664 | 61 | STES | TCGA-BR-8589-01 | 1231 | 41 |
| STAD | TCGA-D7-8575-01 | 59 | 6 | STES | TCGA-BR-4253-01 | 99 | 6 | STES | TCGA-BR-8590-01 | 138 | 6 |
| STAD | TCGA-D7-8576-01 | 69 | 1 | STES | TCGA-BR-4255-01 | 70 | 4 | STES | TCGA-BR-8591-01 | 1787 | 61 |
| STAD | TCGA-D7-8578-01 | 59 | 2 | STES | TCGA-BR-4256-01 | 1177 | 57 | STES | TCGA-BR-8592-01 | 42 | 3 |
| STAD | TCGA-D7-8579-01 | 120 | 2 | STES | TCGA-BR-4257-01 | 1281 | 49 | STES | TCGA-BR-8676-01 | 94 | 10 |
| STAD | TCGA-D7-A4YT-01 | 334 | 17 | STES | TCGA-BR-4267-01 | 104 | 6 | STES | TCGA-BR-8677-01 | 80 | 2 |
| STAD | TCGA-D7-A4YU-01 | 241 | 15 | STES | TCGA-BR-4279-01 | 57 | 4 | STES | TCGA-BR-8678-01 | 126 | 8 |
| STAD | TCGA-D7-A4YV-01 | 1076 | 42 | STES | TCGA-BR-4280-01 | 886 | 37 | STES | TCGA-BR-8679-01 | 175 | 8 |
| STAD | TCGA-D7-A4YX-01 | 91 | 9 | STES | TCGA-BR-4292-01 | 1678 | 58 | STES | TCGA-BR-8680-01 | 6710 | 250 |
| STAD | TCGA-D7-A4YY-01 | 579 | 20 | STES | TCGA-BR-4294-01 | 34 | 1 | STES | TCGA-BR-8682-01 | 82 | 4 |
| STAD | TCGA-D7-A4Z0-01 | 334 | 18 | STES | TCGA-BR-4357-01 | 283 | 12 | STES | TCGA-BR-8683-01 | 167 | 5 |
| STAD | TCGA-EQ-5647-01 | 102 | 5 | STES | TCGA-BR-4361-01 | 3392 | 137 | STES | TCGA-BR-8686-01 | 125 | 5 |
| STAD | TCGA-EQ-8122-01 | 211 | 7 | STES | TCGA-BR-4362-01 | 2090 | 92 | STES | TCGA-BR-8687-01 | 201 | 11 |
| STAD | TCGA-EQ-A4SO-01 | 199 | 13 | STES | TCGA-BR-4363-01 | 702 | 33 | STES | TCGA-BR-8690-01 | 179 | 11 |
| STAD | TCGA-F1-6177-01 | 1597 | 65 | STES | TCGA-BR-4366-01 | 165 | 11 | STES | TCGA-BR-A44T-01 | 29 | 2 |
| STAD | TCGA-F1-6874-01 | 1125 | 47 | STES | TCGA-BR-4368-01 | 1657 | 65 | STES | TCGA-BR-A44U-01 | 102 | 4 |
| STAD | TCGA-F1-6875-01 | 98 | 5 | STES | TCGA-BR-4369-01 | 200 | 9 | STES | TCGA-BR-A452-01 | 128 | 6 |
| STAD | TCGA-F1-A448-01 | 687 | 27 | STES | TCGA-BR-4370-01 | 1346 | 43 | STES | TCGA-BR-A453-01 | 76 | 4 |
| STAD | TCGA-FP-7735-01 | 72 | 6 | STES | TCGA-BR-4371-01 | 182 | 11 | STES | TCGA-BR-A4CQ-01 | 193 | 10 |
| STAD | TCGA-FP-7829-01 | 284 | 9 | STES | TCGA-BR-6452-01 | 8796 | 371 | STES | TCGA-BR-A4CR-01 | 77 | 4 |
| STAD | TCGA-FP-7916-01 | 109 | 6 | STES | TCGA-BR-6453-01 | 221 | 11 | STES | TCGA-BR-A4CS-01 | 182 | 4 |
| STAD | TCGA-FP-7998-01 | 80 | 6 | STES | TCGA-BR-6454-01 | 178 | 20 | STES | TCGA-BR-A4IU-01 | 26 | 4 |
| STAD | TCGA-FP-8099-01 | 156 | 11 | STES | TCGA-BR-6455-01 | 114 | 6 | STES | TCGA-BR-A4IV-01 | 28 | 4 |
| STAD | TCGA-FP-8209-01 | 2 | 0 | STES | TCGA-BR-6456-01 | 51 | 2 | STES | TCGA-BR-A4IY-01 | 158 | 3 |
| STAD | TCGA-FP-8210-01 | 6 | 1 | STES | TCGA-BR-6457-01 | 143 | 5 | STES | TCGA-BR-A4IZ-01 | 10 | 0 |
| STAD | TCGA-FP-8211-01 | 118 | 12 | STES | TCGA-BR-6458-01 | 344 | 6 | STES | TCGA-BR-A4J1-01 | 79 | 1 |
| STAD | TCGA-FP-8631-01 | 108 | 5 | STES | TCGA-BR-6563-01 | 86 | 5 | STES | TCGA-BR-A4J2-01 | 49 | 3 |
| STAD | TCGA-FP-A4BE-01 | 1806 | 64 | STES | TCGA-BR-6564-01 | 42 | 2 | STES | TCGA-BR-A4J4-01 | 90 | 5 |
| STAD | TCGA-FP-A4BF-01 | 125 | 5 | STES | TCGA-BR-6565-01 | 169 | 9 | STES | TCGA-BR-A4J6-01 | 40 | 6 |
| STAD | TCGA-HF-7132-01 | 1503 | 59 | STES | TCGA-BR-6566-01 | 1184 | 48 | STES | TCGA-BR-A4J7-01 | 57 | 4 |
| STAD | TCGA-HF-7133-01 | 88 | 0 | STES | TCGA-BR-6705-01 | 120 | 7 | STES | TCGA-BR-A4J8-01 | 111 | 9 |
| STAD | TCGA-HF-7136-01 | 94 | 3 | STES | TCGA-BR-6706-01 | 256 | 12 | STES | TCGA-BR-A4PD-01 | 173 | 9 |
| STAD | TCGA-HJ-7597-01 | 953 | 47 | STES | TCGA-BR-6707-01 | 80 | 11 | STES | TCGA-BR-A4PE-01 | 239 | 10 |
| STAD | TCGA-HU-8243-01 | 146 | 7 | STES | TCGA-BR-6710-01 | 1 | 0 | STES | TCGA-BR-A4PF-01 | 110 | 6 |
| STAD | TCGA-HU-8245-01 | 78 | 8 | STES | TCGA-BR-6801-01 | 58 | 4 | STES | TCGA-BR-A4QI-01 | 151 | 10 |
| STAD | TCGA-HU-8249-01 | 166 | 6 | STES | TCGA-BR-6802-01 | 585 | 27 | STES | TCGA-BR-A4QL-01 | 2225 | 94 |

# EP 3 423 828 B1

## TABLE 5 (APPENDIX A)

| | | | |
|---|---|---|---|
| STES | TCGA-BR-A4QM-01 | 116 | 5 |
| STES | TCGA-CD-5798-01 | 100 | 5 |
| STES | TCGA-CD-5799-01 | 56 | 5 |
| STES | TCGA-CD-5800-01 | 177 | 11 |
| STES | TCGA-CD-5801-01 | 594 | 28 |
| STES | TCGA-CD-5802-01 | 62 | 1 |
| STES | TCGA-CD-5803-01 | 45 | 4 |
| STES | TCGA-CD-5804-01 | 105 | 4 |
| STES | TCGA-CD-5813-01 | 200 | 11 |
| STES | TCGA-CD-8524-01 | 170 | 5 |
| STES | TCGA-CD-8525-01 | 93 | 2 |
| STES | TCGA-CD-8526-01 | 49 | 4 |
| STES | TCGA-CD-8527-01 | 305 | 11 |
| STES | TCGA-CD-8528-01 | 116 | 3 |
| STES | TCGA-CD-8529-01 | 458 | 17 |
| STES | TCGA-CD-8530-01 | 101 | 6 |
| STES | TCGA-CD-8531-01 | 267 | 25 |
| STES | TCGA-CD-8532-01 | 57 | 3 |
| STES | TCGA-CD-8534-01 | 84 | 6 |
| STES | TCGA-CD-8535-01 | 302 | 17 |
| STES | TCGA-CD-8536-01 | 1080 | 53 |
| STES | TCGA-CD-A486-01 | 106 | 7 |
| STES | TCGA-CD-A487-01 | 203 | 5 |
| STES | TCGA-CD-A489-01 | 87 | 5 |
| STES | TCGA-CD-A48A-01 | 264 | 12 |
| STES | TCGA-CD-A48C-01 | 243 | 16 |
| STES | TCGA-CD-A4MG-01 | 3024 | 134 |
| STES | TCGA-CD-A4MH-01 | 98 | 3 |
| STES | TCGA-CD-A4MI-01 | 1170 | 40 |
| STES | TCGA-CD-A4MJ-01 | 807 | 26 |
| STES | TCGA-CG-4300-01 | 159 | 4 |
| STES | TCGA-CG-4301-01 | 58 | 3 |
| STES | TCGA-CG-4304-01 | 84 | 3 |
| STES | TCGA-CG-4305-01 | 1402 | 65 |
| STES | TCGA-CG-4306-01 | 1333 | 57 |
| STES | TCGA-CG-4436-01 | 207 | 11 |
| STES | TCGA-CG-4437-01 | 607 | 17 |
| STES | TCGA-CG-4438-01 | 156 | 10 |
| STES | TCGA-CG-4440-01 | 138 | 7 |
| STES | TCGA-CG-4441-01 | 137 | 8 |
| STES | TCGA-CG-4442-01 | 1888 | 78 |
| STES | TCGA-CG-4443-01 | 116 | 7 |
| STES | TCGA-CG-4444-01 | 203 | 10 |
| STES | TCGA-CG-4449-01 | 115 | 4 |
| STES | TCGA-CG-4455-01 | 57 | 5 |
| STES | TCGA-CG-4462-01 | 85 | 6 |
| STES | TCGA-CG-4465-01 | 766 | 33 |
| STES | TCGA-CG-4466-01 | 224 | 8 |
| STES | TCGA-CG-4469-01 | 346 | 19 |
| STES | TCGA-CG-4474-01 | 115 | 14 |
| STES | TCGA-CG-4475-01 | 61 | 1 |
| STES | TCGA-CG-4476-01 | 140 | 9 |
| STES | TCGA-CG-4477-01 | 153 | 8 |
| STES | TCGA-CG-5717-01 | 201 | 8 |
| STES | TCGA-CG-5718-01 | 100 | 4 |
| STES | TCGA-CG-5719-01 | 109 | 7 |
| STES | TCGA-CG-5720-01 | 110 | 9 |
| STES | TCGA-CG-5721-01 | 5091 | 210 |
| STES | TCGA-CG-5722-01 | 105 | 4 |
| STES | TCGA-CG-5723-01 | 2107 | 98 |
| STES | TCGA-CG-5724-01 | 132 | 6 |
| STES | TCGA-CG-5726-01 | 1705 | 56 |
| STES | TCGA-CG-5727-01 | 182 | 12 |
| STES | TCGA-CG-5728-01 | 1727 | 67 |
| STES | TCGA-CG-5730-01 | 195 | 8 |
| STES | TCGA-CG-5732-01 | 81 | 4 |
| STES | TCGA-CG-5733-01 | 1422 | 56 |
| STES | TCGA-CG-5734-01 | 121 | 3 |
| STES | TCGA-D7-5577-01 | 134 | 8 |
| STES | TCGA-D7-5578-01 | 290 | 18 |
| STES | TCGA-D7-5579-01 | 173 | 4 |
| STES | TCGA-D7-6518-01 | 113 | 6 |
| STES | TCGA-D7-6519-01 | 65 | 5 |
| STES | TCGA-D7-6520-01 | 76 | 2 |
| STES | TCGA-D7-6521-01 | 93 | 9 |
| STES | TCGA-D7-6522-01 | 84 | 6 |
| STES | TCGA-D7-6524-01 | 120 | 11 |
| STES | TCGA-D7-6525-01 | 186 | 8 |
| STES | TCGA-D7-6526-01 | 247 | 7 |
| STES | TCGA-D7-6527-01 | 251 | 16 |
| STES | TCGA-D7-6528-01 | 394 | 23 |
| STES | TCGA-D7-6815-01 | 171 | 10 |
| STES | TCGA-D7-6817-01 | 102 | 5 |
| STES | TCGA-D7-6818-01 | 175 | 5 |
| STES | TCGA-D7-6820-01 | 111 | 7 |
| STES | TCGA-D7-6822-01 | 279 | 15 |
| STES | TCGA-D7-8570-01 | 146 | 4 |
| STES | TCGA-D7-8572-01 | 196 | 11 |
| STES | TCGA-D7-8573-01 | 97 | 6 |
| STES | TCGA-D7-8574-01 | 35 | 3 |
| STES | TCGA-D7-8575-01 | 59 | 6 |
| STES | TCGA-D7-8576-01 | 69 | 1 |
| STES | TCGA-D7-8578-01 | 59 | 2 |
| STES | TCGA-D7-8579-01 | 120 | 2 |
| STES | TCGA-D7-A4YT-01 | 334 | 17 |
| STES | TCGA-D7-A4YU-01 | 241 | 15 |
| STES | TCGA-D7-A4YV-01 | 1076 | 42 |
| STES | TCGA-D7-A4YX-01 | 91 | 9 |
| STES | TCGA-D7-A4YY-01 | 579 | 20 |
| STES | TCGA-D7-A4Z0-01 | 334 | 18 |
| STES | TCGA-EQ-5647-01 | 102 | 5 |
| STES | TCGA-EQ-8122-01 | 211 | 7 |
| STES | TCGA-EQ-A4SO-01 | 199 | 13 |
| STES | TCGA-F1-6177-01 | 1597 | 65 |
| STES | TCGA-F1-6874-01 | 1125 | 47 |
| STES | TCGA-F1-6875-01 | 98 | 5 |
| STES | TCGA-F1-A448-01 | 687 | 27 |
| STES | TCGA-FP-7735-01 | 72 | 6 |
| STES | TCGA-FP-7829-01 | 284 | 9 |
| STES | TCGA-FP-7916-01 | 109 | 6 |
| STES | TCGA-FP-7998-01 | 80 | 6 |
| STES | TCGA-FP-8099-01 | 156 | 11 |
| STES | TCGA-FP-8209-01 | 2 | 0 |
| STES | TCGA-FP-8210-01 | 6 | 1 |
| STES | TCGA-FP-8211-01 | 118 | 12 |
| STES | TCGA-FP-8631-01 | 108 | 5 |
| STES | TCGA-FP-A4BE-01 | 1806 | 64 |
| STES | TCGA-FP-A4BF-01 | 125 | 5 |
| STES | TCGA-HF-7132-01 | 1503 | 59 |
| STES | TCGA-HF-7133-01 | 88 | 0 |
| STES | TCGA-HF-7136-01 | 94 | 3 |
| STES | TCGA-HJ-7597-01 | 953 | 47 |
| STES | TCGA-HU-8243-01 | 146 | 7 |
| STES | TCGA-HU-8245-01 | 78 | 8 |
| STES | TCGA-HU-8249-01 | 166 | 6 |
| STES | TCGA-HU-8602-01 | 1908 | 75 |
| STES | TCGA-HU-8604-01 | 235 | 12 |
| STES | TCGA-HU-8608-01 | 111 | 9 |
| STES | TCGA-HU-8610-01 | 74 | 2 |
| STES | TCGA-HU-A4G2-01 | 57 | 3 |
| STES | TCGA-HU-A4G3-01 | 118 | 5 |
| STES | TCGA-HU-A4G6-01 | 98 | 6 |
| STES | TCGA-HU-A4G8-01 | 1783 | 65 |
| STES | TCGA-HU-A4G9-01 | 951 | 31 |
| STES | TCGA-HU-A4GC-01 | 168 | 10 |
| STES | TCGA-HU-A4GD-01 | 96 | 1 |
| STES | TCGA-HU-A4GF-01 | 152 | 8 |
| STES | TCGA-HU-A4GH-01 | 854 | 20 |
| STES | TCGA-HU-A4GN-01 | 1405 | 56 |
| STES | TCGA-HU-A4GP-01 | 214 | 8 |
| STES | TCGA-HU-A4GQ-01 | 2206 | 81 |
| STES | TCGA-HU-A4GT-01 | 2026 | 79 |
| STES | TCGA-HU-A4GU-01 | 1764 | 77 |
| STES | TCGA-HU-A4GX-01 | 1490 | 55 |
| STES | TCGA-HU-A4GY-01 | 75 | 2 |
| STES | TCGA-HU-A4H0-01 | 196 | 11 |
| STES | TCGA-HU-A4H2-01 | 189 | 7 |
| STES | TCGA-HU-A4H3-01 | 1094 | 51 |
| STES | TCGA-HU-A4H4-01 | 802 | 41 |
| STES | TCGA-HU-A4H5-01 | 301 | 14 |
| STES | TCGA-HU-A4H6-01 | 102 | 1 |
| STES | TCGA-HU-A4H8-01 | 1453 | 62 |
| STES | TCGA-HU-A4HD-01 | 200 | 8 |
| STES | TCGA-IC-A6RE-01 | 1446 | 58 |
| STES | TCGA-IC-A6RF-01 | 472 | 22 |
| STES | TCGA-IG-A3I8-01 | 333 | 6 |
| STES | TCGA-IG-A3QL-01 | 169 | 15 |
| STES | TCGA-IG-A3Y9-01 | 324 | 12 |
| STES | TCGA-IG-A3YA-01 | 144 | 4 |
| STES | TCGA-IG-A3YB-01 | 146 | 6 |
| STES | TCGA-IG-A3YC-01 | 124 | 4 |
| STES | TCGA-IG-A4P3-01 | 530 | 17 |
| STES | TCGA-IG-A4QS-01 | 342 | 12 |
| STES | TCGA-IG-A4QT-01 | 48 | 0 |
| STES | TCGA-IG-A50L-01 | 204 | 11 |
| STES | TCGA-IG-A51D-01 | 254 | 7 |
| STES | TCGA-IG-A5B8-01 | 396 | 12 |
| STES | TCGA-IG-A5S3-01 | 124 | 5 |
| STES | TCGA-IG-A625-01 | 152 | 5 |
| STES | TCGA-IG-A6QS-01 | 207 | 9 |
| STES | TCGA-IG-A7DP-01 | 110 | 4 |
| STES | TCGA-IG-A8O2-01 | 268 | 7 |
| STES | TCGA-IG-A97H-01 | 272 | 10 |
| STES | TCGA-IG-A97I-01 | 216 | 11 |
| STES | TCGA-IN-7806-01 | 87 | 7 |
| STES | TCGA-IN-7808-01 | 122 | 7 |
| STES | TCGA-IN-8462-01 | 136 | 6 |
| STES | TCGA-IN-8663-01 | 188 | 10 |
| STES | TCGA-IP-7968-01 | 130 | 4 |
| STES | TCGA-JY-A6F8-01 | 444 | 15 |
| STES | TCGA-JY-A6FA-01 | 260 | 16 |
| STES | TCGA-JY-A6FB-01 | 316 | 9 |
| STES | TCGA-JY-A6FD-01 | 342 | 18 |
| STES | TCGA-JY-A6FE-01 | 217 | 5 |
| STES | TCGA-JY-A6FG-01 | 409 | 13 |
| STES | TCGA-JY-A6FH-01 | 345 | 15 |
| STES | TCGA-JY-A938-01 | 259 | 13 |
| STES | TCGA-JY-A939-01 | 229 | 13 |
| STES | TCGA-JY-A93C-01 | 220 | 6 |
| STES | TCGA-JY-A93D-01 | 340 | 10 |
| STES | TCGA-JY-A93E-01 | 345 | 15 |
| STES | TCGA-JY-A93F-01 | 252 | 15 |
| STES | TCGA-KH-A6WC-01 | 254 | 9 |
| STES | TCGA-L5-A43C-01 | 226 | 10 |
| STES | TCGA-L5-A43E-01 | 491 | 16 |
| STES | TCGA-L5-A43H-01 | 151 | 8 |
| STES | TCGA-L5-A43I-01 | 322 | 12 |
| STES | TCGA-L5-A43J-01 | 1294 | 39 |
| STES | TCGA-L5-A43M-01 | 93 | 0 |
| STES | TCGA-L5-A4OE-01 | 450 | 15 |
| STES | TCGA-L5-A4OF-01 | 159 | 6 |
| STES | TCGA-L5-A4OG-01 | 260 | 14 |
| STES | TCGA-L5-A4OH-01 | 441 | 13 |
| STES | TCGA-L5-A4OI-01 | 3225 | 121 |
| STES | TCGA-L5-A4OJ-01 | 559 | 20 |
| STES | TCGA-L5-A4OM-01 | 114 | 4 |
| STES | TCGA-L5-A4ON-01 | 234 | 14 |
| STES | TCGA-L5-A4OO-01 | 175 | 9 |
| STES | TCGA-L5-A4OP-01 | 180 | 10 |
| STES | TCGA-L5-A4OQ-01 | 112 | 6 |
| STES | TCGA-L5-A4OR-01 | 259 | 7 |
| STES | TCGA-L5-A4OS-01 | 159 | 3 |
| STES | TCGA-L5-A4OT-01 | 358 | 13 |
| STES | TCGA-L5-A4OU-01 | 276 | 12 |
| STES | TCGA-L5-A4OW-01 | 362 | 15 |
| STES | TCGA-L5-A4OX-01 | 212 | 5 |
| STES | TCGA-L5-A88S-01 | 218 | 11 |
| STES | TCGA-L5-A88T-01 | 155 | 7 |
| STES | TCGA-L5-A88V-01 | 257 | 12 |
| STES | TCGA-L5-A88W-01 | 234 | 15 |
| STES | TCGA-L5-A88Y-01 | 5 | 1 |
| STES | TCGA-L5-A88Z-01 | 253 | 19 |
| STES | TCGA-L5-A891-01 | 361 | 23 |
| STES | TCGA-L5-A893-01 | 329 | 13 |
| STES | TCGA-L5-A8NE-01 | 425 | 10 |
| STES | TCGA-L5-A8NF-01 | 317 | 16 |
| STES | TCGA-L5-A8NG-01 | 366 | 13 |
| STES | TCGA-L5-A8NH-01 | 361 | 11 |
| STES | TCGA-L5-A8NI-01 | 373 | 13 |
| STES | TCGA-L5-A8NJ-01 | 425 | 17 |
| STES | TCGA-L5-A8NK-01 | 379 | 13 |
| STES | TCGA-L5-A8NL-01 | 296 | 14 |
| STES | TCGA-L5-A8NM-01 | 2624 | 93 |
| STES | TCGA-L5-A8NN-01 | 273 | 16 |
| STES | TCGA-L5-A8NQ-01 | 435 | 15 |
| STES | TCGA-L5-A8NR-01 | 407 | 10 |
| STES | TCGA-L5-A8NS-01 | 581 | 27 |
| STES | TCGA-L5-A8NT-01 | 265 | 11 |
| STES | TCGA-L5-A8NU-01 | 160 | 3 |
| STES | TCGA-L5-A8NV-01 | 309 | 15 |
| STES | TCGA-L5-A8NW-01 | 399 | 12 |
| STES | TCGA-L7-A56G-01 | 132 | 12 |
| STES | TCGA-L7-A6VZ-01 | 462 | 28 |
| STES | TCGA-LN-A49K-01 | 145 | 6 |
| STES | TCGA-LN-A49L-01 | 252 | 5 |
| STES | TCGA-LN-A49M-01 | 339 | 13 |
| STES | TCGA-LN-A49N-01 | 107 | 10 |
| STES | TCGA-LN-A49O-01 | 127 | 6 |
| STES | TCGA-LN-A49P-01 | 122 | 8 |
| STES | TCGA-LN-A49R-01 | 190 | 8 |
| STES | TCGA-LN-A49S-01 | 234 | 10 |
| STES | TCGA-LN-A49U-01 | 242 | 4 |
| STES | TCGA-LN-A49V-01 | 152 | 6 |
| STES | TCGA-LN-A49W-01 | 158 | 11 |
| STES | TCGA-LN-A49X-01 | 150 | 8 |
| STES | TCGA-LN-A49Y-01 | 361 | 11 |
| STES | TCGA-LN-A4A1-01 | 230 | 11 |
| STES | TCGA-LN-A4A2-01 | 251 | 6 |
| STES | TCGA-LN-A4A3-01 | 150 | 4 |
| STES | TCGA-LN-A4A4-01 | 186 | 9 |
| STES | TCGA-LN-A4A5-01 | 142 | 5 |
| STES | TCGA-LN-A4A6-01 | 194 | 9 |
| STES | TCGA-LN-A4A8-01 | 213 | 10 |
| STES | TCGA-LN-A4A9-01 | 297 | 11 |

## TABLE 5 (APPENDIX A)

| | | | |
|---|---|---|---|
| STES | TCGA-LN-A4MQ-01 | 111 | 10 |
| STES | TCGA-LN-A4MR-01 | 235 | 11 |
| STES | TCGA-LN-A5U5-01 | 96 | 7 |
| STES | TCGA-LN-A5U6-01 | 151 | 9 |
| STES | TCGA-LN-A5U7-01 | 226 | 8 |
| STES | TCGA-LN-A7HV-01 | 182 | 8 |
| STES | TCGA-LN-A7HW-01 | 170 | 5 |
| STES | TCGA-LN-A7HX-01 | 362 | 14 |
| STES | TCGA-LN-A7HY-01 | 278 | 9 |
| STES | TCGA-LN-A7HZ-01 | 149 | 7 |
| STES | TCGA-LN-A8HZ-01 | 279 | 15 |
| STES | TCGA-LN-A8I0-01 | 272 | 11 |
| STES | TCGA-LN-A8I1-01 | 269 | 12 |
| STES | TCGA-LN-A9FO-01 | 251 | 10 |
| STES | TCGA-LN-A9FP-01 | 638 | 18 |
| STES | TCGA-LN-A9FQ-01 | 224 | 9 |
| STES | TCGA-LN-A9FR-01 | 203 | 13 |
| STES | TCGA-M9-A5M8-01 | 129 | 11 |
| STES | TCGA-Q9-A6FU-01 | 302 | 8 |
| STES | TCGA-Q9-A6FW-01 | 335 | 11 |
| STES | TCGA-R6-A6DN-01 | 225 | 10 |
| STES | TCGA-R6-A6DQ-01 | 176 | 11 |
| STES | TCGA-R6-A6KZ-01 | 256 | 14 |
| STES | TCGA-R6-A6L4-01 | 181 | 11 |
| STES | TCGA-R6-A6L6-01 | 271 | 11 |
| STES | TCGA-R6-A6XG-01 | 457 | 22 |
| STES | TCGA-R6-A6XQ-01 | 304 | 10 |
| STES | TCGA-R6-A6Y0-01 | 389 | 16 |
| STES | TCGA-R6-A6Y2-01 | 362 | 15 |
| STES | TCGA-R6-A8W5-01 | 239 | 12 |
| STES | TCGA-R6-A8W8-01 | 308 | 12 |
| STES | TCGA-R6-A8WC-01 | 319 | 11 |
| STES | TCGA-R6-A8WG-01 | 286 | 10 |
| STES | TCGA-RE-A7BO-01 | 431 | 12 |
| STES | TCGA-S8-A6BV-01 | 263 | 6 |
| STES | TCGA-S8-A6BW-01 | 243 | 9 |
| STES | TCGA-V5-A7RB-01 | 338 | 10 |
| STES | TCGA-V5-A7RC-01 | 256 | 14 |
| STES | TCGA-V5-A7RE-01 | 350 | 11 |
| STES | TCGA-V5-AASV-01 | 269 | 19 |
| STES | TCGA-V5-AASW-01 | 267 | 4 |
| STES | TCGA-V5-AASX-01 | 747 | 30 |
| STES | TCGA-VR-A8EO-01 | 186 | 8 |
| STES | TCGA-VR-A8EP-01 | 176 | 9 |
| STES | TCGA-VR-A8EQ-01 | 392 | 14 |
| STES | TCGA-VR-A8ER-01 | 198 | 6 |
| STES | TCGA-VR-A8ET-01 | 73 | 2 |
| STES | TCGA-VR-A8EU-01 | 274 | 12 |
| STES | TCGA-VR-A8EW-01 | 217 | 13 |
| STES | TCGA-VR-A8EX-01 | 340 | 13 |
| STES | TCGA-VR-A8EY-01 | 251 | 11 |
| STES | TCGA-VR-A8EZ-01 | 374 | 21 |
| STES | TCGA-VR-A8Q7-01 | 215 | 12 |
| STES | TCGA-VR-AA4D-01 | 236 | 12 |
| STES | TCGA-VR-AA4G-01 | 222 | 11 |
| STES | TCGA-VR-AA7B-01 | 288 | 10 |
| STES | TCGA-VR-AA7D-01 | 190 | 14 |
| STES | TCGA-VR-AA7I-01 | 151 | 3 |
| STES | TCGA-X8-AAAR-01 | 243 | 12 |
| STES | TCGA-XP-A8T6-01 | 277 | 8 |
| STES | TCGA-XP-A8T7-01 | 288 | 13 |
| STES | TCGA-XP-A8T8-01 | 287 | 9 |
| STES | TCGA-Z6-A8JD-01 | 337 | 12 |
| STES | TCGA-Z6-A8JE-01 | 337 | 8 |
| STES | TCGA-Z6-A9VB-01 | 249 | 13 |
| STES | TCGA-Z6-AAPN-01 | 532 | 21 |
| STES | TCGA-ZR-A9CJ-01 | 258 | 9 |
| TGCT | TCGA-2G-AAEW-01 | 73 | 3 |
| TGCT | TCGA-2G-AAEX-01 | 93 | 4 |
| TGCT | TCGA-2G-AAF4-01 | 62 | 4 |
| TGCT | TCGA-2G-AAF6-01 | 68 | 4 |
| TGCT | TCGA-2G-AAF6-01 | 75 | 6 |
| TGCT | TCGA-2G-AAF8-01 | 115 | 6 |
| TGCT | TCGA-2G-AAFG-01 | 93 | 3 |
| TGCT | TCGA-2G-AAFG-05 | 88 | 1 |
| TGCT | TCGA-2G-AAFH-01 | 97 | 6 |
| TGCT | TCGA-2G-AAFI-01 | 67 | 2 |
| TGCT | TCGA-2G-AAFJ-01 | 97 | 1 |
| TGCT | TCGA-2G-AAFL-01 | 74 | 1 |
| TGCT | TCGA-2G-AAFM-01 | 78 | 4 |
| TGCT | TCGA-2G-AAFN-01 | 74 | 2 |
| TGCT | TCGA-2G-AAFO-01 | 61 | 1 |
| TGCT | TCGA-2G-AAFV-01 | 83 | 2 |
| TGCT | TCGA-2G-AAFY-01 | 66 | 3 |
| TGCT | TCGA-2G-AAFZ-01 | 83 | 3 |
| TGCT | TCGA-2G-AAG0-01 | 94 | 3 |
| TGCT | TCGA-2G-AAG3-01 | 86 | 2 |
| TGCT | TCGA-2G-AAG5-01 | 88 | 2 |
| TGCT | TCGA-2G-AAG6-01 | 76 | 1 |
| TGCT | TCGA-2G-AAG7-01 | 86 | 5 |
| TGCT | TCGA-2G-AAG8-01 | 85 | 1 |
| TGCT | TCGA-2G-AAG9-01 | 107 | 3 |
| TGCT | TCGA-2G-AAGA-01 | 106 | 4 |
| TGCT | TCGA-2G-AAGC-01 | 86 | 1 |
| TGCT | TCGA-2G-AAGE-01 | 120 | 5 |
| TGCT | TCGA-2G-AAGF-01 | 92 | 4 |
| TGCT | TCGA-2G-AAGG-01 | 110 | 4 |
| TGCT | TCGA-2G-AAGI-01 | 110 | 7 |
| TGCT | TCGA-2G-AAGI-05 | 99 | 5 |
| TGCT | TCGA-2G-AAGJ-01 | 114 | 6 |
| TGCT | TCGA-2G-AAGK-01 | 75 | 4 |
| TGCT | TCGA-2G-AAGM-01 | 87 | 5 |
| TGCT | TCGA-2G-AAGN-01 | 76 | 0 |
| TGCT | TCGA-2G-AAGO-01 | 97 | 4 |
| TGCT | TCGA-2G-AAGP-01 | 90 | 3 |
| TGCT | TCGA-2G-AAGS-01 | 80 | 1 |
| TGCT | TCGA-2G-AAGT-01 | 99 | 1 |
| TGCT | TCGA-2G-AAGV-01 | 81 | 2 |
| TGCT | TCGA-2G-AAGW-01 | 87 | 6 |
| TGCT | TCGA-2G-AAGX-01 | 75 | 4 |
| TGCT | TCGA-2G-AAGY-01 | 107 | 3 |
| TGCT | TCGA-2G-AAGY-05 | 86 | 2 |
| TGCT | TCGA-2G-AAGZ-01 | 86 | 1 |
| TGCT | TCGA-2G-AAH0-01 | 69 | 1 |
| TGCT | TCGA-2G-AAH2-01 | 81 | 1 |
| TGCT | TCGA-2G-AAH3-01 | 88 | 2 |
| TGCT | TCGA-2G-AAH4-01 | 108 | 3 |
| TGCT | TCGA-2G-AAH8-01 | 95 | 4 |
| TGCT | TCGA-2G-AAHA-01 | 89 | 3 |
| TGCT | TCGA-2G-AAHC-01 | 86 | 3 |
| TGCT | TCGA-2G-AAHG-01 | 79 | 3 |
| TGCT | TCGA-2G-AAHL-01 | 87 | 4 |
| TGCT | TCGA-2G-AAHN-01 | 83 | 4 |
| TGCT | TCGA-2G-AAHP-01 | 101 | 4 |
| TGCT | TCGA-2G-AAHP-05 | 103 | 8 |
| TGCT | TCGA-2G-AAHT-01 | 99 | 3 |
| TGCT | TCGA-2G-AAKD-01 | 58 | 1 |
| TGCT | TCGA-2G-AAKG-01 | 91 | 0 |
| TGCT | TCGA-2G-AAKG-05 | 92 | 1 |
| TGCT | TCGA-2G-AAKH-01 | 156 | 6 |
| TGCT | TCGA-2G-AAKL-01 | 85 | 4 |
| TGCT | TCGA-2G-AAKM-01 | 73 | 4 |
| TGCT | TCGA-2G-AAKO-01 | 89 | 5 |
| TGCT | TCGA-2G-AAKO-05 | 76 | 5 |
| TGCT | TCGA-2G-AAL5-01 | 87 | 4 |
| TGCT | TCGA-2G-AAL7-01 | 88 | 2 |
| TGCT | TCGA-2G-AALF-01 | 91 | 2 |
| TGCT | TCGA-2G-AALG-01 | 83 | 3 |
| TGCT | TCGA-2G-AALN-01 | 76 | 1 |
| TGCT | TCGA-2G-AALO-01 | 81 | 5 |
| TGCT | TCGA-2G-AALP-01 | 104 | 1 |
| TGCT | TCGA-2G-AALQ-01 | 85 | 2 |
| TGCT | TCGA-2G-AALR-01 | 119 | 6 |
| TGCT | TCGA-2G-AALS-01 | 72 | 5 |
| TGCT | TCGA-2G-AALT-01 | 61 | 1 |
| TGCT | TCGA-2G-AALW-01 | 87 | 1 |
| TGCT | TCGA-2G-AALX-01 | 86 | 3 |
| TGCT | TCGA-2G-AALY-01 | 77 | 1 |
| TGCT | TCGA-2G-AALZ-01 | 85 | 2 |
| TGCT | TCGA-2G-AAM2-01 | 50 | 2 |
| TGCT | TCGA-2G-AAM3-01 | 83 | 3 |
| TGCT | TCGA-2G-AAM8-01 | 84 | 3 |
| TGCT | TCGA-2X-A9D5-01 | 86 | 2 |
| TGCT | TCGA-2X-A9D6-01 | 75 | 3 |
| TGCT | TCGA-4K-AA1G-01 | 74 | 2 |
| TGCT | TCGA-4K-AA1H-01 | 59 | 6 |
| TGCT | TCGA-4K-AA1I-01 | 62 | 0 |
| TGCT | TCGA-4K-AAAL-01 | 86 | 7 |
| TGCT | TCGA-S6-A8JW-01 | 72 | 3 |
| TGCT | TCGA-S6-A8JX-01 | 76 | 2 |
| TGCT | TCGA-S6-A8JY-01 | 98 | 0 |
| TGCT | TCGA-SB-A6J6-01 | 88 | 1 |
| TGCT | TCGA-SB-A76C-01 | 85 | 5 |
| TGCT | TCGA-SN-A6IS-01 | 104 | 2 |
| TGCT | TCGA-SN-A84W-01 | 99 | 3 |
| TGCT | TCGA-SN-A84X-01 | 97 | 3 |
| TGCT | TCGA-SN-A84Y-01 | 94 | 1 |
| TGCT | TCGA-SO-A8JP-01 | 108 | 3 |
| TGCT | TCGA-VF-A8A8-01 | 579 | 28 |
| TGCT | TCGA-VF-A8A9-01 | 239 | 2 |
| TGCT | TCGA-VF-A8AA-01 | 125 | 2 |
| TGCT | TCGA-VF-A8AB-01 | 100 | 2 |
| TGCT | TCGA-VF-A8AC-01 | 100 | 2 |
| TGCT | TCGA-VF-A8AD-01 | 91 | 4 |
| TGCT | TCGA-VF-A8AE-01 | 81 | 1 |
| TGCT | TCGA-W4-A7U2-01 | 349 | 4 |
| TGCT | TCGA-W4-A7U3-01 | 472 | 3 |
| TGCT | TCGA-W4-A7U4-01 | 45 | 0 |
| TGCT | TCGA-WZ-A7V3-01 | 98 | 1 |
| TGCT | TCGA-WZ-A7V4-01 | 85 | 4 |
| TGCT | TCGA-WZ-A7V5-01 | 110 | 7 |
| TGCT | TCGA-WZ-A8D5-01 | 84 | 4 |
| TGCT | TCGA-X3-A8G4-01 | 114 | 3 |
| TGCT | TCGA-XE-A8H1-01 | 99 | 7 |
| TGCT | TCGA-XE-A8H4-01 | 96 | 7 |
| TGCT | TCGA-XE-A8H5-01 | 102 | 6 |
| TGCT | TCGA-XE-A9SE-01 | 74 | 6 |
| TGCT | TCGA-XE-AANI-01 | 109 | 6 |
| TGCT | TCGA-XE-AANJ-01 | 98 | 2 |
| TGCT | TCGA-XE-AANR-01 | 81 | 1 |
| TGCT | TCGA-XE-AANV-01 | 77 | 7 |
| TGCT | TCGA-XE-AAO3-01 | 71 | 1 |
| TGCT | TCGA-XE-AAO4-01 | 108 | 4 |
| TGCT | TCGA-XE-AAO6-01 | 80 | 2 |
| TGCT | TCGA-XE-AAOB-01 | 73 | 2 |
| TGCT | TCGA-XE-AAOC-01 | 84 | 5 |
| TGCT | TCGA-XE-AAOD-01 | 78 | 3 |
| TGCT | TCGA-XE-AAOF-01 | 80 | 5 |
| TGCT | TCGA-XE-AAOJ-01 | 82 | 4 |
| TGCT | TCGA-XE-AAOL-01 | 79 | 1 |
| TGCT | TCGA-XY-A89B-01 | 94 | 4 |
| TGCT | TCGA-XY-A8S2-01 | 148 | 7 |
| TGCT | TCGA-XY-A8S3-01 | 67 | 1 |
| TGCT | TCGA-XY-A9T9-01 | 67 | 3 |
| TGCT | TCGA-YU-A90P-01 | 69 | 0 |
| TGCT | TCGA-YU-A90Q-01 | 63 | 4 |
| TGCT | TCGA-YU-A90S-01 | 60 | 4 |
| TGCT | TCGA-YU-A90W-01 | 76 | 2 |
| TGCT | TCGA-YU-A90Y-01 | 70 | 2 |
| TGCT | TCGA-YU-A912-01 | 69 | 3 |
| TGCT | TCGA-YU-A94D-01 | 91 | 4 |
| TGCT | TCGA-YU-A94I-01 | 115 | 6 |
| TGCT | TCGA-YU-AA4L-01 | 71 | 1 |
| TGCT | TCGA-YU-AA61-01 | 62 | 2 |
| TGCT | TCGA-ZM-AA05-01 | 101 | 4 |
| TGCT | TCGA-ZM-AA06-01 | 98 | 3 |
| TGCT | TCGA-ZM-AA08-01 | 77 | 2 |
| TGCT | TCGA-ZM-AA0D-01 | 52 | 4 |
| TGCT | TCGA-ZM-AA0E-01 | 76 | 5 |
| TGCT | TCGA-ZM-AA0F-01 | 49 | 2 |
| TGCT | TCGA-ZM-AA0H-01 | 105 | 3 |
| TGCT | TCGA-ZM-AA0N-01 | 65 | 2 |
| THCA | TCGA-BJ-A0YZ-01 | 41 | 1 |
| THCA | TCGA-BJ-A0Z0-01 | 35 | 6 |
| THCA | TCGA-BJ-A0Z2-01 | 19 | 1 |
| THCA | TCGA-BJ-A0Z3-01 | 18 | 3 |
| THCA | TCGA-BJ-A0Z9-01 | 25 | 1 |
| THCA | TCGA-BJ-A0ZA-01 | 19 | 1 |
| THCA | TCGA-BJ-A0ZB-01 | 62 | 5 |
| THCA | TCGA-BJ-A0ZC-01 | 21 | 1 |
| THCA | TCGA-BJ-A0ZE-01 | 18 | 3 |
| THCA | TCGA-BJ-A0ZG-01 | 18 | 0 |
| THCA | TCGA-BJ-A0ZH-01 | 38 | 3 |
| THCA | TCGA-BJ-A0ZJ-01 | 3 | 0 |
| THCA | TCGA-BJ-A18Y-01 | 16 | 2 |
| THCA | TCGA-BJ-A18Z-01 | 23 | 1 |
| THCA | TCGA-BJ-A191-01 | 24 | 2 |
| THCA | TCGA-BJ-A192-01 | 9 | 1 |
| THCA | TCGA-BJ-A28R-01 | 33 | 4 |
| THCA | TCGA-BJ-A28S-01 | 29 | 3 |
| THCA | TCGA-BJ-A28T-01 | 3 | 1 |
| THCA | TCGA-BJ-A28V-01 | 25 | 2 |
| THCA | TCGA-BJ-A28X-01 | 59 | 2 |
| THCA | TCGA-BJ-A28Z-01 | 27 | 0 |
| THCA | TCGA-BJ-A290-01 | 71 | 6 |
| THCA | TCGA-BJ-A2N7-01 | 13 | 4 |
| THCA | TCGA-BJ-A2N8-01 | 35 | 3 |
| THCA | TCGA-BJ-A2N9-01 | 32 | 2 |
| THCA | TCGA-BJ-A2NA-01 | 90 | 6 |
| THCA | TCGA-BJ-A2P4-01 | 22 | 1 |
| THCA | TCGA-BJ-A3EZ-01 | 29 | 4 |
| THCA | TCGA-BJ-A3F0-01 | 15 | 2 |
| THCA | TCGA-BJ-A3PR-01 | 39 | 2 |
| THCA | TCGA-BJ-A3PT-01 | 29 | 3 |
| THCA | TCGA-BJ-A3PU-01 | 54 | 2 |
| THCA | TCGA-BJ-A45D-01 | 10 | 2 |
| THCA | TCGA-BJ-A45E-01 | 5 | 0 |
| THCA | TCGA-BJ-A45F-01 | 10 | 1 |
| THCA | TCGA-BJ-A45G-01 | 21 | 2 |
| THCA | TCGA-BJ-A45I-01 | 10 | 2 |
| THCA | TCGA-BJ-A45J-01 | 31 | 6 |
| THCA | TCGA-BJ-A45K-01 | 22 | 0 |
| THCA | TCGA-BJ-A4O8-01 | 4 | 1 |
| THCA | TCGA-BJ-A4O9-01 | 33 | 2 |

# TABLE 5 (APPENDIX A)

| | | | | | | | | | | | |
|---|---|---|---|---|---|---|---|---|---|---|---|
| THCA | TCGA-CE-A13K-01 | 15 | 0 | THCA | TCGA-DJ-A4V0-01 | 5 | 0 | THCA | TCGA-EL-A4K0-01 | 11 | 1 |
| THCA | TCGA-CE-A27D-01 | 9 | 0 | THCA | TCGA-DJ-A4V2-01 | 11 | 2 | THCA | TCGA-EL-A4K2-01 | 11 | 3 |
| THCA | TCGA-CE-A3MD-01 | 14 | 0 | THCA | TCGA-DJ-A4V4-01 | 23 | 4 | THCA | TCGA-EL-A4K4-01 | 11 | 1 |
| THCA | TCGA-CE-A3ME-01 | 22 | 1 | THCA | TCGA-DJ-A4V5-01 | 8 | 0 | THCA | TCGA-EL-A4K6-01 | 28 | 3 |
| THCA | TCGA-CE-A4B2-01 | 8 | 1 | THCA | TCGA-DO-A1JZ-01 | 15 | 2 | THCA | TCGA-EL-A4KD-01 | 18 | 2 |
| THCA | TCGA-CE-A484-01 | 18 | 2 | THCA | TCGA-DO-A1K0-01 | 9 | 2 | THCA | TCGA-EL-A4KG-01 | 55 | 4 |
| THCA | TCGA-CE-A485-01 | 3 | 0 | THCA | TCGA-DO-A2HM-01 | 90 | 6 | THCA | TCGA-EL-A4KH-01 | 5 | 1 |
| THCA | TCGA-DE-A0XZ-01 | 32 | 3 | THCA | TCGA-E3-A3DY-01 | 15 | 2 | THCA | TCGA-EL-A4KI-01 | 19 | 2 |
| THCA | TCGA-DE-A0Y2-01 | 26 | 0 | THCA | TCGA-E3-A3DZ-01 | 30 | 3 | THCA | TCGA-EM-A1CS-01 | 29 | 0 |
| THCA | TCGA-DE-A0Y3-01 | 20 | 3 | THCA | TCGA-E3-A3E0-01 | 15 | 0 | THCA | TCGA-EM-A1CT-01 | 55 | 6 |
| THCA | TCGA-DE-A2OL-01 | 13 | 0 | THCA | TCGA-E3-A3E1-01 | 24 | 1 | THCA | TCGA-EM-A1CU-01 | 36 | 2 |
| THCA | TCGA-DE-A3KN-01 | 20 | 1 | THCA | TCGA-E3-A3E2-01 | 11 | 2 | THCA | TCGA-EM-A1CV-01 | 22 | 4 |
| THCA | TCGA-DE-A4M8-01 | 11 | 2 | THCA | TCGA-E3-A3E3-01 | 14 | 3 | THCA | TCGA-EM-A1CW-01 | 32 | 2 |
| THCA | TCGA-DE-A4M9-01 | 10 | 0 | THCA | TCGA-E3-A3E5-01 | 24 | 3 | THCA | TCGA-EM-A1YA-01 | 11 | 0 |
| THCA | TCGA-DJ-A13L-01 | 40 | 4 | THCA | TCGA-E8-A242-01 | 26 | 1 | THCA | TCGA-EM-A1YB-01 | 47 | 1 |
| THCA | TCGA-DJ-A13M-01 | 10 | 1 | THCA | TCGA-E8-A2EA-01 | 30 | 1 | THCA | TCGA-EM-A1YC-01 | 19 | 1 |
| THCA | TCGA-DJ-A13O-01 | 7 | 1 | THCA | TCGA-E8-A413-01 | 5 | 1 | THCA | TCGA-EM-A1YD-01 | 9 | 1 |
| THCA | TCGA-DJ-A13P-01 | 20 | 1 | THCA | TCGA-E8-A415-01 | 10 | 1 | THCA | TCGA-EM-A1YE-01 | 13 | 0 |
| THCA | TCGA-DJ-A13R-01 | 22 | 0 | THCA | TCGA-E8-A418-01 | 35 | 3 | THCA | TCGA-EM-A22I-01 | 12 | 1 |
| THCA | TCGA-DJ-A13S-01 | 13 | 1 | THCA | TCGA-E8-A419-01 | 7 | 1 | THCA | TCGA-EM-A22J-01 | 20 | 1 |
| THCA | TCGA-DJ-A13T-01 | 12 | 1 | THCA | TCGA-E8-A433-01 | 13 | 4 | THCA | TCGA-EM-A22K-01 | 17 | 2 |
| THCA | TCGA-DJ-A13U-01 | 22 | 2 | THCA | TCGA-E8-A436-01 | 15 | 1 | THCA | TCGA-EM-A22L-01 | 10 | 2 |
| THCA | TCGA-DJ-A13V-01 | 12 | 1 | THCA | TCGA-E8-A437-01 | 16 | 1 | THCA | TCGA-EM-A22M-01 | 5 | 1 |
| THCA | TCGA-DJ-A13W-01 | 18 | 1 | THCA | TCGA-E8-A44K-01 | 11 | 2 | THCA | TCGA-EM-A22N-01 | 9 | 1 |
| THCA | TCGA-DJ-A13X-01 | 23 | 1 | THCA | TCGA-E8-A44M-01 | 3 | 0 | THCA | TCGA-EM-A22O-01 | 41 | 3 |
| THCA | TCGA-DJ-A1QD-01 | 11 | 1 | THCA | TCGA-EL-A3CL-01 | 10 | 1 | THCA | TCGA-EM-A22P-01 | 17 | 1 |
| THCA | TCGA-DJ-A1QE-01 | 22 | 1 | THCA | TCGA-EL-A3CM-01 | 15 | 1 | THCA | TCGA-EM-A22Q-01 | 12 | 1 |
| THCA | TCGA-DJ-A1QF-01 | 17 | 1 | THCA | TCGA-EL-A3CN-01 | 33 | 3 | THCA | TCGA-EM-A2CJ-01 | 17 | 0 |
| THCA | TCGA-DJ-A1QG-01 | 23 | 2 | THCA | TCGA-EL-A3CO-01 | 8 | 0 | THCA | TCGA-EM-A2CK-01 | 11 | 1 |
| THCA | TCGA-DJ-A1QH-01 | 27 | 2 | THCA | TCGA-EL-A3CP-01 | 6 | 1 | THCA | TCGA-EM-A2CL-01 | 8 | 0 |
| THCA | TCGA-DJ-A1QI-01 | 16 | 3 | THCA | TCGA-EL-A3CR-01 | 35 | 2 | THCA | TCGA-EM-A2CN-01 | 19 | 2 |
| THCA | TCGA-DJ-A1QL-01 | 21 | 1 | THCA | TCGA-EL-A3CS-01 | 20 | 1 | THCA | TCGA-EM-A2CO-01 | 19 | 1 |
| THCA | TCGA-DJ-A1QM-01 | 13 | 1 | THCA | TCGA-EL-A3CT-01 | 96 | 5 | THCA | TCGA-EM-A2CP-01 | 7 | 0 |
| THCA | TCGA-DJ-A1QN-01 | 9 | 1 | THCA | TCGA-EL-A3CU-01 | 18 | 1 | THCA | TCGA-EM-A2CQ-01 | 9 | 1 |
| THCA | TCGA-DJ-A1QO-01 | 20 | 2 | THCA | TCGA-EL-A3CV-01 | 9 | 1 | THCA | TCGA-EM-A2CR-01 | 14 | 1 |
| THCA | TCGA-DJ-A1QQ-01 | 19 | 2 | THCA | TCGA-EL-A3CW-01 | 12 | 1 | THCA | TCGA-EM-A2CT-01 | 10 | 2 |
| THCA | TCGA-DJ-A2PN-01 | 9 | 1 | THCA | TCGA-EL-A3CX-01 | 10 | 1 | THCA | TCGA-EM-A2CU-01 | 8 | 0 |
| THCA | TCGA-DJ-A2PO-01 | 17 | 1 | THCA | TCGA-EL-A3CY-01 | 4 | 0 | THCA | TCGA-EM-A2OV-01 | 28 | 1 |
| THCA | TCGA-DJ-A2PP-01 | 21 | 1 | THCA | TCGA-EL-A3CZ-01 | 6 | 1 | THCA | TCGA-EM-A2OW-01 | 19 | 2 |
| THCA | TCGA-DJ-A2PQ-01 | 10 | 1 | THCA | TCGA-EL-A3D0-01 | 32 | 2 | THCA | TCGA-EM-A2OX-01 | 9 | 4 |
| THCA | TCGA-DJ-A2PR-01 | 10 | 1 | THCA | TCGA-EL-A3D1-01 | 5 | 2 | THCA | TCGA-EM-A2OY-01 | 16 | 0 |
| THCA | TCGA-DJ-A2PS-01 | 5 | 1 | THCA | TCGA-EL-A3D4-01 | 7 | 0 | THCA | TCGA-EM-A2OZ-01 | 31 | 3 |
| THCA | TCGA-DJ-A2PT-01 | 17 | 2 | THCA | TCGA-EL-A3D5-01 | 14 | 2 | THCA | TCGA-EM-A2P0-01 | 7 | 1 |
| THCA | TCGA-DJ-A2PU-01 | 11 | 2 | THCA | TCGA-EL-A3D6-01 | 32 | 3 | THCA | TCGA-EM-A2P1-01 | 7 | 1 |
| THCA | TCGA-DJ-A2PV-01 | 11 | 2 | THCA | TCGA-EL-A3GO-01 | 14 | 2 | THCA | TCGA-EM-A2P1-06 | 8 | 1 |
| THCA | TCGA-DJ-A2PW-01 | 20 | 3 | THCA | TCGA-EL-A3GP-01 | 24 | 2 | THCA | TCGA-EM-A2P2-01 | 26 | 2 |
| THCA | TCGA-DJ-A2PX-01 | 5 | 1 | THCA | TCGA-EL-A3GQ-01 | 18 | 2 | THCA | TCGA-EM-A2P3-01 | 16 | 3 |
| THCA | TCGA-DJ-A2PY-01 | 15 | 1 | THCA | TCGA-EL-A3GR-01 | 14 | 2 | THCA | TCGA-EM-A3AI-01 | 20 | 0 |
| THCA | TCGA-DJ-A2PZ-01 | 20 | 1 | THCA | TCGA-EL-A3GS-01 | 18 | 2 | THCA | TCGA-EM-A3AJ-01 | 9 | 0 |
| THCA | TCGA-DJ-A2Q0-01 | 34 | 0 | THCA | TCGA-EL-A3GU-01 | 34 | 2 | THCA | TCGA-EM-A3AK-01 | 17 | 1 |
| THCA | TCGA-DJ-A2Q1-01 | 8 | 1 | THCA | TCGA-EL-A3GV-01 | 27 | 3 | THCA | TCGA-EM-A3AL-01 | 30 | 1 |
| THCA | TCGA-DJ-A2Q2-01 | 13 | 1 | THCA | TCGA-EL-A3GW-01 | 16 | 2 | THCA | TCGA-EM-A3AN-01 | 15 | 0 |
| THCA | TCGA-DJ-A2Q3-01 | 20 | 1 | THCA | TCGA-EL-A3GX-01 | 14 | 2 | THCA | TCGA-EM-A3AO-01 | 11 | 0 |
| THCA | TCGA-DJ-A2Q4-01 | 16 | 2 | THCA | TCGA-EL-A3GY-01 | 28 | 3 | THCA | TCGA-EM-A3AP-01 | 25 | 1 |
| THCA | TCGA-DJ-A2Q5-01 | 8 | 1 | THCA | TCGA-EL-A3GZ-01 | 13 | 2 | THCA | TCGA-EM-A3AQ-01 | 26 | 1 |
| THCA | TCGA-DJ-A2Q6-01 | 15 | 1 | THCA | TCGA-EL-A3H1-01 | 30 | 1 | THCA | TCGA-EM-A3AR-01 | 10 | 1 |
| THCA | TCGA-DJ-A2Q7-01 | 18 | 1 | THCA | TCGA-EL-A3H2-01 | 27 | 1 | THCA | TCGA-EM-A3FJ-01 | 3 | 1 |
| THCA | TCGA-DJ-A2Q8-01 | 14 | 2 | THCA | TCGA-EL-A3H3-01 | 4 | 1 | THCA | TCGA-EM-A3FK-01 | 11 | 1 |
| THCA | TCGA-DJ-A2Q9-01 | 22 | 2 | THCA | TCGA-EL-A3H4-01 | 16 | 1 | THCA | TCGA-EM-A3FL-01 | 26 | 1 |
| THCA | TCGA-DJ-A2QA-01 | 19 | 2 | THCA | TCGA-EL-A3H5-01 | 27 | 2 | THCA | TCGA-EM-A3FM-01 | 23 | 1 |
| THCA | TCGA-DJ-A2QB-01 | 9 | 2 | THCA | TCGA-EL-A3H7-01 | 32 | 1 | THCA | TCGA-EM-A3FN-01 | 24 | 1 |
| THCA | TCGA-DJ-A2QC-01 | 21 | 2 | THCA | TCGA-EL-A3H8-01 | 20 | 2 | THCA | TCGA-EM-A3FO-01 | 17 | 1 |
| THCA | TCGA-DJ-A3UK-01 | 23 | 1 | THCA | TCGA-EL-A3MW-01 | 15 | 1 | THCA | TCGA-EM-A3FP-01 | 19 | 0 |
| THCA | TCGA-DJ-A3UM-01 | 6 | 1 | THCA | TCGA-EL-A3MX-01 | 40 | 3 | THCA | TCGA-EM-A3FQ-01 | 10 | 0 |
| THCA | TCGA-DJ-A3UN-01 | 39 | 2 | THCA | TCGA-EL-A3MY-01 | 18 | 1 | THCA | TCGA-EM-A3FQ-06 | 7 | 0 |
| THCA | TCGA-DJ-A3UO-01 | 20 | 1 | THCA | TCGA-EL-A3MZ-01 | 38 | 4 | THCA | TCGA-EM-A3FR-01 | 25 | 2 |
| THCA | TCGA-DJ-A3UP-01 | 30 | 1 | THCA | TCGA-EL-A3N2-01 | 19 | 3 | THCA | TCGA-EM-A3O3-01 | 39 | 1 |
| THCA | TCGA-DJ-A3UQ-01 | 22 | 1 | THCA | TCGA-EL-A3N3-01 | 19 | 1 | THCA | TCGA-EM-A3O6-01 | 21 | 4 |
| THCA | TCGA-DJ-A3UR-01 | 19 | 2 | THCA | TCGA-EL-A3T0-01 | 30 | 0 | THCA | TCGA-EM-A3O7-01 | 24 | 1 |
| THCA | TCGA-DJ-A3US-01 | 20 | 0 | THCA | TCGA-EL-A3T1-01 | 19 | 3 | THCA | TCGA-EM-A3O8-01 | 13 | 1 |
| THCA | TCGA-DJ-A3UT-01 | 21 | 2 | THCA | TCGA-EL-A3T2-01 | 30 | 2 | THCA | TCGA-EM-A3O9-01 | 27 | 1 |
| THCA | TCGA-DJ-A3UU-01 | 17 | 2 | THCA | TCGA-EL-A3T3-01 | 41 | 3 | THCA | TCGA-EM-A3OA-01 | 26 | 1 |
| THCA | TCGA-DJ-A3UW-01 | 13 | 1 | THCA | TCGA-EL-A3T6-01 | 11 | 1 | THCA | TCGA-EM-A3OB-01 | 23 | 1 |
| THCA | TCGA-DJ-A3UX-01 | 18 | 1 | THCA | TCGA-EL-A3T7-01 | 23 | 2 | THCA | TCGA-EM-A4FK-01 | 8 | 1 |
| THCA | TCGA-DJ-A3UY-01 | 5 | 1 | THCA | TCGA-EL-A3T8-01 | 14 | 1 | THCA | TCGA-EM-A4FM-01 | 22 | 1 |
| THCA | TCGA-DJ-A3V7-01 | 18 | 2 | THCA | TCGA-EL-A3T9-01 | 36 | 3 | THCA | TCGA-EM-A4FO-01 | 17 | 2 |
| THCA | TCGA-DJ-A3VA-01 | 14 | 2 | THCA | TCGA-EL-A3TA-01 | 9 | 1 | THCA | TCGA-EM-A4FQ-01 | 8 | 3 |
| THCA | TCGA-DJ-A3VB-01 | 12 | 1 | THCA | TCGA-EL-A3TB-01 | 26 | 1 | THCA | TCGA-EM-A4FR-01 | 5 | 0 |
| THCA | TCGA-DJ-A3VE-01 | 13 | 1 | THCA | TCGA-EL-A3ZH-01 | 10 | 1 | THCA | TCGA-EM-A4FV-01 | 28 | 2 |
| THCA | TCGA-DJ-A3VF-01 | 9 | 1 | THCA | TCGA-EL-A3ZK-01 | 12 | 0 | THCA | TCGA-EM-A4G1-01 | 4 | 1 |
| THCA | TCGA-DJ-A3VJ-01 | 7 | 2 | THCA | TCGA-EL-A3ZN-01 | 4 | 0 | THCA | TCGA-ET-A25G-01 | 13 | 1 |
| THCA | TCGA-DJ-A3VK-01 | 19 | 1 | THCA | TCGA-EL-A3ZQ-01 | 10 | 1 | THCA | TCGA-ET-A25I-01 | 20 | 1 |
| THCA | TCGA-DJ-A3VL-01 | 6 | 1 | THCA | TCGA-EL-A3ZR-01 | 19 | 1 | THCA | TCGA-ET-A25J-01 | 11 | 1 |
| THCA | TCGA-DJ-A3VM-01 | 17 | 0 | THCA | TCGA-EL-A3ZT-01 | 10 | 1 | THCA | TCGA-ET-A25K-01 | 11 | 2 |
| THCA | TCGA-DJ-A4UL-01 | 9 | 1 | THCA | TCGA-EL-A4JV-01 | 7 | 1 | THCA | TCGA-ET-A25O-01 | 13 | 2 |
| THCA | TCGA-DJ-A4UP-01 | 6 | 0 | THCA | TCGA-EL-A4JW-01 | 10 | 1 | THCA | TCGA-ET-A25R-01 | 14 | 2 |
| THCA | TCGA-DJ-A4UT-01 | 40 | 0 | THCA | TCGA-EL-A4JX-01 | 10 | 1 | THCA | TCGA-ET-A2MY-01 | 74 | 5 |
| THCA | TCGA-DJ-A4UW-01 | 10 | 3 | THCA | TCGA-EL-A4JZ-01 | 13 | 2 | THCA | TCGA-ET-A2MZ-01 | 5 | 2 |

# TABLE 5 (APPENDIX A)

| | | | |
|---|---|---|---|
| THCA | TCGA-ET-A2N0-01 | 14 | 1 |
| THCA | TCGA-ET-A2N1-01 | 13 | 0 |
| THCA | TCGA-ET-A2N4-01 | 28 | 1 |
| THCA | TCGA-ET-A2N5-01 | 16 | 4 |
| THCA | TCGA-ET-A39I-01 | 12 | 1 |
| THCA | TCGA-ET-A39J-01 | 8 | 2 |
| THCA | TCGA-ET-A39K-01 | 8 | 1 |
| THCA | TCGA-ET-A39L-01 | 4 | 1 |
| THCA | TCGA-ET-A39M-01 | 28 | 1 |
| THCA | TCGA-ET-A39N-01 | 12 | 2 |
| THCA | TCGA-ET-A39O-01 | 16 | 1 |
| THCA | TCGA-ET-A39P-01 | 19 | 2 |
| THCA | TCGA-ET-A39R-01 | 6 | 0 |
| THCA | TCGA-ET-A39S-01 | 6 | 2 |
| THCA | TCGA-ET-A39T-01 | 17 | 2 |
| THCA | TCGA-ET-A3BN-01 | 9 | 1 |
| THCA | TCGA-ET-A3BO-01 | 7 | 1 |
| THCA | TCGA-ET-A3BP-01 | 5 | 1 |
| THCA | TCGA-ET-A3BQ-01 | 17 | 1 |
| THCA | TCGA-ET-A3BS-01 | 11 | 1 |
| THCA | TCGA-ET-A3BT-01 | 24 | 2 |
| THCA | TCGA-ET-A3BU-01 | 8 | 1 |
| THCA | TCGA-ET-A3BV-01 | 30 | 2 |
| THCA | TCGA-ET-A3BW-01 | 7 | 2 |
| THCA | TCGA-ET-A3BX-01 | 11 | 1 |
| THCA | TCGA-ET-A3DO-01 | 20 | 1 |
| THCA | TCGA-ET-A3DP-01 | 17 | 1 |
| THCA | TCGA-ET-A3DQ-01 | 5 | 0 |
| THCA | TCGA-ET-A3DR-01 | 16 | 0 |
| THCA | TCGA-ET-A3DS-01 | 11 | 1 |
| THCA | TCGA-ET-A3DT-01 | 4 | 2 |
| THCA | TCGA-ET-A3DU-01 | 18 | 5 |
| THCA | TCGA-ET-A3DV-01 | 20 | 0 |
| THCA | TCGA-ET-A3DW-01 | 19 | 2 |
| THCA | TCGA-ET-A40S-01 | 11 | 0 |
| THCA | TCGA-ET-A4KN-01 | 11 | 1 |
| THCA | TCGA-FE-A22Z-01 | 26 | 1 |
| THCA | TCGA-FE-A230-01 | 8 | 1 |
| THCA | TCGA-FE-A231-01 | 17 | 1 |
| THCA | TCGA-FE-A232-01 | 12 | 2 |
| THCA | TCGA-FE-A233-01 | 11 | 1 |
| THCA | TCGA-FE-A234-01 | 15 | 2 |
| THCA | TCGA-FE-A235-01 | 17 | 1 |
| THCA | TCGA-FE-A236-01 | 11 | 1 |
| THCA | TCGA-FE-A237-01 | 9 | 1 |
| THCA | TCGA-FE-A23A-01 | 11 | 1 |
| THCA | TCGA-FE-A3PB-01 | 6 | 1 |
| THCA | TCGA-FE-A3PC-01 | 41 | 2 |
| THCA | TCGA-FE-A3PD-01 | 22 | 0 |
| THCA | TCGA-FK-A3S3-01 | 18 | 1 |
| THCA | TCGA-FK-A3SB-01 | 15 | 1 |
| THCA | TCGA-FK-A3SD-01 | 37 | 1 |
| THCA | TCGA-FK-A3SE-01 | 14 | 1 |
| THCA | TCGA-FK-A3SG-01 | 16 | 0 |
| THCA | TCGA-FK-A3SH-01 | 32 | 4 |
| THCA | TCGA-FY-A2QD-01 | 6 | 0 |
| THCA | TCGA-FY-A3BL-01 | 6 | 1 |
| THCA | TCGA-FY-A3I4-01 | 9 | 1 |
| THCA | TCGA-FY-A3I5-01 | 24 | 1 |
| THCA | TCGA-FY-A3NM-01 | 26 | 1 |
| THCA | TCGA-FY-A3NN-01 | 20 | 2 |
| THCA | TCGA-FY-A3NP-01 | 13 | 1 |
| THCA | TCGA-FY-A3ON-01 | 7 | 2 |
| THCA | TCGA-FY-A3R6-01 | 20 | 0 |
| THCA | TCGA-FY-A3R7-01 | 18 | 1 |
| THCA | TCGA-FY-A3R8-01 | 20 | 1 |
| THCA | TCGA-FY-A3R9-01 | 29 | 2 |
| THCA | TCGA-FY-A3RA-01 | 5 | 1 |
| THCA | TCGA-FY-A3W9-01 | 29 | 1 |
| THCA | TCGA-FY-A3WA-01 | 8 | 0 |
| THCA | TCGA-FY-A40K-01 | 6 | 1 |
| THCA | TCGA-FY-A483-01 | 13 | 1 |
| THCA | TCGA-GE-A2C6-01 | 11 | 4 |
| THCA | TCGA-H2-A26U-01 | 8 | 1 |
| THCA | TCGA-H2-A2K9-01 | 26 | 2 |
| THCA | TCGA-H2-A3RH-01 | 15 | 1 |
| THCA | TCGA-H2-A3RI-01 | 26 | 2 |
| THCA | TCGA-H2-A421-01 | 24 | 2 |
| THCA | TCGA-IM-A3EB-01 | 19 | 1 |
| THCA | TCGA-IM-A3ED-01 | 16 | 3 |
| THCA | TCGA-IM-A3U2-01 | 67 | 5 |
| THCA | TCGA-IM-A3U3-01 | 32 | 1 |
| THCA | TCGA-J8-A3NZ-01 | 22 | 1 |
| THCA | TCGA-J8-A3OO-01 | 13 | 2 |
| THCA | TCGA-J8-A3O1-01 | 17 | 0 |
| THCA | TCGA-J8-A3YE-01 | 7 | 3 |
| THCA | TCGA-J8-A3YH-06 | 4 | 1 |
| THCA | TCGA-J8-A4HW-01 | 16 | 1 |
| THCA | TCGA-J8-A4HW-06 | 9 | 0 |
| THCA | TCGA-KS-A41J-01 | 7 | 1 |
| THCA | TCGA-KS-A4I5-01 | 14 | 3 |
| THCA | TCGA-KS-A4I9-01 | 7 | 1 |
| THCA | TCGA-KS-A4I8-01 | 17 | 3 |
| THCA | TCGA-L6-A4EP-01 | 13 | 2 |
| THCA | TCGA-L6-A4ET-01 | 7 | 1 |
| THCA | TCGA-L6-A4EU-01 | 47 | 4 |
| THCA | TCGA-MK-A4N6-01 | 11 | 1 |
| THCA | TCGA-MK-A4N7-01 | 20 | 3 |
| THCA | TCGA-MK-A4N9-01 | 6 | 1 |
| UCEC | TCGA-A5-A0G3-01 | 42 | 3 |
| UCEC | TCGA-A5-A0G5-01 | 136 | 6 |
| UCEC | TCGA-A5-A0G9-01 | 266 | 10 |
| UCEC | TCGA-A5-A0GA-01 | 310 | 20 |
| UCEC | TCGA-A5-A0GB-01 | 727 | 32 |
| UCEC | TCGA-A5-A0GD-01 | 5 | 0 |
| UCEC | TCGA-A5-A0GE-01 | 34 | 4 |
| UCEC | TCGA-A5-A0GH-01 | 455 | 20 |
| UCEC | TCGA-A5-A0GI-01 | 268 | 11 |
| UCEC | TCGA-A5-A0GJ-01 | 91 | 3 |
| UCEC | TCGA-A5-A0GM-01 | 42 | 9 |
| UCEC | TCGA-A5-A0GN-01 | 60 | 6 |
| UCEC | TCGA-A5-A0GP-01 | 1676 | 64 |
| UCEC | TCGA-A5-A0GQ-01 | 72 | 9 |
| UCEC | TCGA-A5-A0GU-01 | 53 | 6 |
| UCEC | TCGA-A5-A0GV-01 | 57 | 6 |
| UCEC | TCGA-A5-A0GW-01 | 322 | 18 |
| UCEC | TCGA-A5-A0GX-01 | 43 | 6 |
| UCEC | TCGA-A5-A0R6-01 | 42 | 7 |
| UCEC | TCGA-A5-A0R7-01 | 56 | 6 |
| UCEC | TCGA-A5-A0R8-01 | 92 | 7 |
| UCEC | TCGA-A5-A0R9-01 | 50 | 6 |
| UCEC | TCGA-A5-A0RA-01 | 87 | 13 |
| UCEC | TCGA-A5-A0VO-01 | 81 | 9 |
| UCEC | TCGA-A5-A0VP-01 | 1645 | 70 |
| UCEC | TCGA-A5-A0VQ-01 | 411 | 17 |
| UCEC | TCGA-AJ-A23M-01 | 124 | 6 |
| UCEC | TCGA-AP-A051-01 | 9306 | 322 |
| UCEC | TCGA-AP-A052-01 | 32 | 3 |
| UCEC | TCGA-AP-A053-01 | 55 | 4 |
| UCEC | TCGA-AP-A054-01 | 1160 | 51 |
| UCEC | TCGA-AP-A056-01 | 9618 | 325 |
| UCEC | TCGA-AP-A05A-01 | 84 | 7 |
| UCEC | TCGA-AP-A05D-01 | 40 | 5 |
| UCEC | TCGA-AP-A05H-01 | 80 | 6 |
| UCEC | TCGA-AP-A05J-01 | 54 | 5 |
| UCEC | TCGA-AP-A05N-01 | 227 | 15 |
| UCEC | TCGA-AP-A05P-01 | 41 | 3 |
| UCEC | TCGA-AP-A0L8-01 | 69 | 5 |
| UCEC | TCGA-AP-A0L9-01 | 70 | 3 |
| UCEC | TCGA-AP-A0LD-01 | 505 | 22 |
| UCEC | TCGA-AP-A0LE-01 | 531 | 23 |
| UCEC | TCGA-AP-A0LF-01 | 228 | 12 |
| UCEC | TCGA-AP-A0LG-01 | 420 | 15 |
| UCEC | TCGA-AP-A0LH-01 | 131 | 5 |
| UCEC | TCGA-AP-A0LI-01 | 96 | 4 |
| UCEC | TCGA-AP-A0LJ-01 | 49 | 5 |
| UCEC | TCGA-AP-A0LL-01 | 53 | 5 |
| UCEC | TCGA-AP-A0LM-01 | 14698 | 473 |
| UCEC | TCGA-AP-A0LN-01 | 58 | 6 |
| UCEC | TCGA-AP-A0LO-01 | 38 | 3 |
| UCEC | TCGA-AP-A0LP-01 | 286 | 15 |
| UCEC | TCGA-AP-A0LQ-01 | 57 | 5 |
| UCEC | TCGA-AP-A0LT-01 | 684 | 35 |
| UCEC | TCGA-AP-A1DQ-01 | 34 | 6 |
| UCEC | TCGA-AX-A05S-01 | 533 | 27 |
| UCEC | TCGA-AX-A05T-01 | 62 | 7 |
| UCEC | TCGA-AX-A05U-01 | 42 | 6 |
| UCEC | TCGA-AX-A05W-01 | 65 | 9 |
| UCEC | TCGA-AX-A05Y-01 | 185 | 17 |
| UCEC | TCGA-AX-A05Z-01 | 7274 | 261 |
| UCEC | TCGA-AX-A060-01 | 707 | 34 |
| UCEC | TCGA-AX-A062-01 | 36 | 5 |
| UCEC | TCGA-AX-A063-01 | 1006 | 48 |
| UCEC | TCGA-AX-A064-01 | 267 | 11 |
| UCEC | TCGA-AX-A06B-01 | 47 | 4 |
| UCEC | TCGA-AX-A06H-01 | 419 | 28 |
| UCEC | TCGA-AX-A06L-01 | 43 | 7 |
| UCEC | TCGA-AX-A06S-01 | 71 | 6 |
| UCEC | TCGA-AX-A0IU-01 | 57 | 4 |
| UCEC | TCGA-AX-A0IW-01 | 55 | 1 |
| UCEC | TCGA-AX-A0J0-01 | 8884 | 294 |
| UCEC | TCGA-AX-A0J1-01 | 5626 | 202 |
| UCEC | TCGA-AX-A1C7-01 | 54 | 4 |
| UCEC | TCGA-AX-A1C8-01 | 66 | 6 |
| UCEC | TCGA-AX-A1CP-01 | 31 | 2 |
| UCEC | TCGA-AX-A2H5-01 | 57 | 1 |
| UCEC | TCGA-AX-A2HF-01 | 42 | 5 |
| UCEC | TCGA-B5-A0JN-01 | 124 | 3 |
| UCEC | TCGA-B5-A0JR-01 | 631 | 24 |
| UCEC | TCGA-B5-A0JS-01 | 38 | 7 |
| UCEC | TCGA-B5-A0JT-01 | 66 | 8 |
| UCEC | TCGA-B5-A0JV-01 | 293 | 15 |
| UCEC | TCGA-B5-A0JY-01 | 11726 | 39: |
| UCEC | TCGA-B5-A0JZ-01 | 496 | 22 |
| UCEC | TCGA-B5-A0K0-01 | 25 | 4 |
| UCEC | TCGA-B5-A0K1-01 | 60 | 12 |
| UCEC | TCGA-B5-A0K2-01 | 276 | 14 |
| UCEC | TCGA-B5-A0K3-01 | 52 | 8 |
| UCEC | TCGA-B5-A0K4-01 | 64 | 3 |
| UCEC | TCGA-B5-A0K6-01 | 246 | 15 |
| UCEC | TCGA-B5-A0K7-01 | 57 | 8 |
| UCEC | TCGA-B5-A0K8-01 | 57 | 1 |
| UCEC | TCGA-B5-A0K9-01 | 751 | 19 |
| UCEC | TCGA-B5-A11E-01 | 11470 | 40( |
| UCEC | TCGA-B5-A11F-01 | 52 | 8 |
| UCEC | TCGA-B5-A11G-01 | 553 | 33 |
| UCEC | TCGA-B5-A11H-01 | 1049 | 36 |
| UCEC | TCGA-B5-A11I-01 | 51 | 4 |
| UCEC | TCGA-B5-A11J-01 | 453 | 27 |
| UCEC | TCGA-B5-A11M-01 | 37 | 5 |
| UCEC | TCGA-B5-A11N-01 | 2049 | 79 |
| UCEC | TCGA-B5-A11O-01 | 72 | 4 |
| UCEC | TCGA-B5-A11Q-01 | 53 | 5 |
| UCEC | TCGA-B5-A11R-01 | 746 | 45 |
| UCEC | TCGA-B5-A11S-01 | 55 | 6 |
| UCEC | TCGA-B5-A11U-01 | 351 | 17 |
| UCEC | TCGA-B5-A11V-01 | 66 | 10 |
| UCEC | TCGA-B5-A11W-01 | 80 | 9 |
| UCEC | TCGA-B5-A11X-01 | 68 | 10 |
| UCEC | TCGA-B5-A11Y-01 | 1196 | 57 |
| UCEC | TCGA-B5-A11Z-01 | 67 | 5 |
| UCEC | TCGA-B5-A121-01 | 37 | 5 |
| UCEC | TCGA-B5-A1MU-01 | 29 | 4 |
| UCEC | TCGA-B5-A1MY-01 | 52 | 3 |
| UCEC | TCGA-BG-A0LW-01 | 37 | 2 |
| UCEC | TCGA-BG-A0LX-01 | 489 | 22 |
| UCEC | TCGA-BG-A0M0-01 | 153 | 7 |
| UCEC | TCGA-BG-A0M2-01 | 38 | 2 |
| UCEC | TCGA-BG-A0M3-01 | 82 | 10 |
| UCEC | TCGA-BG-A0M4-01 | 477 | 24 |
| UCEC | TCGA-BG-A0M6-01 | 49 | 7 |
| UCEC | TCGA-BG-A0M7-01 | 65 | 5 |
| UCEC | TCGA-BG-A0M8-01 | 70 | 7 |
| UCEC | TCGA-BG-A0M9-01 | 64 | 9 |
| UCEC | TCGA-BG-A0MC-01 | 63 | 5 |
| UCEC | TCGA-BG-A0MG-01 | 122 | 12 |
| UCEC | TCGA-BG-A0MI-01 | 70 | 5 |
| UCEC | TCGA-BG-A0MO-01 | 50 | 4 |
| UCEC | TCGA-BG-A0MQ-01 | 619 | 23 |
| UCEC | TCGA-BG-A0MS-01 | 53 | 7 |
| UCEC | TCGA-BG-A0MT-01 | 58 | 7 |
| UCEC | TCGA-BG-A0MU-01 | 78 | 7 |
| UCEC | TCGA-BG-A0RY-01 | 63 | 6 |
| UCEC | TCGA-BG-A0VT-01 | 38 | 3 |
| UCEC | TCGA-BG-A0VV-01 | 45 | 4 |
| UCEC | TCGA-BG-A0VW-01 | 330 | 21 |
| UCEC | TCGA-BG-A0VX-01 | 229 | 11 |
| UCEC | TCGA-BG-A0VZ-01 | 344 | 15 |
| UCEC | TCGA-BG-A0W1-01 | 84 | 6 |
| UCEC | TCGA-BG-A0W2-01 | 50 | 3 |
| UCEC | TCGA-BG-A0YU-01 | 25 | 2 |
| UCEC | TCGA-BG-A0YV-01 | 89 | 12 |
| UCEC | TCGA-BG-A186-01 | 38 | 5 |
| UCEC | TCGA-BG-A187-01 | 112 | 12 |
| UCEC | TCGA-BG-A18A-01 | 77 | 10 |
| UCEC | TCGA-BG-A18B-01 | 363 | 14 |
| UCEC | TCGA-BG-A18C-01 | 52 | 4 |
| UCEC | TCGA-BG-A2AE-01 | 71 | 8 |
| UCEC | TCGA-BK-A0C9-01 | 472 | 22 |
| UCEC | TCGA-BK-A0CA-01 | 54 | 5 |
| UCEC | TCGA-BK-A0CB-01 | 80 | 9 |
| UCEC | TCGA-BK-A0CC-01 | 48 | 5 |
| UCEC | TCGA-BK-A139-01 | 105 | 7 |
| UCEC | TCGA-BK-A13C-01 | 53 | 4 |
| UCEC | TCGA-BS-A0T9-01 | 65 | 8 |
| UCEC | TCGA-BS-A0TA-01 | 417 | 23 |
| UCEC | TCGA-BS-A0TC-01 | 1525 | 59 |
| UCEC | TCGA-BS-A0TD-01 | 41 | 5 |
| UCEC | TCGA-BS-A0TE-01 | 359 | 9 |
| UCEC | TCGA-BS-A0TG-01 | 48 | 6 |
| UCEC | TCGA-BS-A0TI-01 | 95 | 8 |
| UCEC | TCGA-BS-A0TJ-01 | 663 | 24 |

# TABLE 5 (APPENDIX A)

| | | | | | | | | | | | | |
|---|---|---|---|---|---|---|---|---|---|---|---|---|
| UCEC | TCGA-BS-A0U5-01 | 75 | 6 | | UCS | TCGA-N5-A4RM-01 | 110 | 5 | | UVM | TCGA-VD-A8K7-01 | 10 | 1 |
| UCEC | TCGA-BS-A0U7-01 | 172 | 7 | | UCS | TCGA-N5-A4RN-01 | 121 | 9 | | UVM | TCGA-VD-A8K8-01 | 23 | 1 |
| UCEC | TCGA-BS-A0U8-01 | 416 | 26 | | UCS | TCGA-N5-A4RO-01 | 87 | 4 | | UVM | TCGA-VD-A8K9-01 | 16 | 3 |
| UCEC | TCGA-BS-A0U9-01 | 44 | 8 | | UCS | TCGA-N5-A4RS-01 | 82 | 6 | | UVM | TCGA-VD-A8KA-01 | 21 | 2 |
| UCEC | TCGA-BS-A0UA-01 | 338 | 16 | | UCS | TCGA-N5-A4RT-01 | 144 | 7 | | UVM | TCGA-VD-A8KB-01 | 28 | 4 |
| UCEC | TCGA-BS-A0UF-01 | 9491 | 306 | | UCS | TCGA-N5-A4RU-01 | 110 | 5 | | UVM | TCGA-VD-A8KD-01 | 25 | 0 |
| UCEC | TCGA-BS-A0UJ-01 | 2032 | 77 | | UCS | TCGA-N5-A4RV-01 | 89 | 7 | | UVM | TCGA-VD-A8KE-01 | 29 | 2 |
| UCEC | TCGA-BS-A0UL-01 | 405 | 17 | | UCS | TCGA-N5-A59E-01 | 103 | 9 | | UVM | TCGA-VD-A8KF-01 | 12 | 1 |
| UCEC | TCGA-BS-A0UM-01 | 382 | 17 | | UCS | TCGA-N5-A59F-01 | 104 | 11 | | UVM | TCGA-VD-A8KG-01 | 23 | 1 |
| UCEC | TCGA-BS-A0UT-01 | 49 | 4 | | UCS | TCGA-N6-A4V9-01 | 58 | 6 | | UVM | TCGA-VD-A8KH-01 | 26 | 2 |
| UCEC | TCGA-BS-A0UV-01 | 10353 | 336 | | UCS | TCGA-N6-A4VC-01 | 68 | 8 | | UVM | TCGA-VD-A8KI-01 | 30 | 8 |
| UCEC | TCGA-BS-A0V6-01 | 59 | 7 | | UCS | TCGA-N6-A4VD-01 | 122 | 12 | | UVM | TCGA-VD-A8KJ-01 | 21 | 1 |
| UCEC | TCGA-BS-A0V7-01 | 43 | 7 | | UCS | TCGA-N6-A4VE-01 | 100 | 7 | | UVM | TCGA-VD-A8KK-01 | 24 | 1 |
| UCEC | TCGA-BS-A0V8-01 | 91 | 10 | | UCS | TCGA-N6-A4VF-01 | 73 | 7 | | UVM | TCGA-VD-A8KL-01 | 28 | 2 |
| UCEC | TCGA-BS-A0WQ-01 | 60 | 4 | | UCS | TCGA-N6-A4VG-01 | 74 | 6 | | UVM | TCGA-VD-A8KM-01 | 26 | 2 |
| UCEC | TCGA-D1-A0ZN-01 | 52 | 10 | | UCS | TCGA-N7-A4Y0-01 | 981 | 51 | | UVM | TCGA-VD-A8KN-01 | 18 | 1 |
| UCEC | TCGA-D1-A0ZO-01 | 587 | 31 | | UCS | TCGA-N7-A4Y5-01 | 85 | 5 | | UVM | TCGA-VD-A8KO-01 | 16 | 1 |
| UCEC | TCGA-D1-A0ZP-01 | 41 | 5 | | UCS | TCGA-N7-A4Y8-01 | 116 | 6 | | UVM | TCGA-VD-AA8M-01 | 29 | 2 |
| UCEC | TCGA-D1-A0ZQ-01 | 98 | 11 | | UCS | TCGA-N7-A59B-01 | 78 | 3 | | UVM | TCGA-VD-AA8N-01 | 13 | 3 |
| UCEC | TCGA-D1-A0ZR-01 | 62 | 6 | | UCS | TCGA-N8-A4PI-01 | 80 | 10 | | UVM | TCGA-VD-AA8O-01 | 18 | 0 |
| UCEC | TCGA-D1-A0ZS-01 | 417 | 17 | | UCS | TCGA-N8-A4PL-01 | 86 | 6 | | UVM | TCGA-VD-AA8P-01 | 18 | 2 |
| UCEC | TCGA-D1-A0ZU-01 | 43 | 7 | | UCS | TCGA-N8-A4PM-01 | 90 | 7 | | UVM | TCGA-VD-AA8Q-01 | 27 | 2 |
| UCEC | TCGA-D1-A0ZV-01 | 44 | 5 | | UCS | TCGA-N8-A4PN-01 | 70 | 6 | | UVM | TCGA-VD-AA8R-01 | 16 | 1 |
| UCEC | TCGA-D1-A0ZZ-01 | 78 | 3 | | UCS | TCGA-N8-A4PO-01 | 125 | 3 | | UVM | TCGA-VD-AA8S-01 | 13 | 1 |
| UCEC | TCGA-D1-A101-01 | 289 | 9 | | UCS | TCGA-N8-A4PP-01 | 185 | 6 | | UVM | TCGA-VD-AA8T-01 | 21 | 2 |
| UCEC | TCGA-D1-A102-01 | 59 | 8 | | UCS | TCGA-N8-A4PQ-01 | 116 | 2 | | UVM | TCGA-WC-A87T-01 | 17 | 1 |
| UCEC | TCGA-D1-A103-01 | 8397 | 279 | | UCS | TCGA-N8-A56S-01 | 97 | 4 | | UVM | TCGA-WC-A87U-01 | 20 | 1 |
| UCEC | TCGA-D1-A15V-01 | 46 | 6 | | UCS | TCGA-N9-A4PZ-01 | 113 | 4 | | UVM | TCGA-WC-A87W-01 | 16 | 1 |
| UCEC | TCGA-D1-A15W-01 | 81 | 8 | | UCS | TCGA-N9-A4Q1-01 | 84 | 1 | | UVM | TCGA-WC-A87Y-01 | 18 | 1 |
| UCEC | TCGA-D1-A15X-01 | 1916 | 78 | | UCS | TCGA-N9-A4Q3-01 | 61 | 5 | | UVM | TCGA-WC-A880-01 | 14 | 1 |
| UCEC | TCGA-D1-A15Z-01 | 89 | 11 | | UCS | TCGA-N9-A4Q4-01 | 69 | 2 | | UVM | TCGA-WC-A881-01 | 17 | 3 |
| UCEC | TCGA-D1-A160-01 | 497 | 21 | | UCS | TCGA-N9-A4Q7-01 | 416 | 20 | | UVM | TCGA-WC-A882-01 | 18 | 3 |
| UCEC | TCGA-D1-A161-01 | 102 | 9 | | UCS | TCGA-N9-A4Q8-01 | 65 | 4 | | UVM | TCGA-WC-A883-01 | 17 | 2 |
| UCEC | TCGA-D1-A163-01 | 555 | 22 | | UCS | TCGA-NA-A4QV-01 | 76 | 1 | | UVM | TCGA-WC-A884-01 | 19 | 2 |
| UCEC | TCGA-D1-A16S-01 | 67 | 7 | | UCS | TCGA-NA-A4QW-01 | 77 | 3 | | UVM | TCGA-WC-A885-01 | 31 | 3 |
| UCEC | TCGA-D1-A167-01 | 1997 | 76 | | UCS | TCGA-NA-A4QX-01 | 48 | 6 | | UVM | TCGA-WC-A888-01 | 21 | 3 |
| UCEC | TCGA-D1-A168-01 | 66 | 7 | | UCS | TCGA-NA-A4QY-01 | 76 | 5 | | UVM | TCGA-WC-A88A-01 | 23 | 1 |
| UCEC | TCGA-D1-A169-01 | 55 | 9 | | UCS | TCGA-NA-A4R0-01 | 83 | 4 | | UVM | TCGA-WC-AA9A-01 | 15 | 4 |
| UCEC | TCGA-D1-A16B-01 | 57 | 6 | | UCS | TCGA-NA-A4R1-01 | 183 | 13 | | UVM | TCGA-WC-AA9E-01 | 28 | 3 |
| UCEC | TCGA-D1-A16D-01 | 49 | 8 | | UCS | TCGA-NA-A5I1-01 | 69 | 6 | | UVM | TCGA-YZ-A980-01 | 18 | 3 |
| UCEC | TCGA-D1-A16E-01 | 70 | 8 | | UCS | TCGA-ND-A4W6-01 | 126 | 10 | | UVM | TCGA-YZ-A982-01 | 29 | 2 |
| UCEC | TCGA-D1-A16F-01 | 418 | 19 | | UCS | TCGA-ND-A4WA-01 | 84 | 0 | | UVM | TCGA-YZ-A983-01 | 25 | 4 |
| UCEC | TCGA-D1-A16G-01 | 38 | 4 | | UCS | TCGA-ND-A4WC-01 | 4934 | 184 | | UVM | TCGA-YZ-A984-01 | 16 | 4 |
| UCEC | TCGA-D1-A16I-01 | 44 | 5 | | UCS | TCGA-ND-A4WF-01 | 91 | 4 | | UVM | TCGA-YZ-A985-01 | 581 | 24 |
| UCEC | TCGA-D1-A16J-01 | 239 | 6 | | UCS | TCGA-NF-A4WU-01 | 56 | 5 | | | | | |
| UCEC | TCGA-D1-A16N-01 | 130 | 9 | | UCS | TCGA-NF-A4WX-01 | 59 | 5 | | | | | |
| UCEC | TCGA-D1-A16O-01 | 42 | 6 | | UCS | TCGA-NF-A4X2-01 | 153 | 8 | | | | | |
| UCEC | TCGA-D1-A16Q-01 | 35 | 7 | | UCS | TCGA-NF-A5CP-01 | 95 | 11 | | | | | |
| UCEC | TCGA-D1-A16R-01 | 46 | 11 | | UCS | TCGA-NG-A4VU-01 | 58 | 4 | | | | | |
| UCEC | TCGA-D1-A16S-01 | 51 | 3 | | UCS | TCGA-NG-A4VW-01 | 72 | 4 | | | | | |
| UCEC | TCGA-D1-A16X-01 | 2312 | 69 | | UCS | TCGA-QM-A5NM-01 | 78 | 6 | | | | | |
| UCEC | TCGA-D1-A16Y-01 | 1264 | 42 | | UCS | TCGA-QN-A5NN-01 | 103 | 5 | | | | | |
| UCEC | TCGA-D1-A174-01 | 758 | 24 | | UVM | TCGA-RZ-AB0B-01 | 19 | 3 | | | | | |
| UCEC | TCGA-D1-A176-01 | 386 | 19 | | UVM | TCGA-V3-A9ZX-01 | 12 | 2 | | | | | |
| UCEC | TCGA-D1-A177-01 | 798 | 31 | | UVM | TCGA-V3-A9ZY-01 | 24 | 2 | | | | | |
| UCEC | TCGA-D1-A17A-01 | 248 | 15 | | UVM | TCGA-V4-A9E5-01 | 15 | 2 | | | | | |
| UCEC | TCGA-D1-A178-01 | 279 | 15 | | UVM | TCGA-V4-A9E7-01 | 19 | 3 | | | | | |
| UCEC | TCGA-D1-A17C-01 | 61 | 8 | | UVM | TCGA-V4-A9E8-01 | 20 | 2 | | | | | |
| UCEC | TCGA-D1-A17D-01 | 513 | 20 | | UVM | TCGA-V4-A9E9-01 | 20 | 2 | | | | | |
| UCEC | TCGA-D1-A17F-01 | 240 | 13 | | UVM | TCGA-V4-A9EA-01 | 16 | 2 | | | | | |
| UCEC | TCGA-D1-A17H-01 | 640 | 25 | | UVM | TCGA-V4-A9EC-01 | 28 | 4 | | | | | |
| UCEC | TCGA-D1-A17K-01 | 62 | 3 | | UVM | TCGA-V4-A9ED-01 | 19 | 1 | | | | | |
| UCEC | TCGA-D1-A17L-01 | 71 | 3 | | UVM | TCGA-V4-A9EE-01 | 25 | 2 | | | | | |
| UCEC | TCGA-D1-A17M-01 | 422 | 24 | | UVM | TCGA-V4-A9EF-01 | 12 | 2 | | | | | |
| UCEC | TCGA-D1-A17N-01 | 32 | 5 | | UVM | TCGA-V4-A9EH-01 | 16 | 2 | | | | | |
| UCEC | TCGA-D1-A17Q-01 | 6787 | 237 | | UVM | TCGA-V4-A9EI-01 | 31 | 4 | | | | | |
| UCEC | TCGA-D1-A17R-01 | 180 | 16 | | UVM | TCGA-V4-A9EJ-01 | 27 | 2 | | | | | |
| UCEC | TCGA-D1-A17S-01 | 48 | 6 | | UVM | TCGA-V4-A9EK-01 | 19 | 1 | | | | | |
| UCEC | TCGA-D1-A17T-01 | 71 | 10 | | UVM | TCGA-V4-A9EL-01 | 16 | 4 | | | | | |
| UCEC | TCGA-D1-A17U-01 | 365 | 17 | | UVM | TCGA-V4-A9EM-01 | 25 | 3 | | | | | |
| UCEC | TCGA-D1-A1NU-01 | 95 | 7 | | UVM | TCGA-V4-A9EO-01 | 24 | 4 | | | | | |
| UCEC | TCGA-D1-A1NX-01 | 55 | 4 | | UVM | TCGA-V4-A9EQ-01 | 18 | 1 | | | | | |
| UCEC | TCGA-DI-A0WH-01 | 531 | 26 | | UVM | TCGA-V4-A9ES-01 | 17 | 4 | | | | | |
| UCEC | TCGA-DI-A1NN-01 | 63 | 5 | | UVM | TCGA-V4-A9ET-01 | 17 | 1 | | | | | |
| UCEC | TCGA-E6-A11Z-01 | 218 | 11 | | UVM | TCGA-V4-A9EU-01 | 17 | 2 | | | | | |
| UCEC | TCGA-EO-A1Y5-01 | 88 | 3 | | UVM | TCGA-V4-A9EV-01 | 15 | 2 | | | | | |
| UCEC | TCGA-EO-A1Y8-01 | 69 | 5 | | UVM | TCGA-V4-A9EW-01 | 19 | 2 | | | | | |
| UCEC | TCGA-EY-A1GS-01 | 285 | 18 | | UVM | TCGA-V4-A9EX-01 | 18 | 1 | | | | | |
| UCEC | TCGA-EY-A212-01 | 71 | 4 | | UVM | TCGA-V4-A9EY-01 | 23 | 2 | | | | | |
| UCEC | TCGA-FI-A2D2-01 | 51 | 4 | | UVM | TCGA-V4-A9EZ-01 | 29 | 2 | | | | | |
| UCEC | TCGA-FI-A2EW-01 | 122 | 6 | | UVM | TCGA-V4-A9F0-01 | 16 | 3 | | | | | |
| UCEC | TCGA-FI-A2EX-01 | 68 | 2 | | UVM | TCGA-V4-A9F1-01 | 14 | 1 | | | | | |
| UCEC | TCGA-FI-A2F8-01 | 78 | 3 | | UVM | TCGA-V4-A9F2-01 | 10 | 1 | | | | | |
| UCS | TCGA-N5-A4R8-01 | 75 | 3 | | UVM | TCGA-V4-A9F3-01 | 14 | 2 | | | | | |
| UCS | TCGA-N5-A4RA-01 | 75 | 5 | | UVM | TCGA-V4-A9F4-01 | 25 | 3 | | | | | |
| UCS | TCGA-N5-A4RD-01 | 89 | 7 | | UVM | TCGA-V4-A9F5-01 | 13 | 2 | | | | | |
| UCS | TCGA-N5-A4RF-01 | 123 | 10 | | UVM | TCGA-V4-A9F7-01 | 20 | 3 | | | | | |
| UCS | TCGA-N5-A4RJ-01 | 94 | 9 | | UVM | TCGA-V4-A9F8-01 | 28 | 2 | | | | | |

**Claims**

1. A computer-implemented method of evaluating the tumor mutational burden in a sample, the method comprising:

   a) providing a nucleotide sequence of a set of subgenomic intervals from the sample, wherein the set of subgenomic intervals are from a predetermined set of genes that does not comprise the entire genome or the entire exome, wherein the predetermined set of genes comprises a plurality of genes, which in mutant form, are associated with an effect on cell division, growth or survival, or are associated with cancer, optionally wherein the set of subgenomic intervals is a set of coding subgenomic intervals; and

   b) determining a value for the tumor mutational burden, wherein the value is a function of the number of an alteration in the set of subgenomic intervals, wherein said number of an alteration excludes:

   (i) a functional alteration in a subgenomic interval, wherein the functional alteration is an alteration that, compared with a reference sequence, has an effect on cell division, growth or survival, and wherein the functional alteration is identified as such based on inclusion in a database of functional alterations; and
   (ii) a germline alteration in a subgenomic interval,

   thereby evaluating the tumor mutational burden in the sample, optionally wherein the sample is a tumor sample or a sample derived from a tumor.

2. The method of claim 1, wherein the provided nucleotide sequence has been acquired by:

   (i) acquiring a library comprising a plurality of tumor members from the sample;
   (ii) contacting the library with a bait set to provide selected tumor members, wherein said bait set hybridizes with the tumor member, thereby providing a library catch;
   (iii) acquiring a read for a subgenomic interval comprising an alteration from a tumor member from said library catch, optionally wherein the subgenomic interval is a coding subgenomic interval, further optionally wherein acquiring a read is performed by a next-generation sequencing method;
   (iv) aligning said read by an alignment method;
   (v) assigning a nucleotide value from said read for a preselected nucleotide position; and
   (vi) selecting a set of subgenomic intervals from a set of the assigned nucleotide positions, wherein the set of subgenomic intervals are from a predetermined set of genes that does not comprise the entire genome or the entire exome.

3. The method of claim 1 or 2, wherein

   (i) the value is expressed as a function of the predetermined set of genes, optionally wherein the value is expressed as a function of the coding regions of the predetermined set of genes;
   (ii) the value is expressed as a function of the subgenomic intervals sequenced, optionally wherein the value is expressed as a function of the coding subgenomic intervals sequenced;
   (iii) the value is expressed as a function of the number of an alteration per a preselected unit, optionally wherein the value is expressed as a function of the number of an alteration per megabase;
   (iv) the value is expressed as a function of the number of an alteration in a preselected number of positions of the predetermined set of genes, optionally wherein the value is expressed as a function of the number of an alteration in the coding regions of the predetermined set of genes;
   (v) the value is expressed as a function of the number of an alteration in a preselected number of positions of the subgenomic intervals sequenced, optionally wherein the value is expressed as a function of the number of an alteration in the coding subgenomic intervals sequenced;
   (vi) the value is expressed as a function of the number of an alteration per megabase in the predetermined set of genes, optionally wherein the value is expressed as a function of the number of an alteration per megabase in the coding regions of the predetermined set of genes;
   (vii) the value is expressed as a function of the number of alterations per megabase in the subgenomic intervals (*e.g.,* coding subgenomic intervals) sequenced, optionally wherein the value is expressed as a function of the number of alterations per megabase in the coding subgenomic intervals sequenced;
   (viii) the tumor mutational burden is extrapolated to a larger portion of the genome, optionally wherein the tumor mutational burden is extrapolated to the entire exome or the entire genome;
   (ix) the sample is from a subject having a cancer, or a subject who is receiving, or has received, a therapy;
   (x) the tumor mutational burden is expressed as a percentile, optionally wherein the tumor mutational burden is expressed as a percentile among the tumor mutational burdens in samples from a reference population, optionally wherein the reference population is a reference population of patients having the same type of cancer as the subject, or patients who are receiving, or have received, the same type of therapy as the subject;

(xi) the functional alteration is an alteration that, compared with a wild-type or unmutated sequence, has an effect on cell division, growth or survival, *e.g.,* optionally wherein the functional alteration is an alteration that, compared with a reference sequence, promotes cell division, growth or survival;

(xii) the functional alteration is an alteration with known functional status;

(xiii) the functional alteration is an alteration with a likely functional status, optionally wherein the functional alteration is a truncation in a tumor suppressor gene;

(xiv) the functional alteration is a driver mutation, optionally wherein the driver mutation is an alteration that gives a selective advantage to a clone in its microenvironment, further optionally wherein the driver mutation is an alteration that gives a selective advantage to a clone in its microenvironment by increasing cell survival or reproduction;

(xv) the functional alteration is an alteration capable of causing clonal expansions;

(xvi) the functional alteration is an alteration capable of causing one or more of the following:

  (a) self-sufficiency in a growth signal;
  (b) decreased sensitivity to an antigrowth signal, optionally insensitivity to an antigrowth signal;
  (c) decreased apoptosis;
  (d) increased replicative potential;
  (e) sustained angiogenesis; or
  (f) tissue invasion or metastasis;

(xvii) the functional alteration is not a passenger mutation, optionally wherein the functional alteration is an alteration that has a detectable effect on the fitness of a clone;

(xviii) the functional alteration is not a variant of unknown significance (VUS), optionally wherein the functional alteration is not an alteration, the pathogenicity of which can neither be confirmed nor ruled out;

(xix) a plurality of functional alterations in a preselected gene in the predetermined set of genes are excluded, optionally wherein 10%, 20%, 30%, 40%, 50%, or 75% or more of functional alterations in a preselected in the predetermined set of genes are excluded, further optionally wherein the preselected gene is a tumor gene;

(xx) all functional alterations in a preselected gene in the predetermined set of genes are excluded, further optionally wherein the preselected gene is a tumor gene;

(xxi) a plurality of functional alterations in a plurality of preselected genes in the predetermined set of genes are excluded, optionally wherein the preselected genes are tumor genes;

(xxii) all functional alterations in all genes in the predetermined set of genes are excluded, optionally wherein the genes are tumor genes;

(xxiii) the germline alteration is excluded by use of a method that does not use a comparison with a matched normal sequence;

(xxiv) the germline alteration is excluded by a method comprising the use of an SGZ algorithm;

(xxv) the germline alteration is identified as such by inclusion in a database of germline alterations;

(xxvi) the germline alteration is a single nucleotide polymorphism (SNP), a base substitution, an indel, or a silent mutation, optionally wherein the germline alteration is a synonymous mutation;

(xxvii) the alteration is a silent mutation, a synonymous alteration;

(xxviii) the alteration is a passenger mutation, optionally wherein the alteration is an alteration that has no detectable effect on the fitness of a clone;

(xxix) the alteration is a variant of unknown significance (VUS), optionally wherein the alteration is an alteration, the pathogenicity of which can neither be confirmed nor ruled out;

(xxx) the alteration is a point mutation;

(xxxi) the alteration is a short variant, optionally wherein the short variant is a short coding variant, further optionally wherein the short variant is a base substitution, an indel, an insertion, or a deletion;

(xxxii) the alteration is a non-synonymous single nucleotide variant (SNV);

(xxxiii) the alteration is a splice variant;

(xxxiv) the alteration has not been identified as being associated with a cancer phenotype;

(xxxv) the alteration is other than a rearrangement, optionally wherein the alteration is other than a translocation;

(xxxvi) the predetermined set of genes comprises a plurality of genes, which in mutant form, are associated with an effect on cell division, growth or survival, or are associated with cancer;

(xxxvii) the predetermined set of genes comprise at least about 50 or more, about 100 or more, about 150 or more, about 200 or more, about 250 or more, about 300 or more, about 350 or more, about 400 or more, about 450 or more, or about 500 or more genes; or

(xxxviii) the predetermined set of genes comprise at least about 50 or more, about 100 or more, about 150 or more, about 200 or more, about 250 or more, about 300 or more, or all of the genes or gene products chosen from Tables 1-4 or FIGs. 3A-4D.

4. The method of claim 2, wherein

(i) acquiring a read for the subgenomic interval comprises sequencing a subgenomic interval from at least about 50 or more, about 100 or more, about 150 or more, about 200 or more, about 250 or more, about 300 or more, or all of the genes or gene products chosen from Tables 1-4 or FIGs. 3A-4D;

(ii) acquiring a read for the subgenomic interval comprises sequencing with greater than about 250X, greater than about 500X, or greater than about 1,000X, average unique coverage; or

(iii) acquiring a read for the subgenomic interval comprises sequencing with greater than about 250X, greater than about 500X, or greater than about 1,000X, average unique coverage, at greater than 95%, greater than about 97%, or greater than about 99%, of the genes (*e.g.,* exons) sequenced.

5. The method of claim 1 or 2, further comprising characterizing a variant in the tumor sample by:

a) acquiring:

i) a sequence coverage input (SCI), which comprises, for each of a plurality of selected subgenomic intervals, a value for normalized sequence coverage at the selected subgenomic intervals, wherein SCI is a function of the number of reads for a subgenomic interval and the number of reads for a process-matched control;

ii) an SNP allele frequency input (SAFI), which comprises, for each of a plurality of selected germline SNPs, a value for the allele frequency in the tumor sample, wherein SAFI is based, at least in part, on a minor or alternative allele frequency in the tumor sample; and

iii) a variant allele frequency input (VAFI), which comprises the allele frequency for said variant in the tumor sample;

b) acquiring values, as a function of SCI and SAFI, for:

i) a genomic segment total copy number (C) for each of a plurality of genomic segments;

ii) a genomic segment minor allele copy number (M) for each of a plurality of genomic segments; and

iii) sample purity (p),

wherein the values of C, M, and p are obtained by fitting a genome-wide copy number model to SCI and SAFI; and

c) acquiring:

a value for mutation type, g, for which is indicative of the variant, being somatic, a subclonal somatic variant, germline, or not-distinguishable, and is a function of VAFI, p, C, and M, optionally wherein the variant is an alteration.

6. The method of claim 5, further comprising sequencing each of a plurality of selected subgenomic intervals, each of a plurality of selected germline SNPs, and a variant, wherein the average sequence coverage prior to normalization is at least about 250x, e.g., at least about 500x, optionally wherein the variant is an alteration; or

(ii) wherein fitting the genome-wide copy number model to SCI comprises using the equation of:

$$\log Ratio_i = \log_2 \frac{pC_i + 2(1-p)}{p\psi + 2(1-p)},$$

where $\psi$ is tumor ploidy; or

(iii) wherein fitting the genome-wide copy number model to SAFI comprises using the equation of:

$$AF = \frac{pM + 1(1-p)}{pC + 2(1-p)},$$

where AF is allele frequency; or

(iv) wherein g is determined by determining the fit of values for VAFI, p, C, and M to a model for somatic/germline status; or

(v) wherein the value of g is acquired by:

$$AF = \frac{pM + g(1-p)}{pC + 2(1-p)},$$

where AF is allele frequency; or

(vi) wherein

a value of g that is 0, or close to 0 indicates that the variant is a somatic variant;

a value of g that is 1, or close to 1 indicates that the variant is a germline variant;

a value of g that is less than 1 but more than 0 indicates an indistinguishable result; and

a value of g that is significantly less than 0 indicates that the variant is a subclonal somatic variant.

7. The method of claim 1 or 2, wherein the sample comprises one or more premalignant or malignant cells; cells from a solid tumor, a soft tissue tumor or a metastatic lesion; tissue or cells from a surgical margin; a histologically normal tissue; one or more circulating tumor cells (CTC); a normal adjacent tissue (NAT); a blood sample from the same subject having or at risk of having the tumor; or an FFPE-sample, optionally wherein the sample is a tumor sample or a sample derived from a tumor.

8. The method of claim 1 or 2, wherein the sample is a FFPE sample.

9. The method of claim 8, wherein the FFPE sample has one, two or all of the following properties:

(a) has a surface area of 25 mm$^2$ or greater;

(b) has a sample volume of 1 mm$^3$ or greater; or

(c) has a nucleated cellularity of 80% or more or 30,000 cells or more.

10. The method of claim 1 or 2,

(i) wherein the sample is a sample comprising circulating tumor DNA (ctDNA);

(ii) wherein the sample is acquired from a solid tumor, a hematological cancer, or a metastatic form thereof;

(iii) further comprising classifying the tumor sample or the subject from which the tumor sample was derived responsive to the evaluation of the tumor mutational burden; or

(iv) further comprising generating a report to the patient or to another person or entity, a caregiver, a physician, an oncologist, a hospital, clinic, third-party payor, insurance company or government office; optionally wherein said report comprises output from the method which comprises the tumor mutational burden, optionally wherein the report is an electronic, web-based, or paper report.

11. A system for evaluating the tumor mutational burden in a sample, comprising:

at least one processor operatively connected to a memory, the at least one processor when executing is configured to:

a) indirectly acquire a nucleotide sequence of a set of subgenomic intervals from the tumor sample, wherein the set of subgenomic intervals are from a predetermined set of genes that does not comprise the entire genome or the entire exome, wherein the predetermined set of genes comprises a plurality of genes, which in mutant form, are associated with an effect on cell division, growth or survival, or are associated with cancer; and

b) determine a value for the tumor mutational burden, wherein the value is a function of the number of an alteration in the set of subgenomic intervals, wherein said number of an alteration excludes:

(i) a functional alteration in a subgenomic interval, wherein the functional alteration is an alteration that, compared with a reference sequence, has an effect on cell division, growth or survival, and wherein the functional alteration is identified as such based on inclusion in a database of functional alterations, optionally wherein the subgenomic interval is a coding subgenomic interval; and

(ii) a germline alteration in a subgenomic interval, optionally wherein the subgenomic interval is a coding subgenomic interval.

**Patentansprüche**

1. Computerimplementiertes Verfahren zum Bewerten der Tumormutationslast in einer Probe, wobei das Verfahren Folgendes umfasst:

a) Bereitstellen einer Nucleotidsequenz eines Satzes von subgenomischen Intervallen aus der Probe, wobei der Satz von subgenomischen Intervallen aus einem vorbestimmten Satz von Genen stammt, der nicht das gesamte Genom oder das gesamte Exom umfasst, wobei der vorbestimmte Satz von Genen eine Vielzahl von Genen umfasst, die in mutierter Form mit einem Effekt auf die Zellteilung, das Zellwachstum oder -überleben in Zusammenhang stehen oder mit Krebs in Zusammenhang stehen, wobei der Satz von subgenomischen Intervallen gegebenenfalls ein Satz von kodierenden subgenomischen Intervallen ist; und
b) Bestimmen eines Werts für die Tumormutationslast, wobei der Wert eine Funktion der Anzahl einer Veränderung in dem Satz von subgenomischen Intervallen ist, wobei die Anzahl einer Veränderung Folgendes ausschließt:

(i) eine funktionelle Veränderung in einem subgenomischen Intervall, wobei die funktionelle Veränderung eine Veränderung ist, die verglichen mit einer Referenzsequenz einen Effekt auf die Zellteilung, das Zellwachstum oder -überleben aufweist, und wobei die funktionelle Veränderung als solche basierend auf der Aufnahme in eine Datenbank von funktionellen Veränderungen identifiziert wird; und
(ii) eine Keimbahnveränderung in einem subgenomischen Intervall,

wodurch die Tumormutationslast in der Probe bewertet wird, wobei die Probe gegebenenfalls eine Tumorprobe oder eine Probe, abgeleitet von einem Tumor, ist.

2. Verfahren nach Anspruch 1, wobei die bereitgestellte Nucleotidsequenz erfasst wurde durch:

(i) Erfassen einer Bibliothek, umfassend eine Vielzahl von Tumorelementen, aus der Probe;
(ii) Kontaktieren der Bibliothek mit einem Ködersatz, um ausgewählte Tumorelemente bereitzustellen, wobei der Ködersatz mit dem Tumorelement hybridisiert, wodurch ein Bibliotheksfang bereitgestellt wird;
(iii) Erfassen einer Auslesung für ein subgenomisches Intervall, umfassend eine Veränderung aus einem Tumorelement aus dem Bibliotheksfang, wobei das subgenomische Intervall gegebenenfalls ein kodierendes subgenomisches Intervall ist, wobei das Erfassen einer Auslesung gegebenenfalls weiters durch ein Sequenzierungsverfahren der nächsten Generation durchgeführt wird;
(iv) Abgleichen der Auslesung durch ein Abgleichsverfahren;
(v) Zuweisen eines Nucleotidwerts aus der Auslesung für eine vorausgewählte Nucleotidposition; und
(vi) Auswählen eines Satzes von subgenomischen Intervallen aus einem Satz der zugewiesenen Nucleotidpositionen, wobei der Satz von subgenomischen Intervallen aus einem vorbestimmten Satz von Genen stammt, der nicht das gesamte Genom oder das gesamte Exom umfasst.

3. Verfahren nach Anspruch 1 oder 2, wobei:

(i) der Wert als eine Funktion des vorbestimmten Satzes von Genen ausgedrückt wird, wobei der Wert gegebenenfalls als Funktion der kodierenden Regionen des vorbestimmten Satzes von Genen ausgedrückt wird;
(ii) der Wert als eine Funktion der sequenzierten subgenomischen Intervalle ausgedrückt wird, wobei der Wert gegebenenfalls als Funktion der sequenzierten kodierenden subgenomischen Intervalle ausgedrückt wird;
(iii) der Wert als eine Funktion der Anzahl einer Veränderung pro einer vorausgewählten Einheit ausgedrückt wird, wobei der Wert gegebenenfalls als Funktion der Anzahl einer Veränderung pro Megabase ausgedrückt wird;
(iv) der Wert als eine Funktion der Anzahl einer Veränderung in einer vorausgewählten Anzahl von Positionen des vorbestimmten Satzes von Genen ausgedrückt wird, wobei der Wert gegebenenfalls als Funktion der Anzahl einer Veränderung in den kodierenden Regionen des vorbestimmten Satzes von Genen ausgedrückt wird;
(v) der Wert als eine Funktion der Anzahl einer Veränderung in einer vorausgewählten Anzahl von Positionen der sequenzierten subgenomischen Intervalle ausgedrückt wird, wobei der Wert gegebenenfalls als Funktion der Anzahl einer Veränderung in den sequenzierten kodierenden subgenomischen Intervallen ausgedrückt wird;
(vi) der Wert als eine Funktion der Anzahl einer Veränderung pro Megabase in dem vorbestimmten Satz von Genen ausgedrückt wird, wobei der Wert gegebenenfalls als eine Funktion der Anzahl einer Veränderung pro Megabase in den kodierenden Regionen des vorbestimmten Satzes von Genen ausgedrückt wird;
(vii) der Wert als eine Funktion der Anzahl von Veränderungen pro Megabase in den sequenzierten subgenomischen Intervallen (z. B. kodierenden subgenomischen Intervallen) ausgedrückt wird, wobei der Wert gegebenenfalls als eine Funktion der Anzahl von Veränderungen pro Megabase in den sequenzierten kodierenden subgenomischen Intervallen ausgedrückt wird;

(viii) die Tumormutationslast auf einen größeren Abschnitt des Genoms extrapoliert wird, wobei die Tumormutationslast gegebenenfalls auf das gesamte Exom oder das gesamte Genom extrapoliert wird;

(ix) die Probe von einem Individuum mit Krebs oder einem Individuum, das eine Therapie erhält oder erhalten hat, stammt;

(x) die Tumormutationslast als Perzentile ausgedrückt wird, wobei die Tumormutationslast gegebenenfalls als Perzentile aus den Tumormutationslasten in Proben von einer Referenzpopulation ausgedrückt wird, wobei die Referenzpopulation gegebenenfalls eine Referenzpopulation von Patienten ist, die an derselben Art von Krebserkrankung wie das Individuum leiden, oder von Patienten ist, die dieselbe Art von Therapie wie das Individuum erhalten oder erhalten haben;

(xi) die funktionelle Veränderung eine Veränderung ist, die verglichen mit einer Wildtyp- oder nichtmutierten Sequenz einen Effekt auf die Zellteilung, das Zellwachstum oder -überleben hat, z. B. wobei die funktionelle Veränderung gegebenenfalls eine Veränderung ist, die verglichen mit einer Referenzsequenz die Zellteilung, das Zellwachstum oder -überleben fördert;

(xii) die funktionelle Veränderung eine Veränderung mit bekanntem funktionellem Status ist;

(xiii) die funktionelle Veränderung eine Veränderung mit wahrscheinlichem funktionellem Status ist, wobei die funktionelle Veränderung gegebenenfalls eine Trunkierung in einem Tumorsuppressorgen ist;

(xiv) die funktionelle Veränderung eine Driver-Mutation ist, wobei die Driver-Mutation gegebenenfalls eine Veränderung ist, die einem Klon in dessen Mikroumgebung einen selektiven Vorteil verleiht, wobei die Driver-Mutation weiters gegebenenfalls eine Veränderung ist, die einem Klon in dessen Mikroumgebung einen Vorteil verleiht, indem das Zellüberleben oder die -reproduktion verbessert werden;

(xv) die funktionelle Veränderung eine Veränderung ist, die zum Bewirken von Klonerweiterungen in der Lage ist;

(xvi) die funktionelle Veränderung eine Veränderung ist, die zum Bewirken eines oder mehrerer der Folgenden in der Lage ist:

(a) Autarkie in einem Wachstumssignal;
(b) verringerte Sensibilität gegenüber einem Antiwachstumssignal, gegebenenfalls Insensibilität gegenüber einem Antiwachstumssignal;
(c) verringerte Apoptose;
(d) erhöhtes Replikationspotenzial;
(e) aufrechterhaltene Angiogenese; oder
(f) Gewebeinvasion oder Metastase;

(xvii) die funktionelle Veränderung keine Passenger-Mutation ist, wobei die funktionelle Veränderung gegebenenfalls eine Veränderung ist, die einen detektierbaren Effekt auf die Fitness eines Klons aufweist;

(xviii) die funktionelle Veränderung keine Variante einer unbekannten Signifikanz (VUS) ist, wobei die funktionelle Veränderung gegebenenfalls keine Veränderung ist, deren Pathogenität weder bestätigt noch ausgeschlossen werden kann;

(xix) eine Vielzahl von funktionellen Veränderungen in einem vorausgewählten Gen in dem vorbestimmten Satz von Genen ausgeschlossen ist, wobei gegebenenfalls 10 %, 20 %, 30 %, 40 %, 50 % oder 75 % oder mehr von funktionellen Veränderungen in einem vorausgewählten in dem vorbestimmten Satz von Genen ausgeschlossen sind, wobei das vorausgewählte Gen weiters gegebenenfalls ein Tumorgen ist;

(xx) alle funktionellen Veränderungen in einem vorausgewählten Gen in dem vorbestimmten Satz von Genen ausgeschlossen sind, wobei das vorausgewählte Gen weiters gegebenenfalls ein Tumorgen ist;

(xxi) eine Vielzahl von funktionellen Veränderungen in einer Vielzahl von vorausgewählten Genen in dem vorbestimmten Satz von Genen ausgeschlossen ist, wobei die vorausgewählten Gene gegebenenfalls Tumorgene sind;

(xxii) alle funktionellen Veränderungen in allen Genen in dem vorbestimmten Satz von Genen ausgeschlossen sind, wobei die Gene gegebenenfalls Tumorgene sind;

(xxiii) die Keimbahnveränderung durch Verwendung eines Verfahrens, das keinen Vergleich mit einer abgeglichenen normalen Sequenz verwendet, ausgeschlossen ist;

(xxiv) die Keimbahnveränderung durch ein Verfahren, das die Verwendung eines SGZ-Algorithmus umfasst, ausgeschlossen ist;

(xxv) die Keimbahnveränderung als solche durch Aufnahme in eine Datenbank von Keimbahnveränderungen identifiziert wird;

(xxvi) die Keimbahnveränderung ein Einzelnucleotidpolymorphismus (SNP), eine Basensubstitution, ein Indel oder eine stumme Mutation ist, wobei die Keimbahnveränderung gegebenenfalls eine synonyme Mutation ist;

(xxvii) die Veränderung eine stumme Mutation, eine synonyme Veränderung ist;

(xxviii) die Veränderung eine Passenger-Mutation ist, wobei die Veränderung gegebenenfalls keinen detektierbaren Effekt auf die Fitness eines Klons aufweist;

(xxix) die Veränderung eine Variante unbekannter Signifikanz (VUS) ist, wobei die Veränderung gegebenenfalls eine Veränderung ist, deren Pathogenität weder bestätigt, noch ausgeschlossen werden kann;

(xxx) die Veränderung eine Punktmutation ist;

(xxxi) die Veränderung eine kurze Variante ist, wobei die kurze Variante gegebenenfalls eine kurze kodierende Variante ist, wobei die kurze Variante weiters gegebenenfalls eine Basensubstitution, ein Indel, eine Insertion oder eine Deletion ist;

(xxxii) die Veränderung eine nichtsynonyme Einzelnucleotidvariante (SNV) ist;

(xxxiii) die Veränderung eine Spleiß-Variante ist;

(xxxiv) die Veränderung nicht als mit einem Krebsphänotyp in Zusammenhang stehend identifiziert wurde;

(xxxv) die Veränderung eine andere als eine Umordnung ist, wobei die Veränderung gegebenenfalls eine andere als eine Translokation ist;

(xxxvi) der vorbestimmte Satz von Genen eine Vielzahl von Genen umfasst, die in mutierter Form mit einem Effekt auf die Zellteilung, das Zellwachstum oder -überleben in Zusammenhang stehen oder mit Krebs in Zusammenhang stehen;

(xxxvii) der vorbestimmte Satz von Genen zumindest etwa 50 oder mehr, etwa 100 oder mehr, etwa 150 oder mehr, etwa 200 oder mehr, etwa 250 oder mehr, etwa 300 oder mehr, etwa 350 oder mehr, etwa 400 oder mehr, etwa 450 oder mehr oder etwa 500 oder mehr Gene umfasst; oder

(xxxviii) der vorbestimmte Satz von Genen zumindest etwa 50 oder mehr, etwa 100 oder mehr, etwa 150 oder mehr, etwa 200 oder mehr, etwa 250 oder mehr, etwa 300 oder mehr oder alle der Gene oder Genprodukte umfasst, die aus Tabelle 1 bis 4 oder Fig. 3A bis 4D ausgewählt sind.

4. Verfahren nach Anspruch 2, wobei:

(i) das Erfassen einer Auslesung für das subgenomische Intervall das Sequenzieren eines subgenomischen Intervalls von zumindest etwa 50 oder mehr, etwa 100 oder mehr, etwa 150 oder mehr, etwa 200 oder mehr, etwa 250 oder mehr, etwa 300 oder mehr oder von allen der Gene oder Genprodukte, die aus Tabelle 1 bis 4 oder Fig. 3A bis 4D ausgewählt sind, umfasst;

(ii) das Erfassen einer Auslesung für das subgenomische Intervall das Sequenzieren mit einer mittleren eindeutigen Abdeckung von mehr als etwa 250X, mehr als etwa 500X oder mehr als etwa 1.000X umfasst; oder

(iii) das Erfassen einer Auslesung für das subgenomische Intervall das Sequenzieren mit einer mittleren eindeutigen Abdeckung von mehr als etwa 250X, mehr als etwa 500X oder mehr als etwa 1.000X bei mehr als 95 %, mehr als etwa 97 % oder mehr als etwa 99 % der sequenzierten Gene (z. B. Exons) umfasst.

5. Verfahren nach Anspruch 1 oder 2, das weiters das Charakterisieren einer Variante in der Tumorprobe durch Folgendes umfasst:

a) Erfassen von:

i) einer Sequenzabdeckungseingabe (SCI), die für jedes aus einer Vielzahl von ausgewählten subgenomischen Intervallen einen Wert für eine normalisierte Sequenzabdeckung an den ausgewählten subgenomischen Intervallen umfasst, wobei SCI eine Funktion der Anzahl von Auslesungen für ein subgenomisches Intervall und der Anzahl von Auslesungen für eine prozessangepasste Kontrolle ist;

ii) einer SNP-Allelhäufigkeitseingabe (SAFI), die für jeden aus einer Vielzahl von ausgewählten Keimbahn-SNP einen Wert für die Allelhäufigkeit in der Tumorprobe umfasst, wobei SAFI zumindest teilweise auf einer Neben- oder alternativen Allelhäufigkeit in der Tumorprobe basiert; und

iii) einer Varianten-Allelhäufigkeitseingabe (VAFI), die die Allelhäufigkeit für die Variante in der Tumorprobe umfasst;

b) Erfassen von Werten als eine Funktion von SCI und SAFI für:

i) eine Gesamtkopienanzahl (C) eines genomischen Segments für jedes aus einer Vielzahl von genomischen Segmenten;

ii) eine Nebenallelkopienanzahl (M) eines genomischen Segments für jedes aus einer Vielzahl von genomischen Segmenten; und

iii) die Probenreinheit (p),

wobei die Werte von C, M und p durch Anpassen eines genomweiten Kopienanzahlmodells an SCI und SAFI erhalten werden; und

c) Erfassen von:

einem Wert für einen Mutationstyp, g, der die Variante angibt, die somatisch, eine subklonale somatische Variante, Keimbahn oder nichtunterscheidbar ist, und eine Funktion von VAFI, p, C und M ist, wobei die Variante gegebenenfalls eine Veränderung ist.

6. Verfahren nach Anspruch 5, das weiters das Sequenzieren jedes aus einer Vielzahl von ausgewählten subgenomischen Intervallen, jedes einer Vielzahl von ausgewählten Keimbahn-SNP und einer Variante umfasst, wobei die mittlere Sequenzabdeckung vor der Normalisierung zumindest etwa 250x, z. B. zumindest etwa 500x, beträgt, wobei die Variante gegebenenfalls eine Veränderung ist; oder

(ii) wobei das Anpassen des genomweiten Kopienanzahlmodells an SCI das Verwenden der folgenden Gleichung umfasst:

$$\log Ratio_i = \log_2 \frac{pC_i + 2(1-p)}{p\psi + 2(1-p)},$$

worin $\Psi$ die Tumorploidie ist; oder

(iii) wobei das Anpassen des genomweiten Kopienanzahlmodells an SAFI das Verwenden der folgenden Gleichung umfasst:

$$AF = \frac{pM + 1(1-p)}{pC + 2(1-p)},$$

worin AF die Allelhäufigkeit ist; oder

(iv) wobei g durch Bestimmen der Anpassung von Werten für VAFI, p, C und M an ein Modell für den somatischen/Keimbahnstatus bestimmt wird; oder

(v) wobei der Wert g erfasst wird durch:

$$AF = \frac{pM + g(1-p)}{pC + 2(1-p)},$$

worin AF die Allelhäufigkeit ist; oder

(vi) wobei:

ein Wert von g, der 0 oder nahe 0 ist, angibt, dass die Variante eine somatische Variante ist;
ein Wert von g, der 1 oder nahe 1 ist, angibt, dass die Variante eine Keimbahnvariante ist;
ein Wert von g, der kleiner als 1, aber größer als 0 ist, ein nichtunterscheidbares Ergebnis angibt; und
ein Wert von g, der signifikant kleiner als 0 ist, angibt, dass die Variante eine subklonale somatische Variante ist.

7. Verfahren nach Anspruch 1 oder 2, wobei die Probe eine oder mehrere prämaligne oder maligne Zellen; Zellen aus einem soliden Tumor, einem Weichgewebetumor oder einer metastatischen Läsion; Gewebe oder Zellen aus einem chirurgischen Randbereich; ein histologisch normales Gewebe; eine oder mehrere zirkulierende Tumorzellen (CTC); ein normales benachbartes Gewebe (NAT); eine Blutprobe aus demselben Individuum, das einen Tumor aufweist oder bei dem ein Risiko besteht, dass es einen Tumor aufweist; oder eine FFPE-Probe umfasst, wobei die Probe gegebenenfalls eine Tumorprobe oder eine Probe, die von einem Tumor abgeleitet ist, ist.

8. Verfahren nach Anspruch 1 oder 2, wobei die Probe eine FFPE-Probe ist.

**9.** Verfahren nach Anspruch 8, wobei die FFPE-Probe eine, zwei oder alle der folgenden Eigenschaften aufweist:

(a) weist eine Oberfläche von 25 mm$^2$ oder mehr auf;
(b) weist ein Probenvolumen von 1 mm$^3$ oder mehr auf; oder
(c) weist eine kernhaltige Zellularität von 80 % oder mehr oder 30.000 Zellen oder mehr auf.

**10.** Verfahren nach Anspruch 1 oder 2,

(i) wobei die Probe eine Probe ist, die zirkulierende Tumor-DNA (ctDNA) umfasst;
(ii) wobei die Probe von einem festen Tumor, einem hämatologischen Krebs oder einer metastasierten Form davon erhalten wird;
(iii) das weiters das Klassifizieren der Tumorprobe oder des Individuums, aus dem die Tumorprobe erhalten wurde, als reaktiv auf die Bewertung der Tumormutationslast umfasst; oder
(iv) das weiters das Erstellen eines Berichts für den Patienten oder für eine weitere Person oder Institution, für eine Betreuungsperson, einen Arzt, einen Onkologen, ein Krankenhaus, eine Klinik, einen Dritt-Kostenträger, eine Versicherung oder eine Regierungsbehörde umfasst; wobei der Bericht gegebenenfalls die Ausgabe von dem Verfahren umfasst, die die Tumormutationslast umfasst, wobei der Bericht gegebenenfalls ein elektronischer, webbasierter oder ein Bericht in Papierform ist.

**11.** System zum Bewerten der Tumormutationslast in einer Probe, umfassend:
zumindest einen Prozessor, der operabel mit einem Speicher verbunden ist, wobei der zumindest eine Prozessor beim Ausführen ausgelegt ist zum:

a) indirekten Erfassen einer Nucleotidsequenz eines Satzes von subgenomischen Intervallen aus der Tumorprobe, wobei der Satz von subgenomischen Intervallen aus einem vorbestimmten Satz von Genen stammt, der nicht das gesamte Genom oder das gesamte Exom umfasst, wobei der vorbestimmte Satz von Genen eine Vielzahl von Genen umfasst, die in mutierter Form mit einem Effekt auf die Zellteilung, das Zellwachstum oder -überleben in Zusammenhang stehen oder mit Krebs in Zusammenhang stehen; und
b) Bestimmen eines Werts für die Tumormutationslast, wobei der Wert eine Funktion der Anzahl einer Veränderung in dem Satz von subgenomischen Intervallen ist, wobei die Anzahl einer Veränderung Folgendes ausschließt:

(i) eine funktionelle Veränderung in einem subgenomischen Intervall, wobei die funktionelle Veränderung eine Veränderung ist, die verglichen mit einer Referenzsequenz einen Effekt auf die Zellteilung, das Zellwachstums oder das -überleben aufweist, und wobei die funktionelle Veränderung als solche basierend auf der Aufnahme in eine Datenbank von funktionellen Veränderungen identifiziert wird, wobei das subgenomische Intervall gegebenenfalls ein kodierendes subgenomisches Intervall ist; und
(ii) eine Keimbahnveränderung in einem subgenomischen Intervall, wobei das subgenomische Intervall gegebenenfalls ein kodierendes subgenomisches Intervall ist.

**Revendications**

**1.** Procédé, mis en œuvre par ordinateur, d'évaluation de la charge mutationnelle tumorale dans un échantillon, le procédé comprenant le fait de :

a) fournir une séquence nucléotidique d'un ensemble d'intervalles sous-génomiques provenant de l'échantillon, dans lequel l'ensemble d'intervalles sous-génomiques provient d'un ensemble prédéterminé de gènes qui ne comprend pas le génome entier ou l'exome entier, dans lequel l'ensemble prédéterminé de gènes comprend une pluralité de gènes qui, sous forme mutante, sont associés à un effet sur la division, la croissance ou la survie cellulaire, ou sont associés au cancer, éventuellement dans lequel l'ensemble d'intervalles sous-génomiques est un ensemble d'intervalles sous-génomiques codants; et
b) déterminer une valeur pour la charge mutationnelle tumorale, dans laquelle la valeur est fonction du nombre d'altérations dans l'ensemble d'intervalles sous-génomiques, dans lequel ledit nombre d'altérations exclut :

(i) une altération fonctionnelle dans un intervalle sous-génomique, dans laquelle l'altération fonctionnelle est une altération qui, par rapport à une séquence de référence, a un effet sur la division, la croissance ou la survie cellulaire, et dans laquelle l'altération fonctionnelle est identifiée comme telle sur la base de son inclusion dans une base de données d'altérations fonctionnelles ; et

(ii) une altération germinale dans un intervalle sous-génomique,

ce qui permet d'évaluer la charge mutationnelle tumorale dans l'échantillon, éventuellement, dans lequel l'échantillon est un échantillon tumoral ou un échantillon dérivé d'une tumeur.

**2.** Procédé selon la revendication 1, dans lequel la séquence nucléotidique fournie a été acquise par :

(i) l'acquisition d'une bibliothèque comprenant une pluralité d'éléments tumoraux à partir de l'échantillon ;

(ii) la mise en contact de la bibliothèque avec un ensemble de leurres pour fournir des éléments tumoraux sélectionnés, dans lequel ledit ensemble de leurres s'hybride avec l'élément tumoral, fournissant ainsi une capture de bibliothèque ;

(iii) l'acquisition d'une lecture pour un intervalle sous-génomique comprenant une altération à partir d'un élément tumoral provenant de ladite capture de bibliothèque, éventuellement dans lequel l'intervalle sous-génomique est un intervalle sous-génomique codant, en outre éventuellement dans lequel l'acquisition d'une lecture est effectuée par un procédé de séquençage de nouvelle génération ;

(iv) l'alignement de ladite lecture par un procédé d'alignement ;

(v) l'attribution d'une valeur de nucléotide à partir de ladite lecture pour une position de nucléotide présélectionnée ; et

(vi) la sélection d'un ensemble d'intervalles sous-génomiques à partir d'un ensemble des positions de nucléotides attribuées, dans lequel l'ensemble d'intervalles sous-génomiques provient d'un ensemble prédéterminé de gènes qui ne comprend pas le génome entier ou l'exome entier.

**3.** Procédé selon la revendication 1 ou 2, dans lequel

(i) la valeur est exprimée en fonction de l'ensemble prédéterminé de gènes, éventuellement dans laquelle la valeur est exprimée en fonction des régions codantes de l'ensemble prédéterminé de gènes ;

(ii) la valeur est exprimée en fonction des intervalles sous-génomiques séquencés, éventuellement dans laquelle la valeur est exprimée en fonction des intervalles sous-génomiques codants séquencés ;

(iii) la valeur est exprimée en fonction du nombre d'altérations par unité présélectionnée, éventuellement dans laquelle la valeur est exprimée en fonction du nombre d'altérations par mégabase ;

(iv) la valeur est exprimée en fonction du nombre d'altérations dans un nombre présélectionné de positions de l'ensemble prédéterminé de gènes, éventuellement dans laquelle la valeur est exprimée en fonction du nombre d'altérations dans les régions codantes de l'ensemble prédéterminé de gènes ;

(v) la valeur est exprimée en fonction du nombre d'altérations dans un nombre présélectionné de positions des intervalles sous-génomiques séquencés, éventuellement dans laquelle la valeur est exprimée en fonction du nombre d'altérations dans les intervalles sous-génomiques codants séquencés ;

(vi) la valeur est exprimée en fonction du nombre d'altérations par mégabase dans l'ensemble prédéterminé de gènes, éventuellement dans laquelle la valeur est exprimée en fonction du nombre d'altérations par mégabase dans les régions codantes de l'ensemble prédéterminé de gènes ;

(vii) la valeur est exprimée en fonction du nombre d'altérations par mégabase dans les intervalles sous-génomiques (par exemple, les intervalles sous-génomiques codants) séquencés, éventuellement dans laquelle la valeur est exprimée en fonction du nombre d'altérations par mégabase dans les intervalles sous-génomiques codants séquencés ;

(viii) la charge mutationnelle tumorale est extrapolée à une plus grande partie du génome, éventuellement dans laquelle la charge mutationnelle tumorale est extrapolée à l'exome entier ou au génome entier ;

(ix) l'échantillon provient d'un sujet atteint d'un cancer ou d'un sujet qui reçoit ou a reçu un traitement ;

(x) la charge mutationnelle tumorale est exprimée en centile, éventuellement dans laquelle la charge mutationnelle tumorale est exprimée en centile parmi les charges mutationnelles tumorales dans des échantillons provenant d'une population de référence, éventuellement dans laquelle la population de référence est une population de référence de patients atteints du même type de cancer que le sujet, ou de patients qui reçoivent ou ont reçu le même type de traitement que le sujet ;

(xi) l'altération fonctionnelle est une altération qui, par rapport à une séquence de type sauvage ou non mutée, a un effet sur la division, la croissance ou la survie cellulaire, par exemple, éventuellement dans laquelle l'altération fonctionnelle est une altération qui, par rapport à une séquence de référence, favorise la division, la croissance ou la survie cellulaire ;

(xii) l'altération fonctionnelle est une altération présentant un état fonctionnel connu ;

(xiii) l'altération fonctionnelle est une altération présentant un état fonctionnel probable, éventuellement dans laquelle l'altération fonctionnelle est une troncation dans un gène suppresseur de tumeur;

(xiv) l'altération fonctionnelle est une mutation conductrice, éventuellement dans laquelle la mutation conductrice est une altération qui confère un avantage sélectif à un clone dans son microenvironnement, éventuellement dans laquelle la mutation conductrice est une altération qui confère un avantage sélectif à un clone dans son microenvironnement en augmentant la survie ou la reproduction cellulaire;

(xv) l'altération fonctionnelle est une altération capable de provoquer des expansions clonales;

(xvi) l'altération fonctionnelle est une altération capable de provoquer un ou plusieurs des effets suivants :

    (a) autosuffisance dans un signal de croissance;

    (b) diminution de la sensibilité à un signal d'anti-croissance, éventuellement insensibilité à un signal d'anti-croissance;

    (c) diminution de l'apoptose;

    (d) augmentation du potentiel réplicatif;

    (e) angiogenèse soutenue; ou

    (f) invasion tissulaire ou métastase;

(xvii) l'altération fonctionnelle n'est pas une mutation passagère, éventuellement dans laquelle l'altération fonctionnelle est une altération qui a un effet détectable sur la valeur d'adaptation d'un clone;

(xviii) l'altération fonctionnelle n'est pas un variant de signification inconnue (VUS), éventuellement dans laquelle l'altération fonctionnelle n'est pas une altération dont la pathogénicité ne peut être ni confirmée ni exclue;

(xix) une pluralité d'altérations fonctionnelles dans un gène présélectionné de l'ensemble prédéterminé de gènes sont exclues, éventuellement dans lequel 10 %, 20 %, 30 %, 40 %, 50 % ou 75 % ou plus des altérations fonctionnelles dans un gène présélectionné de l'ensemble prédéterminé de gènes sont exclues, en outre éventuellement dans lequel le gène présélectionné est un gène tumoral;

(xx) toutes les altérations fonctionnelles dans un gène présélectionné de l'ensemble prédéterminé de gènes sont exclues, en outre éventuellement dans lequel le gène présélectionné est un gène tumoral;

(xxi) une pluralité d'altérations fonctionnelles dans une pluralité de gènes présélectionnés de l'ensemble prédéterminé de gènes sont exclues, éventuellement dans lequel les gènes présélectionnés sont des gènes tumoraux;

(xxii) toutes les altérations fonctionnelles dans tous les gènes de l'ensemble prédéterminé de gènes sont exclues, éventuellement dans lequel les gènes sont des gènes tumoraux;

(xxiii) l'altération germinale est exclue par l'utilisation d'un procédé qui n'utilise pas de comparaison avec une séquence normale appariée;

(xxiv) l'altération germinale est exclue par un procédé comprenant l'utilisation d'un algorithme SGZ;

(xxv) l'altération germinale est identifiée comme telle par son inclusion dans une base de données d'altérations germinales;

(xxvi) l'altération germinale est un polymorphisme mono-nucléotidique (SNP), une substitution de base, une insertion-délétion ou une mutation silencieuse, éventuellement dans lequel l'altération germinale est une mutation synonyme;

(xxvii) l'altération est une mutation silencieuse, une altération synonyme;

(xxviii) l'altération est une mutation passagère, éventuellement dans laquelle l'altération est une altération qui n'a aucun effet détectable sur la valeur d'adaptation d'un clone;

(xxix) l'altération est un variant de signification inconnue (VUS), éventuellement dans laquelle l'altération est une altération dont la pathogénicité ne peut être ni confirmée ni exclue;

(xxx) l'altération est une mutation ponctuelle;

(xxxi) l'altération est un variant court, éventuellement dans lequel le variant court est un variant codant court, en outre éventuellement dans lequel le variant court est une substitution de base, une insertion-délétion, une insertion ou une délétion;

(xxxii) l'altération est un variant mono-nucléotidique non synonyme (SNV) ;

(xxxiii) l'altération est un variant d'épissage;

(xxxiv) l'altération n'a pas été identifiée comme étant associée à un phénotype cancéreux;

(xxxv) l'altération est autre qu'un réarrangement, éventuellement dans laquelle l'altération est autre qu'une translocation;

(xxxvi) l'ensemble prédéterminé de gènes comprend une pluralité de gènes qui, sous forme mutante, sont associés à un effet sur la division, la croissance ou la survie cellulaire, ou sont associés au cancer;

(xxxvii) l'ensemble prédéterminé de gènes comprend au moins environ 50 gènes ou plus, environ 100 gènes ou plus, environ 150 gènes ou plus, environ 200 gènes ou plus, environ 250 gènes ou plus, environ 300 gènes ou plus, environ 350 gènes ou plus, environ 400 gènes ou plus, environ 450 gènes ou plus, ou environ gènes 500 ou plus; ou

(xxxviii) l'ensemble prédéterminé de gènes comprend au moins environ 50 gènes ou plus, environ 100 gènes ou plus, environ 150 gènes ou plus, environ 200 gènes ou plus, environ 250 gènes ou plus, environ 300 gènes ou plus, ou tous les gènes ou produits géniques choisis parmi les tableaux 1 à 4 ou les figures 3A à 4D.

4. Procédé selon la revendication 2, dans lequel

(i) l'acquisition d'une lecture pour l'intervalle sous-génomique comprend le séquençage d'un intervalle sous-génomique à partir d'au moins environ 50 gènes ou plus, environ 100 gènes ou plus, environ 150 gènes ou plus, environ 200 gènes ou plus, environ 250 gènes ou plus, environ 300 gènes ou plus, ou tous les gènes ou produits géniques choisis parmi les tableaux 1 à 4 ou les figures 3A à 4D;

(ii) l'acquisition d'une lecture pour l'intervalle sous-génomique comprend le séquençage avec une couverture unique moyenne supérieure à environ 250X, supérieure à environ 500X, ou supérieure à environ 1000X;

(iii) l'acquisition d'une lecture pour l'intervalle sous-génomique comprend le séquençage avec une couverture unique moyenne supérieure à environ 250X, supérieure à environ 500X, ou supérieure à environ 1000X, à plus de 95 %, à plus de 97 % environ, ou à plus de 99 % environ des gènes (par exemple, des exons) séquencés.

5. Procédé selon la revendication 1 ou 2, comprenant en outre le fait de caractériser un variant dans l'échantillon tumoral par :

a) l'acquisition :

i) d'une entrée de couverture de séquence (SCI), qui comprend, pour chacun d'une pluralité d'intervalles sous-génomiques sélectionnés, une valeur pour une couverture de séquence normalisée au niveau des intervalles sous-génomiques sélectionnés, dans laquelle l'entrée de couverture de séquence, SCI, est fonction du nombre de lectures pour un intervalle sous-génomique et du nombre de lectures pour un contrôle à processus apparié;

ii) d'une entrée de fréquence allélique de SNP (SAFI), qui comprend, pour chacun d'une pluralité de polymorphismes mono-nucléotidiques, SNP, germinaux sélectionnés, une valeur pour la fréquence allélique dans l'échantillon tumoral, dans laquelle SAFI est basée, au moins en partie, sur une fréquence allélique mineure ou alternative dans l'échantillon tumoral; et

iii) d'une entrée de fréquence allélique de variant (VAFI), qui comprend la fréquence allélique pour ledit variant dans l'échantillon tumoral;

b) l'acquisition de valeurs, en fonction de SCI et SAFI, pour :

i) un nombre de copies total de segments génomiques (C) pour chacun d'une pluralité de segments génomiques;

ii) un nombre de copies d'allèles mineurs de segments génomiques (M) pour chacun d'une pluralité de segments génomiques ; et

iii) une pureté d'échantillon (p),

dans lequel les valeurs de C, M et p sont obtenues en ajustant un modèle de nombre de copies à l'échelle du génome à SCI et SAFI; et

c) l'acquisition :

d'une valeur pour le type de mutation, g, qui est indicative du fait que le variant est somatique, un variant somatique sous-clonal, germinal, ou indiscernable, et qui est fonction de VAFI, p, C et M, éventuellement dans lequel le variant est une altération.

6. Procédé selon la revendication 5, comprenant en outre le séquençage de chacun d'une pluralité d'intervalles sous-génomiques sélectionnés, de chacun d'une pluralité de SNP germinaux sélectionnés, et d'un variant, dans lequel la couverture de séquence moyenne avant normalisation est d'au moins environ 250x, par exemple d'au moins environ 500x, éventuellement dans lequel le variant est une altération; ou

(ii) dans lequel l'ajustement du modèle de nombre de copies à l'échelle du génome à SCI comprend l'utilisation de l'équation suivante :

$$\log Ratio_i = \log_2 \frac{pC_i + 2(1-p)}{p\psi + 2(1-p)},$$

où $\psi$ est la ploïdie tumorale; ou

(iii) dans lequel l'ajustement du modèle de nombre de copies à l'échelle du génome à SAFI comprend l'utilisation de l'équation suivante :

$$AF = \frac{pM + 1(1-p)}{pC + 2(1-p)},$$

où AF est la fréquence allélique; ou

(iv) dans lequel g est déterminé en déterminant l'ajustement des valeurs de VAFI, p, C et M à un modèle pour un état somatique/germinal; ou

(v) dans lequel la valeur de g est acquise par :

$$AF = \frac{pM + g(1-p)}{pC + 2(1-p)},$$

où AF est la fréquence allélique; ou

(vi) dans lequel

une valeur de g égale à 0 ou proche de 0 indique que le variant est un variant somatique;

une valeur de g égale à 1 ou proche de 1 indique que le variant est un variant germinal;

une valeur de g inférieure à 1 mais supérieure à 0 indique un résultat indiscernable; et

une valeur de g significativement inférieure à 0 indique que le variant est un variant somatique sous-clonal.

7. Procédé selon la revendication 1 ou 2, dans lequel l'échantillon comprend une ou plusieurs cellules pré-malignes ou malignes; des cellules provenant d'une tumeur solide, d'une tumeur des tissus mous ou d'une lésion métastatique; des tissus ou des cellules provenant d'une marge chirurgicale; un tissu histologiquement normal; une ou plusieurs cellules tumorales circulantes (CTC) ; un tissu adjacent normal (NAT) ; un échantillon de sang provenant du même sujet présentant ou susceptible de présenter la tumeur; ou un échantillon FFPE, éventuellement dans lequel l'échantillon est un échantillon tumoral ou un échantillon dérivé d'une tumeur.

8. Procédé selon la revendication 1 ou 2, dans lequel l'échantillon est un échantillon FFPE.

9. Procédé selon la revendication 8, dans lequel l'échantillon FFPE présente une, deux ou toutes les propriétés suivantes :

(a) il a une surface de 25 mm$^2$ ou plus ;
(b) il a un volume d'échantillon de 1 mm$^3$ ou plus; ou
(c) il a une cellularité nucléée de 80 % ou plus ou 30000 cellules ou plus.

10. Procédé selon la revendication 1 ou 2,

(i) dans lequel l'échantillon est un échantillon comprenant de l'ADN tumoral circulant (ctDNA) ;
(ii) dans lequel l'échantillon est acquis à partir d'une tumeur solide, d'un cancer hématologique ou d'une forme métastatique de celui/celle-ci;
(iii) comprenant en outre le classement de l'échantillon tumoral ou du sujet à partir duquel l'échantillon tumoral a été prélevé en réponse à l'évaluation de la charge mutationnelle tumorale; ou
(iv) comprenant en outre la génération d'un rapport destiné au patient ou à une autre personne ou entité, un soignant, un médecin, un oncologue, un hôpital, une clinique, un tiers payeur, une compagnie d'assurance ou un organisme gouvernemental; éventuellement dans lequel ledit rapport comprend le résultat du procédé qui comprend la charge mutationnelle tumorale, éventuellement dans lequel le rapport est un rapport électronique, sur le web ou sur papier.

**11.** Système pour évaluer la charge mutationnelle tumorale dans un échantillon, comprenant :
au moins un processeur connecté de manière opérationnelle à une mémoire, l'au moins un processeur, lorsqu'il est en cours d'exécution, étant configuré pour :

a) acquérir indirectement une séquence nucléotidique d'un ensemble d'intervalles sous-génomiques à partir de l'échantillon tumoral, dans lequel l'ensemble d'intervalles sous-génomiques provient d'un ensemble prédéterminé de gènes qui ne comprend pas le génome entier ou l'exome entier, dans lequel l'ensemble prédéterminé de gènes comprend une pluralité de gènes qui, sous forme mutante, sont associés à un effet sur la division, la croissance ou la survie cellulaire, ou sont associés au cancer; et

b) déterminer une valeur pour la charge mutationnelle tumorale, dans laquelle la valeur est fonction du nombre d'altérations dans l'ensemble d'intervalles sous-génomiques, dans lequel ledit nombre d'altérations exclut :

(i) une altération fonctionnelle dans un intervalle sous-génomique, dans laquelle l'altération fonctionnelle est une altération qui, par rapport à une séquence de référence, a un effet sur la division, la croissance ou la survie cellulaire, et dans laquelle l'altération fonctionnelle est identifiée comme telle sur la base de son inclusion dans une base de données d'altérations fonctionnelles, éventuellement dans lequel l'intervalle sous-génomique est un intervalle sous-génomique codant; et

(ii) une altération germinale dans un intervalle sous-génomique, éventuellement dans lequel l'intervalle sous-génomique est un intervalle sous-génomique codant.

Sample Receipt and QC; DNA Isolation

Fig. 1A

DNA QC and Library Generation

Fig. 1B

Fig. 1C

# Sequence data QC and mutation calling

## Quality Control

Fig. 1D

# Report Generation

Fig. 1E

Fig. 1F

Impact of prior expectation and read depth on mutation detection

Estimated power to detect mutation (sensitivity)

Coverage depth at mutated site

—O— 1% mutation, 1e-6 prior
--O-- 1% mutation, 10% prior
—□— 5% mutation, 1e-6 prior
--□-- 5% mutation, 10% prior
—△— 10% mutation, 1e-6 prior
--△-- 10% mutation, 10% prior

Notes:

1) Assumed sequencing error rate = 0.002 (~Q27)

2) Mutation called if P(Mutation|Reads)>0.99

2) Specificity in all scenarios >99.9%

Fig. 2

| | | | | | | | | | |
|---|---|---|---|---|---|---|---|---|---|
| ABL1 | BRAF | CHEK1 | FANCC | GATA3 | JAK2 | MITF | PDCD1LG2 | RBM10 | STAT4 |
| ABL2 | BRCA1 | CHEK2 | FANCD2 | GATA4 | JAK3 | MLH1 | PDGFRA | RET | STK11 |
| ACVR1B | BRCA2 | CIC | FANCE | GATA6 | JUN | MPL | PDGFRB | RICTOR | SUFU |
| AKT1 | BRD4 | CREBBP | FANCF | GID4 (C17orf39) | KAT6A (MYST3) | MRE11A | PDK1 | RNF43 | SYK |
| AKT2 | BRIP1 | CRKL | FANCG | GLI1 | KDM5A | MSH2 | PIK3C2B | ROS1 | TAF1 |
| AKT3 | BTG1 | CRLF2 | FANCL | GNA11 | KDM5C | MSH6 | PIK3CA | RPTOR | TBX3 |
| ALK | BTK | CSF1R | FAS | GNA13 | KDM6A | MTOR | PIK3CB | RUNX1 | TERC |
| AMER1 (FAM123B) | C11orf30 (EMSY) | CTCF | FAT1 | GNAQ | KDR | MUTYH | PIK3CG | RUNX1T1 | TERT (PROMOTER ONLY) |
| APC | CARD11 | CTNNA1 | FBXW7 | GNAS | KEAP1 | MYC | PIK3R1 | SDHA | TET2 |
| AR | CBFB | CTNNB1 | FGF10 | GPR124 | KEL | MYCL (MYCL1) | PIK3R2 | SDHB | TGFBR2 |
| ARAF | CBL | CUL3 | FGF14 | GRIN2A | KIT | MYCN | PLCG2 | SDHC | TNFAIP3 |
| ARFRP1 | CCND1 | CYLD | FGF19 | GRM3 | KLHL6 | MYD88 | PMS2 | SDHD | TNFRSF14 |
| ARID1A | CCND2 | DAXX | FGF23 | GSK3B | KMT2A (MLL) | NF1 | POLD1 | SETD2 | TOP1 |
| ARID1B | CCND3 | DDR2 | FGF3 | H3F3A | KMT2C (MLL3) | NF2 | POLE | SF3B1 | TOP2A |
| ARID2 | CCNE1 | DICER1 | FGF4 | HGF | KMT2D (MLL2) | NFE2L2 | PPP2R1A | SLIT2 | TP53 |
| ASXL1 | CD274 | DNMT3A | FGF6 | HNF1A | KRAS | NFKBIA | PRDM1 | SMAD2 | TSC1 |
| ATM | CD79A | DOT1L | FGFR1 | HRAS | LMO1 | NKX2-1 | PREX2 | SMAD3 | TSC2 |
| ATR | CD79B | EGFR | FGFR2 | HSD3B1 | LRP1B | NOTCH1 | PRKAR1A | SMAD4 | TSHR |
| ATRX | CDC73 | EP300 | FGFR3 | HSP90AA1 | LYN | NOTCH2 | PRKCI | SMARCA4 | U2AF1 |
| AURKA | CDH1 | EPHA3 | FGFR4 | IDH1 | LZTR1 | NOTCH3 | PRKDC | SMARCB1 | VEGFA |
| AURKB | CDK12 | EPHA5 | FH | IDH2 | MAGI2 | NPM1 | PRSS8 | SMO | VHL |
| AXIN1 | CDK4 | EPHA7 | FLCN | IGF1R | MAP2K1 | NRAS | PTCH1 | SNCAIP | WISP3 |
| AXL | CDK6 | EPHB1 | FLT1 | IGF2 | MAP2K2 | NSD1 | PTEN | SOCS1 | WT1 |
| BAP1 | CDK8 | ERBB2 | FLT3 | IKBKE | MAP2K4 | NTRK1 | PTPN11 | SOX10 | XPO1 |
| BARD1 | CDKN1A | ERBB3 | FLT4 | IKZF1 | MAP3K1 | NTRK2 | QKI | SOX2 | ZBTB2 |
| BCL2 | CDKN1B | ERBB4 | FOXL2 | IL7R | MCL1 | NTRK3 | RAC1 | SOX9 | ZNF217 |
| BCL2L1 | CDKN2A | ERG | FOXP1 | INHBA | MDM2 | NUP93 | RAD50 | SPEN | ZNF703 |
| BCL2L2 | CDKN2B | ERRFI1 | FRS2 | INPP4B | MDM4 | PAK3 | RAD51 | SPOP | |
| BCL6 | CDKN2C | ESR1 | FUBP1 | IRF2 | MED12 | PALB2 | RAF1 | SPTA1 | |
| BCOR | CEBPA | EZH2 | GABRA6 | IRF4 | MEF2B | PARK2 | RANBP2 | SRC | |
| BCORL1 | CHD2 | FAM46C | GATA1 | IRS2 | MEN1 | PAX5 | RARA | STAG2 | |
| BLM | CHD4 | FANCA | GATA2 | JAK1 | MET | PBRM1 | RB1 | STAT3 | |

Fig. 3A

| SELECT REARRANGEMENTS | | | | | | | | | |
|------|-------|------|------|-------|------|--------|--------|------|---------|
| ALK | BRAF | BRD4 | ETV4 | FGFR1 | KIT | MYC | NTRK2 | RARA | TMPRSS2 |
| BCL2 | BRCA1 | EGFR | ETV5 | FGFR2 | MSH2 | NOTCH2 | PDGFRA | RET | |
| BCR | BRCA2 | ETV1 | ETV6 | FGFR3 | MYB | NTRK1 | RAF1 | ROS1 | |

Fig. 3B

| DNA Gene List: Entire Coding Sequence (Base Substitutions, Indels, Copy Number Alterations) | | | | | | | | |
|---|---|---|---|---|---|---|---|---|
| ABL1 | BTLA | CXCR4 | FGF23 | HIST1H2BJ | LRRK2 | NOTCH1 | RAD21 | STAT5A |
| ACTB | C11orf30 (EMSY) | DAXX | FGF3 | HIST1H2BK | MAF | NOTCH2 | RAD50 | STAT5B |
| AKT1 | CAD | DDR2 | FGF4 | HIST1H2BO | MAFB | NPM1 | RAD51 | STAT6 |
| AKT2 | CAD11 | DDX3X | FGF6 | HIST1H3B | MAGED1 | NRAS | RAF1 | STK11 |
| AKT3 | CBFB | DNM2 | FGFR1 | HNF1A | MALT1 | NT5C2 | RARA | SUFU |
| ALK | CBL | DNMT3A | FGFR2 | HRAS | MAP2K1 | NTRK1 | RASGEF1A | SUZ12 |
| AMER1 (FAM123B or WTX) | CCND1 | DOT1L | FGFR3 | HSP90AA1 | MAP2K2 | NTRK2 | RB1 | TAF1 |
| APC | CCND2 | DTX1 | FGFR4 | ICK | MAP2K4 | NTRK3 | RELN | TBL1XR1 |
| APH1A | CCND3 | DUSP2 | FHIT | ID3 | MAP3K1 | NUP93 | RET | TCF3 (E2A) |
| AR | CCNE1 | DUSP9 | FLCN | IDH1 | MAP3K14 | NUP98 | RHOA | TCL1A (TCL1) |
| ARAF | CCT68 | EBF1 | FLT1 | IDH2 | MAP3K6 | P2RY8 | RICTOR | TET2 |

# Fig. 4A

| DNA Gene List: Entire Coding Sequence (Base Substitutions, Indels, Copy Number Alterations) | | | | | | | | |
|---|---|---|---|---|---|---|---|---|
| ARFRP1 | CD22 | ECT2L | FLT3 | IGF1R | MAP3K7 | PAG1 | RNF43 | TGFBR2 |
| ARHGAP26 (GRAF) | CD274 (PDL1) | EED | FLT4 | IKBKE | MAPK1 | PAK3 | ROS1 | TLL2 |
| ARID1A | CD36 | EGFR | FLYWCH1 | IKZF1 | MCL1 | PALB2 | RPTOR | TMEM30A |
| ARID2 | CD58 | ELP2 | FOXL2 | IKZF2 | MDM2 | PASK | RUNX1 | TMSB4XP8 (TMSL3) |
| ASMTL | CD70 | E9300 | FOXO1 | IKZF3 | MDM4 | PAX5 | S1PR2 | TNFAIP3 |
| ASXL1 | CD79A | EPHA3 | FOXO3 | IL7R | MED12 | PBRM1 | SDHA | TNFRSF11A |
| ATM | CD79B | EPHAS | FOXP1 | INHBA | MEF2B | PC | SDHB | TNFRSF14 |
| ATR | CDC73 | EPHA7 | FRS2 | INPP4B | MEF2C | PCBP1 | SDHC | TNFRSF17 |
| ATRX | CDH1 | EPHB1 | GADD45B | INPP5D (SHIP) | MEN1 | PCLO | SDHD | TOP1 |
| AURKA | CDK12 | ERBB2 | GATA1 | IRF1 | MET | PDCD1 | SERP2 | TP53 |
| AURKB | CDK4 | ERBB3 | GATA2 | IRF4 | MIB1 | PDCD11 | SETBP1 | TP63 |
| AXIN1 | CDK6 | ERBB4 | GATA3 | IRF8 | MITF | PDCD1LG2 (PDL2) | SETBP1 | TP63 |
| AXL | CDK8 | ERG | GID4 (C17orf39) | IRS2 | MKI67 | PDGFRA | SF3B1 | TRAF3 |
| B2M | CDKN1B | ESR1 | GNA11 | JAK1 | MLH1 | PDGFRB | SGK1 | TRAF5 |
| BAP1 | CDKN2A | ETS1 | GNA12 | JAK2 | MPL | PDK1 | SMAD2 | TSC1 |
| BARD1 | CDKN2B | ETV6 | GNA13 | JAK3 | MRE11A | PHF6 | SMAD4 | TSC2 |
| BCL10 | CDKN2C | EXOSC6 | GNAQ | JARID2 | MSH2 | PIK3CA | SMARCA1 | TSHR |
| BCL11B | CEBPA | EZH2 | GNAS | JUN | MSH3 | PIK3CG | SMARCA4 | TUSC3 |
| BCL2 | CHD2 | FAF1 | GPR124 | KAT6A (MYST3) | MSH6 | PIK3R1 | SMARCB1 | TYK2 |
| BCL2L2 | CHEK1 | FAM46C | GRIN2A | KDM2B | MTOR | PIK3R2 | SMC1A | U2AF1 |
| BCL6 | CHEK2 | FANCA | GSK3B | KDM4C | MUTYH | PIM1 | SMC3 | U2AF2 |
| BCL7A | CIC | FANCC | GTSE1 | KDM5A | MYC | PLCG2 | SMO | VHL |
| BCOR | IITA | FANCD2 | HDAC1 | KDM5C | MYCL (MYCL1) | POT1 | SOCS1 | WDR90 |
| BCORL1 | CKS1B | FANCE | HDAC4 | KDM6A | MYCN | PPP2R1A | SOCS2 | WHSC1 (MMSET or NSD2) |
| BIRC3 | CPS1 | FANCF | HDAC7 | KDR | MYD88 | PRDM1 | SOCS3 | WISP3 |
| BLM | CREBBP | FANCG | HGF | KEAP1 | MYO18A | PRKAR1A | SOX10 | WT1 |
| BRAF | CRKL | FANCL | HIST1H1C | KIT | NCOR2 | PRKDC | SOX2 | XBP1 |
| BRCA1 | CRLF2 | FAS (TNFRSF6) | HIST1H1D | KLHL6 | NCSTN | PRSS8 | SPEN | XBO1 |
| BRCA2 | CRF1R | FBXO11 | HIST1H1E | KMT2A (MLL) | NF1 | PTCH1 | SPOP | YY1AP1 |
| BRD4 | CRF3R | FBXO31 | HIST1H2AC | KMT2C (MLL3) | NF2 | PTEN | SRC | ZMYM3 |
| BRIP1 | CTCF | FBXW7 | HIST1H2AG | KMT2D (MLL2) | NFE2L2 | PTPN11 | SRSF2 | ZNF217 |
| BRSK1 | CTNNA1 | FGF10 | HIST1H2AL | KRAS | NFKBIA | PTPN2 | STAG2 | ZNF24 (ZSCAN3) |
| BTG2 | CTNNB1 | FGF14 | HIST1H2AM | LEF1 | NKX2-1 | PTPN6 (SHP-1) | STAT3 | ZNF703 |
| BTK | CUX1 | FGF19 | HIST1H2BC | RP1B | NOD1 | PTPRO | STAT4 | ZRSR2 |

## Fig. 4B

| Select DNA Rearrangements | | | | | | | | |
|---|---|---|---|---|---|---|---|---|
| ALK | BRAF | EPOR | ETV6 | IGK | JAK2 | NTRK1 | RAF1 | ROS1 |
| BCL2 | CCND1 | ETV1 | EWSR1 | IGL | KMT2A (MLL) | PDGFRA | RARA | TMPRSS2 |
| BCL6 | CRLF2 | ETV4 | FGFR2 | JAK1 | MYC | PDGFRB | RET | TRG |
| BCR | EGFR | ETV5 | IGH | | | | | |

## Fig. 4C

| Select Gene Fusions | | | | | | | | |
|---|---|---|---|---|---|---|---|---|
| ABI1 | CBFA2T3 | EIF4A2 | FUS | JAK1 | MUC1 | PBX1 | RNF213 | TET1 |
| ABL1 | CBFB | ELF4 | GAS7 | JAK2 | MYB | PCM1 | ROS1 | TFE3 |
| ABL2 | CBL | ELL | GLI1 | JAK3 | MYC | PCSK7 | RPL22 | TFG |
| ACSL6 | CCND1 | ELN | GMPS | JAZF1 | MYH11 | PDCD1LG2 (PDL2) | RPN1 | TFPT |
| AFF1 | CCND2 | EML4 | GPHN | KAT6A (MYST3) | MYH9 | PDE4DIP | RUNX1 | TFRC |
| AFF4 | CCND3 | EP300 | HERPUD1 | KDSR | NACA | PDGFB | RUNX1T1 (ETO) | TLX1 |
| ALK | CD274 (PDL1) | EPOR | HEY1 | KIF5B | NBEAP1 (BCL8) | PDGFRA | RUNX2 | TLX3 |
| ARHGAP26 (GRAF) | CDK6 | EPS15 | HIP1 | KMT2A (MLL) | NCOA2 | PDGFRB | SEC31A | TMPRSS2 |
| ARHGEF12 | CDX2 | ERBB2 | HIST1H4I | LASP1 | NDRG1 | PER1 | SEPT5 | TNFRSF11A |
| ARID1A | CHIC2 | ERG | HLF | LCP1 | NF1 | PHF1 | SEPT6 | TOP1 |
| ARNT | CHN1 | ETS1 | HMGA1 | LMO1 | NF2 | PICALM | SEPT9 | TP63 |
| ASXL1 | CIC | ETV1 | HMGA2 | LMO2 | NFKB2 | PIM1 | SET | TPM3 |
| ATF1 | CIITA | ETV4 | HOXA11 | LPP | NIN | PLAG1 | SH3GL1 | TPM4 |
| ATG5 | CLP1 | ETV5 | HOXA13 | LYL1 | NOTCH1 | PML | SLC1A2 | TRIM24 |
| ATIC | CLTC | ETV6 | HOXA3 | MAF | NPM1 | POU2AF1 | SNX29 (RUNDC2A) | TRIP11 |
| BCL10 | CLTCL1 | EWSR1 | HOXA9 | MAF8 | NR4A3 | PPP1CB | SRSF3 | TTL |
| BCL11A | CNTRL (CEP110) | FCGR2B | HOXC11 | MALT1 | NSD1 | PRDM1 | SS18 | TYK2 |
| BCL11B | COL1A1 | FCRL4 | HOXC13 | MDS2 | NTRK1 | PRDM16 | SSX1 | USP6 |
| BCL2 | VREB3L1 | FEV | HOXD11 | MECOM | NTRK2 | PRRX1 | SSX2 | WHSC1 (MMSET or NSD2) |
| BCL3 | CREB3L2 | FGFR1 | HOXD13 | MKL1 | NTRK3 | PSIP1 | SSX4 | WHSC1L1 |
| BCL6 | CREBBP | FGFR1OP | HSP90AA1 | MLF1 | NUMA1 | PTCH1 | STAT6 | YPEL5 |
| BCL7A | CRLF2 | FGFR2 | HSP90AB1 | MLLT1 (ENL) | NUP214 | PTK7 | STL | ZBTB16 |
| BCL9 | CSF1 | FGFR3 | IGH | MLLT10 (AF10) | NUP98 | RABEP1 | SYK | ZMYM2 |
| BCOR | CTNNB1 | FLI1 | IGK | MLLT3 | NUTM2A | RAF1 | TAF15 | ZNF384 |
| BCR | DDIT3 | FNBP1 | IGL | MLLT4 (AF6) | OMD | RALGDS | TAL1 | ZNF521 |
| BIRC3 | DDX10 | FOXO1 | IKZF1 | MLLT6 | P2RY8 | RAP1GDS1 | TAL2 | |
| BRAF | DDX6 | FOXO3 | IL21R | MN1 | PAFAH182 | RARA | TBL1XR1 | |
| BTG1 | DEK | FOXO4 | IL3 | MNX1 | PAX3 | RBM15 | TCF3 (E2A) | |
| CAMTA1 | DUSP22 | FOXP1 | IRF4 | MSI2 | PAX5 | RET | TCL1A (TCL1) | |
| CARS | EGFR | FSTL3 | ITK | MSN | PAX7 | RHOH | TEC | |

Fig. 4D

136

Fig. 5

Fig. 6

Lung Adenocarcinoma TMB Distribution

Fig. 7A

Lung Squamous Cell Carcinoma TMB Distribution

Fig. 7B

Lung Large Cell Carcinoma TMB Distribution

Fig. 7C

Lung Small Cell Carcinoma TMB Distribution

Fig. 7D

Fig. 8A

Fig. 8B

EP 3 423 828 B1

Fig. 8C

EP 3 423 828 B1

Fig. 8D

Fig. 8E

Colon Adenocarcinoma TMB Distribution

Fig. 9A

Rectum Adenocarcinoma TMB Distribution

Fig. 9B

Fig. 10A

EP 3 423 828 B1

Fig. 10B

EP 3 423 828 B1

Gene Prevalence in Colorectal Adenocarcinoma

Fig. 10C

TMB Distribution in Twenty-Four Types of
Neoplasms

Fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62301534
- WO 2014183078 A
- US 20140336996
- WO 2012092426 A
- US 20100029498 A
- WO 2016090273 A

### Non-patent literature cited in the description

- *Science*, 2015, vol. 350, 158-9
- **CAMPESATO, L.F. et al.** Comprehensive cancer-gene panels can be used to estimate mutational load and predict clinical benefit to PD-1 blockade in clinical practice. *Oncotarget*, 2017, vol. 6 (33), 34221-34227
- **FORBES et al.** *Nucl. Acids Res.*, 2015, vol. 43, D805-D811
- **SHERRY et al.** *Nucleic Acids Res.*, 2001, vol. 29 (1), 308-311
- **CONSORTIUM et al.** Analysis of protein-coding genetic variation in 60,706 humans. *bioRxiv preprint.*, 30 October 2015
- **MCVEAN et al.** *Nature*, 2012, vol. 491, 56-65
- **SUN et al.** *Cancer Research*, 2014, vol. 74 (19S), 1893-1893
- **METZKER, M.** *Nature Biotechnology Reviews*, 2010, vol. 11, 31-46
- **GUBIN ; SCHREIBER.** *Science*, 2015, vol. 350, 158-9
- **CRONIN M. et al.** *Am J Pathol.*, 2004, vol. 164 (1), 35-42
- **MASUDA N. et al.** *Nucleic Acids Res.*, 1999, vol. 27 (22), 44364443
- **PECHT K. et al.** *Am J Pathol.*, 2001, vol. 158 (2), 419-429
- **GNIRKE, A. et al.** *Nat Biotechnol.*, 2009, vol. 27 (2), 182-189
- **ALBERT, T.J. et al.** *Nat. Methods*, 2007, vol. 4 (11), 903-5
- **HODGES, E et al.** *Nat. Genet.*, 2007, vol. 39 (12), 1522-7
- **OKOU, D.T. et al.** *Nat. Methods*, 2007, vol. 4 (11), 907-9
- **KAUR, H ; ARORA, A ; WENGEL. J ; MAITI, S ; ARORA, A. ; WENGEL, J ; MAITI, S.** Thermodynamic, Counterion, and Hydration Effects for the Incorporation of Locked Nucleic Acid Nucleotides into DNA Duplexes. *Biochemistry*, 2006, vol. 45 (23), 7347-55
- **EGHOLM, M et al.** *Nature*, 1993, vol. 365 (6446), 566-8
- **TRAPNELL C. ; SALZBERG S.L.** *Nature Biotech.*, 2009, vol. 27, 455-457
- **WARREN R. et al.** *Bioinformatics*, 2007, vol. 23, 500-501
- **BUTLER J et al.** *Genome Res.*, 2008, vol. 18, 810-820
- **ZERBINO D.R. ; BIRNEY E.** *Genome Res.*, 2008, vol. 18, 821-829
- **LI H. ; DURBIN R.** *Bioinformatics*, 2010, vol. 26 (5), 589-95
- **BROWNING B.L. ; YU Z.** *Am. J. Hum. Genet.*, 2009, vol. 85 (6), 847-61
- **LI Y. et al.** *Annu. Rev. Genomics Hum. Genet.*, 2009, vol. 10, 387-406
- *SNVMix -Bioinformatics.*, 15 March 2010, vol. 26 (6), 730-736
- **MCKENNA A. et al.** *Genome Res.*, 2010, vol. 20 (9), 1297-303
- **YE K. et al.** *Bioinformatics*, 2009, vol. 25 (21), 2865-71
- **LUNTER G. ; GOODSON M.** *Genome Res.*, 2010
- **LI H. et al.** *Bioinformatics*, 2009, vol. 25 (16), 2078-9
- **ALBERS C.A. et al.** *Genome Res.*, 2011, vol. 21 (6), 961-73
- **S.Q. ; DURBIN R.** *Genome Res.*, 2011, vol. 21 (6), 952-60
- **CANCER GENOME ATLAS RESEARCH NETWORK et al.** *Nat Genet*, 2013, vol. 45, 1113-20
- **BERGER et al.** *Nature*, 2012, vol. 485, 502-6
- **BAMFORD et al.** *Br J Cancer*, 2004, vol. 91, 355-8
- **LEK et al.** *Nature*, 2016, vol. 536, 285-91
- **FRAMPTON et al.** *Nat Biotech*, 2013, vol. 31, 1023-1031
- **HE et al.** *Blood*, 2016, vol. 127, 3004-14

- **ALEXANDROV** et al. *Nature*, 2013, vol. 500, 415-21
- **LAWRENCE** et al. *Nature*, 2013, vol. 499, 214-8